# EUROPEAN PATENT SPECIFICATION

(11) **EP 1 525 209 B1**
(45) Date of publication and mention of the grant of the patent: **15.11.2017**
(21) Application number: 03761749.5
(22) Date of filing: 27.06.2003
(51) Int. Cl.: C07H 19/06, C07H 19/10, C07H 19/16, C07H 19/20, A61K 31/7064, A61K 31/7076, A61P 31/14

(54) **1'-, 2'- AND 3' -MODIFIED NUCLEOSIDE DERIVATIVES FOR TREATING FLAVIVIRIDAE INFECTIONS**
1'-, 2'- UND 3'-MODIFIZIERTE NUKLEOSIDDERIVATE ZUR BEHANDLUNG VON FLAVIVIRIDAE INFEKTIONEN
DES 1'-, 2'- ET 3'- DÉRIVES MODIFIÉS DES NUCLÉOSIDES PERMETTANT DE TRAITER DES INFECTIONS PAR LES FLAVIVIRIDAE

(30) Priority: 28.06.2002 US 392351 P; 28.06.2002 US 392350 P; 28.04.2003 US 466194 P; 14.05.2003 US 470949 P
(43) Date of publication of application: 27.04.2005
(73) Proprietor: Idenix Pharmaceuticals LLC, Cambridge, MA 02141 (US); CENTRE NATIONAL DE LA RECHERCHE SCIENTIFIQUE -CNRS-, 75794 Paris Cedex 16 (FR); Università Degli Studi di Cagliari, 09124 Cagliari (IT); Université de Montpellier, 34090 Montpellier (FR)
(72) Inventor: SOMMADOSSI, Jean-Pierre, Cambridge, MA 02138 (US); LA COLLA, Paolo, 00128 Roma (IT); STORER, Richard, Folkestone, Kent CT20 2JE (GB); GOSSELIN, Gilles, 34070 Montpellier (FR)
(74) Representative: Savic, Bojan
(86) International application number: PCT/IB2003/003901
(87) International publication number: WO 2004/003000

(56) References cited:
- EP-A- 0 747 389
- WO-A-01/90121
- WO-A-01/92282
- GB-A- 1 163 102
- GB-A- 1 163 103
- GB-A- 1 209 654
- FARKAS J ET AL: "NUCLEIC ACIDS COMPONENTS AND THEIR ANALOGUES. XCIV. SYNTHESIS OF 6-AMINO-9-(1-DEOXY-BETA-D-PSICOFURANOSYL)P URINE" COLLECTION OF CZECHOSLOVAK CHEMICAL COMMUNICATIONS, ACADEMIC PRESS, LONDON, GB, vol. 32, no. 7, 1967, pages 2663-2667, XP001016337 ISSN: 0010-0765
- HREBABECKY H ET AL: "Synthesis of 7- and 9-beta-D-psicofuranosylguanine and their 1'-deoxy derivatives" COLLECTION OF CZECHOSLOVAK CHEMICAL COMMUNICATIONS, ACADEMIC PRESS, LONDON, GB, vol. 39, 1974, pages 2115-2123, XP002176340 ISSN: 0010-0765
- L. CAPPELLACCI ET AL.: "Ribose-modified nucleosides as ligands for adenosine receptors: Synthesis, conformational analysis, and biological evaluation of 1'-C-methyl denosine analogues" J. MED. CHEM., vol. 45, 2002, pages 1196-1202, XP002268196
- FRANCHETTI P ET AL: "2'-C-Methyl analogues of selective adenosine receptor agonists: Synthesis and binding studies" JOURNAL OF MEDICINAL CHEMISTRY, AMERICAN CHEMICAL SOCIETY. WASHINGTON, US, vol. 41, 1998, pages 1708-1715, XP002189348 ISSN: 0022-2623
- HARRY-O'KURU R E ET AL: "A SHORT, FLEXIBLE ROUTE TOWARD 2'-C-BRANCHED RIBONUCLEOSIDES" JOURNAL OF ORGANIC CHEMISTRY, AMERICAN CHEMICAL SOCIETY. EASTON, US, vol. 62, no. 6, 1997, pages 1754-1759, XP002250483 ISSN: 0022-3263
- WOLF J ET AL: "NEW 2'-C-BRANCHED-CHAIN SUGAR NUCLEOSIDE ANALOGS WITH POTENTIAL ANTIVIRAL OR ANTITUMOR ACTIVITY" SYNTHESIS, GEORG THIEME VERLAG. STUTTGART, DE, no. 8, August 1992 (1992-08), pages 773-778, XP001154225 ISSN: 0039-7881
- J.M.J. TRONCHET, J.F. TRONCHET: "72. Synthèse et désamination enzymatique des C-hydroxyméthyl-3'-et C-méthyl-3'-beta-D-xylofurannosyl-9-adénin es" HELV. CHIM. ACTA, vol. 62, 1979, pages 689-695, XP002287426
- ONG ET AL: "Synthesis of 3'-C-methyladenosine and 3'-C-methyluridine diphosphates and their interaction with the ribonucleoside diphosphate reductase from Corynebacterium nephridii" BIOCHEMISTRY, AMERICAN CHEMICAL SOCIETY. EASTON, PA, US, vol. 31, 1992, pages 11210-11215, XP002083792 ISSN: 0006-2960
- HREBABECKY H ET AL: "Nucleic acid components and their analogues. CXLIX. Synthesis of pyrimidine nucleosides derived from 1-deoxy-D-psicose" COLLECTION OF CZECHOSLOVAK CHEMICAL COMMUNICATIONS, ACADEMIC PRESS, LONDON, GB, vol. 37, 1972, pages 2059-2065, XP002176338 ISSN: 0010-0765
- MATSUDA A ET AL: "Radical deoxygenation of tert-alcohols in 2'-branched-chain sugar pyrimidine nucleosides: synthesis and antileukemic activity of 2'-deoxy-2'(S)-methylcytidine" CHEMICAL AND PHARMACEUTICAL BULLETIN, PHARMACEUTICAL SOCIETY OF JAPAN. TOKYO, JP, vol. 35, 1987, pages 3967-3970, XP002189355 ISSN: 0009-2363
- MATSUDA A ET AL: "Nucleosides and nucleotides. 94. Radical deoxygenation of tert-alcohols in 1-(2-C-alkylpentafuranosyl)pyrimidines: Synthesis of (2'S)-2'-deoxy-2'-C-methylcytidine, an antileukemic nucleoside" JOURNAL OF MEDICINAL CHEMISTRY, AMERICAN CHEMICAL SOCIETY. WASHINGTON, US, vol. 34, 1991, pages 234-239, XP002178370 ISSN: 0022-2623
- BEIGELMAN L N ET AL: "A GENERAL METHOD FOR SYNTHESIS OF 3'-C-ALKYLNUCLEOSIDES" NUCLEIC ACIDS SYMPOSIUM SERIES, IRL PRESS, OXFORD, GB, vol. 9, no. 9, 6 September 1981 (1981-09-06), pages 115-118, XP001059721 ISSN: 0261-3166
- ROSENTHAL A ET AL: "Branched-chain sugar nucleosides. Synthesis of 3'-C-ethyl (and 3'-C-butyl)uridine" CARBOHYDRATE RESEARCH, ELSEVIER SCIENTIFIC PUBLISHING COMPANY. AMSTERDAM, NL, vol. 79, 1980, pages 235-242, XP002189368 ISSN: 0008-6215
- MIKHAILOV S N ET AL: "Synthesis and properties of 3'-C-methylnucleosides and their phosphoric esters" CARBOHYDRATE RESEARCH, ELSEVIER SCIENTIFIC PUBLISHING COMPANY. AMSTERDAM, NL, vol. 124, 1983, pages 75-96, XP002189357 ISSN: 0008-6215
- AWANO H ET AL: "NUCLEOSIDES AND NUCLEOTIDES, PART 144 SYNTHESIS AND ANTIVIRAL ACTIVITY OF 5-SUBSTITUTED (2'S)-2'-DEOXY-2'-C-METHYLCYTIDINES AND -URIDINES" ARCHIV DER PHARMAZIE, VCH VERLAGSGESELLSCHAFT MBH, WEINHEIM, DE, vol. 329, 1 February 1996 (1996-02-01), pages 66-72, XP000611444 ISSN: 0365-6233
- N.-S. LI ET AL.: "2'-C-Branched ribonucleosides. 2. Synthesis of 2'-C-beta-trifluoromethyl pyrimidine ribonucleosides" ORG. LETT., vol. 3, 2001, pages 1025-1028, XP002287440
- SCHMIT C: "SYNTHESIS OF 2'-DEOXY-2'-ALPHA-MONOFLUOROMETHYL AND TRIFLUOROMETHYLNUCLEOSIDES" SYNLETT, THIEME VERLAG, STUTTGART, DE, no. 4, 1994, pages 241-242, XP002056163 ISSN: 0936-5214
- JOHNSON ET AL: "3'-C-Trifluoromethyl ribonucleosides" NUCLEOSIDES & NUCLEOTIDES, MARCEL DEKKER, INC, US, vol. 14, no. 1/2, 1995, pages 185-194, XP002083791 ISSN: 0732-8311
- SHARMA P K ET AL: "SYNTHESIS OF 3'-TRIFLUOROMETHYL NUCLEOSIDES AS POTENTIAL ANTIVIRAL AGENTS" NUCLEOSIDES, NUCLEOTIDES AND NUCLEIC ACIDS, MARCEL DEKKER, ANN HARBOR, MI, US, vol. 19, no. 4, 2000, pages 757-774, XP001050475 ISSN: 1525-7770
- MURAL Y ET AL: "A SYNTHESIS AND AN X-RAY ANALYSIS OF 2'-C-, 3'-C- AND 5'-C-METHYLSANGIVAMYCINS" HETEROCYCLES, XX, XX, vol. 1, no. 33, 1992, pages 391-404, XP008004758 ISSN: 0385-5414
- FEDEROV I I ET AL: "3'-C-Branched 2'-deoxy-5-methyluridines: Synthesis, enzyme inhibition, and antiviral properties" JOURNAL OF MEDICINAL CHEMISTRY, AMERICAN CHEMICAL SOCIETY. WASHINGTON, US, vol. 35, 1992, pages 4567-4575, XP002189365 ISSN: 0022-2623
- H. HATTORI ET AL.: "Nucleosides and nucleotides. 158." J. MED. CHEM., vol. 39, 1996, pages 5005-5011, XP002287441

## Description

### CROSS-REFERENCE To RELATED APPLICATIONS

### FIELD OF THE INVENTION

This invention is in the area of pharmaceutical chemistry, and is in particular, a 2' and/or 3' prodrug of a 1', 2', 3' or 4'-branched nucleosides for the treatment of a *Flaviviridae* infection, such as a hepatitis C virus infection.

### BACKGROUND OF THE INVENTION

### Flaviviridae Viruses

The Flaviviridae family of viruses comprises at least three distinct genera: *pestiviruses,* which cause disease in cattle and pigs; *flaviviruses,* which are the primary cause of diseases such as dengue fever and yellow fever; and *hepaciviruses,* whose sole member is HCV. The flavivirus genus includes more than 68 members separated into groups on the basis of serological relatedness (Calisher et al., J. Gen. Virol, 1993, 70, 37-43). Clinical symptoms vary and include fever, encephalitis and hemorrhagic fever (Fields Virology, Editors: Fields, B. N., Knipe, D. M., and Howley, P. M., Lippincott-Raven Publishers, Philadelphia, PA, 1996, Chapter 31, 931-959). Flaviviruses of global concern that are associated with human disease include the dengue hemorrhagic fever viruses (DHF), yellow fever virus, shock syndrome and Japanese encephalitis virus (Halstead, S. B., Rev. Infect. Dis., 1984, 6, 251-264; Halstead, S. B., Science, 239:476-481, 1988; Monath, T. P., New Eng. J. Med., 1988, 319, 641-643).

The pestivirus genus includes bovine viral diarrhea virus (BVDV), classical swine fever virus (CSFV, also called hog cholera virus) and border disease virus (BDV) of sheep (Moennig, V. et al. Adv. Vir. Res. 1992, 41, 53-98). Pestivirus infections of domesticated livestock (cattle, pigs and sheep) cause significant economic losses worldwide. BVDV causes mucosal disease in cattle and is of significant economic importance to the livestock industry (Meyers, G. and Thiel, H.-J., Advances in Virus Research, 1996, 47, 53-118; Moennig V., et al, Adv. Vir. Res. 1992, 41, 53-98). Human pestiviruses have not been as extensively characterized as the animal pestiviruses. However, serological surveys indicate considerable pestivirus exposure in humans.

*Pestiviruses* and *hepaciviruses* are closely related virus groups within the Flaviviridae family. Other closely related viruses in this family include the GB virus A, GB virus A-like agents, GB virus-B and GB virus-C (also called hepatitis G virus, HGV). The hepacivirus group (hepatitis C virus; HCV) consists of a number of closely related but genotypically distinguishable viruses that infect humans. There are approximately 6 HCV genotypes and more than 50 subtypes. Due to the similarities between pestiviruses and hepaciviruses, combined with the poor ability of hepaciviruses to grow efficiently in cell culture, bovine viral diarrhea virus (BVDV) is often used as a surrogate to study the HCV virus.

The genetic organization of pestiviruses and hepaciviruses is very similar. These positive stranded RNA viruses possess a single large open reading frame (ORF) encoding all the viral proteins necessary for virus replication. These proteins are expressed as a polyprotein that is co- and post-translationally processed by both cellular and virus-encoded proteinases to yield the mature viral proteins. The viral proteins responsible for the replication of the viral genome RNA are located within approximately the carboxy-terminal. Two-thirds of the ORF are termed nonstructural (NS) proteins. The genetic organization and polyprotein processing of the nonstructural protein portion of the ORF for pestiviruses and hepaciviruses is very similar. For both the pestiviruses and hepaciviruses, the mature nonstructural (NS) proteins, in sequential order from the amino-terminus of the nonstructural protein coding region to the carboxy-terminus of the ORF, consist of p7, NS2, NS3, NS4A, NS4B, NS5A, and NS5B.

The NS proteins of pestiviruses and hepaciviruses share sequence domains that are characteristic of specific protein functions. For example, the NS3 proteins of viruses in both groups possess amino acid sequence motifs characteristic of serine proteinases and of helicases (Gorbalenya et al. (1988) Nature 333:22; Bazan and Fletterick (1989) Virology 171:637-639; Gorbalenya et al. (1989) Nucleic Acid Res. 17.3889-3897). Similarly, the NS5B proteins of pestiviruses and hepaciviruses have the motifs characteristic of RNA-directed RNA polymerases (Koonin, E.V. and Dolja, V.V. (1993) Crit. Rev. Biochem. Molec. Biol. 28:375-430).

The actual roles and functions of the NS proteins of pestiviruses and hepaciviruses in the lifecycle of the viruses are directly analogous. In both cases, the NS3 serine proteinase is responsible for all proteolytic processing of polyprotein precursors downstream of its position in the ORF (Wiskerchen and Collett (1991) Virology 184:341-350; Bartenschlager et al. (1993) J. Virol. 67:3835-3844; Eckart et al. (1993) Biochem. Biophys. Res. Comm. 192:399-406; Grakoui et al. (1993) J. Virol. 67:2832-2843; Grakoui et al. (1993) Proc. Natl. Acad. Sci. USA 90:10583-10587; Hijikata et al. (1993) J. Virol. 67:4665-4675; Tome et al. (1993) J. Virol. 67:4017-4026). The NS4A protein, in both cases, acts as a cofactor with the NS3 serine protease (Bartenschlager et al. (1994) J. Virol. 68:5045-5055; Failla et al. (1994) J. Virol. 68: 3753-3760; Lin et al. (1994) 68:8147-8157; Xu et al. (1997) J. Virol. 71:5312-5322). The NS3 protein of both viruses also functions as a helicase (Kim et al. (1995) Biochem. Biophys. Res. Comm. 215: 160-166; Jin and Peterson (1995) Arch. Biochem. Biophys., 323:47-53; Warrener and Collett (1995) J. Virol. 69:1720-1726). Finally, the NS5B proteins of pestiviruses and hepaciviruses have the predicted RNA-directed RNA polymerases activity (Behrens et al.(1996) EMBO J. 15:12-22; Lchmannet al.(1997) J. Virol. 71:8416-8428; Yuan et al.(1997) Biochem. Biophys. Res. Comm. 232:231-235; Hagedorn, PCT WO 97/12033; Zhong et al.(1998) J. Virol. 72.9365-9369).

### Hepatitis C Virus

The hepatitis C virus (HCV) is the leading cause of chronic liver disease worldwide. (Boyer, N. et al. J. Hepatol. 32:98-112, 2000). HCV causes a slow growing viral infection and is the major cause of cirrhosis and hepatocellular carcinoma (Di Besceglie, A. M. and Bacon, B. R., Scientific American, Oct.: 80-85, (1999); Boyer, N. et al. J. Hepatol. 32:98-112, 2000). An estimated 170 million persons are infected with HCV worldwide. (Boyer, N. et al. J. Hepatol. 32:98-112, 2000). Cirrhosis caused by chronic hepatitis C infection accounts for 8,000-12,000 deaths per year in the United States, and HCV infection is the leading indication for liver transplantation.

HCV is known to cause at least 80% of posttransfusion hepatitis and a substantial proportion of sporadic acute hepatitis. Preliminary evidence also implicates HCV in many cases of "idiopathic" chronic hepatitis, "cryptogenic" cirrhosis, and probably hepatocellular carcinoma unrelated to other hepatitis viruses, such as Hepatitis B Virus (HBV). A small proportion of healthy persons appear to be chronic HCV carriers, varying with geography and other epidemiological factors. The numbers may substantially exceed those for HBV, though information is still preliminary; how many of these persons have subclinical chronic liver disease is unclear. (The Merck Manual, ch. 69, p. 901, 16th ed., (1992)).

HCV is an enveloped virus containing a positive-sense single-stranded RNA genome of approximately 9.4kb. The viral genome consists of a 5' untranslated region (UTR), a long open reading frame encoding a polyprotein precursor of approximately 3011 amino acids, and a short 3' UTR. The 5' UTR is the most highly conserved part of the HCV genome and is important for the initiation and control of polyprotein translation. Translation of the HCV genome is initiated by a cap-independent mechanism known as internal ribosome entry. This mechanism involves the binding of ribosomes to an RNA sequence known as the internal ribosome entry site (IRES). An RNA pseudoknot structure has recently been determined to be an essential structural element of the HCV IRES. Viral structural proteins include a nucleocapsid core protein (C) and two envelope glycoproteins, E1 and E2. HCV also encodes two proteinases, a zinc-dependent metalloproteinase encoded by the NS2-NS3 region and a serine proteinase encoded in the NS3 region. These proteinases are required for cleavage of specific regions of the precursor polyprotein into mature peptides. The carboxyl half of nonstructural protein 5, NS5B, contains the RNA-dependent RNA polymerase. The function of the remaining nonstructural proteins, NS4A and NS4B, and that of NS5A (the amino-terminal half of nonstructural protein 5) remain unknown.

A significant focus of current antiviral research is directed to the development of improved methods of treatment of chronic HCV infections in humans (Di Besceglie, A. M. and Bacon, B. R., Scientific American, Oct.: 80-85, (1999)).

### Treatment of HCV Infection with Interferon

Interferons (IFNs) have been commercially available for the treatment of chronic hepatitis for nearly a decade. IFNs are glycoproteins produced by immune cells in response to viral infection. IFNs inhibit replication of a number of viruses, including HCV, and when used as the sole treatment for hepatitis C infection, IFN can in certain cases suppress serum HCV-RNA to undetectable levels. Additionally, IFN can normalize serum amino transferase levels. Unfortunately, the effect of IFN is temporary and a sustained response occurs in only 8%-9% of patients chronically infected with HCV (Gary L. Davis. Gastroenterology 118:S104-S114, 2000). Most patients, however, have difficulty tolerating interferon treatment, which causes severe flu-like symptoms, weight loss, and lack of energy and stamina.

A number of patents disclose Flaviviridae, including HCV, treatments, using interferon-based therapies. For example, U.S. Patent No. 5,980,884 to Blatt et al. discloses methods for retreatment of patients afflicted with HCV using consensus interferon. U.S. Patent No. 5,942,223 to Bazer et al. discloses an anti-HCV therapy using ovine or bovine interferon-tau. U.S. Patent No. 5,928,636 to Alber et al. discloses the combination therapy of interleukin-12 and interferon alpha for the treatment of infectious diseases including HCV. U.S. Patent No. 5,849,696 to Chretien et al. discloses the use of thymosins, alone or in combination with interferon, for treating HCV. U.S. Patent No. 5,830,455 to Valtuena et al. discloses a combination HCV therapy employing interferon and a free radical scavenger. U.S. Patent No. 5,738,845 to Imakawa discloses the use of human interferon tau proteins for treating HCV. Other interferon-based treatments for HCV are disclosed in U.S. Patent No. 5,676,942 to Testa et al.*,* U.S. Patent No. 5,372,808 to Blatt et al.*,* and U.S. Patent No. 5,849,696. A number of patents also disclose pegylated forms of interferon, such as U.S. Patent Nos. 5,747,646, 5,792,834 and 5,834,594 to Hoffmann-La Roche Inc; PCT Publication No. WO 99/32139 and WO 99/32140 to Enzon; WO 95/13090 and US Patent Nos. 5,738,846 and 5,711,944 to Schering; and U.S. Patent No. 5,908,621 to Glue et al.*.*

Interferon alpha-2a and interferon alpha-2b are currently approved as monotherapy for the treatment of HCV. ROFERON®-A (Roche) is the recombinant form of interferon alpha-2a. PEGASYS® (Roche) is the pegylated (i.e. polyethylene glycol modified) form of interferon alpha-2a. INTRON®A (Schering Corporation) is the recombinant form of Interferon alpha-2b, and PEG-INTRON® (Schering Corporation) is the pegylated form of interferon alpha-2b.

Other forms of interferon alpha, as well as interferon beta, gamma, tau and omega are currently in clinical development for the treatment of HCV. For example, INFERGEN (interferon alphacon-1) by InterMune, OMNIFERON (natural interferon) by Viragen, ALBUFERON by Human Genome Sciences, REBIF (interferon beta-1a) by Ares-Serono, Omega Interferon by BioMedicine, Oral Interferon Alpha by Amarillo Biosciences, and interferon gamma, interferon tau, and interferon gamma- 1b by InterMune are in development.

### Ribivarin

Ribavirin (1-p-D-ribofuranosyl-1-1,2,4-triazole-3-carboxamide) is a synthetic, non-interferon-inducing, broad spectrum antiviral nucleoside analog sold under the trade name, Virazole (The Merck Index, 11h edition, Editor: Budavari, S., Merck & Co., Inc., Rahway, NJ, p1304, 1989). United States Patent No. 3,798,209 and RE29,835 disclose and claim ribavirin. Ribavirin is structurally similar to guanosine, and has in vitro activity against several DNA and RNA viruses including *Flaviviridae* (Gary L. Davis. Gastroenterology 118:S104-S114, 2000).

Ribavirin reduces serum amino transferase levels to normal in 40% of patients, but it does not lower serum levels of HCV-RNA (Gary L. Davis. Gastroenterology 118:S104-S114, 2000). Thus, ribavirin alone is not effective in reducing viral RNA levels. Additionally, ribavirin has significant toxicity and is known to induce anemia.

Ribavirin is not approved for monotherapy against HCV. It has been approved in combination with interferon alpha-2a or interferon alpha-2b for the treatment of HCV.

### Combination of Interferon and Ribavirin

The current standard of care for chronic hepatitis C is combination therapy with an alpha interferon and ribavirin. The combination of interferon and ribavirin for the treatment of HCV infection has been reported to be effective in the treatment of interferon naïve patients (Battaglia, A.M. et al., Ann. Pharmacother. 34:487-494, 2000), as well as for treatment of patients when histological disease is present (Berenguer, M. et al. Antivir. Ther. 3(Suppl. 3):125-136, 1998). Studies have show that more patients with hepatitis C respond to pegylated interferon-alpha/ribavirin combination therapy than to combination therapy with unpegylated interferon alpha. However, as with monotherapy, significant side effects develop during combination therapy, including hemolysis, flu-like symptoms, anemia, and fatigue. (Gary L. Davis. Gastroenterology 118:S104-S114, 2000).

Combination therapy with PEG-INTRON® (peginterferon alpha-2b) and REBETOL® (Ribavirin, USP) Capsules is available from Schering Corporation. REBETOL® (Schering Corporation) has also been approved in combination with INTRON® A (Interferon alpha-2b, recombinant, Schering Corporation). Roche's PEGASYS® (pegylated interferon alpha-2a) and COPEGUS® (ribavirin) are also approved for the treatment of HCV.

PCT Publication Nos. WO 99/59621, WO 00/37110, WO 01/81359, WO 02/32414 and WO 03/024461 by Schering Corporation disclose the use of pegylated interferon alpha and ribavirin combination therapy for the treatment of HCV. PCT Publication Nos. WO 99/15194, WO 99/64016, and WO 00/24355 by Hoffmann-La Roche Inc also disclose the use of pegylated interferon alpha and ribavirin combination therapy for the treatment of HCV.

### Additional Methods to Treat Flaviviridae Infections

The development of new antiviral agents for flaviviridae infections, especially hepatitis C, is currently underway. Specific inhibitors of HCV-derived enzymes such as protease, helicase, and polymerase inhibitors are being developed. Drugs that inhibit other steps in HCV replication are also in development, for example, drugs that block production of HCV antigens from the RNA (IRES inhibitors), drugs that prevent the normal processing of HCV proteins (inhibitors of glycosylation), drugs that block entry of HCV into cells (by blocking its receptor) and nonspecific cytoprotective agents that block cell injury caused by the virus infection. Further, molecular approaches are also being developed to treat hepatitis C, for example, ribozymes, which are enzymes that break down specific viral RNA molecules, and antisense oligonucleotides, which are small complementary segments of DNA that bind to viral RNA and inhibit viral replication, are under investigation. A number of HCV treatments are reviewed by Bymock et al. in Antiviral Chemistry & Chemotherapy, 11:2; 79-95 (2000) and De Francesco et al. in Antiviral Research, 58: 1-16 (2003).

Examples of classes of drugs that are being developed to treat Flaviviridae infections include:
(1) Protease inhibitors
   Substrate-based NS3 protease inhibitors (Attwood *et al., Antiviral peptide derivatives,* PCT WO 98/22496, 1998**;** Attwood et al., Antiviral Chemistry and Chemotherapy 1999, 10, 259-273; Attwood *et al., Preparation and use of amino acid derivatives as anti-viral agents,* German Patent Pub. DE 19914474; Tung *et al. Inhibitors of serine proteases, particularly hepatitis C virus NS3 protease,* PCT WO 98/17679), including alphaketoamides and hydrazinoureas, and inhibitors that terminate in an electrophile such as a boronic acid or phosphonate (Llinas-Brunet et al, *Hepatitis C inhibitor peptide analogues,* PCT WO 99/07734) are being investigated.
   Non-substrate-based NS3 protease inhibitors such as 2,4,6-trihydroxy-3-nitrobenzamide derivatives (Sudo K. et al., Biochemical and Biophysical Research Communications, 1997, 238, 643-647; Sudo K. et al. Antiviral Chemistry and Chemotherapy, 1998, 9, 186), including RD3-4082 and RD3-4078, the former substituted on the amide with a 14 carbon chain and the latter processing a *para*-phenoxyphenyl group are also being investigated.
   Sch 68631, a phenanthrenequinone, is an HCV protease inhibitor (Chu M. et al., Tetrahedron Letters 37:7229-7232, 1996). In another example by the same authors, Sch 351633, isolated from the fungus *Penicillium griseofulvum,* was identified as a protease inhibitor (Chu M. et al., Bioorganic and Medicinal Chemistry Letters 9:1949-1952). Nanomolar potency against the HCV NS3 protease enzyme has been achieved by the design of selective inhibitors based on the macromolecule eglin c. Eglin c, isolated from leech, is a potent inhibitor of several serine proteases such as S. griseus proteases A and B, α-chymotrypsin, chymase and subtilisin. Qasim M.A. et al., Biochemistry 36:1598-1607, 1997.
   Several U.S. patents disclose protease inhibitors for the treatment of HCV. For example, U.S. Patent No. 6,004,933 to Spruce et al. discloses a class of cysteine protease inhibitors for inhibiting HCV endopeptidase 2. U.S. Patent No. 5,990,276 to Zhang et al. discloses synthetic inhibitors of hepatitis C virus NS3 protease. The inhibitor is a subsequence of a substrate of the NS3 protease or a substrate of the NS4A cofactor. The use of restriction enzymes to treat HCV is disclosed in U.S. Patent No. 5,538,865 to Reyes et al. Peptides as NS3 serine protease inhibitors of HCV are disclosed in WO 02/008251 to Corvas International, Inc, and WO 02/08187 and WO 02/008256 to Schering Corporation. HCV inhibitor tripeptides are disclosed in US Patent Nos. 6,534,523, 6,410,531, and 6,420,380 to Boehringer Ingelheim and WO 02/060926 to Bristol Myers Squibb. Diaryl peptides as NS3 serine protease inhibitors of HCV are disclosed in WO 02/48172 to Schering Corporation. Imidazoleidinones as NS3 serine protease inhibitors of HCV are disclosed in WO 02/08198 to Schering Corporation and WO 02/48157 to Bristol Myers Squibb. WO 98/17679 to Vertex Pharmaceuticals and WO 02/48116 to Bristol Myers Squibb also disclose HCV protease inhibitors.
(2) Thiazolidine derivatives which show relevant inhibition in a reverse-phase HPLC assay with an NS3/4A fusion protein and NS5A/5B substrate (Sudo K. et al., Antiviral Research, 1996, 32, 9-18), especially compound RD-1-6250, possessing a fused cinnamoyl moiety substituted with a long alkyl chain, RD4 6205 and RD4 6193;
(3) Thiazolidines and benzanilides identified in Kakiuchi N. et al. J. EBS Letters 421, 217-220; Takeshita N. et al. Analytical Biochemistry, 1997, 247, 242-246;
(4) A phenan-threnequinone possessing activity against protease in a SDS-PAGE and autoradiography assay isolated from the fermentation culture broth of *Streptomyces* sp., Sch 68631 (Chu M. et al., Tetrahedron Letters, 1996, 37, 7229-7232), and Sch 351633, isolated from the fungus *Penicillium griseofulvum,* which demonstrates activity in a scintillation proximity assay (Chu M. et al., Bioorganic and Medicinal Chemistry Letters 9, 1949-1952);
(5) Helicase inhibitors (Diana G.D. *et al., Compounds, compositions and methods for treatment of hepatitis C,* U.S. Pat. No. 5,633,358; Diana G.D. *et al., Piperidine derivatives, pharmaceutical compositions thereof and their use in the treatment of hepatitis C,* PCT WO 97/36554);
(6) Nucleotide polymerase inhibitors and gliotoxin (Ferrari R. et al. Journal of Virology, 1999, 73, 1649-1654), and the natural product cerulenin (Lohmann V. et al., Virology, 1998, 249, 108-118);
(7) Antisense phosphorothioate oligodeoxynucleotides (S-ODN) complementary to sequence stretches in the 5' non-coding region (NCR) of the virus (Alt M. et al., Hepatology, 1995, 22, 707-717), or nucleotides 326-348 comprising the 3' end of the NCR and nucleotides 371-388 located in the core coding region of the HCV RNA (Alt M. et al., Archives of Virology, 1997, 142, 589-599; Galderisi U. et al., Journal of Cellular Physiology, 1999, 181, 251-257);
(8) Inhibitors of IRES-dependent translation (Ikeda N *et al., Agent for the prevention and treatment of hepatitis C,* Japanese Patent Pub. JP-08268890; Kai Y. *et al. Prevention and treatment of viral diseases,* Japanese Patent Pub. JP-10101591);
(9) Ribozymes, such as nuclease-resistant ribozymes (Maccjak, D. J. et al., Hepatology 1999, 30, abstract 995) and those disclosed in U.S. Patent No. 6,043,077 to Barber et al.*,* and U.S. Patent Nos. 5,869,253 and 5,610,054 to Draper et al.; and
(10) Nucleoside analogs have also been developed for the treatment of Flaviviridae infections.
   Idenix Pharmaceuticals discloses the use of branched in the treatment of flaviviruses (including HCV) and pestiviruses in International Publication Nos. WO 01/90121 and WO 01/92282. Specifically, a method for the treatment of hepatitis C infection (and flaviviruses and pestiviruses) in humans and other host animals is disclosed in the Idenix publications that includes administering an effective amount of a biologically active 1', 2', 3' or 4'-branched β-D or β-L nucleosides or a pharmaceutically acceptable salt or derivative thereof, administered either alone or in combination with another antiviral agent, optionally in a pharmaceutically acceptable carrier.
   Other patent applications disclosing the use of certain nucleoside analogs to treat hepatitis C virus include: PCT/CA00/01316 (WO 01/32153; filed November 3, 2000) and PCT/CA01/00197 (WO 01/60315; filed February 19, 2001) filed by BioChem Pharma, Inc. (now Shire Biochem, Inc.); PCT/US02/01531 (WO 02/057425; filed January 18, 2002) and PCT/US02/03086 (WO 02/057287; filed January 18, 2002) filed by Merck & Co., Inc., PCT/EP01/09633 (WO 02/18404; published August 21, 2001) filed by Roche, and PCT Publication Nos. WO 01/79246 (filed April 13, 2001), WO 02/32920 (filed October 18, 2001) and WO 02/48165 by Pharmasset, Ltd.
   PCT Publication No. WO 99/43691 to Emory University, entitled "2'-Fluoronucleosides" discloses the use of certain 2'-fluoronucleosides to treat HCV.
   Eldrup et al. (Oral Session V, Hepatitis C Virus, Flaviviridae; 16th International Conference on Antiviral Research (April 27, 2003, Savannah, Ga.)) described the structure activity relationship of 2'-modified nucleosides for inhibition of HCV.
   Bhat et al. (Oral Session V, Hepatitis C Virus, Flaviviridae, 2003 (Oral Session V, Hepatitis C Virus, Flaviviridae; 16th International Conference on Antiviral Research (April 27, 2003, Savannah, Ga.); p A75) describe the synthesis and pharmacokinetic properties of nucleoside analogues as possible inhibitors of HCV RNA replication. The authors report that 2'-modified nucleosides demonstrate potent inhibitory activity in cell-based replicon assays.
   Olsen et al. (Oral Session V, Hepatitis C Virus, Flaviviridae; 16^{th} International Conference on Antiviral Research (April 27, 2003, Savannah, Ga.) p A76) also described the effects of the 2'-modified nucleosides on HCV RNA replication.
(11) Other miscellaneous compounds including 1-amino-alkylcyclohexanes (U.S. Patent No. 6,034,134 to Gold et al.)*,* alkyl lipids (U.S. Pat. No. 5,922,757 to Chojkier et al.)*,* vitamin E and other antioxidants (U.S. Pat. No. 5,922,757 to Chojkier et al.)*,* squalene, amantadine, bile acids (U.S. Pat. No. 5,846,964 to Ozeki et al.)*,* N-(phosphonoacetyl)-L-aspartic acid, (U.S. Pat. No. 5,830,905 to Diana et al.)*,* benzenedicarboxamides (U.S. Pat. No. 5,633,388 to Diana et al.)*,* polyadenylic acid derivatives (U.S. Pat. No. 5,496,546 to Wang et al.)*,* 2',3'-dideoxyinosine (U.S. Pat. No. 5,026,687 to Yarchoan et al.)*,* benzimidazoles (U.S. Pat. No. 5,891,874 to Colacino et al.)*,* plant extracts (U.S. Patent No. 5,837,257 to Tsai et al.*,* U.S. Patent No. 5,725,859 to Omer et al.*,* and U.S. Patent No. 6,056,961), and piperidenes (U.S. Patent No. 5,830,905 to Diana et al.)*.*
(12) Other compounds currently in preclinical or clinical development for treatment of hepatitis C virus include: Interleukin-10 by Schering-Plough, IP-501 by Interneuron, Merimebodib (VX-497) by Vertex, AMANTADINE® (Symmetrel) by Endo Labs Solvay, HEPTAZYME® by RPI, IDN-6556 by Idun Pharma., XTL-002 by XTL., HCV/MF59 by Chiron, CIVACIR® (Hepatitis C Immune Globulin) by NABI, LEVOVIRIN® by ICN/Ribapharm, VIRAMIDINE® by ICN/Ribapharm, ZADAXIN® (thymosin alpha-1) by Sci Clone, thymosin plus pegylated interferon by Sci Clone, CEPLENE® (histamine dihydrochloride) by Maxim, VX 950 / LY 570310 by Vertex/Eli Lilly, ISIS 14803 by Isis Pharmaceutical/Elan, IDN-6556 by Idun Pharmaceuticals, Inc., JTK 003 by AKROS Pharma, BILN-2061 by Boehringer Ingelheim, CellCept (mycophenolate mofetil) by Roche, T67, a β-tubulin inhibitor, by Tularik, a therapeutic vaccine directed to E2 by Innogenetics, FK788 by Fujisawa Healthcare, Inc., IdB 1016 (Siliphos, oral silybin-phosphatdylcholine phytosome), RNA replication inhibitors (VP50406) by ViroPharma/Wyeth, therapeutic vaccine by Intercell, therapeutic vaccine by Epimmune/Genencor, IRES inhibitor by Anadys, ANA 245 and ANA 246 by Anadys, immunotherapy (Therapore) by Avant, protease inhibitor by Corvas/SChering, helicase inhibitor by Vertex, fusion inhibitor by Trimeris, T cell therapy by CellExSys, polymerase inhibitor by Biocryst, targeted RNA chemistry by PTC Therapeutics, Dication by Immtech, Int., protease inhibitor by Agouron, protease inhibitor by Chiron/Medivir, antisense therapy by AVI BioPharma, antisense therapy by Hybridon, hemopurifier by Aethlon Medical, therapeutic vaccine by Merix, protease inhibitor by Bristol-Myers Squibb/Axys, Chron-VacC, a therapeutic vaccine, by Tripep, UT 231B by United Therapeutics, protease, helicase and polymerase inhibitors by Genelabs Technologies, IRES inhibitors by Immusol, R803 by Rigel Pharmaceuticals, INFERGEN® (interferon alphacon-1) by InterMune, OMNIFERON® (natural interferon) by Viragen, ALBUFERON® by Human Genome Sciences, REBIF® (interferon beta-1a) by Ares-Serono, Omega Interferon by BioMedicine, Oral Interferon Alpha by Amarillo Biosciences, interferon gamma, interferon tau, and Interferon gamma- 1b by InterMune.

Nucleoside prodrugs have been previously described for the treatment of other forms of hepatitis. WO 00/09531 (filed August 10, 1999) and WO 01/96353 (filed June 15, 2001) to Idenix Pharmaceuticals, discloses 2'-deoxy-β-L-nucleosides and their 3'-prodrugs for the treatment of HBV. U.S. Patent No. 4,957,924 to Beauchamp discloses various therapeutic esters of acyclovir.

In light of the fact that HCV infection has reached epidemic levels worldwide, and has tragic effects on the infected patient, there remains a strong need to provide new effective pharmaceutical agents to treat hepatitis C that have low toxicity to the host.

Further, given the rising threat of other flaviviridae infections, there remains a strong need to provide new effective pharmaceutical agents that have low toxicity to the host.

Therefore, it is an object of the present invention to provide a compound and composition for the treatment of a host infected with flaviviridae, including hepatitis C virus.

It is another object of the present invention to provide a compound and composition generally for the treatment of patients infected with *pestiviruses, flaviviruses,* or *hepaciviruses.*

WO 01/90121 and WO 01/92282 disclose methods and compositions for treating a host infected with hepatitis C comprising administering 1', 2' or 3'-modified nucleosides or a pharmaceutically acceptable salt or prodrug thereof.
Franchetti P. et al. describe the synthesis and binding studies of 2'-C-Methyl analogues of selective adenosine receptor agonists (Journal of Medicinal Chemistry, 1998, 41, 1708-1715).
Wolf J. et al. describe 2'-C-branched-chain sugar nucleoside analogs with potential antiviral or antitumor activity (Synthesis, Georg Thieme Verlag. 1992**)**
Matsuda A. et al. describe the synthesis and antileukimic activity of deoxy-2'(S)-methylcytidine (Chemical and Pharmaceutical Bulletin, 1987, 35, 3967-3970; Journal of Medicinal Chemistry, 1991, 34, 234-239).
Awano et al. describe the synthesis and antiviral activity of 5-substituted (2'S)-2'-deoxy-2'-C-methylcytidines and -uridines (Archiv der Pharmazie, 1996, 329, 66-72).
Li N.-S. et al. describe the synthesis of 2'-C-beta-trifluoromethyl pyrimidine ribonucleosides (Org. Lett., 2001, 3, 1025-1028)
Schmit et al. describe the synthesis of 2'-deoxy-2'-alpha-monofluoromethyl and trifluoromethylnucleosides (Synlett, 1994, 4, 241-242).

### SUMMARY OF THE INVENTION

In a first embodiment, the present invention provides a compound of formula 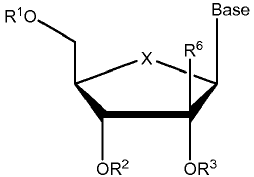 or a pharmaceutically acceptable salt thereof, for use in a method of treating a *Flaviviridae* virus infection in a host wherein:
Base is a purine or pyrimidine base;
R¹ is H or monophosphate, diphosphate, triphosphate, or a stabilized phosphate;
R² is an amino acid residue;
R³ is H;
R⁶ is unsubstituted alkyl; and
X is O, S, SO₂ or CH₂.

In a second embodiment, the present invention provides the use of a compound of formula 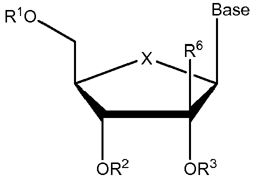 or a pharmaceutically acceptable salt thereof, in the manufacture of a medicament for the treatment of a *Flaviviridae* virus infection in a host
wherein:
Base is a purine or pyrimidine base;
R¹ is H or monophosphate, diphosphate, triphosphate, or a stabilized phosphate;
R² is an amino acid residue;
R³ is H;
R⁶ is unsubstituted alkyl; and
X is O, S, SO₂ or CH₂.

2'- and 3'-prodrugs of 1', 2', 3' or 4'-branched β-D or β-L nucleosides, or their pharmaceutically acceptable salts, or pharmaceutically acceptable formulations containing these compounds are useful in the prevention and treatment of *Flaviviridae* infections and other related conditions such as anti-*Flaviviridae* antibody positive and *Flaviviridae*-positive conditions, chronic liver inflammation caused by HCV, cirrhosis, acute hepatitis, fulminant hepatitis, chronic persistent hepatitis, and fatigue. These compounds or formulations can also be used prophylactically to prevent or retard the progression of clinical illness in individuals who are anti-*Flaviviridae* antibody or *Flaviviridae*-antigen positive or who have been exposed to a *Flaviviridae.* In one specific embodiment, the *Flaviviridae* is hepatitis C. In an alternative embodiment, the compound is used to treat any virus that replicates through an RNA-dependent RNA polymerase.

A method for the treatment of a *Flaviviridae* infection in a host, including a human, is also disclosed that includes administering an effective amount of a 2'- or 3'- prodrug of a biologically active 1', 2', 3' or 4'-branched β-D or β-L nucleosides or a pharmaceutically acceptable salt thereof, administered either alone or in combination or alternation with another anti-*Flaviviridae* agent, optionally in a pharmaceutically acceptable carrier. The term 1', 2', 3' or 4'-branched, as used in this specification, refers to a nucleoside that has an additional non-natural substituent in the 1', 2', 3' or 4'-position (i.e., carbon is bound to four nonhydrogen substituents). The term 2'-prodrug, as used herein, refers to a 1', 2', 3' or 4'-branched β-D or β-L nucleoside that has a biologically cleavable moiety at the 2'-position, including, but not limited to acyl, and in one embodiment, a natural or synthetic L- or D-amino acid, preferably an L-amino acid. The term 3'-prodrug, as used herein, refers to a 1', 2', 3' or 4'-branched β-D or β-L nucleoside that has a biologically cleavable moiety at the 3'-position, including, but not limited to acyl, and in one embodiment, a natural or synthetic L- or D-amino acid, preferably an L-amino acid. Certain other alternative embodiments are also included.

In one embodiment of the disclosure, the prodrug of 1', 2', 3' or 4'-branched β-D or β-L nucleoside includes biologically cleavable moieties at the 2' and/or 5' positions. Preferred moieties are natural or synthetic D or L amino acid esters, including D or L-valyl, though preferably L-amino acid esters, such as L-valyl, and alkyl esters including acetyl. Therefore, this disclosure contemplates prodrugs such as 2'-L or D-amino acid ester and 2',5'-L or D-diamino acid ester of 1', 2', 3' or 4'-branched β-D or β-L nucleosides, preferably L-amino acid, with any desired purine or pyrimidine base, wherein the parent drug optionally has an EC₅₀ of less than 15 micromolar, and even more preferably less than 10 micromolar; 2'-(alkyl or aryl) ester or 2',5'-L-di(alkyl or aryl) ester of 1', 2', 3' or 4'-branched β-D or β-L nucleosides with any desired purine or pyrimidine base, wherein the parent drug optionally has an EC₅₀ of less than 10 or 15 micromolar; and prodrugs of 2',5'-diesters of 1', 2', 3' or 4'-branched β-D or β-L nucleosides wherein (i) the 2' ester is an amino acid ester and the 5'-ester is an alkyl or aryl ester; (ii) both esters are amino acid esters; (iii) both esters are independently alkyl or aryl esters; and (iv) the 2' ester is independently an alkyl or aryl ester and the 5'-ester is an amino acid ester, wherein the parent drug optionally has an EC₅₀ of less than 10 or 15 micromolar.

Examples of prodrugs contemplated by this disclosure are 2'-L-valine ester of β-D-2'-methyl-cytidine; 2'-L-valine ester of β-D-2'-methyl-thymidine; 2'-L-valine ester of P-D-2'-methyl-adenosine; 2'-L-valine ester of β-D-2'-methyl-guanosine; 2'-L-valine ester of β-D-2'-methyl-5-fluorocytidine; 2'-L-valine ester of β-D-2'-methyl-uridine; 2'-acetyl ester of β-D-2'-methyl-cytidine; 2'-acetyl ester of β-D-2'-methyl-thymidine; 2'-acetyl ester of P-D-2'-methyl-adenosine; 2'-acetyl ester of β-D-2'-methyl-guanosine; 2'-acetyl ester of β-D-2'-methyl-5-fluoro-cytidine; and 2'-esters of β-D-2'-methyl-(cytidine, 5-fluorocytidine, guanosine, uridine, adenosine, or thymidine) wherein (i) the 2' ester is an amino acid ester; or (ii) the 2' ester is an alkyl or aryl ester.

Additional examples of prodrugs contemplated by this disclosure are 2',5'-L-divaline ester of β-D-2'-methyl-cytidine (dival-2'-Me-L-dC); 2',5'-L-divaline ester of β-D-2'-methyl-thymidine; 2',5'-L-divaline ester of β-D-2'-methyl-adenosine; 2',5'-L-divaline ester of β-D-2'-methyl-guanosine; 2',5'-L-divaline ester of β-D-2'-methyl-5-fluoro-cytidine; 2',5'-L-divaline ester of β-D-2'-methyl-uridine; 2',5'-diacetyl ester of β-D-2'-methyl-cytidine; 2',5'-diacetyl ester of β-D-2'-methyl-thymidine; 2',5'-diacetyl ester of β-D-2'-methyl-adenosine; 2',5'-diacetyl ester of β-D-2'-methyl-guanosine; 2',5'-diacetyl ester of β-D-2'-methyl-5-fluoro-cytidine; and 2',5'-diesters of β-D-2'-methyl-(cytidine, 5-fluorocytidine, guanosine, uridine, adenosine, or thymidine) wherein (i) the 2' ester is an amino acid ester and the 5'-ester is an alkyl or aryl ester; (ii) both esters are amino acid esters; (iii) both esters are independently alkyl or aryl esters; or (iv) the 2' ester is an alkyl or aryl ester and the 5'-ester is an amino acid ester.

In another embodiment of this disclosure, the prodrug of 1', 2', 3' or 4'-branched β-D or β-L nucleoside includes biologically cleavable moieties at the 3' and/or 5' positions. Preferred moieties are natural or synthetic D or L amino acid esters, including D or L-valyl, though preferably L-amino acid esters, such as L-valyl, and alkyl esters including acetyl. Therefore, this invention specifically includes 3'-L or D-amino acid ester and 3',5'-L or D-diamino acid ester of 2', branched β-D nucleosides, preferably L-amino acid, with any desired purine or pyrimidine base, wherein the parent drug optionally has an EC₅₀ of less than 15 micromolar, and even more preferably less than 10 micromolar and prodrugs of 3',5'-diesters of 2'-branched β-D nucleosides wherein (i) the 3' ester is an amino acid ester and the 5'-ester is an alkyl or aryl ester; (ii) both esters are amino acid esters wherein the parent drug optionally has an EC₅₀ of less than 10 or 15 micromolar.

Examples of prodrugs falling within the invention are 3'-L-valine ester of β-D-2'-methyl-cytidine; 3'-L-valine ester of β-D-2'-methyl-thymidine; 3'-L-valine ester of β-D-2'-methyl-adenosine; 3'-L-valine ester of β-D-2' -methyl-guanosine; 3'-L-valine ester of β-D-2'-methyl-5-fluorocytidine; 3'-L-valine ester of β-D-2'-methyl-uridine and 3'-esters of β-D-2'-methyl-(cytidine, 5-fluorocytidine, guanosine, uridine, adenosine, or thymidine) wherein (i) the 3' ester is an amino acid ester.

Additional examples of prodrugs falling within the invention are 3',5'-L-divaline ester of β-D-2'-methyl-cytidine (dival-2'-Me-L-dC); 3',5'-L-divaline ester of β-D-2'-methyl-thymidine; 3',5'-L-divaline ester of β-D-2'-methyl-adenosine; 3',5'-L-divaline ester of β-D-2'-methyl-guanosine; 3',5'-L-divaline ester of β-D-2'-methyl-5-fluoro-cytidine; 3',5'-L-divaline ester of β-D-2'-methyl-uridine, and 3',5'-diesters of β-D-2'-methyl-(cytidine, 5-fluorocytidine, guanosine, uridine, adenosine, or thymidine) wherein (i) the 3' ester is an amino acid ester and the 5'-ester is an alkyl or aryl ester; (ii) both esters are amino acid esters.

In another embodiment of this disclosure, the prodrug of 1', 2', 3' or 4'-branched β-D or β-L nucleoside includes biologically cleavable moieties at the 2', 3' and/or 5' positions. Preferred moieties are D or L amino acid esters, including D or L-valyl, though preferably L-amino acid esters, such as L-valyl, and alkyl esters including acetyl. Therefore, this invention specifically includes 2',3'-L or D-diamino acid ester and 2',3',5'-L or D-triamino acid ester of 2' -branched β-D nucleosides, preferably L-amino acid, with any desired purine or pyrimidine base, wherein the parent drug optionally has an EC₅₀ of less than 15 micromolar, and even more preferably less than 10 micromolar; and prodrugs of 2',3'-diesters of 2'-branched β-D nucleosides wherein (ii) both esters are amino acid esters; and (iv) the 2' ester is independently an alkyl or aryl ester and the 3'-ester is an amino acid ester, wherein the parent drug optionally has an EC₅₀ of less than 10 or 15 micromolar. Further, 2',3',5'-triesters of 2'-branched β-D nucleosides wherein (i) all three esters are amino acid esters; (iii) the 2' ester is an amino acid ester, the 3' ester is an amino acid ester and the 5'-ester is an alkyl or aryl ester; (vi) the 2' ester is an alkyl or aryl ester, the 3' ester is an amino acid ester and the 5'-ester is an amino acid ester; (vii) the 2' ester is an alkyl or aryl ester, the 3' ester is an amino acid ester and the 5'-ester is an alkyl or aryl ester wherein the parent drug optionally has an EC₅₀ of less than 10 or 15 micromolar.

Examples of prodrugs falling within the invention include 2',3'-L-divaline ester of β-D-2'-methyl-cytidine (dival-2'-Me-L-dC); 2',3'-L-divaline ester of P-D-2'-methyl-thymidine; 2',3'-L-divaline ester of β-D-2'-methyl-adenosine; 2',3'-L-divaline ester of β-D-2'-methyl-guanosine; 2',3'-L-divaline ester of β-D-2'-methyl-5-fluoro-cytidine; 2',3'-L-divaline ester of β-D-2'-methyl-uridine; and 2',3'-diesters of β-D-2'-methyl-(cytidine, 5-fluorocytidine, guanosine, uridine, adenosine, or thymidine) wherein (ii) both esters are amino acid esters; or (iv) the 2' ester is an alkyl or aryl ester and the 3'-ester is an amino acid ester.

Additional examples of prodrugs falling within the invention include 2',3',5'-L-trivaline ester of β-D-2'-methyl-cytidine (trival-2'-Me-L-dC); 2',3',5'-L-trivaline ester of β-D-2'-methyl-thymidine; 2',3',5'-L-trivaline ester of β-D-2'-methyl-adenosine; 2',3',5'-L-trivaline ester of β-D-2'-methyl-guanosine; 2',3',5'-L-trivaline ester of β-D-2'-methyl-5-fluoro-cytidine; 2',3',5'-L-trivaline ester of β-D-2'-methyl-uridine; and 2',3',5'-triesters of β-D-2'-methyl-(cytidine, 5-fluorocytidine, guanosine, uridine, adenosine, or thymidine) wherein (i) all three esters are amino acid esters; (iii) the 2' ester is an amino acid ester, the 3' ester is an amino acid ester and the 5'-ester is an alkyl or aryl ester; (vi) the 2' ester is an alkyl or aryl ester, the 3' ester is an amino acid ester and the 5'-ester is an amino acid ester; (vii) the 2' ester is an alkyl or aryl ester, the 3' ester is an amino acid ester and the 5'-ester is an alkyl or aryl ester.

Pharmaceutically acceptable salts of tosylate, methanesulfonate, acetate, citrate, malonate, tartarate, succinate, benzoate, ascorbate, α-ketoglutarate, and α-glycerophosphate, formate, fumarate, propionate, glycolate, lactate, pyruvate, oxalate, maleate, salicyate, sulfate, sulfonate, nitrate, bicarbonate, hydrobromate, hydrobromide, hydroiodide, carbonate, and phosphoric acid salts are provided. A particularly preferred embodiment is the mono or dihydrochloride pharmaceutically acceptable salts.

In as far as the compounds in the enclosed claims are encompassed by formula (II), the present invention also relates to acceptable salts thereof compound of Formula II, or pharmaceutically 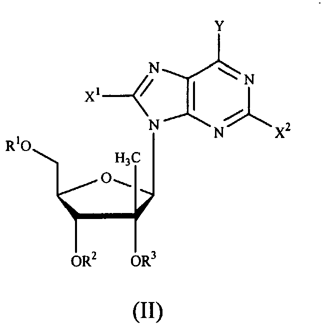 wherein:
R¹ is H mono-, di- or triphosphate or a stabilized phosphate
R2 is an amino acid residue;
R3 is H;
Y is hydrogen, bromo, chloro, fluoro, iodo, OR⁴, NR⁴R⁵ or SR⁴;
X¹ and X² are independently selected from the group consisting of H, straight chained, branched or cyclic alkyl, CO-alkyl, CO-aryl, CO-alkoxyalkyl, chloro, bromo, fluoro, iodo, OR⁴, NR⁴R⁵ or SR⁵; and
R⁴ and R⁵ are independently hydrogen, acyl (including lower acyl), or alkyl (including but not limited to methyl, ethyl, propyl and cyclopropyl).

In as far as the compounds referred to in the enclosed claims are encompassed by Formula V, the present invention relates to acceptable salts thereof of Formula V, or pharmaceutically 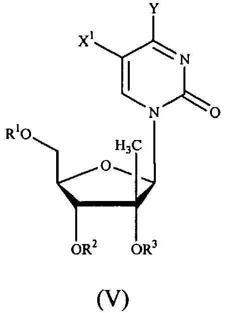 wherein:
R¹ is H, mono-, di- or triphosphate a stabilized phosphate
R2 is an amino acid residue;
R3 is H;
Y is hydrogen, bromo, chloro, fluoro, iodo, OR⁴, NR⁴R⁵ or SR⁴;
X¹ is selected from the group consisting of H, straight chained, branched or cyclic alkyl, CO-alkyl, CO-aryl, CO-alkoxyalkyl, chloro, bromo, fluoro, iodo, OR⁴, NR⁴R⁵ or SR⁵; and
R⁴ and R⁵ are independently hydrogen, acyl (including lower acyl), or alkyl (including but not limited to methyl, ethyl, propyl and cyclopropyl).

In as far as the compounds referred to in the enclosed claims are encompassed by Formuae IX or X, the present invention relates to compounds of Formulas IX and X, or a pharmaceutically acceptable salts thereof 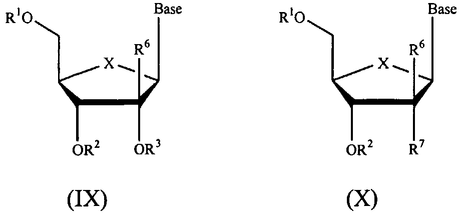 wherein:
Base is a purine or pyrimidine base as defined herein;
R¹ is H or mono-, di- or triphosphate or a stabilized phosphate
R2 is an amino acid residue; R3 is H;
R⁶ is unsubstituted alkyl
R⁷ is OR³; and
X is O, S, SO₂ or CH₂.

In one embodiment, the amino acid residue is of the formula C(O)C(R¹¹)(R¹²)(NR¹³R¹⁴), wherein
R¹¹ is the side chain of an amino acid and wherein, as in proline, R¹¹ can optionally be attached to R¹³ to form a ring structure; or alternatively, R¹¹ is an alkyl, aryl, heteroaryl or heterocyclic moiety;
R¹² is hydrogen, alkyl (including lower alkyl) or aryl; and
R¹³ and R¹⁴ are independently hydrogen, acyl (including an acyl derivative attached to R¹¹) or alkyl (including but not limited to methyl, ethyl, propyl, and cyclopropyl).

In another preferred embodiment, R² is an amino acid residue, and is preferably L-valinyl.

The β-D- and β-L-nucleosides of this invention may inhibit HCV polymerase activity. Nucleosides can be screened for their ability to inhibit HCV polymerase activity *in vitro* according to screening methods set forth more particularly herein. One can readily determine the spectrum of activity by evaluating the compound in the assays described herein or with another confirmatory assay.

In one embodiment the efficacy of the anti-HCV compound is measured according to the concentration of compound necessary to reduce the plaque number of the virus *in vitro,* according to methods set forth more particularly herein, by 50% (i.e. the compound's EC₅₀). In preferred embodiments the parent of the prodrug compound exhibits an EC₅₀ of less than 25, 15, 10, 5, or I micromolar. In one embodiment the efficacy of the anti*-Flaviviridae* compound is measured according to the concentration of compound necessary to reduce the plaque number of the virus *in vitro,* according to methods set forth more particularly herein, by 50% (i.e. the compound's EC₅₀). In preferred embodiments the compound exhibits an EC₅₀ of less than 15 or 10 micromolar, when measured according to the polymerase assay described in Ferrari et al., J. Virol., 73:1649-1654, 1999; Ishii et al., Hepatology, 29:1227-1235,1999; Lohmann et al., J. Biol. Chem., 274:10807-10815, 1999; or Yamashita et al, J. Biol. Chem., 273:15479-15486, 1998.

In another embodiment, combination and/or alternation therapy are provided. In combination therapy, an effective dosage of two or more agents are administered together, whereas during alternation therapy an effective dosage of each agent is administered serially. The dosages will depend on absorption, inactivation, and excretion rates of the drug as well as other factors known to those of skill in the art. It is to be noted that dosage values will also vary with the severity of the condition to be alleviated. It is to be further understood that for any particular subject, specific dosage regimens and schedules should be adjusted over time according to the individual need and the professional judgment of the person administering or supervising the administration of the compositions.

The invention also provides combinations of at least two of the herein described prodrugs. The invention further provides at least one of the described 2' and 3'-prodrugs in combination or alternation with a second nucleoside that exhibits activity against a *Flaviviridae* virus, including but not limited to a parent drug of any of the prodrugs defined herein, i.e. β-D-2'-methyl-cytidine, β-D-2'-methyl-thymidine, β-D-2'-methyl-adenosine, β-D-2'-methyl-guanosine, β-D-2'-methyl-5-fluorocytidine and/or β-D-2'-methyl-uridine. Alternatively, the 2' or 3'-prodrugs can be administered in combination or alternation with other *anti-Flaviviridae* agent exhibits an EC₅₀ of less than 10 or 15 micromolar, or their prodrugs or pharmaceutically acceptable salts.

Nonlimiting examples of antiviral agents that can be used in combination with the compounds disclosed herein include:
(1) an interferon and/or ribavirin; (2) Substrate-based NS3 protease inhibitors;. (3) Non-substrate-based inhibitors; (4) Thiazolidine derivatives; (5) Thiazolidines and benzanilides; (6) A phenan-threnequinone; (7) NS3 inhibitors; (8) HCV helicase inhibitors; (9) polymerase inhibitors, including RNA-dependent RNA-polymerase inhibitors; (10) Antisense oligodeoxynucleotides (11) Inhibitors of IRES-dependent translation; (12) Nuclease-resistant ribozymes; and (13) other compounds that exhibit activity against a flaviviridae. The invention further includes administering the prodrug in combination or alternation with an immune modulator or other pharmaceutically active modifer of viral replication, including a biological material such as a protein, peptide, oligonucleotide, or gamma globulin, including but not limited to interfereon, interleukin, or an antisense oligonucleotides to genes which express or regulate *Flaviviridae* replication.

Within the scope of the claims, the compounds described herein have a number of enantiomeric configurations, any of which can be used as desired. In the claims, the parent nucleoside framework exists as a β-D nucleoside. In a preferred embodiment, the compound is administered in a form that is at least 90% of the β-D enantiomer. In another embodiment, the compound is at least 95% of the β-D enantiomer. Certain prodrug acyl esters, specifically including amino acid esters, also have enantiomeric forms. The compounds may comprise an L or D amino acid.

In an alternative embodiment, the parent nucleoside compounds of any of the 2' or 3'-prodrugs (i.e., the nucleosides without the 2' or 3'cleavable moieties) provided for the treatment of a *Flaviviridae,* and in particular, an HCV infection.

### BRIEF DESCRIPTION OF THE FIGURES

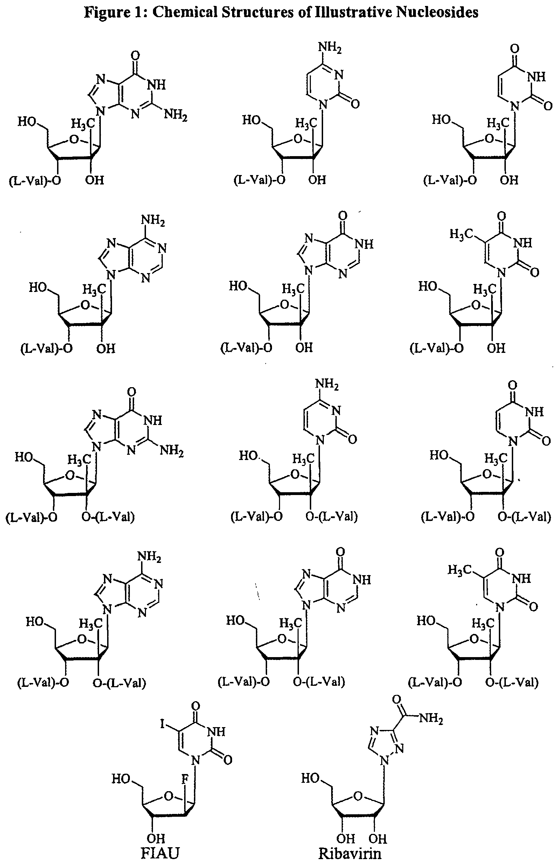

**Figure 1** provides the structure of various non-limiting examples of nucleosides of the present invention, as well as other known nucleosides, in particular FIAU and ribavirin.
**Figure 2** provides a non-limiting example of the steps involved in esterification of the 1', 2', 3' or 4'-branched β-D or β-L nucleoside to obtain a 2'-prodrug. The same general procedure can be used to obtain the 3'-prodrug by selectively protecting the 2' and 5'-hydroxyl groups or protecting the 2', 3' and 5'-hydroxyl groups and selectively deprotecting the 3'-hydroxyl.
**Figure 3** provides a non-limiting example of the steps involved in esterification of the 1', 2', 3' or 4'-branched β-D or β-L nucleoside to obtain a 3'-prodrug.
**Figure 4** provides a non-limiting example of the esterification of the 1', 2', 3' or 4'-branched β-D or β-L nucleoside to obtain a 2',3'-prodrug.
**Figure 5** is an illustration of a process of synthesizing a β-D-2'-C-methyl-ribofuransyl-cytidine or a 3'-O-L-valine ester thereof.
**Figure 6** is an illustration of another process of synthesizing a P-D-2'-C-methyl-ribofuransyl-cytidine or a 3'-O-L-valine ester thereof.
**Figure 7** is a diagram of a process of synthesizing a β-D-2'-C-methyl-2'-acetyl-ribofuransyl-cytidine-3'-O-L-valine ester.
**Figure 8** is a diagram of a process of synthesizing a β-D-2'-C-methyl-2'-acetyl-ribofuransyl-cytidine-3'-O-L-proline ester.
**Figure 9** is a diagram of a process of synthesizing a β-D-2'-C-methyl-2'-acetyl-ribofuransyl-cytidine-3'-O-L-alanine ester.
**Figure 10** is a diagram of a process of synthesizing a β-D-2'-C-methyl-2'-(cyclohexane carboxylate)-ribofuransyl-cytidine-3'-O-L-valine ester.
**Figure 11** is a graph showing the concentration of BVDB (Log₁₀ units/ml) over a concentration range of four test compounds and ribavirin as a control in a cell based assay using de novo BVDV infected MDBK cells. This graph shows the antiviral potency of these compounds.
**Figure 12** is a photocopy of a gel illustrating the site-specific chain termination of *in vitro* RNA synthesis by β-D-2'-C-methyl-ribofuranosyl cytidine triphosphate at specified guanine residues in RNA templates, as described in Example 32.
**Figure 13** is a graph of the the titer of bovine viral diarrhea virus (BVDV) over number of passages of BVDV infected MDBK cells, indicating eradication of a persistent BVDV infection by prolonged treatment with β-D-2'-C-methyl-ribofuranosyl cytidine (16uM) as described in Example 33. Arrows indicate points at which a portion of cells were withdrawn from drug treatment.

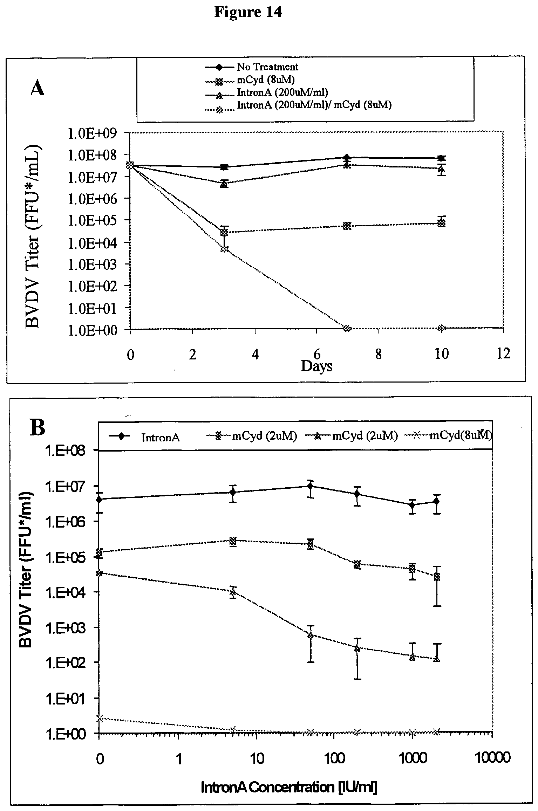

**Figure 14a** and **14b** are graphs of the concentration of bovine viral diarrhea virus (BVDV) in MDBK cells persistently infected with the virus, as described in Example 34. These graphs indicate the synergy between β-D-2'-C-methyl-ribofuranosyl cytidine and interferon alpha 2b (IntronA) in reducing the viral titer. Figure 14a is a graph of the effect of β-D-2'-C-methyl-ribofuranosyl cytidine and IntronA on BVDV (strain NY-1) titers in persistently infected MDBK cells over time. Figure 14b is a graph of the effect of β-D-2'-C-methyl-ribofuranosyl cytidine in combination with IntronA on BVDV (strain I-N-dIns) titers in persistently-infected MDBK cells.

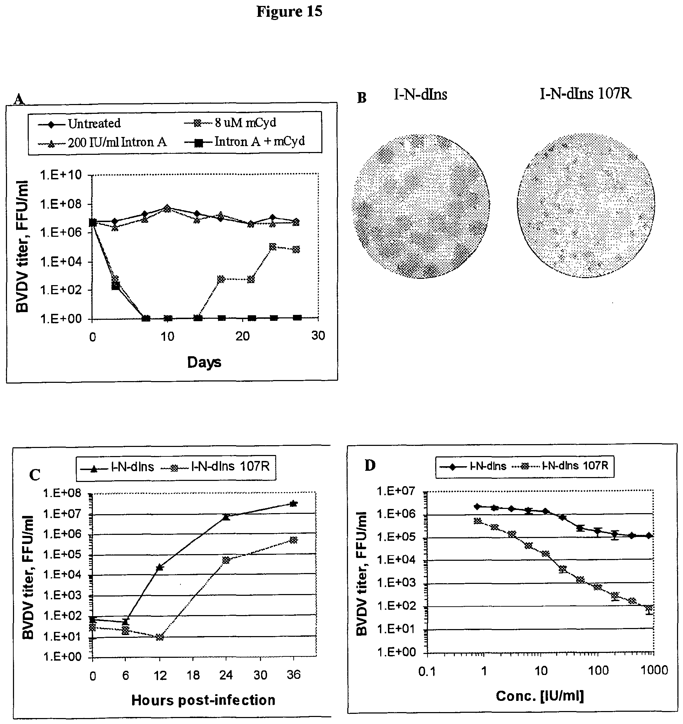

**Figure 15a****-d** illustrate the results of experiments studying the development of resistance to β-D-2'-C-methyl-ribofuranosyl cytidine treated MDBK cells, infected with bovine viral diarrhea virus (BVDV), as described in Example 35. Figure 15a is a graph of a representative experiment showing the effect over twenty eight days of β-D-2'-C-methyl-ribofuranosyl cytidine or IntronA treatment on BVDV (strain I-N-dIns) titers in persistently infected MDBK cells. Figure 15b is a photocopy of a dish plated with infected MDBK cells that illustrates the size of the foci formed by phenotypes of the wild-type BVDV (strain I-N-dIns), versus the β-D-2'-C-methyl-ribofuranosyl cytidine-resistant BVDV (I-N-dIns 107R), indicating that the resistant virus formed much smaller foci than the wild-type, I-N-dIns strain. Figure 15c is a graph of the titer of BVDV strains I-N-dIns or I-N-dIns-107R over hours post-infection in infected MDBK cells. Figure 15d is a graph of the effect of Intron A on the BVDV viral titer yield in *de novo*-infected MDBK cells treated with IntronA.
**Figure 16** is a graph of the concentration of hepatitis C virus (Log₁₀) in individual chimpanzees over days of treatment with β-D-2'-C-methyl-ribofuranosyl cytidine-3'-O-L-valine ester as described in Example 36.
**Figure 17** is a graph of the concentration of hepatitis C virus in individual chimpanzees over days of treatment with β-D-2'-C-methyl-ribofuranosyl cytidine-3'-O-L-valine ester as compared to baseline, as described in Example 36.
**Figure 18** is a graph of percent of total β-D-2'-C-methyl-ribofuranosyl cytidine-3'-O-L- valine ester remaining in samples over time after incubation of the drug in human plasma at 4°C, 21°C, and 37°C, as described in Example 37.

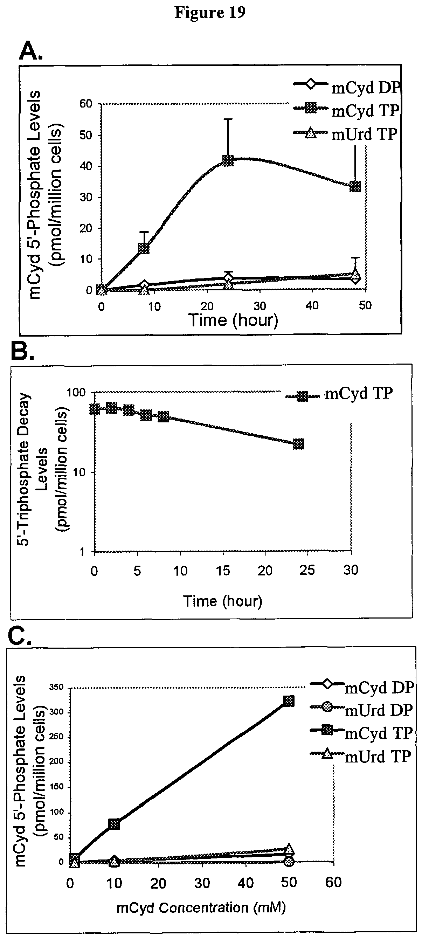

**Figure 19a** is a graph showing the relative levels of the di- and tri-phosphate derivatives of β-D-2'-C-methyl-ribofuranosyl cytidine and β-D-2'-C-methyl-ribofuranosyl uridine (mUrd) after incubation of HepG2 cells with 10 µM β-D-2'-C-methyl-ribofuranosyl cytidine over time, as described in Example 37. **Figure 19b** is a graph of the decay of the tri-phosphate derivative of β-D-2'-C-methyl-ribofuranosyl cytidine after incubation of HepG2 cells with 10 µM β-D-2'-C-methyl-ribofuranosyl cytidine over time. **Figure 19c** is a graph of the concentration of the di- and tri-phosphate derivatives of β-D-2'-C-methyl-ribofuranosyl cytidine and β-D-2'-C-methyl-ribofuranosyl uridine (mUrd) after incubation of HepG2 cells with 10µM β-D-2'-C-methyl-ribofuranosyl cytidine at increasing concentrations of the drug (µM).
**Figure 20** is a graph of the concentration (ng/ml) of β-D-2'-C-methyl-ribofuranosyl cytidine in human serum after administration of β-D-2'-C-methyl-ribofuranosyl cytidine-3'-O-L- valine ester to patients, as described in Example 40.
**Figure 21** is a graph of the median change of the titer of hepatitis C virus in human patients after administration of β-D-2'-C-methyl-ribofuranosyl cytidine-3'-O-L- valine ester, as described in Example 40. The graph indicates change from baseline in Log₁₀ HCV RNA by patient visit.
**Figure 22** is a table of the EC₅₀ and CC₅₀ of representative compounds in a BVDV cell protection assay.

### DETAILED DESCRIPTION OF THE INVENTION

The invention as disclosed herein is a compound and composition for the treatment of a *Flaviviridae* infection in humans and other host animals. The invention includes the administration of an effective HCV or *Flaviviridae* treatment amount of a 3'-prodrug of a 2'-branched β-D nucleoside as described herein or a pharmaceutically acceptable salt, derivative or prodrug thereof, optionally in a pharmaceutically acceptable carrier. The compound of this invention either possesses antiviral (i.e., anti-HCV) activity, or is metabolized to a compound that exhibits such activity.

The 2'- or 3'- prodrug of a 1', 2', 3' or 4'-branched β-D or β-L nucleoside are acyl derivates of a secondary or tertiary alcohol alpha to a secondary or tertiary carbon. Due to the steric hindrance of these prodrugs over the 5'-prodrugs, an acyl derivative of a primary alcohol, these prodrugs differently modulate the biological properties of the molecule *in vivo.* It has been discovered that the 2'- and 3'-prodrugs of a 1', 2', 3' or 4'-branched β-D or β-L nucleoside can provide a drug with increased half-life and improved pharmacokinetic profile.

The 2'- and 3'- prodrugs in a preferred embodiment is a cleavable acyl group, and most particularly, an amino acid moiety, prepared from any naturally occurring or synthetic α, β γ or δ amino acid, including but is not limited to, glycine, alanine, valine, leucine, isoleucine, methionine, phenylalanine, tryptophan, proline, serine, threonine, cysteine, tyrosine, asparagine, glutamine, aspartate, glutamate, lysine, arginine and histidine. In a preferred embodiment, the amino acid is in the L-configuration. Alternatively, the amino acid can be a derivative of alanyl, valinyl, leucinyl, isoleuccinyl, prolinyl, phenylalaninyl, tryptophanyl, methioninyl, glycinyl, serinyl, threoninyl, cysteinyl, tyrosinyl, asparaginyl, glutaminyl, aspartoyl, glutaroyl, lysinyl, argininyl, histidinyl, β-alanyl, β-valinyl, β-leucinyl, β-isoleuccinyl, β-prolinyl, β-phenylalaninyl, β-tryptophanyl, β-methioninyl, β-glycinyl, β-serinyl, β-threoninyl, β-cysteinyl, β-tyrosinyl, β-asparaginyl, β-glutaminyl, β-aspartoyl, β-glutaroyl, β-lysinyl, β-argininyl or β-histidinyl. In one particular, embodiment, the moiety is a valine ester. One particularly preferred compound is the 3'-valine ester of 2'-methyl-ribo-cytidine.

The oral bio-availability of 1', 2', 3' or 4'-branched β-D or β-L nucleoside as the neutral base and the HCl salt is low in rodents and non-human primates. It has been discovered that there is significant competition of 1', 2', 3' or 4'-branched β-D or β-L nucleoside with other nucleosides or nucleoside analogs for absorption, or transport, from the gastrointestinal tract and competition of other nucleosides or nucleoside analogs for the absorption with 1', 2', 3' or 4'-branched β-D or β-L nucleoside. In order to improve oral bioavailability and reduce the potential for drug-drug interaction, 2' and 3'-prodrugs of 1', 2', 3' or 4'-branched β-D or β-L nucleoside were obtained with higher oral bioavailability than the parent molecule and a reduced effect on the bioavailability of other nucleosides or nucleoside analogs used in combination.

The 2', 3', and/or 5'-mono, di or trivaline ester of a 1', 2', 3' or 4'-branched β-D or β-L nucleoside have higher oral bioavailability than the parent 1', 2', 3' or 4'-branched β-D or β-L nucleoside and reduced interaction with other nucleosides or nucleoside analogs when used in combination as compared to 1', 2', 3' or 4'-branched β-D or β-L nucleoside.

The 2', 3', and/or 5'-mono, di or trivaline ester of a 1', 2', 3' or 4'-branched β-D or β-L nucleoside can be converted to the parent 1', 2', 3' or 4'-branched β-D or β-L nucleoside through de-esterification in the gastrointestinal mucosa, blood or liver. The 2', 3', and/or 5'-mono, di or trivaline ester of a 1', 2', 3' or 4'-branched β-D or β-L nucleoside can be actively transported from the gastrointestinal lumen after oral delivery into the bloodstream by an amino acid transporter function in the mucosa of the gastrointestinal tract. This accounts for the increase in oral bioavailability compared to the parent 1', 2', 3' or 4'-branched β-D or β-L nucleoside that is transported primarily by a nucleoside transporter function. There is reduced competition for uptake of the 2', 3', and/or 5'-mono, di or trivaline ester of 1', 2', 3' or 4'-branched β-D or β-L nucleoside with other nucleosides or nucleoside analogs that are transported by the nucleoside transporter function and not the amino acid transporter function. As partial de-esterification of the di or trivaline ester or 1', 2', 3' or 4'-branched β-D or β-L nucleoside occurs prior to complete absorption, the mono or divaline ester continues to be absorbed using the amino acid transporter function. Therefore, the desired outcome of better absorption, or bioavailability, and reduced competition with other nucleosides or nucleoside analogs for uptake into the bloodstream can be maintained.

In summary, the present invention includes the following features:
(a) a 3'-prodrug of a 2'-branched β-D nucleoside, as described herein, and pharmaceutically acceptable salts and compositions thereof;
(b) a 3'-prodrug of a 2'-branched β-D nucleoside as described herein, and pharmaceutically acceptable salts and compositions thereof for use in the treatment and/or prophylaxis of a *Flaviviridae* infection, especially in individuals diagnosed as having a *Flaviviridae* infection or being at risk of becoming infected by hepatitis C;
(c) a 3'-prodrug of a 2'-branched β-D nucleoside, or their pharmaceutically acceptable salts and compositions as described herein substantially in the absence of the opposite enantiomers of the described nucleoside, or substantially isolated from other chemical entities;
(e) pharmaceutical formulations comprising a 3'-prodrug of a 2' branched β-D nucleoside or a pharmaceutically acceptable salt thereof together with a pharmaceutically acceptable carrier or diluent;
(f) pharmaceutical formulations comprising a 3'-prodrug of a 2'-branched β-D nucleoside or a pharmaceutically acceptable salt thereof together with one or more other effective anti-HCV agents, optionally in a pharmaceutically acceptable carrier or diluent;
(g) pharmaceutical formulations comprising a 3'-prodrug of a 2'-branched β-D nucleoside or a pharmaceutically acceptable salt thereof together with the parent of a different a 1', 2', 3' or 4'-branched β-D or β-L nucleoside, optionally in a pharmaceutically acceptable carrier or diluent;
(m) a 3'-prodrug of a 2'-branched β-D nucleoside, and pharmaceutically acceptable salts and compositions thereof for the treatment and/or prophylaxis of a *Flaviviridae* infection in a host;
(n) a 3'-prodrug of a, 2'-branched β-D nucleoside, its pharmaceutically acceptable salt or composition in combination and/or alternation with one or more effective anti-HCV agent for the treatment and/or prophylaxis of a *Flaviviridae* infection in a host;
(o) a 3'-prodrug of a 2' branched β-D nucleoside, or its pharmaceutically acceptable salt or composition with the parent of a different a 1', 2', 3' or 4'-branched β-D or β-L nucleoside for the treatment and/or prophylaxis of a *Flaviviridae* infection in a host;
(p) a 3'-prodrug of a β-D-2'-methyl-cytidine, or its pharmaceutically acceptable salt or composition thereof for the treatment and/or prophylaxis of a *Flaviviridae* infection in a host;
(q) the 3',5'-divalyl ester of β-D-2'-methyl-cytidine, or its pharmaceutically acceptable salt or composition thereof for the treatment and/or prophylaxis of a *Flaviviridae* infection in a host;
(r) use of a 3'-prodrug of a 2'-branched β-D nucleoside, and pharmaceutically acceptable salts and compositions thereof in the manufacture of a medicament for treatment and/or prophylaxis of a *Flaviviridae* infection;
(s) use of a 3'-prodrug of a 2'-branched β-D nucleoside, its pharmaceutically acceptable salt or composition in combination and/or alternation with one or more effective anti-HCV agent in the manufacture of a medicament for the treatment and/or prophylaxis of a *Flaviviridae* infection in a host;
(t) use of a 3'-prodrug of a 2'-branched β-D nucleoside, or its pharmaceutically acceptable salt or composition with the parent of a different a 1', 2', 3' or 4'-branched β-D or β-L nucleoside in the manufacture of a medicament for the treatment and/or prophylaxis of a *Flaviviridae* infection in a host;
(u) use of a 3'-prodrug of a β-D-2'-methyl-cytidine, or its pharmaceutically acceptable salt or composition thereof in the manufacture of a medicament for the treatment and/or prophylaxis of a *Flaviviridae* infection in a host; and
(v) use of the 3',5'-divalyl ester of β-D-2'-methyl-cytidine, or its pharmaceutically acceptable salt or composition thereof in the manufacture of a medicament for the treatment and/or prophylaxis of a *Flaviviridae* infection in a host.

*Flaviviridae* included within the scope of this invention are discussed generally in Fields Virology, Editors: Fields, B. N., Knipe, D. M., and Howley, P. M., Lippincott-Raven Publishers, Philadelphia, PA, Chapter 31, 1996. In a particular embodiment of the invention, the *Flaviviridae* is HCV. In an alternate embodiment of the invention, the *Flaviviridae* is a flavivirus or pestivirus. Specific flaviviruses include, without limitation: Absettarov, Alfuy, Apoi, Aroa, Bagaza, Banzi, Bouboui, Bussuquara, Cacipacore, Carey Island, Dakar bat, Dengue 1, Dengue 2, Dengue 3, Dengue 4, Edge Hill, Entebbe bat, Gadgets Gully, Hanzalova, Hypr, Ilheus, Israel turkey meningoencephalitis, Japanese encephalitis, Jugra, Jutiapa, Kadam, Karshi, Kedougou, Kokobera, Koutango, Kumlinge, Kunjin, Kyasanur Forest disease, Langat, Louping ill, Meaban, Modoc, Montana myotis leukoencephalitis, Murray valley encephalitis, Naranjal, Negishi, Ntaya, Omsk hemorrhagic fever, Phnom-Penh bat, Powassan, Rio Bravo, Rocio, Royal Farm, Russian spring-summer encephalitis, Saboya, St. Louis encephalitis, Sal Vieja, San Perlita, Saumarez Reef, Sepik, Sokuluk, Spondweni, Stratford, Tembusu, Tyuleniy, Uganda S, Usutu, Wesselsbron, West Nile, Yaounde, Yellow fever, and Zika.

Pestiviruses included within the scope of this invention are discussed generally in Fields Virology, Editors: Fields, B. N., Knipe, D. M., and Howley, P. M., Lippincott-Raven Publishers, Philadelphia, PA, Chapter 33, 1996. Specific pestiviruses include, without limitation: bovine viral diarrhea virus ("BVDV"), classical swine fever virus ("CSFV," also called hog cholera virus), and border disease virus ("BDV").

### I. Active Compounds

In one embodiment, a compound of Formula II, or a pharmaceutically acceptable salt thereof, is provided: 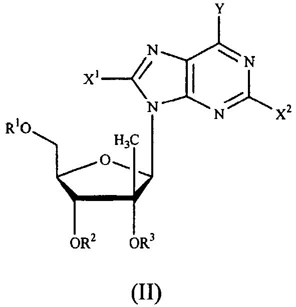 wherein:
R¹ H mono-, di- or triphosphate a stabilized phosphate
R2 is an amino acid residue
R3 is H;
Y is hydrogen, bromo, chloro, fluoro, iodo, OR⁴, NR⁴R⁵ or SR⁴;
X¹ and X² are independently selected from the group consisting of H, straight chained, branched or cyclic alkyl, CO-alkyl, CO-aryl, CO-alkoxyalkyl, chloro, bromo, fluoro, iodo, OR⁴, NR⁴R⁵ or SR⁵; and
R⁴ and R⁵ are independently hydrogen, acyl (including lower acyl), or alkyl (including but not limited to methyl, ethyl, propyl and cyclopropyl).

In a preferred subembodiment, a compound of Formula II, or a pharmaceutically acceptable salt thereof, is provided wherein:
X¹ is H;
X² is H or NH₂; and
Y is hydrogen, bromo, chloro, fluoro, iodo, NH₂ or OH.

In one embodiment, a compound of Formula V, or a pharmaceutically acceptable salt thereof, is provided: 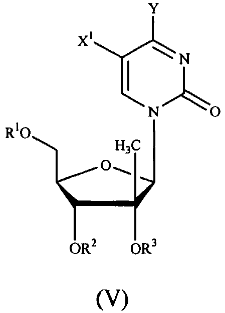 wherein:
R¹ is H or mono-, di- or triphosphate or a stabilized phosphate
R2 is an amino acid residue;
R3 is H;
Y is hydrogen, bromo, chloro, fluoro, iodo, OR⁴, NR⁴R⁵ or SR⁴;
X¹ is selected from the group consisting of H, straight chained, branched or cyclic alkyl, CO-alkyl, CO-aryl, CO-alkoxyalkyl, chloro, bromo, fluoro, iodo, OR⁴, NR⁴NR⁵ or SR⁵; and
R⁴ and R⁵ are independently hydrogen, acyl (including lower acyl), or alkyl (including but not limited to methyl, ethyl, propyl and cyclopropyl).

In a preferred subembodiment, a compound of Formula V, or a pharmaceutically acceptable salt thereof, is provided wherein:
X¹ is H or CH₃; and
Y is hydrogen, bromo, chloro, fluoro, iodo, NH₂ or OH.

### Stereochemistry

It is appreciated that nucleosides of the present invention have several chiral centers and may exist in and be isolated in optically active forms. Some compounds may exhibit polymorphism. It is to be understood that, within the scope of the claims, the present invention encompasses any optically-active, diastereomeric, polymorphic, or stereoisomeric form, or mixtures thereof, of a compound of the invention, which possess the useful properties described herein. It being well known in the art how to prepare optically active forms (for example, by resolution of the racemic form by recrystallization techniques, by synthesis from optically-active starting materials, by chiral synthesis, or by chromatographic separation using a chiral stationary phase).

In particular, since the 1' and 4' carbons of the nucleoside are chiral, their nonhydrogen substituents (the base and the CHOR groups, respectively) can be either cis (on the same side) or trans (on opposite sides) with respect to the sugar ring system. The four optical isomers therefore are represented by the following configurations (when orienting the sugar moiety in a horizontal plane such that the oxygen atom is in the back): cis (with both groups "up", which corresponds to the configuration of naturally occurring β-D nucleosides), cis (with both groups "down", which is a nonnaturally occurring β-L configuration), trans (with the C2' substituent "up" and the C4' substituent "down"), and trans (with the C2' substituent "down" and the C4' substituent "up"). The "D-nucleosides" are cis nucleosides in a natural configuration and the "L-nucleosides" are cis nucleosides in the non-naturally occurring configuration.

Likewise, most amino acids are chiral (designated as L or D, wherein the L enantiomer is the naturally occurring configuration) and can exist as separate enantiomers.

Examples of methods to obtain optically active materials are known in the art, and include at least the following.
i) physical separation of crystals - a technique whereby macroscopic crystals of the individual enantiomers are manually separated. This technique can be used if crystals of the separate enantiomers exist, i.e., the material is a conglomerate, and the crystals are visually distinct;
ii) simultaneous crystallization - a technique whereby the individual enantiomers are separately crystallized from a solution of the racemate, possible only if the latter is a conglomerate in the solid state;
iii) enzymatic resolutions - a technique whereby partial or complete separation of a racemate by virtue of differing rates of reaction for the enantiomers with an enzyme;
iv) enzymatic asymmetric synthesis - a synthetic technique whereby at least one step of the synthesis uses an enzymatic reaction to obtain an enantiomerically pure or enriched synthetic precursor of the desired enantiomer;
v) chemical asymmetric synthesis - a synthetic technique whereby the desired enantiomer is synthesized from an achiral precursor under conditions that produce asymmetry (i.e., chirality) in the product, which may be achieved using chiral catalysts or chiral auxiliaries;
vi) diastereomer separations - a technique whereby a racemic compound is reacted with an enantiomerically pure reagent (the chiral auxiliary) that converts the individual enantiomers to diastereomers. The resulting diastereomers are then separated by chromatography or crystallization by virtue of their now more distinct structural differences and the chiral auxiliary later removed to obtain the desired enantiomer;
vii) first- and second-order asymmetric transformations - a technique whereby diastereomers from the racemate equilibrate to yield a preponderance in solution of the diastereomer from the desired enantiomer or where preferential crystallization of the diastereomer from the desired enantiomer perturbs the equilibrium such that eventually in principle all the material is converted to the crystalline diastereomer from the desired enantiomer. The desired enantiomer is then released from the diastereomer;
viii) kinetic resolutions - this technique refers to the achievement of partial or complete resolution of a racemate (or of a further resolution of a partially resolved compound) by virtue of unequal reaction rates of the enantiomers with a chiral, non-racemic reagent or catalyst under kinetic conditions;
ix) enantiospecific synthesis from non-racemic precursors - a synthetic technique whereby the desired enantiomer is obtained from non-chiral starting materials and where the stereochemical integrity is not or is only minimally compromised over the course of the synthesis;
x) chiral liquid chromatography - a technique whereby the enantiomers of a racemate are separated in a liquid mobile phase by virtue of their differing interactions with a stationary phase. The stationary phase can be made of chiral material or the mobile phase can contain an additional chiral material to provoke the differing interactions;
xi) chiral gas chromatography - a technique whereby the racemate is volatilized and enantiomers are separated by virtue of their differing interactions in the gaseous mobile phase with a column containing a fixed non-racemic chiral adsorbent phase;
xii) extraction with chiral solvents - a technique whereby the enantiomers are separated by virtue of preferential dissolution of one enantiomer into a particular chiral solvent;
xiii) transport across chiral membranes - a technique whereby a racemate is placed in contact with a thin membrane barrier. The barrier typically separates two miscible fluids, one containing the racemate, and a driving force such as concentration or pressure differential causes preferential transport across the membrane barrier. Separation occurs as a result of the non-racemic chiral nature of the membrane which allows only one enantiomer of the racemate to pass through.

### II. Definitions

The term "alkyl", as used herein, unless otherwise specified, refers to a saturated straight, branched, or cyclic, primary, secondary, or tertiary hydrocarbon of typically C₁ to C₁₀, and specifically includes methyl, CF₃, CCl₃, CFCl₂, CF₂Cl, ethyl, CH₂CF₃, CF₂CF₃, propyl, isopropyl, cyclopropyl, butyl, isobutyl, secbutyl, *t*-butyl, pentyl, cyclopentyl, isopentyl, neopentyl, hexyl, isohexyl, cyclohexyl, cyclohexylmethyl, 3-methylpentyl, 2,2-dimethylbutyl, and 2,3-dimethylbutyl. The term includes both substituted and unsubstituted alkyl groups, and particularly includes halogenated alkyl groups, and even more particularly fluorinated alkyl groups. Non-limiting examples of moieties with which the alkyl group can be substituted are selected from the group consisting of halogen (fluoro, chloro, bromo or iodo), hydroxyl, amino, alkylamino, arylamino, alkoxy, aryloxy, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991.

The term "lower alkyl", as used herein, and unless otherwise specified, refers to a C₁ to C₄ saturated straight, branched, or if appropriate, a cyclic (for example, cyclopropyl) alkyl group, including both substituted and unsubstituted moieties.

The term "alkylamino" or "arylamino" refers to an amino group that has one or two alkyl or aryl substituents, respectively. Unless otherwise specifically stated in this application, when alkyl is a suitable moiety, lower alkyl is preferred. Similarly, when alkyl or lower alkyl is a suitable moiety, unsubstituted alkyl or lower alkyl is preferred.

The term "protected" as used herein and unless otherwise defined refers to a group that is added to an oxygen, nitrogen, or phosphorus atom to prevent its further reaction or for other purposes. A wide variety of oxygen and nitrogen protecting groups are known to those skilled in the art of organic synthesis.

The term "aryl", as used herein, and unless otherwise specified, refers to phenyl, biphenyl, or naphthyl, and preferably phenyl. The term includes both substituted and unsubstituted moieties. The aryl group can be substituted with any described moiety, including, but not limited to, one or more moieties selected from the group consisting of halogen (fluoro, chloro, bromo or iodo), hydroxyl, amino, alkylamino, arylamino, alkoxy, aryloxy, nitro, cyano, sulfonic acid, sulfate, phosphonic acid, phosphate, or phosphonate, either unprotected, or protected as necessary, as known to those skilled in the art, for example, as taught in Greene, et al., Protective Groups in Organic Synthesis, John Wiley and Sons, Second Edition, 1991**.**

The term "alkaryl" or "alkylaryl" refers to an alkyl group with an aryl substituent. The term aralkyl or arylalkyl refers to an aryl group with an alkyl substituent.

The term "halo", as used herein, includes chloro, bromo, iodo, and fluoro.

The term "purine" or "pyrimidine" base includes, but is not limited to, adenine, N⁶-alkylpurines, N⁶-acylpurines (wherein acyl is C(O)(alkyl, aryl, alkylaryl, or arylalkyl), N⁶-benzylpurine, N⁶-halopurine, N⁶-vinylpurine, N⁶-acetylenic purine, N⁶-acyl purine, N⁶-hydroxyalkyl purine, N⁶-alkylaminopurine, N⁶-thioalkyl purine, N²-alkylpurines, N²-alkyl-6-thiopurines, thymine, cytosine, 5-fluorocytosine, 5-methylcytosine, 6-azapyrimidine, including 6-azacytosine, 2- and/or 4-mercaptopyrmidine, uracil, 5-halouracil, including 5-fluorouracil, C⁵-alkylpyrimidines, C⁵-benzylpyrimidines, C⁵-halopyrimidines, C⁵-vinylpyrimidine, C⁵-acetylenic pyrimidine, C⁵-acyl pyrimidine, C⁵-hydroxyalkyl purine, C⁵-amidopyrimidine, C⁵-cyanopyrimidine, ,C⁵-iodopyrimidine, C⁶-iodo-pyrimidine, C⁵-Br-vinyl pyrimidine, C⁶-Br-vinyl pyrimidine, C⁵-nitropyrimidine, C⁵-amino-pyrimidine, N²-alkylpurines, N²-alkyl-6-thiopurines, 5-azacytidinyl, 5-azauracilyl, triazolopyridinyl, imidazolopyridinyl, pyrrolopyrimidinyl, and pyrazolopyrimidinyl. Purine bases include, but are not limited to, guanine, adenine, hypoxanthine, 2,6-diaminopurine, and 6-chloropurine. Functional oxygen and nitrogen groups on the base can be protected as necessary or desired. Suitable protecting groups are well known to those skilled in the art, and include trimethylsilyl, dimethylhexylsilyl, *t*-butyldimethylsilyl, and *t*-butyldiphenylsilyl, trityl, alkyl groups, and acyl groups such as acetyl and propionyl, methanesulfonyl, and p-toluenesulfonyl.

The term "acyl" or "O-linked ester" refers to a group of the formula C(O)R', wherein R' is an straight, branched, or cyclic alkyl (including lower alkyl), carboxylate reside of amino acid, aryl including phenyl, alkaryl, aralkyl including benzyl, alkoxyalkyl including methoxymethyl, aryloxyalkyl such as phenoxymethyl; or substituted alkyl (including lower alkyl), aryl including phenyl optionally substituted with chloro, bromo, fluoro, iodo, C₁ to C₄ alkyl or C₁ to C₄ alkoxy, sulfonate esters such as alkyl or aralkyl sulphonyl including methanesulfonyl, the mono, di or triphosphate ester, trityl or monomethoxy-trityl, substituted benzyl, alkaryl, aralkyl including benzyl, alkoxyalkyl including methoxymethyl, aryloxyalkyl such as phenoxymethyl. Aryl groups in the esters optimally comprise a phenyl group. In particular, acyl groups include acetyl, trifluoroacetyl, methylacetyl, cyclpropylacetyl, propionyl, butyryl, hexanoyl, heptanoyl, octanoyl, neo-heptanoyl, phenylacetyl, 2-acetoxy-2-phenylacetyl, diphenylacetyl, α-methoxy-α-trifluoromethyl-phenylacetyl, bromoacetyl, 2-nitro-benzeneacetyl, 4-chloro-benzeneacetyl, 2-chloro-2,2-diphenylacetyl, 2-chloro-2-phenylacetyl, trimethylacetyl, chlorodifluoroacetyl, perfluoroacetyl, fluoroacetyl, bromodifluoroacetyl, methoxyacetyl, 2-thiopheneacetyl, chlorosulfonylacetyl, 3-methoxyphenylacetyl, phenoxyacetyl, tert-butylacetyl, trichloroacetyl, monochloro-acetyl, dichloroacetyl, 7H-dodecafluoro-heptanoyl, perfluoro-heptanoyl, 7H-dodeca-fluoroheptanoyl, 7-chlorododecafluoro-heptanoyl, 7-chloro-dodecafluoro-heptanoyl, 7H-dodecafluoroheptanoyl, 7H-dodeca-fluoroheptanoyl, nona-fluoro-3,6-dioxa-heptanoyl, nonafluoro-3,6-dioxaheptanoyl, perfluoroheptanoyl, methoxybenzoyl, methyl 3-amino-5-phenylthiophene-2-carboxyl, 3,6-dichloro-2-methoxy-benzoyl, 4-(1,1,2,2-tetrafluoro-ethoxy)-benzoyl, 2-bromo-propionyl, omega-aminocapryl, decanoyl, n-pentadecanoyl, stearyl, 3-cyclopentyl-propionyl, 1-benzene-carboxyl, O-acetylmandelyl, pivaloyl acetyl, 1-adamantane-carboxyl, cyclohexane-carboxyl, 2,6-pyridinedicarboxyl, cyclopropane-carboxyl, cyclobutane-carboxyl, perfluorocyclohexyl carboxyl, 4-methylbenzoyl, chloromethyl isoxazolyl carbonyl, perfluorocyclohexyl carboxyl, crotonyl, 1-methyl-1H-indazole-3-carbonyl, 2-propenyl, isovaleryl, 1-pyrrolidinecarbonyl, 4-phenylbenzoyl.

The term "amino acid" includes naturally occurring and synthetic α, β γ or δ amino acids, and includes but is not limited to, amino acids found in proteins, *i.e.* glycine, alanine, valine, leucine, isoleucine, methionine, phenylalanine, tryptophan, proline, serine, threonine, cysteine, tyrosine, asparagine, glutamine, aspartate, glutamate, lysine, arginine and histidine. In a preferred embodiment, the amino acid is in the L-configuration. Alternatively, the amino acid can be a derivative of alanyl, valinyl, leucinyl, isoleuccinyl, prolinyl, phenylalaninyl, tryptophanyl, methioninyl, glycinyl, serinyl, threoninyl, cysteinyl, tyrosinyl, asparaginyl, glutaminyl, aspartoyl, glutaroyl, lysinyl, argininyl, histidinyl, β-alanyl, β-valinyl, β-leucinyl, β-isoleuccinyl, β-prolinyl, β-phenylalaninyl, β-tryptophanyl, β-methioninyl, β-glycinyl, β-serinyl, β-threoninyl, β-cysteinyl, β-tyrosinyl, β-asparaginyl, β-glutaminyl, β-aspartoyl, β-glutaroyl, β-lysinyl, β-argininyl or β-histidinyl.

As used herein, the term "substantially free of" or "substantially in the absence of" refers to a nucleoside composition that includes at least 85 or 90% by weight, preferably 95%, 98 % , 99% or 100% by weight, of the designated enantiomer of that nucleoside. In a preferred embodiment, in the compounds of this invention, the compounds are substantially free of enantiomers.

Similarly, the term "isolated" refers to a nucleoside composition that includes at least 85, 90%, 95%, 98%, 99% to 100% by weight, of the nucleoside, the remainder comprising other chemical species or enantiomers.

The term "host", as used herein, refers to an unicellular or multicellular organism in which the virus can replicate, including cell lines and animals, and preferably a human. Alternatively, the host can be carrying a part of the *Flaviviridae* viral genome, whose replication or function can be altered by the compounds of the present invention. The term host specifically refers to infected cells, cells transfected with all or part of the *Flaviviridae* genome and animals, in particular, primates (including chimpanzees) and humans. In most animal applications of the present invention, the host is a human patient. Veterinary applications, in certain indications, however, are clearly anticipated by the present invention (such as chimpanzees).

The term "pharmaceutically acceptable salt or prodrug" is used throughout the specification to describe any pharmaceutically acceptable form (such as an ester, phosphate ester, salt of an ester or a related group) of a nucleoside compound which, upon administration to a patient, provides the nucleoside compound. Pharmaceutically acceptable salts include those derived from pharmaceutically acceptable inorganic or organic bases and acids. Suitable salts include those derived from alkali metals such as potassium and sodium, alkaline earth metals such as calcium and magnesium, among numerous other acids well known in the pharmaceutical art. Pharmaceutically acceptable prodrugs refer to a compound that is metabolized, for example hydrolyzed or oxidized, in the host to form the compound of the present invention. Typical examples of prodrugs include compounds that have biologically labile protecting groups on a functional moiety of the active compound. Prodrugs include compounds that can be oxidized, reduced, aminated, deaminated, hydroxylated, dehydroxylated, hydrolyzed, dehydrolyzed, alkylated, dealkylated, acylated, deacylated, phosphorylated, dephosphorylated to produce the active compound. The compounds of this invention possess antiviral activity against a *Flaviviridae,* or are metabolized to a compound that exhibits such activity.

### III. Prodrugs and Derivatives

### Pharmaceutically Aceptable Salts

In cases where compounds are sufficiently basic or acidic to form stable nontoxic acid or base salts, administration of the compound as a pharmaceutically acceptable salt may be appropriate. Examples of pharmaceutically acceptable salts are organic acid addition salts formed by addition of acids, which form a physiological acceptable anion, for example, tosylate, methanesulfonate, acetate, citrate, malonate, tartarate, succinate, benzoate, ascorate, α-ketoglutarate, α-glycerophosphate, formate, fumarate, propionate, glycolate, lactate, pyruvate, oxalate, maleate, and salicylate. Suitable inorganic salts may also be formed, including, sulfate, nitrate, bicarbonate, carbonate salts, hydrobromate and phosphoric acid. In a preferred embodiment, the salt is a mono- or di-hydrochloride salt.

Pharmaceutically acceptable salts may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made. In one embodiment, the salt is a hydrochloride salt of the compound. In a further embodiment, the pharmaceutically acceptable salt is a dihydrochloride salt.

### Nucleotide Prodrug Formulations

The nucleosides described herein can be administered as a nucleotide prodrug to increase the activity, bioavailability, stability or otherwise alter the properties of the nucleoside. A number of nucleotide prodrug ligands are known. In general, alkylation, acylation or other lipophilic modification of the mono-, di- or triphosphate of the nucleoside reduces polarity and allows passage into cells. Examples of substituent groups that can replace one or more hydrogens on the phosphate moiety are alkyl, aryl, steroids, carbohydrates, including sugars, 1,2-diacylglycerol and alcohols.

In an alternative embodiment, the compound is administered as a phosphonate, phosphorothioate or SATE derivative.

Many are described in R. Jones and N. Bischoferger, Antiviral Research, 1995, 27:1-17. Any of these can be used in combination with the disclosed nucleosides to achieve a desired effect. Nonlimiting examples of U.S. patents that disclose suitable lipophilic substituents that can be covalently incorporated into the nucleoside, preferably at the 5'-OH position of the nucleoside or lipophilic preparations, include U.S. Patent Nos. 5,149,794 (Sep. 22, 1992, Yatvin et al.); 5,194,654 (Mar. 16, 1993, Hostetler et al.*,* 5,223,263 (June 29, 1993, Hostetler et al.); 5,256,641 (Oct. 26, 1993, Yatvin et al.); 5,411,947 (May 2, 1995, Hostetler et al.); 5,463,092 (Oct. 31, 1995, Hostetler et al.); 5,543,389 (Aug. 6, 1996, Yatvin et al.); 5,543,390 (Aug. 6, 1996, Yatvin et al.); 5,543,391 (Aug. 6, 1996, Yatvin et al.); and 5,554,728 (Sep. 10, 1996; Basava et al.). Foreign patent applications that disclose lipophilic substituents that can be attached to the nucleosides of the present invention, or lipophilic preparations, include WO 89/02733, WO 90/00555, WO 91/16920, WO 91/18914, WO 93/00910, WO 94/26273, WO 96/15132, EP 0 350 287, EP 93917054.4, and WO 91/19721.

The active nucleoside can also be provided as a 2', 3' and/or 5'-phosphoether lipid or a 2', 3' and/or 5'-ether lipid, as disclosed in the following references: Kucera, L.S., N. Iyer, et al. 1990 AIDS Res. Hum. Retro Viruses. 6:491-501; Piantadosi, C., J. Marasco C.J., et al. 1991 J. Med. Chem. 34:1408.1414; Hosteller, K.Y., D.D. Richman, et al. 1992 Antimicrob. Agents Chemother. 36:2025.2029; Hosetler, K.Y., L.M. Stuhmiller, 1990. J. Biol. Chem. 265:61127.

Nonlimiting examples of U.S. patents that disclose suitable lipophilic substituents that can be covalently incorporated into the nucleoside, preferably at the 2', 3' and/or 5'-OH position of the nucleoside or lipophilic preparations, include U.S. Patent Nos. 5,149,794 (Sep. 22, 1992, Yatvin et al.); 5,194,654 (Mar. 16, 1993, Hostetler et al.*,* 5,223,263 (June 29, 1993, Hostetler et al.); 5,256,641 (Oct. 26, 1993, Yatvin et al.); 5,411,947 (May 2, 1995, Hostetler et al.); 5,463,092 (Oct. 31, 1995, Hostetler et al.); 5,543,389 (Aug. 6, 1996, Yatvin et al.); 5,543,390 (Aug. 6, 1996, Yatvin et al.); 5,543,391 (Aug. 6, 1996, Yatvin et al.); and 5,554,728 (Sep. 10, 1996; Basava et al.). Foreign patent applications that disclose lipophilic substituents that can be attached to the nucleosides of the present invention, or lipophilic preparations, include WO 89/02733, WO 90/00555, WO 91/16920, WO 91/18914, WO 93/00910, WO 94/26273, WO 96/15132, EP 0 350 287, EP 93917054.4, and WO 91/19721.

Aryl esters, especially phenyl esters, are also provided. Nonlimiting examples are disclosed in DeLambert et al., J. Med. Chem. 37: 498 (1994). Phenyl esters containing a carboxylic ester ortho to the phosphate are also provided. Khamnei and Torrence, J. Med. Chem.; 39:4109-4115 (1996). In particular, benzyl esters, which generate the parent compound, in some cases using substituents at the ortho- or para-position to accelerate hydrolysis, are provided. Examples of this class of prodrugs are described by Mitchell et al., J. Chem. Soc. Perkin Trans. I 2345 (1992); Brook, et al. WO 91/19721; and Glazier et al. WO 91/19721.

Cyclic phosphonate esters are also provided. Nonlimiting examples are disclosed in Hunston et al., J. Med Chem. 27: 440-444 (1984) and Starrett et al. J. Med. Chem. 37: 1857-1864 (1994). Additionally, cyclic 3',5'-phosphate esters are provided. Nonlimiting examples are disclosed in Meier et al. J. Med. Chem. 22: 811-815 (1979). Cyclic 1',3'-propanyl phosphonate and phosphate esters, such as ones containing a fused aryl ring, i.e. the cyclosaligenyl ester, are also provided (Meier et al., Bioorg. Med. Chem. Lett. 7: 99-104 (1997)). Unsubstituted cyclic 1',3'-propanyl esters of the monophosphates are also provided (Farquhar et al., J. Med. Chem. 26: 1153 (1983); Farquhar et al., J. Med. Chem. 28: 1358 (1985)) were prepared. In addition, cyclic 1',3'-propanyl esters substituted with a pivaloyloxy methyloxy group at C-1' are provided (Freed et al., Biochem. Pharmac. 38: 3193 (1989); Biller et al., U.S. Pat. No. 5,157,027).

Cyclic phosphoramidates are known to cleave in vivo by an oxidative mechanism. Therefore, in one embodiment of the present invention, a variety of substituted 1',3' propanyl cyclic phosphoramidates are provided. Non-limiting examples are disclosed by Zon, Progress in Med. Chem. 19, 1205 (1982). Additionally, a number of 2'- and 3'-substituted proesters are provided. 2'-Substituents include methyl, dimethyl, bromo, trifluoromethyl, chloro, hydroxy, and methoxy; 3'-substituents including phenyl, methyl, trifluoromethyl, ethyl, propyl, i-propyl, and cyclohexyl. A variety of 1'-substituted analogs are also provided.

Cyclic esters of phosphorus-containing compounds are also provided. Non-limiting examples are described in the following:
- [1] di and tri esters of phosphoric acids as reported in Nifantyev et al., Phosphorus, Sulfur Silicon and Related Eelements, 113: 1 (1996); Wijnberg et al., EP-180276 A1;
- [2] phosphorus (III) acid esters. Kryuchkov et al., Izv. Akad. Nauk SSSR, Ser. Khim. 6: 1244 (1987). Some of the compounds were claimed to be useful for the asymmetric synthesis of L-Dopa precursors. Sylvain et al., DE3512781 A1;
- [3] phosphoramidates. Shih et al., Bull. Inst. Chem. Acad. Sin, 41: 9 (1994); Edmundson et al., J. Chem. Res. Synop. 5: 122 (1989); and
- [4] phosphonates. Neidlein et al., Heterocycles 35: 1185 (1993).

Further, nonlimiting examples of U.S. and International Patent Applications that disclose suitable cyclic phosphoramidate prodrugs include U.S. Patent No. 6,312,662; WO 99/45016; WO 00/52015; WO 01/47935; and WO 01/18013 to Erion, et al. from Metabasis Therapeutics, Inc. Specifically, prodrugs of Formula A below are provided: 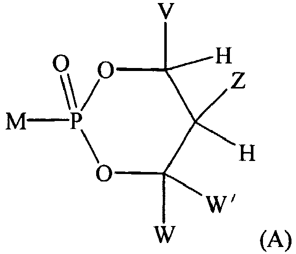 wherein:
- together V and Z are connected via an additional 3-5 atoms to form a cyclic group containing 5-7 atoms, optionally 1 heteroatom, substituted with hydroxy, acyloxy, alkoxycarbonyloxy, or aryloxycarbonyloxy attached to a carbon atom that is three atoms from both O groups attached to the phosphorus; or
- together V and Z are connected via an additional 3-5 atoms to form a cyclic group, optionally containing 1 heteroatom, that is fused to an aryl group at the beta and gamma position to the O attached to the phosphorus;
- together V and W are connected via an additional 3 carbon atoms to form an optionally substituted cyclic group containing 6 carbon atoms and substituted with one substituent selected from the group consisting of hydroxy, acyloxy, alkoxycarbonyloxy, alkylthiocarbonyloxy, and aryloxycarbonyloxy, attached to one of said carbon atoms that is three atoms from an O attached to the phosphorus;
- together Z and W are connected via an additional 3-5 atoms to form a cyclic group, optionally containing one heteroatom, and V must be aryl, substituted aryl, heteroaryl, or substituted heteroaryl;
- together W and W' are connected via an additional 2-5 atoms to form a cyclic group, optionally containing 0-2 heteroatoms, and V must be aryl, substituted aryl, heteroaryl, or substituted heteroaryl;
- Z is selected from the group consisting of -CHR² OH, -CHR² OC(O)R³, -CHR² OC(S)R³, -CHR² OC(S)OR³, -CHR² OC(O)SR³, -CHR² OCO₂ R³, -OR², -SR², -CHR² N₃, -CH² aryl, -CH(aryl)OH, -CH(CH=CR²₂)OH, -CH(C.ident.CR²)OH, -R², -NR²₂, - OCOR³, -OCO₂ R³, -SCOR³, -SCO₂ R³, -NHCOR², -NHCO₂ R³, -CH₂ NHaryl, - (CH₂)ₚ -OR¹², and -(CH₂)ₚ -SR¹²;
- p is an integer 2 or 3;
- with the provisos that:
   - a) V, Z, W, W' are not all -H; and
   - b) when Z is -R², then at least one of V, W, and W' is not -H, alkyl, aralkyl, or alicyclic;
- R² is selected from the group consisting of R³ and -H;
- R³ is selected from the group consisting of alkyl, aryl, alicyclic, and aralkyl;
- R¹² is selected from the group consisting of -H, and lower acyl;
- M is the biologically active agent, and that is attached to the phosphorus in Formula A via the 2', 3' and/or 5'-hydroxyl.

### IV. Combination or Alternation Therapy

The active compounds of the present invention can be administered in combination or alternation with another anti-flavivirus or pestivirus agent, or in particular an anti-HCV agent. In combination therapy, effective dosages of two or more agents are administered together, whereas in alternation or sequential-step therapy, an effective dosage of each agent is administered serially or sequentially. The dosages given will depend on absorption, inactivation and excretion rates of the drug as well as other factors known to those of skill in the art. It is to be noted that dosage values will also vary with the severity of the condition to be alleviated. It is to be further understood that for any particular subject, specific dosage regimens and schedules should be adjusted over time according to the individual need and the professional judgment of the person administering or supervising the administration of the compositions. In preferred embodiments, an anti-HCV (or anti-pestivirus or anti-flavivirus) compound that exhibits an EC₅₀ of 10-15 µM, or preferably less than 1-5 µM, is desirable.

It has been recognized that drug-resistant variants of flaviviruses, pestiviruses or HCV can emerge after prolonged treatment with an antiviral agent. Drug resistance most typically occurs by mutation of a gene that encodes for an enzyme used in viral replication. The efficacy of a drug against the viral infection can be prolonged, augmented, or restored by administering the compound in combination or alternation with a second, and perhaps third, antiviral compound that induces a different mutation from that caused by the principle drug. Alternatively, the pharmacokinetics, biodistribution or other parameter of the drug can be altered by such combination or alternation therapy. In general, combination therapy is typically preferred over alternation therapy because it induces multiple simultaneous stresses on the virus.

Any of the viral treatments described in the Background of the Invention can be used in combination or alternation with the compounds described in this specification. Nonlimiting examples include:
1) Protease inhibitors
   Examples include substrate-based NS3 protease inhibitors (Attwood et al., Antiviral peptide derivatives, PCT WO 98/22496, 1998; Attwood et al., Antiviral Chemistry and Chemotherapy 1999, 10, 259-273; Attwood et al., Preparation and use of amino acid derivatives as anti-viral agents, German Patent Pub. DE 19914474; Tung et al. Inhibitors of serine proteases, particularly hepatitis C virus NS3 protease, PCT WO 98/17679), including alphaketoamides and hydrazinoureas, and inhibitors that terminate in an electrophile such as a boronic acid or phosphonate (Llinas-Brunet et al, Hepatitis C inhibitor peptide analogues, PCT WO 99/07734); Non-substrate-based NS3 protease inhibitors such as 2,4,6-trihydroxy-3-nitro-benzamide derivatives (Sudo K. et al., Biochemical and Biophysical Research Communications, 1997, 238, 643-647; Sudo K. et al. Antiviral Chemistry and Chemotherapy, 1998, 9, 186), including RD3-4082 and RD3-4078, the former substituted on the amide with a 14 carbon chain and the latter processing a para-phenoxyphenyl group; and Sch 68631, a phenanthrenequinone, an HCV protease inhibitor (Chu M. et al., Tetrahedron Letters 37:7229-7232, 1996).
   Sch 351633, isolated from the fungus Penicillium griseofulvum, was identified as a protease inhibitor (Chu M. et al., Bioorganic and Medicinal Chemistry Letters 9:1949-1952). Eglin c, isolated from leech, is a potent inhibitor of several serine proteases such as S. griseus proteases A and B, α-chymotrypsin, chymase and subtilisin. Qasim M.A. et al., Biochemistry 36:1598-1607, 1997.
   U.S. patents disclosing protease inhibitors for the treatment of HCV include, for example, U.S. Patent No. 6,004,933 to Spruce et al. which discloses a class of cysteine protease inhibitors for inhibiting HCV endopeptidase 2; U.S. Patent No. 5,990,276 to Zhang et al. which discloses synthetic inhibitors of hepatitis C virus NS3 protease; U.S. Patent No. 5,538,865 to Reyes et a; WO 02/008251 to Corvas International, Inc, and WO 02/08187 and WO 02/008256 to Schering Corporation. HCV inhibitor tripeptides are disclosed in US Patent Nos. 6,534,523, 6,410,531, and 6,420,380 to Boehringer Ingelheim and WO 02/060926 to Bristol Myers Squibb. Diaryl peptides as NS3 serine protease inhibitors of HCV are disclosed in WO 02/48172 to Schering Corporation. Imidazoleidinones as NS3 serine protease inhibitors of HCV are disclosed in WO 02/08198 to Schering Corporation and WO 02/48157 to Bristol Myers Squibb. WO 98/17679 to Vertex Pharmaceuticals and WO 02/48116 to Bristol Myers Squibb also disclose HCV protease inhibitors.
2) Thiazolidine derivatives which show relevant inhibition in a reverse-phase HPLC assay with an NS3/4A fusion protein and NS5A/5B substrate (Sudo K. et al., Antiviral Research, 1996, 32, 9-18), especially compound RD-1-6250, possessing a fused cinnamoyl moiety substituted with a long alkyl chain, RD4 6205 and RD4 6193;
3) Thiazolidines and benzanilides identified in Kakiuchi N. et al. J. EBS Letters 421, 217-220; Takeshita N. et al. Analytical Biochemistry, 1997, 247, 242-246;
4) A phenan-threnequinone possessing activity against protease in a SDS-PAGE and autoradiography assay isolated from the fermentation culture broth of Streptomyces sp., Sch 68631 (Chu M. et al., Tetrahedron Letters, 1996, 37, 7229-7232), and Sch 351633, isolated from the fungus Penicillium griseofulvum, which demonstrates activity in a scintillation proximity assay (Chu M. et al., Bioorganic and Medicinal Chemistry Letters 9, 1949-1952);
5) Helicase inhibitors (Diana G.D. et al., Compounds, compositions and methods for treatment of hepatitis C, U.S. Pat. No. 5,633,358; Diana G.D. et al., Piperidine derivatives, pharmaceutical compositions thereof and their use in the treatment of hepatitis C, PCT WO 97/36554);
6) Nucleotide polymerase inhibitors and gliotoxin (Ferrari R. et al. Journal of Virology, 1999, 73, 1649-1654), and the natural product cerulenin (Lohmann V. et al., Virology, 1998, 249, 108-118);
7) Antisense phosphorothioate oligodeoxynucleotides (S-ODN) complementary to sequence stretches in the 5' non-coding region (NCR) of the virus (Alt M. et al., Hepatology, 1995, 22, 707-717), or nucleotides 326-348 comprising the 3' end of the NCR and nucleotides 371-388 located in the core coding region of the HCV RNA (Alt M. et al., Archives of Virology, 1997, 142, 589-599; Galderisi U. et al., Journal of Cellular Physiology, 1999, 181, 251-257);
8) Inhibitors of IRES-dependent translation (Ikeda N et al., Agent for the prevention and treatment of hepatitis C, Japanese Patent Pub. JP-08268890; Kai Y. et al. Prevention and treatment of viral diseases, Japanese Patent Pub. JP-10101591);
9) Ribozymes, such as nuclease-resistant ribozymes (Maccjak, D. J. et al., Hepatology 1999, 30, abstract 995) and those disclosed in U.S. Patent No. 6,043,077 to Barber et al., and U.S. Patent Nos. 5,869,253 and 5,610,054 to Draper et al.; and
10) Nucleoside analogs have also been developed for the treatment of Flaviviridae infections.
11) Any of the compounds described by Idenix Pharmaceuticals in International Publication Nos. WO 01/90121 and WO 01/92282;.
12) Other patent applications disclosing the use of certain nucleoside analogs to treat hepatitis C virus include: PCT/CA00/01316 (WO 01/32153; filed November 3, 2000) and PCT/CA01/00197 (WO 01/60315; filed February 19, 2001) filed by BioChem Pharma, Inc. (now Shire Biochem, Inc.); PCT/US02/01531 (WO 02/057425; filed January 18, 2002) and PCT/US02/03086 (WO 02/057287; filed January 18, 2002) filed by Merck & Co., Inc., PCT/EP01/09633 (WO 02/18404; published August 21, 2001) filed by Roche, and PCT Publication Nos. WO 01/79246 (filed April 13, 2001), WO 02/32920 (filed October 18, 2001) and WO 02/48165 by Pharmasset, Ltd.
13) PCT Publication No. WO 99/43691 to Emory University, entitled "2'-Fluoronucleosides" discloses the use of certain 2'-fluoronucleosides to treat HCV.
14) Other miscellaneous compounds including 1-amino-alkylcyclohexanes (U.S. Patent No. 6,034,134 to Gold et al.), alkyl lipids (U.S. Pat. No. 5,922,757 to Chojkier et al.), vitamin E and other antioxidants (U.S. Pat. No. 5,922,757 to Chojkier et al.), squalene, amantadine, bile acids (U.S. Pat. No. 5,846,964 to Ozeki et al.), N-(phosphonoacetyl)-L-aspartic acid, (U.S. Pat. No. 5,830,905 to Diana et al.), benzenedicarboxamides (U.S. Pat. No. 5,633,388 to Diana et al.), polyadenylic acid derivatives (U.S. Pat. No. 5,496,546 to Wang et al.), 2',3'-dideoxyinosine (U.S. Pat. No. 5,026,687 to Yarchoan et al.), benzimidazoles (U.S. Pat. No. 5,891,874 to Colacino et al.), plant extracts (U.S. Patent No. 5,837,257 to Tsai et al., U.S. Patent No. 5,725,859 to Omer et al., and U.S. Patent No. 6,056,961), and piperidenes (U.S. Patent No. 5,830,905 to Diana et al.).
15) Any other compounds currently in preclinical or clinical development for treatment of hepatitis C virus including: Interleukin-10 by Schering-Plough, IP-501 by Interneuron, Merimebodib (VX-497) by Vertex, AMANTADINE® (Symmetrel) by Endo Labs Solvay, HEPTAZYME® by RPI, IDN-6556 by Idun Pharma., XTL-002 by XTL., HCV/MF59 by Chiron, CIVACIR® (Hepatitis C Immune Globulin) by NABI, LEVOVIRIN® by ICN/Ribapharm, VIRAMIDINE® by ICN/Ribapharm, ZADAXIN® (thymosin alpha-1) by Sci Clone, thymosin plus pegylated interferon by Sci Clone, CEPLENE® (histamine dihydrochloride) by Maxim, VX 950 / LY 570310 by Vertex/Eli Lilly, ISIS 14803 by Isis Pharmaceutical/Elan, IDN-6556 by Idun Pharmaceuticals, Inc., JTK 003 by AKROS Pharma, BILN-2061 by Boehringer Ingelheim, CellCept (mycophenolate mofetil) by Roche, T67, a β-tubulin inhibitor, by Tularik, a therapeutic vaccine directed to E2 by Innogenetics, FK788 by Fujisawa Healthcare, Inc., IdB 1016 (Siliphos, oral silybin-phosphatdylcholine phytosome), RNA replication inhibitors (VP50406) by ViroPharma/Wyeth, therapeutic vaccine by Intercell, therapeutic vaccine by Epimmune/Genencor, IRES inhibitor by Anadys, ANA 245 and ANA 246 by Anadys, immunotherapy (Therapore) by Avant, protease inhibitor by Corvas/SChering, helicase inhibitor by Vertex, fusion inhibitor by Trimeris, T cell therapy by CellExSys, polymerase inhibitor by Biocryst, targeted RNA chemistry by PTC Therapeutics, Dication by Immtech, Int., protease inhibitor by Agouron, protease inhibitor by Chiron/Medivir, antisense therapy by AVI BioPharma, antisense therapy by Hybridon, hemopurifier by Aethlon Medical, therapeutic vaccine by Merix, protease inhibitor by Bristol-Myers Squibb/Axys, Chron-VacC, a therapeutic vaccine, by Tripep, UT 231B by United Therapeutics, protease, helicase and polymerase inhibitors by Genelabs Technologies, IRES inhibitors by Immusol, R803 by Rigel Pharmaceuticals, INFERGEN® (interferon alphacon-1) by InterMune, OMNIFERON® (natural interferon) by Viragen, ALBUFERON® by Human Genome Sciences, REBIF® (interferon beta-1a) by Ares-Serono, Omega Interferon by BioMedicine, Oral Interferon Alpha by Amarillo Biosciences, interferon gamma, interferon tau, and Interferon gamma- 1b by InterMune.

### V. Pharmaceutical Compositions

Hosts, including humans, infected with pestivirus, flavivirus, HCV or another organism replicating through a RNA-dependent RNA viral polymerase, or for treating any other disorder described herein, can be treated by administering to the patient an effective amount of the active compound or a pharmaceutically acceptable prodrug or salt thereof in the presence of a pharmaceutically acceptable carrier or dilutent. The active materials can be administered by any appropriate route, for example, orally, parenterally, intravenously, intradermally, subcutaneously, or topically, in liquid or solid form.

A preferred dose of the compound for pestivirus, flavivirus or HCV infection or any other condition described herein will be in the range from about 1 to 50 mg/kg, preferably 1 to 20 mg/kg, of body weight per day, more generally 0.1 to about 100 mg per kilogram body weight of the recipient per day. Lower doses may be preferable, for example doses of 0.5-100 mg, 0.5-50 mg, 0.5-10 mg, or 0.5-5 mg per kilogram body weight per day. Even lower doses may be useful, and thus ranges can also include from 0.1-0.5 mg per kilogram body weight per day. The effective dosage range of the pharmaceutically acceptable salts and prodrugs can be calculated based on the weight of the parent nucleoside to be delivered. If the salt or prodrug exhibits activity in itself, the effective dosage can be estimated as above using the weight of the salt or prodrug, or by other means known to those skilled in the art.

The compound is conveniently administered in a unit of any suitable dosage form, including but not limited to one containing 7 to 3000 mg, preferably 70 to 1400 mg of active ingredient per unit dosage form. An oral dosage of 50-1000 mg is usually convenient, including in one or multiple dosages of 50, 100, 200, 250, 300, 400, 500, 600, 700, 800, 900 or 1000 mgs. Lower doses may be preferable, for example from 10-100 or 1-50 mg. Also contemplated are doses of 0.1-50 mg, or 0.1-20 mg or 0.1-10.0 mg. Furthermore, lower doses may be utilized in the case of administration by a non-oral route, as, for example, by injection or inhalation.

Ideally the active ingredient should be administered to achieve peak plasma concentrations of the active compound of from about 0.2 to 70 µM, preferably about 1.0 to 10 µM. This may be achieved, for example, by the intravenous injection of a 0.1 to 5% solution of the active ingredient, optionally in saline, or administered as a bolus of the active ingredient.

The concentration of active compound in the drug composition will depend on absorption, inactivation and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the concentration ranges set forth herein are exemplary only and are not intended to limit the scope or practice of the claimed composition. The active ingredient may be administered at once, or may be divided into a number of smaller doses to be administered at varying intervals of time.

A preferred mode of administration of the active compound is oral. Oral compositions will generally include an inert diluent or an edible carrier. They may be enclosed in gelatin capsules or compressed into tablets. For the purpose of oral therapeutic administration, the active compound can be incorporated with excipients and used in the form of tablets, troches, or capsules. Pharmaceutically compatible binding agents, and/or adjuvant materials can be included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring. When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or other enteric agents.

The compound can be administered as a component of an elixir, suspension, syrup, wafer, chewing gum or the like. A syrup may contain, in addition to the active compounds, sucrose as a sweetening agent and certain preservatives, dyes and colorings and flavors.

The compound or a pharmaceutically acceptable prodrug or salts thereof can also be mixed with other active materials that do not impair the desired action, or with materials that supplement the desired action, such as antibiotics, antifungals, anti-inflammatories, or other antivirals, including other nucleoside compounds. Solutions or suspensions used for parenteral, intradermal, sucutaneous, or topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parental preparation can be enclosed in ampoules, disposable syringes or multiple dose vials made of glass or plastic.

If administered intravenously, preferred carriers are physiological saline or phosphate buffered saline (PBS).

In a preferred embodiment, the active compounds are prepared with carriers that will protect the compound against rapid elimination from the body, such as a controlled release formulation, including implants and microencapsulated delivery systems. biodegradable, biocompatible polymers can be used, such as ethylene vinyl acetate, polyanhydrides, polyglycolic acid, collagen, polyorthoesters and polylactic acid. Methods for preparation of such formulations will be apparent to those skilled in the art. The materials can also be obtained commercially from Alza Corporation.

Liposomal suspensions (including liposomes targeted to infected cells with monoclonal antibodies to viral antigens) are also preferred as pharmaceutically acceptable carriers. These may be prepared according to methods known to those skilled in the art, for example, as described in U.S. Patent No. 4,522,811. For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an inorganic solvent that is then evaporated, leaving behind a thin film of dried lipid on the surface of the container. An aqueous solution of the active compound or its monophosphate, diphosphate, and/or triphosphate derivatives is then introduced into the container. The container is then swirled by hand to free lipid material from the sides of the container and to disperse lipid aggregates, thereby forming the liposomal suspension.

### VI. Processes for the Preparation of Active Compounds

The nucleosides of the present invention can be synthesized by any means known in the art. In particular, the synthesis of the present nucleosides can be achieved by either alkylating the appropriately modified sugar, followed by glycosylation or glycosylation followed by alkylation of the nucleoside. The following non-limiting embodiments illustrate some general methodology to obtain the nucleosides of the present disclosure.

### A. General Synthesis of 1'-C-Branched Nucleosides (Reference example)

### 1'-C-Branched ribonucleosides of the following structure:

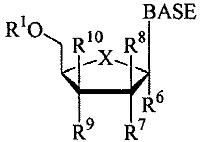

wherein BASE is a purine or pyrimidine base as defined herein;
R⁷ and R⁹ are independently hydrogen, OR², hydroxy, alkyl (including lower alkyl), azido, cyano, alkenyl, alkynyl, Br-vinyl, -C(O)O(alkyl), -C(O)O(lower alkyl), -O(acyl), -O(lower acyl), -O(alkyl), -O(lower alkyl), -O(alkenyl), chlorine, bromine, iodine, NO₂, NH₂, -NH(lower alkyl), -NH(acyl), -N(lower alkyl)₂, -N(acyl)₂;
R⁸ and R¹⁰ are independently H, alkyl (including lower alkyl), chlorine, bromine or iodine;
alternatively, R⁷ and R⁹, R⁷ and R¹⁰, R⁸ and R⁹, or R⁸ and R¹⁰ can come together to form a pi bond;
R¹ and R² are independently H; phosphate (including monophosphate, diphosphate, triphosphate, or a stabilized phosphate prodrug); acyl (including lower acyl); alkyl (including lower alkyl); sulfonate ester including alkyl or arylalkyl sulfonyl including methanesulfonyl and benzyl, wherein the phenyl group is optionally substituted with one or more substituents as described in the definition of aryl given herein; a lipid, including a phospholipid; an amino acid; a carbohydrate; a peptide; cholesterol; or other pharmaceutically acceptable leaving group which is capable of providing a compound wherein R¹ or R² is independently H or phosphate, for example when administered *in vivo*;
R⁶ is an alkyl, chloro-, bromo-, fluoro-, or iodo-alkyl (i.e. CF₃), alkenyl, or alkynyl (i.e. allyl); and
X is O, S, SO₂ or CH₂
can be prepared by one of the following general methods.

### 1) Modification from the lactone

The key starting material for this process is an appropriately substituted lactone. The lactone can be purchased or can be prepared by any known means including standard epimerization, substitution and cyclization techniques. The lactone can be optionally protected with a suitable protecting group, preferably with an acyl or silyl group, by methods well known to those skilled in the art, as taught by Greene et al. Protective Groups in Organic Synthesis, John Wiley and Sons, Second Edition, 1991**.** The protected lactone can then be coupled with a suitable coupling agent, such as an organometallic carbon nucleophile, such as a Grignard reagent, an organolithium, lithium dialkylcopper or R⁶-SiMe₃ in TBAF with the appropriate non-protic solvent at a suitable temperature, to give the 1'-alkylated sugar.

The optionally activated sugar can then be coupled to the BASE by methods well known to those skilled in the art, as taught by Townsend Chemistry of Nucleosides and Nucleotides, Plenum Press, 1994**.** For example, an acylated sugar can be coupled to a silylated base with a Lewis acid, such as tin tetrachloride, titanium tetrachloride or trimethylsilyltriflate in the appropriate solvent at a suitable temperature.

Subsequently, the nucleoside can be deprotected by methods well known to those skilled in the art, as taught by Greene et al. Protective Groups in Organic Synthesis, John Wiley and Sons, Second Edition, 1991**.**

In a particular embodiment, the 1'-C-branched ribonucleoside is desired. The synthesis of a ribonucleoside is shown in **Scheme 1.** Alternatively, deoxyribo-nucleoside is desired. To obtain these nucleosides, the formed ribonucleoside can optionally be protected by methods well known to those skilled in the art, as taught by Greene et al. Protective Groups in Organic Synthesis, John Wiley and Sons, Second Edition, 1991**,** and then the 2'-OH can be reduced with a suitable reducing agent. Optionally, the 2'-hydroxyl can be activated to facilitate reduction; i.e. via the Barton reduction.

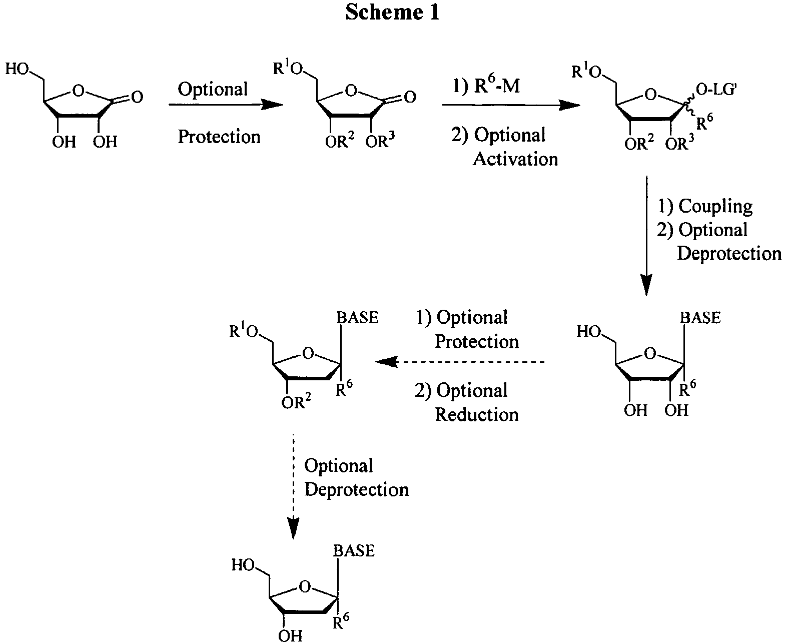

### 2. Alternative method for the preparation of 1'-C-branched nucleosides

The key starting material for this process is an appropriately substituted hexose. The hexose can be purchased or can be prepared by any known means including standard epimerization, such as alkaline treatment, substitution and coupling techniques. The hexose can be selectively protected to give the appropriate hexa-furanose, as taught by Townsend Chemistry of Nucleosides and Nucleotides, Plenum Press, 1994**.**

The 1'-hydroxyl can be optionally activated to a suitable leaving group such as an acyl group or a chloro, bromo, fluoro, iodo via acylation or halogenation, respectively. The optionally activated sugar can then be coupled to the BASE by methods well known to those skilled in the art, as taught by Townsend Chemistry of Nucleosides and Nucleotides, Plenum Press, 1994**.** For example, an acylated sugar can be coupled to a silylated base with a Lewis acid, such as tin tetrachloride, titanium tetrachloride or trimethylsilyltriflate in the appropriate solvent at a suitable temperature. Alternatively, a halo-sugar can be coupled to a silylated base with the presence of trimethylsilyltriflate.

The 1'-CH₂-OH, if protected, can be selectively deprotected by methods well known in the art. The resultant primary hydroxyl can be functionalized to yield various C-branched nucleosides. For example, the primary hydroxyl can be reduced to give the methyl, using a suitable reducing agent. Alternatively, the hydroxyl can be activated prior to reduction to facilitate the reaction; i.e. via the Barton reduction. In an alternate embodiment, the primary hydroxyl can be oxidized to the aldehyde, then coupled with a carbon nucleophile, such as a Grignard reagent, an organolithium, lithium dialkylcopper or R⁶-SiMe₃ in TBAF with the appropriate non-protic solvent at a suitable temperature.

In a particular embodiment, the 1'-C-branched ribonucleoside is desired. The synthesis of a ribonucleoside is shown in **Scheme 2.** Alternatively, deoxyribo-nucleoside is desired. To obtain these nucleosides, the formed ribonucleoside can optionally be protected by methods well known to those skilled in the art, as taught by Greene et al. Protective Groups in Organic Synthesis, John Wiley and Sons, Second Edition, 1991**,** and then the 2'-OH can be reduced with a suitable reducing agent. Optionally, the 2'-hydroxyl can be activated to facilitate reduction; i.e. via the Barton reduction.

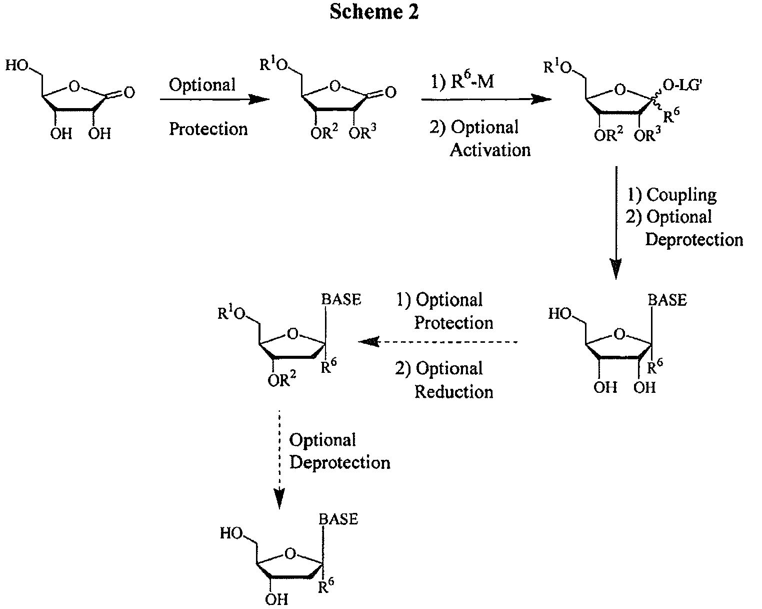

In addition, the L-enantiomers corresponding to the compounds of this reference example can be prepared following the same general methods (1 or 2), beginning with the corresponding L-sugar or nucleoside L-enantiomer as starting material.

### B. General Synthesis of 2'-C-Branched Nucleosides

### 2'-C-Branched ribonucleosides of the following structure:

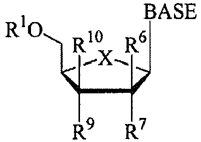

wherein BASE is a purine or pyrimidine base as defined herein;
R⁷ and R⁹ are independently hydrogen, OR², hydroxy, alkyl (including lower alkyl), azido, cyano, alkenyl, alkynyl, Br-vinyl, -C(O)O(alkyl), -C(O)O(lower alkyl), -O(acyl), -O(lower acyl), -O(alkyl), -O(lower alkyl), -O(alkenyl), chlorine, bromine, iodine, NO₂, NH₂, -NH(lower alkyl), -NH(acyl), -N(lower alkyl)₂, -N(acyl)₂;
R¹⁰ is H, alkyl (including lower alkyl), chlorine, bromine or iodine;
alternatively, R⁷ and R⁹, or R⁷ and R¹⁰ can come together to form a pi bond;
R¹ and R² are independently H; phosphate (including monophosphate, diphosphate, triphosphate, or a stabilized phosphate prodrug); acyl (including lower acyl); alkyl (including lower alkyl); sulfonate ester including alkyl or arylalkyl sulfonyl including methanesulfonyl and benzyl, wherein the phenyl group is optionally substituted with one or more substituents as described in the definition of aryl given herein; a lipid, including a phospholipid; an amino acid; a carbohydrate; a peptide; cholesterol; or other pharmaceutically acceptable leaving group which when administered *in vivo* is capable of providing a compound wherein R¹ or R² is independently H or phosphate;
R⁶ is an alkyl, chloro-, bromo-, fluoro-, iodo-alkyl (i.e. CF₃), alkenyl, or alkynyl (i.e. allyl); and
X is O, S, SO₂ or CH₂
can be prepared by one of the following general methods.

### 1. Glycosylation of the nucleobase with an appropriately modified sugar

The key starting material for this process is an appropriately substituted sugar with a 2'-OH and 2'-H, with the appropriate leaving group (LG), for example an acyl group or a chloro, bromo, fluoro or iodo. The sugar can be purchased or can be prepared by any known means including standard epimerization, substitution, oxidation and reduction techniques. The substituted sugar can then be oxidized with the appropriate oxidizing agent in a compatible solvent at a suitable temperature to yield the 2'-modified sugar. Possible oxidizing agents are Jones reagent (a mixture of chromic acid and sulfuric acid), Collins's reagent (dipyridine Cr(VI) oxide, Corey's reagent (pyridinium chlorochromate), pyridinium dichromate, acid dichromate, potassium permanganate, MnO₂, ruthenium tetroxide, phase transfer catalysts such as chromic acid or permanganate supported on a polymer, Cl₂-pyridine, H₂O₂-ammonium molybdate, NaBrO₂-CAN, NaOCl in HOAc, copper chromite, copper oxide, Raney nickel, palladium acetate, Meerwin-Pondorf-Verley reagent (aluminum *t*-butoxide with another ketone) and *N*-bromosuccinimide.

Then coupling of an organometallic carbon nucleophile, such as a Grignard reagent, an organolithium, lithium dialkylcopper or R⁶-SiMe₃ in TBAF with the ketone with the appropriate non-protic solvent at a suitable temperature, yields the 2'-alkylated sugar. The alkylated sugar can be optionally protected with a suitable protecting group, preferably with an acyl or silyl group, by methods well known to those skilled in the art, as taught by Greene et al. Protective Groups in Organic Synthesis, John Wiley and Sons, Second Edition, 1991**.**

The optionally protected sugar can then be coupled to the BASE by methods well known to those skilled in the art, as taught by Townsend Chemistry of Nucleosides and Nucleotides, Plenum Press, 1994**.** For example, an acylated sugar can be coupled to a silylated base with a Lewis acid, such as tin tetrachloride, titanium tetrachloride or trimethylsilyltriflate in the appropriate solvent at a suitable temperature. Alternatively, a halo-sugar can be coupled to a silylated base with the presence of trimethylsilyltriflate.

Subsequently, the nucleoside can be deprotected by methods well known to those skilled in the art, as taught by Greene et al. Protective Groups in Organic Synthesis, John Wiley and Sons, Second Edition, 1991**.**

In a particular embodiment, the 2'-C-branched ribonucleoside is desired. The synthesis of a ribonucleoside is shown in **Scheme 3.** Alternatively, deoxyribo-nucleoside is desired. To obtain these nucleosides, the formed ribonucleoside can optionally be protected by methods well known to those skilled in the art, as taught by Greene et al. Protective Groups in Organic Synthesis, John Wiley and Sons, Second Edition, 1991**,** and then the 2'-OH can be reduced with a suitable reducing agent. Optionally, the 2'-hydroxyl can be activated to facilitate reduction; i.e. via the Barton reduction.

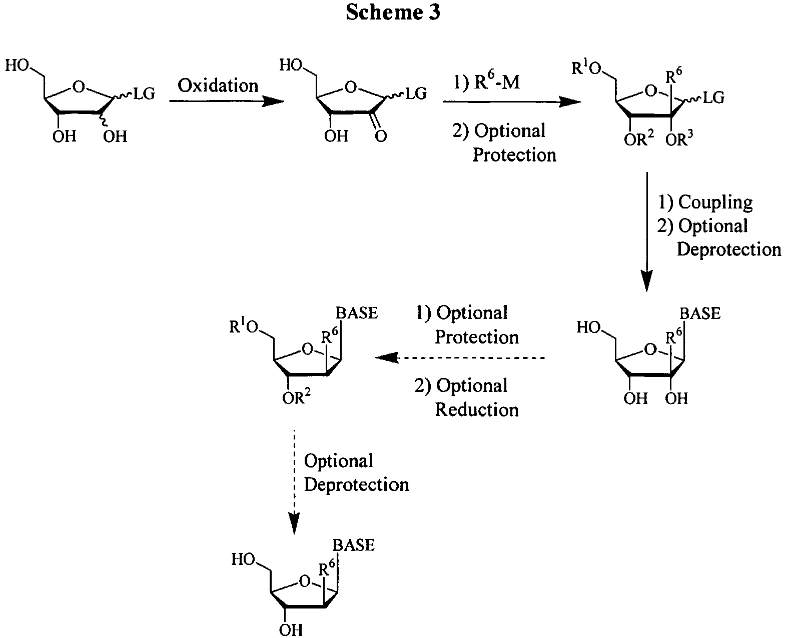

### 2. Modification of a pre-formed nucleoside

The key starting material for this process is an appropriately substituted nucleoside with a 2'-OH and 2'-H. The nucleoside can be purchased or can be prepared by any known means including standard coupling techniques. The nucleoside can be optionally protected with suitable protecting groups, preferably with acyl or silyl groups, by methods well known to those skilled in the art, as taught by Greene et al. Protective Groups in Organic Synthesis, John Wiley and Sons, Second Edition, 1991**.**

The appropriately protected nucleoside can then be oxidized with the appropriate oxidizing agent in a compatible solvent at a suitable temperature to yield the 2'-modified sugar. Possible oxidizing agents are Jones reagent (a mixture of chromic acid and sulfuric acid), Collins's reagent (dipyridine Cr(VI) oxide, Corey's reagent (pyridinium chlorochromate), pyridinium dichromate, acid dichromate, potassium permanganate, MnO₂, ruthenium tetroxide, phase transfer catalysts such as chromic acid or permanganate supported on a polymer, Cl₂-pyridine, H₂O₂-ammonium molybdate, NaBrO₂-CAN, NaOCl in HOAc, copper chromite, copper oxide, Raney nickel, palladium acetate, Meerwin-Pondorf-Verley reagent (aluminum *t*-butoxide with another ketone) and *N*-bromosuccinimide.

Subsequently, the nucleoside can be deprotected by methods well known to those skilled in the art, as taught by GreeneGreene et al. Protective Groups in Organic Synthesis, John Wiley and Sons, Second Edition, 1991**.**

In a particular embodiment, the 2'-C-branched ribonucleoside is desired. The synthesis of a ribonucleoside is shown in **Scheme 4.** Alternatively, deoxyribo-nucleoside is desired. To obtain these nucleosides, the formed ribonucleoside can optionally be protected by methods well known to those skilled in the art, as taught by Greene et al._Protective Groups in Organic Synthesis, John Wiley and Sons, Second Edition, 1991**,** and then the 2'-OH can be reduced with a suitable reducing agent. Optionally, the 2'-hydroxyl can be activated to facilitate reduction; i.e. via the Barton reduction.

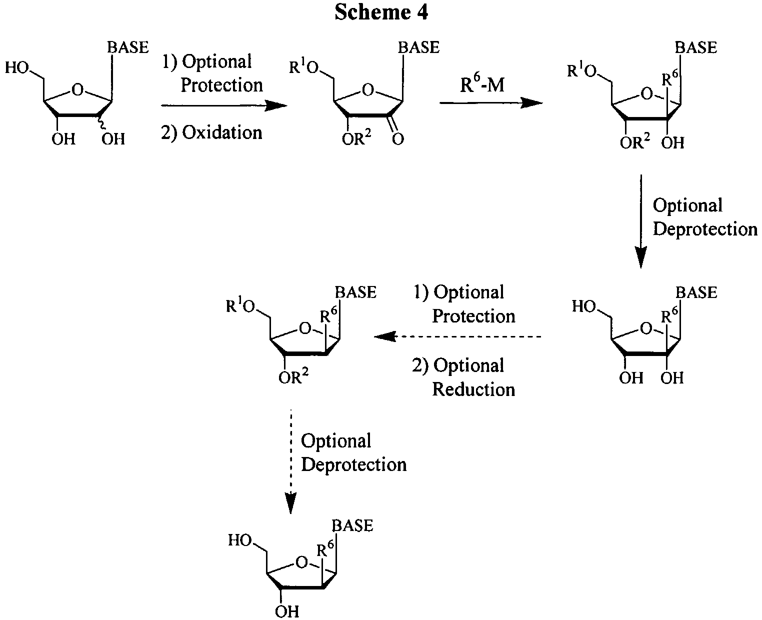

### 3. Synthesis Of β-D-2'-C-Methyl-Ribofuranosyl Cytidine-3'-O-L- Valine Ester

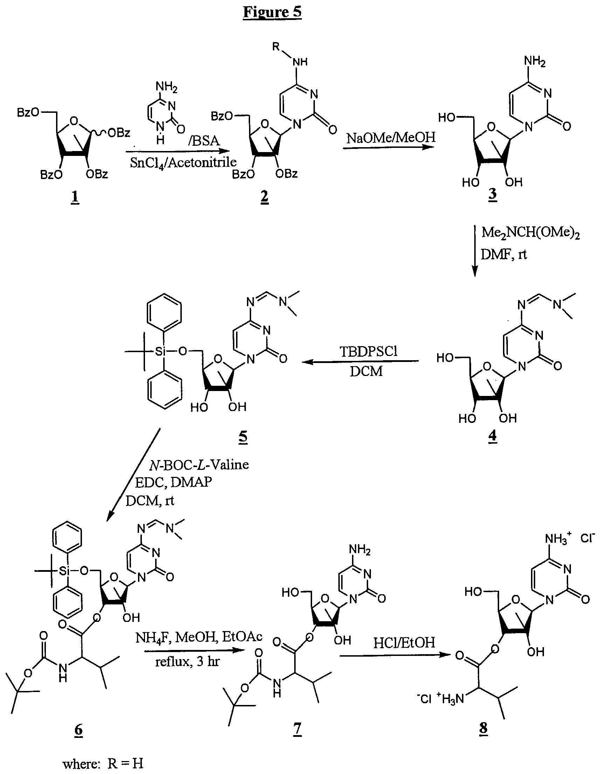

In one synthesis method, depicted in Figure 5, the synthesis comprises reacting cytosine, BSA and SnCl₄/acetonitrile with 1,2,3,5-tetra-*O*-benzoyl-2-C-methyl-β-D-ribofuranose (Figure 5, compound **1**) to form 4-amino-1-(3,4-dibenzoyloxy-5-benzoyloxymethyl-3-methyl-tetrahydro-furan-2-yl)-*1H*-pyrimidin-2-one (Figure 5, compound **2**); and reacting (Figure 5, compound **2**) with NaOMe/MeOH to provide 4-amino-1-(3,4-dihydroxy-5-hydroxymethyl-3-methyl-tetrahydro-furan-2-yl)-*1H*-pyrimidin-2-one (Figure 5, compound **3**), also known as 2-C-methyl- β-D-ribofuranose. The use of cytosine as a starting material rather than benzoyl-cytosine improves the "atom economy" of the process and simplifies purification at later steps.

The next steps in this process comprise reacting (Figure 5, compound **3**) with Me₂NCH(OMe)₂ in DMF to form (Figure 5, compound **4**), *N*-[1-(3,4-dihydroxy-5-hydroxymethyl-3-methyl-tetrahydro-furan-2-yl)-2-oxo-1, 2-dihydro-pyrimidin-4-yl]-*N,N-*dimethyl-formamidine, which is the amino-protected form of (Figure 5, compound **3**); reacting (Figure 5, compound **4**) with TBDPSCl and imidazole in DCM to provide the 5'-silyl-protected form of (Figure 5, compound **4**) as *N'*-{1-[5-(*tert*-butyl-diphenyl-silanyloxymethyl)-3,4-dihydroxy-3-methyl-tetrahydro-furan-2-yl]-2-oxo-1, 2-dihydro-pyrimidin-4-yl}-*N,N*-dimethyl-formamidine (Figure 5, compound **5**), where the use of DCM provides the advantage of having greater control over disilyl by-product formation; reacting (Figure 5, compound **5**) with *N*-Boc-*L*-valine, EDC and DMAP in DCM at room temperature to form 2-*tert*-butoxycarbonylamino-3-methyl-butyric acid 2-(*tert*-butyl-diphenyl-silanyloxy-methyl)-5-[4-(dimethylamino-methyleneamino)-2-oxo-2*H*-pyrimidin-1-yl]-4-hydroxy-4-methyl-tetrahydro-furan-3-yl ester (Figure 5, compound **6**); removing the silyl and amino-protecting groups by reacting (Figure 5, compound **6**) with NH₄F in MeOH in the presence of approximately 10 mole equivalents of ethyl acetate to prevent cleavage of the 3'-O-valinyl ester by liberated ammonia, and refluxing the mixture to provide 2-*tert-*butoxycarbonylamino-3-methyl-butyric acid 5-(4-amino-2-oxo-2*H*-pyrimidin-1-yl)-4-hydroxy-2-hydroxymethyl-4-methyl-tetrahydro-furan-3-yl ester to provide (Figure 5, compound **7**); and finally, reacting (Figure 5, compound **7**) with HCl in EtOH to provide 2-amino-3-methyl-butyric acid 5-(4-amino-2-oxo-*2H*-pyrimidin-1-yl)-4-hydroxy-2-hydroxymethyl-4-methyl-tetrahydro-furan-3-yl ester, dihydrochloride salt (Figure 5, compound **8**) as a final product.

### 6. Alternative Synthesis of β-D-2'-C-Methyl-Ribofuranosyl Cytidine-3'-O-L- Valine Ester

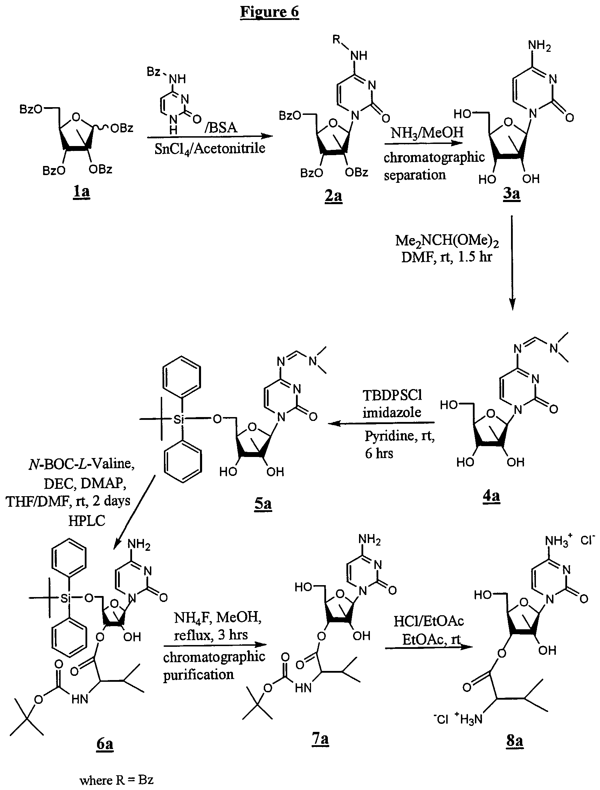

In another method to synthesize the compounds of the invention, shown in Figure 6, benzoylcytosine, BSA and SnCl₄/acetonitrile are reacted with 1,2,3,5-tetra-*O*-benzoyl-2-C-methyl-β-D-ribofuranose (Figure 6, compound **1a**) to form 4-benzoylamino-1-(3,4-dibenzoyloxy-5-benzoyloxymethyl-3-methyl-tetrahydro-furan-2-yl)-*1H*-pyrimidin-2-one (Figure 6, compound **2a**); reacting (Figure 6, compound **2a**) with NH₃ in methanol and chromatographically separating the product, 4-amino-1-(3,4-dihydroxy-5-hydroxymethyl-3-methyl-tetrahydro-furan-2-yl)-*1H*-pyrimidin-2-one (Figure 6, compound **3a**), also known as β-D-2'-C-methyl-cytidine; reacting (Figure 6, compound **3a**) with Me₂NCH(OMe)₂ in DMF at room temperature for 1.5 hours to form *N*-[1-(3,4-dihydroxy-5-hydroxymethyl-3-methyl-tetrahydro-furan-2-yl)-2-oxo-1,2-dihydro-pyrimidin-4-yl]-*N,N*-dimethyl-formamidine (Figure 6, compound **4a**); reacting (Figure 6, compound **4a**) with TBDPSCl and pyridine at room temperature for 6 hours to provide *N'*-{1-[5-(*tert*-butyl-diphenyl-silanyloxymethyl)-3,4-dihydroxy-3-methyl-tetrahydro-furan-2-yl]-2-oxo-1,2-dihydro-pyrimidin-4-yl}-*N,N-*dimethyl-formamidine (Figure 6, compound **5a**); reacting (Figure 6, compound **5a**) with N-Boc-L-valine, DEC and DMAP in THF/DMF at room temperature for 2 days and subjecting the product formed from this reaction to HPLC in order to provide 2-*tert-*butoxycarbonylamino-3-methyl-butyric acid 2-(*tert*-butyl-diphenyl-silanyloxy-methyl)-5-[4-(dimethylaminomethyleneamino)-2-oxo-*2H*-pyrimidin-1-yl]-4-hydroxy-4-methyl-tetrahydrofuran-3-yl ester (Figure 6, compound **6a**); refluxing (Figure 6, compound **6a**) with NH₄F in MeOH for about 3 hours to remove the silyl and amino-protecting groups, and subjecting the product to chromatographic purification to provide 2-*tert*-butoxycarbonylamino-3-methyl-butyric acid 5-(4-amino-2-oxo-*2H*-pyrimidin-1-yl)-4-hydroxy-2-hydroxymethyl-4-methyl-tetrahydro-furan-3-yl ester (Figure 6, compound **7a**); and finally reacting (Figure 6, compound **7a**) with HCl in EtOAc at room temperature to provide 2-amino-3-methyl-butyric acid 5-(4-amino-2-oxo-*2H*-pyrimidin-1-yl)-4-hydroxy-2-hydroxymethyl-4-methyl-tetrahydro-furan-3-yl ester, dihydrochloride salt (Figure 6, compound **8a**) as a final product.

The synthesis of 2'-C-methyl-cytidine-3'-O-L-valine ester (val-mcyd) is depicted in **scheme 5** and **scheme 6,** and described below.

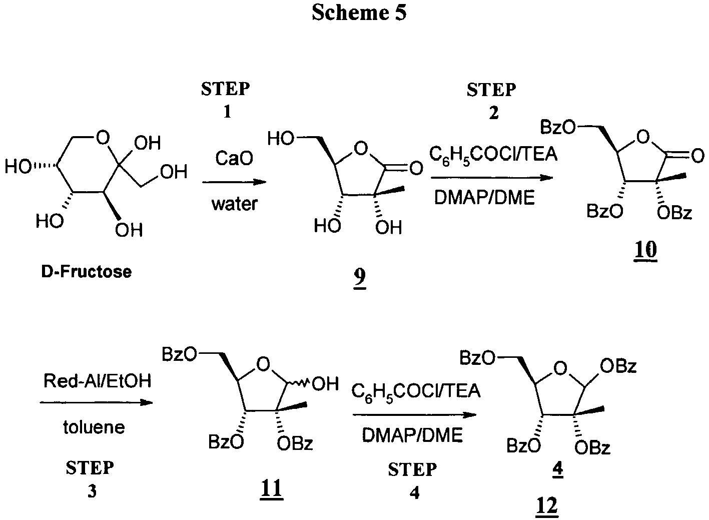

### Step 1: Synthesis of scheme 5, compound 9: 2-C-Methyl-D-ribonic-γ-lactone

De-ionized water (100 mL) was stirred in a 250 mL 3-necked round bottom flask, equipped with an overhead stirrer, a stirring shaft, a digital temperature read-out device and an argon line. Argon was bubbled into water for thirty minutes and D-fructose (20.0 g, 0.111 mole) was added and the solution became clear in a few minutes. Calcium oxide (12.5 g, 0.223 mole) was added in portions over a period of five minutes and the mixture was vigorously stirred. An exotherm was observed and reaction temperature reached 39.6°C after 10 minutes from the start of the calcium oxide addition. After about fifteen minutes, the reaction mixture developed a yellow color that deepened with time. After three hours, an aliquot was withdrawn for TLC analysis. The aliquot was acidified to pH 2 using saturated aqueous solution of oxalic acid. The resulting white suspension was evaporated under reduced pressure to remove the water. Toluene (2 mL) was added to the residue and the mixture was evaporated under reduced pressure (at 45-50°C) to remove any trace of water. The residual solid was re-constituted in 2 mL of 1:1 tetrahydrofuran : methanol mixture. After thorough mixing, the suspension was allowed to settle and the supernatant clear solution was spotted for TLC (silica plate was developed in 2% methanol in ethyl acetate and stained in 1% alkaline potassium permanganate dip. The plate was then heated, using a heat gun, until the appearance of yellowish spots on the pink background). The desired lactone typically appears at an R_{f} value of 0.33 under the above conditions. More polar by-products and unreacted material are detected in the R_{f} value range of 0.0 to 0.2.

Although product formation was observed after 3 hours, the reaction was allowed to continue for 22 hours during which time the reaction mixture was stirred at 25 °C. At the end of this period, pH of the mixture was 13.06. Carbon dioxide gas was bubbled into the reaction mixture for about 2.5 hours (pH was 7.25). The formed calcium carbonate solid was removed by vacuum filtration, filter cake washed with 50 mL of de-ionized water. The aqueous layers were combined and treated with oxalic acid (5.0 g, 0.056 mole) and the mixture was vigorously stirred at 25°C for 30 minutes (The initial dark color largely disappeared and the mixture turned into a milky white slurry). The pH of the mixture at this stage is typically 2-3. The slurry mixture was stirred at 45-50°C overnight. The mixture was then evaporated under reduced pressure and at 45-50°C to remove 75 mL of water. Sodium chloride (30 g) and tetrahydrofuran (100 mL) were added to the aqueous slurry (about 75 mL) and the mixture was vigorously stirred at 25°C for 30 minutes. The layers were separated and the aqueous layer was stirred for 10 minutes with 75 mL of fresh tetrahydrofuran. This process was repeated for three times and the tetrahydrofuran solutions were combined and stirred with 10 g of anhydrous magnesium sulfate for 30 minutes. The mixture was filtered and the magnesium sulfate filter cake was washed with 60 mL of tetrahydrofuran. The filtrate was evaporated under reduced pressure and at 40°C to give 10.86 g of crude product as a dark orange semisolid. (For scale up runs tetrahydrofuran will be replaced with acetone instead of evaporation of crude product to dryness). Crude product was stirred with acetone (20 mL) at 20°C for 3 hours. Product was collected by vacuum filtration and the filter cake washed with 12 mL of acetone to give the desired product 9 as white crystalline solid. Product was dried in vacuum to give 2.45 g (13.6% yield). Melting point of compound 9: 158-162°C (literature melting point: 160-161 °C). ¹H NMR (DMSO-*d₆*) δ ppm 5.69 (s, 1H, exch. With D₂O), 5.41 (d, 1H, exch. With D₂O), 5.00 (t, 1H, exch. With D₂O), 4.15 (m, 1H), 3.73 (m, 2H), 3.52 (m, 1H), 1.22 (s, 3H). ¹³C NMR (DMSO-*d₆*) δ ppm 176.44, 82.95, 72.17, 72.02, 59.63, 20.95. (C₆H₁₀O₅: calcd C, 44.45; H, 6.22. Found: C, 44.34; H, 6.30).

### Step 2: Synthesis of scheme 5, compound 10: 2,3,5-Tri-O-benzoyl-2-C-methyl-D-ribonic-γ-lactone

A mixture of lactone 1 (3.0 g, 18.50 mmol.), 4-dimethylaminopyridine (0.45 g, 3.72 mmol.) and triethylamine (25.27 g, 249.72 mmol.) in 1,2-dimethoxy ethane(50 mL) was stirred at 25°C under argon atmosphere for thirty minutes. This white suspension was cooled to 5°C and benzoyl chloride (11.7 g, 83.23 mmol.) was added over a period of fifteen minutes. The mixture was stirred at 25°C for two hours. TLC analysis (silica, 2% methanol in ethyl acetate) indicated complete consumption of starting material. Ice cold water (100 g) was added to the reaction mixture and stirring was continued for thirty minutes. The formed white solids were collected by vacuum filtration and filter cake washed with cold water (50 mL). This crude product was stirred with tert-butyl methyl ether (60 mL) at 20 °C for thirty minutes, then filtered, filter cake washed with tert-butyl methyl ether (25 mL) and dried in vacuum to give 7.33 g (83.4% yield) of compound 10 as a white solid in 97.74% purity (HPLC/AUC). Melting point of compound 10: 137-140 °C (literature melting point: 141-142 °C). ¹H NMR (CDCl₃) δ ppm 8.04 (d, 2H), 7.92 (d, 2H), 7.73 (d, 2H), 7.59 (t, 1H), 7.45 (m, 4H), 7.32 (t, 2H), 7.17 (t, 2H), 5.51 (d, 1H), 5.17 (m, 1H), 4.82-4.66 (d of an AB quartet, 2H) 1.95, (s, 3H). ¹³C NMR (CDCl₃) δ ppm 172.87, 166.17, 166.08, 165.58, 134.06, 133.91, 133.72, 130.09, 129.85, 129.80, 129.37, 128.78, 128.60, 128.49, 127.96, 127.89, 79.67, 75.49, 72.60, 63.29, 23.80. TOF MS ES+ (M+1: 475).

### Step 3: Synthesis of scheme 5, compound 11: 2,3,5-Tri-O-benzoyl-2-C-methyl-β-D-ribofuranose:

A solution of Red-Al (65wt.% in toluene, 2.0 mL, 6.56 mmol.) in anhydrous toluene (2.0 mL) was stirred at 0°C under argon atmosphere. A solution of anhydrous ethanol (0.38 mL, 6.56 mmol.) in anhydrous toluene (1.6 mL) was added to the toluene solution over a period of five minutes. The resulting mixture was stirred at 0 °C for fifteen minutes and 2 mL (2.18 mmol.) of this Red-Al/ethanol reagent was added to a cold (-5°C) solution of 2,3,5-triO-benzoyl-2-C-methyl-D-ribonolactone (475 mg, 1.0 mmol.) in anhydrous toluene (10 mL) over a period of 10 minutes. The reaction mixture was stirred at -5 °C for forty minutes. TLC analysis (silica plates, 35% ethyl acetate in heptane) indicated complete consumption of starting material. HPLC analysis indicated only 0.1% of starting material remaining. The reaction was quenched with acetone (0.2 mL), water (15 mL) and 1 N HCl (15 mL) at 0 °C and allowed to warm to room temperature. 1 N HCl (5 mL) was added to dissolve the inorganic salts (pH : 2-3). The mixture was extracted with ethyl acetate (3 x 25 mL) and the organic solution washed with brine (25 mL), dried (anhydrous sodium sulfate, 10 g) and solvent removed under reduced pressure and at temperature of 40°C to give the desired product 11 in quantitative yield (480 mg). This material was used as is for the subsequent step.

### Step 4: Synthesis of scheme 5, compound 12: 1,2,3,5-tetra-O-benzoyl-2-C-methyl-β-D-ribofuranose:

Benzoyl chloride (283 mg, 2.0 mmol.) was added, over a period of five minutes, to a cold solution (5°C) of compound 11 (480 mg, 1.0 mmol.), 4-dimethylaminopyridine (12.3 mg, 0.1 mmol.) and triethylamine (506 mg, 5.0 mmol.) in anhydrous tetrahydrofuran (5 mL). The reaction mixture was stirred at room temperature and under argon atmosphere overnight. HPLC analysis indicated 0.25% of un-reacted starting material. The reaction was quenched by adding ice-cold water (10 g) and saturated aqueous solution of sodium bicarbonate. Tetrahydrofuran was removed under reduced pressure and the mixture was extracted with ethyl acetate (50 mL). The organic solution was washed with water (25 mL), brine (25 mL), dried (anhydrous sodium sulfate, 12 g) and solvent removed under reduced pressure to give 650 mg of thick oily product. This crude product was stirred with 5 mL of tert-butyl methyl ether for 5 minutes and heptane (5 mL) and water (0.1 mL) were added and stirring was continued for an additional period of two hours at 20°C. Solids were collected by vacuum filtration and filter caked washed with 1:1 heptane:tert-butyl methyl ether solution (6 mL) and tert-butyl methyl ether (2 mL). Drying the solid in vacuum gave 300 mg (52%) of desired product 12 (98.43% pure by HPLC/AUC) as a white solid that melted at 154-156.3°C (literature melting point: 155-156°C). ¹H NMR (CDCl₃) δ ppm 8.13 (m, 4H), 8.07 (d, 2H), 7.89 (d, 2H), 7.63 (m, 3H), 7.48 (m, 6H), 7.15 (m, 3H), 7.06 (s, 1H), 5.86 (dd, 1H), 4.79 (m, 1H), 4.70-4.52 (d of an AB quartet, 2H), 1.95, (s, 3H). ¹³C NMR (CDCl₃) δ ppm 166.31, 165.83, 165.01, 164.77, 134.01, 133.86, 133.70, 133.17, 130.44, 130.13, 129.97, 129.81, 129.59, 129.39, 129.07, 128.84, 128.76, 128.37, 98.01, 86.87, 78.77, 76.35, 64.05, 17.07. (C₃₄H₂₈O₉: calcd C, 70.34; H, 4.86. Found: C, 70.20; H, 4.95).

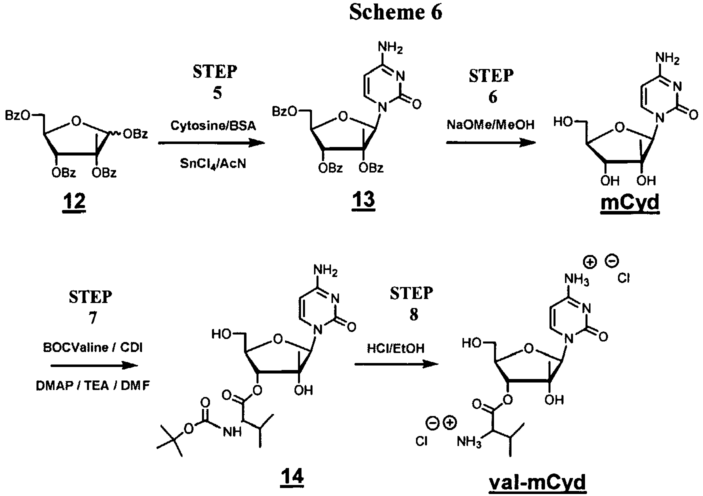

### Step 5: Synthesis of scheme 6, compound 13: 4-Amino-1-(3,4-dibenzoyloxy-5-benzyloxymethyl-3-methyl-tetrahydro-furan-2-yl)-1H-pyrimidine-2-one

Cytosine (89 g, 0.80 mol) was suspended in acetonitrile (900 ml) in a 12 L round bottomed flask equipped with a reflux condenser, overhead stirrer and an argon inlet adapter. The suspension was stirred at 20°C under argon atmosphere and N,O-bis(trimethylsilyl)acetamide (537 ml, 2.2 mol) was added in one portion. The resulting solution was heated to 80° C and stirred for an additional hour at the same temperature. 1,2,3,5-tetra-O-benzoyl-2-C-methyl-□-D-ribofuranose (425.0 g, 0.73 mol) was suspended in acetonitrile (4000 ml) and added to the reaction mixture. The reaction mixture became clear after a few minutes and the temperature dropped to ca. 50° C. Tin(IV) chloride (154 ml, 1.31 mol) was added over a period of 15 minutes and stirring was continued at 80°C. After one hour, an aliquot of reaction mixture was quenched by adding aqueous sodium bicarbonate solution and extracting the aqueous layer with ethyl acetate. The ethyl acetate layer was examined by TLC (silica gel, 20% ethyl acetate in heptane, R_{f} for sugar derivative: 0.40). TLC analysis indicated the complete consumption of the sugar derivative. Desired product was detected by TLC using 10% methanol in dichloromethane (R_{f} : 0.37). The reaction was also monitored by HPLC (Method # 2). Reaction mixture was cooled to 20°C and quenched by adding saturated aqueous sodium bicarbonate solution (3000 ml) over a period of 30 minutes (observed an exotherm when added the first few drops of the sodium bicarbonate solution). Solid sodium bicarbonate (1350 g) was added in portions to avoid foaming. The mixture was checked to make sure that its pH is ≥ 7. Agitation was stopped and layers were allowed to separate for 20 minutes. The aqueous layer was drained and stirred with ethyl acetate (1500 ml) and the mixture was allowed to separate (30 minutes). The organic layer was isolated and combined with the acetonitrile solution. The organic solution was washed with brine (500 ml) and then solvent stripped to a volume of ca. 750 ml. Product can be used as is in the subsequent reaction. It may also be further stripped to white foamy solid, in quantitative yield. Structure of compound 13 was confirmed by ¹H NMR analysis.

### Step 6: Synthesis of scheme 6, compound mCyd: 4-Amino-1-(3,4-dihydroxy-5-hydroxymethyl-3-methyl-tetrahydro-furan-2-yl)-1H-pyrimidine-2-one

Sodium methoxide (13.8 g, 0.26 mol) was added to a solution of compound 10 (416 g, 0.73mol) in methanol (2000 ml). The reaction mixture was stirred at room temperature and monitored by TLC (silica gel, 10% methanol in dichloromethane, R_{f} of compound 9: 0.53) and (silica gel, 30% methanol in dichloromethane, R_{f} of compound 11: 0.21). Product started to precipitate after 30 minutes and TLC indicated reaction completion after two hours. The reaction was also monitored by HPLC (Method # 2). Methanol was removed under reduced pressure to a volume of ca. 500 ml chased with ethanol (2 x 500 ml) to a volume of ca. 500 ml. The residual thick slurry was diluted with 750 ml of ethanol and the mixture was stirred at 20 ° C for one hour. Product was collected by filtration, filter cake washed with ethanol (100 ml) and tert-butyl-methyl ether (100 ml) and dried to give 168 g (90% yield for the two steps) of product 11 in purity of > 97% (HPLC/AUC). Product was also analyzed by ¹H and ¹³C NMR.

### Step 7: Synthesis of scheme 6, compound 14: 2-Tert-butoxycarbonylamino-3-methyl-butyric acid 5-(4-amino-2-oxo-2H-pyrimidin-1-yl)-4-hydroxy-2-hydroxymethyl-4-methyl-tetrahydro-furan-3-yl ester

A solution of N-(tert-butoxycarbonyl)-L-valine (46.50 g, 214 mmol.), carbonyldiimidazole (34.70 g, 214 mmol.), and anhydrous tetrahydrofuran (1000 mL) in a 2 L round bottom flask, was stirred at 25°C under argon for 1.5 hours and then at 40-50°C for 20 minutes. In a separate 5 L 5-necked round bottom flask, equipped with an overhead stirrer, cooling tower, temperature probe, addition funnel, and an argon line was added 4-amino-1-(3,4-dihydroxy-5-hydroxymethyl-3-methyl-tetrahydro-furan-2-yl)-*1H*-pyrimidine-2-one (50.0 g, 195 mmol.) and anhydrous N,N-dimethylformamide (1000 mL). This mixture was heated at 100°C for 20 minutes until all of the pyrimidine-2-one derivative compound went into solution, and then triethyl amine (500 mL) and 4-dimethylaminopyridine (2.38 g, 19 mmol) were added to the solution. The mixture was next heated at 97° C for 20 minutes and the tetrahydrofuran solution was added slowly through an addition funnel over a period of 2 hours, maintaining the temperature no lower than 82°C. The reaction mixture was heated at 82 °C for 1 hour and monitored by HPLC (product = 68%, SM = 11%, and impurity at about 12 min = 17%, excluding dimethylaminopyridine). The reaction mixture was cooled to room temperature and then triethylamine and tetrahydrofuran were removed under vacuum at 30°C. The solution was then neutralized with acetic acid to a pH of 7.69. N,N-dimethylformamidine was removed under vacuum at 35°C and chased with ethyl acetate (2 x 200 mL). The crude product was stirred with ethyl acetate (500 mL) and water (300 mL). The two layers were separated and the aqueous layer was extracted with ethyl acetate (500 mL). The combined organic layers were washed with an aqueous saturated brine solution (500 mL). Next the organic layer was extracted with an aqueous solution of malonic acid (4 x 400 mL, 10wt.%). The organic layer was checked by TLC (silica, 20% methanol in dichloromethane) to make sure that all the desired product was removed from the organic layer. The acidic aqueous extracts were combined and cooled in an ice bath and neutralized with triethylamine to a pH of 7.40 so that the solids fell out of solution. Ethyl acetate then was added to the aqueous layer. The white solids were collected by vacuum filtration. Drying the obtained solids in vacuum gave 81.08 g of 99.01 pure (HPLC) first crop.

### Step 8: Synthesis of scheme 6, val-mCyd - 2-Amino-3-methyl-butyric acid 5-(4-amino-2-oxo-2H-pyrimidine-1-yl)-4-hydroxy-2 hydroxy-methyl-4-methyl-tetrahydro-furan-3-yl ester (dihydrochloride salt)

A solution of compound 14 (21.0 g, 0.046 mol) in ethanol (168 ml) was stirred in a round bottomed flask equipped with an overhead stirrer, temperature probe, argon line and hydrogen chloride gas bubbler. Hydrogen chloride gas (22 g) was bubbled into the clear solution over a period of one hour. The reaction temperature was kept below 30°C using an ice-water bath. Solid formation started after a few minutes of introducing the hydrogen chloride gas. After 4 hours, HPLC (method # 3) showed only 0.8% of starting material. Solids were collected by filtration and filter cake washed with ethanol (20 ml) and di-ethyl ether (100 ml). After drying product under vacuum for 16 hours, 19.06 g (96.5%) of val-mCyd was obtained in 97.26% purity (HPLC, method # 3); m.p. 210°C (brown), 248-250°C (melted); ¹H NMR (DMSO-*d*₆) δ ppm 10.0 (s, 1H, 1/2N*H₂*, D₂O exchangeable), 8.9-8.6 (2 br s, 4H, 1/2N*H₂*, N*H*₃, D₂O exchangeable), 8.42 (d, 1H, *H*-6, J ₅₋₆= 7.9 Hz), 6.24 (d, 1H, *H*-5, J₅₋₆= 7.9 Hz), 5.84 (s, 1H, *H*-1'), 5.12 (d, 1H, *H*-3', J_{3'-4'}= 8.8 Hz), 4.22 (d, 1H, *H*-4, J_{3'-4'}= 8.7 Hz), 4.0-3.9 (m, 1H, CH), 3.8-3.5 (m, 2H, *H*-5', *H*-5"), 2.3-2.1 (m, 1H, C*H*), 1.16 (s, 3H, C*H*₃), 1.0 (m, 6H, (C*H*₃)₂CH); FAB>0 (GT) 713 (2M+H)⁺, 449 (M+G+H)⁺, 357 (M+H)⁺, 246 (S)⁺, 112 (B+2H)⁺; FAB<0 (GT) 747 (2M+Cl)⁻, 483 (M+G+Cl)', 391 (M+Cl)⁻, 355 (M-H)⁻, 116 (Val)⁻, 110 (B)-, 35 (Cl).

Two different HPLC methods were used to analyze the above compounds. Both methods use the following reverse phase column. In method 1, the column was run at a flow rate of 1.00 ml / min of an acetonitrile / water linear gradient for a 20 minute run time. Five-minute equilibration was allowed between runs. The measurements were at 254 nm.

**Table A: Retention time of key intermediates:**

| **Scheme 5, Compound** | **Retention Time** |
|---|---|
| Compund 10 | 10.2 min |
| Compund 11 | 9.4 min |
| Compund 12 | 12.9 min |

In the second method, identification was determined at 272 nm. A Waters Novapak C18, 3.9 x 150 mm ID, 4µm particle size, 60A pore size or equivalent can be used. The chromatographic conditions are as follows: injection volume = 10µl, column temperature = 25°C, flow rate = 1.00 ml / min, ultraviolet detector at 272 nm, run time is 35 minutes. The system suitability requirement for the percent relative standard deviation for the reference standard is not more than 1.0%.

**Table B: Purity and impurities are determined at 272 nm**

| **Time (minutes)** | **Solvent A - 20nM triethylammonium acetate buffer** | **Solvent B-Acetonitrile, HPLC grade.** |
|---|---|---|
| 0.00 | 100.0 | 0.0 |
| 10.00 | 85.0 | 15.0 |
| 25.00 | 5.0 | 95.0 |
| 35.0 | 5.0 | 95.0 |

**Table C: Retention times of key intermediates and final drug substance**

| **Scheme 6, Compound** | **Retention Time (minutes)** |
|---|---|
| Compound mCyd | 2.7-2.8 |
| Compund 14 | 15.5 |
| val-mCyd | 9.1 |

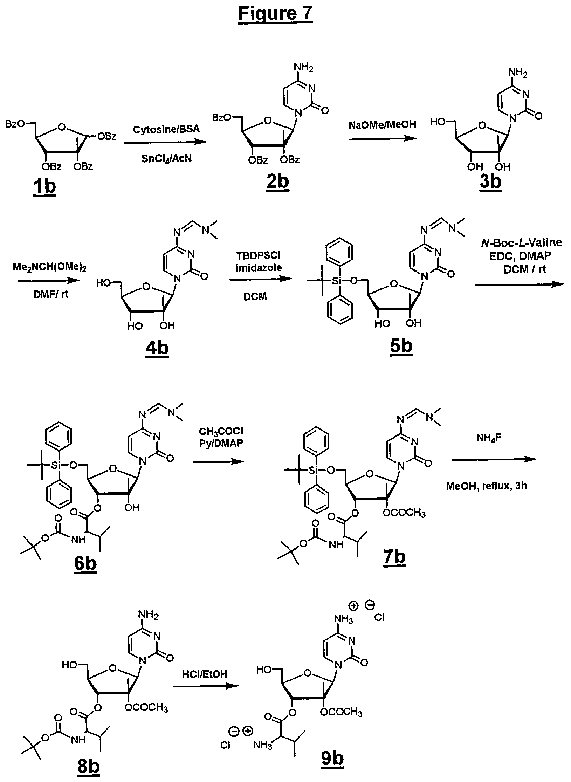

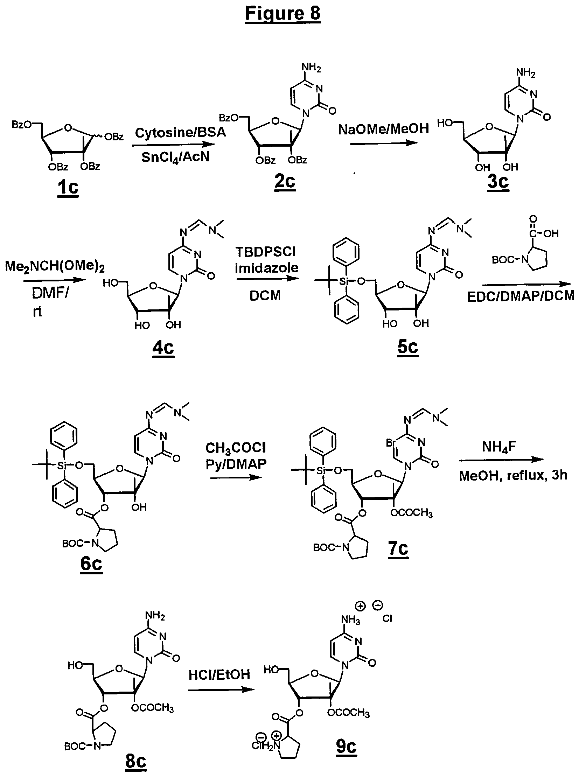

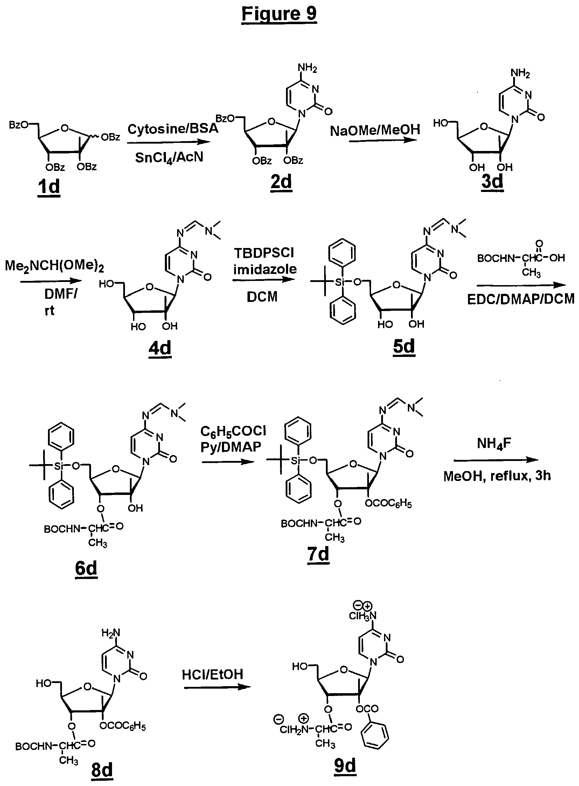

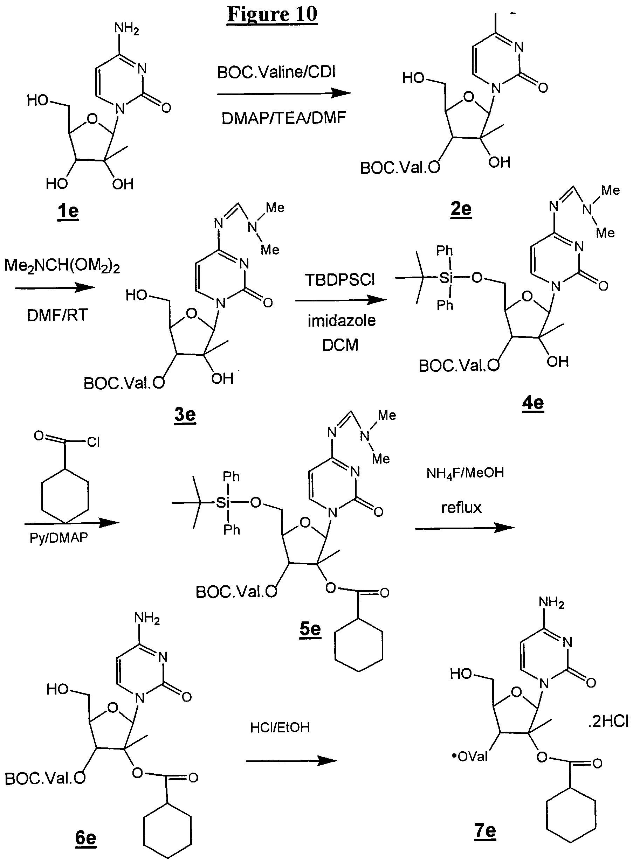

A process of synthesizing a β-D-2'-C-methyl-2'-acetyl-ribofuransyl-cytidine-3'-O-L-valine ester is detailed in Figure 7. A process of synthesizing a β-D-2'-C-methyl-2'-acetyl-ribofuransyl-cytidine-3'-O-L-proline ester is detailed in Figure 8. A process for synthesizing a β-D-2'-C-methyl-2'-acetyl-ribofuransyl-cytidine-3'-O-L-alanine ester is depicted in Figure 9. A process of synthesizing a β-D-2'-C-methyl-2'-(cyclohexane carboxylate)-ribofuransyl-cytidine-3'-O-L-valine ester is depicted in Figure 10. These processes can be accomplished using techniques similar to those described above.

### C. General Synthesis of 3'-C-Branched Nucleosides (Reference Example)

### 3'-C-Branched ribonucleosides of the following structure:

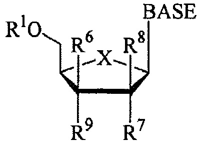

wherein BASE is a purine or pyrimidine base as defined herein;
R⁷ and R⁹ are independently hydrogen, OR², hydroxy, alkyl (including lower alkyl), azido, cyano, alkenyl, alkynyl, Br-vinyl, -C(O)O(alkyl), -C(O)O(lower alkyl), -O(acyl), -O(lower acyl), -O(alkyl), -O(lower alkyl), -O(alkenyl), chlorine, bromine, iodine, NO₂, NH₂, -NH(lower alkyl), -NH(acyl), -N(lower alkyl)₂, -N(acyl)₂;
R⁸ is H, alkyl (including lower alkyl), chlorine, bromine or iodine;
alternatively, R⁷ and R⁹, or R⁸ and R⁹ can come together to form a pi bond;
R¹ and R² are independently H; phosphate (including monophosphate, diphosphate, triphosphate, or a stabilized phosphate prodrug); acyl (including lower acyl); alkyl (including lower alkyl); sulfonate ester including alkyl or arylalkyl sulfonyl including methanesulfonyl and benzyl, wherein the phenyl group is optionally substituted with one or more substituents as described in the definition of aryl given herein; a lipid, including a phospholipid; an amino acid; a carbohydrate; a peptide; cholesterol; or other pharmaceutically acceptable leaving group which when administered *in vivo* is capable of providing a compound wherein R¹ or R² is independently H or phosphate;
R⁶ is an alkyl, chloro-, fluoro-, bromo-, iodo-alkyl (i.e. CF₃), alkenyl, or alkynyl (i.e. allyl); and
X is O, S, SO₂ or CH₂
can be prepared by one of the following general methods.

### 1. Glycosylation of the nucleobase with an appropriately modified sugar

The key starting material for this process is an appropriately substituted sugar with a 3'-OH and 3'-H, with the appropriate leaving group (LG), for example an acyl group or a chloro, bromo, fluoro, iodo. The sugar can be purchased or can be prepared by any known means including standard epimerization, substitution, oxidation and reduction techniques. The substituted sugar can then be oxidized with the appropriate oxidizing agent in a compatible solvent at a suitable temperature to yield the 3'-modified sugar. Possible oxidizing agents are Jones reagent (a mixture of chromic acid and sulfuric acid), Collins's reagent (dipyridine Cr(VI) oxide, Corey's reagent (pyridinium chlorochromate), pyridinium dichromate, acid dichromate, potassium permanganate, MnO₂, ruthenium tetroxide, phase transfer catalysts such as chromic acid or permanganate supported on a polymer, Cl₂-pyridine, H₂O₂-ammonium molybdate, NaBrO₂-CAN, NaOCl in HOAc, copper chromite, copper oxide, Raney nickel, palladium acetate, Meerwin-Pondorf-Verley reagent (aluminum *t*-butoxide with another ketone) and *N*-bromosuccinimide.

Then coupling of an organometallic carbon nucleophile, such as a Grignard reagent, an organolithium, lithium dialkylcopper or R⁶-SiMe₃ in TBAF with the ketone with the appropriate non-protic solvent at a suitable temperature, yields the 3'-C-branched sugar. The 3'-C-branched sugar can be optionally protected with a suitable protecting group, preferably with an acyl or silyl group, by methods well known to those skilled in the art, as taught by Greene et al. Protective Groups in Organic Synthesis, John Wiley and Sons, Second Edition, 1991.

The optionally protected sugar can then be coupled to the BASE by methods well known to those skilled in the art, as taught by Townsend Chemistry of Nucleosides and Nucleotides, Plenum Press, 1994**.** For example, an acylated sugar can be coupled to a silylated base with a Lewis acid, such as tin tetrachloride, titanium tetrachloride or trimethylsilyltriflate in the appropriate solvent at a suitable temperature. Alternatively, a halo-sugar can be coupled to a silylated base with the presence of trimethylsilyltriflate.

Subsequently, the nucleoside can be deprotected by methods well known to those skilled in the art, as taught by Greene et al. Protective Groups in Organic Synthesis, John Wiley and Sons, Second Edition, 1991**.**

In a particular embodiment, the 3'-C-branched ribonucleoside is desired. The synthesis of a ribonucleoside is shown in **Scheme 7.** Alternatively, deoxyribo-nucleoside is desired. To obtain these nucleosides, the formed ribonucleoside can optionally be protected by methods well known to those skilled in the art, as taught by Greene et al. Protective Groups in Organic Synthesis, John Wiley and Sons, Second Edition, 1991**,** and then the 2'-OH can be reduced with a suitable reducing agent. Optionally, the 2'-hydroxyl can be activated to facilitate reduction; i.e. via the Barton reduction.

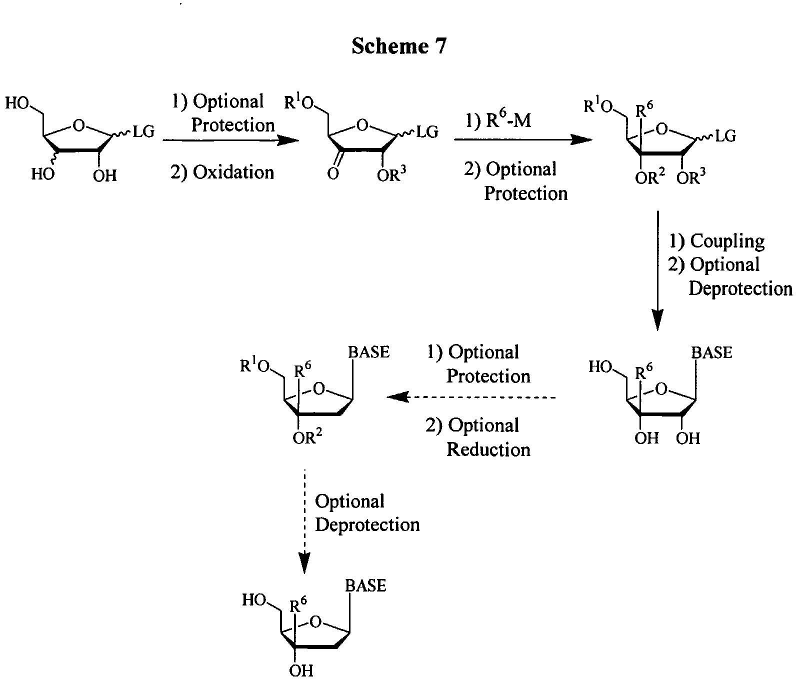

### 2. Modification of a pre-formed nucleoside

The key starting material for this process is an appropriately substituted nucleoside with a 3'-OH and 3'-H. The nucleoside can be purchased or can be prepared by any known means including standard coupling techniques. The nucleoside can be optionally protected with suitable protecting groups, preferably with acyl or silyl groups, by methods well known to those skilled in the art, as taught by Greene et al. Protective Groups in Organic Synthesis, John Wiley and Sons, Second Edition, 1991**.**

The appropriately protected nucleoside can then be oxidized with the appropriate oxidizing agent in a compatible solvent at a suitable temperature to yield the 2'-modified sugar. Possible oxidizing agents are Jones reagent (a mixture of chromic acid and sulfuric acid), Collins's reagent (dipyridine Cr(VI) oxide, Corey's reagent (pyridinium chlorochromate), pyridinium dichromate, acid dichromate, potassium permanganate, MnO₂, ruthenium tetroxide, phase transfer catalysts such as chromic acid or permanganate supported on a polymer, Cl₂-pyridine, H₂O₂-ammonium molybdate, NaBrO₂-CAN, NaOCl in HOAc, copper chromite, copper oxide, Raney nickel, palladium acetate, Meerwin-Pondorf-Verley reagent (aluminum *t*-butoxide with another ketone) and *N*-bromosuccinimide.

Subsequently, the nucleoside can be deprotected by methods well known to those skilled in the art, as taught by GreeneGreene et al. Protective Groups in Organic Synthesis, John Wiley and Sons, Second Edition, 1991**.**

In a particular embodiment, the 3'-C-branched ribonucleoside is desired. The synthesis of a ribonucleoside is shown in **Scheme 8.** Alternatively, deoxyribo-nucleoside is desired. To obtain these nucleosides, the formed ribonucleoside can optionally be protected by methods well known to those skilled in the art, as taught by Greene et al. Protective Groups in Organic Synthesis, John Wiley and Sons, Second Edition, 1991, and then the 2'-OH can be reduced with a suitable reducing agent. Optionally, the 2'-hydroxyl can be activated to facilitate reduction; i.e. via the Barton reduction.

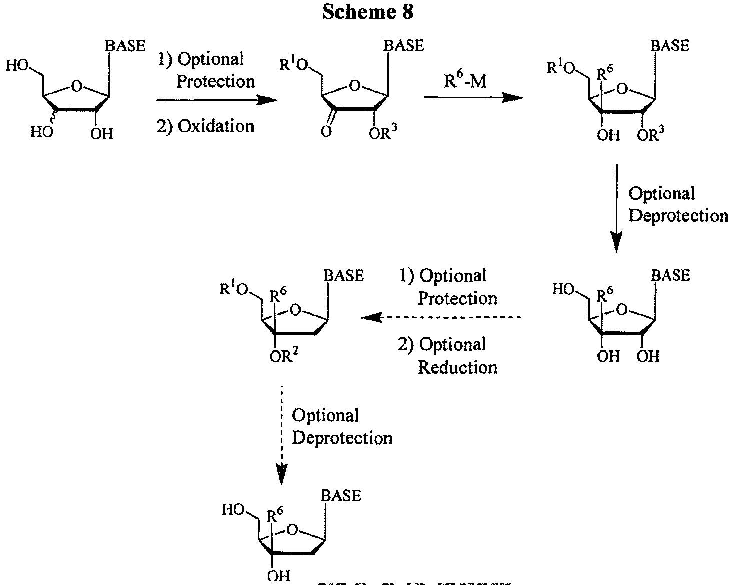

In another embodiment this reference example the L-enantiomers are desired. Therefore, the L-enantiomers can be corresponding to the compounds of the disclosure can be prepared following the same foregoing general methods, beginning with the corresponding L-sugar or nucleoside L-enantiomer as starting material.

### D. General Synthesis of 4'-C-Branched Nucleosides (Reference example)

### 4'-C-Branched ribonucleosides of the following structure:

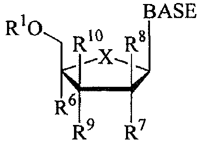

wherein BASE is a purine or pyrimidine base as defined herein;
R⁷ and R⁹ are independently hydrogen, OR², hydroxy, alkyl (including lower alkyl), azido, cyano, alkenyl, alkynyl, Br-vinyl, -C(O)O(alkyl), -C(O)O(lower alkyl), -O(acyl), -O(lower acyl), -O(alkyl), -O(lower alkyl), -O(alkenyl), chlorine, bromine, iodine, NO₂, NH₂, -NH(lower alkyl), -NH(acyl), -N(lower alkyl)₂, -N(acyl)₂;
R⁸ and R¹⁰ are independently H, alkyl (including lower alkyl), chlorine, bromine or iodine;
alternatively, R⁷ and R⁹, R⁷ and R¹⁰, R⁸ and R⁹, or R⁸ and R¹⁰ can come together to form a pi bond;
R¹ and R² are independently H; phosphate (including monophosphate, diphosphate, triphosphate, or a stabilized phosphate prodrug); acyl (including lower acyl); alkyl (including lower alkyl); sulfonate ester including alkyl or arylalkyl sulfonyl including methanesulfonyl and benzyl, wherein the phenyl group is optionally substituted with one or more substituents as described in the definition of aryl given herein; a lipid, including a phospholipid; an amino acid; a carbohydrate; a peptide; cholesterol; or other pharmaceutically acceptable leaving group which when administered *in vivo* is capable of providing a compound wherein R¹ is independently H or phosphate;
R⁶ is an alkyl, halogeno-alkyl (i.e. CF₃), alkenyl, or alkynyl (i.e. allyl); and
X is O, S, SO₂ or CH₂
can be prepared by the following general method.

### Modification from the pentodialdo-furanose

The key starting material for this process is an appropriately substituted pentodialdo-furanose. The pentodialdo-furanose can be purchased or can be prepared by any known means including standard epimerization, substitution and cyclization techniques.

In a preferred embodiment, the pentodialdo-furanose is prepared from the appropriately substituted hexose. The hexose can be purchased or can be prepared by any known means including standard epimerization (e.g. via alkaline treatment), substitution and coupling techniques. The hexose can be either in the furanose form, or cyclized via any means known in the art, such as methodology taught by Townsend Chemistry of Nucleosides and Nucleotides, Plenum Press, 1994**,** preferably by selectively protecting the hexose, to give the appropriate hexafuranose.

The 4'-hydroxymethylene of the hexafuranose then can be oxidized with the appropriate oxidizing agent in a compatible solvent at a suitable temperature to yield the 4'-aldo-modified sugar. Possible oxidizing agents are Swern reagents, Jones reagent (a mixture of chromic acid and sulfuric acid), Collins's reagent (dipyridine Cr(VI) oxide, Corey's reagent (pyridinium chlorochromate), pyridinium dichromate, acid dichromate, potassium permanganate, MnO₂ ruthenium tetroxide, phase transfer catalysts such as chromic acid or permanganate supported on a polymer, Cl₂-pyridine, H₂O₂-ammonium molybdate, NaBrO₂-CAN, NaOCl in HOAc, copper chromite, copper oxide, Raney nickel, palladium acetate, Meerwin-Pondorf-Verley reagent (aluminum *t*-butoxide with another ketone) and *N-*bromosuccinimide, though preferably using H₃PO₄, DMSO and DCC in a mixture of benzene/pyridine at room temperature.

Then, the pentodialdo-furanose can be optionally protected with a suitable protecting group, preferably with an acyl or silyl group, by methods well known to those skilled in the art, as taught by Greene et al. Protective Groups in Organic Synthesis, John Wiley and Sons, Second Edition, 1991**.** In the presence of a base, such as sodium hydroxide, the protected pentodialdo-furanose can then be coupled with a suitable electrophilic alkyl, halogeno-alkyl (i.e. CF₃), alkenyl or alkynyl (i.e. allyl), to obtain the 4'-alkylated sugar. Alternatively, the protected pentodialdo-furanose can be coupled with the corresponding carbonyl, such as formaldehyde, in the presence of a base, such as sodium hydroxide, with the appropriate polar solvent, such as dioxane, at a suitable temperature, which can then be reduced with an appropriate reducing agent to give the 4'-alkylated sugar. In one embodiment, the reduction is carried out using PhOC(S)Cl, DMAP, preferably in acetonitrile at room temperature, followed by treatment of ACCN and TMSS refluxed in toluene.

The optionally activated sugar can then be coupled to the BASE by methods well known to those skilled in the art, as taught by Townsend Chemistry of Nucleosides and Nucleotides, Plenum Press, 1994**.** For example, an acylated sugar can be coupled to a silylated base with a Lewis acid, such as tin tetrachloride, titanium tetrachloride or trimethylsilyltriflate in the appropriate solvent at a suitable temperature.

Subsequently, the nucleoside can be deprotected by methods well known to those skilled in the art, as taught by Greene et al. Protective Groups in Organic Synthesis, John Wiley and Sons, Second Edition, 1991**.**

In a particular embodiment, the 4'-C-branched ribonucleoside is desired. Alternatively, deoxyribo-nucleoside is desired. To obtain these deoxyribo-nucleosides, a formed ribo-nucleoside can optionally be protected by methods well known to those skilled in the art, as taught by Greene et al. Protective Groups in Organic Synthesis, John Wiley and Sons, Second Edition, 1991**,** and then the 2'-OH can be reduced with a suitable reducing agent. Optionally, the 2'-hydroxyl can be activated to facilitate reduction; i.e. via the Barton reduction.

In another embodiment of the disclosure the L-enantiomers are desired. Therefore, the L-enantiomers can be corresponding to the compounds of this reference example can be prepared following the same foregoing general methods, beginning with the corresponding L-pentodialdo-furanose as starting material.

### E. General Synthesis of 2' and/or 3'-Prodrugs

The key starting material for this process is an appropriately substituted 1', 2', 3' or 4'-branched β-D or β-L nucleosides. The branched nucleoside can be purchased or can be prepared by any known means including the techniques disclosed herein. The branched nucleoside can be optionally protected with a suitable protecting group, preferably with a silyl group, by methods well known to those skilled in the art, as taught by Greene et al. Protective Groups in Organic Synthesis, John Wiley and Sons, Second Edition, 1991. The optionally protected branched nucleoside can then be coupled with a suitable acyl doner, such as an acyl chloride and/or an acyl anhydride with the appropriate protic or aprotic solvent at a suitable temperature, to give the 2' and/or 3' prodrug of 1', 2', 3' or 4'-branched β-D or β-L nucleoside. (Synthetic Communications, 1978, 8(5), 327-333; J. Am. Chem. Soc., 1999, 121(24), 5661-5664.) Alternatively, the optionally protected branched nucleoside can then be coupled with a suitable acyl, such as a carboxylic acid, such as alkanoic acid and/or amino acid residue, optionally with a suitable coupling agent, with the appropriate aprotic solvent at a suitable temperature, to give the 2' and/or 3' prodrug of 1', 2', 3' or 4'-branched β-D or β-L nucleoside. Possible coupling reagents are any reagents that promote coupling, including but are not limiting to, Mitsunobu reagents (e.g. diisopropyl azodicarboxylate and diethyl azodicarboxylate) with triphenylphosphine or various carbodiimides. In one embodiment, for a 3'-prodrug of a 2'-branched nucleoside, the nucleoside is preferably not protected and is directly coupled to an alkanoic acid or amino acid residue with an appropriate coupling reagient, such as a carbodiimide.

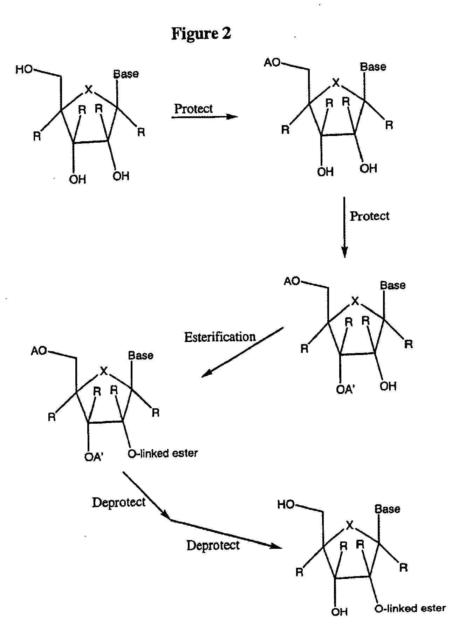

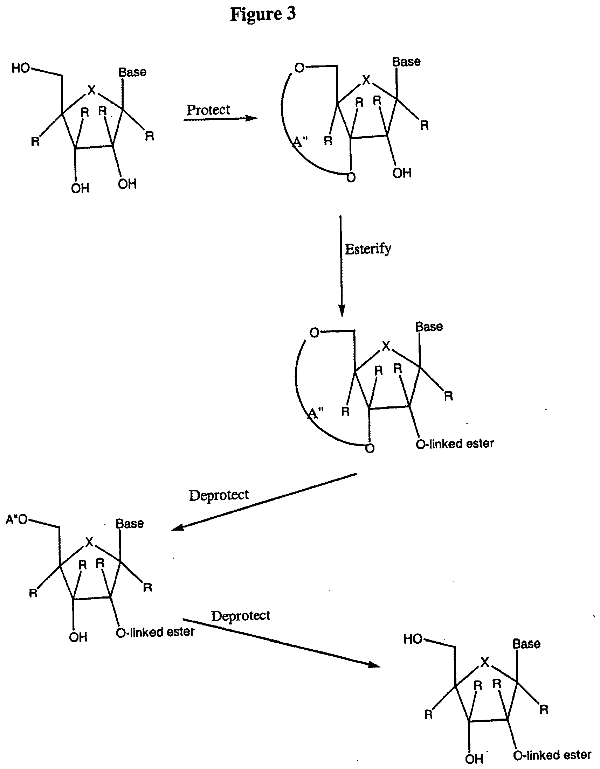

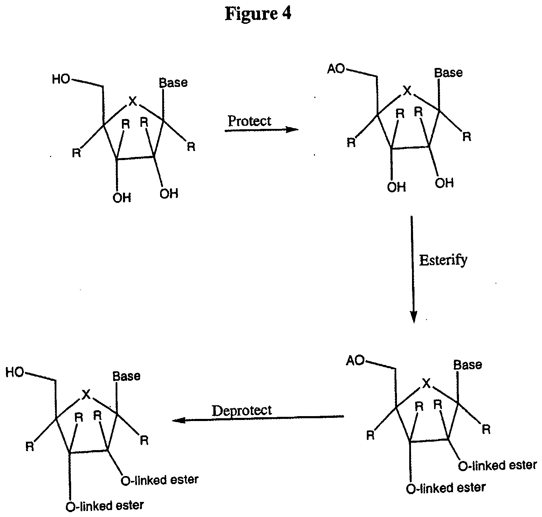

For example, simple amino-alcohols can be esterified using acid chlorides in refluxing acetonitrile-benzene mixture (See **Scheme 9** below: Synthetic Communications, 1978, 8(5), 327-333). Alternatively, esterification can be achieved using an anhydride, as described in J. Am. Chem, Soc., 1999, 121(24), 5661-5664. See **Figures 2****,** **3** and **4****.**

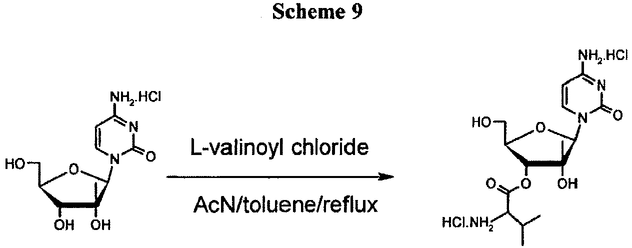

The present invention is described by way of illustration, in the following examples. It will be understood by one of ordinary skill in the art that these examples are in no way limiting and that, within the scope of the claims, variations of detail can be made without departing from the scope of the present invention.

### EXAMPLE 2: PREPARATION OF 2'-C-METHYLRIBOADENINE

The title compound was prepared according to a published procedure (R.E. Harry-O'kuru, J.M. Smith, and M.S. Wolfe, "A short, flexible route toward 2'-C-branched ribonucleosides", J.Org. Chem. 1997, 62, 1754-1759) **(Scheme 11).**

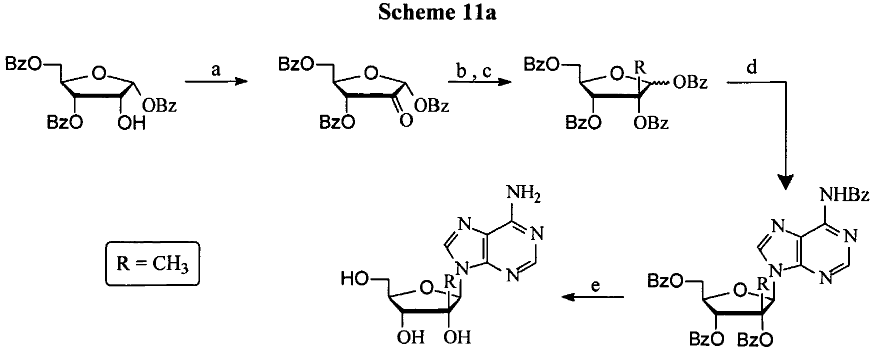

### (a) Dess-Martin periodinane; (b) MeMgBr / TiCl₄; (c) BzCl, DMAP, Et₃N; (d) bis(trimethylsilyl)acetamide, N⁶-benzoyl adenine, TMSOTf; (e) NH₃ / MeOH

The 3'-prodrug of the 2'-branched nucleoside was prepared according to published procedure *(*Synthetic Communications, 1978, 8(5), 327-333; J. Am. Chem. Soc., 1999, 121(24), 5661-5664). Alternatively, the 2'-branched nucleoside can be esterified without protection **(Scheme 11b).** Carbonyldiimidazole (377 mg, 2.33 mmol) was added to a solution of N-(tert-butoxycarbonyl)-L-valine (507 mg, 2.33 mmol) in 15 mL of anhydrous tetrahydrofuran. The mixture was stirred at 20 °C for one hour and at 50 °C for 10 minutes and then added to a solution of 4-Amino-1-(3,4-dihydroxy-5-hydroxymethyl-3-methyl-tetrahydro-furan-2-yl)-*1H*-pyrimidine-2-one (500 mg, 1.95 mmol), 4-(dimethylamino)pyridine (25 mg, 0.195 mmol), triethylamine (5 mL) in anhydrous N,N-dimethylformamide (10 mL), which is also stirring at 50 °C. The reaction mixture was stirred at 50 °C for one hour and then examined by HPLC*. HPLC analysis indicated the formation of 52% of the desired ester, 17% of starting material in addition to undesired by-products. The 3'-OH of 4-amino-1-(3,4-dihydroxy-5-hydroxymethyl-3-methyl-tetrahydro-furan-2-yl)-*1H-*pyrimidine-2-one tends to react selectively when coupled with BOC-Val.

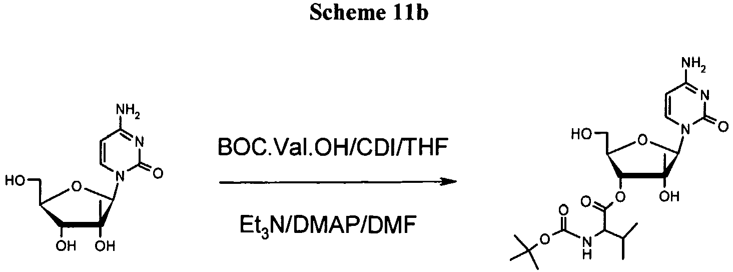

The product was analyzed by HPLC using a reverse phase column: Waters part # WAT086344; Nova-Pak C18, 60Å pore size, 4µm particle size, 3.9 x 150 mm. Chromatograms were generated using a Waters 2695 HPLC and 996 PDA detector. Mobile Phase: HPLC grade acetonitrile and water were bought from JT Baker and 1M solution of triethylammonium acetate from Fluka.
Flow rate: 1.00 mL/min. of an acetonitrile / 20mM aqueous triethylammonium acetate buffer gradient as described below.
System is equilibrated for five minutes between runs.
Wave length: 272 nm.

**Table D: Column Specifications**

| **Time** | **% Acetonitrile** | **% Buffer** |
|---|---|---|
| 0.00 | 0.00 | 100.0 |
| 15.00 | 80.0 | 20.0 |
| 30.00 | 80.0 | 20.0 |

**Table E: Description of compounds vs. retention times:**

| **Compound** | **RETENTION TIME (IN MINUTES)** |
|---|---|
| Desired ester | 8.3 |
| DMAP | 3.7 (Broad Peak) |
| Starting material | 2.7 |

In a similar manner, but using the appropriate sugar and pyrimidine or purine bases, the following nucleosides of Formula II are prepared. 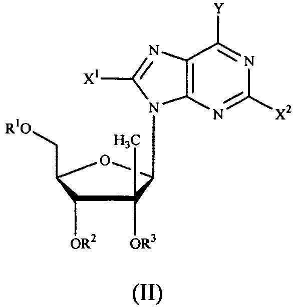 wherein R², R³, X¹, X², and Y are defined in Table 7.

Alternatively, the following nucleosides of Formula V are prepared, using the appropriate sugar and pyrimidine or purine bases. 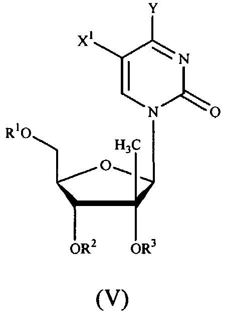 wherein R¹, R², R³, X¹ and Y are defined in Table 8.

Alternatively, the following nucleosides of Formula IX are prepared, using the appropriate sugar and pyrimidine or purine bases. 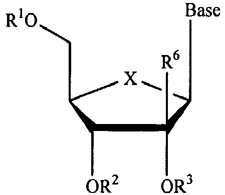 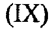 wherein R¹, R², R³, R⁶, X, and Base are defined in Table 9.

Alternatively, the following nucleosides of Formula X are prepared, using the appropriate sugar and pyrimidine or purine bases. 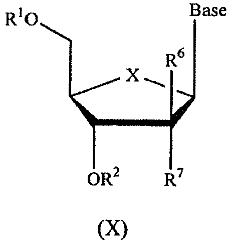 wherein R¹, R², R⁷, R⁶, X, and Base are defined in Table 10.

Alternatively, the following nucleosides of Formula XIV are prepared, using the appropriate sugar and pyrimidine or purine bases. 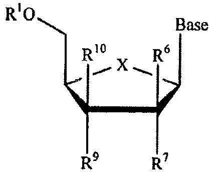 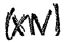 wherein R¹, R⁶, R⁷, X, Base, R⁹ and R¹⁰ are defined in Table 12.

Tables 7-10 and 12 set out examples of species within the present disclosure. When the amino acid appears in the table, it is considered to be a specific and independent disclosure of each of the esters of α, β γ or δ glycine, alanine, valine, leucine, isoleucine, methionine, phenylalanine, tryptophan, proline, serine, threonine, cysteine, tyrosine, asparagine, glutamine, aspartate, glutamate, lysine, arginine and histidine in the D and L-configurations. When the term acyl is used in the tables, it is meant to be a specific and independent disclosure of any of the acyl groups as defined herein, including but not limited to acetyl, trifluoroacetyl, methylacetyl, cyclopropylacetyl, cyclopropylcarboxy, propionyl, butyryl, hexanoyl, heptanoyl, octanoyl, neo-heptanoyl, phenylacetyl, diphenylacetyl, α-trifluoromethyl-phenylacetyl, bromoacetyl, 4-chloro-benzeneacetyl, 2-chloro-2,2-diphenylacetyl, 2-chloro-2-phenylacetyl, trimethylacetyl, chlorodifluoroacetyl, perfluoroacetyl, fluoroacetyl, bromodifluoroacetyl, 2-thiopheneacetyl, tert-butylacetyl, trichloroacetyl, monochloro-acetyl, dichloroacetyl, methoxybenzoyl, 2-bromo-propionyl, decanoyl, n-pentadecanoyl, stearyl, 3-cyclopentyl-propionyl, 1-benzene-carboxyl, pivaloyl acetyl, 1-adamantane-carboxyl, cyclohexane-carboxyl, 2,6-pyridinedicarboxyl, cyclopropane-carboxyl, cyclobutane-carboxyl, 4-methylbenzoyl, crotonyl, 1-methyl-1H-indazole-3-carbonyl, 2-propenyl, isovaleryl, 4-phenylbenzoyl.

### F. Biological Assays

Compounds can exhibit anti-flavivirus or pestivirus activity by inhibiting flavivirus or pestivirus polymerase, by inhibiting other enzymes needed in the replication cycle, or by other pathways.

### Phosphorylation Assay of Nucleoside to Active Triphosphate

To determine the cellular metabolism of the compounds, HepG2 cells were obtained from the American Type Culture Collection (Rockville, MD), and were grown in 225 cm² tissue culture flasks in minimal essential medium supplemented with non-essential amino acids, 1% penicillin-streptomycin. The medium was renewed every three days, and the cells were subcultured once a week. After detachment of the adherent monolayer with a 10 minute exposure to 30 mL of trypsin-EDTA and three consecutive washes with medium, confluent HepG2 cells were seeded at a density of 2.5 x 10⁶ cells per well in a 6-well plate and exposed to 10 µM of [³H] labeled active compound (500 dpm/pmol) for the specified time periods. The cells were maintained at 37°C under a 5% CO₂ atmosphere. At the selected time points, the cells were washed three times with ice-cold phosphate-buffered saline (PBS). Intracellular active compound and its respective metabolites were extracted by incubating the cell pellet overnight at -20°C with 60% methanol followed by extraction with an additional 20 µL of cold methanol for one hour in an ice bath. The extracts were then combined, dried under gentle filtered air flow and stored at -20°C until HPLC analysis.

### BioavailabilityAssay in Cynomolgus Monkeys

Within 1 week prior to the study initiation, the cynomolgus monkey was surgically implanted with a chronic venous catheter and subcutaneous venous access port (VAP) to facilitate blood collection and underwent a physical examination including hematology and serum chemistry evaluations and the body weight was recorded. Each monkey (six total) receives approximately 250 µCi of ³H activity with each dose of active compound at a dose level of 10 mg/kg at a dose concentration of 5 mg/mL, either via an intravenous bolus (3 monkeys, IV), or via oral gavage (3 monkeys, PO). Each dosing syringe was weighed before dosing to gravimetrically determine the quantity of formulation administered. Urine samples were collected via pan catch at the designated intervals (approximately 18-0 hours pre-dose, 0-4, 4-8 and 8-12 hours post-dosage) and processed. Blood samples were collected as well (pre-dose, 0.25, 0.5, 1, 2, 3, 6, 8, 12 and 24 hours post-dosage) via the chronic venous catheter and VAP or from a peripheral vessel if the chronic venous catheter procedure should not be possible. The blood and urine samples were analyzed for the maximum concentration (Cₘₐₓ), time when the maximum concentration was achieved (Tₘₐₓ), area under the curve (AUC), half life of the dosage concentration (T_{½}), clearance (CL), steady state volume and distribution (Vₛₛ) and bioavailability (F).

### Bone Marrow Toxicity Assay

Human bone marrow cells were collected from normal healthy volunteers and the mononuclear population were separated by Ficoll-Hypaque gradient centrifugation as described previously by Sommadossi J-P, Carlisle R. Antimicrobial Agents and Chemotherapy 1987; 31:452-454; and Sommadossi J-P, Schinazi RF, et al. Biochemical Pharmacology 1992; 44:1921-1925. The culture assays for CFU-GM and BFU-E were performed using a bilayer soft agar or methylcellulose method. Drugs were diluted in tissue culture medium and filtered. After 14 to 18 days at 37°C in a humidified atmosphere of 5% CO₂ in air, colonies of greater than 50 cells were counted using an inverted microscope. The results are presented as the percent inhibition of colony formation in the presence of drug compared to solvent control cultures.

### Mitochondria Toxicity Assay

HepG2 cells were cultured in 12-well plates as described above and exposed to various concentrations of drugs as taught by Pan-Zhou X-R, Cui L, et al. Antimicrob. Agents Chemother. 2000; 44:496-503. Lactic acid levels in the culture medium after 4 day drug exposure were measured using a Boehringer lactic acid assay kit. Lactic acid levels were normalized by cell number as measured by hemocytometer count.

### Cytotoxicity Assay

Cells were seeded at a rate of between 5 x 10³ and 5 x 10⁴/well into 96-well plates in growth medium overnight at 37°C in a humidified CO₂ (5%) atmosphere. New growth medium containing serial dilutions of the drugs was then added. After incubation for 4 days, cultures were fixed in 50% TCA and stained with sulforhodamineB. The optical density was read at 550 nm. The cytotoxic concentration was expressed as the concentration required to reduce the cell number by 50% (CC₅₀).

### Cell Protection Assay (CPA)

The assay was performed essentially as described by Baginski, S. G.; Pevear, D. C.; et al. PNAS USA 2000, 97(14), 7981-7986. MDBK cells (ATCC) were seeded onto 96-well culture plates (4,000 cells per well) 24 hours before use. After infection with BVDV (strain NADL, ATCC) at a multiplicity of infection (MOI) of 0.02 plaque forming units (PFU) per cell, serial dilutions of test compounds were added to both infected and uninfected cells in a final concentration of 0.5% DMSO in growth medium. Each dilution was tested in quadruplicate. Cell densities and virus inocula were adjusted to ensure continuous cell growth throughout the experiment and to achieve more than 90% virus-induced cell destruction in the untreated controls after four days post-infection. After four days, plates were fixed with 50% TCA and stained with sulforhodamine B. The optical density of the wells was read in a microplate reader at 550 nm. The 50% effective concentration (EC₅₀) values were defined as the compound concentration that achieved 50% reduction of cytopathic effect of the virus.

### Plaque Reduction Assay

For each compound the effective concentration was determined in duplicate 24-well plates by plaque reduction assays. Cell monolayers were infected with 100 PFU/well of virus. Then, serial dilutions of test compounds in MEM supplemented with 2% inactivated serum and 0.75% of methyl cellulose were added to the monolayers. Cultures were further incubated at 37°C for 3 days, then fixed with 50% ethanol and 0.8% Crystal Violet, washed and air-dried. Then plaques were counted to determine the concentration to obtain 90% virus suppression.

### Yield Reduction Assay

For each compound the concentration to obtain a 6-log reduction in viral load was determined in duplicate 24-well plates by yield reduction assays. The assay was performed as described by Baginski, S. G.; Pevear, D. C.; et al. PNAS USA 2000, 97(14), 7981-7986, with minor modifications. Briefly, MDBK cells were seeded onto 24-well plates (2 x 105 cells per well) 24 hours before infection with BVDV (NADL strain) at a multiplicity of infection (MOI) of 0.1 PFU per cell. Serial dilutions of test compounds were added to cells in a final concentration of 0.5% DMSO in growth medium. Each dilution was tested in triplicate. After three days, cell cultures (cell monolayers and supernatants) were lysed by three freeze-thaw cycles, and virus yield was quantified by plaque assay. Briefly, MDBK cells were seeded onto 6-well plates (5 x 105 cells per well) 24 h before use. Cells were inoculated with 0.2 mL of test lysates for 1 hour, washed and overlaid with 0.5% agarose in growth medium. After 3 days, cell monolayers were fixed with 3.5% formaldehyde and stained with 1% crystal violet (w/v in 50% ethanol) to visualize plaques. The plaques were counted to determine the concentration to obtain a 6-log reduction in viral load.

### EXAMPLE 25: ANTIVIRAL POTENCY OF TEST COMPOUNDS IN A CELL BASED ASSAY

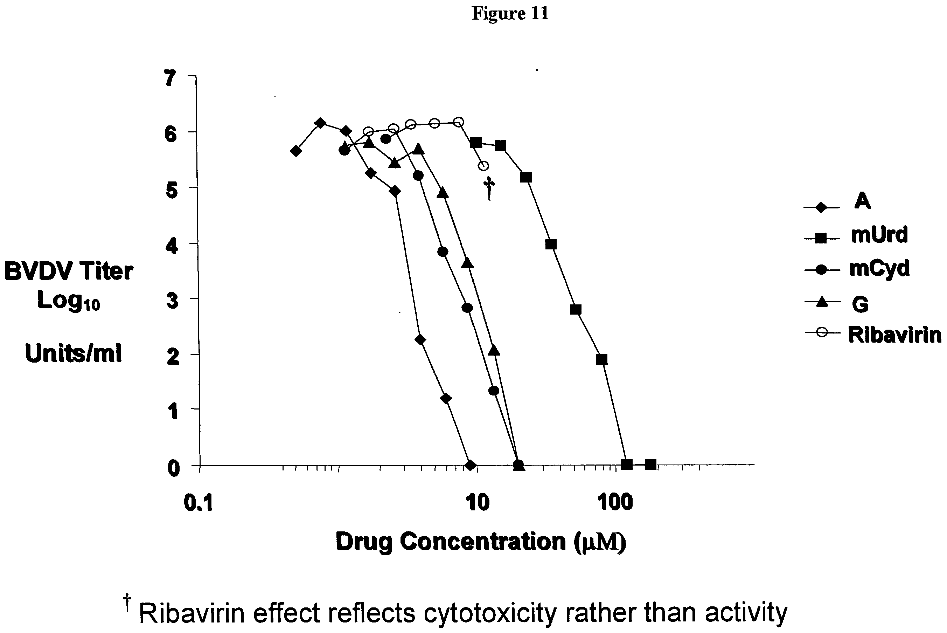

The titer of BVDB (Log₁₀ units/ml) were identified after treatment of infected MDBK cells with increasing concentrations of four test compounds. Ribavirin was used as a standard. This data is shown in Figure 11. The graph shows the antiviral potency of these compounds.

### EXAMPLE 26:CELLULAR PHARMACOLOGY OF 2'-C-METHYL-CYTIDINE- 3'-O-L-VALINE ESTER (VAL-MCYD)

### Phosphorylation Assay of Nucleoside to Active Triphosphate

To determine the cellular metabolism of the compounds, HepG2 cells were obtained from the American Type Culture Collection (Rockville, MD), and were grown in 225 cm² tissue culture flasks in minimal essential medium supplemented with non-essential amino acids, 1% penicillin-streptomycin. The medium was renewed every three days, and the cells were subcultured once a week. After detachment of the adherent monolayer with a 10 minute exposure to 30 mL of trypsin-EDTA and three consecutive washes with medium, confluent HepG2 cells were seeded at a density of 2.5 x 10⁶ cells per well in a 6-well plate and exposed to 10 µM of [³H] labeled active compound (500 dpm/pmol) for the specified time periods. The cells were maintained at 37°C under a 5% CO₂ atmosphere. At the selected time points, the cells were washed three times with ice-cold phosphate-buffered saline (PBS). Intracellular active compound and its respective metabolites were extracted by incubating the cell pellet overnight at -20°C with 60% methanol followed by extraction with an additional 20 µL of cold methanol for one hour in an ice bath. The extracts were then combined, dried under gentle filtered air flow and stored at -20°C until HPLC analysis.

Antiviral nucleosides and nucleoside analogs were generally converted into the active metabolite, the 5'-triphosphate (TP) derivatives by intracellular kinases. The nucleoside-TPs then exert their antiviral effect by inhibiting the viral polymerase during virus replication. In primary human hepatocyte cultures, in a human hepatoma cell line (HepG2), and in a bovine kidney cell line (MDBK), mCyd was converted into a major metabolite, 2'-C-methyl-cytidine-5'- triphosphate (mCyd-TP), along with smaller amounts of a uridine 5'-triphosphate derivative, 2'-C-methyl-uridine-5'- triphosphate (mUrd-TP). mCyd-TP is inhibitory when tested *in vitro* against the BVDV replication enzyme, the NS5B RNA dependent RNA polymerase, and is thought to be responsible for the antiviral activity of mCyd.

The cellular metabolism of mCyd was examined using MDBK cells, HepG2 cells and human primary hepatocytes exposed to 10 µM [³H]-mCyd. High-pressure liquid chromatography (HPLC) analysis demonstrated that mCyd was phosphorylated in all three cell types, with mCyd-TP being the predominant metabolite after 24 h. The metabolic profile obtained over a 48-hour exposure of human hepatoma HepG2 cells to 10 µM [³H]-mCyd was tested. In HepG2 cells, levels of mCyd-TP peaked at 41.5 ± 13.4 µM after 24 hours (see Table 25) and fell slowly thereafter. In primary human hepatocytes, the peak mCyd-TP concentration at 24 hours was 4 fold lower at 10.7 ± 6.7 µM. MDBK bovine kidney cells yielded intermediate levels of mCyd-TP (30.1 ± 6.9 µM at 24 hours).

Exposure of hepatocytes to mCyd led to production of a second 5'-triphosphate derivative, mUrd-TP. In HepG2 cells exposed to 10 µM [³H]-mCyd, the mUrd-TP level reached 1.9 ± 1.6 µM at 24 hours, compared to 8.1 ± 3.4 µM in MDBK cells and 3.2 ± 2.0 µM in primary human hepatocytes. While MDBK and HepG2 cells produced comparable total amounts of phosphorylated species (approximately 43 versus 47 µM, respectively) at 24h, mUrd-TP comprised 19% of the total product in MDBK cells versus only 4% in HepG2 cells. mUrd-TP concentration increased steadily over time, however reached a plateau or declined after 24 hours.

**Table 25: Activation of mCyd (10 µM) in Hepatocytes and MDBK Cells**

| | | **Metabolite (µM)** | | | | | |
|---|---|---|---|---|---|---|---|
| **Cells^{a}** | **n** | **mCyd-MP** | **mUrd-MP** | **mCyd-DP** | **mUrd-DP** | **mCyd-TP** | **mUrd-TP** |
| HepG2 | 6 | ND | ND | 3.7 ± 2.1 | ND | 41.5 ± 13.4 | 1.9 ± 1.6 |
| Human Primary Hepatocytes | 5 | ND | ND | 1.15 ± 1.1 | 0.26 ± 0.4 C | 10.7 ± 6.7 | 3.2 ± 2.0 |
| MDBK Bovine Kidney Cells | 7 | ND | ND | 4.2 ± 2.7 | 0.76 ± 0.95 | 30.1 ± 6.9 | 8.1 ± 3.4 |

| | | | | | | | |
|---|---|---|---|---|---|---|---|
| a. Cells were incubated for 24 hours with [³H]-mCyd, specific activity: HepG2 assay = 0.5 Ci/mmol; human and monkey hepatocyte assay = 1.0 Ci/mmol. b. The concentrations of metabolites were determined as pmoles per million cells. One pmole per million cells is roughly equivalent to 1 µM. ND, not detected. | | | | | | | |

The apparent intracellular half-life of the mCyd-TP was 13.9 ± 2.2 hours in HepG2 cells and 7.6 ± 0.6 hours in MDBK cells: the data were not suitable for calculating the half life of mUrd-TP. Other than the specific differences noted above, the phosphorylation pattern detected in primary human hepatocytes was qualitatively similar to that obtained using HepG2 or MDBK cells.

### EXAMPLE 27: CELL CYTOTOXICITY

### Mitochondria Toxicity Assay

HepG2 cells were cultured in 12-well plates as described above and exposed to various concentrations of drugs as taught by Pan-Zhou X-R, Cui L, et al.. Antimicrob. Agents Chemother. 2000; 44:496-503. Lactic acid levels in the culture medium after 4 day drug exposure were measured using a Boehringer lactic acid assay kit. Lactic acid levels were normalized by cell number as measured by hemocytometer count.

### Cytotoxicity Assays

Cells were seeded at a rate of between 5 x 10³ and 5 x 10⁴/well into 96-well plates in growth medium overnight at 37°C in a humidified CO₂ (5%) atmosphere. New growth medium containing serial dilutions of the drugs was then added. After incubation for 4 days, cultures were fixed in 50% TCA and stained with sulforhodamineB. The optical density was read at 550 nm. The cytotoxic concentration was expressed as the concentration required to reduce the cell number by 50% (CC₅₀).

Conventional cell proliferation assays were used to assess the cytotoxicity of mCyd and its cellular metabolites in rapidly dividing cells. The inhibitory effect of mCyd was determined to be cytostatic in nature since mCyd showed no toxicity in confluent cells at concentrations far in excess of the corresponding CC₅₀ for a specific cell line. mCyd was not cytotoxic to rapidly growing Huh7 human hepatoma cells or H9c2 rat myocardial cells at the highest concentration tested (CC₅₀ >250 µM). The mCyd CC₅₀ values were 132 and 161 µM in BHK-21 hamster kidney and HepG2 human hepatoma cell lines, respectively. The CC₅₀ for mCyd in HepG2 cells increased to 200 µM when the cells were grown on collagen-coated plates for 4 or 10 days. For comparison, CC₅₀ values of 35-36 µM were derived when ribavirin was tested in HepG2 and Huh7 cells. In the MDBK bovine kidney cells used for BVDV antiviral studies, the CC₅₀ of mCyd was 36 µM. A similar CC₅₀ value (34 µM) was determined for mCyd against MT-4 human T-lymphocyte cells. In addition, mCyd was mostly either non-cytotoxic or weakly cytotoxic (CC₅₀ >50 to >200 µM) to numerous other cell lines of human and other mammalian origin, including several human carcinoma cell lines, in testing conducted by the National Institutes of Health (NIH) Antiviral Research and Antimicrobial Chemistry Program. Exceptions to this were rapidly proliferating HFF human foreskin fibroblasts and MEF mouse embryo fibroblasts, where mCyd showed greater cytotoxicity (CC₅₀s 16.9 and 2.4 µM, respectively). Again, mCyd was much less toxic to stationary phase fibroblasts.

The cytotoxic effect of increasing amounts of mCyd on cellular DNA or RNA synthesis was examined in HepG2 cells exposed to [³H]-thymidine or [³H]-uridine. In HepG2 cells, the CC₅₀s of mCyd required to cause 50% reductions in the incorporation of radiolabeled thymidine and uridine into cellular DNA and RNA, were 112 and 186 µM, respectively. The CC₅₀ values determined for ribavirin (RBV) for DNA and RNA synthesis, respectively, were 3.16 and 6.85 µM. These values generally reflect the CC₅₀s of 161 and 36 µM determined for mCyd and RBV, respectively, in conventional cell proliferation cytotoxicity assays. To assess the incorporation of mCyd into cellular RNA and DNA, HepG2 cells were exposed to 10 µM [³H]-mCyd or control nucleosides (specific activity 5.6 - 8.0 Ci/mmole, labeled in the base) for 30 hours. Labeled cellular RNA or DNA species were separately isolated and incorporation was determined by scintillation counting. Exposure of HepG2 cells to mCyd resulted in very low levels of incorporation of the ribonucleoside analog into either cellular DNA or RNA (0.0013 - 0.0014 pmole/µg of nucleic acid). These levels were similar to the 0.0009 and 0.0013 pmole/µg values determined for the incorporation of ZDV and ddC, respectively, into RNA: since these deoxynucleosides were not expected to incorporate into RNA, these levels likely reflect the assay background. The incorporation of ZDV and ddC into DNA was significantly higher (0.103 and 0.0055 pmole/µg, respectively). Ribavirin (RBV) incorporated into both DNA and RNA at levels 10-fold higher than mCyd.

**Table 26a: Cellular Nucleic Acid Synthesis and Incorporation Studies in HepG2 Cells (10 µM Drug and Nucleoside Controls)**

| | **CC₅₀ (µM)** | | **Incorporated drug amount** | |
|---|---|---|---|---|
| **Compound** | **DNA ([³H]Thymidine)** | **RNA ([³H]Uridine)** | **pmole/µg DNA** | **pmole/µg RNA** |
| mCyd | 112.3 ± 34.5 | 186.1 ± 28.2 | 0.0013 ± 0.0008^{a} | 0.0014 ± 0.0008^{a} |
| ZDV | nd | nd | 0.103 ± 0.0123^{a} | 0.0009 ± 0.0003^{a} |
| ddC | nd | nd | 0.0055^{b} | 0.0013^{b} |
| Ribavirin | 3.16 ± 0.13 | 6.85 ± 1.83 | 0.0120^{b} | 0.0132^{c} |

| | | | | |
|---|---|---|---|---|
| a. Data represent mean of three experiments b. Data represent one experiment c. Data represent mean of two experiments nd, not determined | | | | |

**Table 26b: Cytotoxicity of mCyd in Mammalian Cell Lines**

| **Cell Line^{a}** | **n** | **CC₅₀(µM)** |
|---|---|---|
| Huh 7 | 7 | > 250 |
| Hep G2 | 6 | 161 ± 19 |
| Hep G2^{b} | 2 | > 200 |
| MDBK | 7 | 36 ± 7 |
| BHK-21 | 2 | 132 ± 6 |
| H9c2 | 2 | > 250 |

| | | |
|---|---|---|
| a. All cytotoxicity testing was one under conditions of rapid cell division b. Cells were grown on collagen coated plates for 4 or 10 d | | |

### Bone Marrow Toxicity Assay

Human bone marrow cells were collected from normal healthy volunteers and the mononuclear population were separated by Ficoll-Hypaque gradient centrifugation as described previously by Sommadossi J-P, Carlisle R. Antimicrobial Agents and Chemotherapy 1987; 31:452-454; and Sommadossi J-P, Schinazi RF, et al. Biochemical Pharmacology 1992; 44:1921-1925. The culture assays for CFU-GM and BFU-E were performed using a bilayer soft agar or methylcellulose method. Drugs were diluted in tissue culture medium and filtered. After 14 to 18 days at 37°C in a humidified atmosphere of 5% CO₂ in air, colonies of greater than 50 cells were counted using an inverted microscope. The results are presented as the percent inhibition of colony formation in the presence of drug compared to solvent control cultures.

### Cell Protection Assay (CPA)

The assay was performed essentially as described by Baginski, S. G.; Pevear, D. C.; et al. PNAS USA 2000, 97(14), 7981-7986. MDBK cells (ATCC) were seeded onto 96-well culture plates (4,000 cells per well) 24 hours before use. After infection with BVDV (strain NADL, ATCC) at a multiplicity of infection (MOI) of 0.02 plaque forming units (PFU) per cell, serial dilutions of test compounds were added to both infected and uninfected cells in a final concentration of 0.5% DMSO in growth medium. Each dilution was tested in quadruplicate. Cell densities and virus inocula were adjusted to ensure continuous cell growth throughout the experiment and to achieve more than 90% virus-induced cell destruction in the untreated controls after four days post-infection. After four days, plates were fixed with 50% TCA and stained with sulforhodamine B. The optical density of the wells was read in a microplate reader at 550 nm. The 50% effective concentration (EC₅₀) values were defined as the compound concentration that achieved 50% reduction of cytopathic effect of the virus.

The myelosuppressive effects of certain nucleoside analogs have highlighted the need to test for potential effects of investigational drugs on the growth of human bone marrow progenitor cells in clonogenic assays. In particular, anemia and neutropenia are the most common drug-related clinical toxicities associated with the anti-HIV drug zidovudine (ZDV) or the ribavirin (RBV) component of the standard of care combination therapy used for HCV treatment. These toxicities have been modeled in an *in vitro* assay that employed bone marrow cells obtained from healthy volunteers (Sommadossi J-P, Carlisle R. Antimicrob. Agents Chemother. 1987;31(3): 452-454). ZDV has been previously shown to directly inhibit human granulocyte-macrophage colony-forming (CFU-GM) and erythroid burst-forming M in this model (BFU-E) activity at clinically relevant concentrations of 1-2 (Berman E, et al. Blood 1989;74(4):1281-1286; Yoshida Y, Yoshida C. AIDS Res. Hum. Retroviruses 1990;6(7):929-932.; Lerza R, et al. Exp. Hematol. 1997;25(3):252-255; Dornsife RE, Averett DR. Antimicrob. Agents Chemother. 1996;40(2):514-519; Kurtzberg J, Carter SG. Exp. Hematol. 1990;18(10):1094-1096 ; Weinberg RS, et al. Mt. Sinai J. Med. 1998;65(1):5-13). Using human bone marrow clonogenic assays, the CC₅₀ values of mCyd in CFU-GM and BFU-E were 14.1 ± 4.5 and 13.9 ± 3.2 µM (see Table 27). mCyd was significantly less toxic to bone marrow cells than both ZDV and RBV (Table 27).

**Table 27: Bone Marrow Toxicity of mCyd in Granulocyte Macrophage Progenitor and Erythrocyte Precursor Cells**

| **Compound** | **CFU-GM^{a} CC₅₀(µM)** | **BFU-E^{a} CC₅₀ (µM)** |
|---|---|---|
| mCyd | 14.1 ± 4.5 µM | 13.9 ± 3.2 |
| ZDV | 0.89 ± 0.47 | 0.35 ± 0.28 |
| RBV | 7.49 ± 2.20 | 0.99 ± 0.24 |

| | | |
|---|---|---|
| a. Data from 3 independent experiments for RBV and 5-8 independent experiments for mCyd and ZDV. All experiments were done in triplicate. | | |

### Effect on Mitochondrial Function

Antiviral nucleoside analogs approved for HIV therapy such as ZDV, stavudine (d4T), didanosine (ddI), and zalcitabine (ddC) have been occasionally associated with clinically limiting delayed toxicities such as peripheral neuropathy, myopathy, and pancreatitis (Browne MJ, et al. J. Infect. Dis. 1993;167(1):21-29; Fischl MA, et al. Ann. Intern. Med. 1993;18(10):762-769.; Richman DD, et al. N. Engl. J. Med. 1987;317(4): 192-197; Yarchoan R, et al. Lancet 1990;336(8714):526-529). These clinical adverse events have been attributed by some experts to inhibition of mitochondrial function due to reduction in mitochondrial DNA (mtDNA) content and nucleoside analog incorporation into mtDNA. In addition, one particular nucleoside analog, fialuridine (1,-2'-deoxy-2'-fluoro-1-β-D-arabinofuranosyl-5-iodo-uracil; FIAU), caused hepatic failure, pancreatitis, neuropathy, myopathy and lactic acidosis due to direct mitochondrial toxicity (McKenzie R, et al. N. Engl. J. Med. 1995;333(17):1099-1105). Drug-associated increases in lactic acid production can be considered a marker of impaired mitochondrial function or oxidative phosphorylation. (Colacino, J. M. Antiviral Res. 1996 29(2-3): 125-39).

To assess the potential of mCyd to produce mitochondrial toxicity, several *in vitro* studies were conducted using the human hepatoma cell lines HepG2 or Huh7. These studies included analysis of lactic acid production, mtDNA content, and determination of changes in morphology (e.g., loss of cristae, matrix dissolution and swelling, and lipid droplet formation) of mitochondrial ultrastructure.

The effects of mCyd on mitochondria are presented in Table 28. No differences were observed in lactic acid production in mCyd-treated cells versus untreated cells at up to 50 µM mCyd in Huh7 cells or10 µM mCyd in HepG2 cells. A modest (38%) increase in lactic acid production was seen in HepG2 cells treated with 50 µM mCyd. The significance of this finding is unclear, particularly since mCyd is unlikely to attain a plasma concentration of 50 µM in the clinic. For comparison, lactic acid production increases by 100% over control cells in cells treated with 10 µM FIAU (Cui L, Yoon, et al. J. Clin. Invest. 1995;95:555-563). Exposure of HepG2 cells to mCyd for 6 or 14 days at concentrations up to 50 µM had no negative effect on mitochondrial DNA content compared to a 56 or 80% reduction in ddC-treated cells, respectively.

Following M mCyd, the ultrastructure of HepG2 cells, and in□14 days of exposure to 10 particular mitochondria, was examined by transmission electron microscopy. No changes in cell architecture, or in mitochondrial number or morphology (including cristae), were observed in the majority of cells. In 17% of the cells, 1 to 2 mitochondria out of an average of 25 per cell appeared enlarged. Such minor changes would be unlikely to have any significant impact on mitochondrial function. ddC-treated cells showed abnormal mitochondrial morphology with loss of cristae, and the accumulation of fat droplets. (Medina, D. J., C. H. Tsai, et al. Antimicrob. Agents Chemother. 1994 38(8): 1824-8; Lewis W, et al. J. Clin. Invest. 1992;89(4):1354-1360., Lewis, L. D., F. M. Hamzeh, et al. Antimicrob. Agents Chemother. 1992 36(9): 2061-5).

**Table 28: Effect of mcyd on Hepatocyte Proliferation, Mitochondrial Function, and Morphology in HepG2 Cells**

| | | **L-Lactate (% of Control^{a})** | | **mtDNA/nuclear DNA (% of Control^{b})** | | **Electron Microscopy^{c}** | |
|---|---|---|---|---|---|---|---|
| Agent | Conc (µM) | HepG2 Cells | Huh7 Cells | 6 day Treatment | 14 day Treatment | Lipid Droplet Form. | Mito.Morphol. |
| Cont. | 0 | 100 | 100 | 100 | 100 | Negative | Normal |
| mCyd | 10 | 98.6 ± 7.3 | 98.0 ± 12.3 | 117.3 ± 17.5 | 99.7 ± 23.9 | Negative | Normal^{d} |
| | 50 | 138.0 ± 8.9 | 97.1 ± 10.1 | 158.2 ± 17.5 | 83.0 ± 15.5 | nd | nd |
| ddC | 1 | nd | nd | 44.3 ± 9.3 | 19.6 ± 8.2 | nd | nd |
| | 10 | nd | nd | nd | nd | Positive | Loss of Cristae |

### Effect on Human DNA Polymerases α, β, and γ

The cellular DNA polymerases are responsible for normal nuclear and mitochondrial DNA synthesis and repair. Nucleoside analog triphosphates are potential inhibitors of DNA polymerases and hence could disrupt critical cell functions. In particular, the inhibition of human polymerase γ, the enzyme responsible for mitochondrial DNA synthesis, has been linked to defects in mitochondrial function (Lewis, W., E. S. Levine, et al. Proceedings of the National Academy of Sciences, USA 1996 93(8): 3592-7.). Experiments were undertaken to determine if mCyd-TP inhibited human DNA polymerases. As shown in Table 29 mCyd-TP was not a substrate for human DNA polymerases α, β, or γ. Even 1 mM mCyd-TP failed to inhibit these enzymes by 50% in the majority of replicate assays and IC₅₀ values could only be determined to be in excess of 880-1000 µM. In contrast, ddC was a potent inhibitor of all three human DNA polymerases and of polymerases β and γ in particular (IC₅₀s of 4.8 and 2.7 µM, respectively). Potent inhibition was also seen for the control drug, actinomycin D, a known inhibitor of DNA-dependent-DNA polymerases.

**Table 29: Inhibition of Human Polymerases by mCyd-Triphosphate**

| | **IC₅₀ (µM)** | | |
|---|---|---|---|
| | **mCyd-TP^{a}** | **ddC-TP^{b}** | **Act. D^{a}** |
| **Pol α** | >1000 | 78 ± 23.4 | 5.8 ± 3.1 |
| **Pol β** | ≥883.3 ± 165 | 4.8 ± 1 | 7.9 ± 3 |
| **Pol γ** | ≥929.3 ± 100 | 2.7 ± 1 | 15.5 ± 4 |

| | | | |
|---|---|---|---|
| a. Mean ± S.D. from 4 data sets b. Mean ± S.D. from 2 data sets | | | |

a. HepG2 or huh7 cells were treated with compounds for 4 days, data represent at least three independent experiments
b. HepG2 cells were treated with compounds for 6 and 14 days, data represents at least three independent experiments
c. HepG2 cells were treated with compounds for 14 days
d. 17% cells (11 of 64) contained 1 or 2 enlarged mitochondria out of 25 in two independent experiments nd, not determined

### EXAMPLE 28: IN VITRO ANTIVIRAL ACTIVITY AGAINST BVDV

Compounds can exhibit anti-flavivirus or pestivirus activity by inhibiting flavivirus or pestivirus polymerase, by inhibiting other enzymes needed in the replication cycle, or by other pathways.

### Plaque Reduction Assay

For each compound the effective concentration was determined in duplicate 24-well plates by plaque reduction assays. Cell monolayers were infected with 100 PFU/well of virus. Then, serial dilutions of test compounds in MEM supplemented with 2% inactivated serum and 0.75% of methyl cellulose were added to the monolayers. Cultures were further incubated at 37°C for 3 days, then fixed with 50% ethanol and 0.8% Crystal Violet, washed and air-dried. Then plaques were counted to determine the concentration to obtain 90% virus suppression.

### Yield Reduction Assay

For each compound the concentration to obtain a 6-log reduction in viral load was determined in duplicate 24-well plates by yield reduction assays. The assay was performed as described by Baginski, S. G.; Pevear, D. C.; Seipel, M.; et al. PNAS USA 2000, 97(14), 7981-7986, with minor modifications. Briefly, MDBK cells were seeded onto 24-well plates (2 x 105 cells per well) 24 hours before infection with BVDV (NADL strain) at a multiplicity of infection (MOI) of 0.1 PFU per cell. Serial dilutions of test compounds were added to cells in a final concentration of 0.5% DMSO in growth medium. Each dilution was tested in triplicate. After three days, cell cultures (cell monolayers and supernatants) were lysed by three freeze-thaw cycles, and virus yield was quantified by plaque assay. Briefly, MDBK cells were seeded onto 6-well plates (5 x 105 cells per well) 24 h before use. Cells were inoculated with 0.2 mL of test lysates for 1 hour, washed and overlaid with 0.5% agarose in growth medium. After 3 days, cell monolayers were fixed with 3.5% formaldehyde and stained with 1% crystal violet (w/v in 50% ethanol) to visualize plaques. The plaques were counted to determine the concentration to obtain a 6-log reduction in viral load.

Studies on the antiviral activity of mCyd in cultured cells were conducted. The primary assay used to determine mCyd antiviral potency was a BVDV-based cell-protection assay (CPA). This assay measures the ability of mCyd to protect growing MDBK bovine kidney cells from destruction by a cytopathic NADL strain of BVDV. The cytotoxicity of the test drug on uninfected cells was measured in parallel. The antiviral activities of mCyd and ribavirin in the CPA are compared in Table 30a. mCyd effectively protected *de novo-*infected MDBK cells in a concentration-dependent manner with an EC₅₀ = 0.67 ± 0.22 µM (Table 30a). mCyd afforded complete cytoprotection at concentrations well below the CC₅₀ for mCyd in this assay (38 ± 9 µM). In the CPA, as well as in other assays described below, ribavirin showed no clear antiviral effect: significant (50% or more) cell protection was not achieved in most assays as the cytotoxicity of ribavirin overlaps and masks the protective effect. Thus, ribavirin gave a CC₅₀ of 4.3 ± 0.6 µM and an EC₅₀> 4.3 µM in the CPA.

For Tables 30a-30o below, cell lines utilized include MT-4 for HIV; Vero 76, African green monkey kidney cells for SARS; BHK for Bovine Viral Diarrhea Virus; Sb-1 for poliovirus Sabin type-1; CVB-2, CVB-3, CVB-4, and CVA-9 for Coxsackieviruses B-2, B-3, B-4 and A-9; and REO-1 for double-stranded RNA viruses. Note: BVDV = bovine viral diarrhea virus; YFV = yellow fever virus; DENV = dengue virus; WNV = West Nile virus; CVB-2 = Coxsackie B-2 virus; Sb-1 = Sabin type 1 poliomyelitis virus; and REO = double-stranded RNA Reovirus.

**Table 30a: In Vitro Activity of mCyd Against BVDV in the Cell Protection Assay**

| **Compound** | **n** | **EC₅₀, µM** | **CC₅₀, µM** |
|---|---|---|---|
| mCyd | 11 | 0.67 ± 0.22 | 38 ± 9 |
| RBV | 3 | >4.3 | 4.3 ± 0.6 |

**Table 30b: CC₅₀ Test Results for β-D-2'-C-methyl-cytidine (Compound G), 3'-O-valinyl ester of β-D-2'-C-methyl-cytidine dihydrochloride salt (Compound M), and β-D-2'-C-methyl-uracil (Compound N)**

| | CC₅₀ | | | | | | | |
|---|---|---|---|---|---|---|---|---|
| Compound | | BVDV | YFV | DENV 2 | WNV | CVB-2 | Sb-1 | REO |
| G | 34 | 2.3 | 54 | 95 | 80 | 12 | 11.5 | 13 |
| M | 24 | 5.8 | 82 | >100 | 82 | 12 | 14 | 22 |
| N | >100 | 18 | 100 | > or = 100 | 80 | >100 | 55 | >100 |

**Table 30c. CC₅₀ and EC₅₀ Test Results for β-D-2'-C-methyl-cytidine Compound G)**

| | CC₅₀ | CC₅₀ | CC₅₀ | EC₅₀ | EC₅₀ | EC₅₀ | EC₅₀ | EC₅₀ | EC₅₀ |
|---|---|---|---|---|---|---|---|---|---|
| Compound | MT-4 | Vero 76 | BHK | Sb-1 | CVB-2 | CVB-3 | CVB-4 | CVA-9 | REO-1 |
| G | 34 | >100 | >100 | 6 | 11 | 9 | 13 | 26 | 13 |

**Table 30d. CC₅₀ and EC₅₀ Test Results for β-D-2'-C-methyl-adenosine (Compound A) and β-D-2'-C-methyl-2-amino adenosine (Compound B)**

| | CC₅₀ | CC₅₀ | CC₅₀ | EC₅₀ | EC₅₀ | EC₅₀ | EC₅₀ | EC₅₀ | EC₅₀ |
|---|---|---|---|---|---|---|---|---|---|
| Compound | MT-4 | Vero 76 | BHK | Sb-1 | CVB-2 | CVB-3 | CVB-4 | CVA-9 | REO-1 |
| A | 4 | 80 | 70 | 10 | 10 | 14 | 13 | 12 | >70 |
| B | >100 | >100 | 50 | 90 | 75 | 23 | 32 | 39 | 2 |

**Table 30e. CC₅₀ and EC₅₀ Test Results for β-D-2'-C-methyl-guanosine Compound C) and β-D-2'-C-methyl-6-chloro-guanosine (Compound D)**

| | CC₅₀ | CC₅₀ | CC₅₀ | EC₅₀ | EC₅₀ | EC₅₀ | EC₅₀ | EC₅₀ | EC₅₀ |
|---|---|---|---|---|---|---|---|---|---|
| Compound | MT-4 | Vero 76 | BHK | Sb-1 | CVB-2 | CVB-3 | CVB-4 | CVA-9 | REO-1 |
| C | >100 | >100 | 100 | 22 | 30 | 22 | 12 | 46 | 2 |
| D | >100 | >100 | 30 | 50 | 25 | 21 | 25 | 37 | 0.4 |

**Table 30f. CC₅₀ and EC₅₀ Test Results for 3',5'-di-O-valinyl ester of β-D-2'-C-methyl-guanosine dihydrochloride salt (Compound E)**

| | CC₅₀ | CC₅₀ | CC₅₀ | EC₅₀ | EC₅₀ | EC₅₀ | EC₅₀ | EC₅₀ | EC₅₀ |
|---|---|---|---|---|---|---|---|---|---|
| Compound | MT-4 | Vero 76 | BHK | Sb-1 | CVB-2 | CVB-3 | CVB-4 | CVA-9 | REO-1 |
| E | >100 | >100 | 100 | 30 | 33 | 30 | 35 | 40 | 2 |

**Table 30g. CC₅₀ and EC₅₀ Test Results for β-D-2'-C-methyl-cytidine Compound G)**

| | CC₅₀ | CC₅₀ | CC₅₀ | EC₅₀ | EC₅₀ | EC₅₀ | EC₅₀ | EC₅₀ | EC₅₀ |
|---|---|---|---|---|---|---|---|---|---|
| Compound | MT-4 | Vero 76 | BHK | Sb-1 | CVB-2 | CVB-3 | CVB-4 | CVA-9 | REO-1 |
| G | 34 | >100 | >100 | 6 | 11 | 9 | 13 | 26 | 13 |

**Table 30h. CC₅₀ and EC₅₀ Test Results for β-D-2'-C-ethynyl-adenosine (Compound H)**

| | CC₅₀ | CC₅₀ | CC₅₀ | EC₅₀ | EC₅₀ | EC₅₀ | EC₅₀ | EC₅₀ | EC₅₀ |
|---|---|---|---|---|---|---|---|---|---|
| Compound | MT-4 | Vero 76 | BHK | Sb-1 | CVB-2 | CVB-3 | CVB-4 | CVA-9 | REO-1 |
| H | 4.6 | 60 | 15 | 1 | 1.5 | 1 | 2 | 2.5 | 6 |

**Table 30i. CC₅₀ and EC₅₀ Test Results for β-D-2'-C-ethynyl-cytidine (Compound I)**

| | CC₅₀ | CC₅₀ | CC₅₀ | EC₅₀ | EC₅₀ | EC₅₀ | EC₅₀ | EC₅₀ | EC₅₀ |
|---|---|---|---|---|---|---|---|---|---|
| Compound | MT-4 | Vero 76 | BHK | Sb-1 | CVB-2 | CVB-3 | CVB-4 | CVA-9 | REO-1 |
| I | > or = 100 | >100 | >100 | 26 | 33 | 33 | 24 | 59 | >100 |

**Table 30j. CC₅₀ and EC₅₀ Test Results for β-D-2-amino-adenosine (Compound J)**

| | CC₅₀ | CC₅₀ | CC₅₀ | EC₅₀ | EC₅₀ | EC₅₀ | EC₅₀ | EC₅₀ | EC₅₀ |
|---|---|---|---|---|---|---|---|---|---|
| Compound | MT-4 | Vero 76 | BHK | Sb-1 | CVB-2 | CVB-3 | CVB-4 | CVA-9 | REO-1 |
| J | 50 | >100 | >100 | 40 | 53 | 55 | 50 | 53 | >100 |

**Table 30k. CC₅₀ Test Results for β-D-2'-C-methyl-adenosine (Compound A), β-D-2'-C-methyl-2-amino adenosine (Compound B), and β-D-2'-C-methyl-2-amino-6-cyclopropyl adenosine(Compound K)**

| | CC₅₀ | | | | | | | |
|---|---|---|---|---|---|---|---|---|
| Compound | | BVDV | YFV | DENV 2 | WNV | CVB-2 | Sb-1 | REO |
| A | 4.0 | 1.2 | 2.7 | 2.7 | 3.6 | 7 | 7 | >70 |
| B | >100 | 2.1 | 0.8 | 0.7 | 0.3 | 76 | 90 | 2 |
| K | >100 | 18 | 10 | 4.9 | 3.5 | >100 | >100 | 9.5 |

**Table 30l. CC₅₀ Test Results for β-D-2'-C-methyl-guanosine (Compound C), β-D-2'-C-methyl-1-(methyl-2-oxo-2-phenyl ethyl)guanosine (Compound L), and β-D-2'-C-methyl-6-chloro guanosine (Compound D)**

| | CC₅₀ | | | | | | | |
|---|---|---|---|---|---|---|---|---|
| Compound | | BVDV | YFV | DENV 2 | WNV | CVB-2 | Sb-1 | REO |
| C | >100 | 3.5 | 1.2 | 1.4 | 0.6 | 29 | 50 | 2 |
| L | >100 | 12 | 6 | 4.4 | 3 | >100 | >100 | 12 |
| D | >100 | 0.7 | 1.0 | 0.7 | 0.3 | 25 | 50 | 0.4 |

**Table 30m. CC₅₀ Test Results for 3',5'-di-O-valinyl ester of β-D-2'-C-methyl-guanosine dihydrochloride salt (Compound E)**

| | CC₅₀ | | | | | | | |
|---|---|---|---|---|---|---|---|---|
| Compound | | BVDV | YFV | DENV 2 | WNV | CVB-2 | Sb-1 | REO |
| E | >100 | 4.9 | 1.0 | 1.4 | 1 | 33 | 55 | 2.1 |

**Table 30n. CC₅₀ Test Results for β-D-2'-C-ethynyl-adenosine (Compound H)**

| | CC₅₀ | | | | | | | |
|---|---|---|---|---|---|---|---|---|
| Compound | | BVDV | YFV | DENV 2 | WNV | CVB-2 | Sb-1 | REO |
| H | 4.6 | 0.4 | 2.0 | 1.1 | 1 | 1.2 | 0.7 | 6 |

**Table 30o. CC₅₀ Test Results for β-D-2'-C-methyl-cytidine (Compound G), 3'-O-valinyl ester of β-D-2'-C-methyl-cytidine dihydrochloride salt (Compound M), and β-D-2'-C-methyl-uracil (Compound N)**

| | CC₅₀ | | | | | | | |
|---|---|---|---|---|---|---|---|---|
| Compound | | BVDV | YFV | DENV 2 | WNV | CVB-2 | Sb-1 | REO |
| G | 34 | 2.3 | 54 | 95 | 80 | 12 | 11.5 | 13 |
| M | 24 | 5.8 | 82 | >100 | 82 | 12 | 14 | 22 |
| N | >100 | 18 | 100 | > or = 100 | 80 | >100 | 55 | >100 |

The overall antiviral potency of mCyd was determined against different strains of BVDV and both cytopathic (cp) and noncytopathic (ncp) biotypes in cell protection assays as well as in plaque reduction and yield reduction assays. The latter assays measure the output of infectious virus from cells and hence provide a stringent test of antiviral efficacy. The different data sets from all three assays show agreement as summarized in Table 31. The range of 50% and 90% effective inhibitory concentration (EC₅₀ and EC₉₀) values for mCyd was 0.3 to 2.8 µM and 0.87 to 4.8 µM, respectively.

In the BVDV yield reduction assay, subcytotoxic concentrations (circa 20 µM) of mCyd suppressed *de novo* BVDV production by up to 6 logs, to the point where no infectious virus was detected. A 4 log₁₀ effective reduction in BVDV production (EC_{4log10} or EC_{99.99}) was attained between 6.0 and 13.9 µM mCyd. In contrast, interferon alpha 2b (IFN α2b), although active against BVDV in this assay (EC₅₀ 2.6 IU per ml), never gave more than 2 logs of viral reduction, even at 1000 IU per ml. Thus, the antiviral effect of mCyd against BVDV was much greater than that of IFNa2b or RBV.

### EXAMPLE 29:: IN VITRO ANTIVIRAL ACTIVITY AGAINST OTHER POSITIVE-STRAND RNA VIRUSES

mCyd has been tested for efficacy against positive-strand RNA viruses other than BVDV. Data obtained are summarized in Table 31 and 32. Against flaviruses, mCyd showed modest activity. The composite EC₅₀ ranges (in µM) determined from both sites were: West Nile virus (46-97); Yellow Fever virus (9-80); and Dengue Virus (59-95). For mCyd against the alpha virus, Venezuelan Equine Encephalitis virus, EC₅₀ values were 1.3 - 45 µM. mCyd was broadly active against Picornoviruses, such as Polio virus (EC₅₀ = 6 µM), Coxsackie virus (EC₅₀ = 15 µM), Rhinovirus types 5 and 14 (EC₅₀s = <0.1 and 0.6 µg/ml) and Rhinovirus type 2 (EC₅₀ 2 - 10 µM). mCyd was generally inactive against all RNA and DNA viruses tested except for the positive-strand RNA viruses. mCyd was also found to have no activity against HIV in MT-4 human T lymphocyte cells or HBV in HepG2.2.15 cells.

**Table 31: In Vitro Antiviral Activity of mCyd Against Plus-Strand RNA Viruses**

| Method of Assay | Virus Type | Cell Type | n | Antiviral Efficacy (µM) | | |
|---|---|---|---|---|---|---|
| | | | | EC₅₀ | EC₉₀ | EC_{4 log} |
| Cell Protection Assay | BVDV NADL cp | MDBK | 11 | 0.67 ± 0.22 | | |
| Yield Reduction Assay | BVDV NADL cp | MDBK | 3 | 2.77 ± 1.16 | 4.8 ± 1.55 | 13.9 ± 3.07 |
| | BVDV New York-1 ncp | MDBK | 6 | 0.30 ± 0.07 | 0.87 ± 0.18 | 6.03 ± 1.41 |
| | BVDV I-NADL cp | MDBK | 1 | 0.68 | 1.73 | 8.22 |
| | BVDV I-N-dIns ncp | MDBK | 1 | 0.59 | 1.49 | 7.14 |
| Plaque Reduction Assay | BVDV NADL cp | MDBK | 3 | 2.57 ± 0.35 | 4.63 ± 0.72 | |
| Cell Protection Assay | West Nile Virus | BHK | 3 | 63-97 | | |
| Cell Protection Assay | Yellow Fever Virus 17D | BHK | 1 | 60-80 | | |
| | DENV-2 | BHK | 2 | 95 | | |
| Cell Protection Assay | DENV-4 | BHK | 1 | 59 | | |
| | Polio Virus | | | | | |
| Plaque Reduction | Sb-1 | VERO | 1 | 6 | | |
| Assay | | | | | | |
| Plaque Reduction Assay | Coxsackie Virus B2 | VERO | 1 | 15 | | |

| | | | | | | |
|---|---|---|---|---|---|---|
| cp, cytopathic virus; ncp noncytopathic virus I-NADL cp and I-N-dIns ncp represent recombinant BVDV viruses | | | | | | |

**Table 32: In Vitro Antiviral Activity, Selectivity, and Cytotoxicity of mCyd**

| **Virus (Cell line)^{a}** | **EC₅₀^{b}(µM)** | **CC₅₀^{c}(µM)** |
|---|---|---|
| WNV (Vero) | 46 | 114-124 |
| YFV (Vero) | 9-30 | 150->200 |
| VEE (Vero) | 1.3-45 | >200 |
| HSV-1 (HFF)^{d} | >100 | >100 |
| HSV-2 (HFF)^{d} | >100 | >100 |
| VZV (HFF)^{d} | >20 | 67.8 |
| EBV (Daudi)^{d} | 25.5 | >50 |
| HCMV (HFF)^{d} | 9.9-15.6 | 67-73 |
| MCMV (MEF) | >0.8 | 2.4 |
| Influenza A/H1N1 (MDCK) | >200 | >200 |
| Influenza A/H3N2 (MDCK) | >20 | 45-65 |
| Influenza B (MDCK) | >200 | 55-140 |
| Adenovirus type 1 (A549) | >200 | >200 |
| Parainfluenza type 3 (MA-104) | >200 | >200 |
| Rhinovirus type 2 (KB) | 2-10 | >200 |
| Rhinovirus type 5 (KB)^{d} | 0.6 | 20-30 |
| Rhinovirus type 14 (HeLa-Ohio)^{d} | <0.1 | 20->100 |
| RSV type A (MA-104) | >200 | 200 |
| Punta Toro A (LLC-MK2) | >200 | >200 |

| | | |
|---|---|---|
| a. HFF, human foreskin fibroblast; Daudi, Burkitt's B-cell lymphoma; MDCK, canine kidney cells; CV-1, African green monkey kidney cells; KB, human nasopharyngeal carcinoma; MA-104, Rhesus monkey kidney cells; LLC-MK2, Rhesus monkey kidney cells; A549, Human lung carcinoma cells; MEF, mouse embryo fibroblast; Vero, African green monkey kidney cells; HeLa, human cervical adenocarcinoma cells. b. EC₅₀ = 50% effective concentration. c. CC₅₀ = 50% cytotoxic concentration. d. Result presented in µg/mL rather than µM. | | |

### EXAMPLE 30: MULTIPLICITY OF INFECTION (MOI) AND ANTIVIRAL EFFICACY

The cell protection assay format was used to test the effect of increasing the amount of BVDV virus on the EC₅₀ of mCyd. Increasing the MOI of BVDV in this assay from 0.04 to 0.16, caused the EC₅₀ of mCyd to increase linearly from 0.5 µM to approximately 2.2 µM.

### EXAMPLE 31: VIRAL REBOUND IN MCYD TREATED CELLS

The effect of discontinuing treatment with mCyd was tested in MDBK cells persistently infected with a noncytopathic strain (strain I-N-dIns) of BVDV. Upon passaging in cell culture, these cells continuously produce anywhere from 10⁶ to >10⁷ infectious virus particles per ml of media. This virus can be measured by adding culture supernatants from treated MDBK (BVDV) cells to uninfected MDBK cells and counting the number of resultant viral foci after disclosure by immunostaining with a BVDV-specific antibody. Treatment of a persistently infected cell line with 4 µM mCyd for one cell passage (3 days) reduced the BVDV titer by approximately 3 log₁₀ from pretreatment and control cell levels of just under 10⁷ infectious units per ml. At this point, mCyd treatment was discontinued. Within a single passage, BVDV titers rebounded to untreated control levels of just over 10⁷ infectious units per ml.

### EXAMPLE 32: MECHANISM OF ACTION

In standard BVDV CPA assays, mCyd treatment results in a marked increase in total cellular RNA content as cells grow, protected from the cytopathic effects of BVDV. This was coupled with a marked decrease in the production of BVDV RNA due to mCyd. Conversely, in the absence of mCyd, total cellular RNA actually decreases as BVDV RNA rises due to the destruction of the cells by the cytopathic virus. To further test the effect of mCyd on viral and cellular RNAs, the accumulation of intracellular BVDV RNA was monitored in MDBK cells 18-hours post infection (after approximately one cycle of virus replication) using Real Time RT-PCR. In parallel, a cellular housekeeping ribosomal protein mRNA (rig S15 mRNA) was also quantitated by RT-PCR using specific primers. The results showed that mCyd dramatically reduced BVDV RNA levels in *de novo*-infected MDBK cells with an EC₅₀ of 1.7 µM and an EC₉₀ of 2.3 µM. The maximum viral RNA reduction was 4 log₁₀ at the highest inhibitor concentration tested (125 µM). No effect on the level of the rig S15 cellular mRNA control was observed. Together, the preceding findings suggest that mCyd inhibits BVDV by specifically interfering with viral genome RNA synthesis without impacting cellular RNA content. This idea is further supported by the observation (Table 26a) that inhibition of RNA synthesis as measured by [³H]-uridine uptake in HepG2 cells requires high concentrations of mCyd (EC₅₀ = 186 µM).

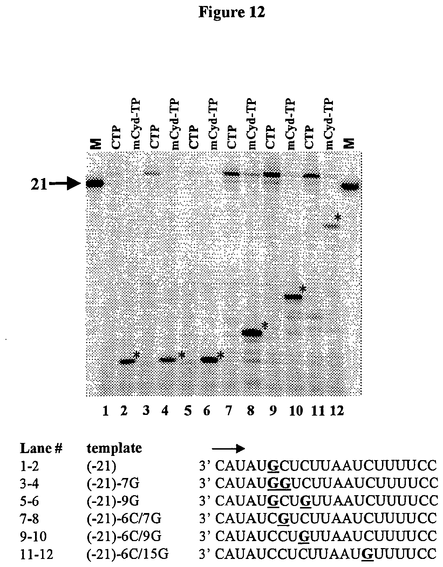

In *in vitro* studies using purified BVDV NS5B RNA-dependent RNA polymerase (Kao, C. C., A. M. Del Vecchio, et al. 1999. Virology 253(1): 1-7) and synthetic RNA templates, mCyd-TP inhibited RNA synthesis with an IC₅₀ of 0.74 µM and was a competitive inhibitor of BVDV NS5B RNA-dependent RNA polymerase with respect to the natural CTP substratE. The inhibition constant (K*ᵢ*) for mCyd-TP was 0.16 µM and the Michaelis-Menten constant (K*ₘ*) for CTP was 0.03 µM. Inhibition of RNA synthesis by mCyd-TP required the presence of a cognate G residue in the RNA template. The effect of mCyd-TP on RNA synthesis in the absence of CTP was investigated in more detail using a series of short (21 mer) synthetic RNA templates containing a single G residue, which was moved progressively along the template. Analysis of the newly synthesized transcripts generated from these templates in the presence of mCyd-TP revealed that RNA elongation continued only as far as the G residue, then stopped (Figure 12). In templates containing more than one G residue, RNA synthesis stopped at the first G residue encountered by the polymerase. These data strongly suggest that m-Cyd-TP is acting as a non-obligate chain terminator. The mechanism of this apparent chain termination is under further investigation.

### EXAMPLE 33: Eradication Of A Persistent Bvdv Infection

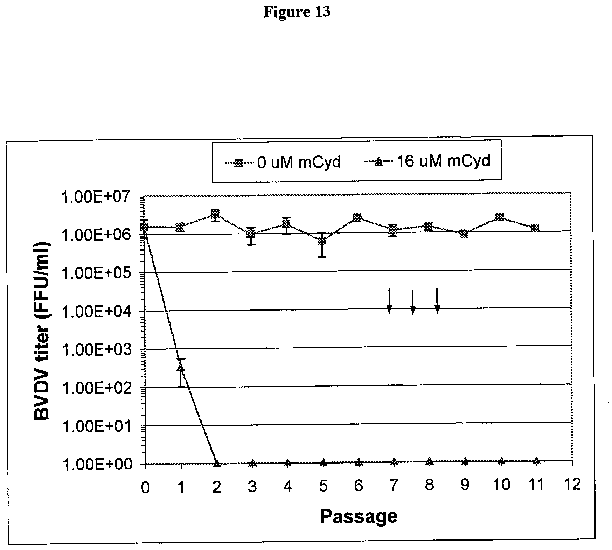

The ability of mCyd to eradicate a viral infection was tested in MDBK cells persistently infected with a noncytopathic strain of BVDV (strain I-N-dIns). (Vassilev, V. B. and R. O. Donis Virus Res. 2000 69(2): 95-107.) Compared to untreated cells, treatment of persistently infected cells with 16 µM mCyd reduced virus production from more than 6 logs of virus per ml to undetectable levels within two cell passages (3 to 4 days per passage). No further virus production was seen upon continued treatment with mCyd through passage 12. At passages 8, 9 and 10 (arrows, Figure 13), a portion of cells was cultured for two further passages in the absence of drug to give enough time for mCyd-TP to decay and virus replication to resume. The culture media from the cells were repeatedly tested for the re-emergence of virus by adding culture supernatants from treated MDBK (BVDV) cells to uninfected MDBK cells and counting the resultant viral foci after disclosure by immunostaining with a BVDV-specific antibody. Although this assay can detect a single virus particle, no virus emerged from the cells post drug treatment. Thus, treatment with mCyd for 8 or more passages was sufficient to eliminate virus from the persistently infected cells.

### EXAMPLE 34: COMBINATION STUDIES WITH INTERFERON ALPHA 2_{B}

The first study, performed in MDBK cells persistently infected with the New York-1 (NY-1) strain of BVDV, compared the effect of monotherapy with either mCyd (8 µM) or interferon alpha 2b (200 IU/ml), or the two drugs in combination (Figure 14A). In this experiment, 8 µM mCyd alone reduced viral titers by approximately 3.5 log₁₀ after one passage to a level that was maintained for two more passages. Interferon alpha 2b alone was essentially inactive against persistent BVDV infection (approximately 0.1 log₁₀ reduction in virus titer) despite being active against *de novo* BVDV infection. However, the combination of mCyd plus interferon alpha 2b reduced virus to undetectable levels by the second passage and clearly showed better efficacy to either monotherapy.

In a follow up study (Figure 14B) of MDBK cells persistently infected with the I-N-dIns noncytopathic strain of BVDV, mCyd was supplied at fixed doses of 0, 2, 4 and 8 µM, while interferon alpha 2b was titrated from 0 to 2,000 IU per ml. Again, interferon alpha 2b was essentially inactive (0.1 log reduction in viral titer), while mCyd alone inhibited BVDV (strain I-N-dIns) propagation in a dose-dependent manner. mCyd at 8 µM reduced virus production by 6.2 log₁₀, to almost background levels.

### EXAMPLE 35: RESISTANCE DEVELOPMENT

In early cell culture studies, repeated passaging of a cytopathic strain of BVDV in MDBK cells in the presence of mCyd failed to generate resistant mutants, suggesting that the isolation mCyd-resistant BVDV mutants is difficult. However, studies in cell lines persistently infected with noncytopathic forms of BVDV led to the selection of resistant virus upon relatively prolonged treatment with mCyd at suboptimal therapeutic concentrations of drug (2 to 8 µM, depending on the experiment). In the representative experiment shown in Figure 15A, the virus was no longer detectable after two passages in the presence of 8 µM mCyd, but re-emerged by passage 6. The lower titer of the re-emergent virus is apparent from the data: resistant virus typically has a 10 fold or more lower titer than the wild-type virus and was easily suppressed by co-therapy with IntronA (Figure 15A). The phenotype of the virus that re-emerged was remarkably different from the initial wild-type virus: as shown in Figure 15B, it yielded much smaller foci (typically, 3 to 10 times smaller in diameter then those of the wild-type virus). This phenotype did not change after prolonged passaging in culture in the presence of the inhibitor (at least 72 days), however, it quickly reverted to the wild-type phenotype (large foci) after the discontinuation of the treatment.

RT-PCR sequencing of the resistant mutant was used to identify the mutation responsible for resistance. Sequencing efforts were focused on the NS5B RNA-dependent RNA polymerase region of BVDV, which was assumed to be the likely target for a nucleoside inhibitor. A specific S405T amino-acid substitution was identified at the start of the highly conserved B domain motif of the polymerase. The B domain is part of the polymerase active site and is thought to be involved in nucleoside binding (Lesburg, C. A., M. B. Cable, et al. Nature Structural Biology 1999 6(10): 937-43). Resistance to nucleosides has been mapped to this domain for other viruses such as HBV (Ono et al, J. Clin. Invest. 2001 Feb;107(4):449-55.). To confirm that this mutation was responsible for the observed resistance, the mutation was reintroduced into the backbone of a recombinant molecular clone of BVDV. The resulting clone was indistinguishable in phenotypic properties from the isolated mutant virus, confirming that the S405T mutation is responsible for resistance and that the NS5B RNA-dependent RNA polymerase is the molecular target for mCyd. The highly conserved nature of this motif at the nucleotide sequence (Lai, V. C., C. C. Kao, et al. J. Virol. 1999 73(12): 10129-36) and structural level among positive-strand RNA viruses (including HCV) allows a prediction that the equivalent mutation in the HCV NS5B RNA-dependent RNA polymerase would likely be S282T.

S405T mutant BVDV was refractory to mCyd up to the highest concentrations that could be tested (EC₅₀>32 µM), but was also significantly impaired in viability compared to wild-type virus. As noted above, the S405T mutant exhibited a 1-2 log₁₀ lower titer than wild-type. BVDV and produced much smaller viral plaques. In addition, the mutant virus showed a marked reduction in the rate of a single cycle of replication (>1000-fold lower virus titer at 12h), and accumulated to about 100 fold lower levels than the wild-type virus even after 36 h of replication (Figure 15C). The virus also quickly reverted to wild-type virus upon drug withdrawal. Finally, the mutant was also more sensitive (∼40 fold) to treatment with IFN alpha 2b than wild-type as shown in Figure 15D.

A second, additional mutation, C446S, was observed upon further passaging of the S405T mutant virus in the presence of drug. This mutation occurs immediately prior to the essential GDD motif in the C domain of BVDV NS5B RNA-dependent RNA polymerase. Preliminary studies suggest that a virus bearing both mutations does not replicate significantly better than the S405T mutant, hence the contribution of this mutation to viral fitness remains unclear. Further studies to characterize resistance development are ongoing.

### EXAMPLE 36: IN VIVO ANTIVIRAL ACTIVITY OF VAL-MCYD IN AN ANIMAL EFFICACY MODEL

Chimpanzees chronically infected with HCV are the most widely accepted animal model of HCV infection in human patients (Lanford, R. E., C. Bigger, et al. Ilar J. 2001 42(2): 117-26; Grakoui, A., H. L. Hanson, et al. Hepatology 2001 33(3): 489-95). A single *in vivo* study of the oral administration of val-mCyd in the chimpanzee model of chronic hepatitis C virus infection has been conducted.

HCV genotyping on the five chimpanzees was performed by the Southwest Foundation Primate Center as part of their mandated internal Health and Maintenance Program, designed to ascertain the disease status of all animals in the facility to identify potential safety hazards to employees. The five chimpanzees used in this study exhibited a high HCV titer in a genotyping RT PCR assay that distinguishes genotype 1 HCV from all other genotypes, but does not distinguish genotype 1a from 1b. This indicates that the chimpanzees used in this study were infected with genotype 1 HCV (HCV-1).

**Table 33: Summary of Val-mCyd In Vivo Activity Study in the Chimpanzee Model of Chronic HCV Infection**

| **Study Description** | **Species (N)** | **Val-mCyd Doses (mg/kg) (n)** | **Frequency/ Route of Administration** | **Study Endpoints** |
|---|---|---|---|---|
| One-week antiviral activity of mCyd in chronically hepatitis C virus (genotype 1)-infected chimpanzees | Chimpanzee (5) | 10 and 20 (2 each) [equivalent to 8.3 and 16.6 mpk of free base], and vehicle control (1) | QD x 7 days (PO) | Serum HCV RNA, serum chemistries, CBCs, general well being, and clinical observations |

### Seven-Day Antiviral Activity Study in the Chimpanzee Model of Chronic Hepatitis C Virus Infection

Four chimpanzees (2 animals per dose group at 10 mg/kg/day or 20 mg/kg/day) received val-mCyd dihydrochloride, freshly dissolved in a flavorful fruit drink vehicle. These doses were equivalent to 8.3 and 16.6 mg/kg/day of the val-mCyd free base, respectively. A fifth animal dosed with vehicle alone provided a placebo control. The study design included three pretreatment bleeds to establish the baseline fluctuation of viral load and three bleeds during the one week of treatment (on days 2, 5 and 7 of therapy) to evaluate antiviral efficacy. The analysis was completed at the end of the one-week dosing period, with no further follow up.

### HCV RNA Determination

Serum levels of HCV RNA throughout the study were determined independently by two clinical hospital laboratories. HCV RNA was assayed using a quantitative RT-PCR nucleic acid amplification test (Roche Amplicor HCV Monitor Test, version 2.0). This assay has a lower limit of detection (LLOD) of 600 IU/mL and a linear range of 600-850,000 IU/mL.

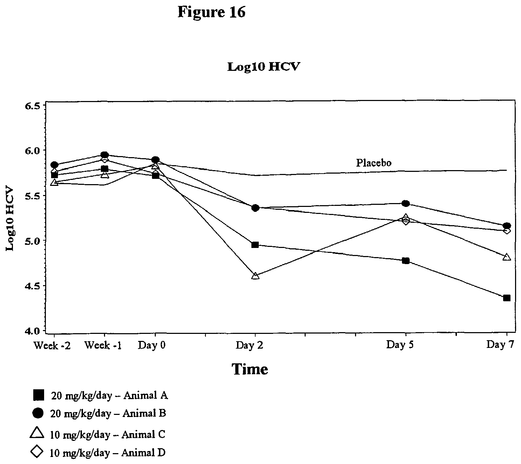

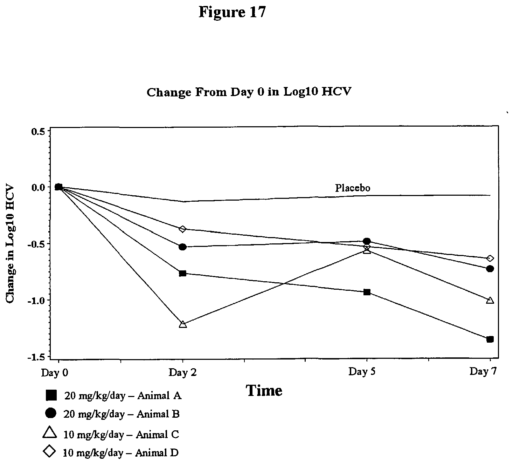

To aid in interpretation of the viral load declines seen during therapy, emphasis was placed on determining (i) the extent of fluctuations in baseline HCV viral load in individual animals, and (ii) the inherent variability and reproducibility of the HCV viral load assay. To address these issues, full viral load data sets obtained from the two laboratories were compared. The results from both sites were found to be closely comparable and affirmed both the stability of the pretreatment HCV viral loads as well as the reliability of the HCV Roche Amplicor assay. To present the most balanced view of the study, the mean values derived by combining both data sets were used to generate the results presented in Figures 16 and 17. Figure 16 presents the averaged data for dose cohorts, while Figure 17 presents the individual animal data. The changes in viral load from baseline seen during therapy for each animal at each site are also summarized in Table 34.

The HCV viral load analysis from the two sites revealed that pretreatment HCV viral loads were (i) very similar among all five animals and all 3 dose groups, and (ii) very stable over the 3-week pretreatment period. The mean pretreatment log₁₀ viral load and standard deviations among the five individual animals were 5.8 ± 0.1 (site 1) and 5.6 ± 0.1(site 2). These data indicate that the c.v. (coefficient of variance) of the assay is only around 2% at both sites. The largest fluctuation in HCV viral load seen in any animal during pretreatment was approximately 0.3 log₁₀.

As seen in Figures 16 and 17, once a day oral delivery of val-mCyd produced a rapid antiviral effect that was not seen for the placebo animal, nor during the pretreatment period. Viral titers were substantially reduced from baseline after two days of therapy for all animals receiving val-mCyd, and tended to fall further under continued therapy in the two treatment arms. By the end of treatment (day 7), the mean reductions from baseline HCV viral load were 0.83 log₁₀ and 1.05 log₁₀ for the 8.3 and 16.6 mg/kg/day dose groups, respectively. The titer of the placebo animal remained essentially unchanged from baseline during the therapy period.

An analysis of the data from the two quantification sites on the changes in baseline HCV viral load in response to therapy is presented in Table 34. Overall, the two data sets agree well, confirming the reliability of the assay. With the exception of animal 501, the difference in viral load between the two sites was generally 0.3 log₁₀ or less, similar to the fluctuation observed during the pretreatment period. For animal 501, the discrepancy was closer to 0.5 log₁₀. The viral load drop seen in response to therapy varied from 0.436 (animal 501, site 1) to 1.514 log₁₀ (animal 497, site 2). The latter corresponds to a change in HCV viral load from 535,000 (pretreatment) to 16,500 (day 7) genomes per ml.

**Table 34: Summary of Changes in Baseline Log₁₀ HCV RNA Viral Load During Therapy**

| **Dose (mpk)** | **Animal ID** | **Site** | **Day 2** | **Day 5** | **Day 7** |
|---|---|---|---|---|---|
| 0 | 499 | 1 | -0.00041 | -0.11518 | 0.14085 |
| | | 2 | -0.06604 | 0.10612 | -0.16273 |
| 8.3 | 500 | 1 | -1.15634 | -0.40385 | -0.80507 |
| | | 2 | -1.07902 | -0.55027 | -1.06259 |
| 8.3 | 501 | 1 | -0.25180 | -0.36179 | -0.43610 |
| | | 2 | -0.45201 | -0.71254 | -0.90034 |
| 16.6 | 497 | 1 | -0.72148 | -0.90704 | -1.27723 |
| | | 2 | -0.85561 | -1.01993 | -1.51351 |
| 16.6 | 498 | 1 | -0.29472 | -0.28139 | -0.60304 |
| | | 2 | -0.65846 | -0.55966 | -0.69138 |

### Exposure of Chimpanzees to mCyd

Limited HPLC analyses were perfomed to determine the concentration of mCyd attained in the sera of chimpanzees following dosing with val-mCyd. In sera drawn 1 to 2 hours post dose on days 2 and 5 of dosing, mCyd levels were typically between 2.9 and 12.1 µM (750 and 3100 ng/mL, respectively) in treated animals. No mCyd was detected in pretreatment sera or in the placebo control sera. Within 24 hours of the final dose, serum levels of mCyd had fallen to 0.2 to 0.4 µM (50 and 100 ng/mL, respectively). No mUrd was detected in any sera samples although the methodology used has a lower limit of quantification of 0.4 µM (100 ng/mL) for mUrd.

### Safety of mCyd in the Chimpanzee Model of Chronic HCV Infection

Chimpanzees were monitored by trained veterinarians throughout the study for weight loss, temperature, appetite, and general well being, as well as for blood chemistry profile and CBCs. No adverse events due to drug were noted. The drug appeared to be well tolerated by all four treated animals. All five animals lost some weight during the study and showed some aspartate aminotransferase (AST)elevations, but these are normal occurrences related to sedation procedures used, rather than study drug. A single animal experienced an alanine aminotransferase (ALT) flare in the pretreatment period prior to the start of dosing, but the ALT levels diminished during treatment. Thus, this isolated ALT event was not attributable to drug.

### EXAMPLE 37: IN VITRO METABOLISM

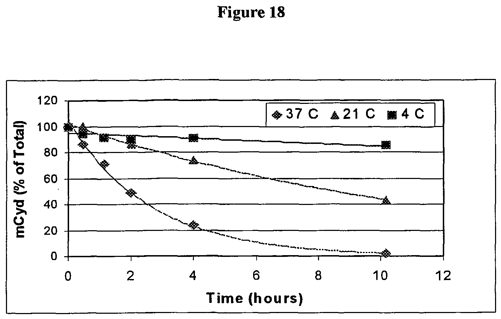

Studies were conducted to determine the stability of val-mCyd and mCyd in human plasma. Val-mCyd was incubated in human plasma at 0, 21 or 37°C and samples analyzed at various time points up to 10 hours (Figure 18). At 37°C, val-mCyd was effectively converted to mCyd, with only 2% of the input val-mCyd remaining after 10 hours. The *in vitro* half-life of val-mCyd in human plasma at 37°C was 1.81 hours. In studies of the *in vitro* stability of mCyd in human plasma, or upon treatment with a crude preparation enriched in human cytidine/deoxycytidine deaminase enzymes, mCyd remained essentially unchanged and no deamination to the uridine derivative of mCyd (mUrd) occurred after incubation at 37°C. Only in rhesus and cynomologus monkey plasma was limited deamination observed. Incubation of mCyd at 37°C in cynomologus monkey plasma yielded 6.7 and 13.0% of mUrd deamination product after 24 and 48 hours, respectively, under conditions where control cytidine analogs were extensively deaminated.

In addition to the TP derivatives of mCyd and mUrd, minor amounts of mCyd-5'-diphosphate, mCyd-DP, roughly 10% the amount of the corresponding TP, were seen in all three cell types. Lesser amounts of mUrd-DP were detected only in two cell types (primary human hepatocytes and MDBK cells). No monophosphate (MP) metabolites were detected in any cell type. There was no trace of any intracellular mUrd and no evidence for the formation of liponucleotide metabolites such as the 5'-diphosphocholine species seen upon the cellular metabolism of other cytidine analogs.

Figure 19 shows the decay profiles of mCyd-TP determined following exposure of HepG2 cells to 10 µM [³H]-mCyd for 24 hours. The apparent intracellular half-life of the mCyd-TP was 13.9 ± 2.2 hours in HepG2 cells and 7.6 ± 0.6 hours in MDBK cells: the data were not suitable for calculating the half life of mUrd-TP. The long half life of mCyd-TP in human hepatoma cells supports the notion of once-a-day dosing for val-mCyd in clinical trials for HCV therapy. Phosphorylation of mCyd occurred in a dose-dependent manner up to 50 µM drug in all three cell types, as shown for HepG2 cells in Figure 19C. Other than the specific differences noted above, the phosphorylation pattern detected in primary human hepatocytes was qualitatively similar to that obtained using HepG2 or MDBK cells.

### Contribution of m Urd

In addition to the intracellular active moiety, mCyd-TP, cells from different species have been shown to produce variable and lesser amounts of a second triphosphate, mUrd-TP, via deamination of intracellular mCyd species. The activity of mUrd-TP against BVDV NS5B RNA-dependent RNA polymerase has not been tested to date but is planned. To date, data from exploratory cell culture studies on the antiviral efficacy and cytotoxicity of mUrd suggest that mUrd (a) is about 10-fold less potent than mCyd against BVDV; (b) has essentially no antiviral activity against a wide spectrum of other viruses; and (c) is negative when tested at high concentrations in a variety of cytotoxicity tests (including bone marrow assays, mitochondrial function assays and incorporation into cellular nucleic acid). Based on these results, it appears that the contribution of mUrd to the overall antiviral activity or cytotoxicity profile of mCyd is likely to be minor. Extensive toxicology coverage for the mUrd metabolite of mCyd exists from subchronic studies conducted with val-mCyd in the monkey.

### EXAMPLE 38: CELLULAR PATHWAYS FOR METABOLIC ACTIVATION

The nature of the enzyme responsible for the phosphorylation of mCyd was investigated in substrate competition experiments. Cytidine (Cyd) is a natural substrate of cytosolic uridine-cytidine kinase (UCK), the pyrimidine salvage enzyme responsible for conversion of Cyd to Cyd-5'-monophosphate (CMP). The intracellular phosphorylation of mCyd to mCyd-TP was reduced in the presence of cytidine or uridine in a dose-dependent fashion with EC₅₀ values of 19.17 ± 4.67 µM for cytidine and 20.92 ± 7.10 µM for uridine. In contrast, deoxycytidine, a substrate for the enzyme deoxycytidine kinase (dCK), had little effect on the formation of mCyd-TP with an EC₅₀ > 100 µM. The inhibition of mCyd phosphorylation by both cytidine and uridine, but not deoxycytidine, suggests that mCyd is phosphorylated by the pyrimidine salvage enzyme, uridine-cytidine kinase (Van Rompay, A. R., A. Norda, et al. Mol Pharmacol 2001 59(5): 1181-6). Further studies are required to confirm the proposed role of this kinase in the activation of mCyd.

### EXAMPLE 39: PATHWAYS FOR THE CELLULAR BIOSYNTHESIS OF MURD-TP

As outlined above, mUrd-TP is a minor metabolite arising to varying extents in cells from different species. mUrd does not originate via extracellular deamination of mCyd since mUrd is not seen in the cell medium which also lacks any deamination activities. The cellular metabolism data are consistent with the idea that mUrd-TP arises via the biotransformation of intracellular mCyd species. Consideration of the known ribonucleoside metabolic pathways suggests that the most likely routes involve deamination of one of two mCyd species by two distinct deamination enzymes: either mCyd-MP by a cytidylate deaminase (such as deoxycytidylate deaminase, dCMPD), or of mCyd by cytidine deaminase (CD). Further phosphorylation steps lead to mUrd-TP. These possibilities are under further investigation.

### EXAMPLE 40: CLINICAL EVALUATION OF VAL-MCYD

Patients who met eligibility criteria were randomized into the study at Baseline (Day 1), the first day of study drug administration. Each dosing cohort was 12 patients, randomized in a 10:2 ratio to treatment with drug or matching placebo. Patients visited the study center for protocol evaluations on Days 1, 2, 4, 8, 11, and 15. After Day 15, study drug was stopped. Thereafter, patients attended follow-up visits on Days 16, 17, 22, and 29. Pharmacokinetic sampling was performed on the first and last days of treatment (Day 1 and Day 15) on all patients, under fasting conditions.

The antiviral effect of val-mCyd was assessed by (i) the proportion of patients with a ≥ 1.0 log₁₀ decrease from baseline in HCV RNA level at Day 15, (ii) the time to a ≥ 1.0 log₁₀ decrease in serum HCV RNA level, (iii) the change in HCV RNA level from Day 1 to Day 15, (iv) the change in HCV RNA level from Day 1 to Day 29, (v) the proportion of patients who experience return to baseline in serum HCV RNA level by Day 29, and (vi) the relationship of val-mCyd dose to HCV RNA change from Day 1 to Day 15.

### Clinical Pharmacokinetics of mCyd after Oral Administration of Escalating Doses of Val-mCyd

Pharmacokinetics were evaluated over a period of 8 h after the first dose on day 1 and after the last dose on day 15, with 24-h trough levels monitored on days 2, 4, 8, 11 and 16, and a 48-h trough on day 17. Plasma concentrations of mCyd, mUrd and Val-mCyd were measured by a HPLC/MS/MS methodology with a lower limit of quantitation (LOQ) at 20 ng/ml.

The pharmacokinetics of mCyd was analyzed using a non-compartmental approach. As presented in the tables below, the principal pharmacokinetic parameters were comparable on day 1 and day 15, indicative of no plasma drug accumulation after repeated dosing. The plasma exposure also appears to be a linear function of dose. As shown in the tables below, principal pharmacokinetic parameters of drug exposure (Cmax and AUC) doubled as doses escalated from 50 to 100 mg.

**Table 35: Pharmacokinetic parameters of mCyd at 50 mg**

| Parameters | Cₘₐₓ | Tₘₐₓ | AUC_{0-inf} | t_{1/2} |
|---|---|---|---|---|
| | (ng/ml) | (h) | (ng/ml×h) | (h) |
| Day 1 | | | | |
| Mean | 428.1 | 2.5 | 3118.7 | 4.1 |
| SD | 175.5 | 1.1 | 1246.4 | 0.6 |
| CV % | 41.0 | 43.2 | 40.0 | 13.8 |
| | | | | |
| Day 15 | | | | |
| Mean | 362.7 | 2.2 | 3168.4 | 4.6 |
| SD | 165.7 | 1.0 | 1714.8 | 1.3 |
| CV % | 45.7 | 46.9 | 54.1 | 28.6 |

**Table 36: Pharmacokinetic parameters of mCyd at 100 mg**

| Parameters | Cₘₐₓ | Tₘₐₓ | AUC_{0-inf} | t_{1/2} |
|---|---|---|---|---|
| | (ng/ml) | (h) | (ng/ml×h) | (h) |
| Day 1 | | | | |
| Mean | 982.1 | 2.6 | 6901.7 | 4.4 |
| SD | 453.2 | 1.0 | 2445.7 | 1.1 |
| CV % | 46.1 | 36.2 | 35.4 | 25.2 |
| | | | | |
| Day 15 | | | | |
| Mean | 1054.7 | 2.0 | 7667.5 | 4.2 |
| SD | 181.0 | 0.0 | 1391.5 | 0.5 |
| CV % | 17.2 | 0.0 | 18.1 | 11.7 |

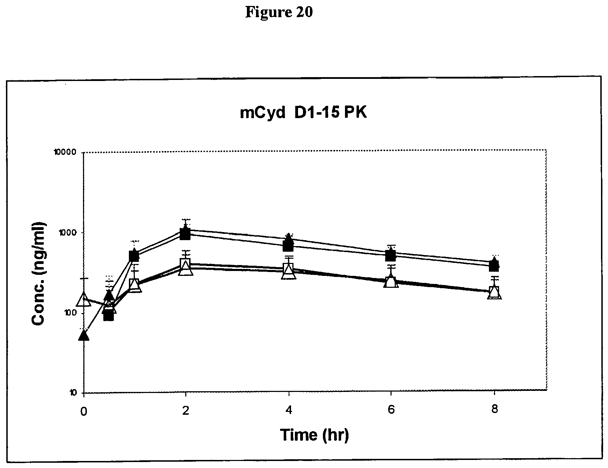

The mean day 1 and day 15 plasma kinetic profiles of mCyd at 50 and 100 mg are depicted in the Figure 20.

In summary, following oral administration of val-mCyd, the parent compound mCyd was detectable in the plasma of HCV-infected subjects. mCyd exhibits linear plasma pharmacokinetics in these subjects across the two dose levels thus far examined. There was no apparent accumulation of mCyd in subjects' plasma following 15 days of daily dosing at the doses thus far examined.

### Antiviral Activity of mCyd after Oral Administration of Escalating Doses of Vial-mCyd Starting at 50 mg/day for 15 Days in HCV-Infected Patients

Serum HCV RNA level were determined with the use of the Amplicor HCV Monitor™ assay v2.0 (Roche Molecular Systems, Branchburg, NJ, USA), which utilizes polymerase chain reaction (PCR) methods. The lower limit of quantification (LLOQ) with this assay was estimated to be approximately 600 IU/mL and the upper limit of quantification (ULOQ) with this assay was estimated to be approximately 500,000 IU/mL.

Serum samples for HCV RNA were obtained at screening (Day -42 to -7) to determine eligibility for the study. The Screening serum HCV RNA values must be ≥ 5 log₁₀ IU/mL by the Amplicor HBV Monitor™ assay at the central study laboratory.

During the study period, serum samples for HCV RNA were obtained at Baseline (Day 1), and at every protocol-stipulated post-Baseline study visit (Days 2, 4, 8, 11, 15, 16, 17, 22, and 29). Serum samples for HCV RNA were also collected during protocol-stipulated follow-up visits for patients prematurely discontinued from the study.

The antiviral activity associated with the first two cohorts (50 and 100 mg per day) in the ongoing study is summarized in the following tables and graphs. Although the duration of dosing was short (15 days) and the initial dose levels low, there were already apparent effects on the levels of HCV RNA in the plasma of infected patients.

**Table 37: Summary Statistics of HCV RNA in log₁₀ Scale**

| | | | | | | | | | | | | |
|---|---|---|---|---|---|---|---|---|---|---|---|---|
| | | **Day** | | | | | | | | | | |
| | | **-1** | **1** | **2** | **4** | **8** | **11** | **15** | **16** | **17** | **22** | **29** |
| **Treatment** | | 6 | 5 | 5 | 4 | 4 | 4 | 4 | 4 | 3 | 4 | 3 |
| **Placebo** | **N** | | | | | | | | | | | |
| | **Median** | 6.45 | 6.25 | 6.25 | 6.52 | 6.42 | 6.28 | 6.58 | 6.51 | 6.64 | 6.35 | 6.61 |
| | **Mean** | 6.45 | 6.28 | 6.40 | 6.48 | 6.36 | 6.34 | 6.54 | 6.52 | 6.50 | 6.40 | 6.40 |
| | **StdErr** | 0.25 | 0.12 | 0.15 | 0.18 | 0.24 | 0.16 | 0.11 | 0.19 | 0.31 | 0.23 | 0.30 |
| **50 mg** | **N** | 10 | 10 | 10 | 10 | 10 | 10 | 10 | 10 | 10 | 10 | 10 |
| | **Median** | 6.81 | 6.69 | 6.58 | 6.55 | 6.56 | 6.46 | 6.57 | 6.45 | 6.54 | 6.73 | 6.67 |
| | **Mean** | 6.72 | 6.72 | 6.60 | 6.56 | 6.62 | 6.47 | 6.57 | 6.57 | 6.54 | 6.64 | 6.71 |
| | **StdErr** | 0.11 | 0.11 | 0.12 | 0.06 | 0.10 | 0.09 | 0.08 | 0.11 | 0.08 | 0.10 | 0.09 |
| **100 mg** | **N** | 11 | 10 | 10 | 10 | 9 | 10 | 10 | 9 | 9 | 10 | 4 |
| | **Median** | 6.75 | 6.93 | 6.80 | 6.46 | 6.59 | 6.56 | 6.41 | 6.40 | 6.72 | 6.66 | 6.71 |
| | **Mean** | 6.60 | 6.68 | 6.52 | 6.43 | 6.42 | 6.36 | 6.30 | 6.23 | 6.65 | 6.53 | 6.67 |
| | **StdErr** | 0.16 | 0.24 | 0.23 | 0.21 | 0.24 | 0.22 | 0.22 | 0.23 | 0.16 | 0.18 | 0.17 |

**Table 38: Summary Statistics of Change From Baseline (Day 1) in log₁₀ HCV RNA**

| | | | | | | | | | | |
|---|---|---|---|---|---|---|---|---|---|---|
| | | **Day** | | | | | | | | |
| | | **2** | **4** | **8** | **11** | **15** | **16** | **17** | **22** | **29** |
| **Treatment** | | | | | | | | | | |
| **Placebo** | **N** | 5 | 4 | 4 | 4 | 4 | 4 | 3 | 4 | 3 |
| | **Median** | 0.17 | 0.21 | 0.15 | 0.08 | 0.31 | 0.21 | 0.27 | 0.17 | 0.09 |
| | **Mean** | 0.12 | 0.22 | 0.10 | 0.08 | 0.28 | 0.25 | 0.15 | 0.14 | 0.09 |
| | **StdErr** | 0.09 | 0.12 | 0.16 | 0.06 | 0.15 | 0.10 | 0.18 | 0.09 | 0.16 |
| **50 mg** | **N** | 10 | 10 | 10 | 10 | 10 | 10 | 10 | 10 | 10 |
| | **Median** | -0.07 | -0.13 | -0.06 | -0.26 | -0.10 | -0.13 | -0.21 | -0.09 | -0.04 |
| | **Mean** | -0.13 | -0.16 | -0.11 | -0.26 | -0.15 | -0.15 | -0.18 | -0.09 | -0.01 |
| | **StdErr** | 0.05 | 0.07 | 0.05 | 0.06 | 0.08 | 0.05 | 0.07 | 0.06 | 0.10 |
| **100 mg** | **N** | 10 | 10 | 9 | 10 | 10 | 9 | 9 | 10 | 4 |
| | **Median** | -0.12 | -0.24 | -0.20 | -0.28 | -0.43 | -0.49 | -0.24 | -0.19 | -0.12 |
| | **Mean** | -0.16 | -0.25 | -0.21 | -0.32 | -0.38 | -0.39 | -0.18 | -0.15 | 0.13 |
| | **StdErr** | 0.07 | 0.10 | 0.16 | 0.13 | 0.12 | 0.14 | 0.15 | 0.13 | 0.28 |

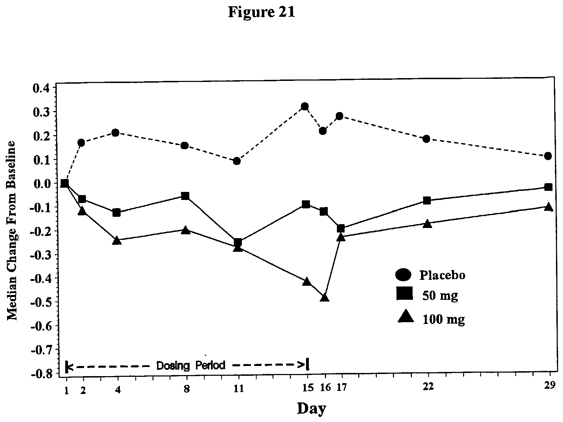

The clinical evaluation of val-mCyd in the tested patients is shown in Figure 21. This figure depicts the median change from baseline in log₁₀ HCV RNA by visit.

### EXAMPLE 41: EVALUATION OF TEST COMPOUNDS

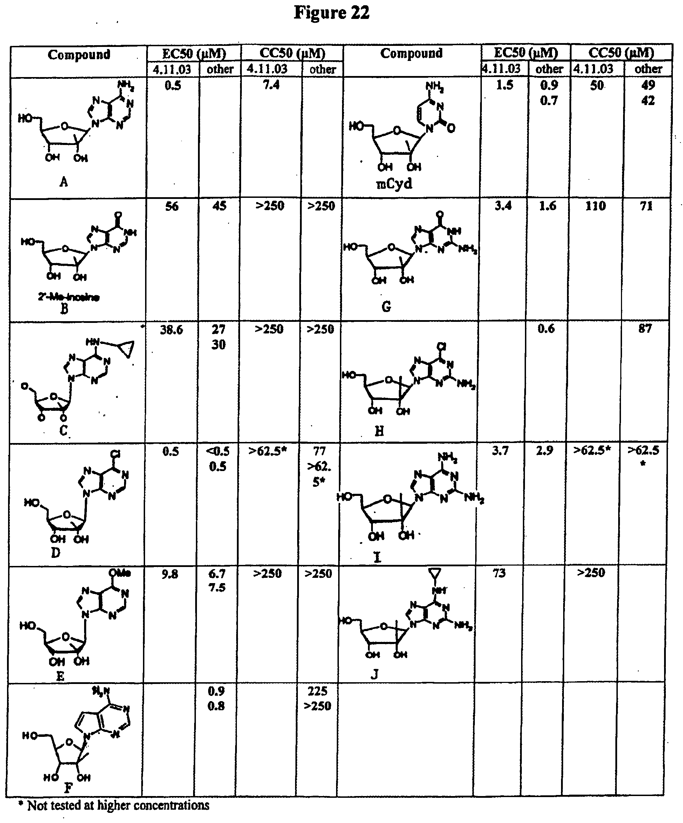

Several of the compounds described herein were tested in the BVDV cell protection assay described above. Figure 22 is a table of the EC₅₀ and CC₅₀ of representative compounds in a BVDV cell protection assay, to show the efficacy of the compounds.

This invention has been described with reference to its preferred embodiments. Variations and modifications of the invention, will be obvious to those skilled in the art from the foregoing detailed description of the invention. It is intended that all of these variations and modifications be included within the scope of this invention.

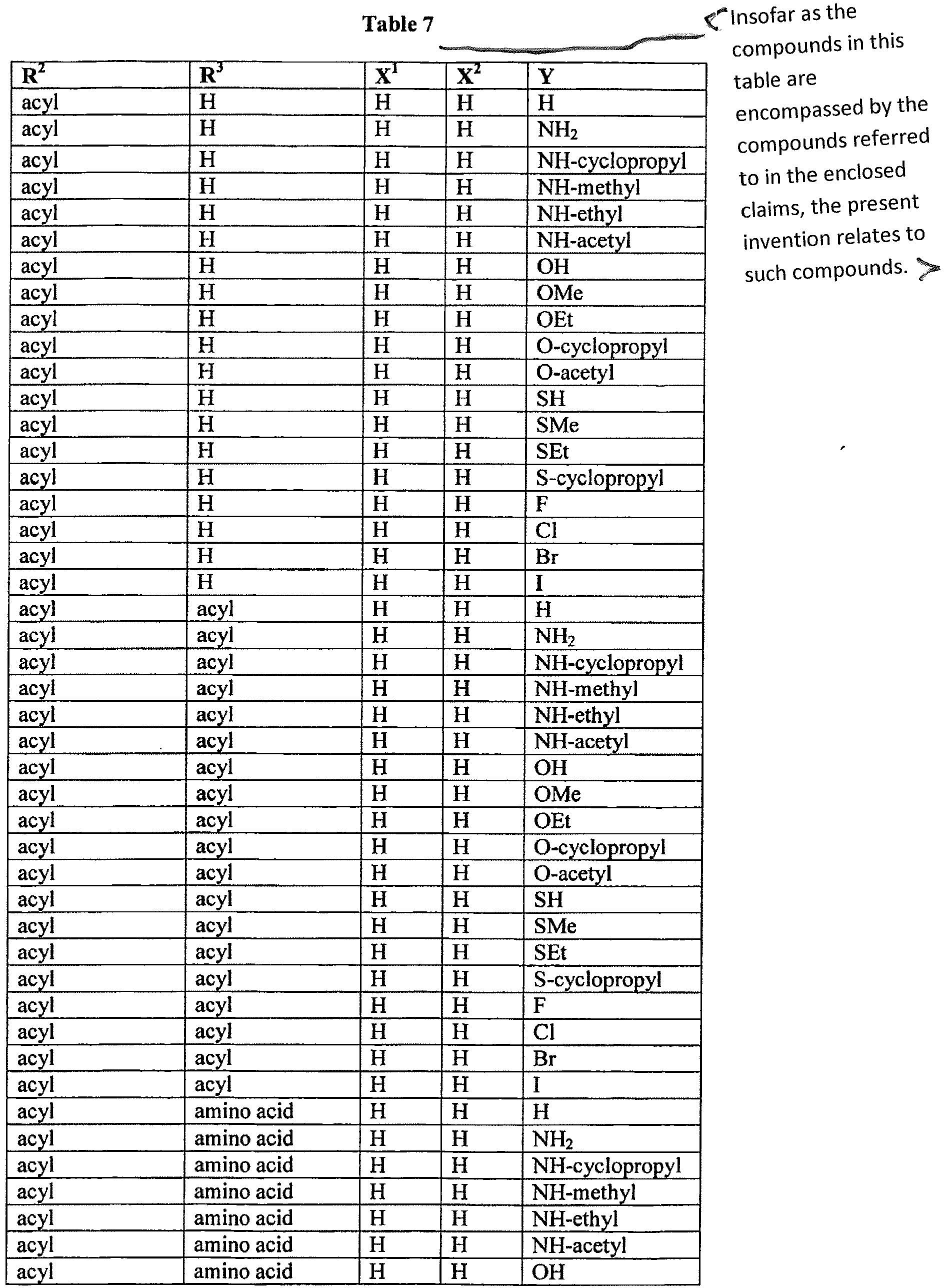

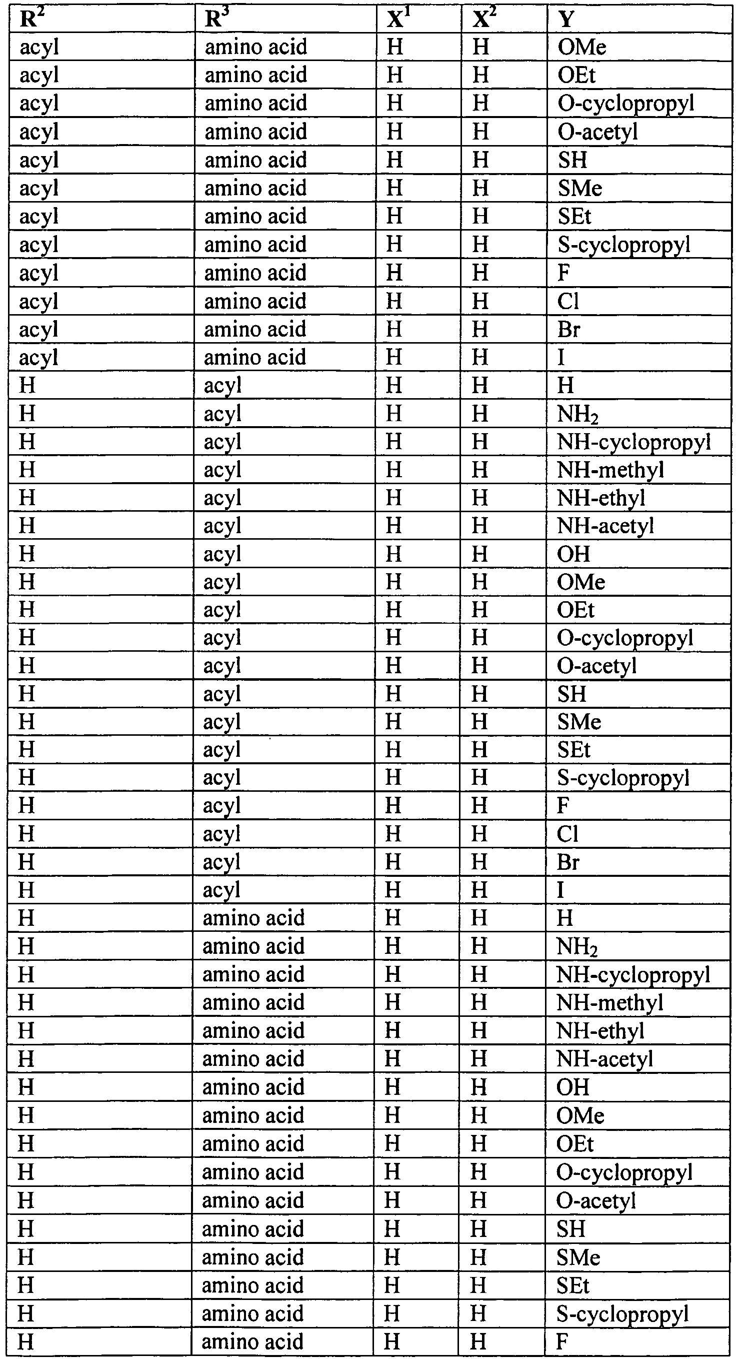

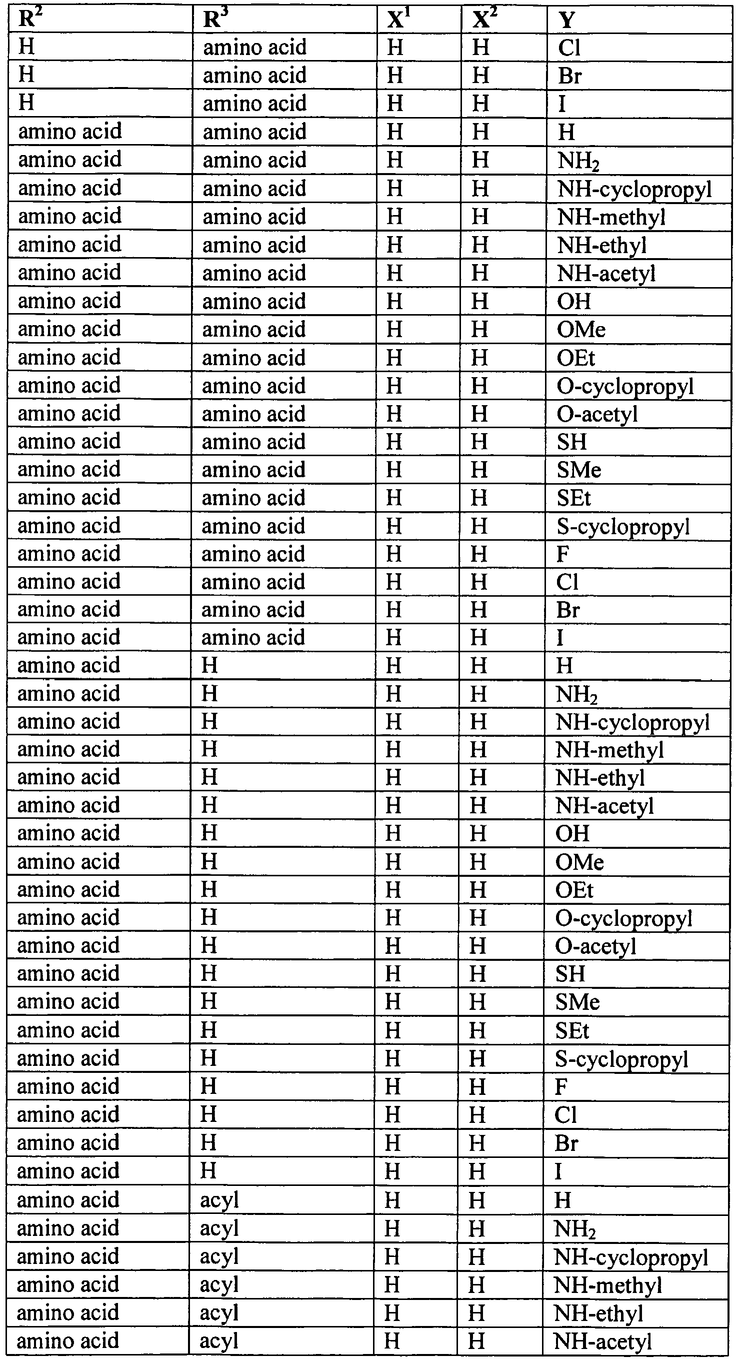

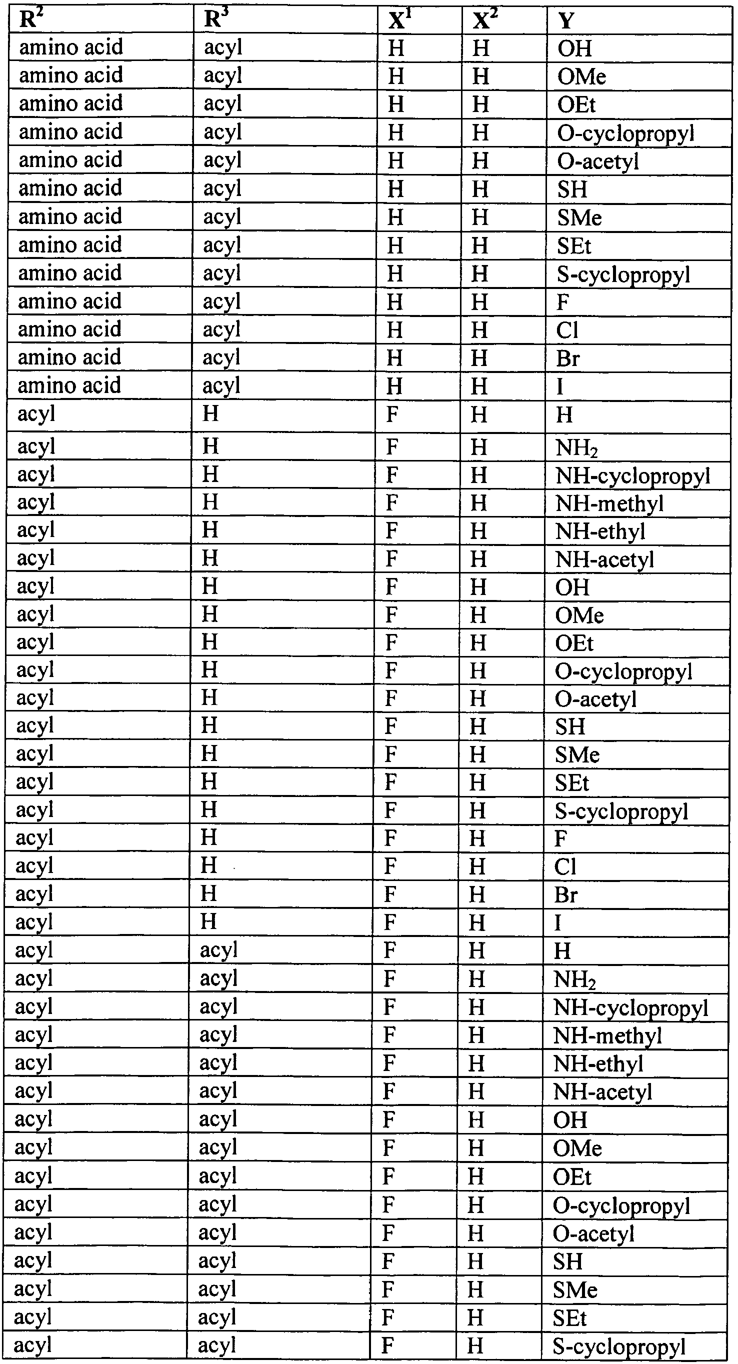

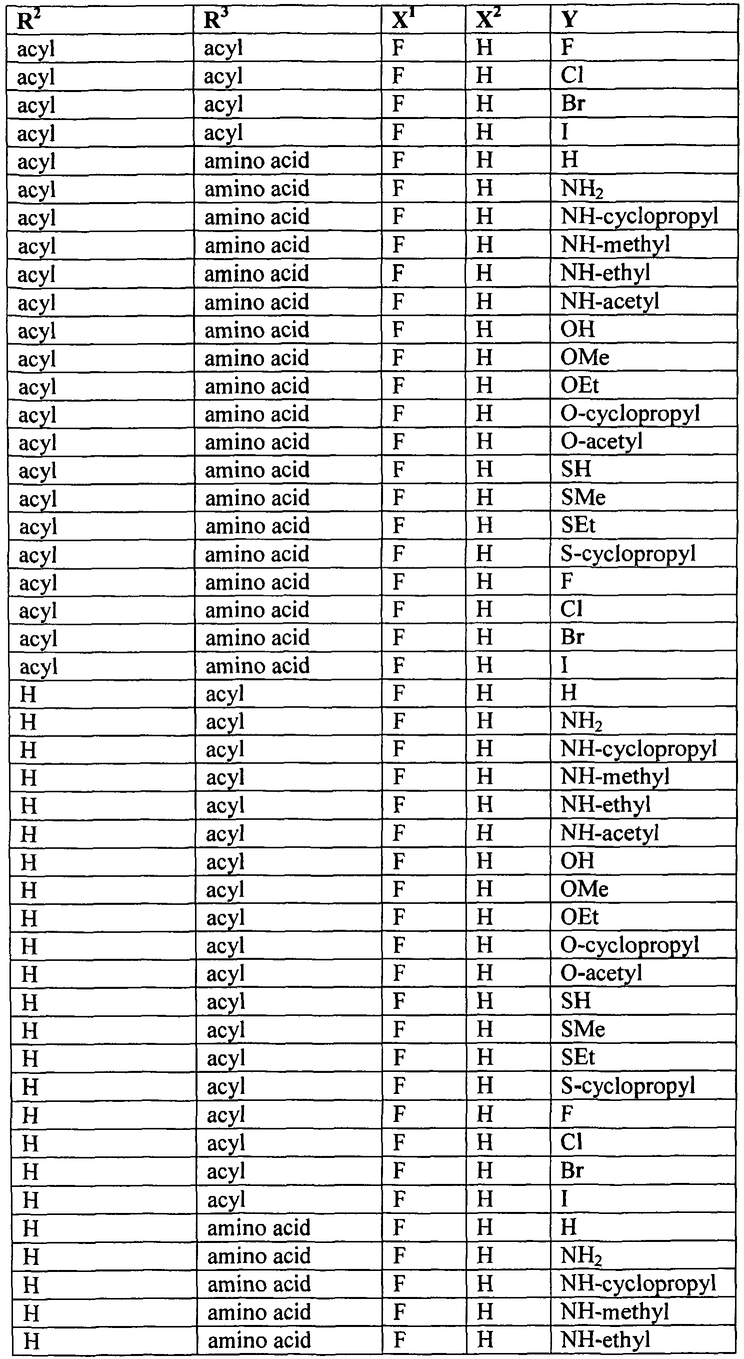

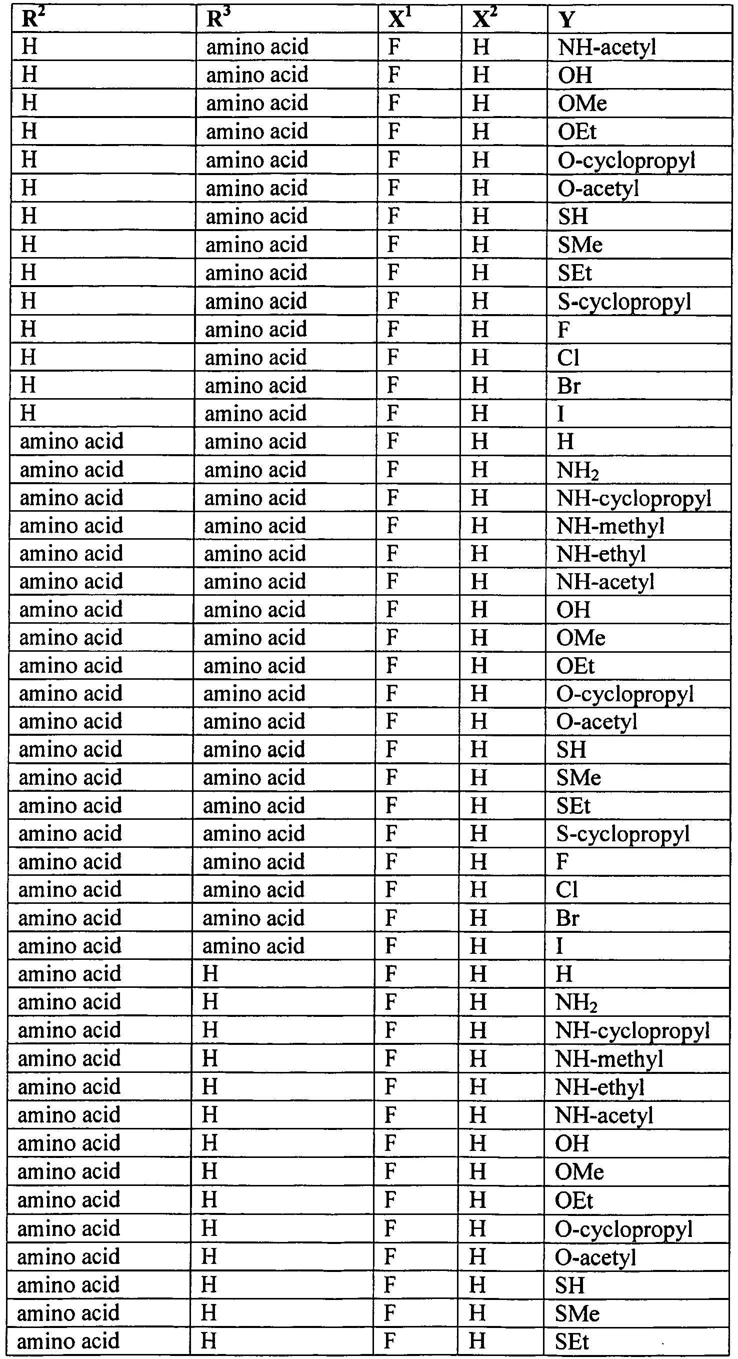

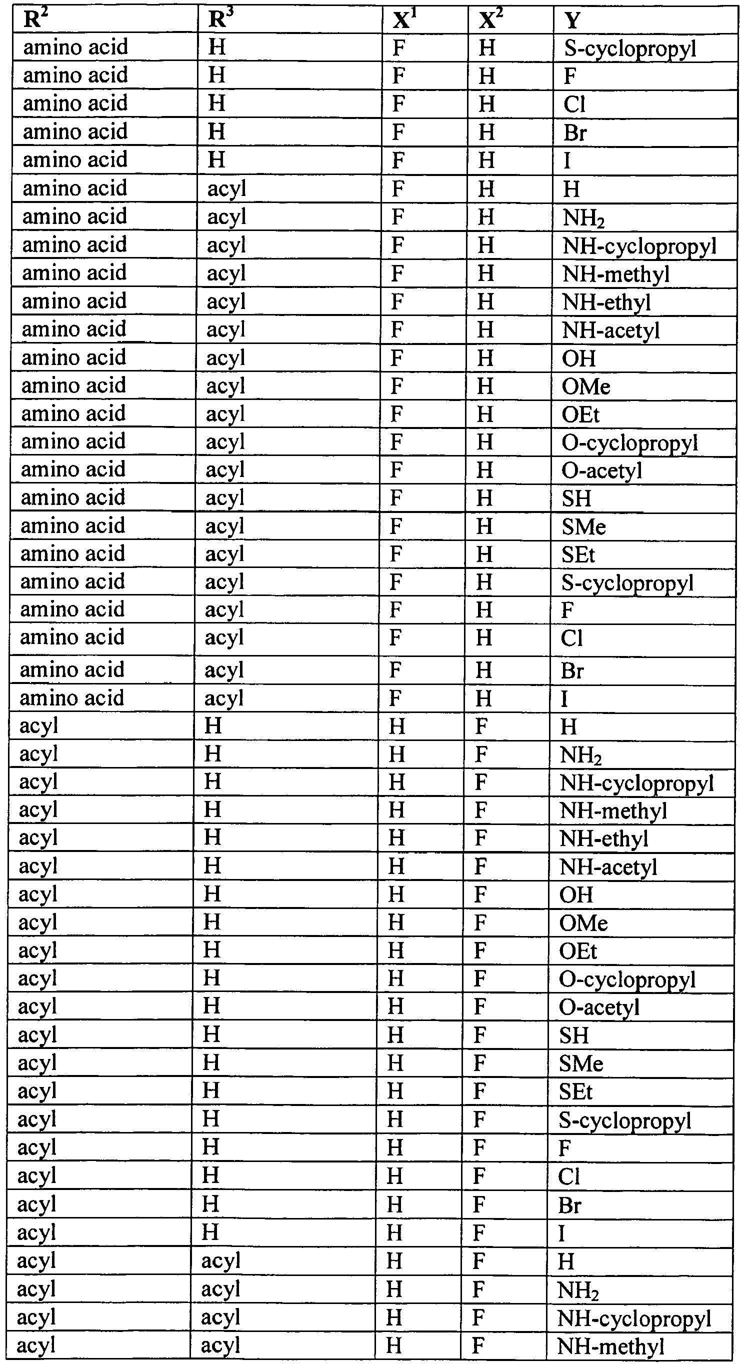

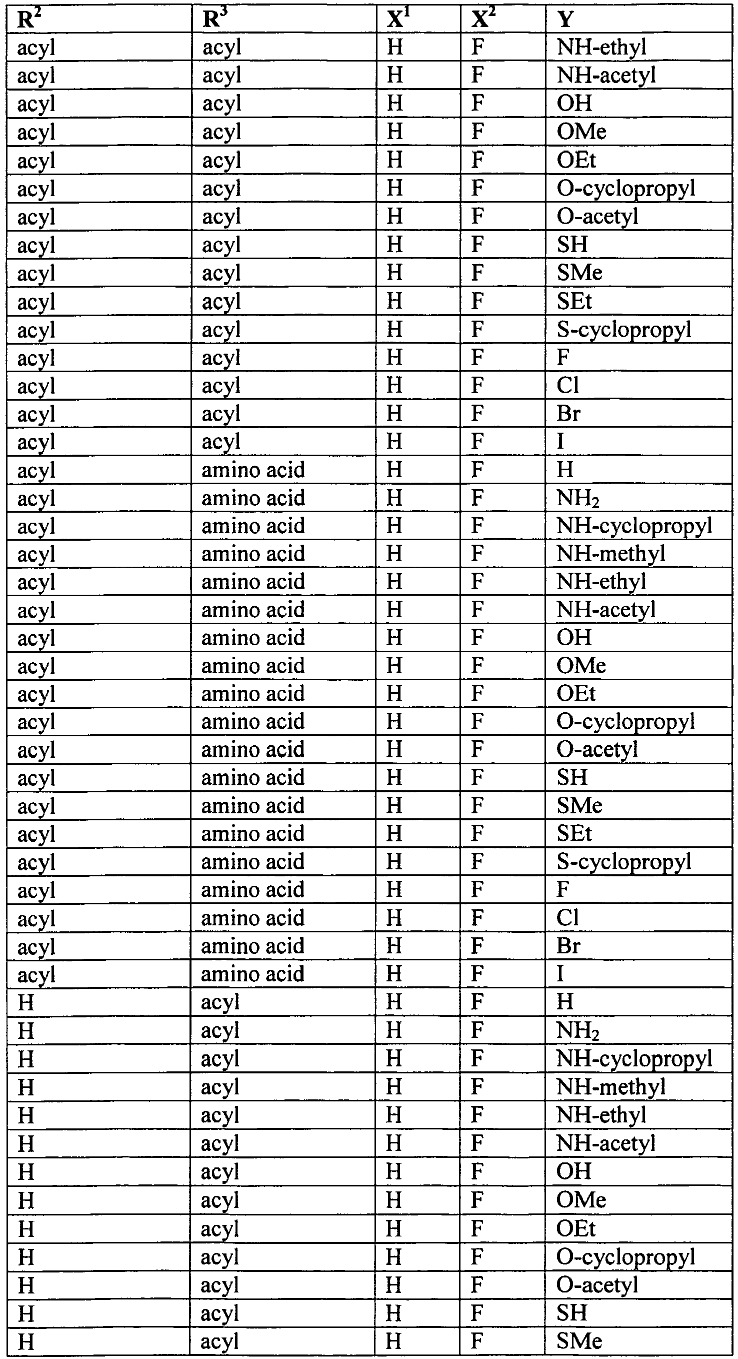

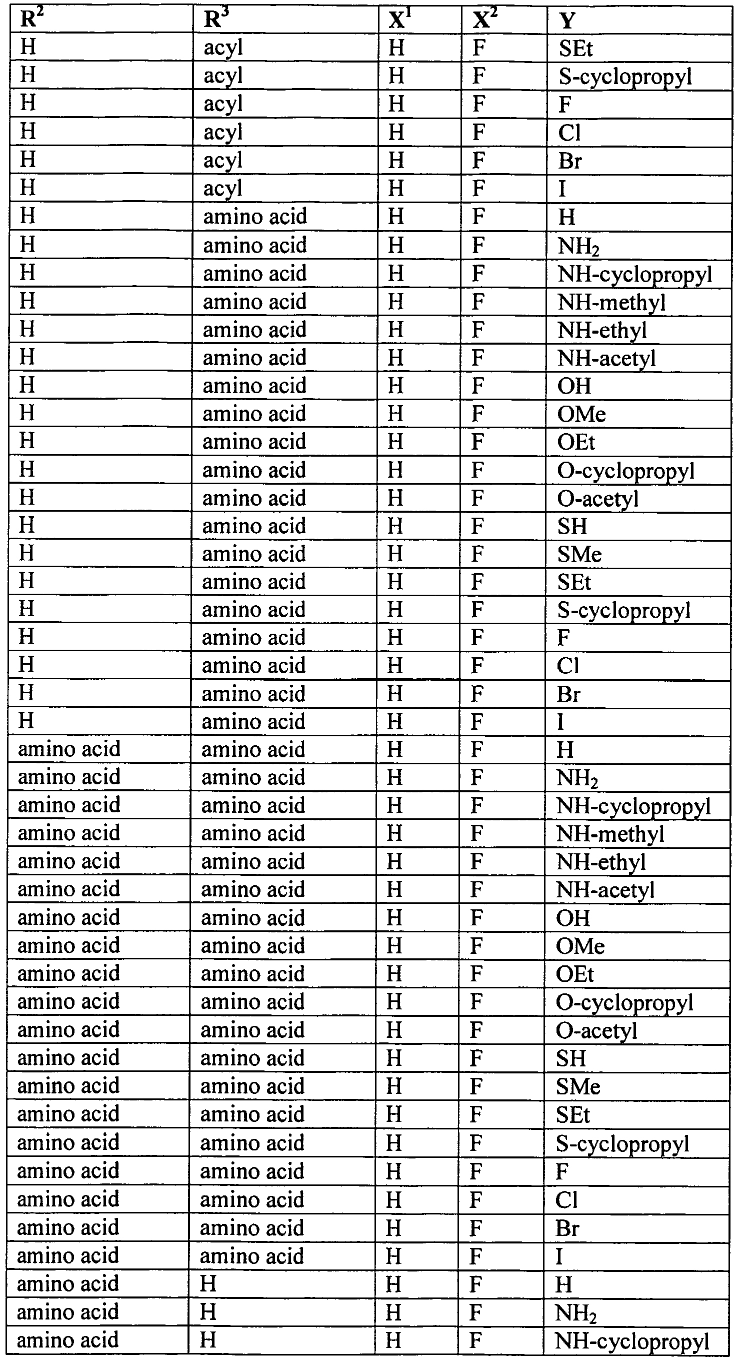

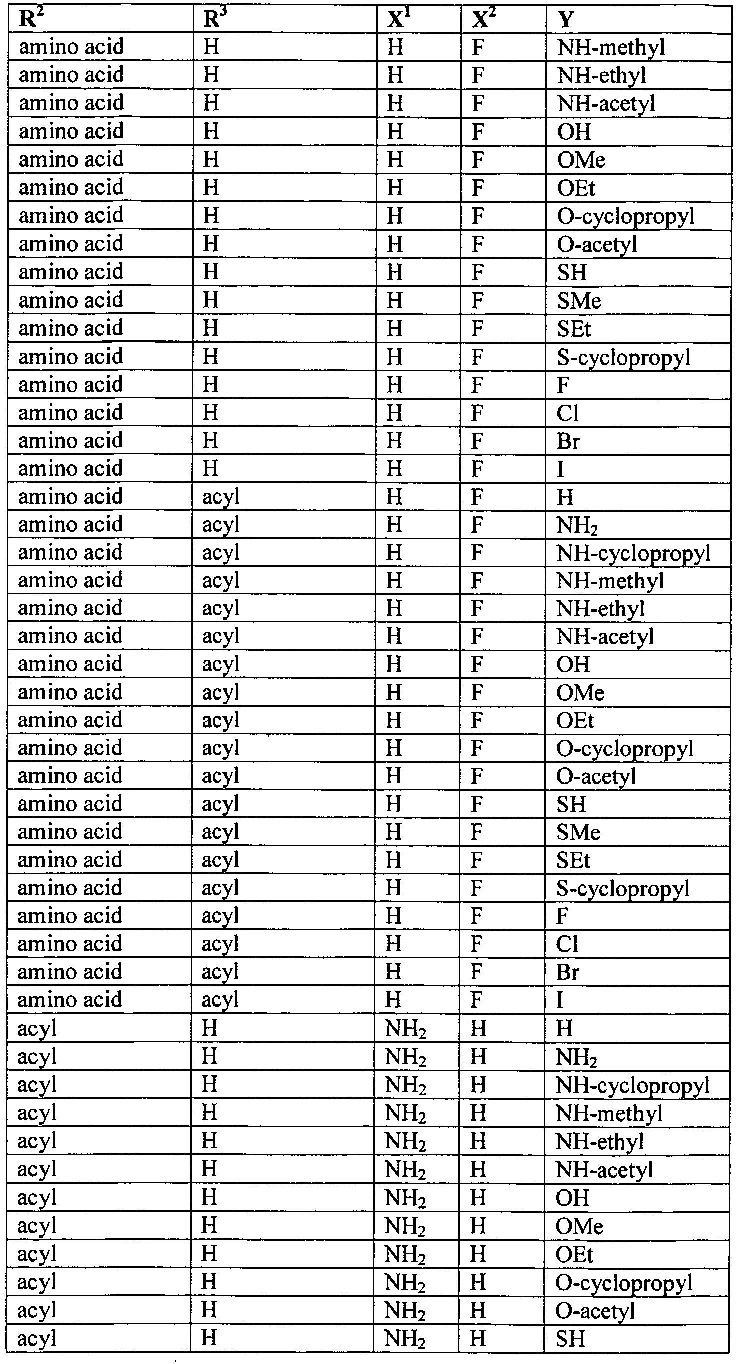

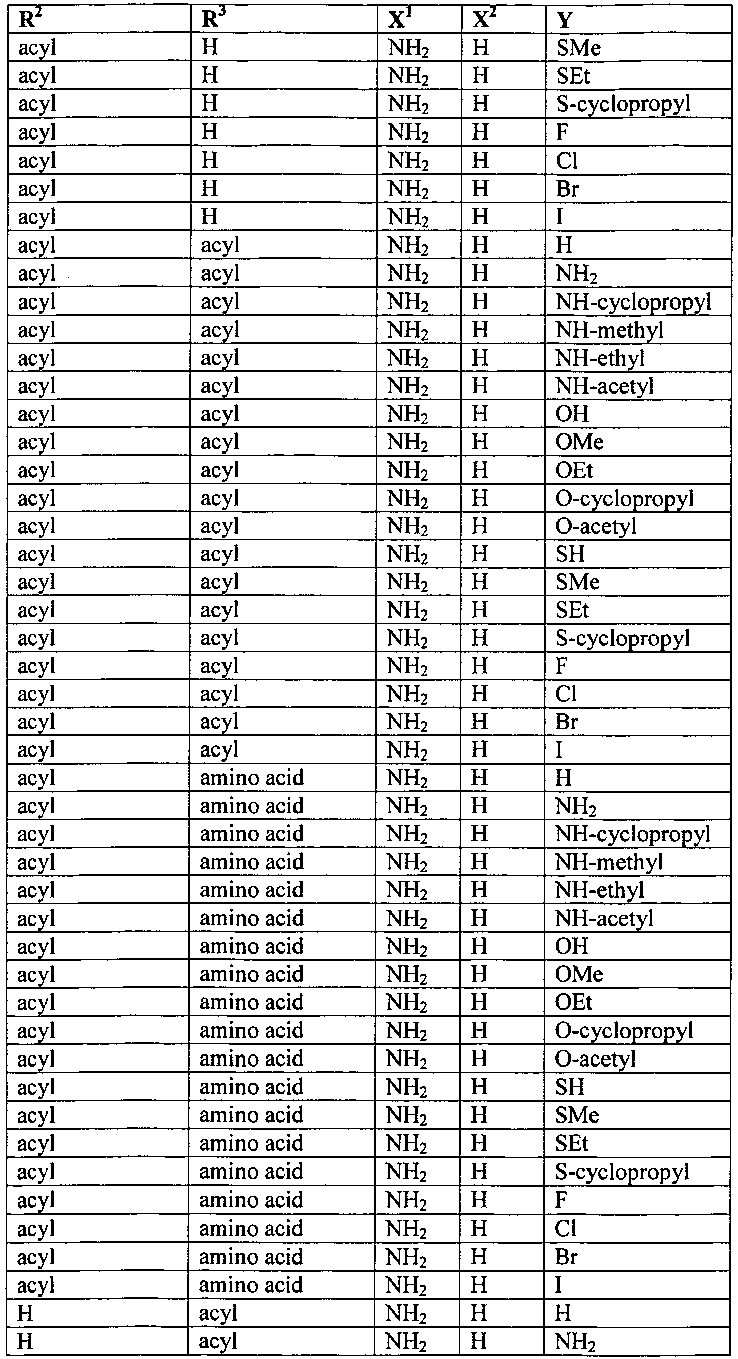

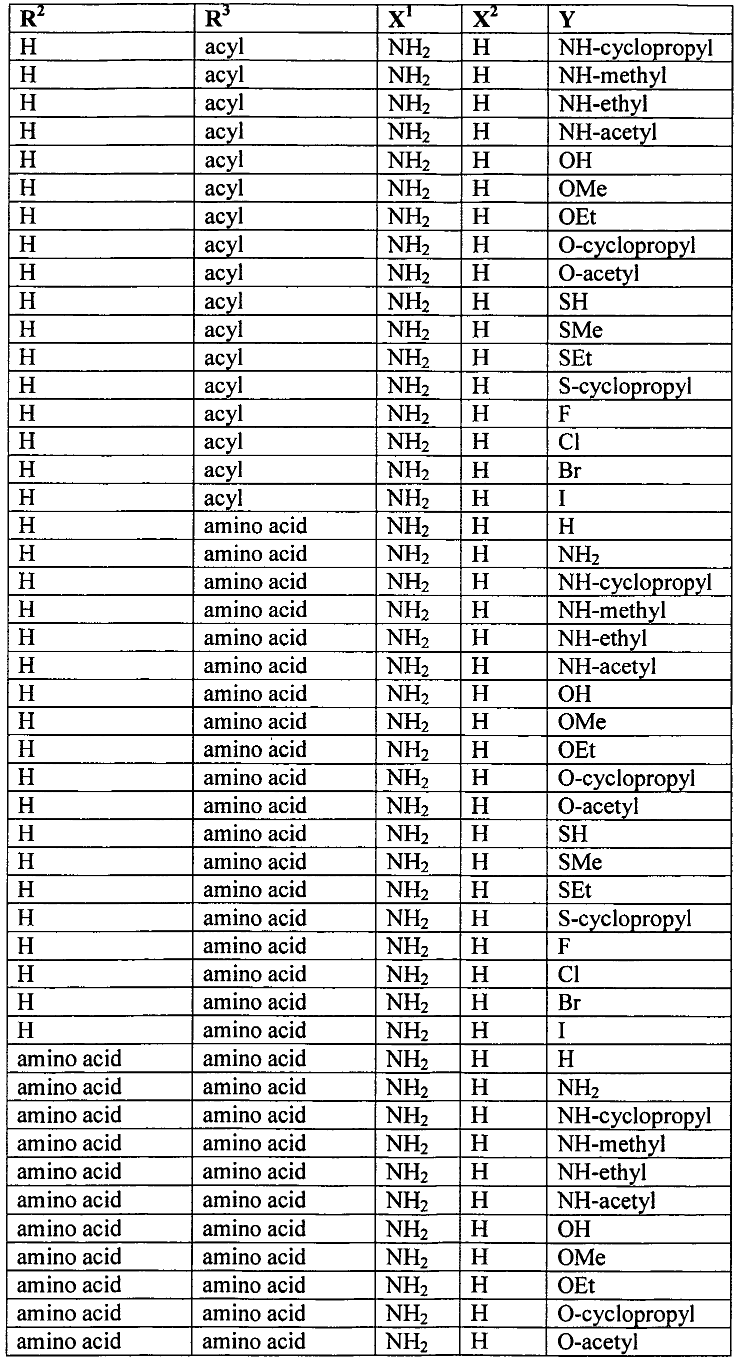

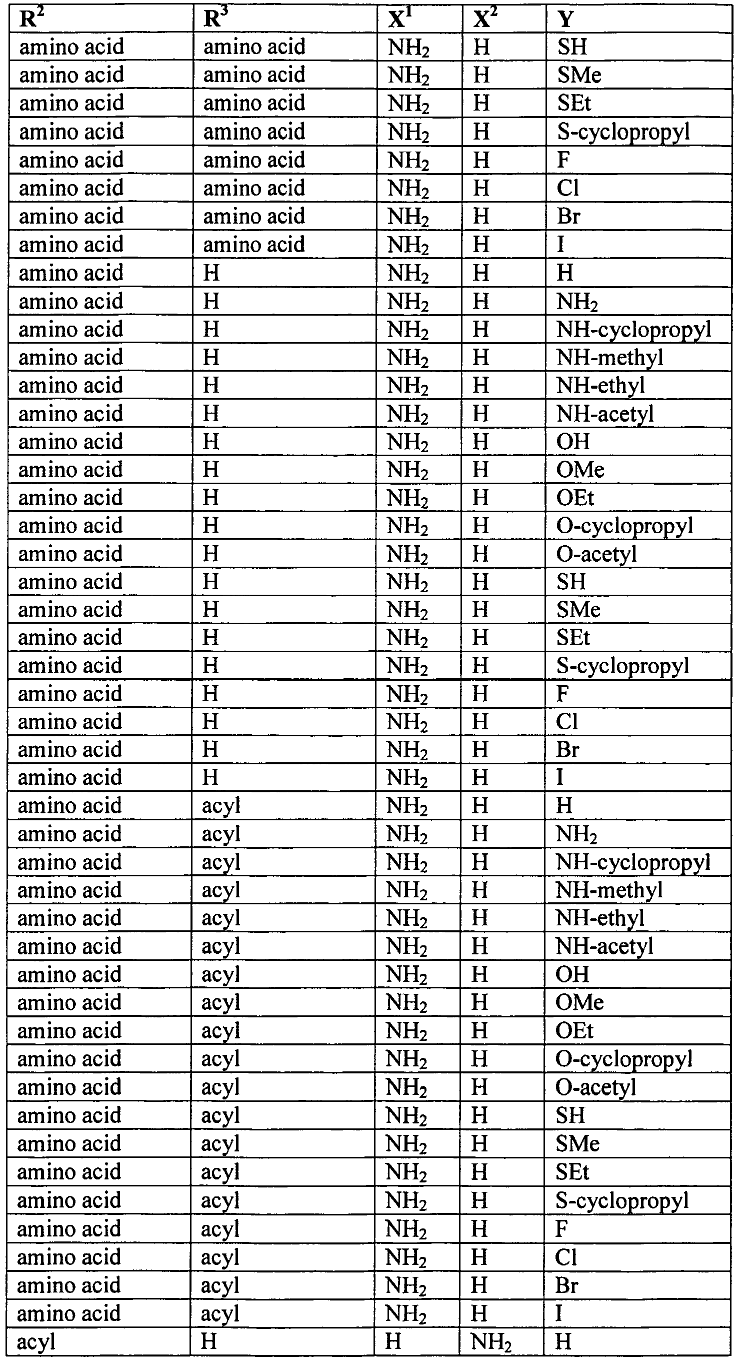

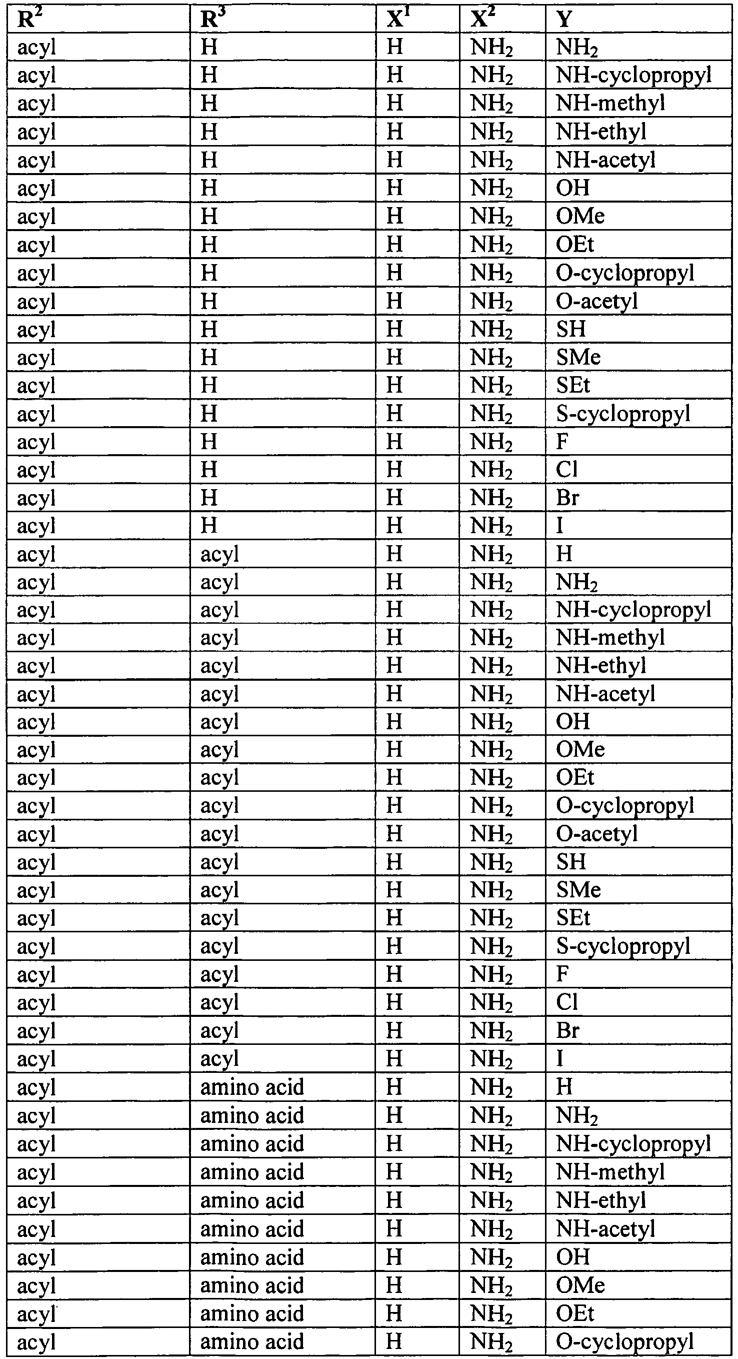

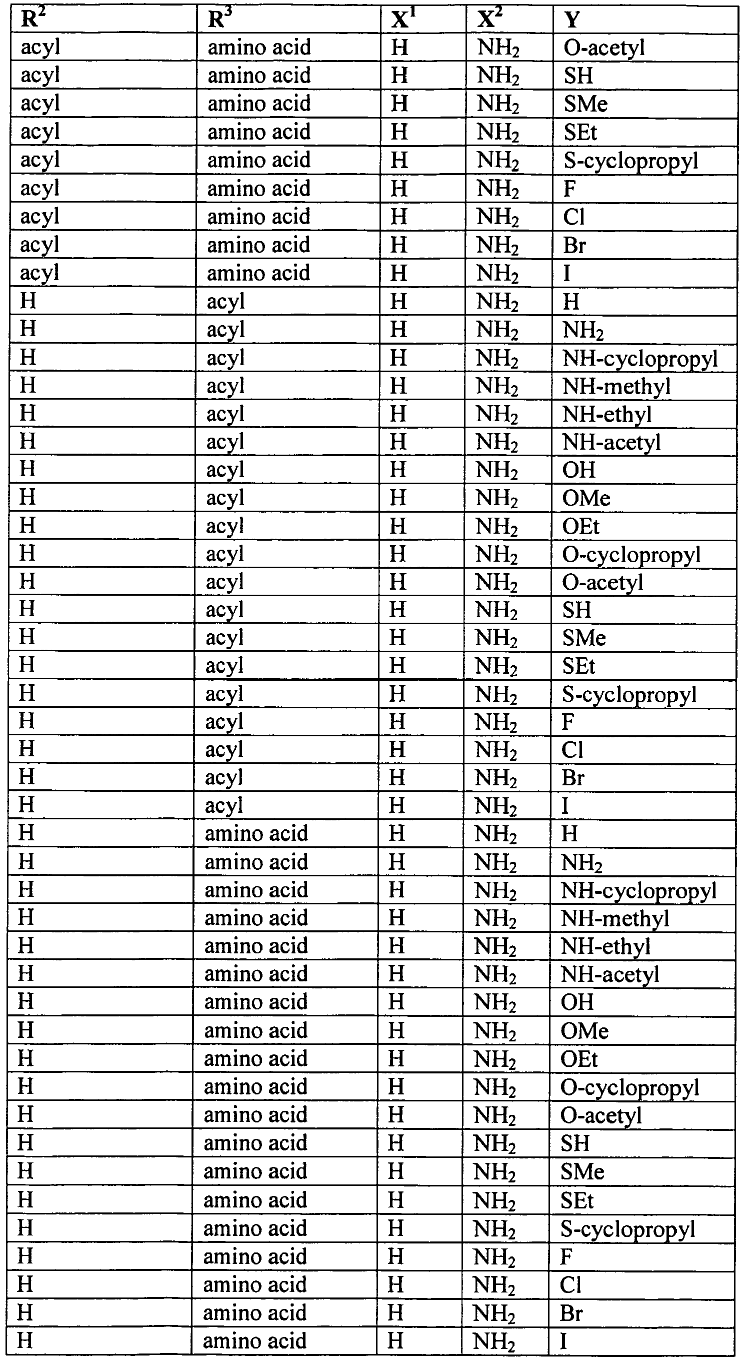

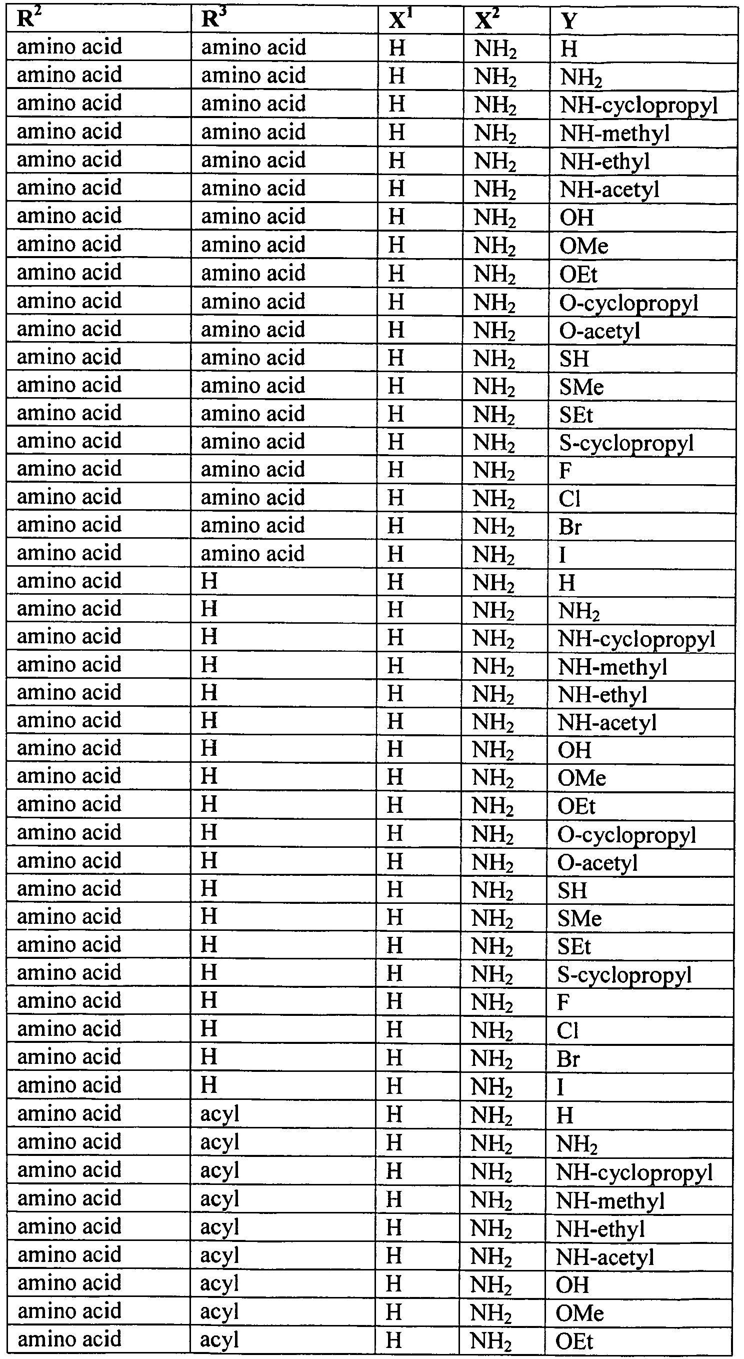

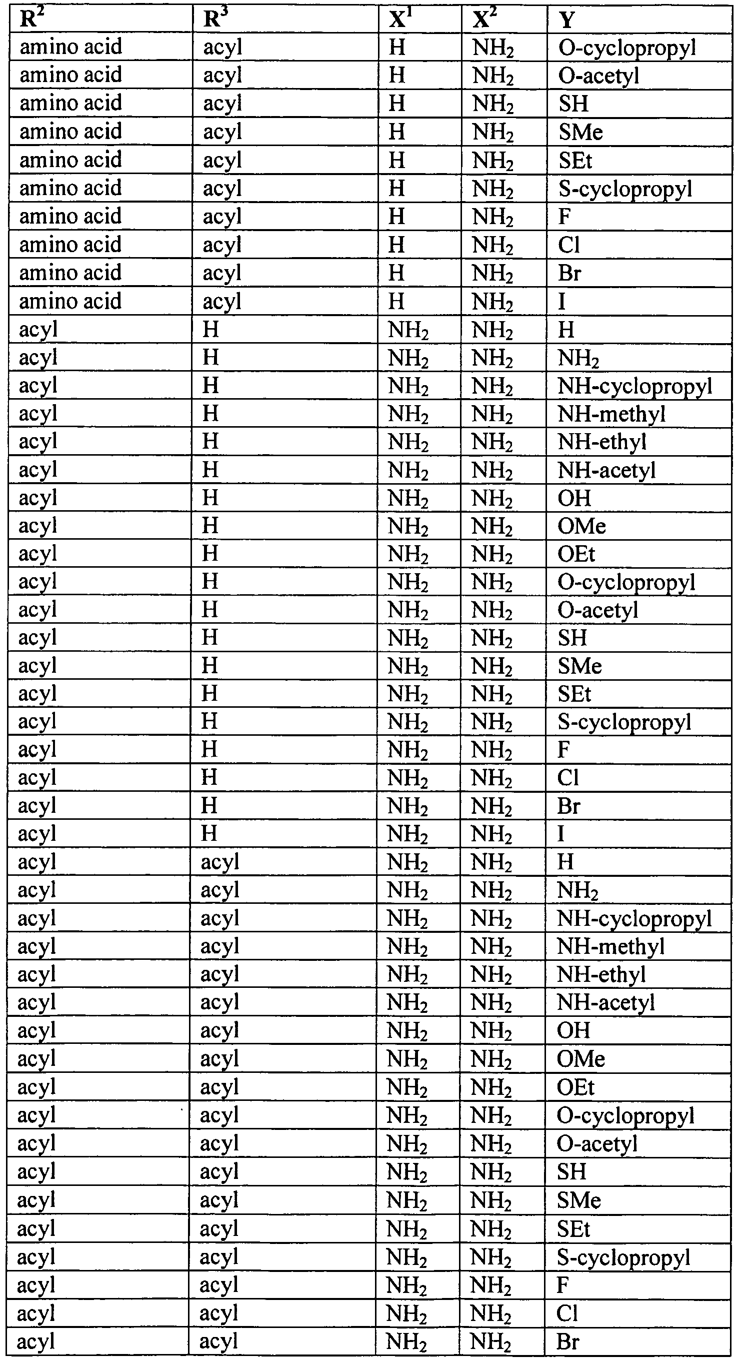

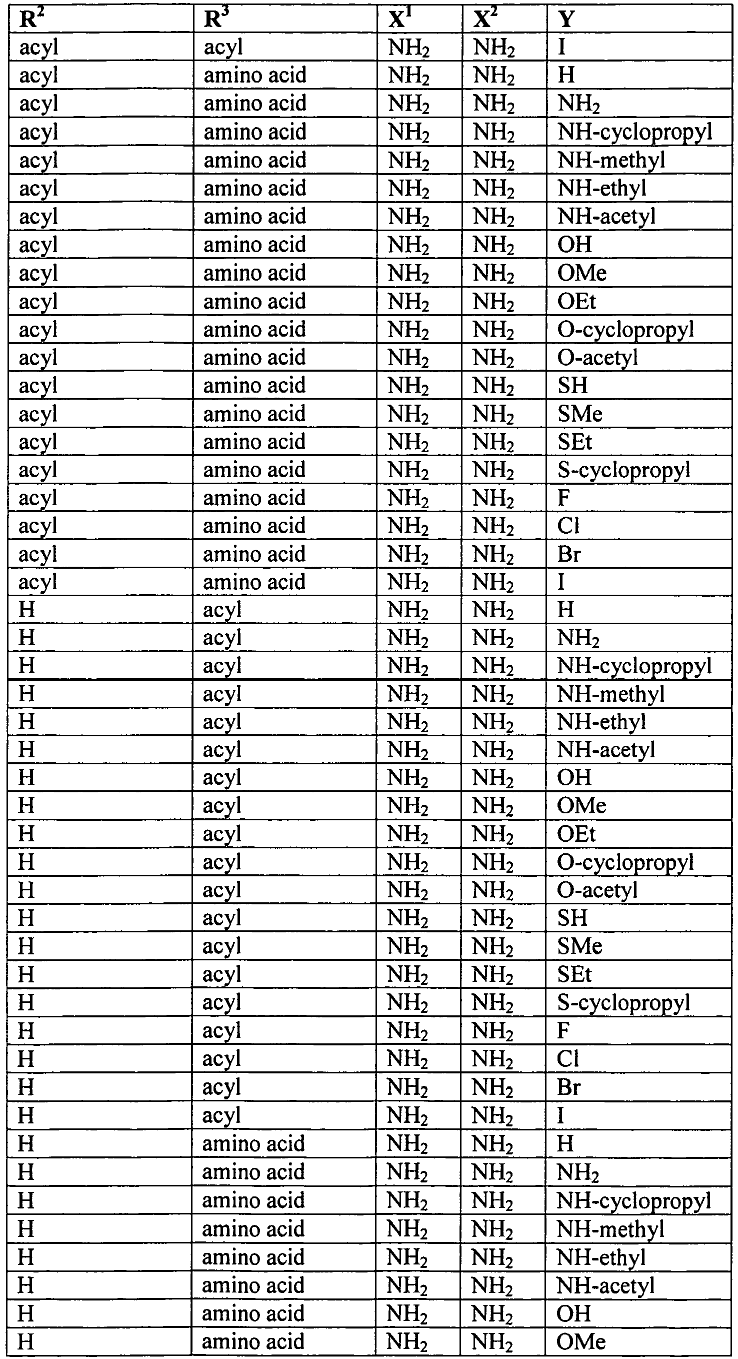

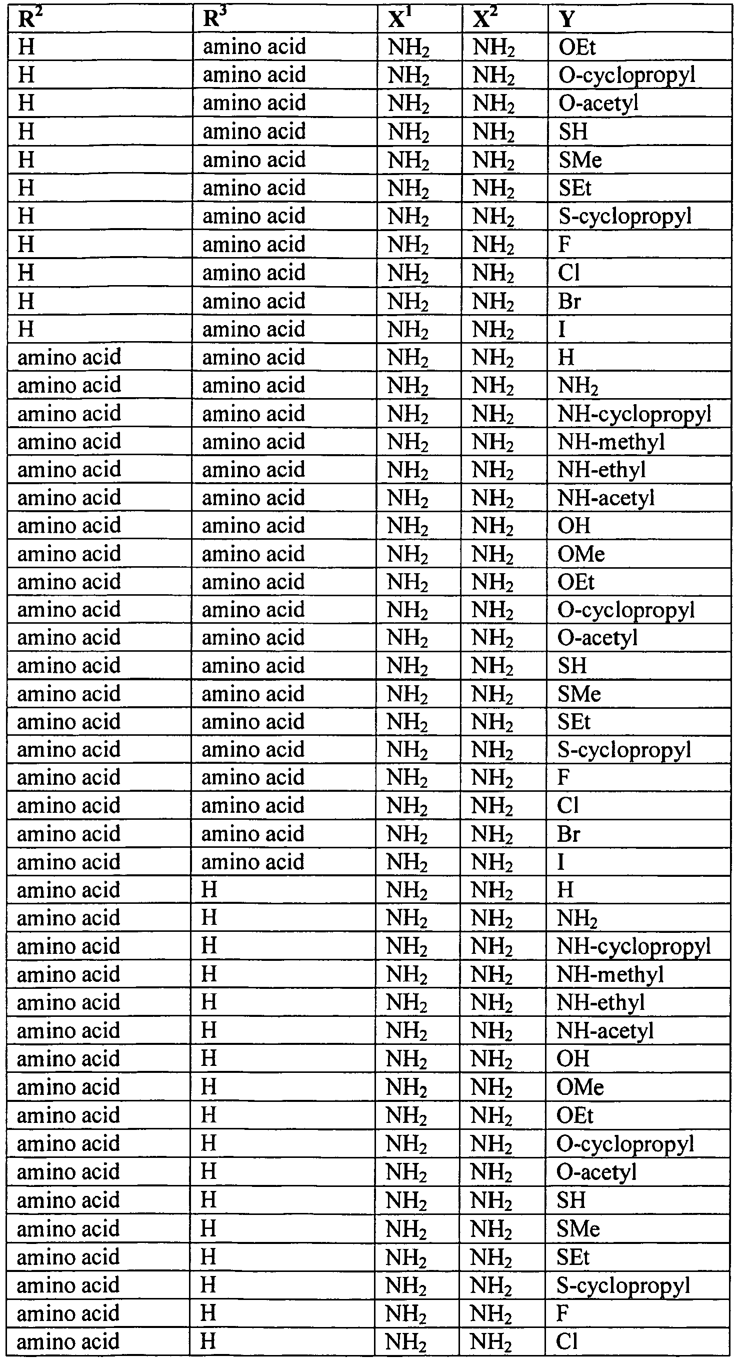

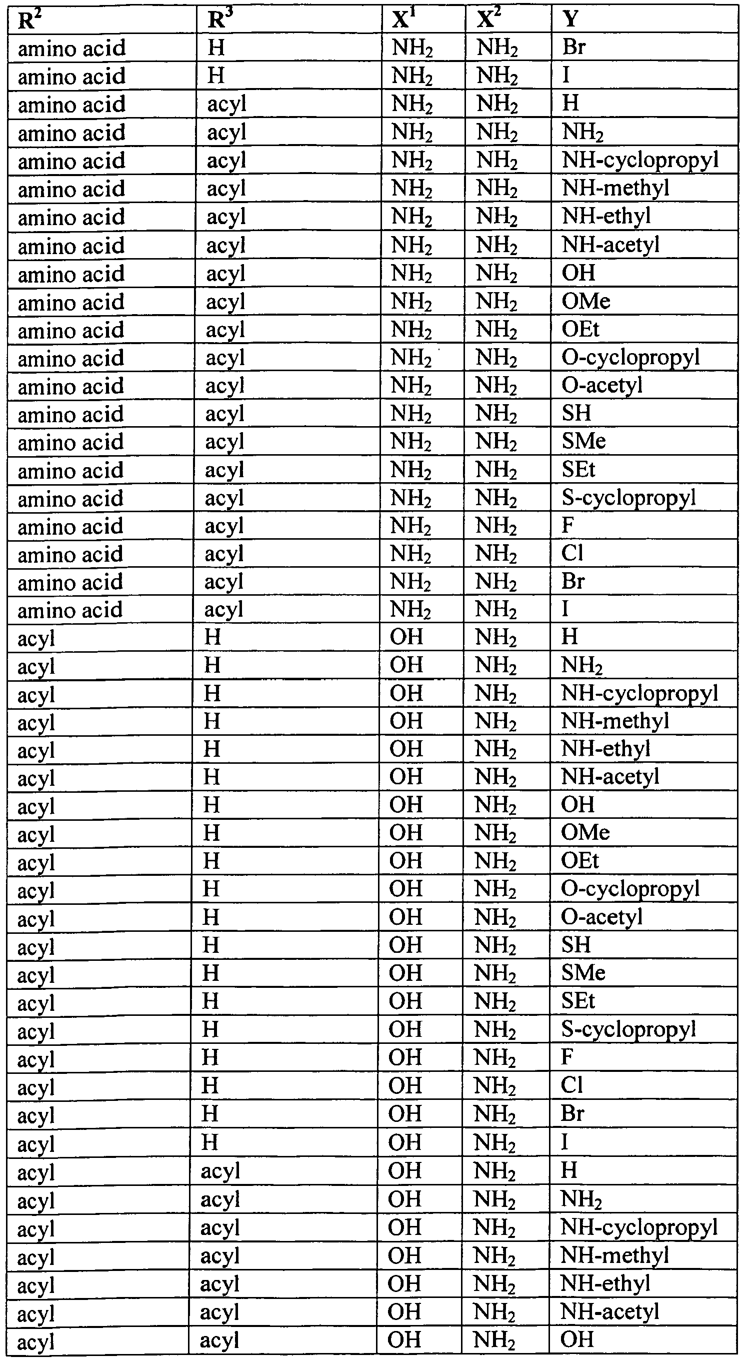

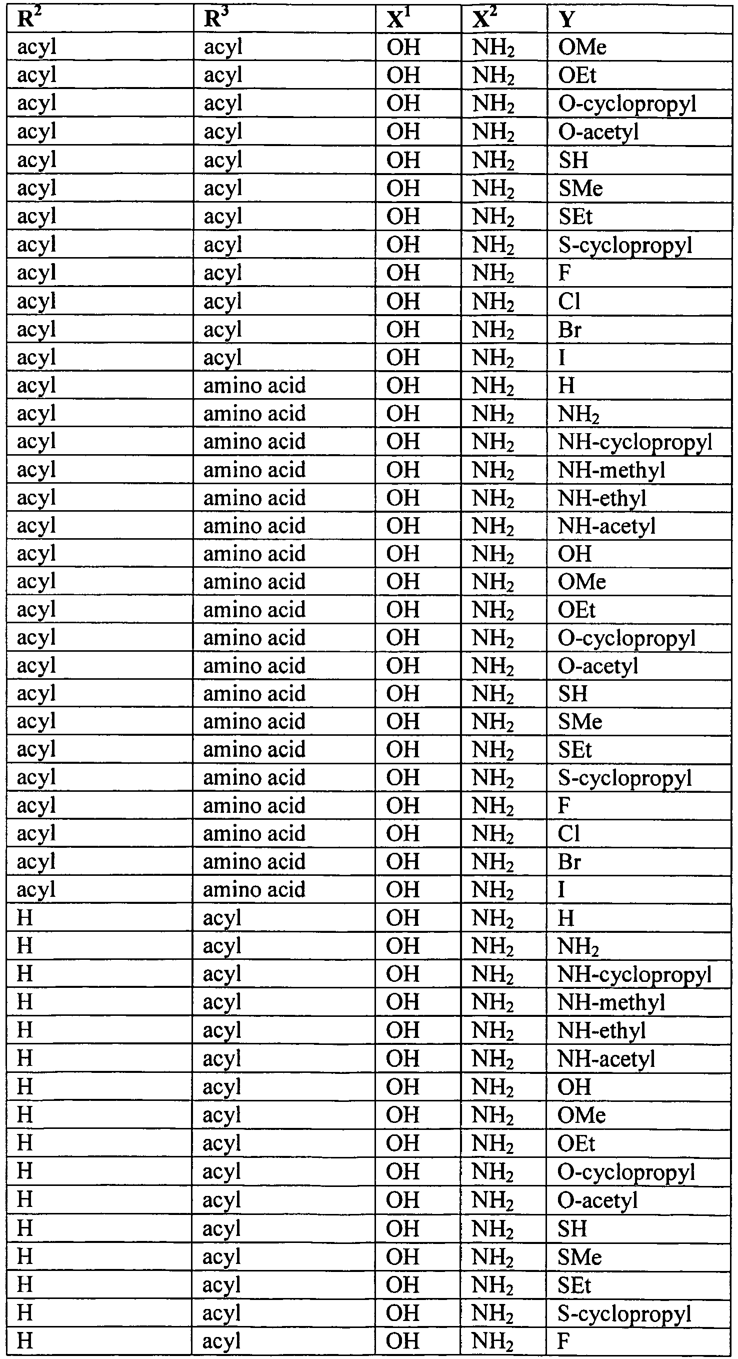

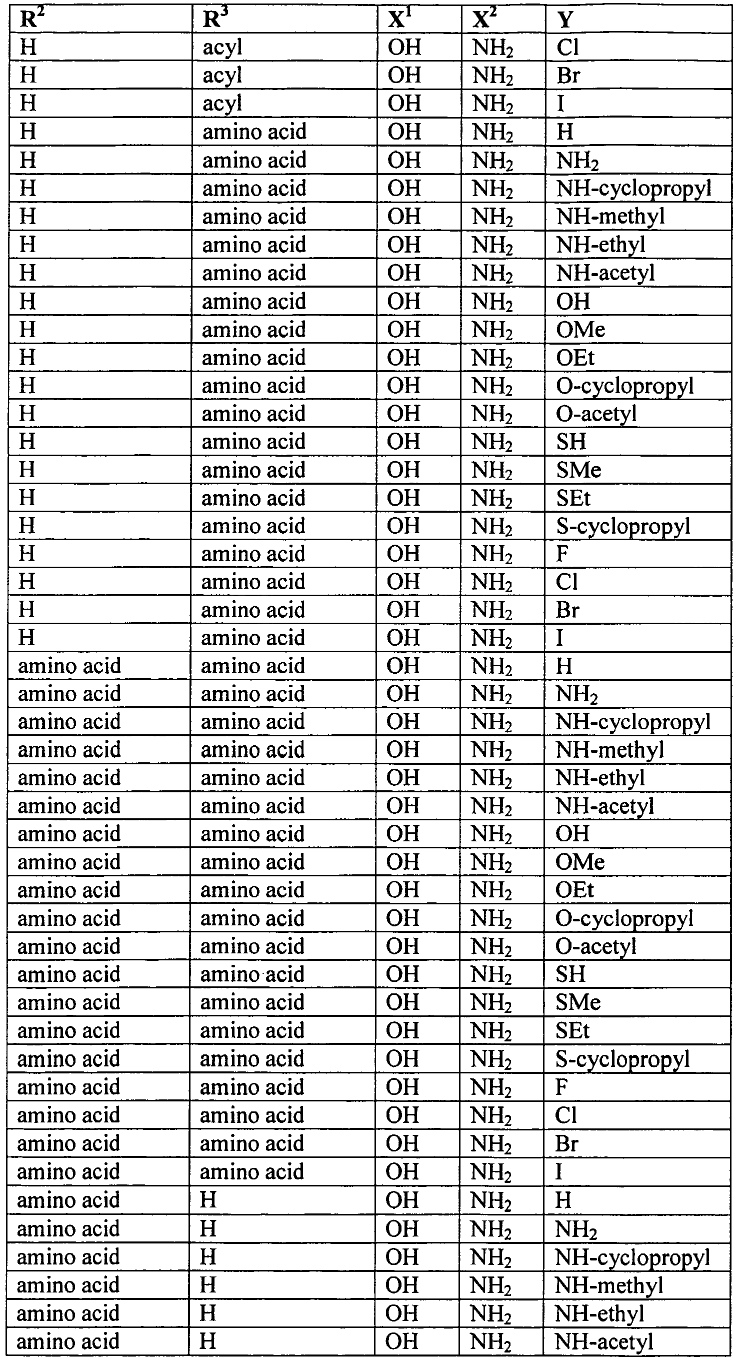

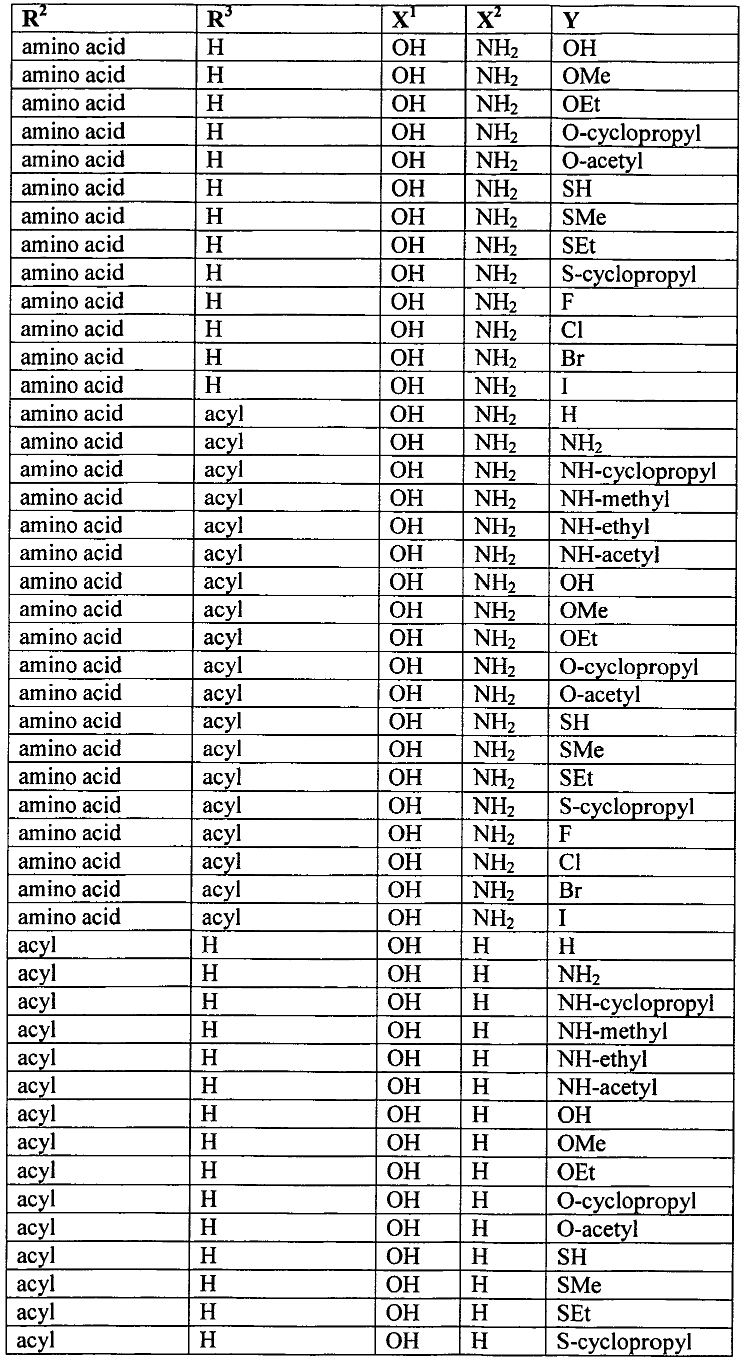

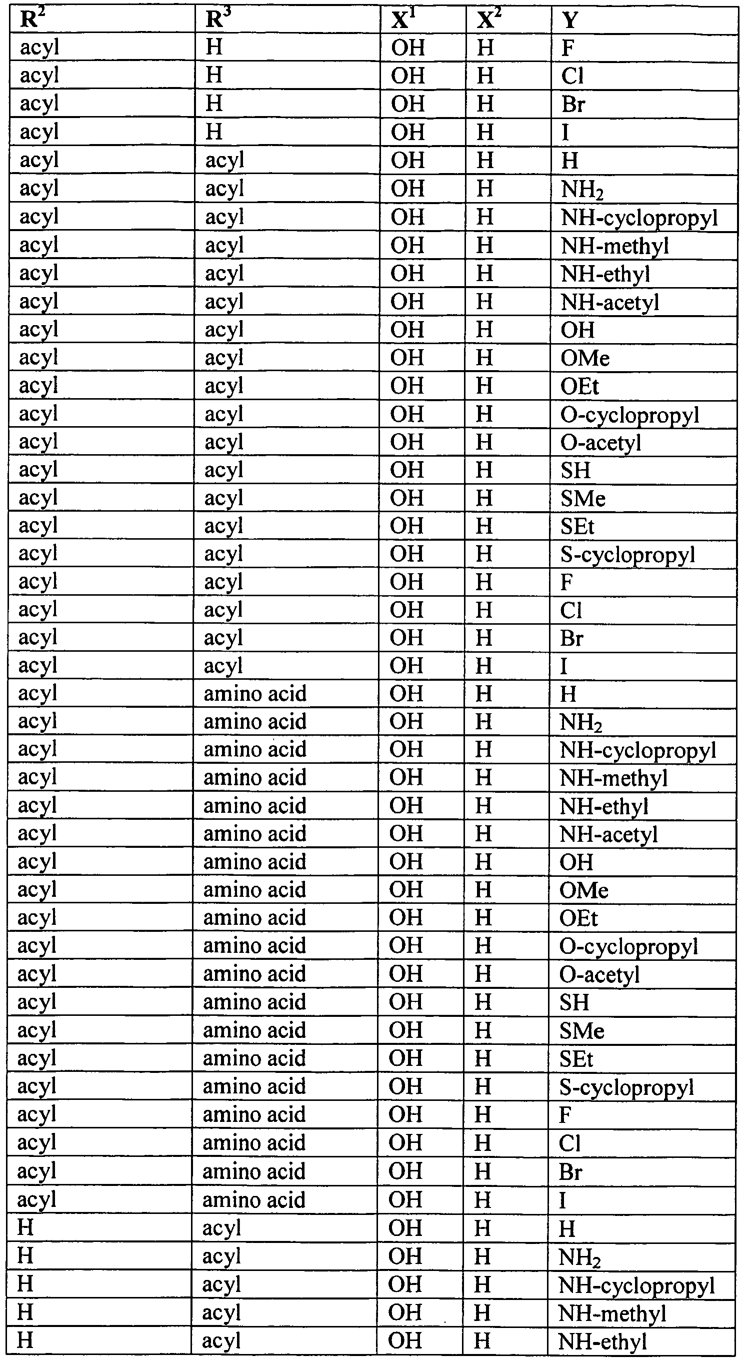

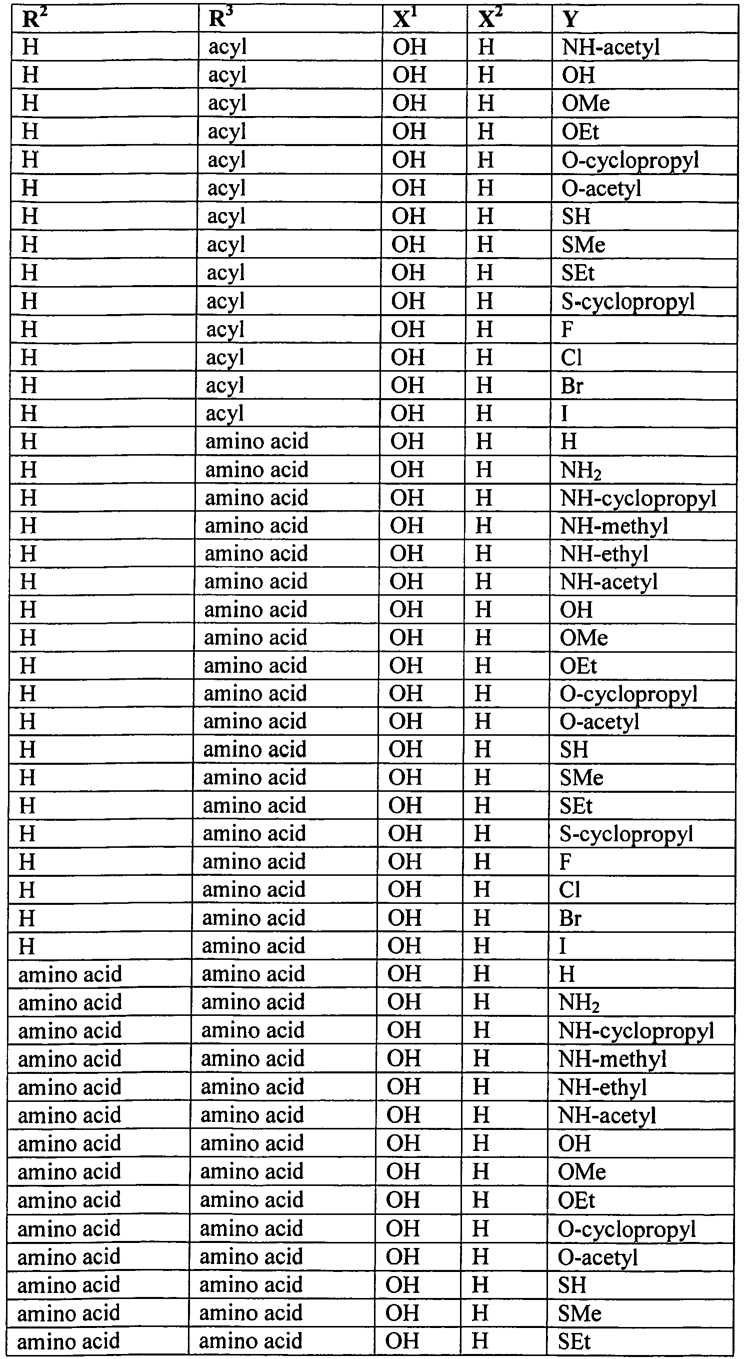

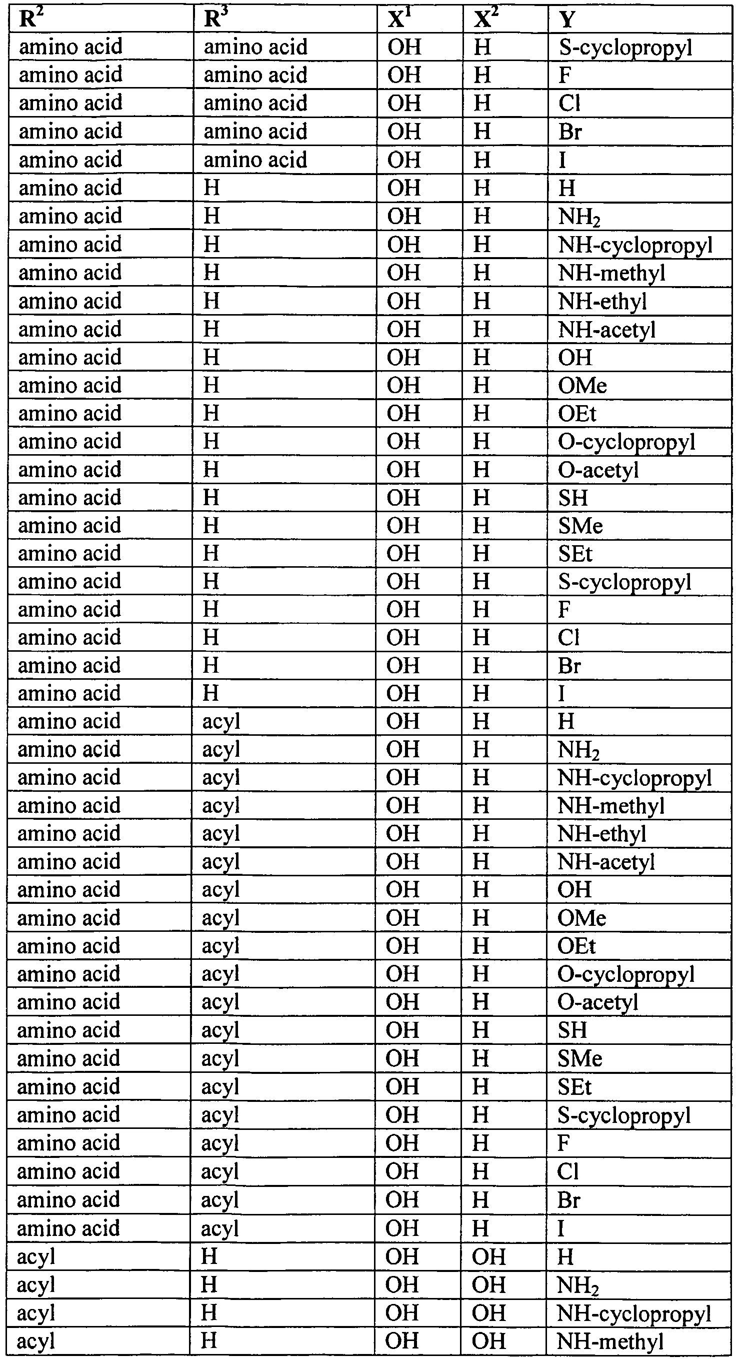

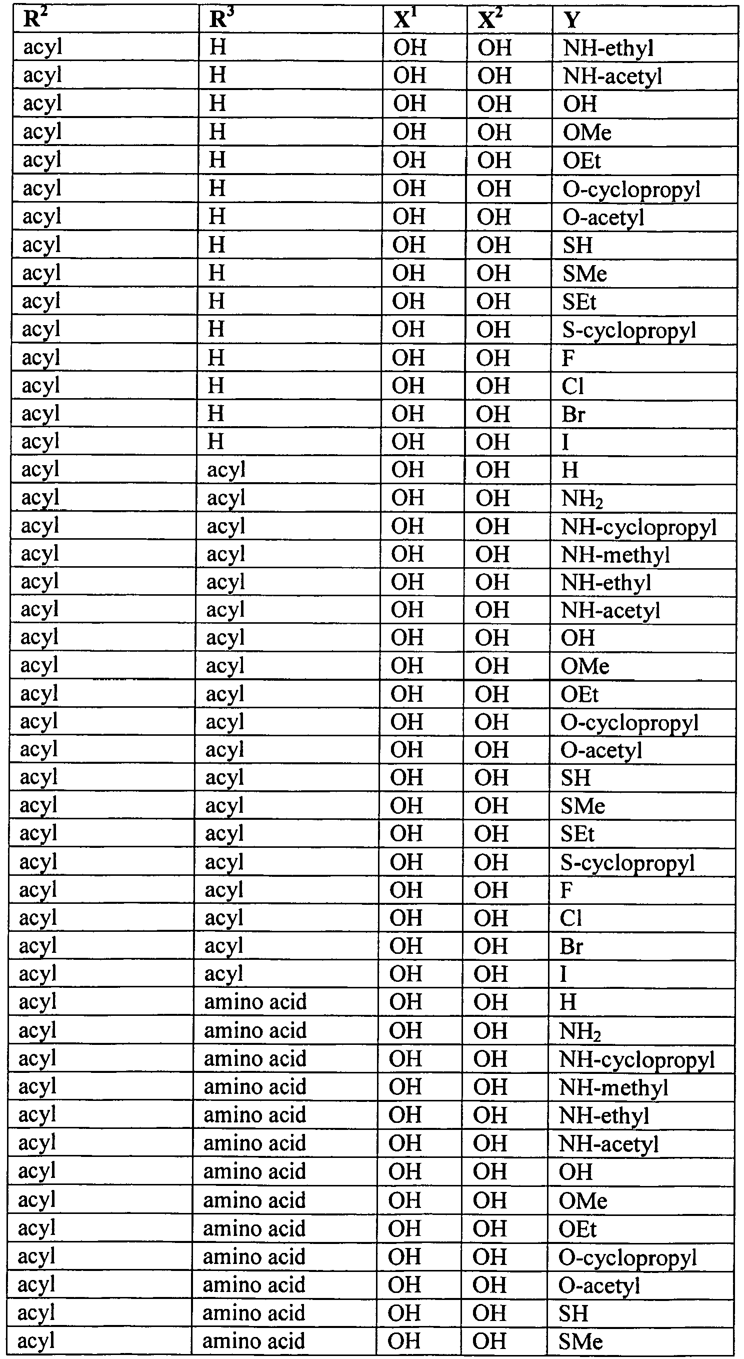

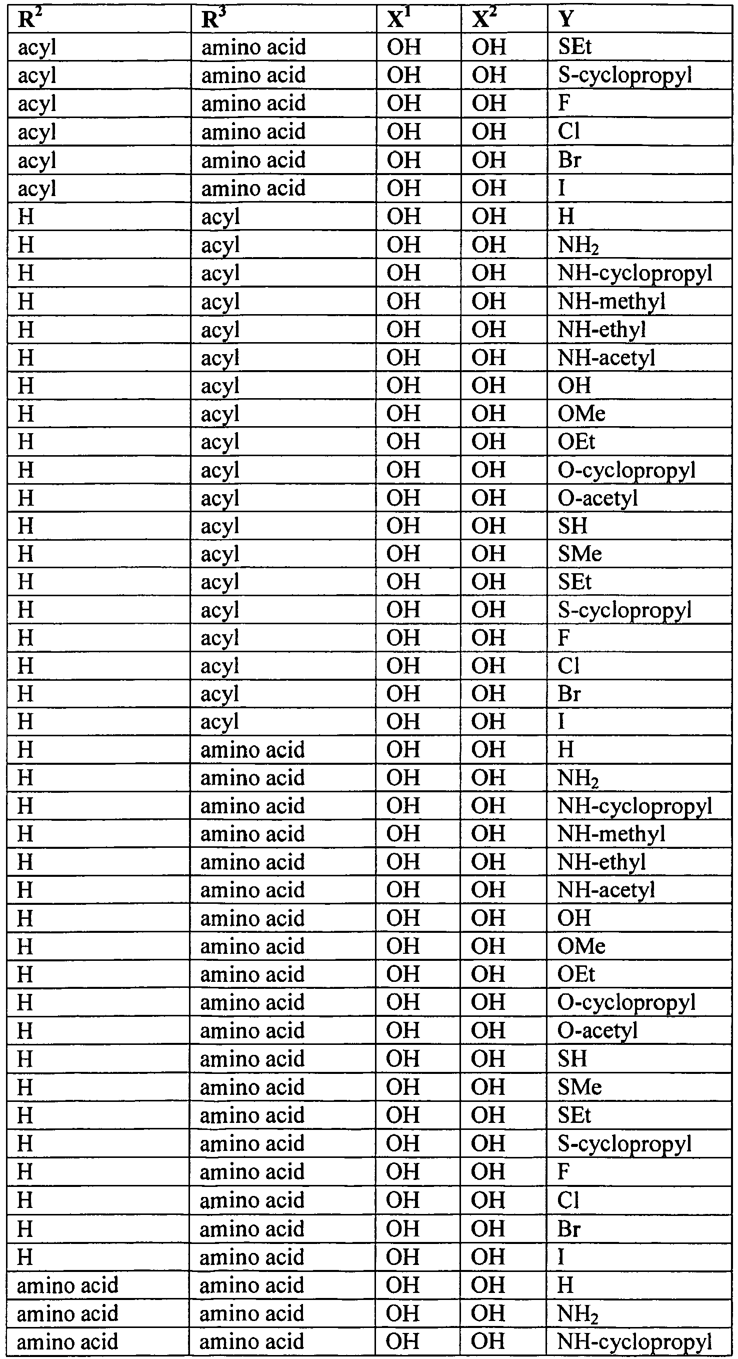

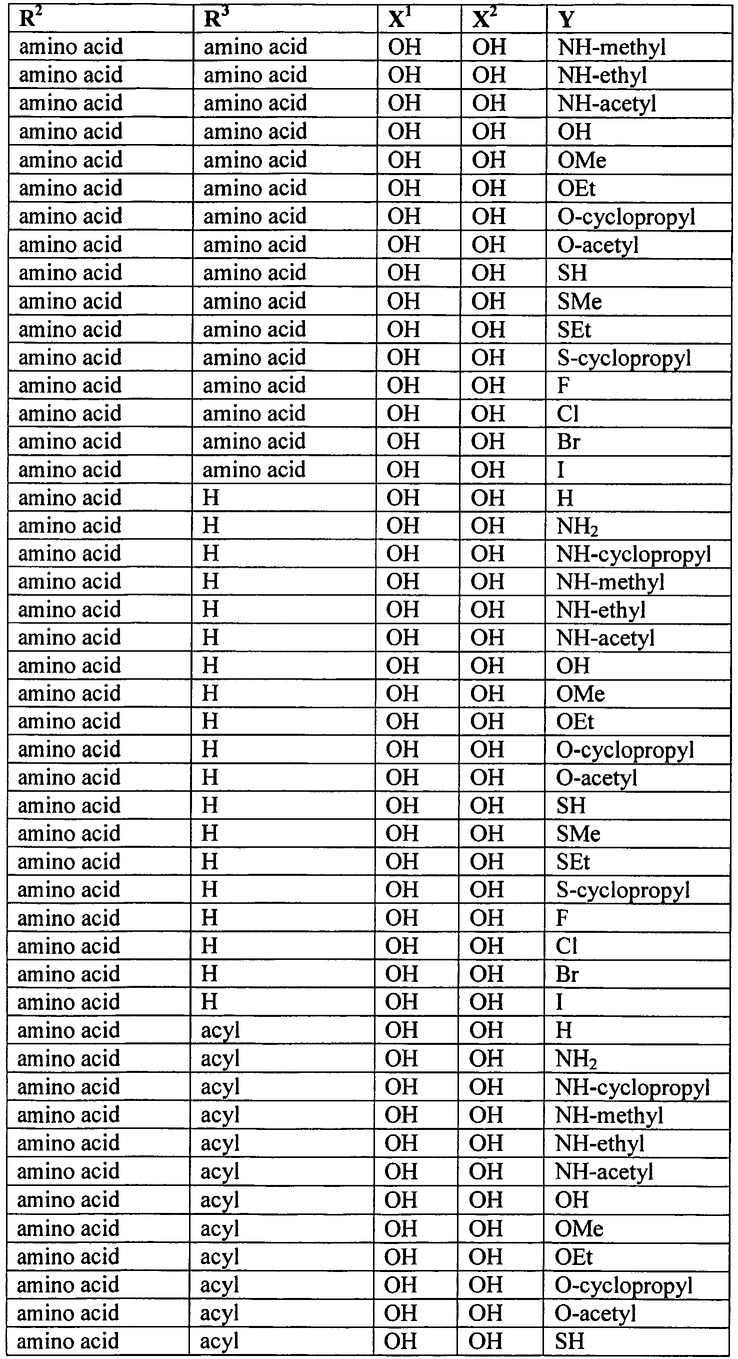

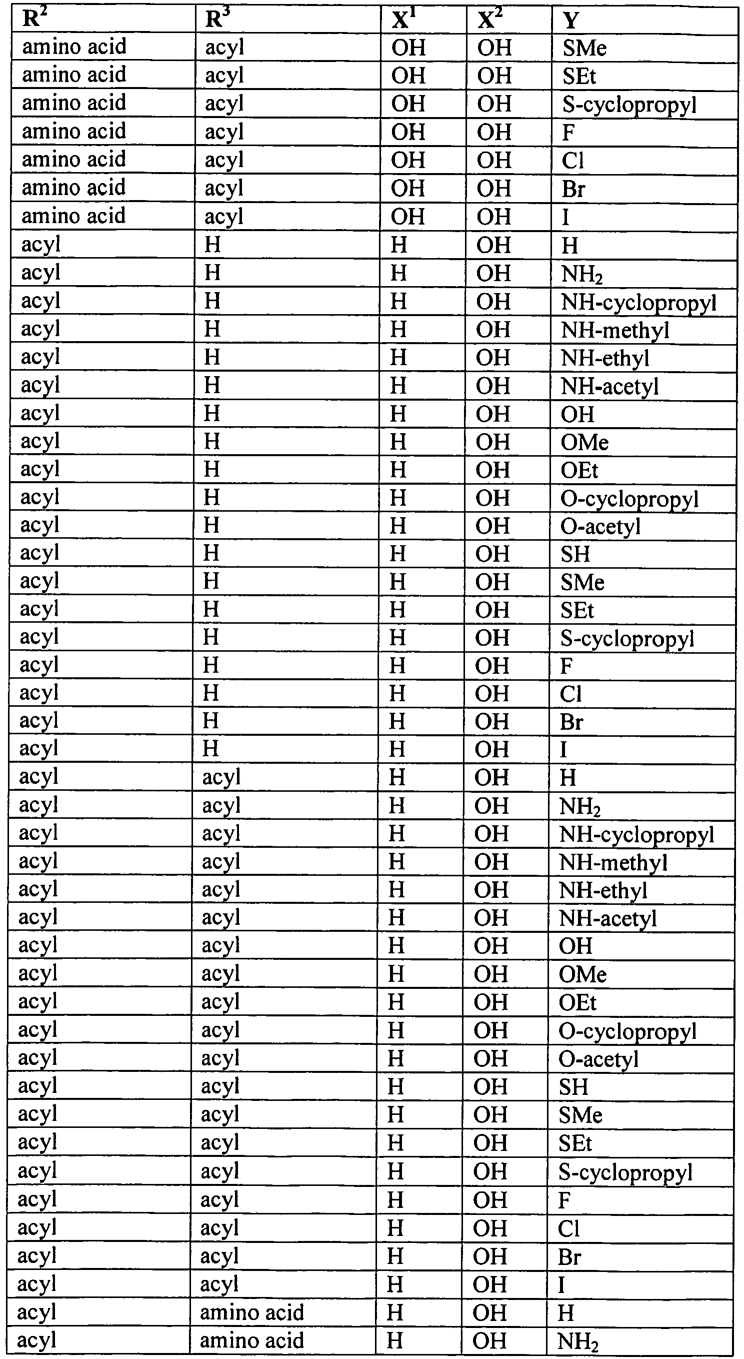

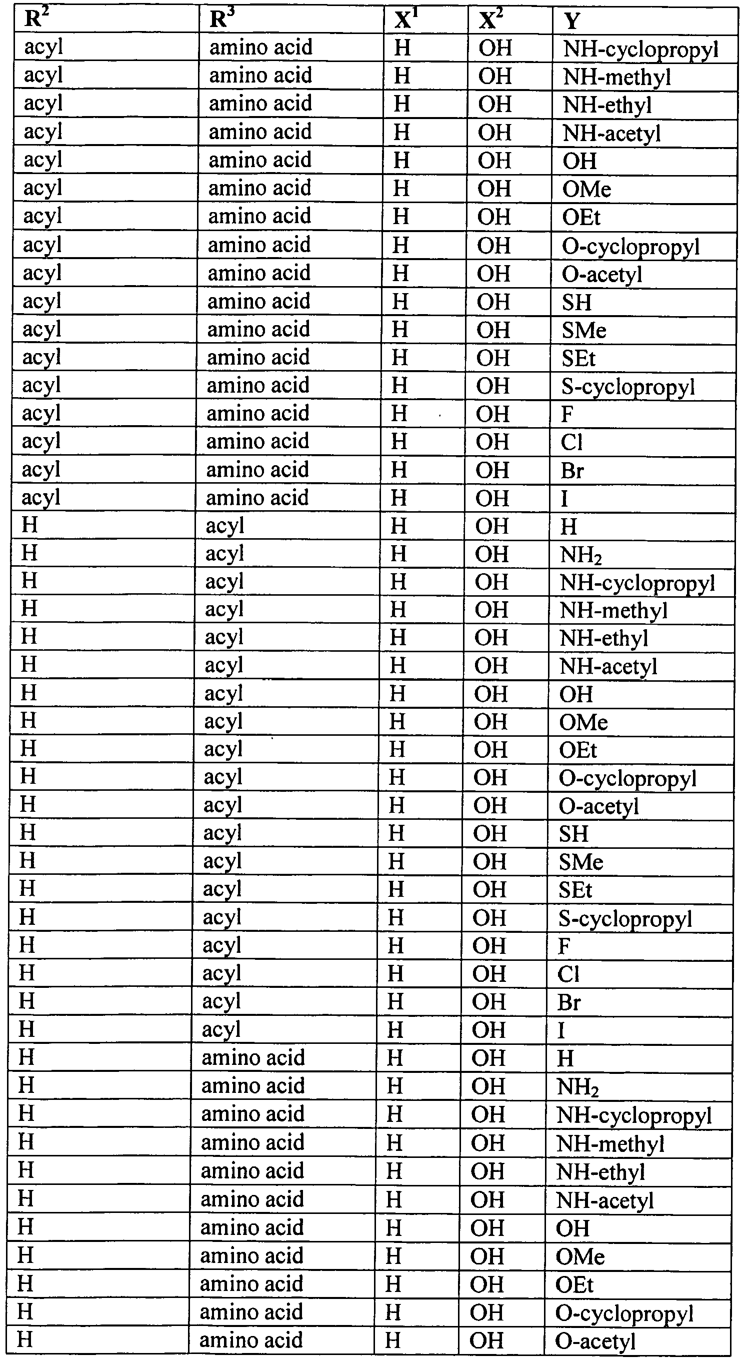

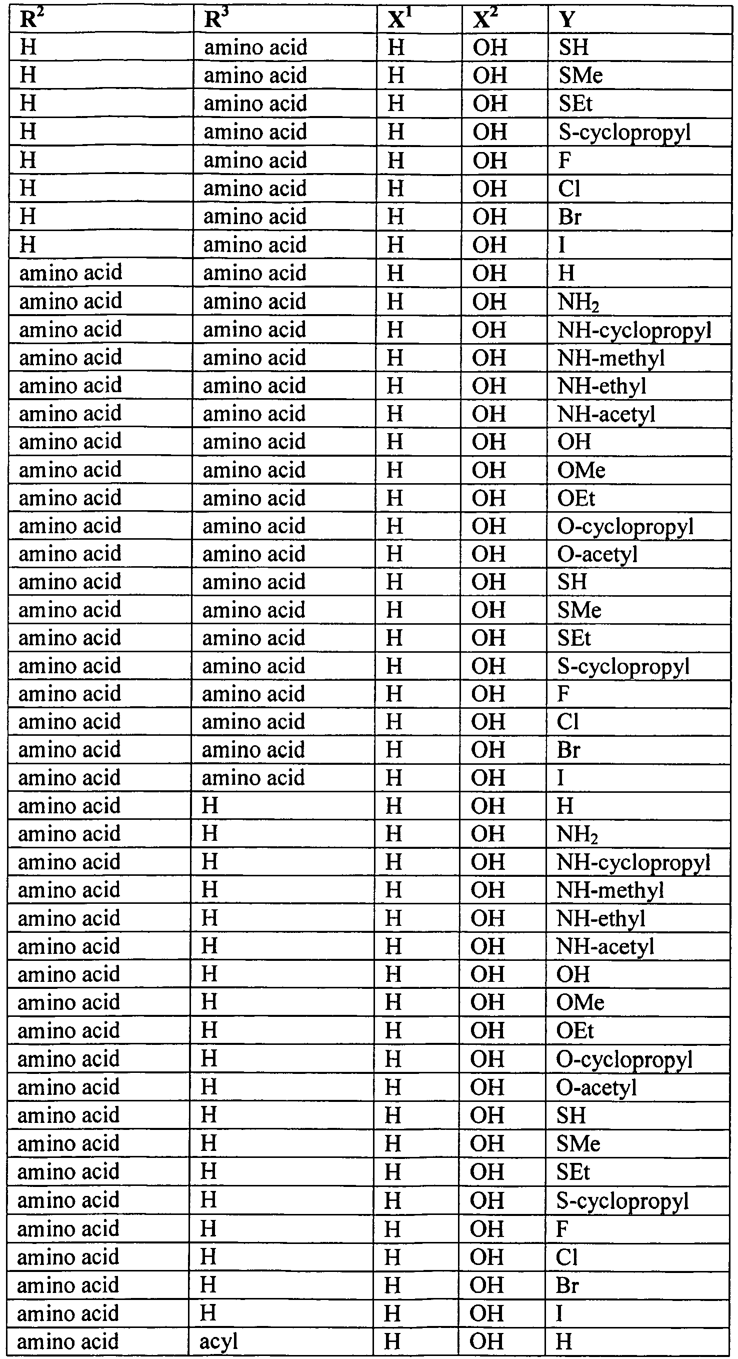

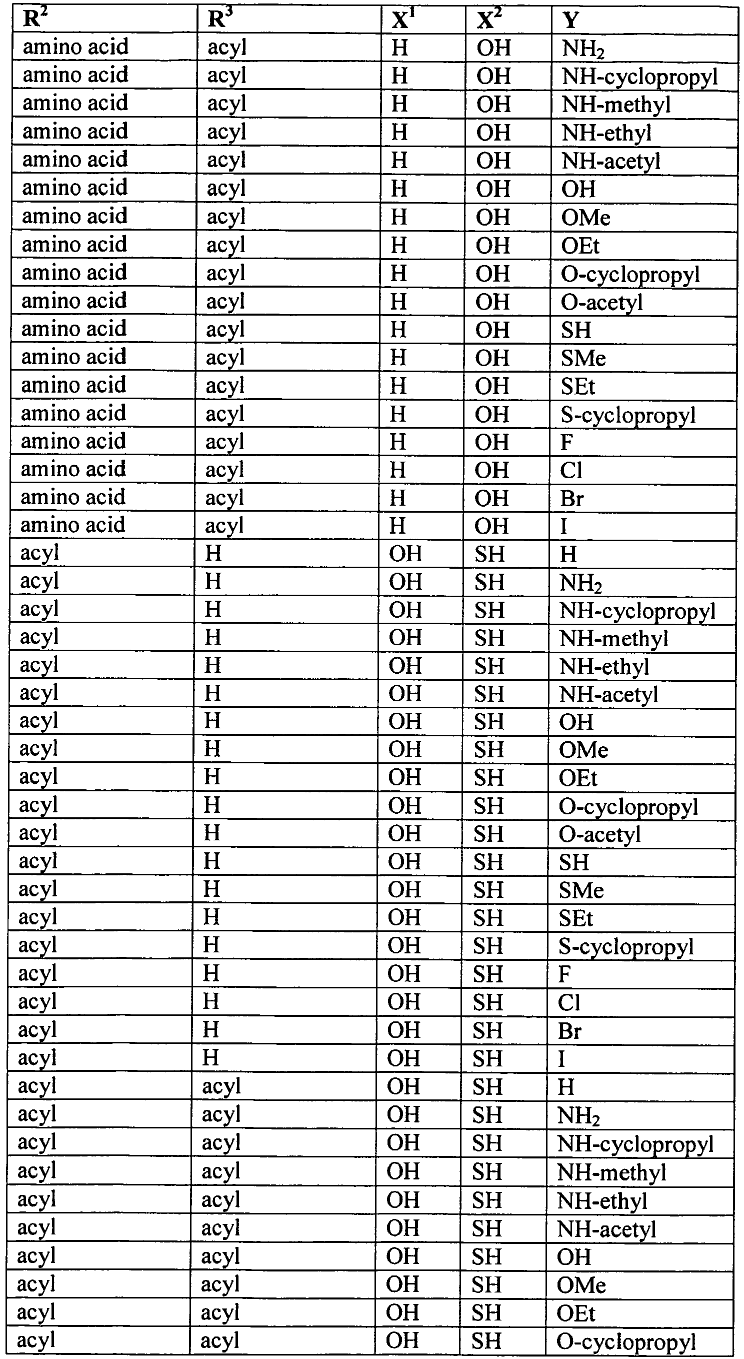

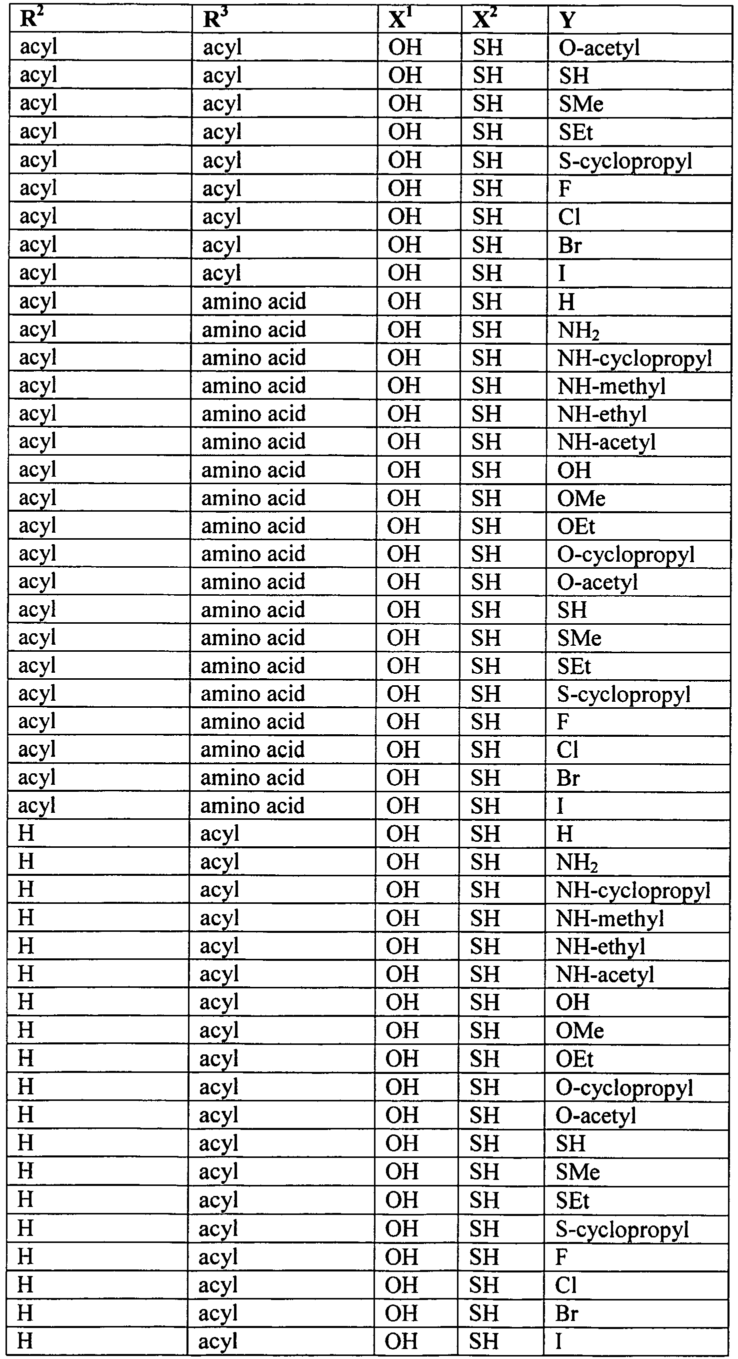

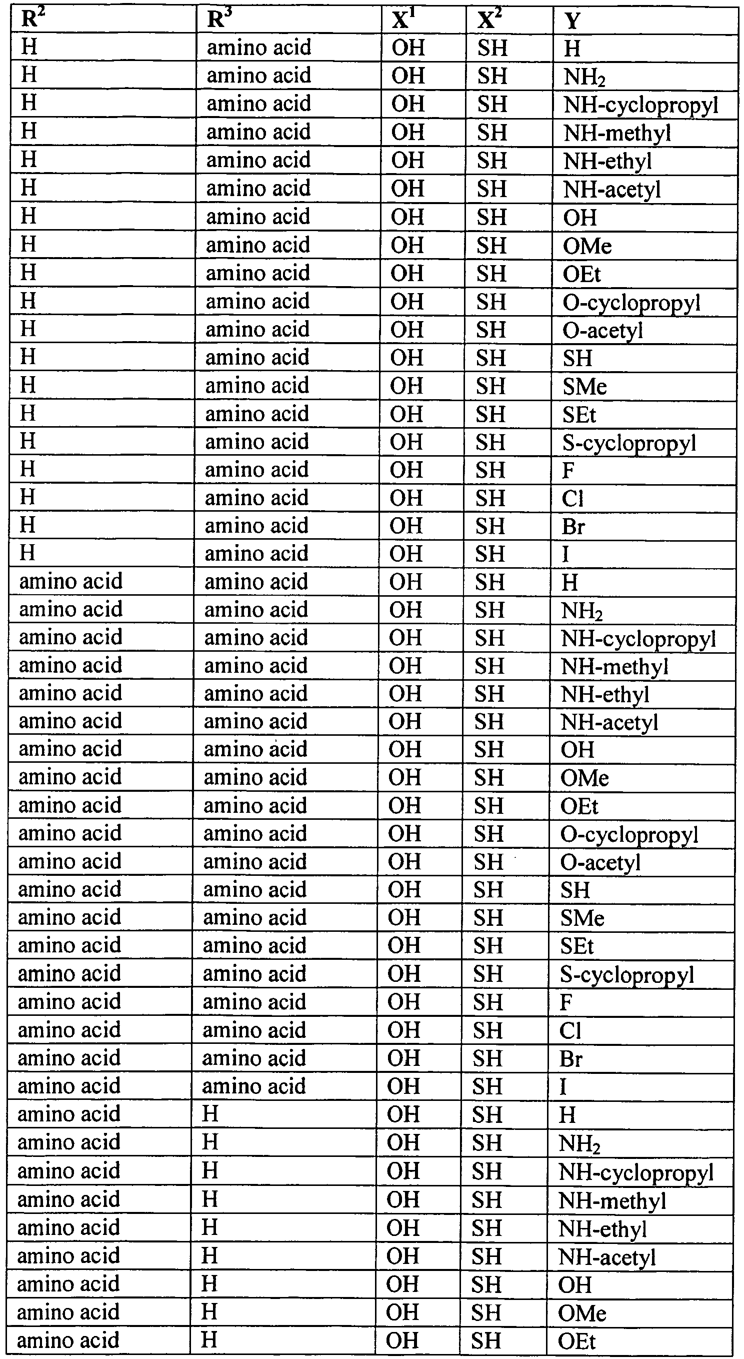

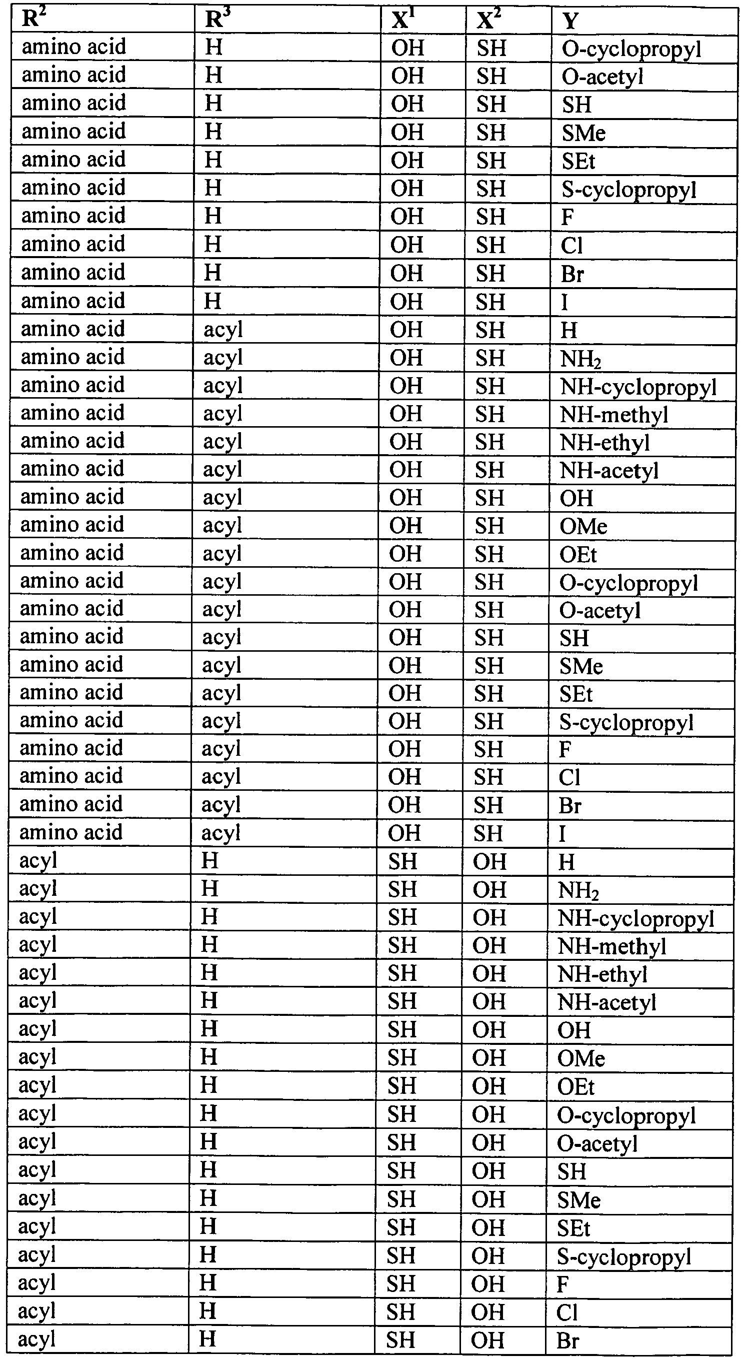

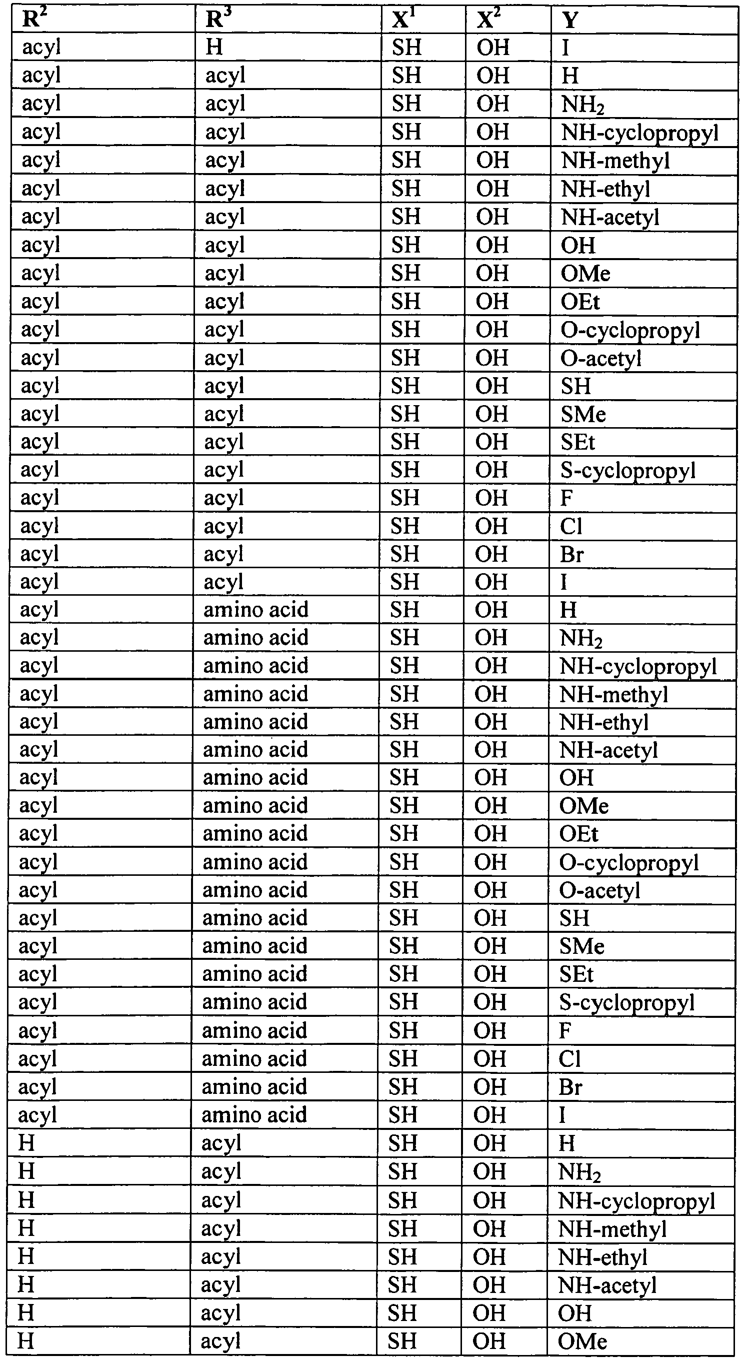

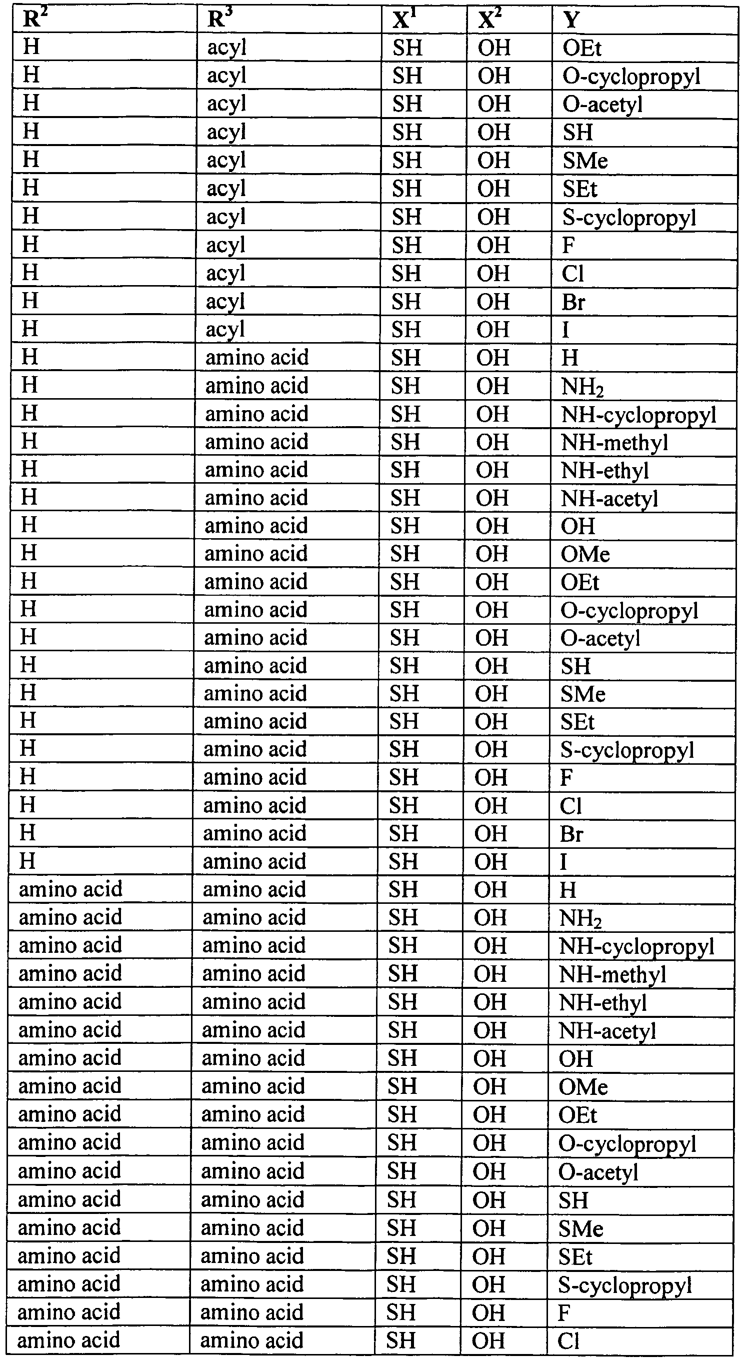

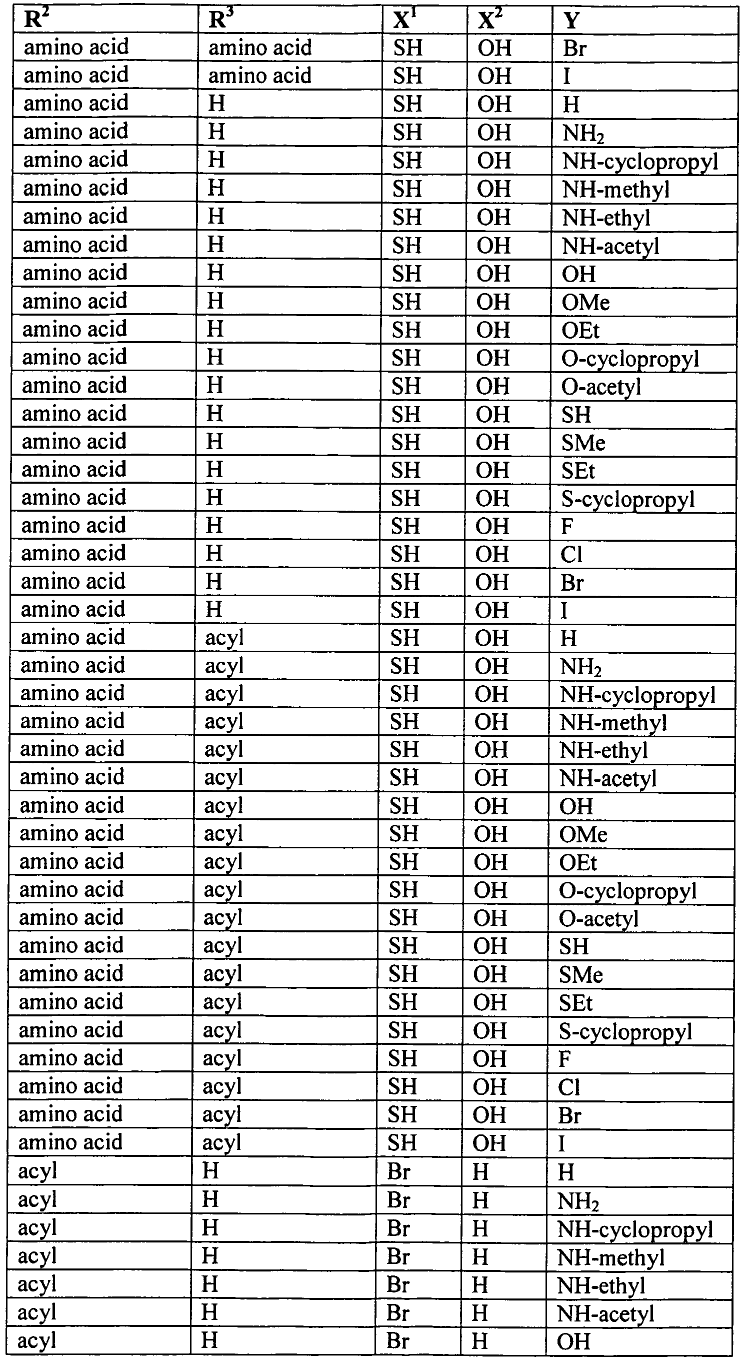

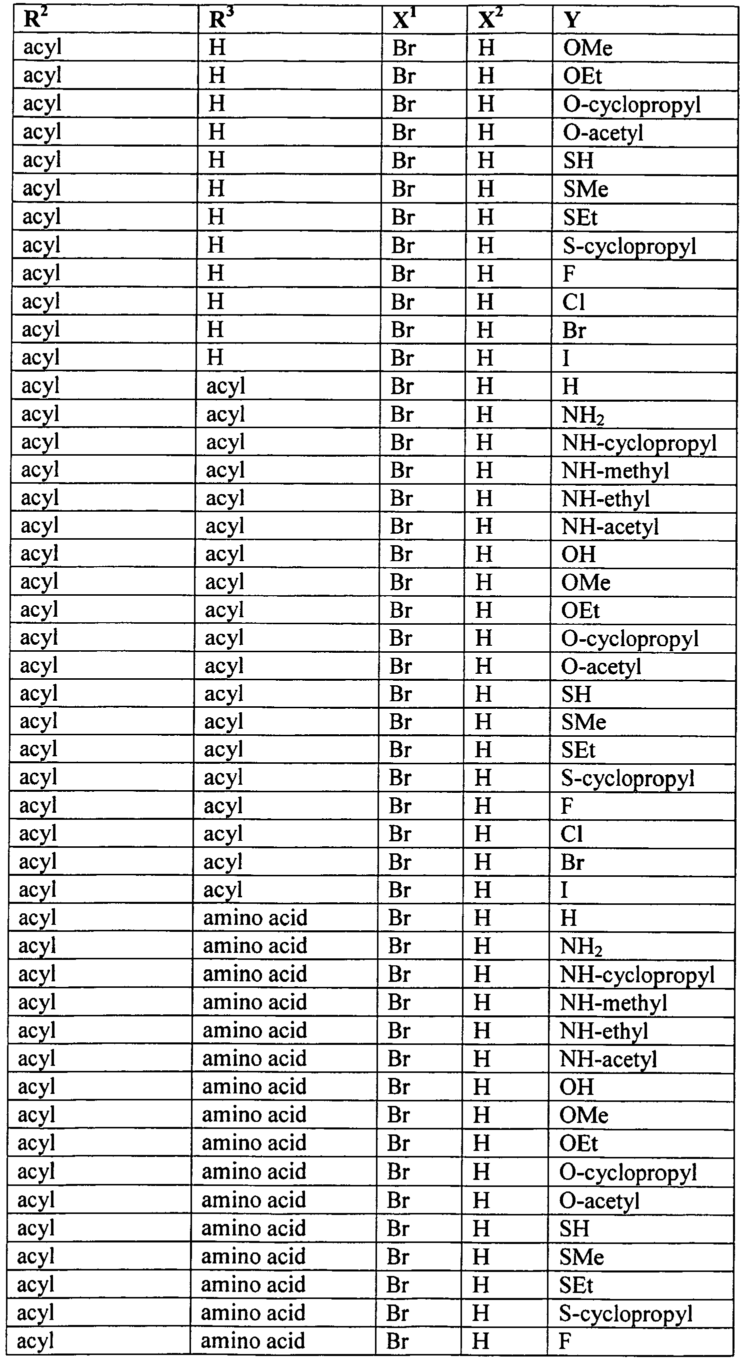

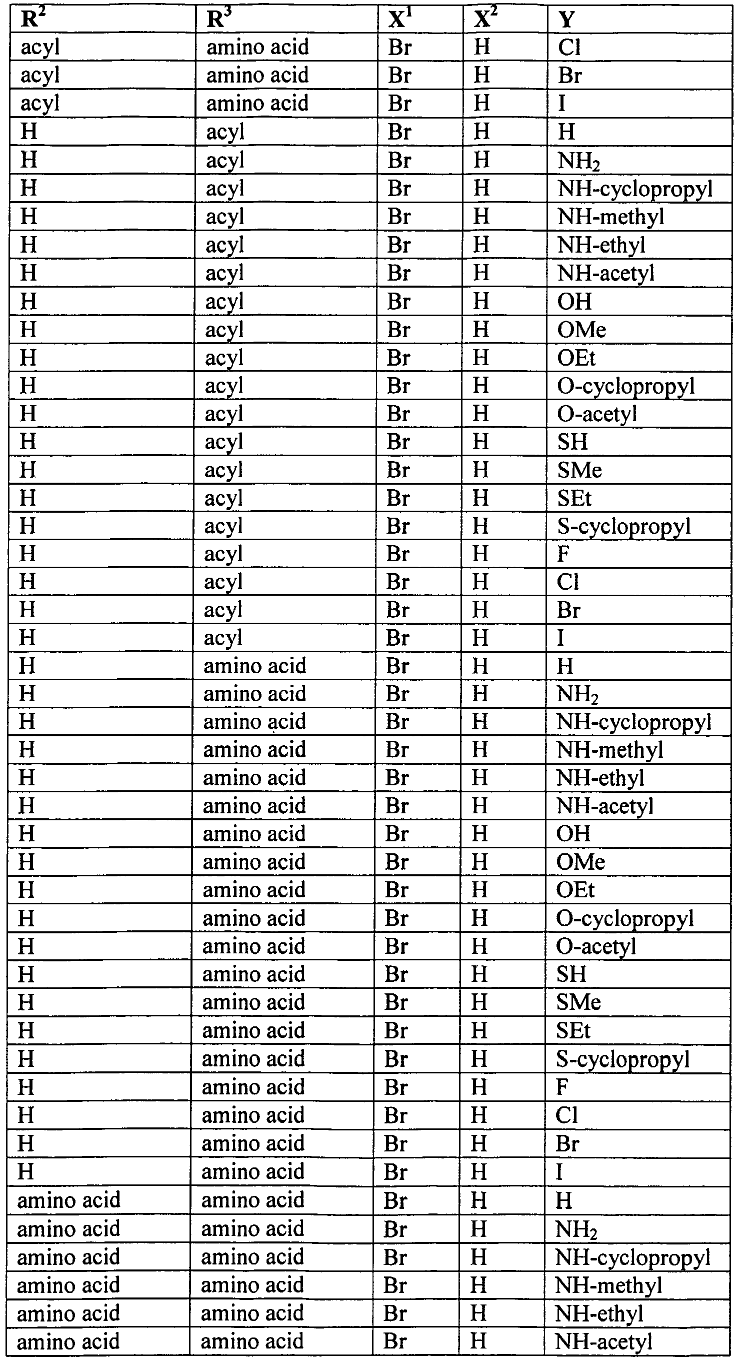

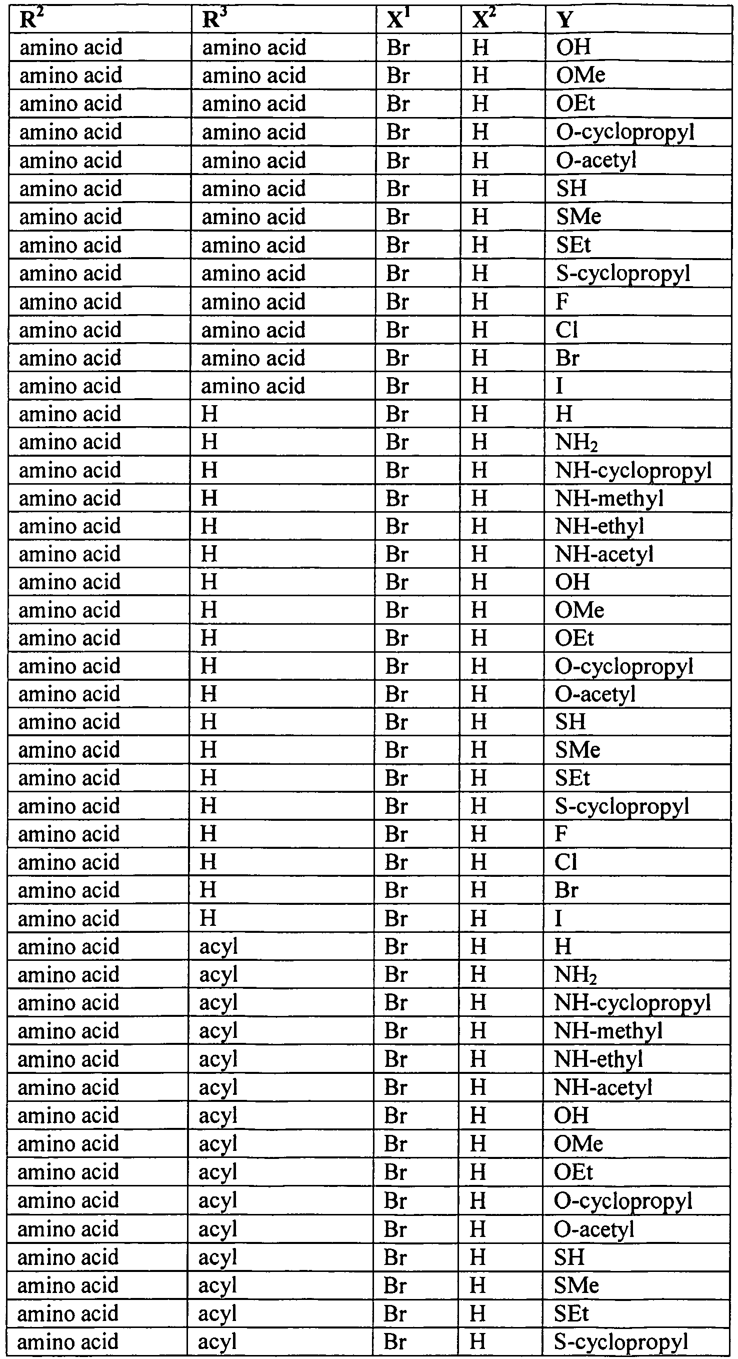

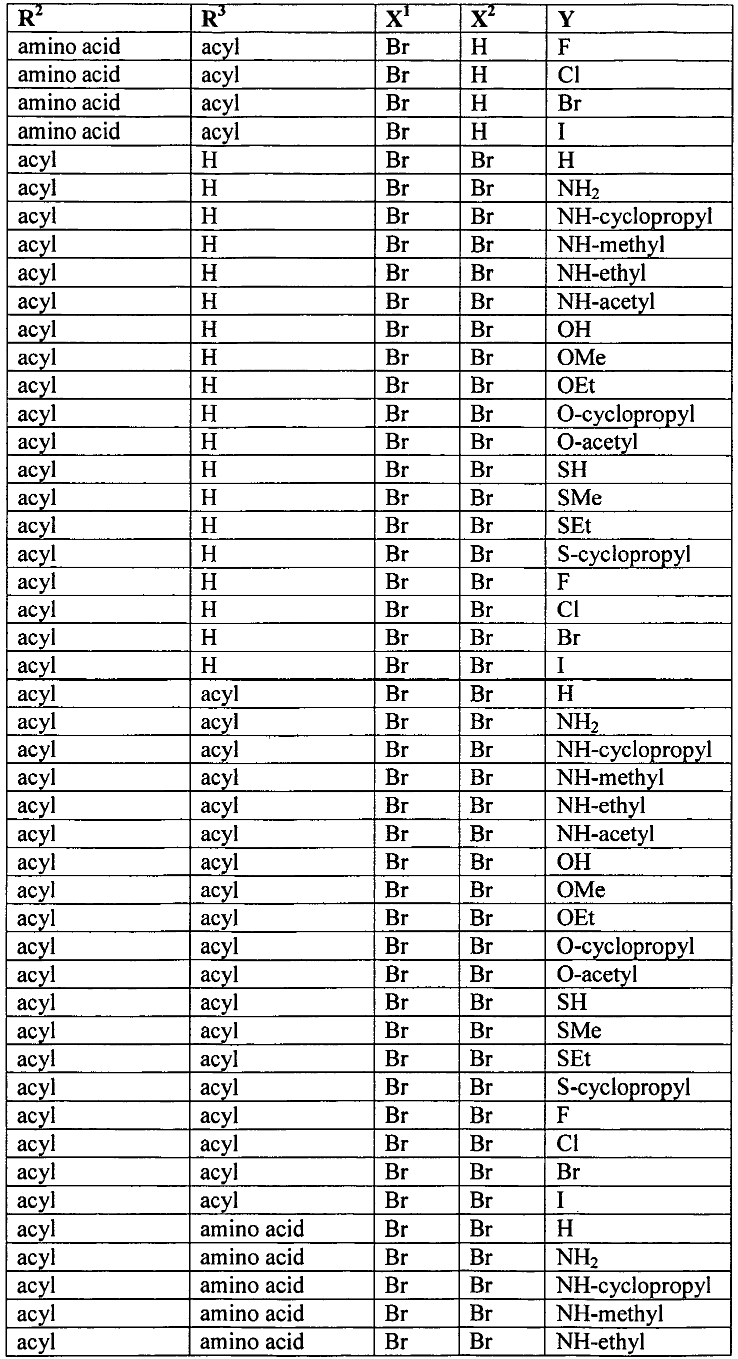

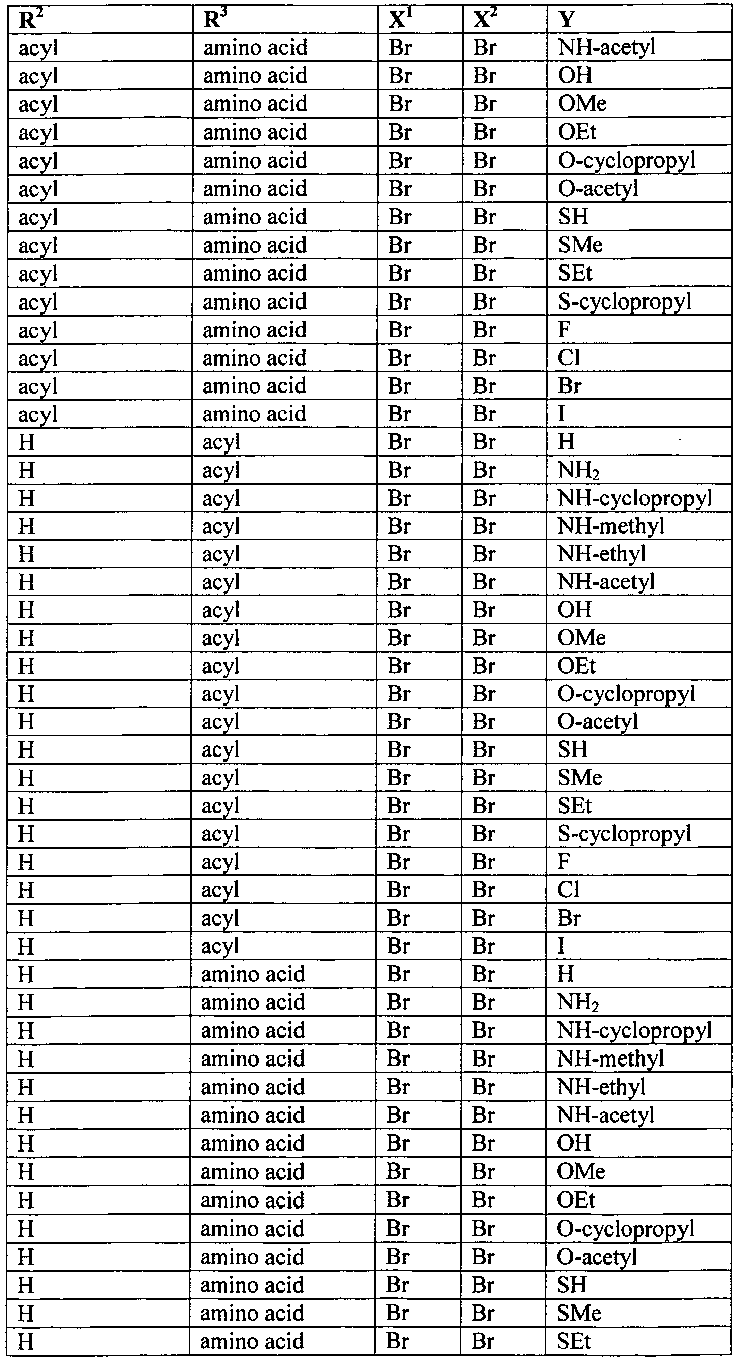

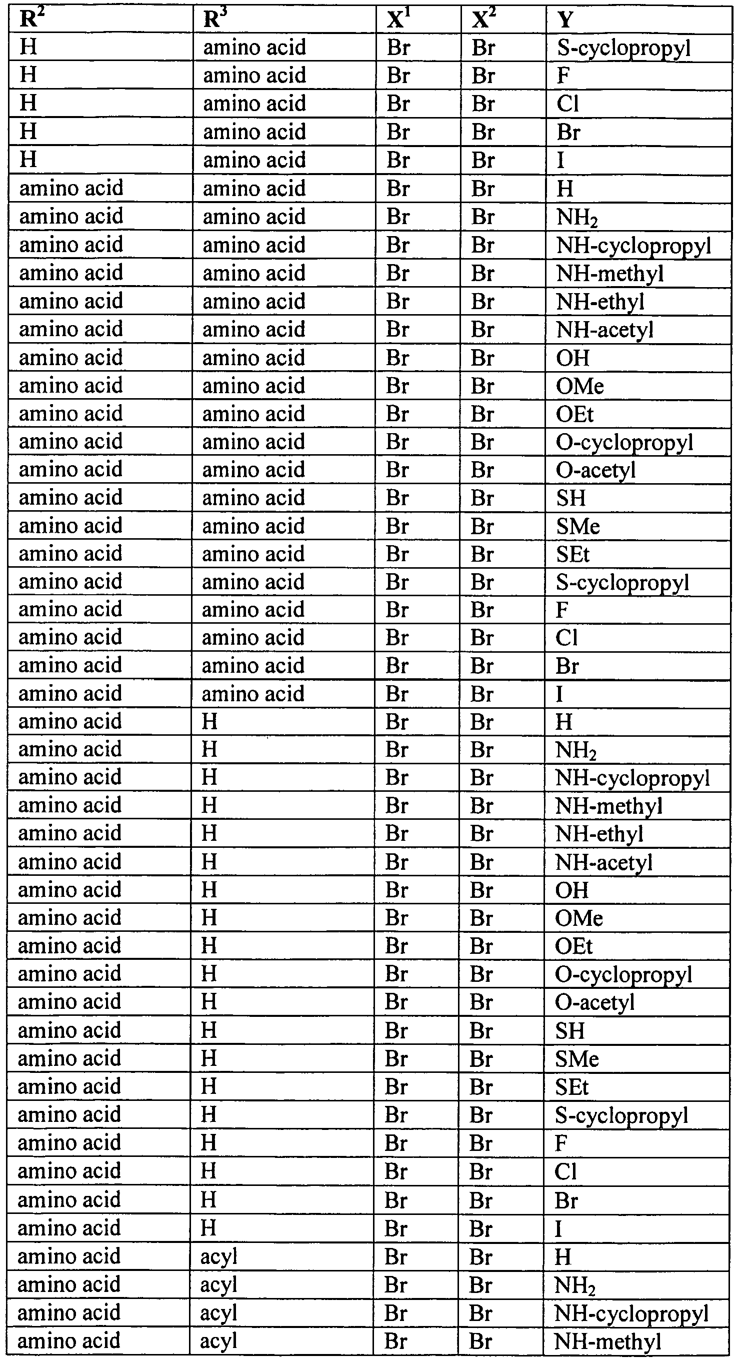

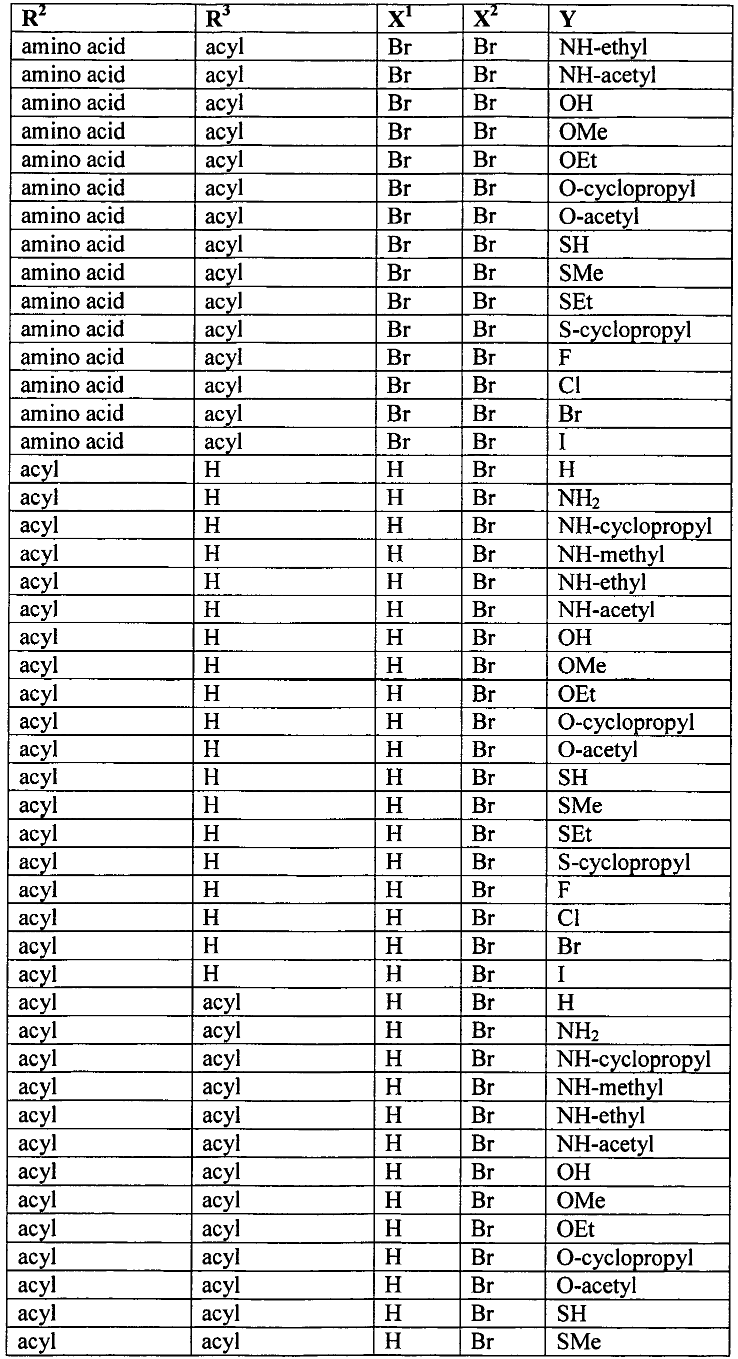

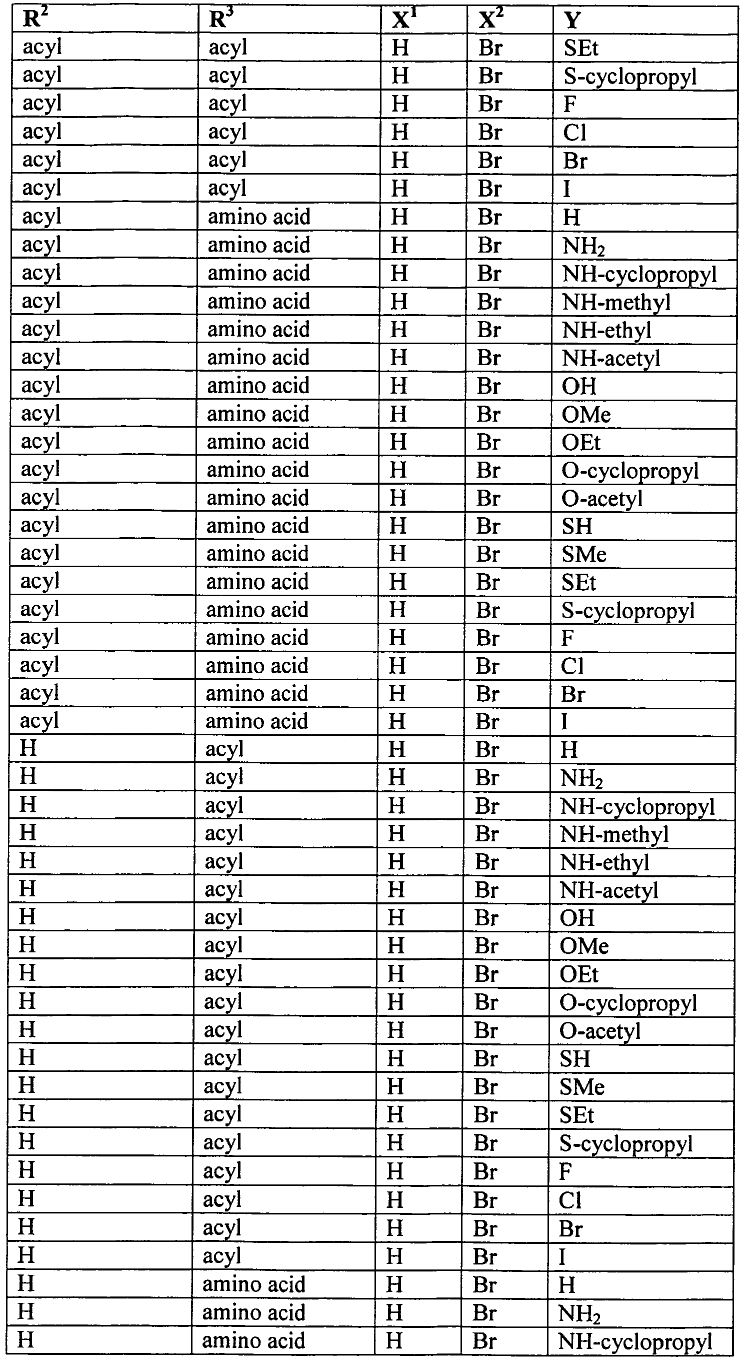

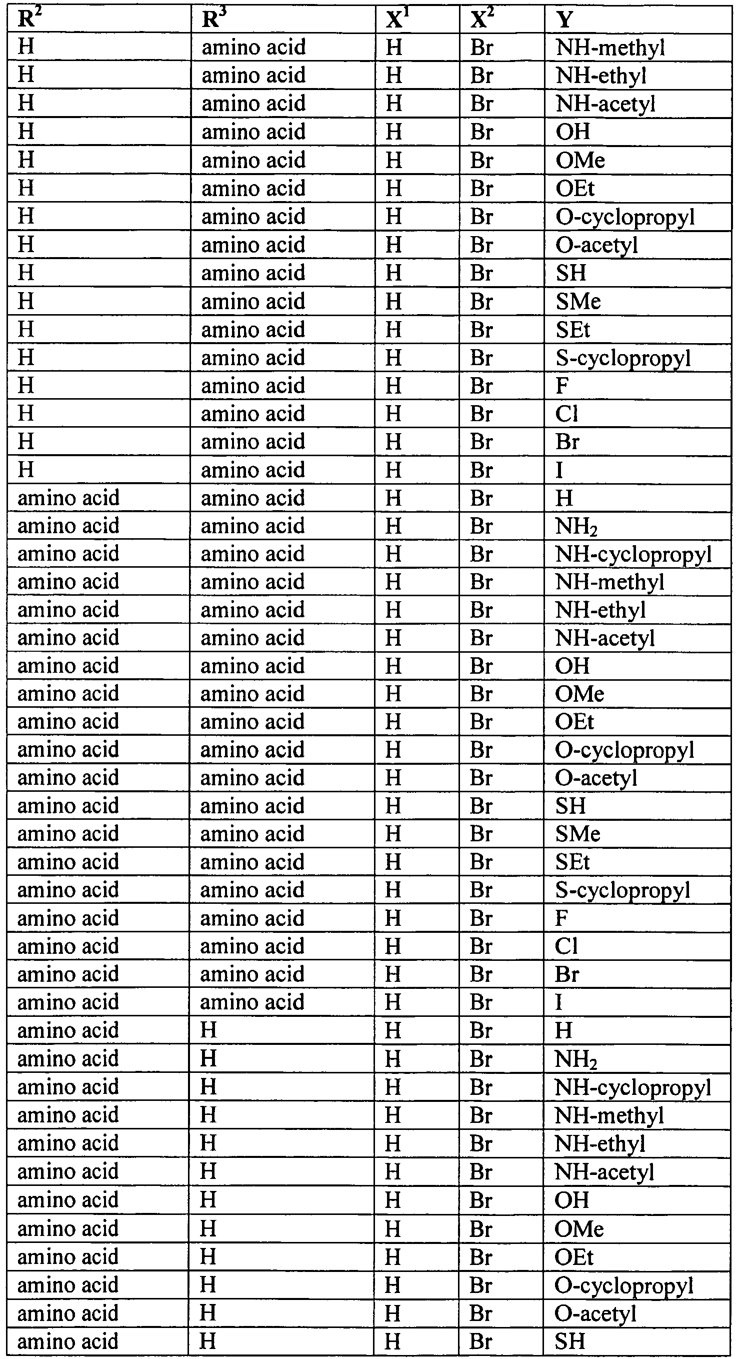

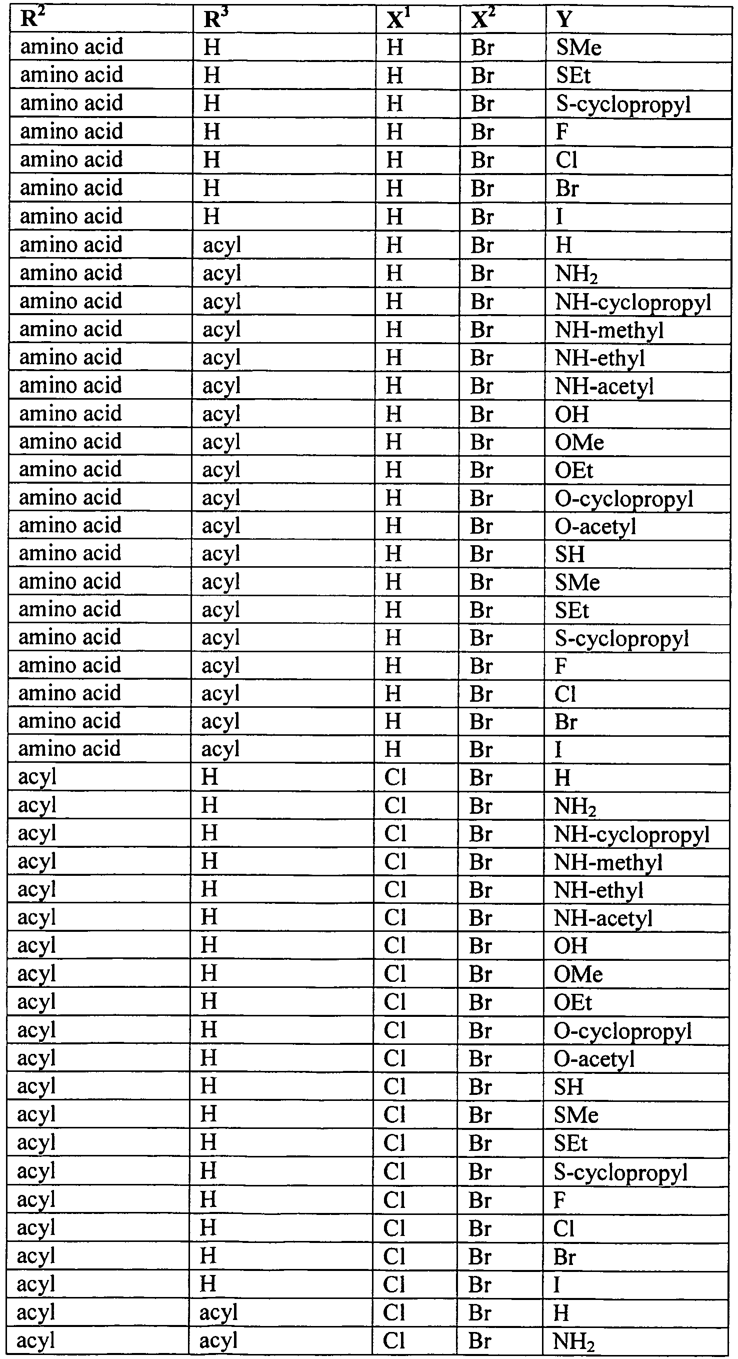

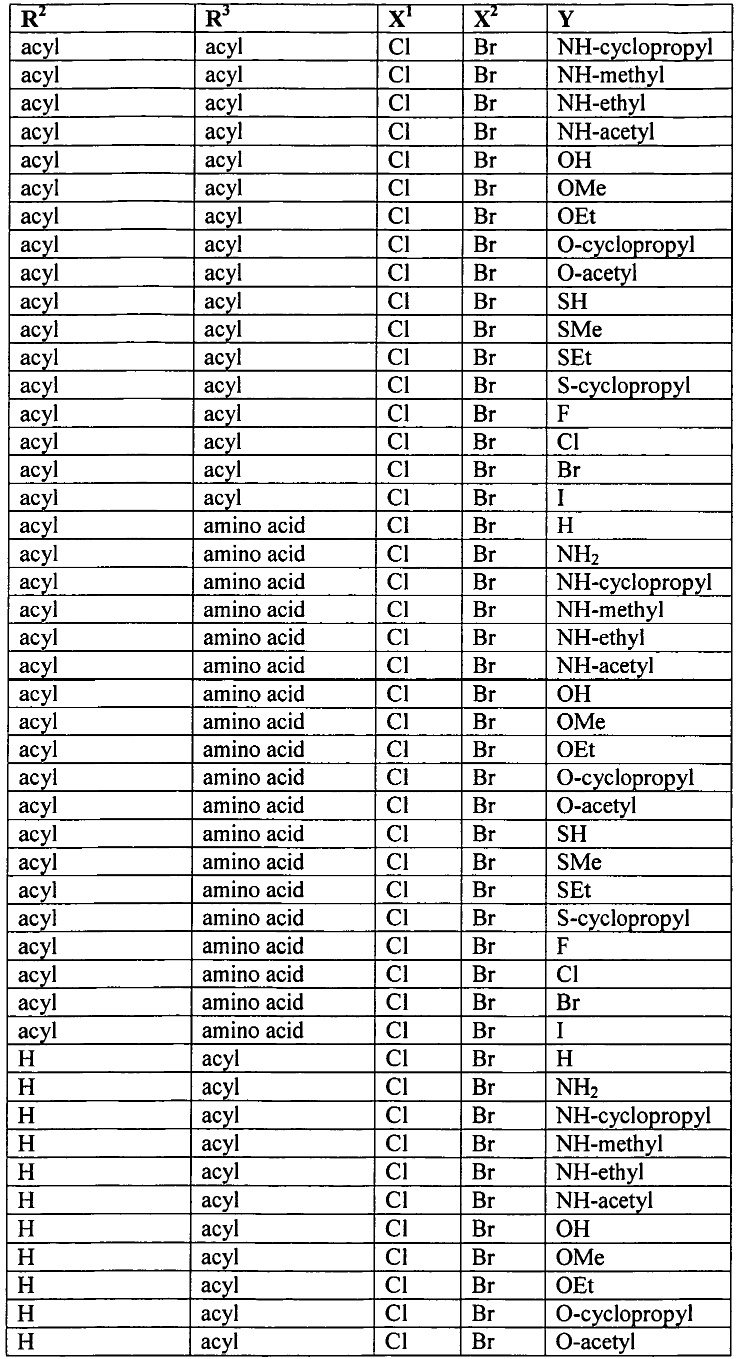

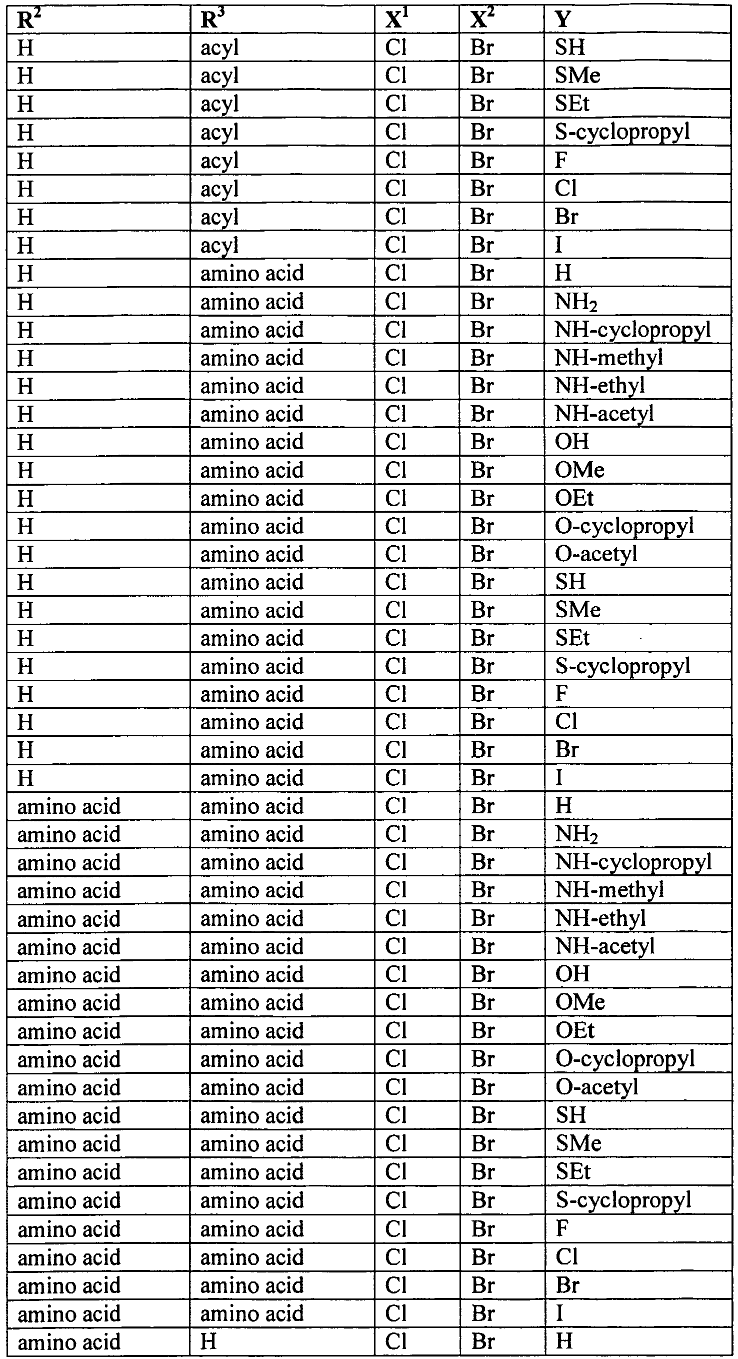

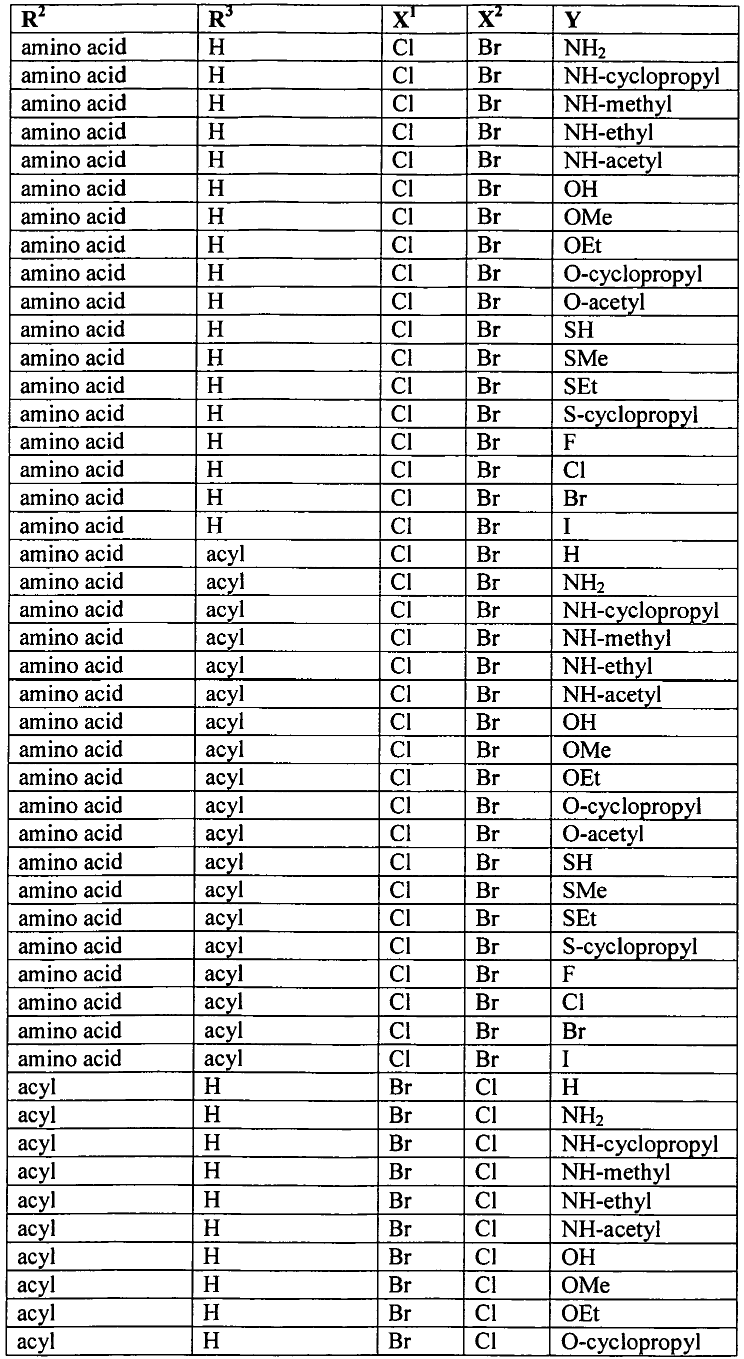

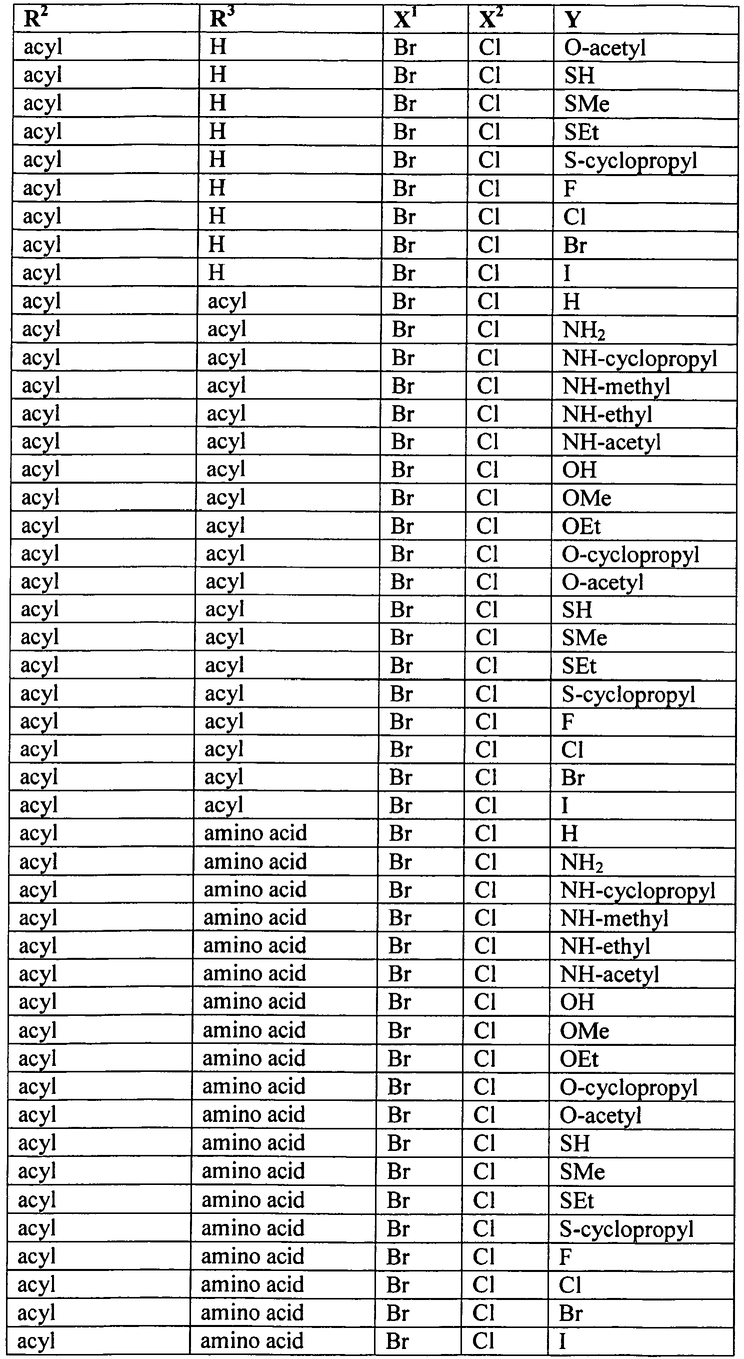

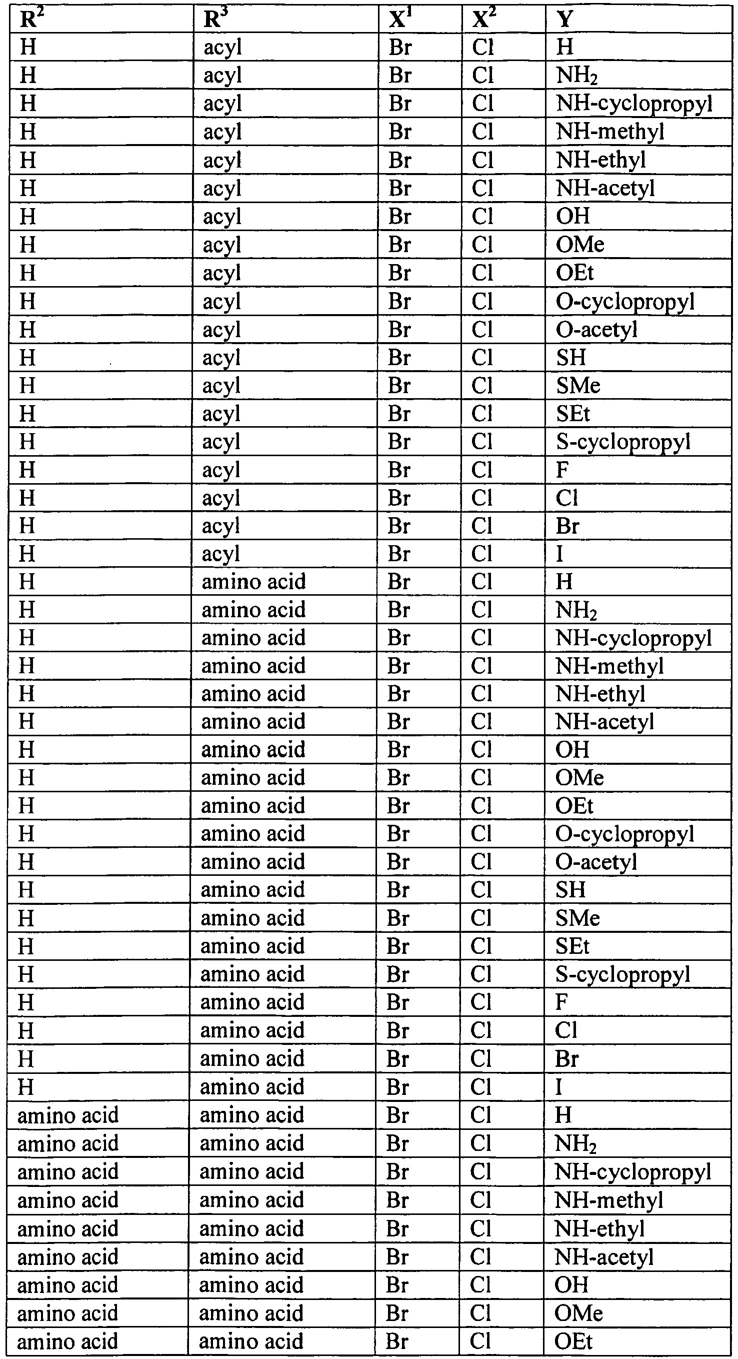

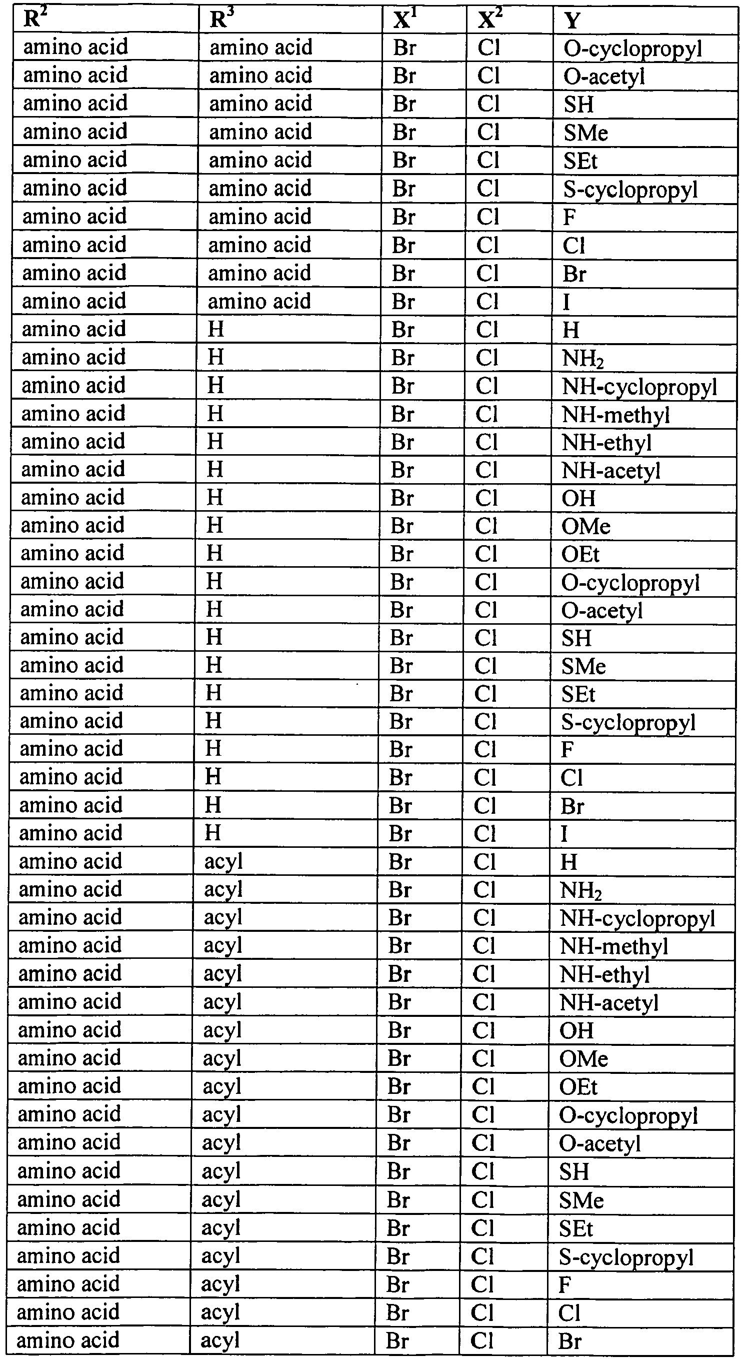

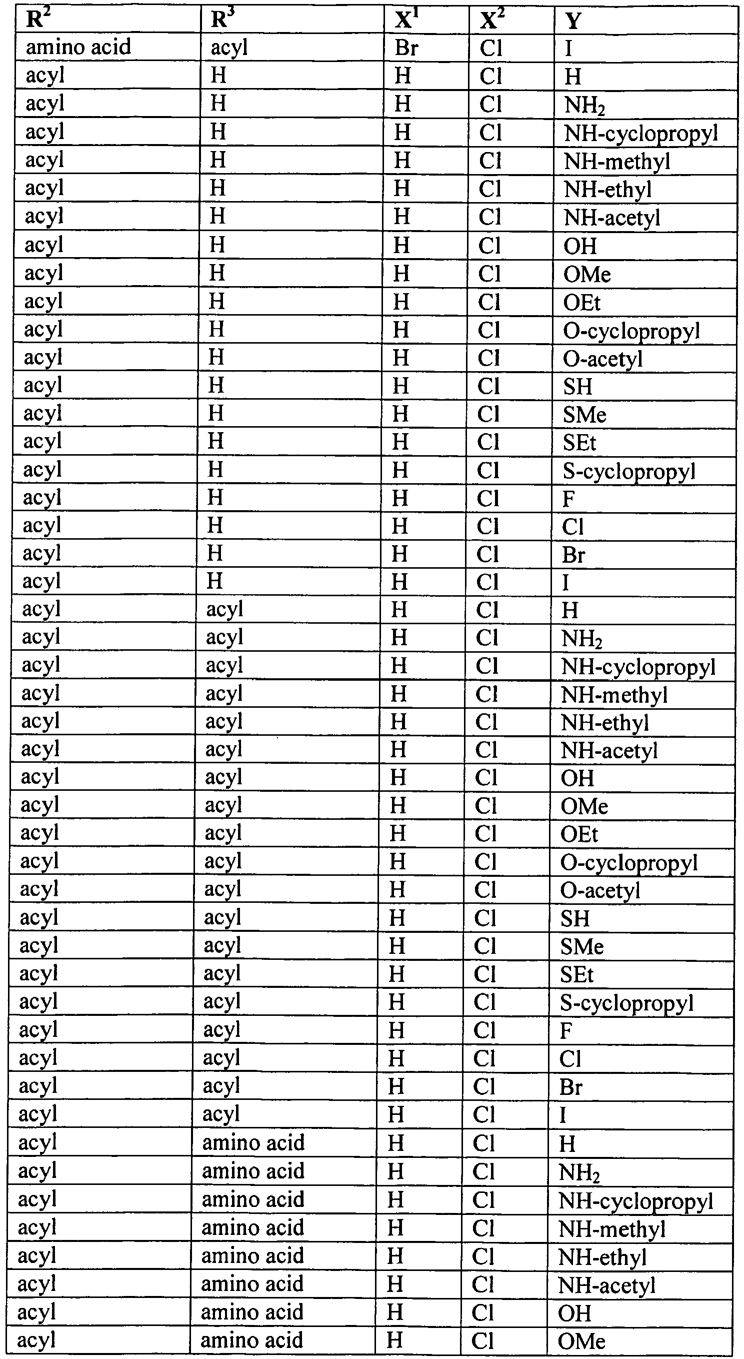

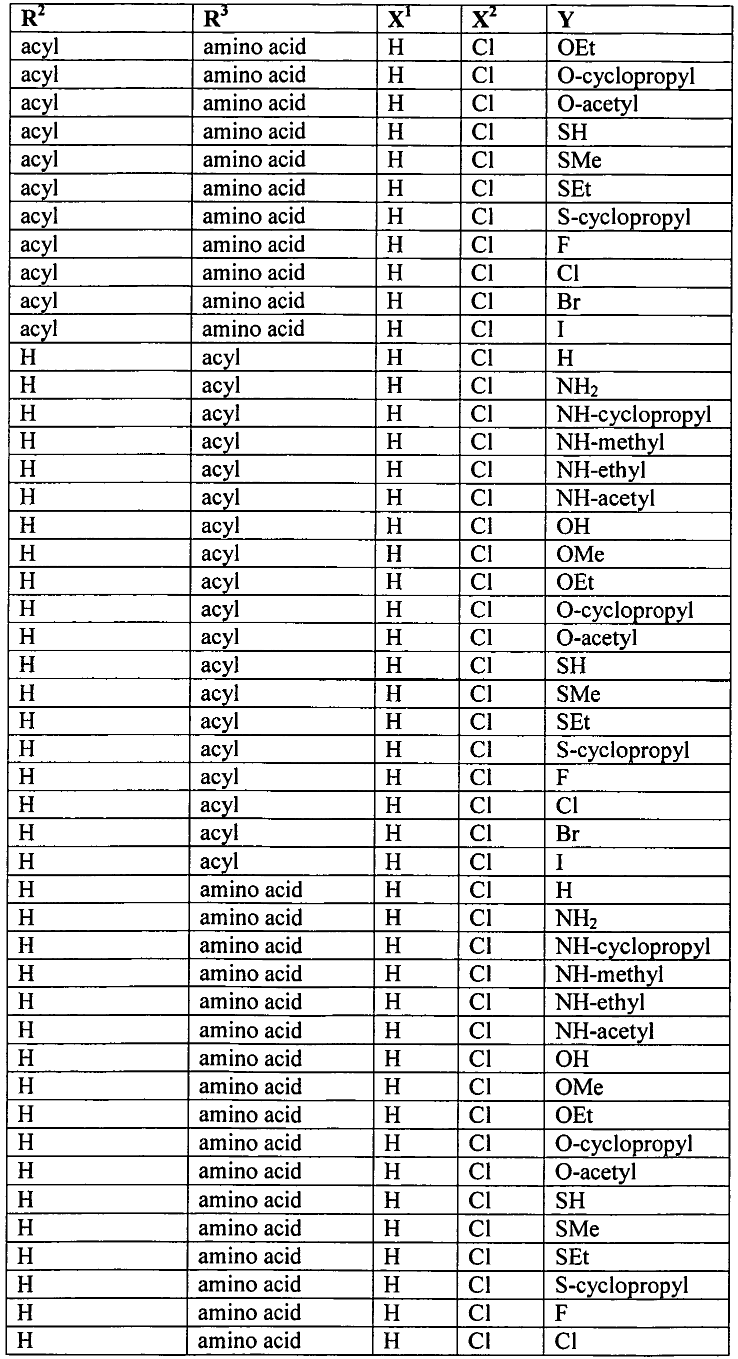

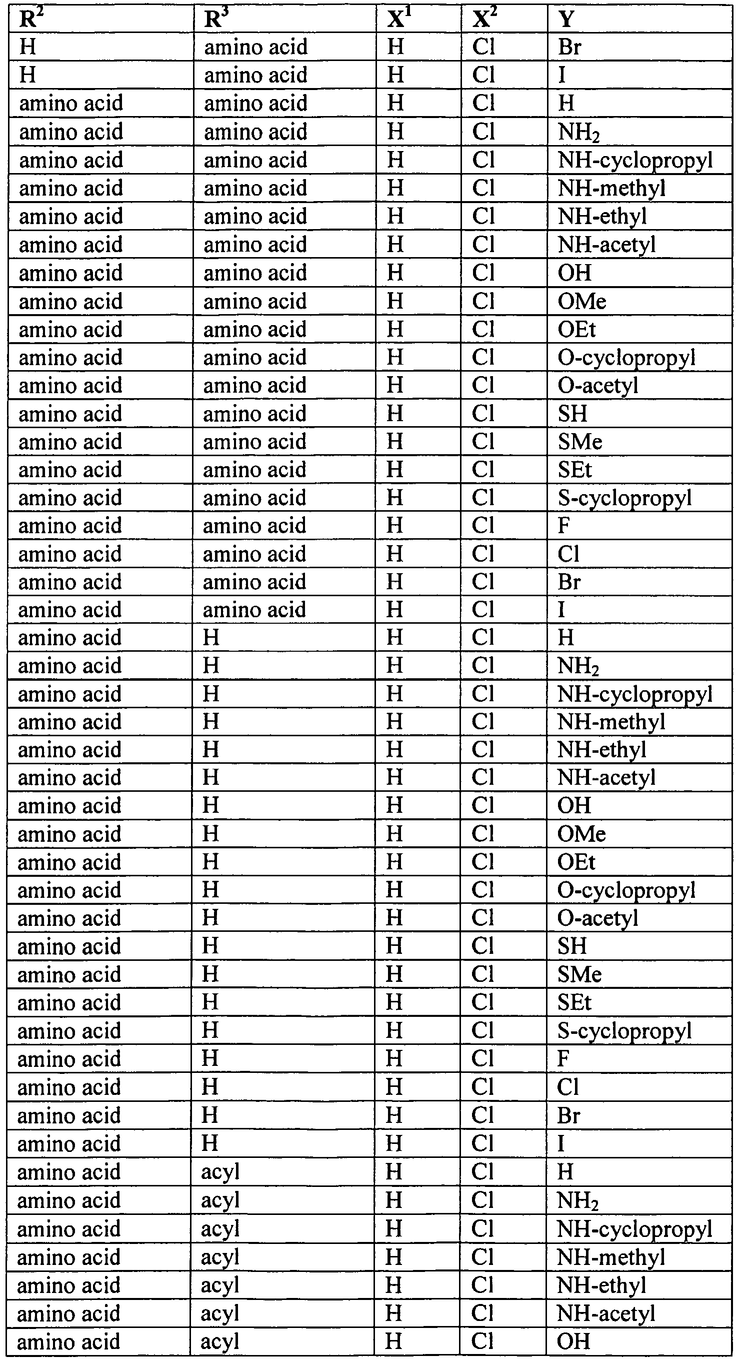

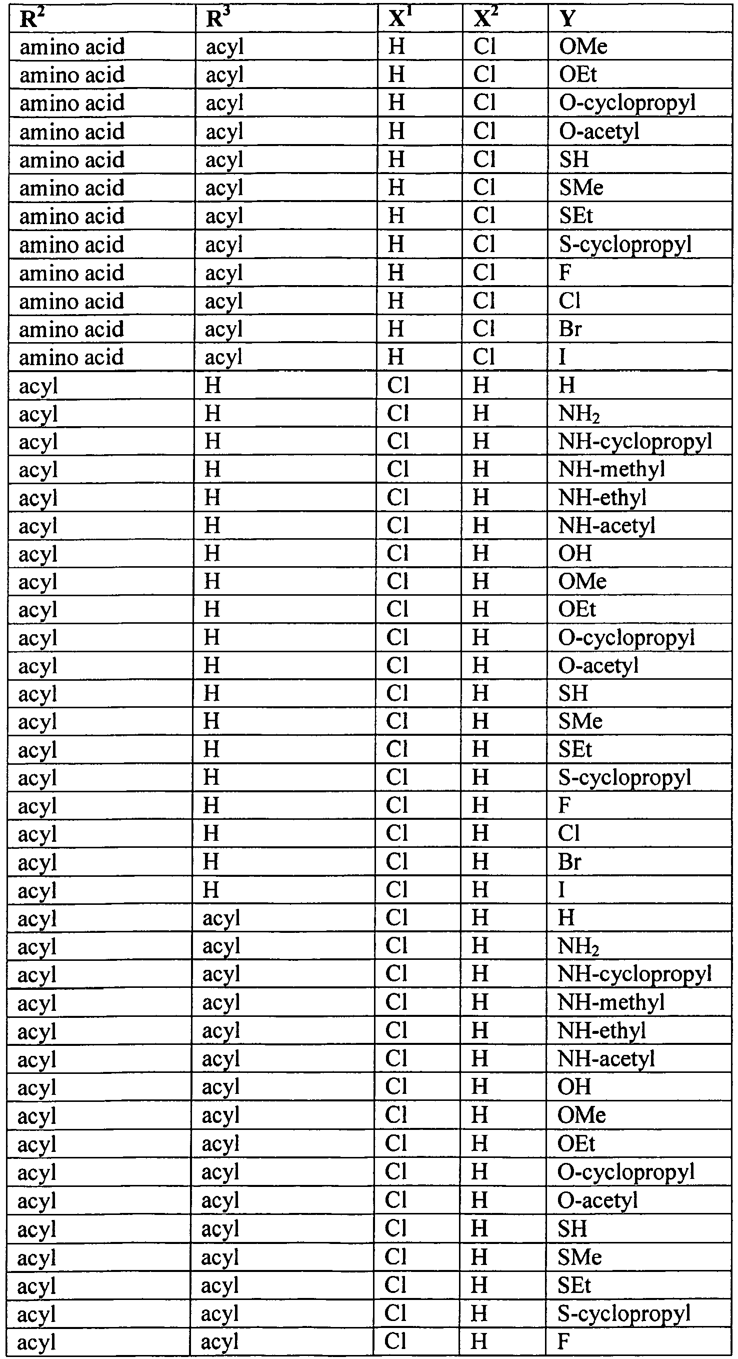

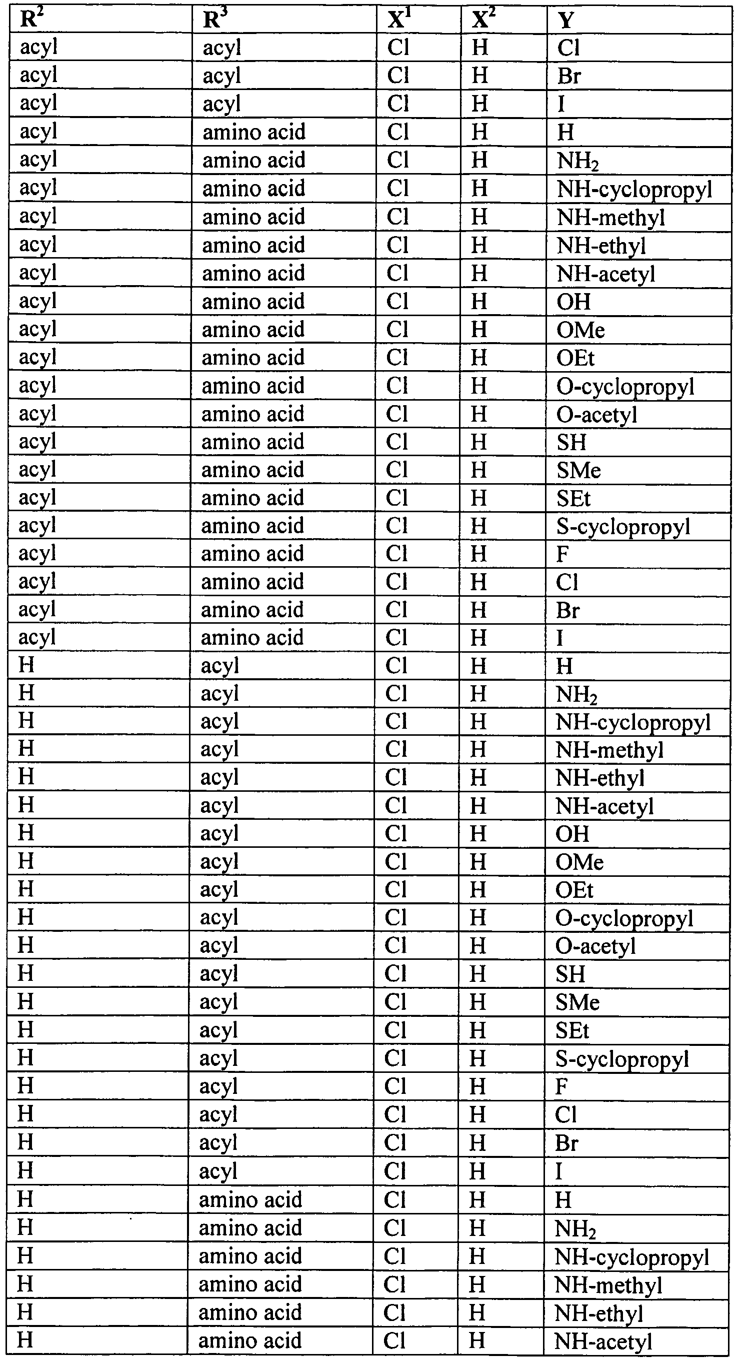

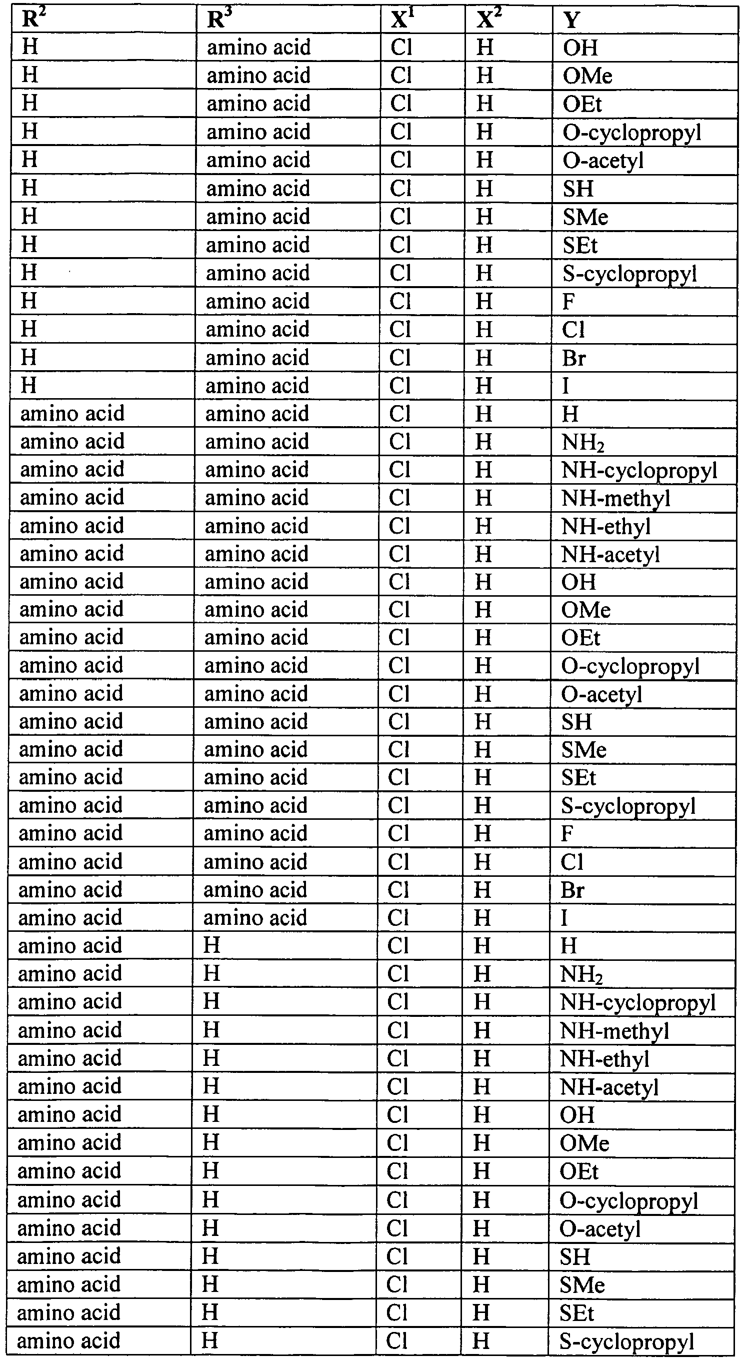

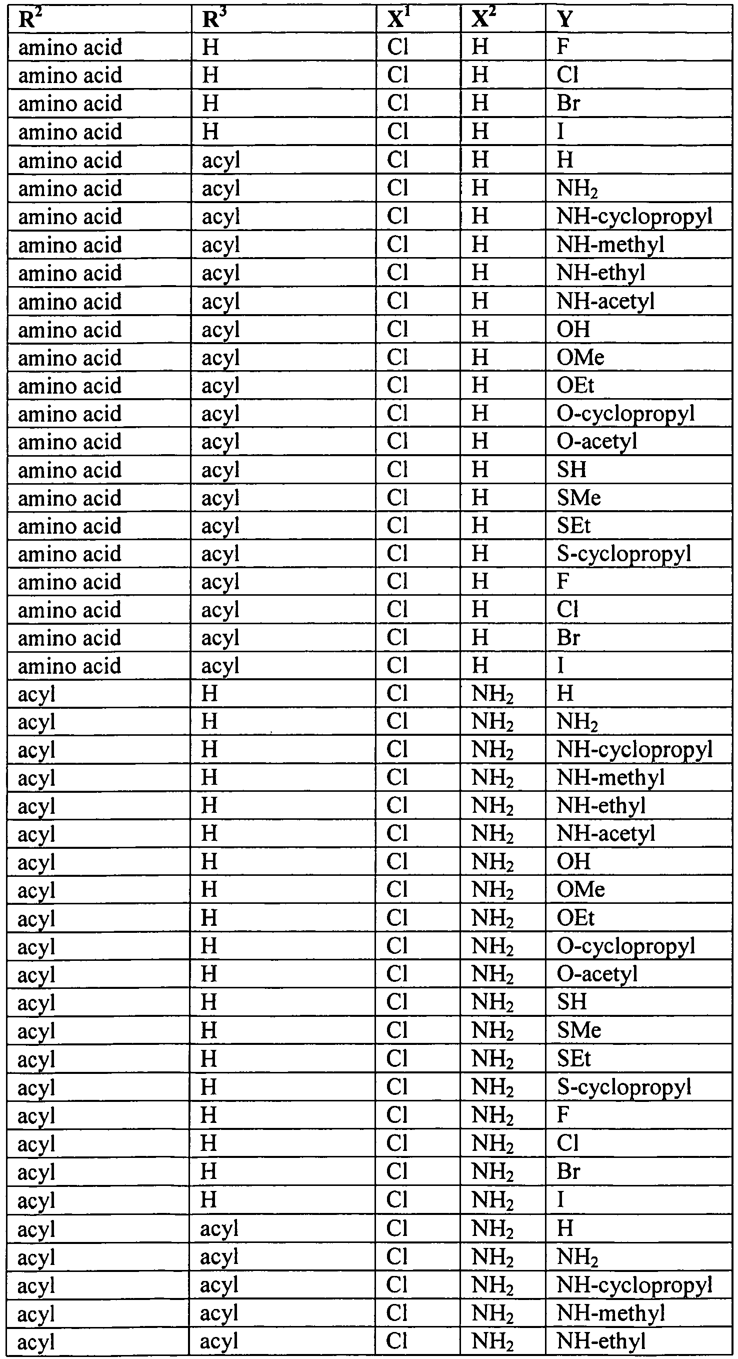

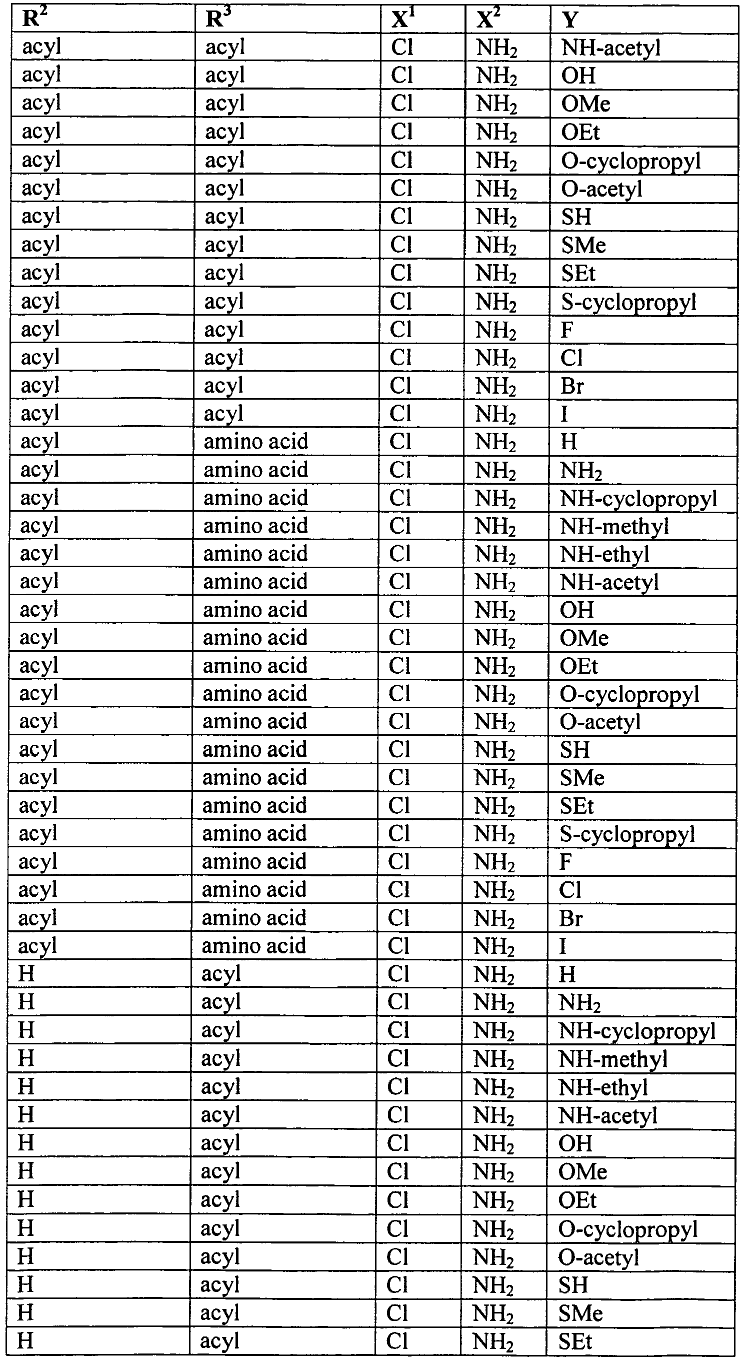

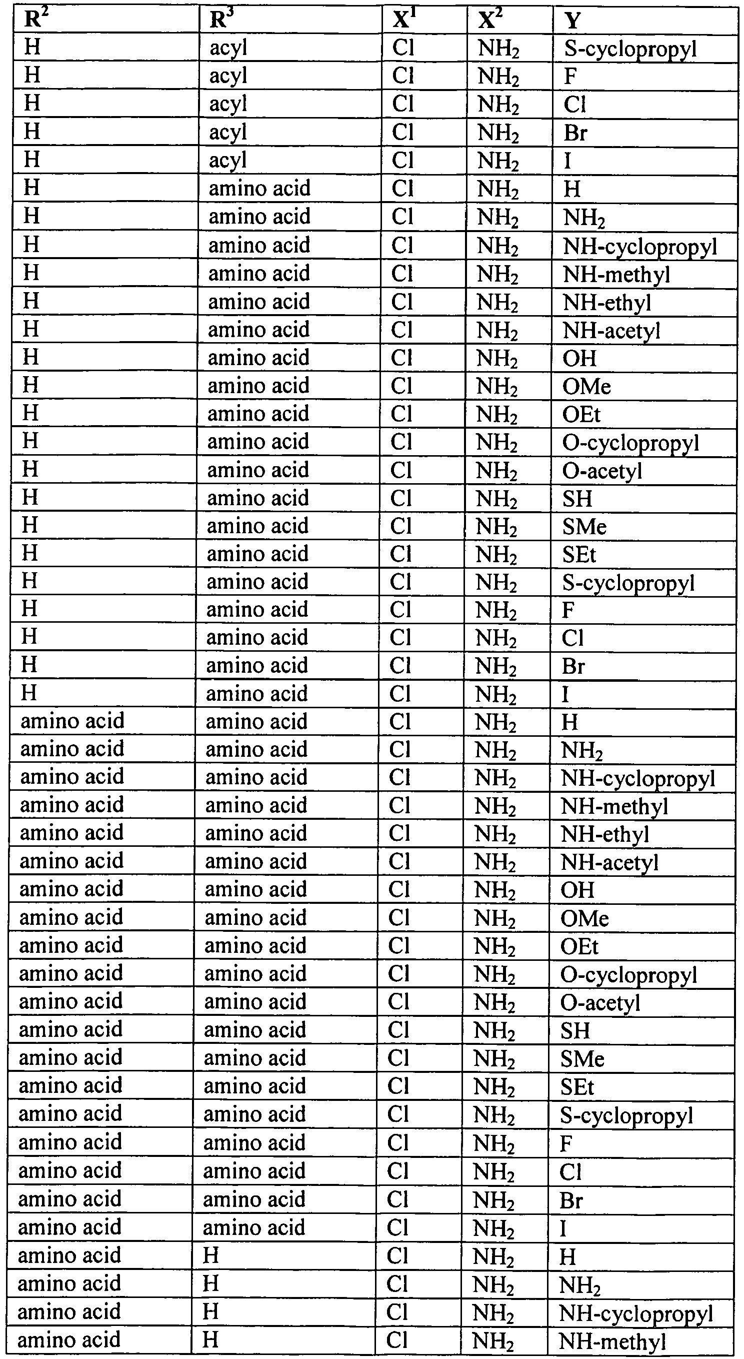

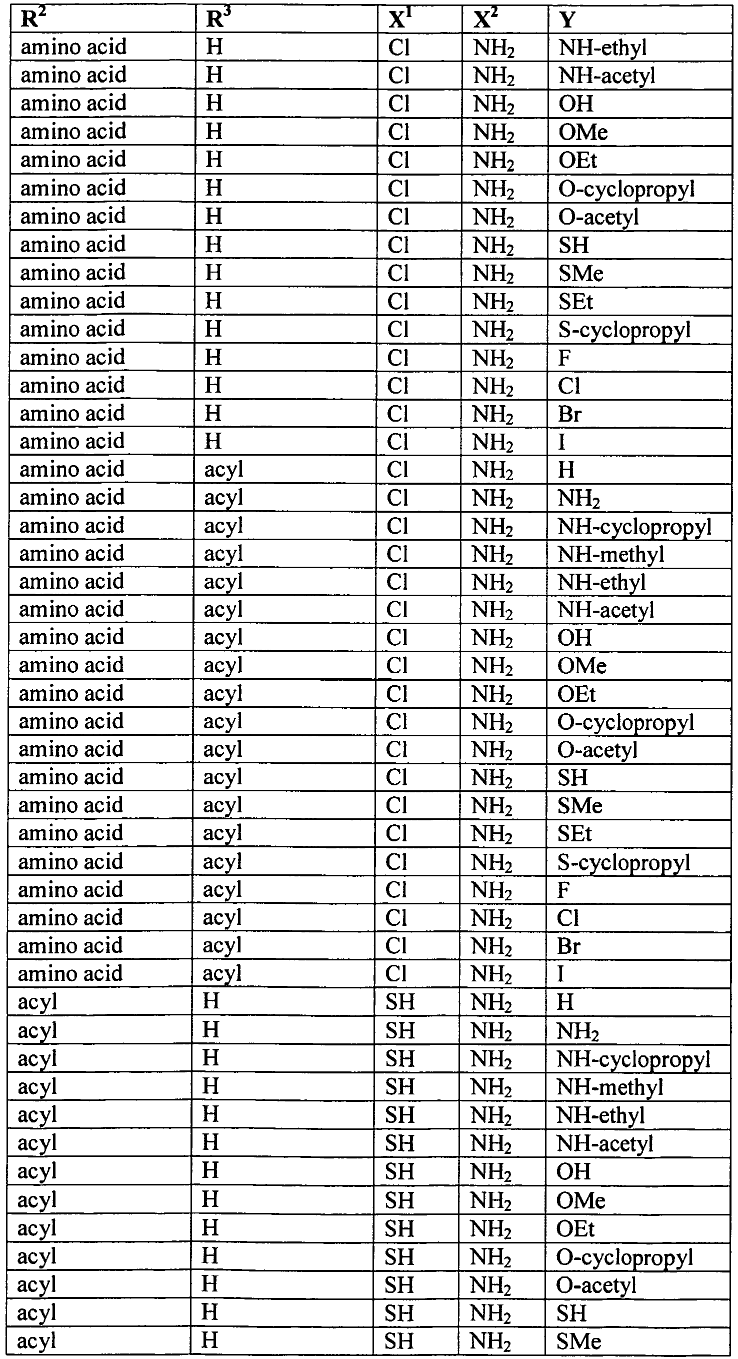

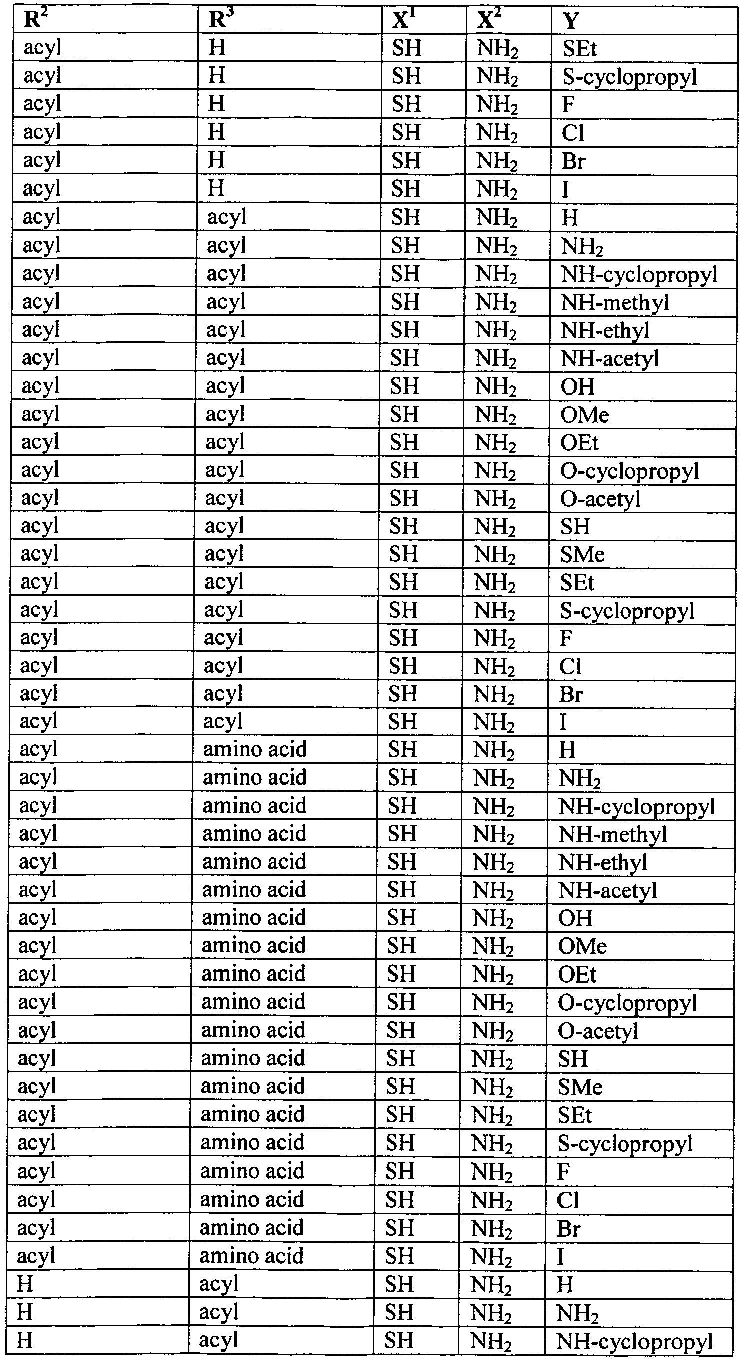

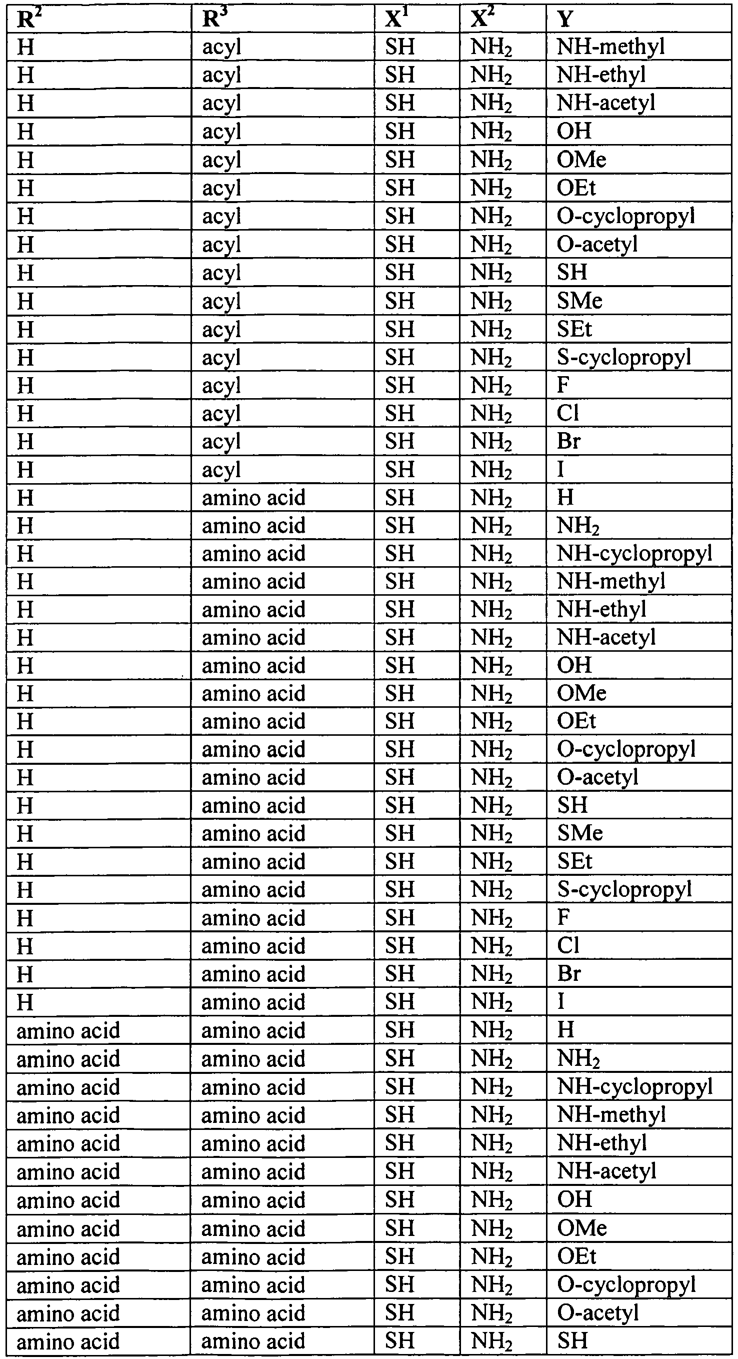

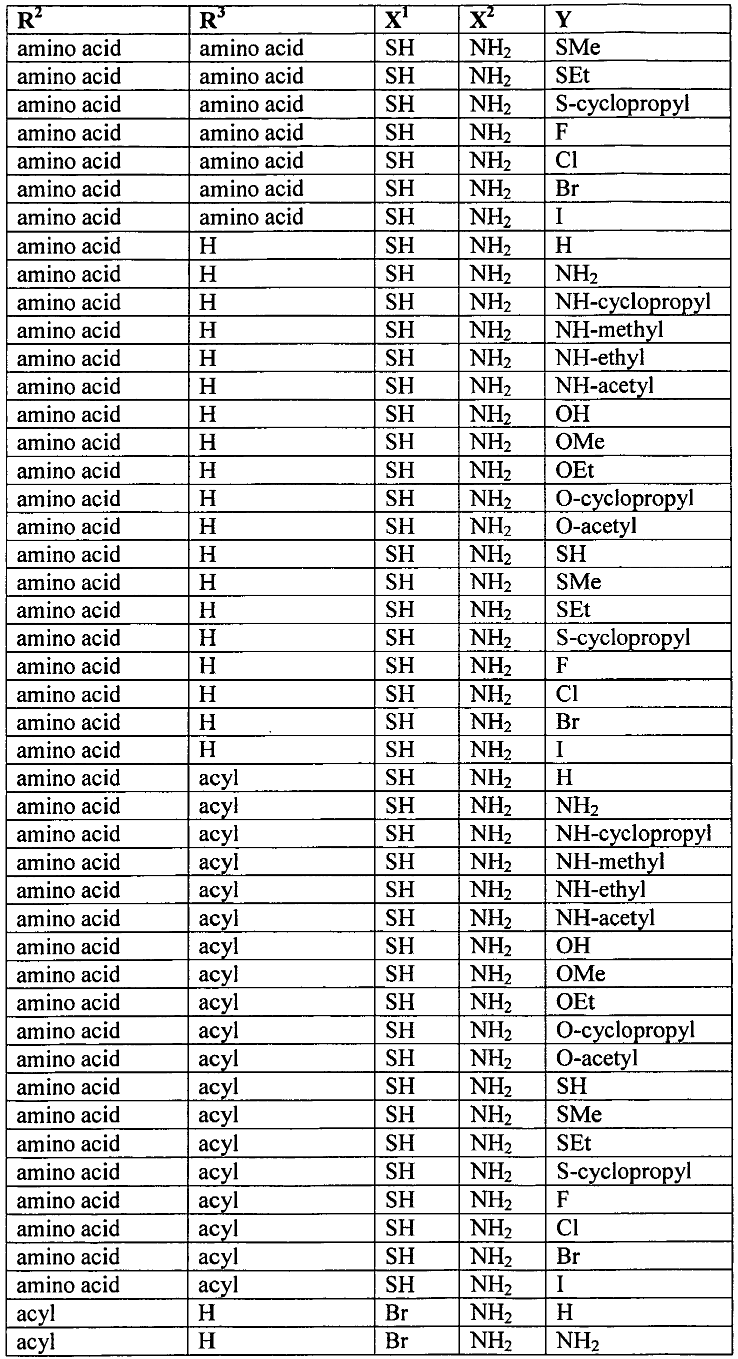

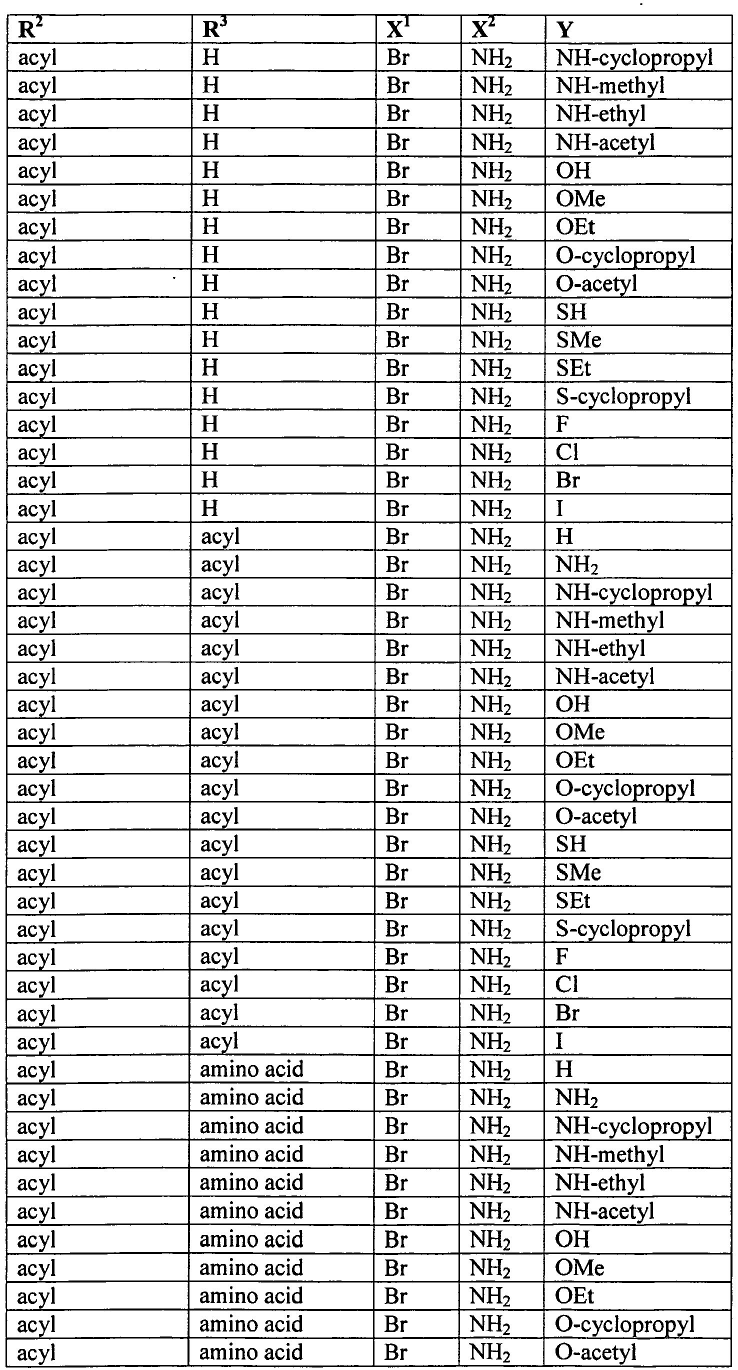

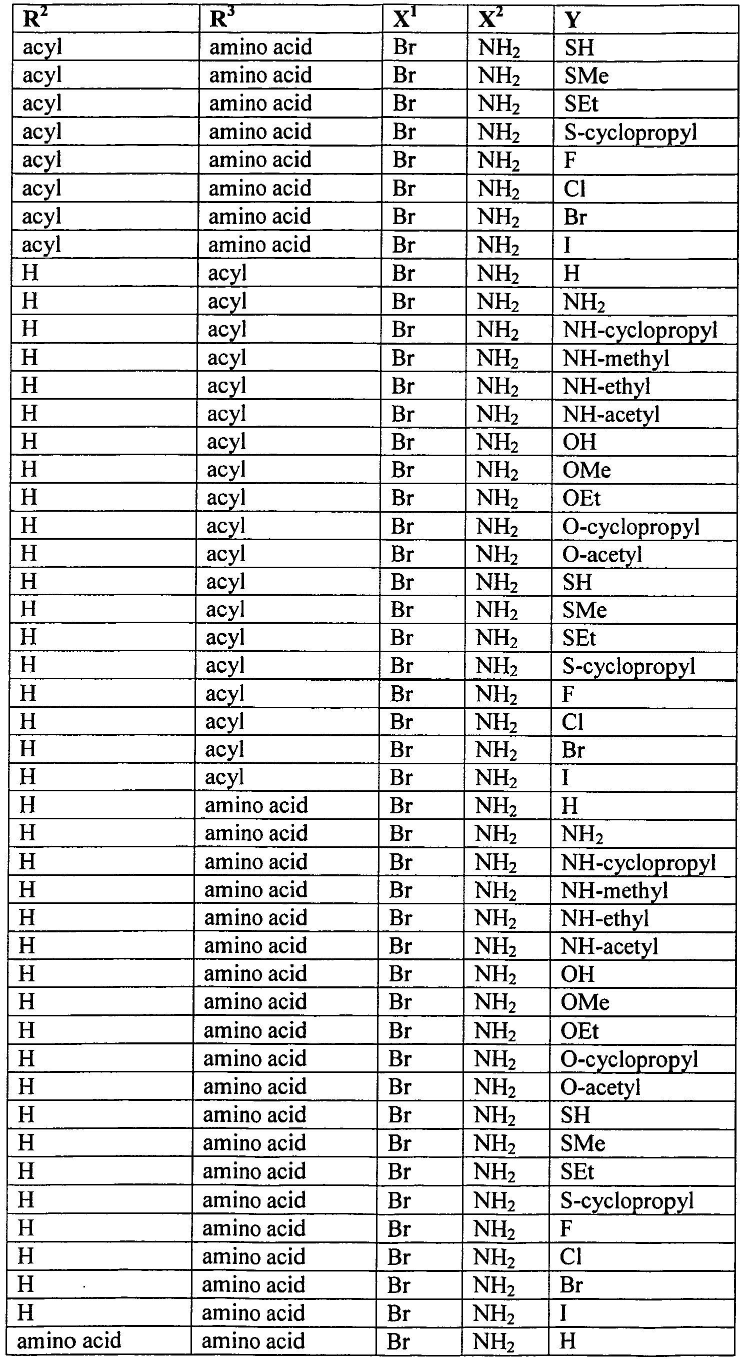

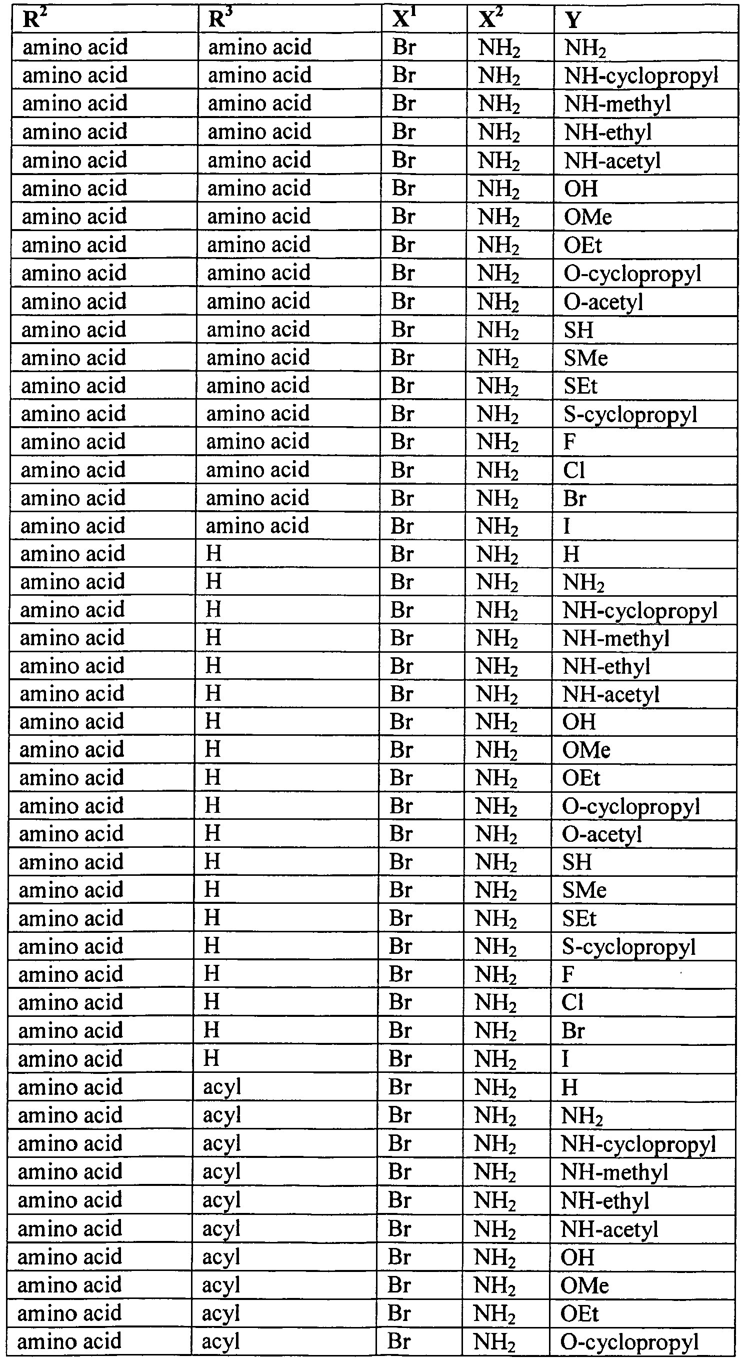

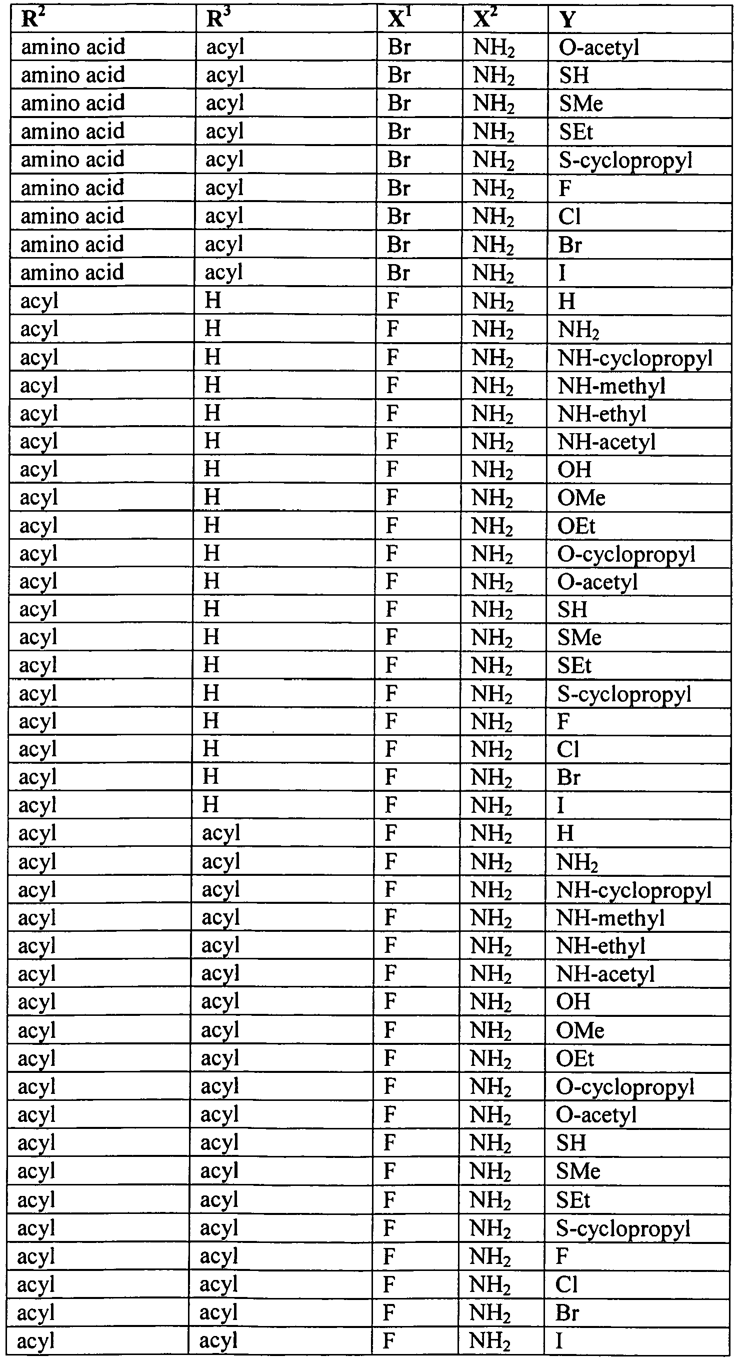

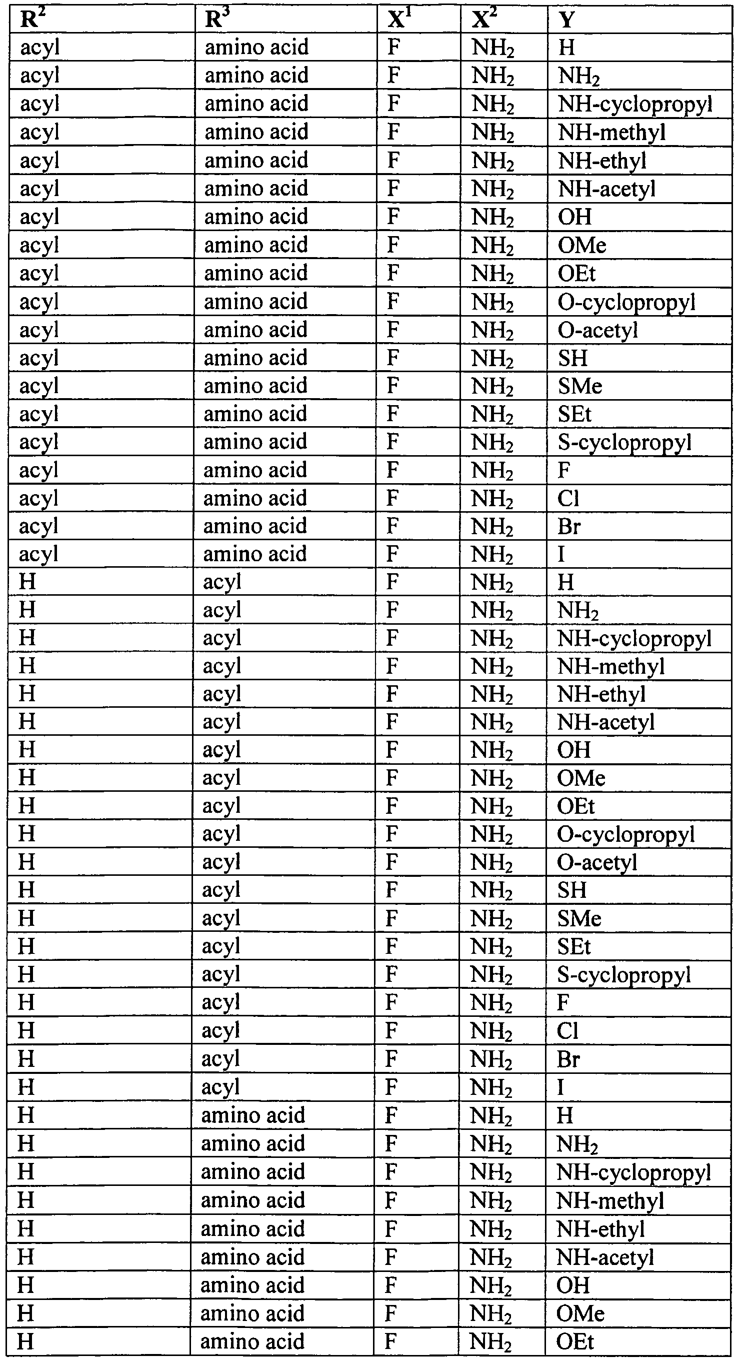

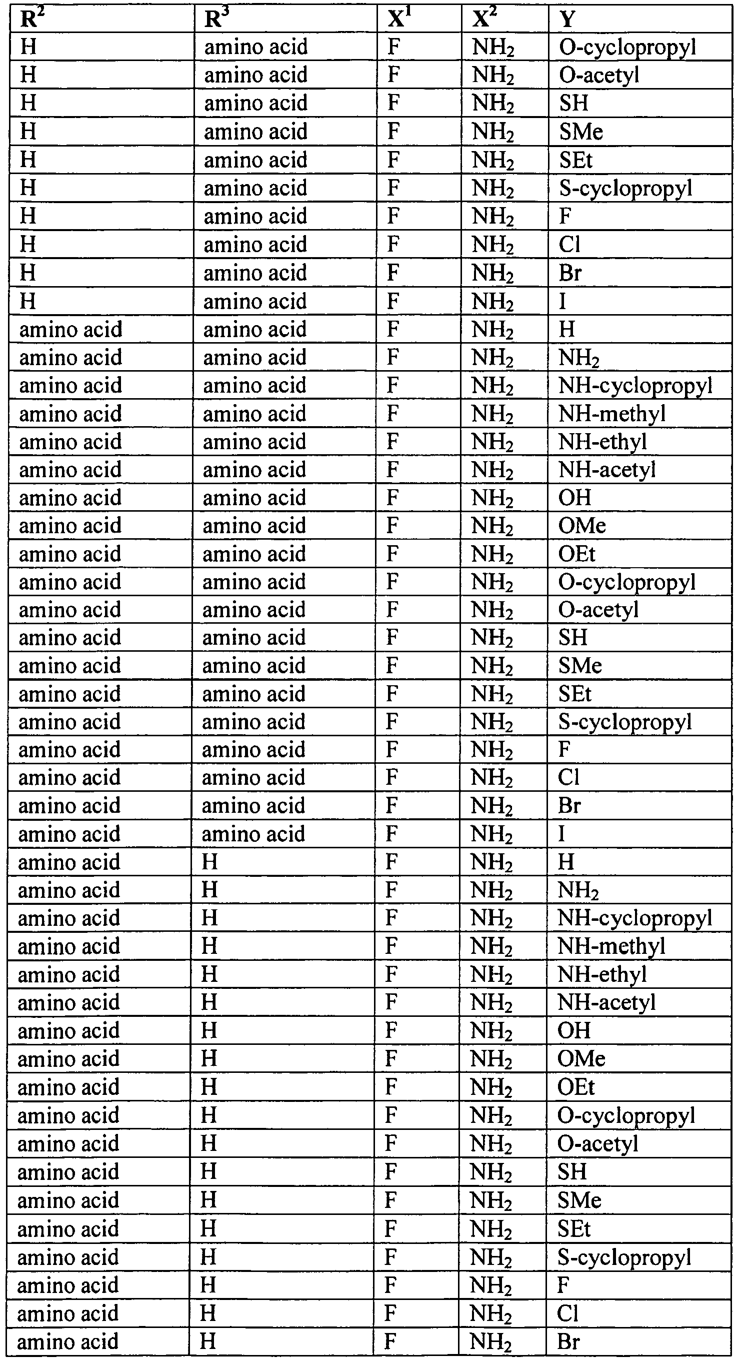

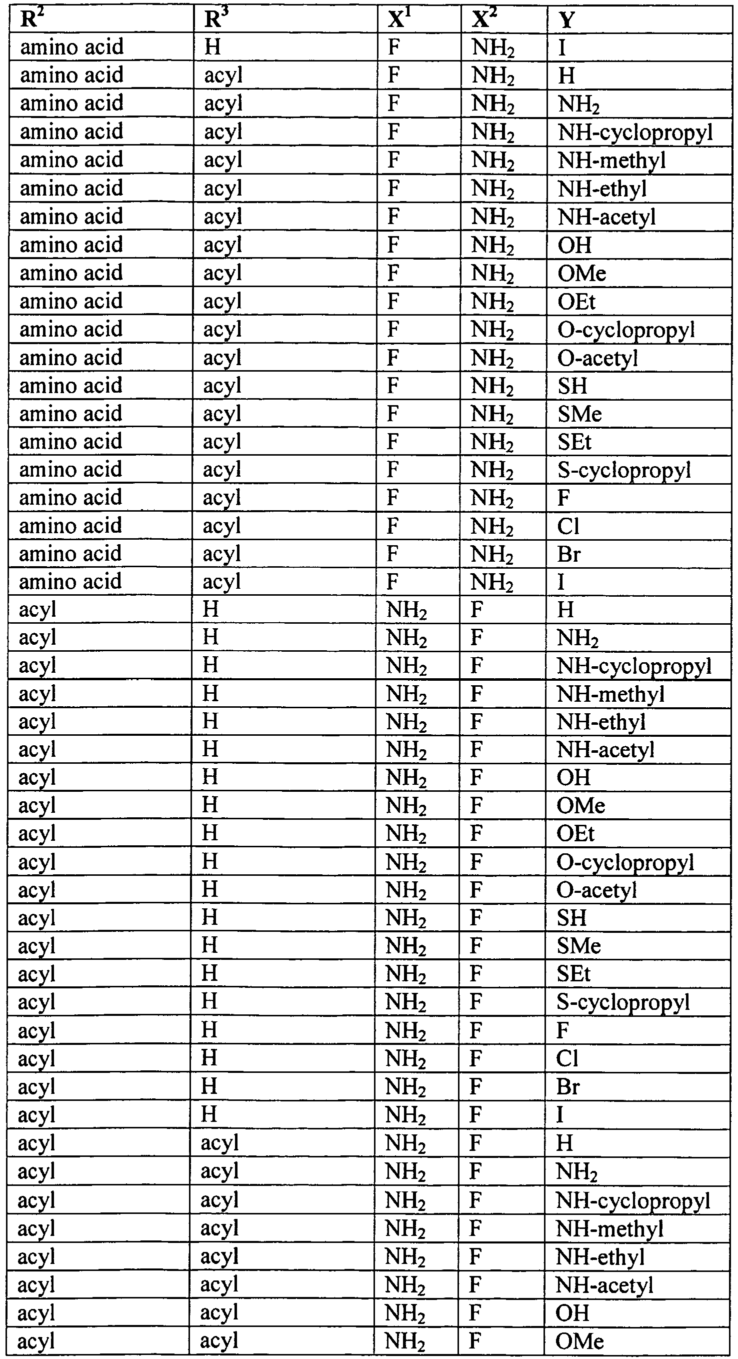

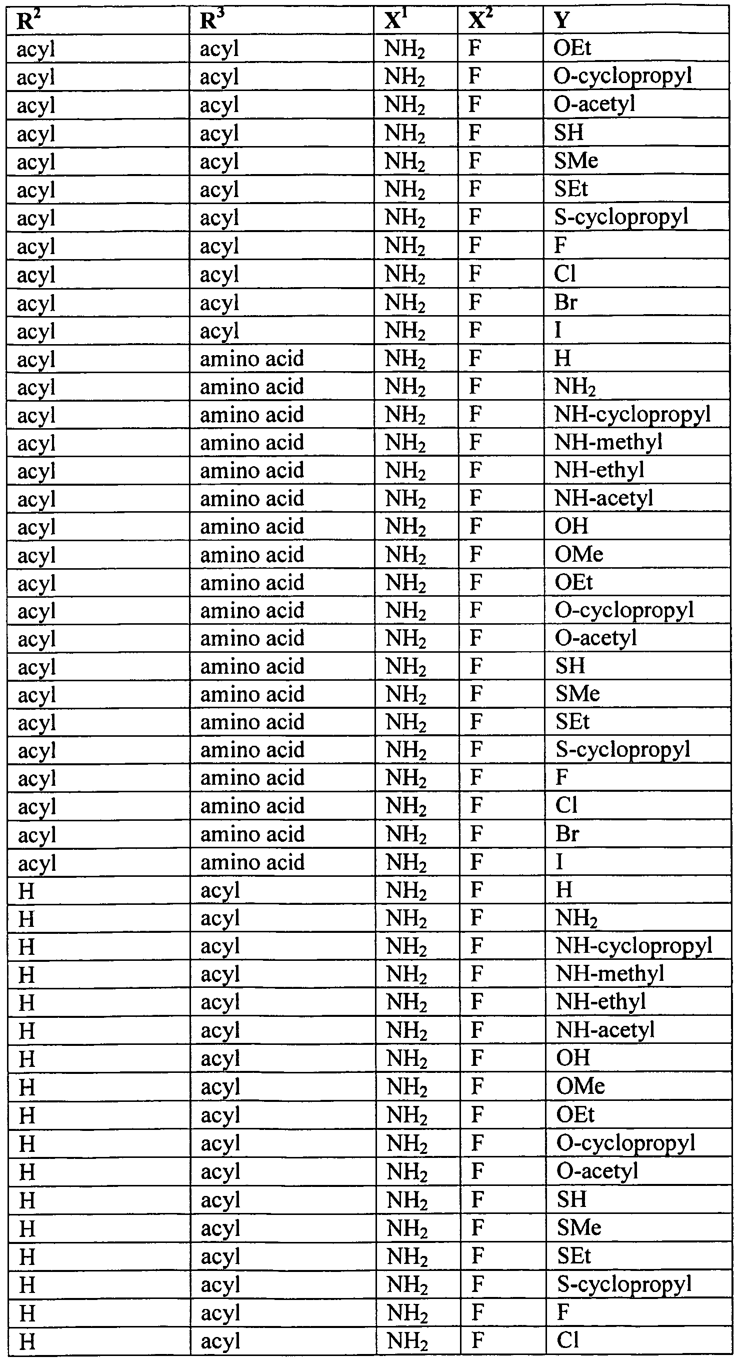

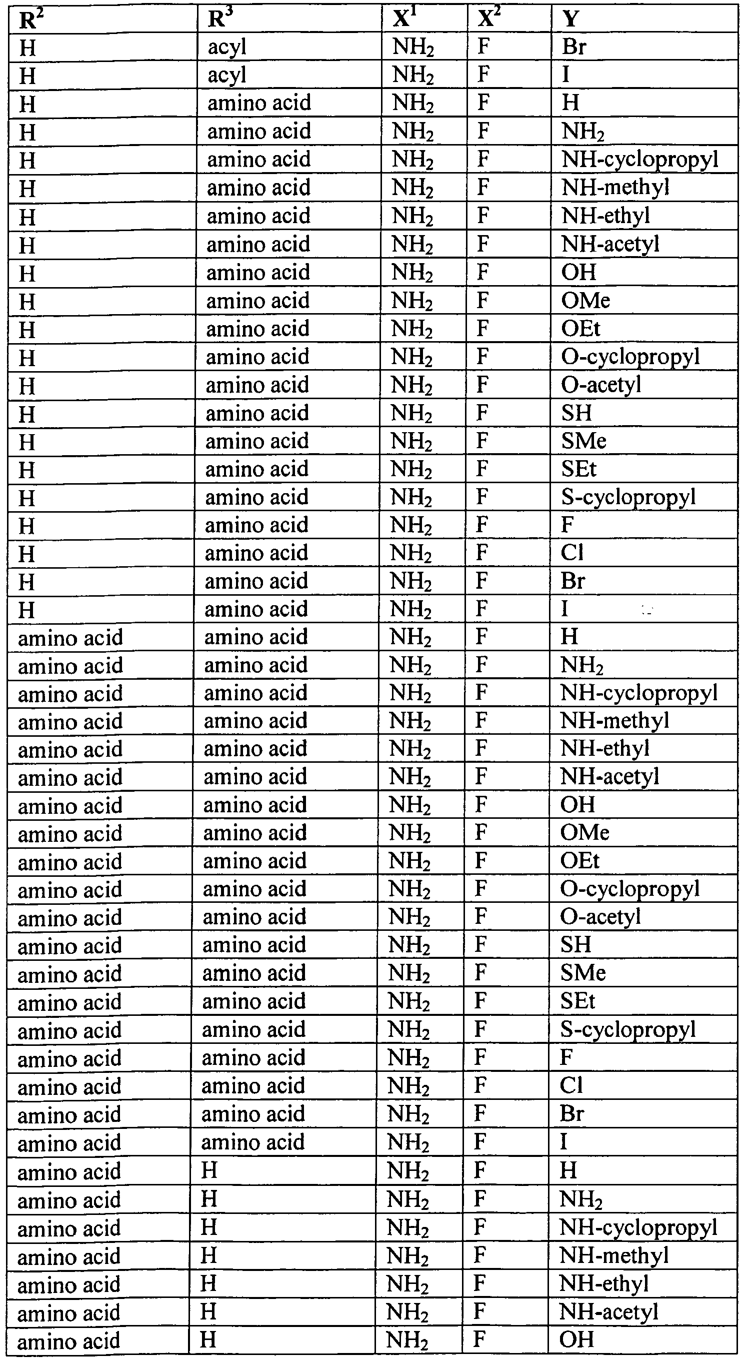

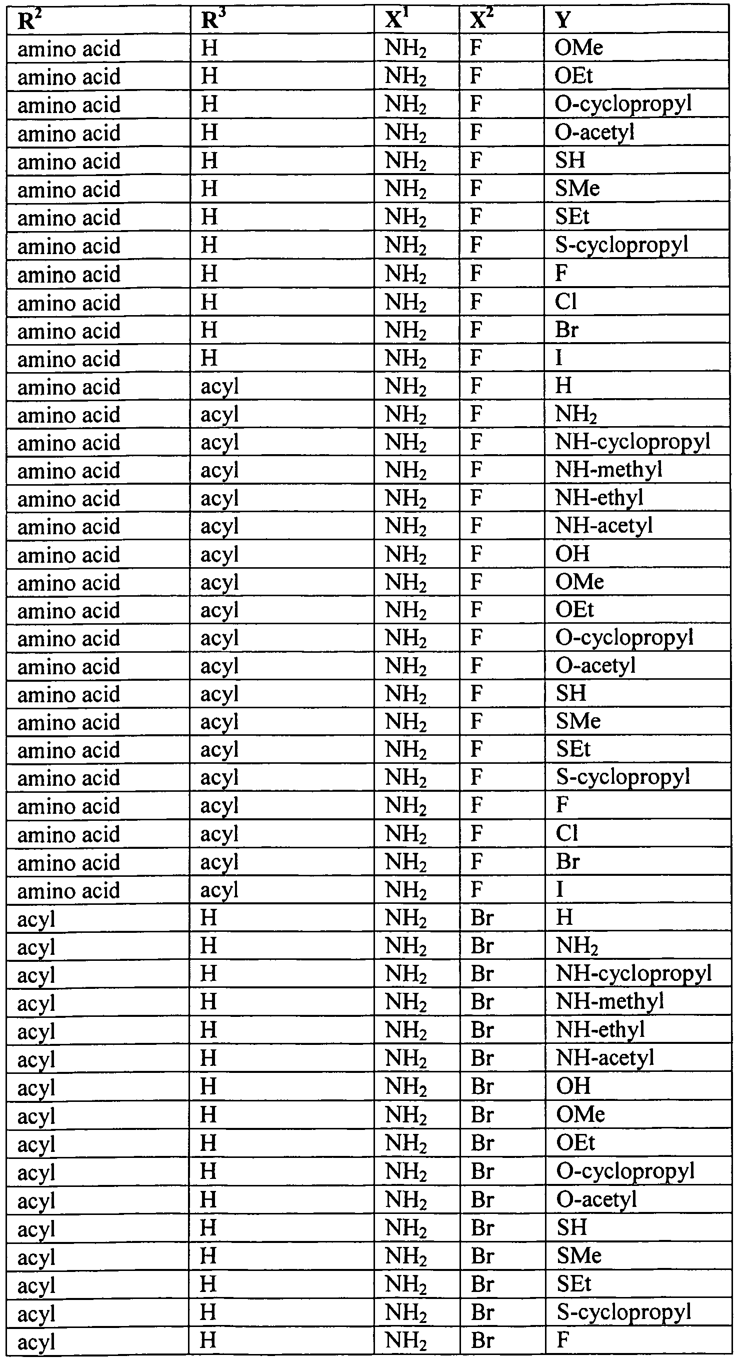

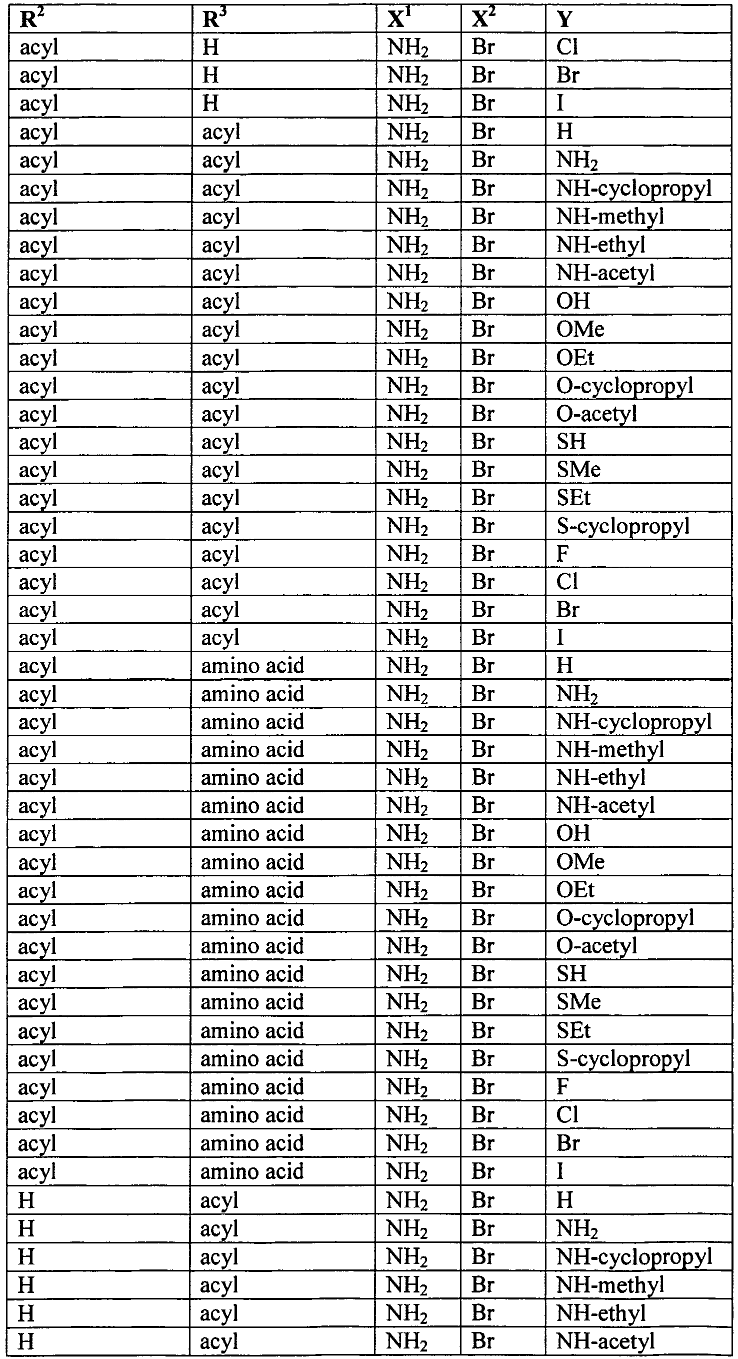

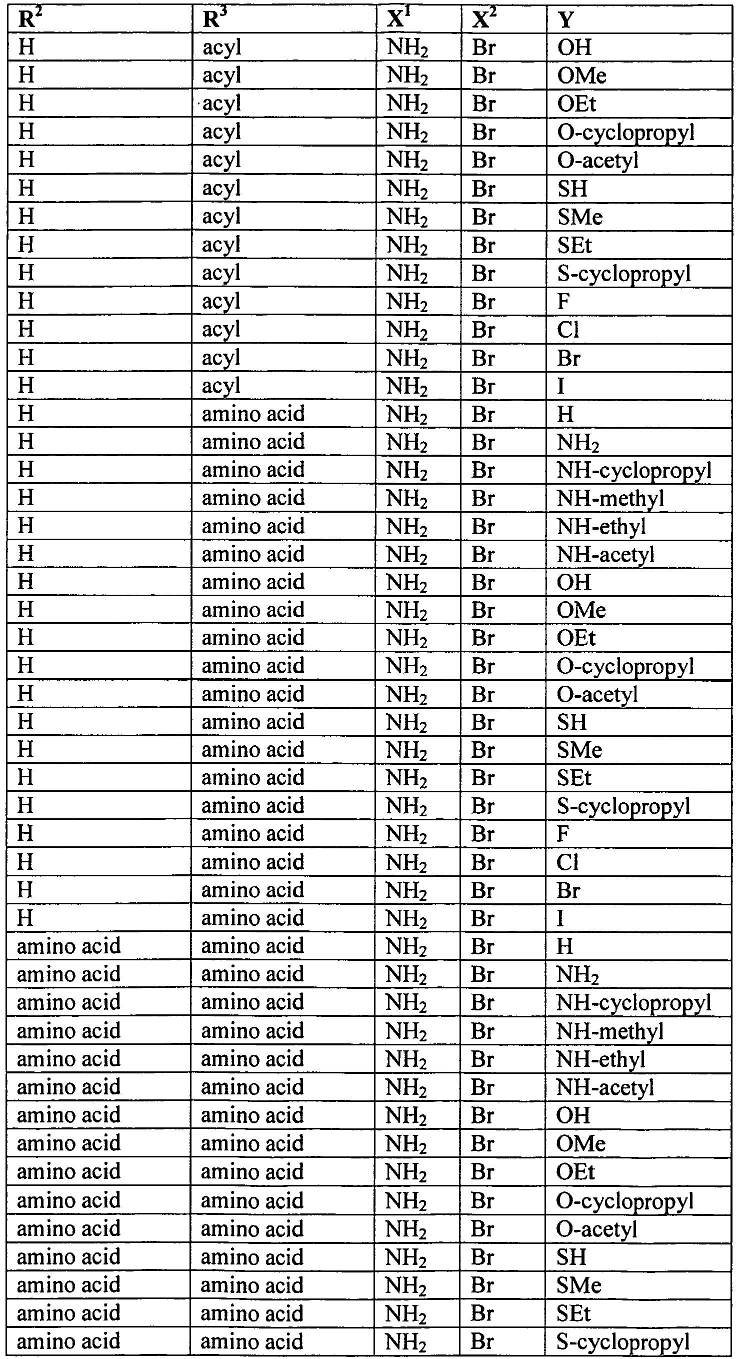

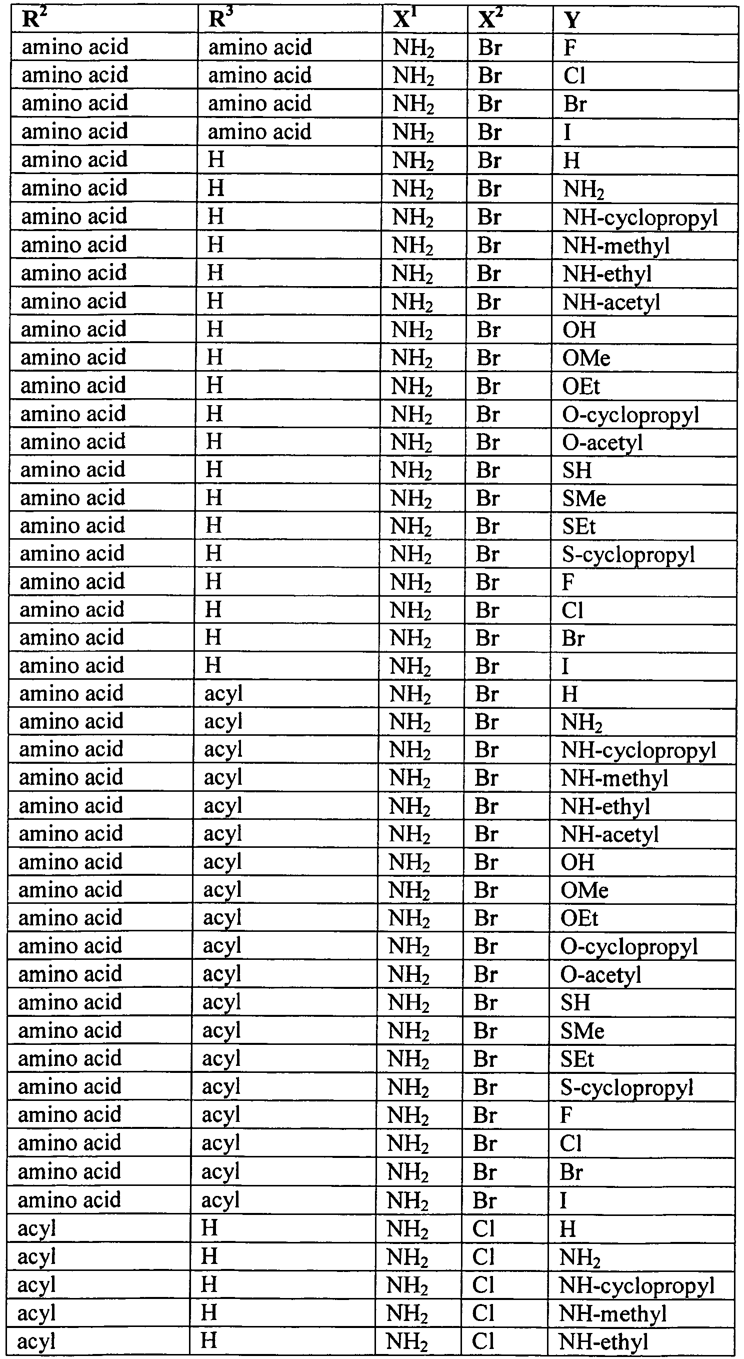

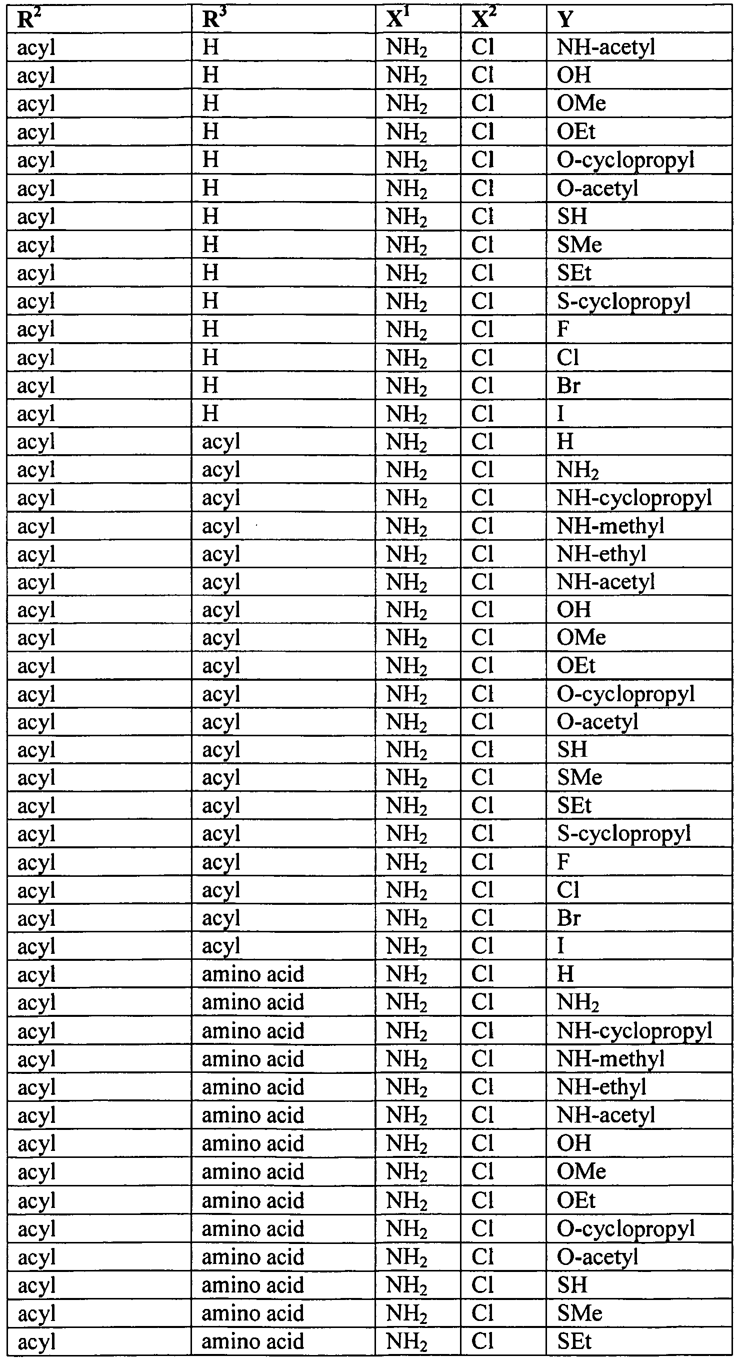

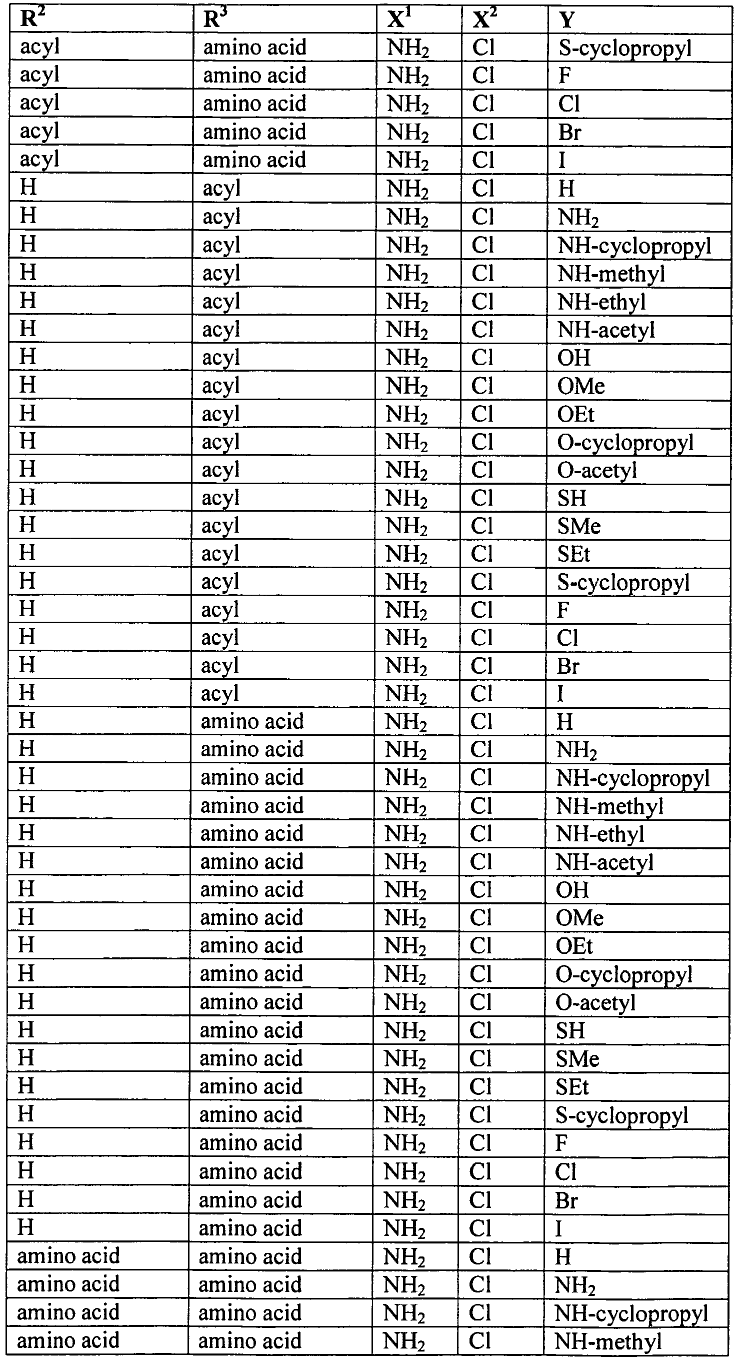

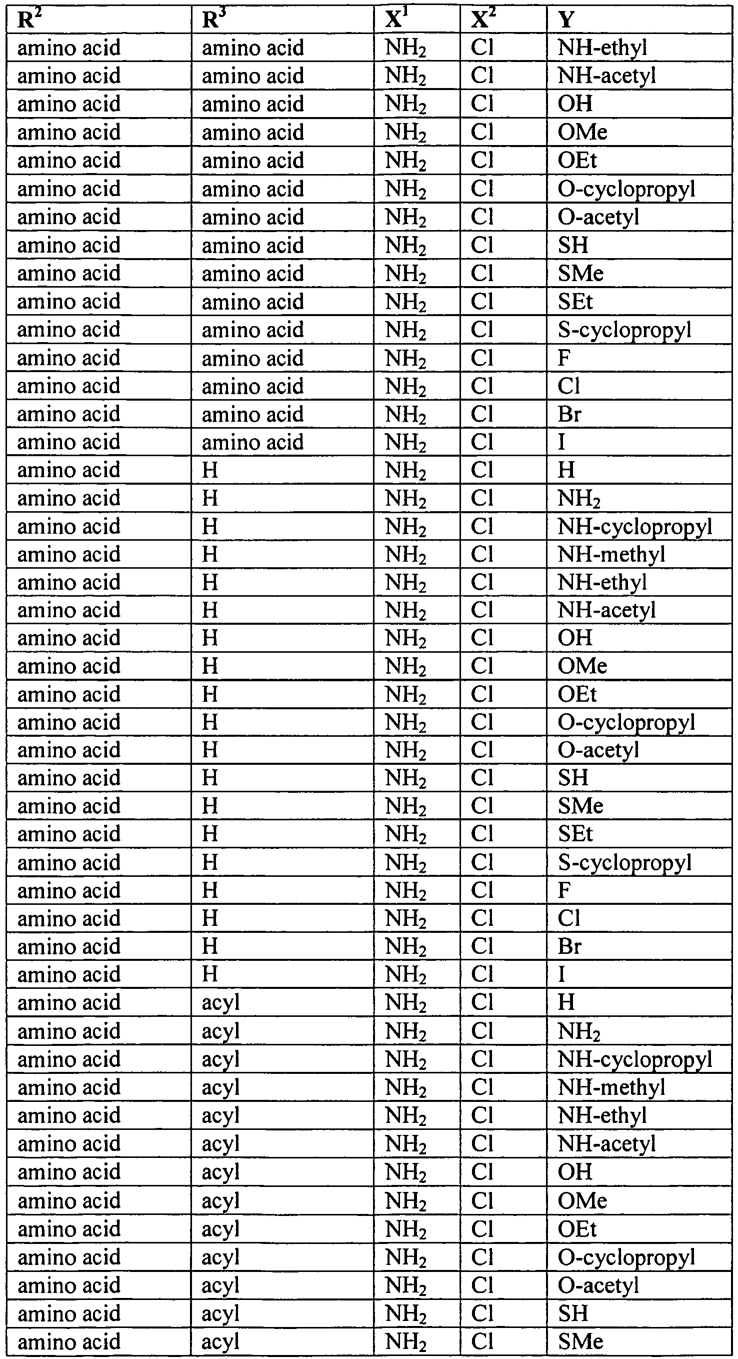

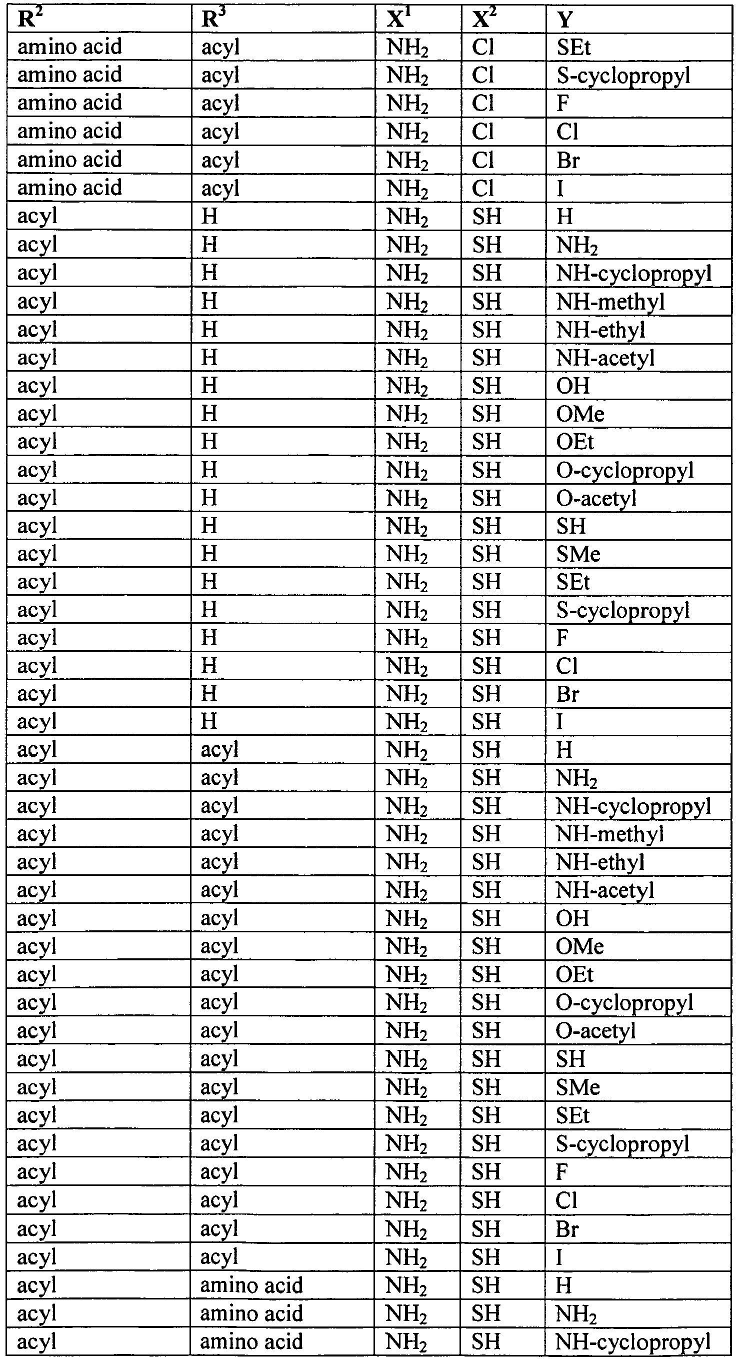

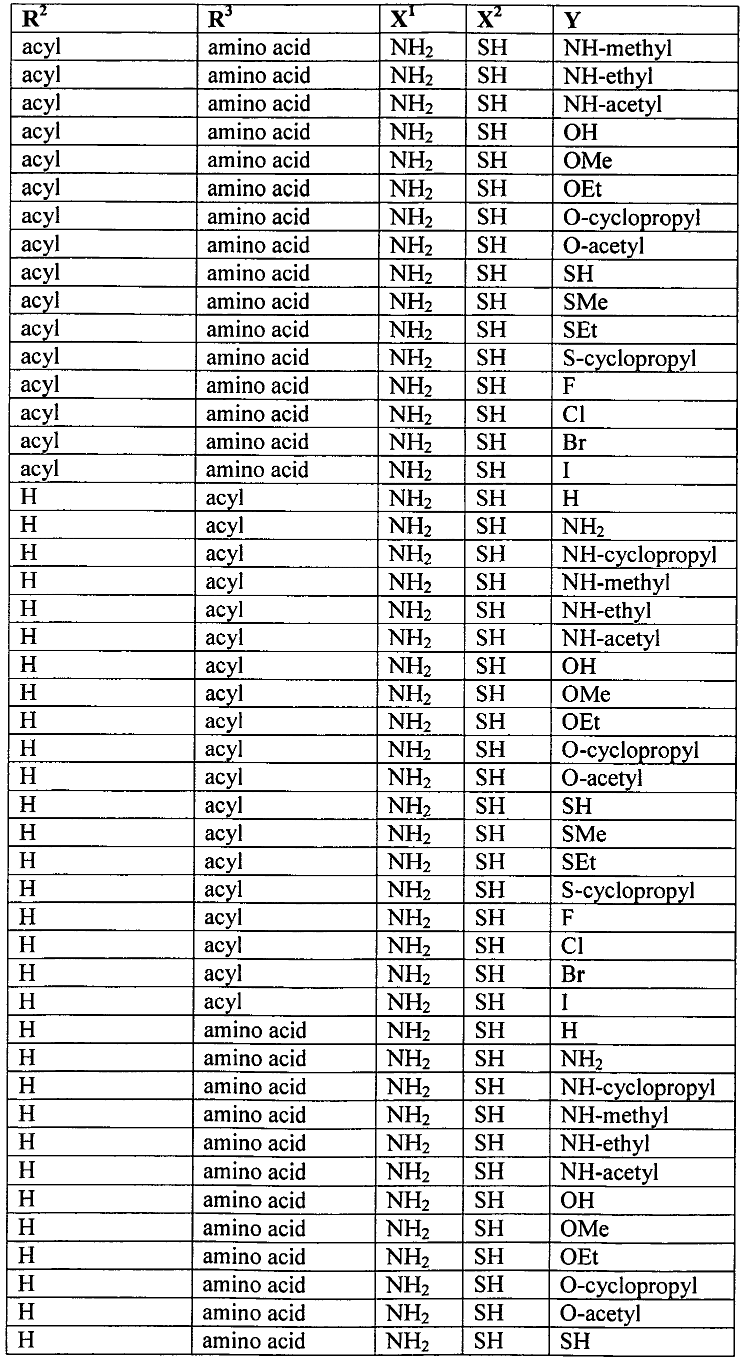

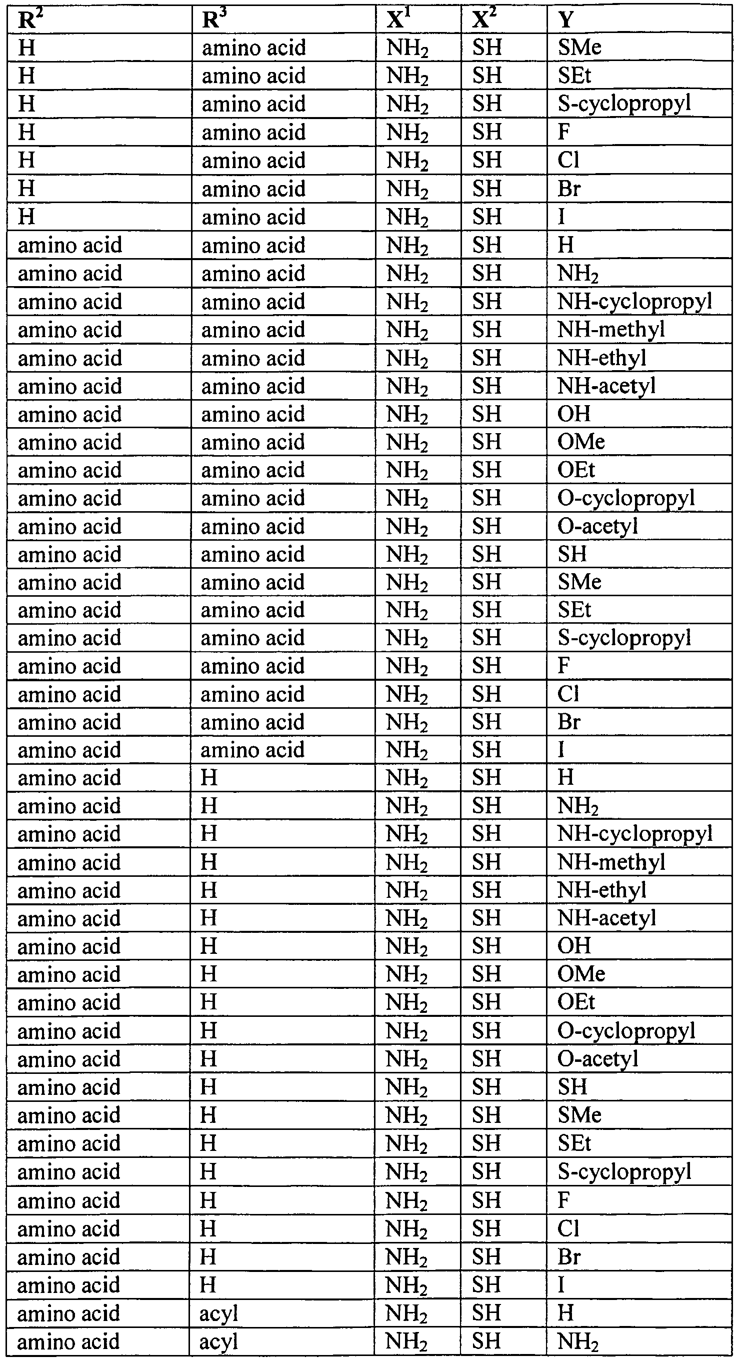

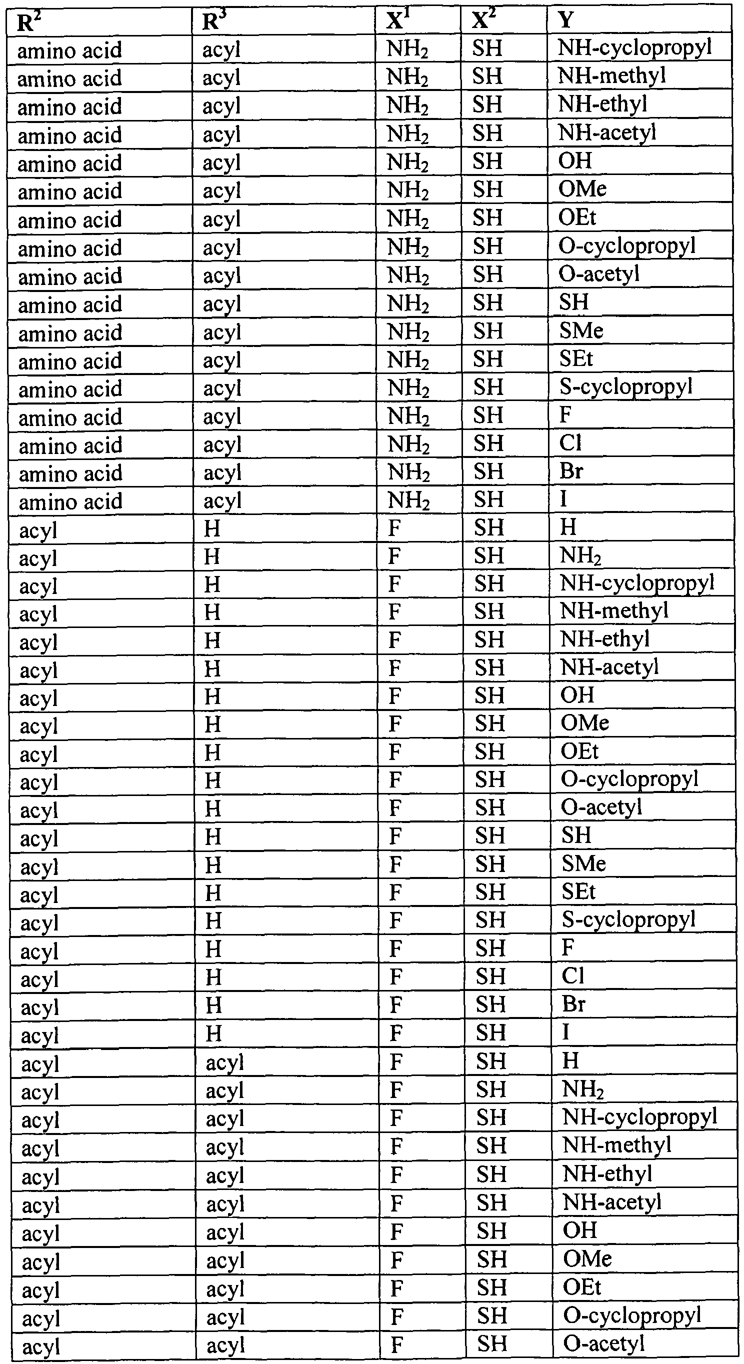

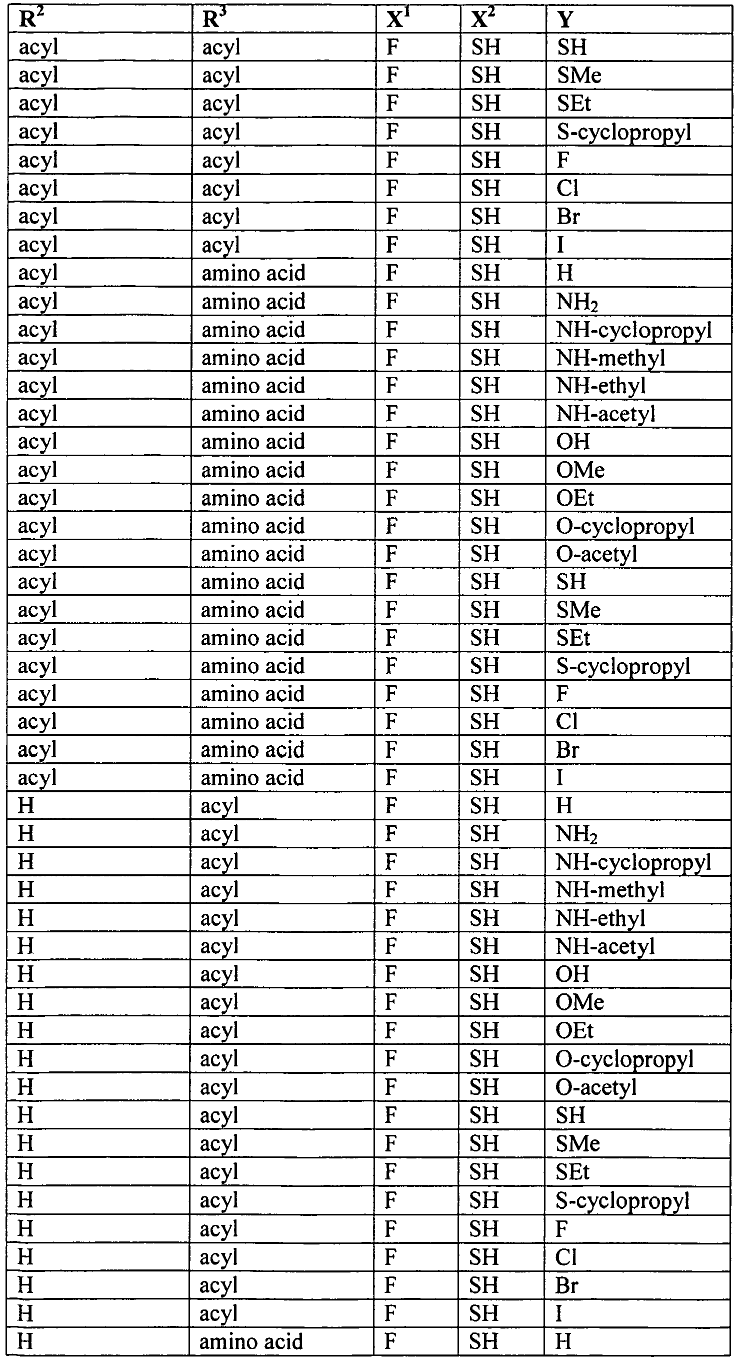

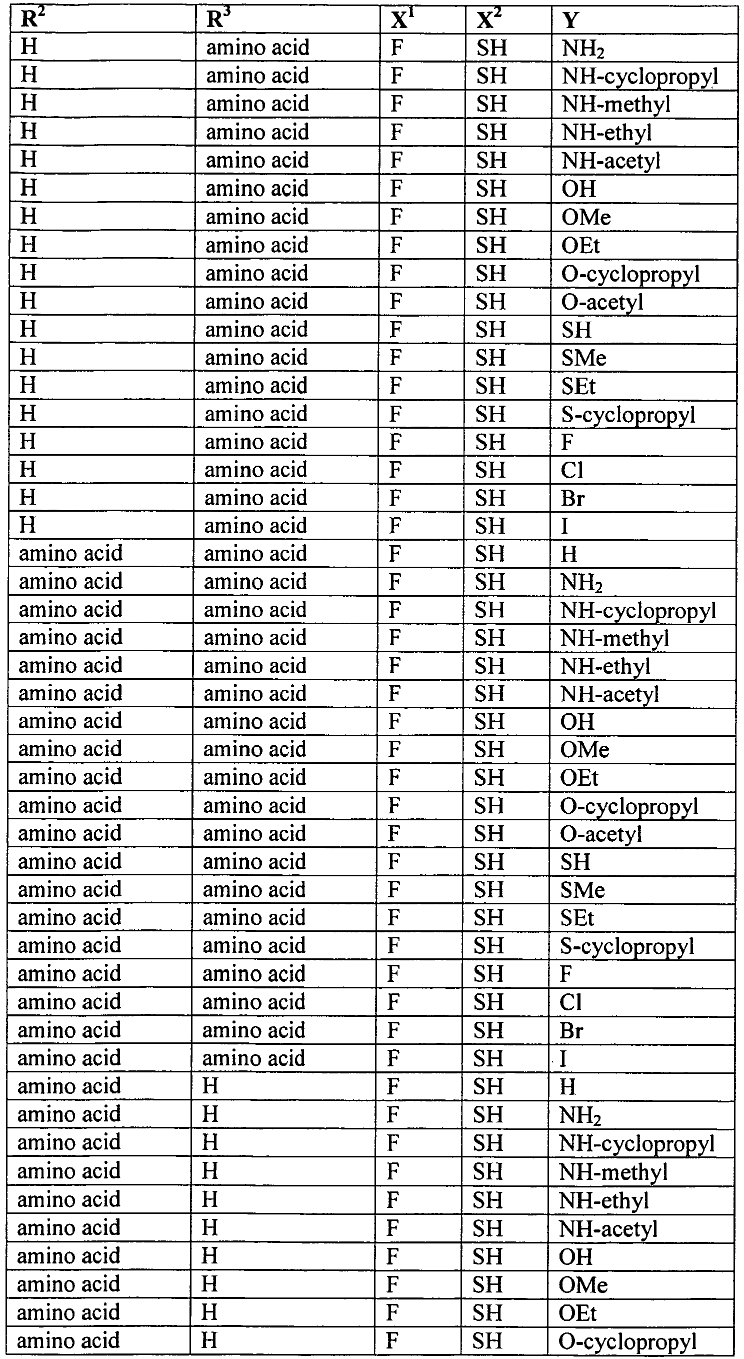

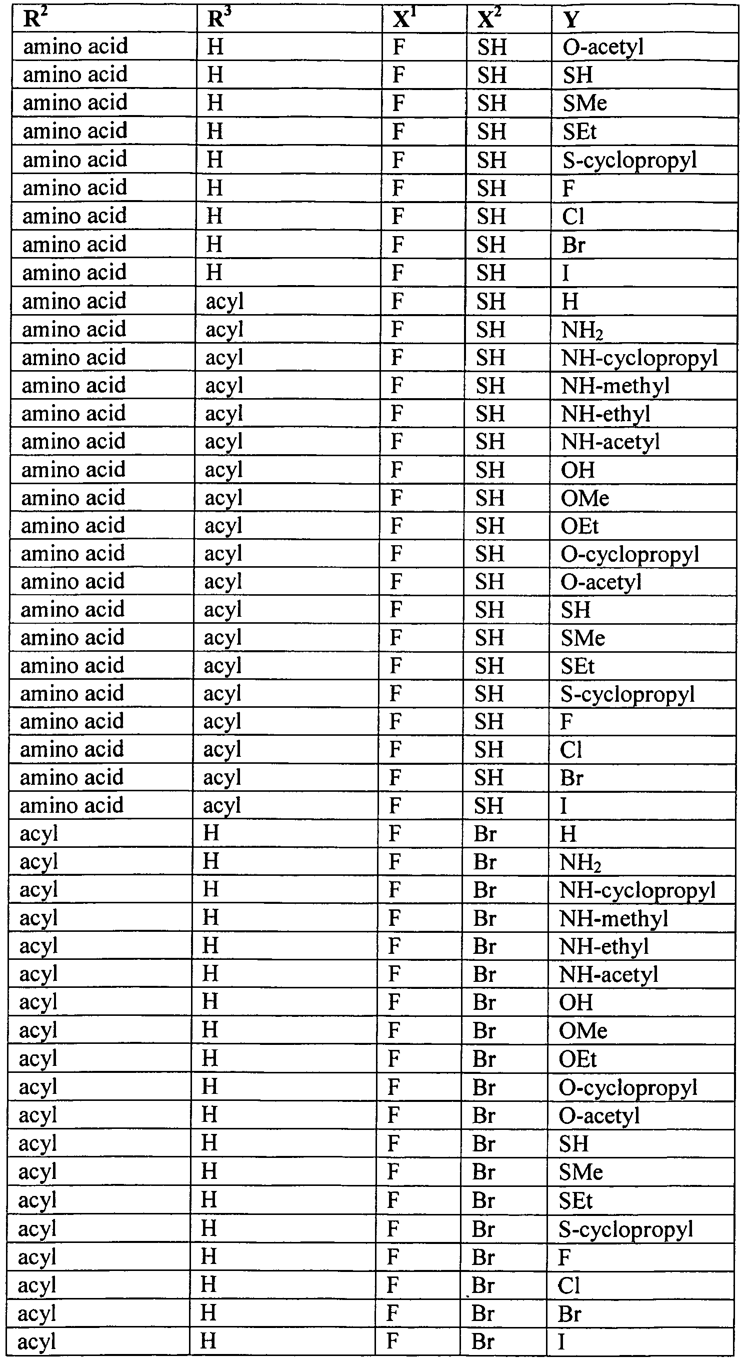

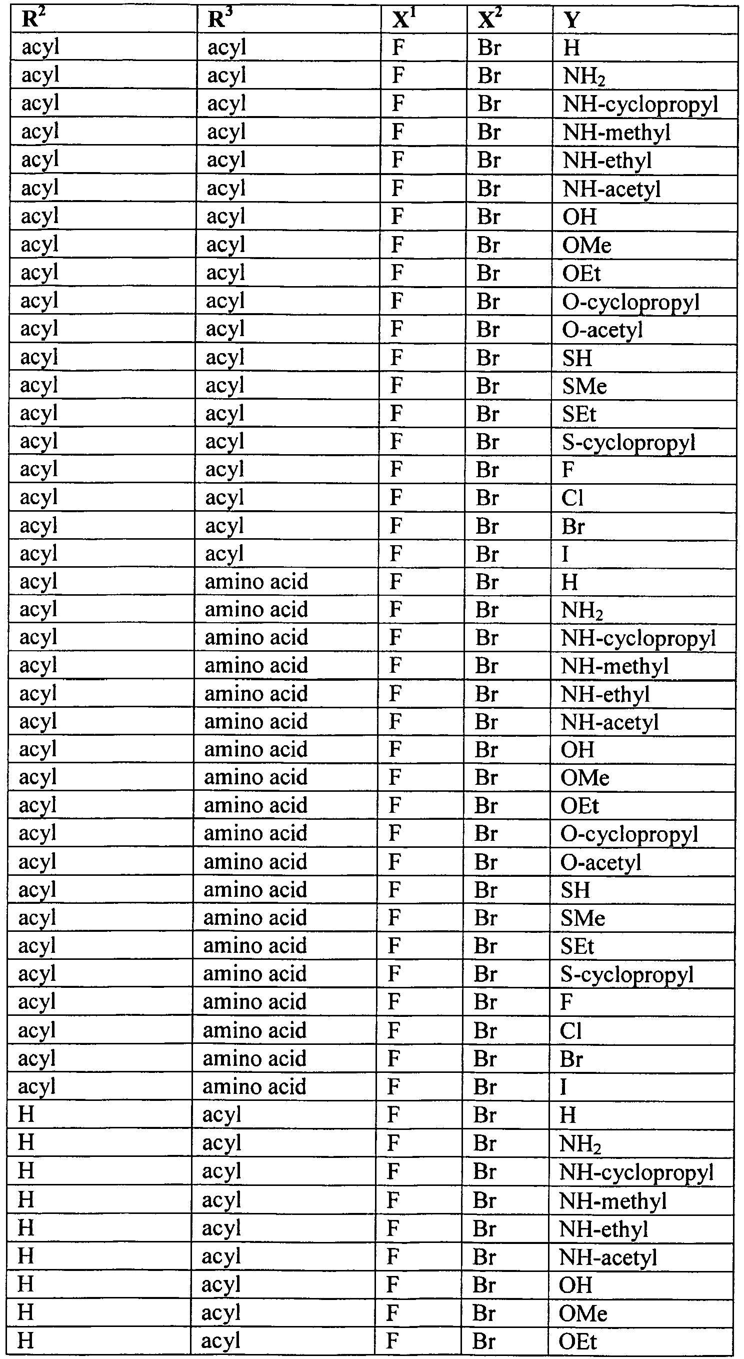

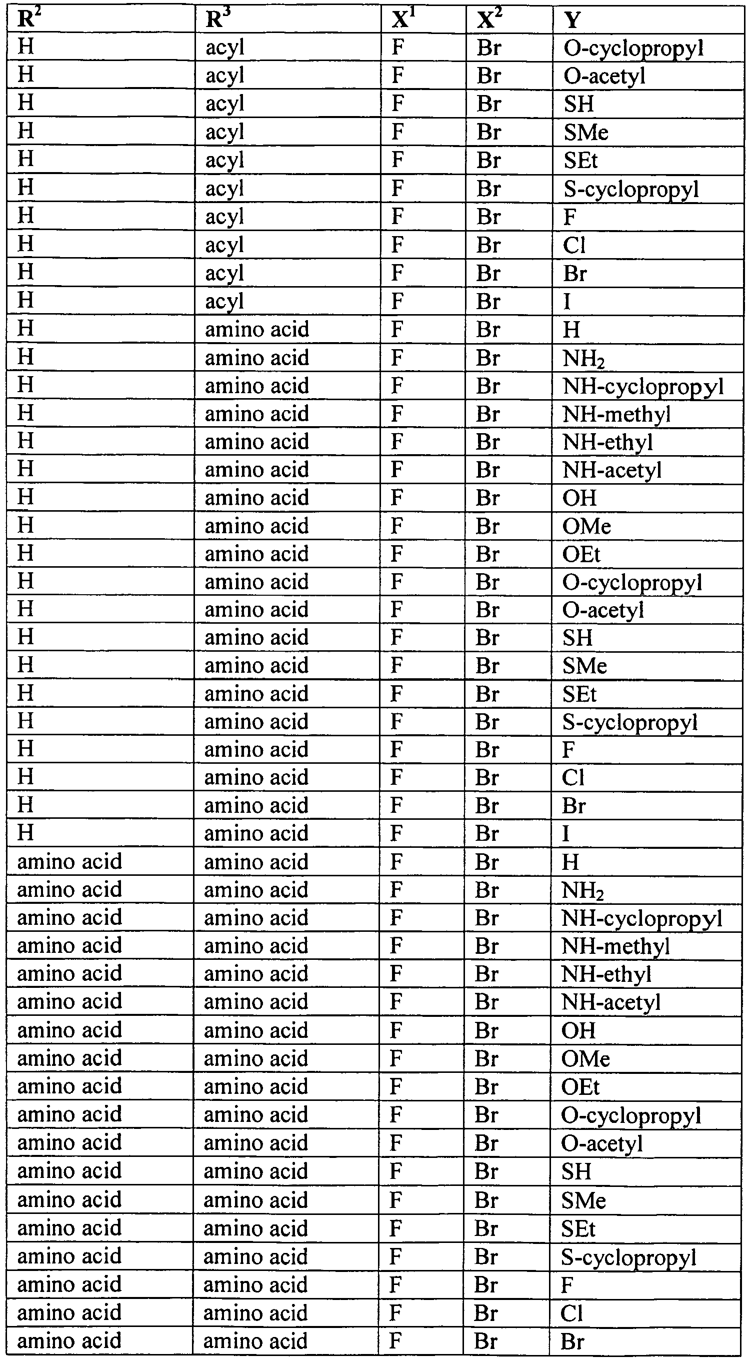

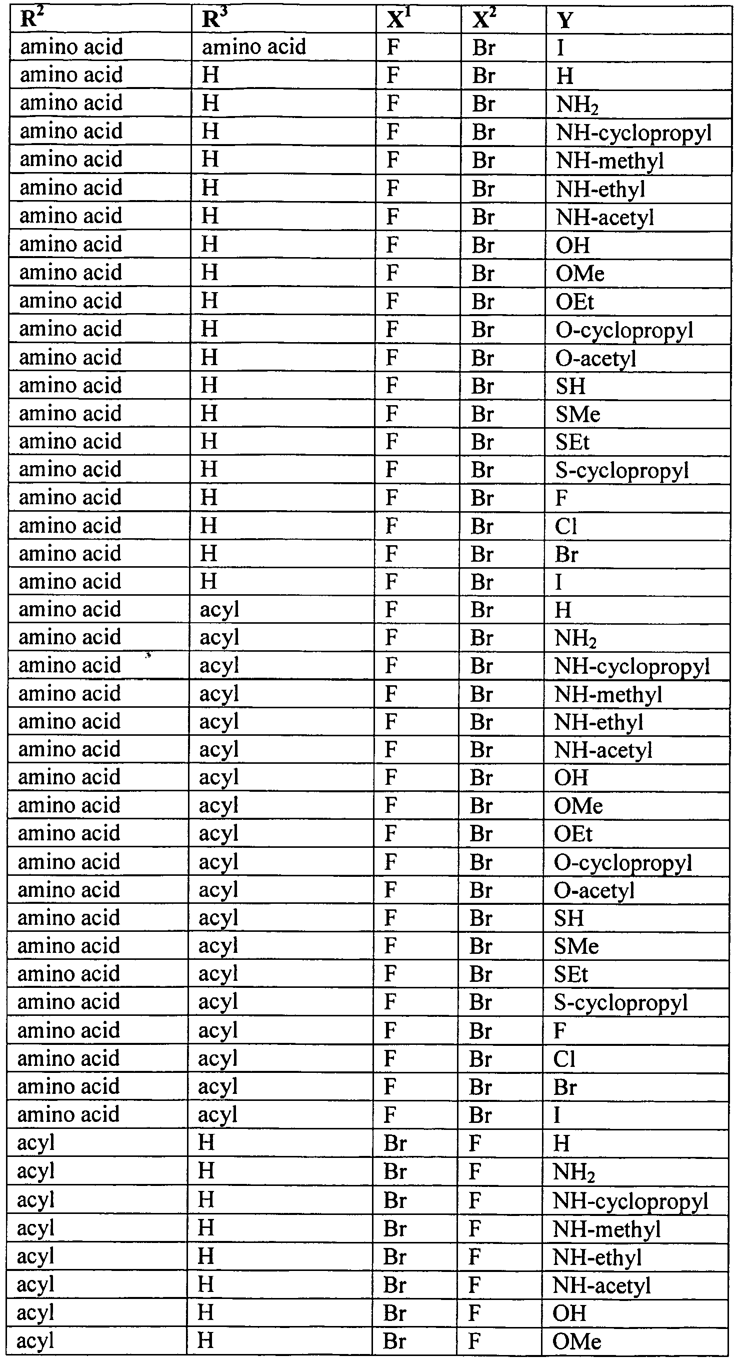

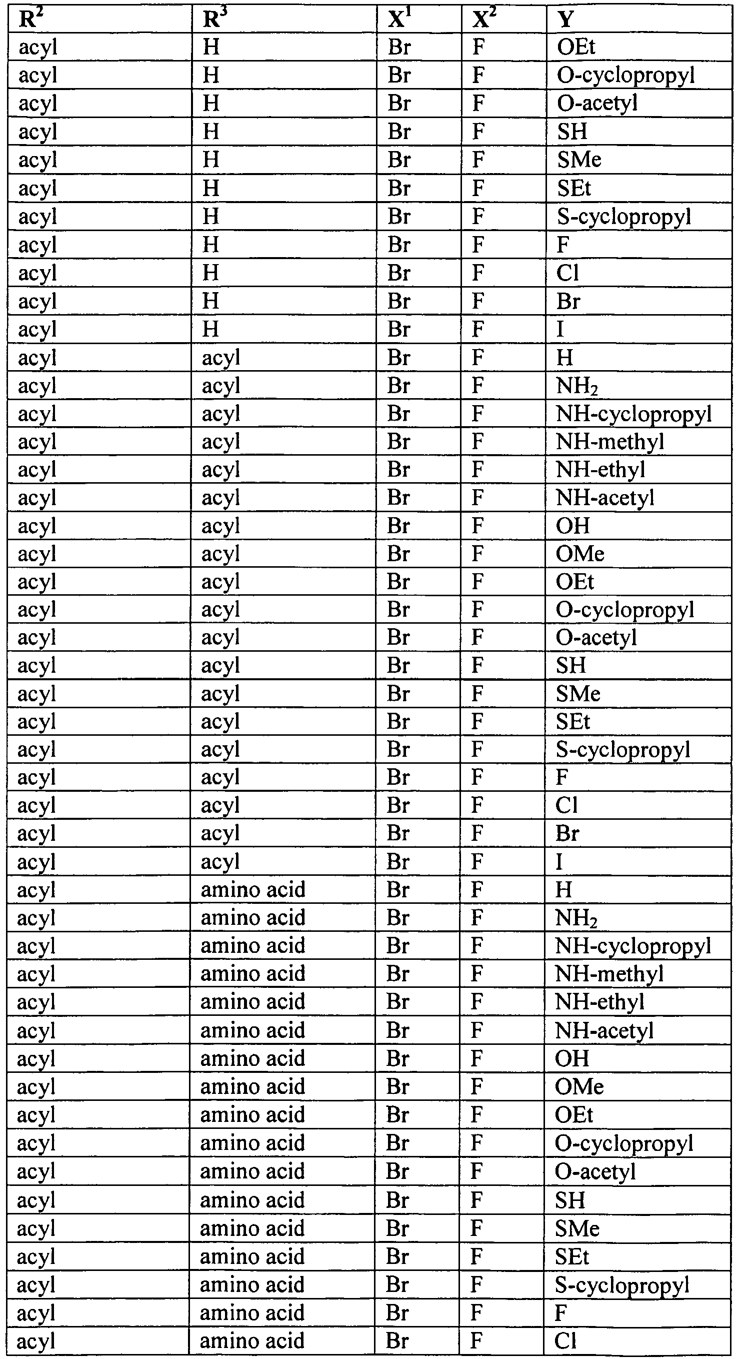

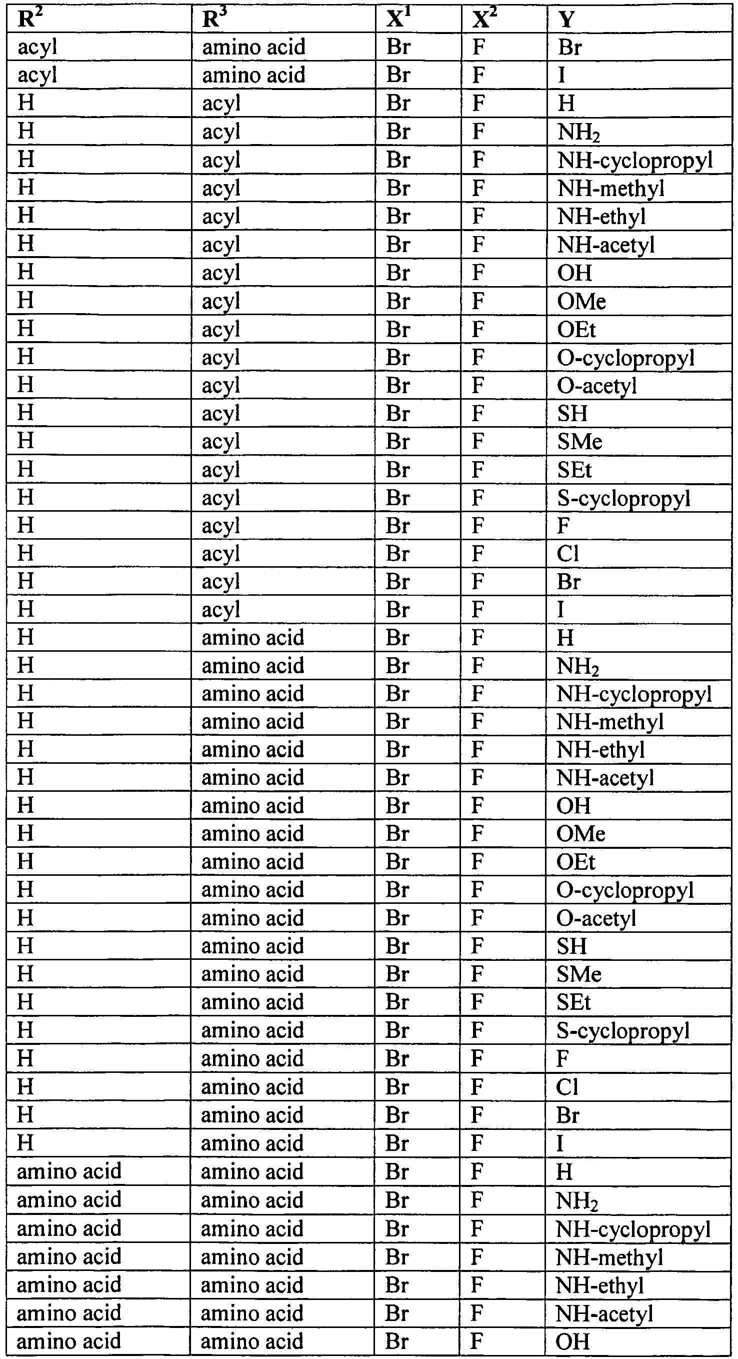

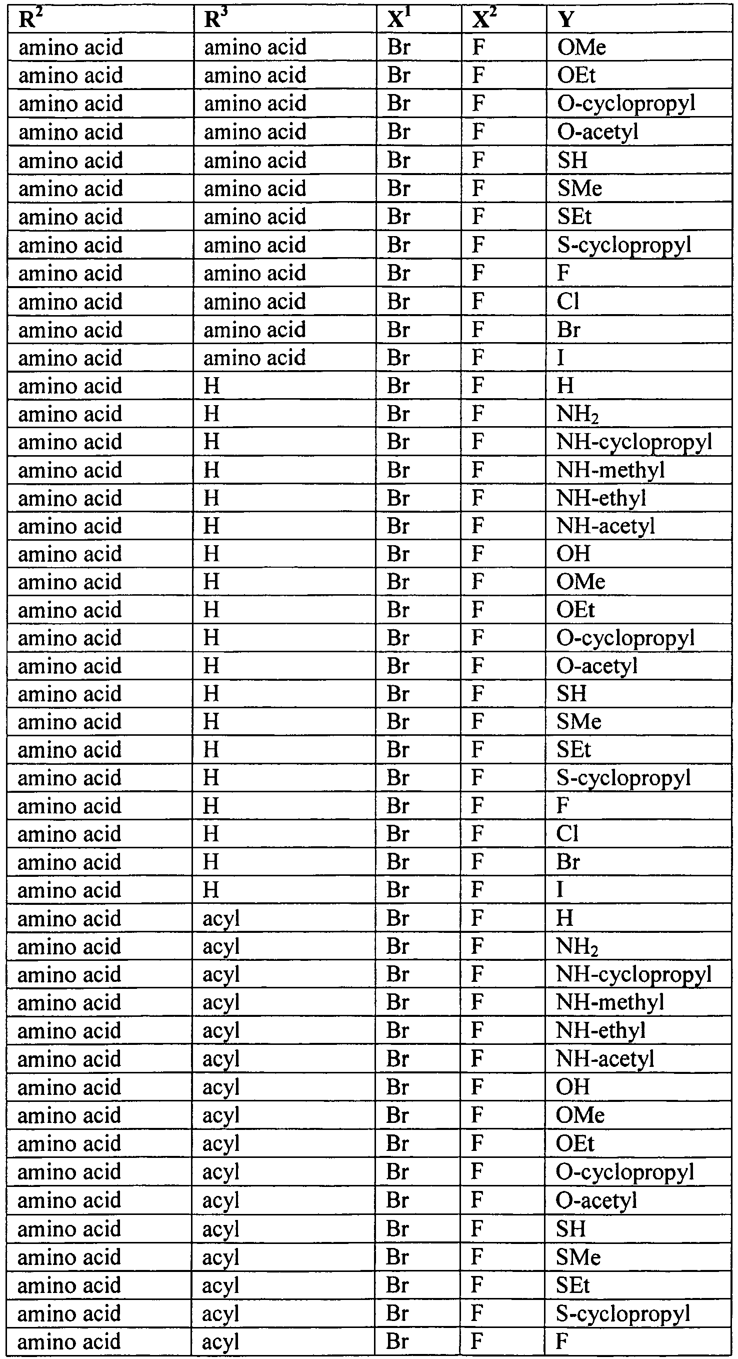

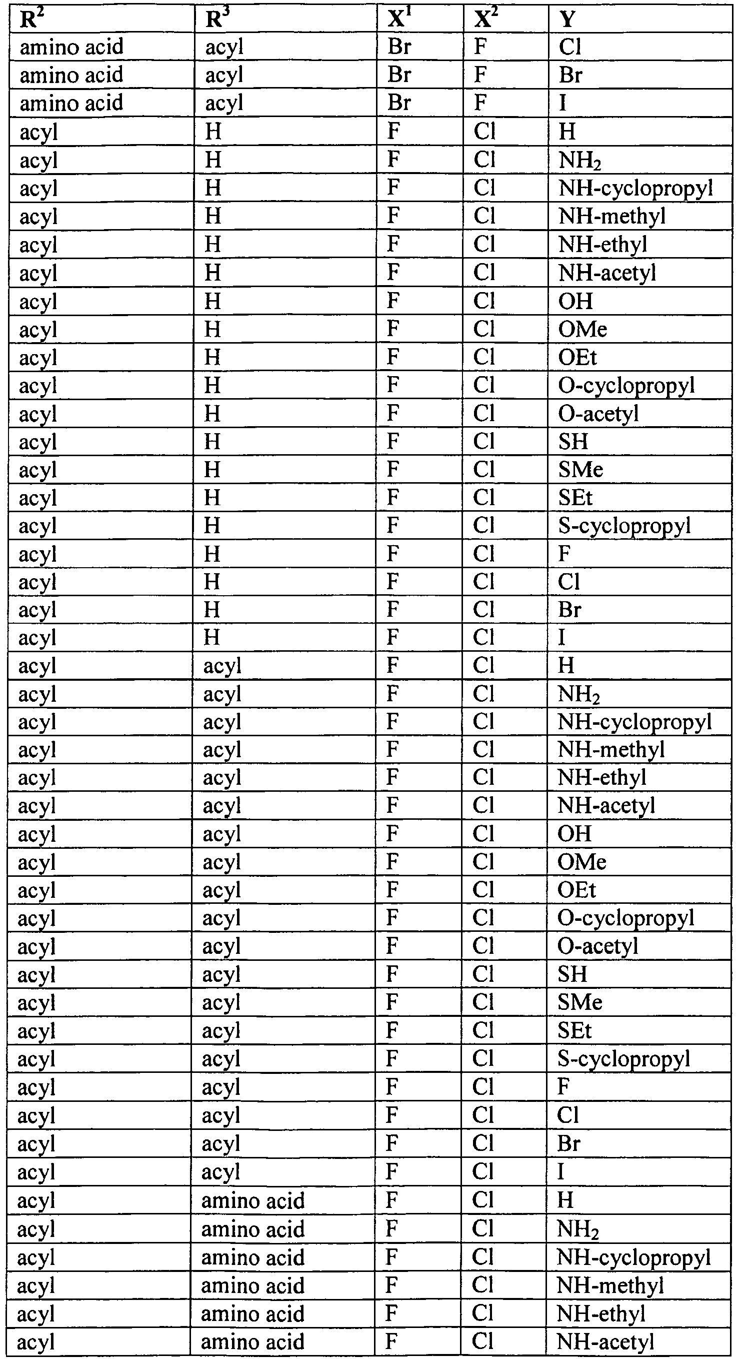

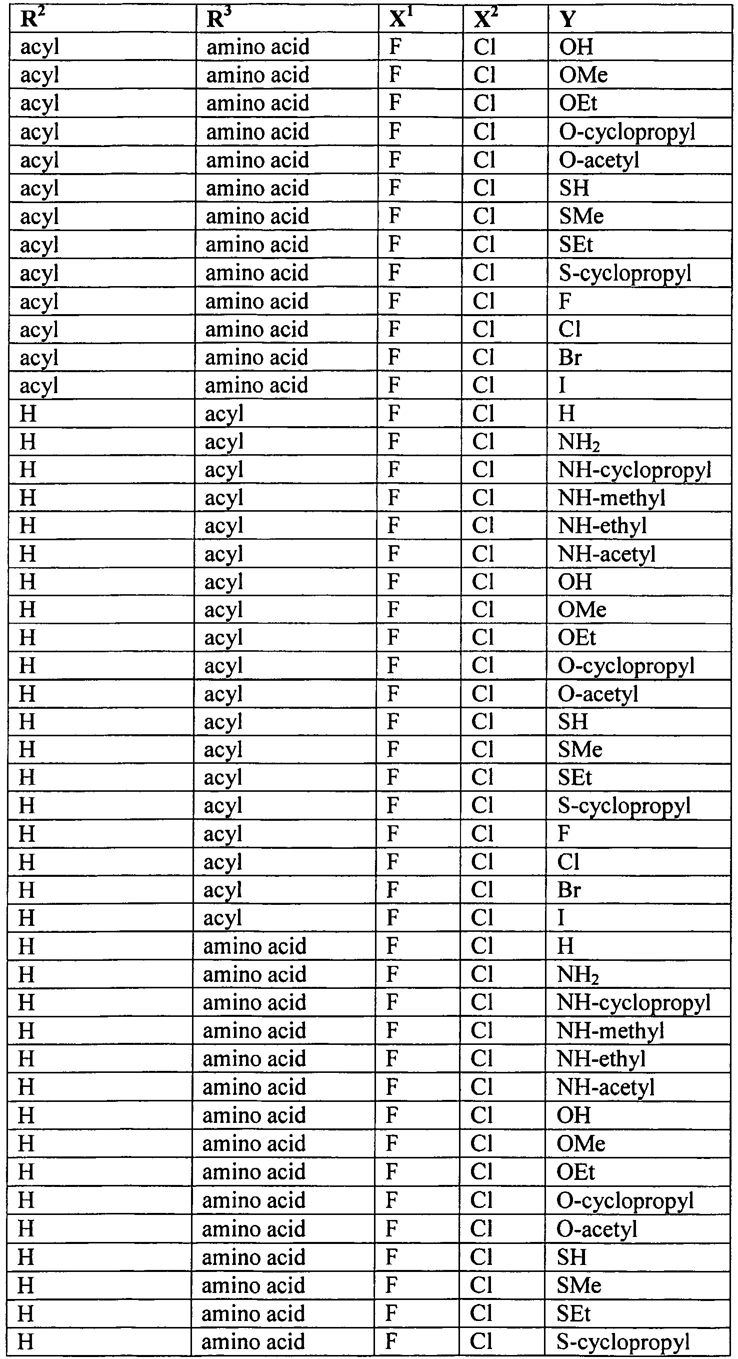

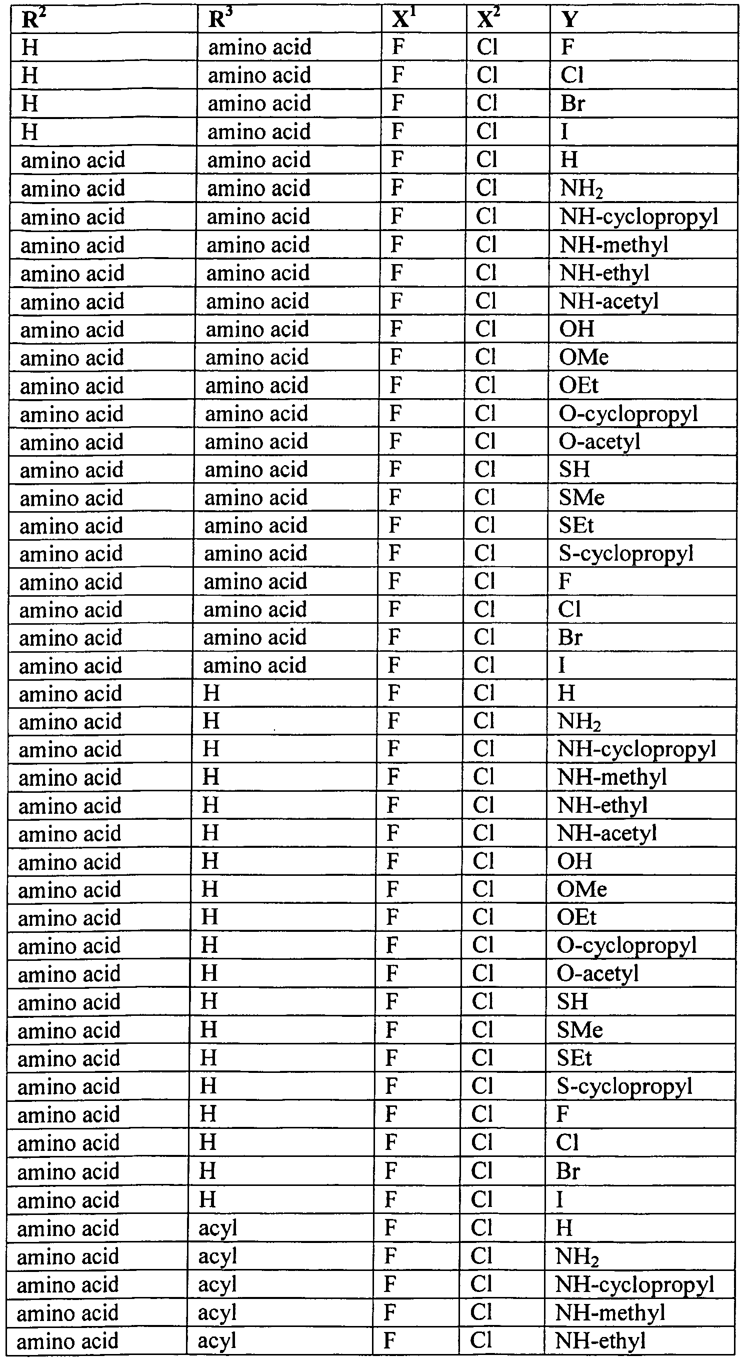

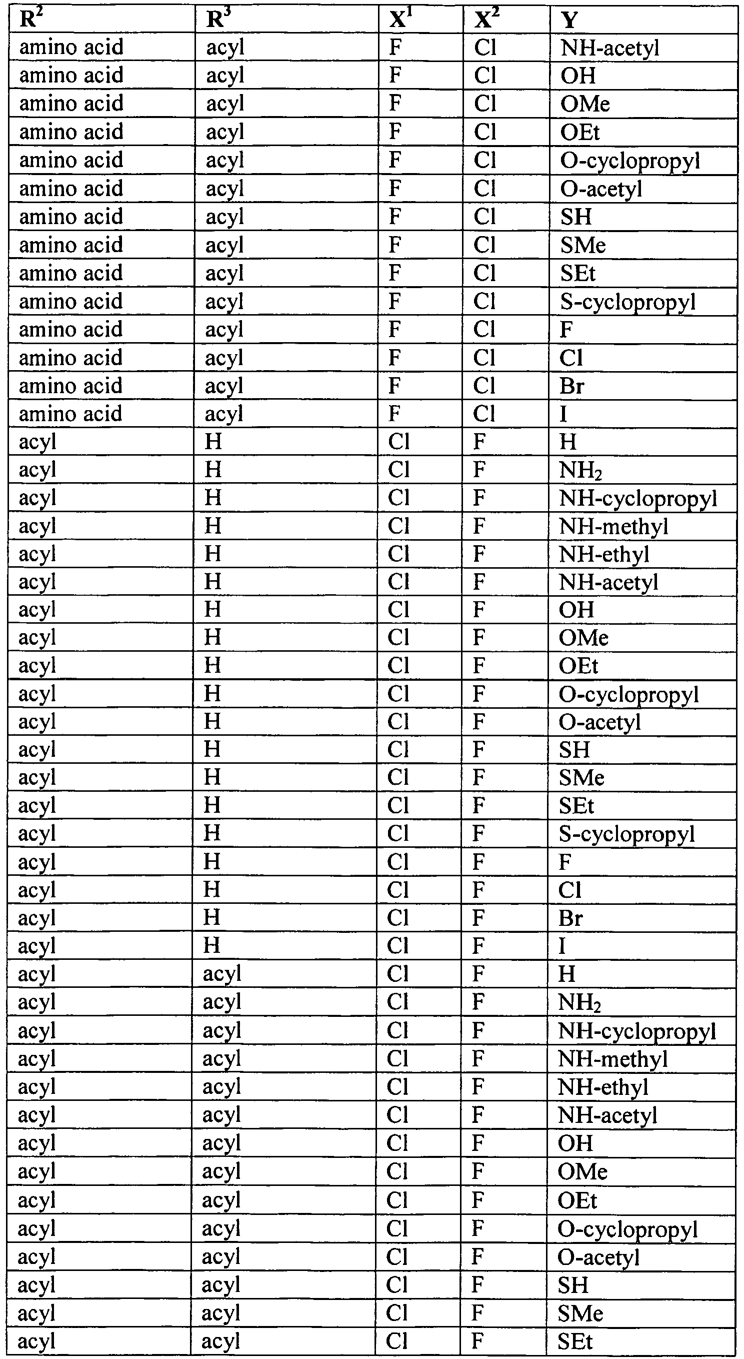

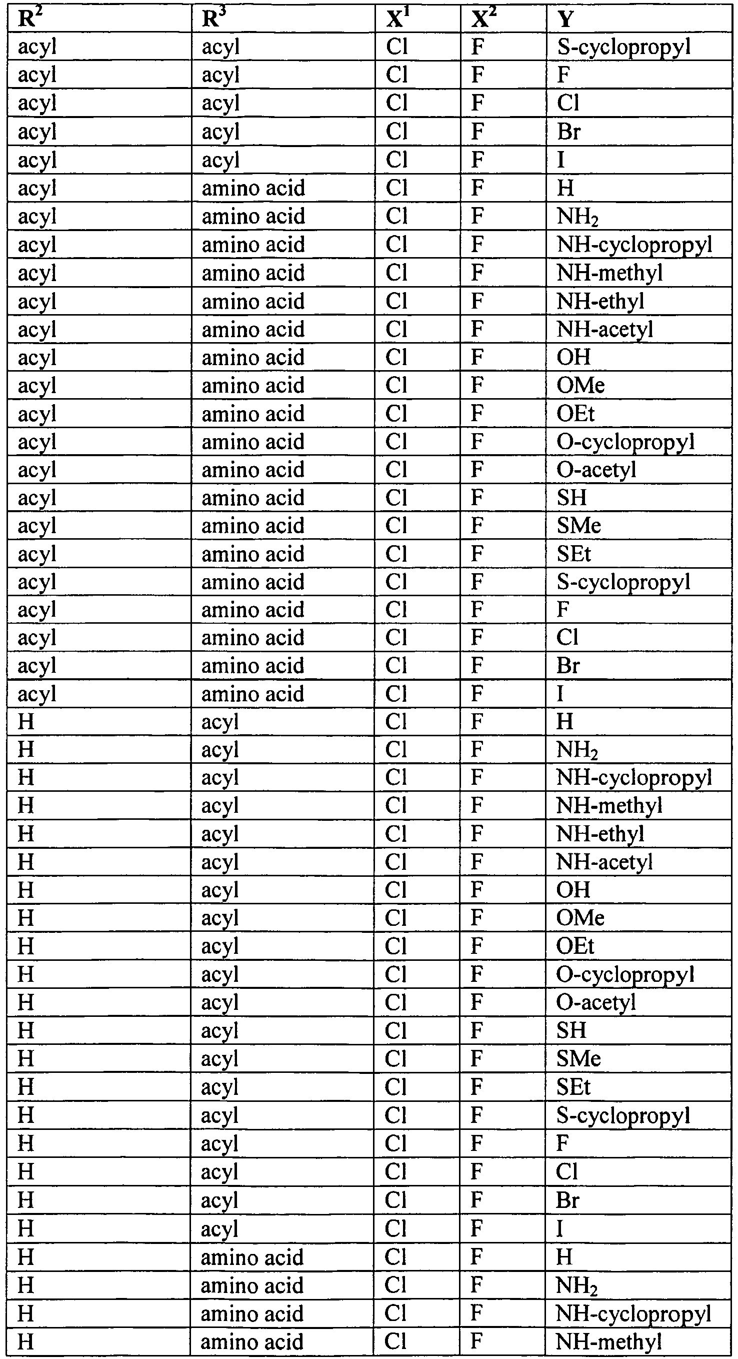

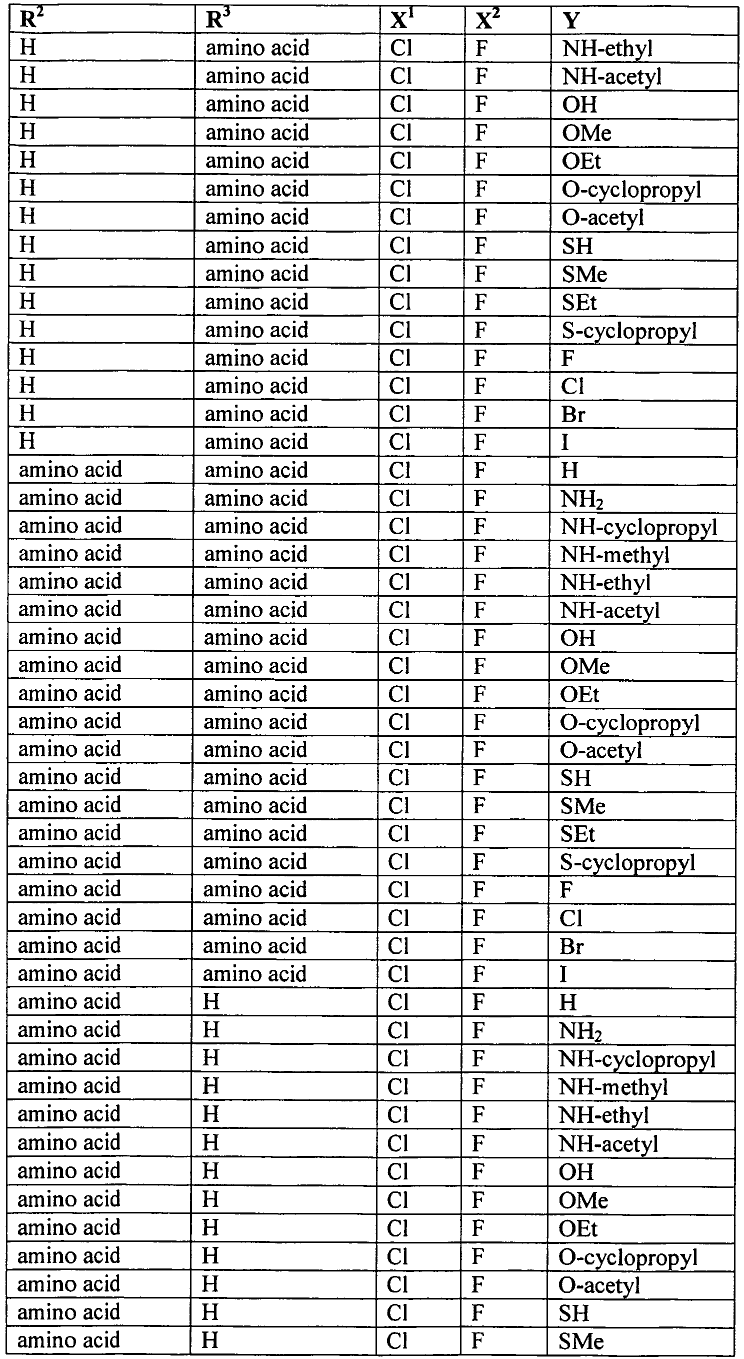

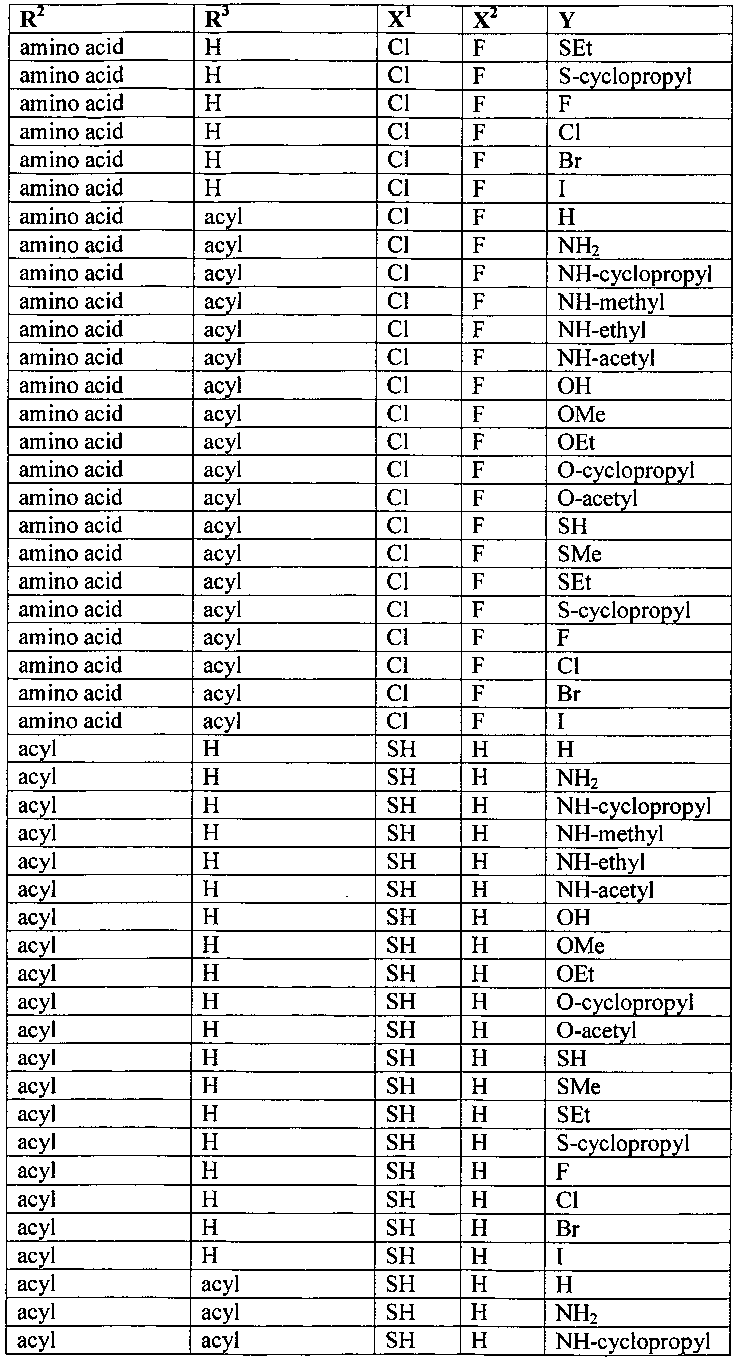

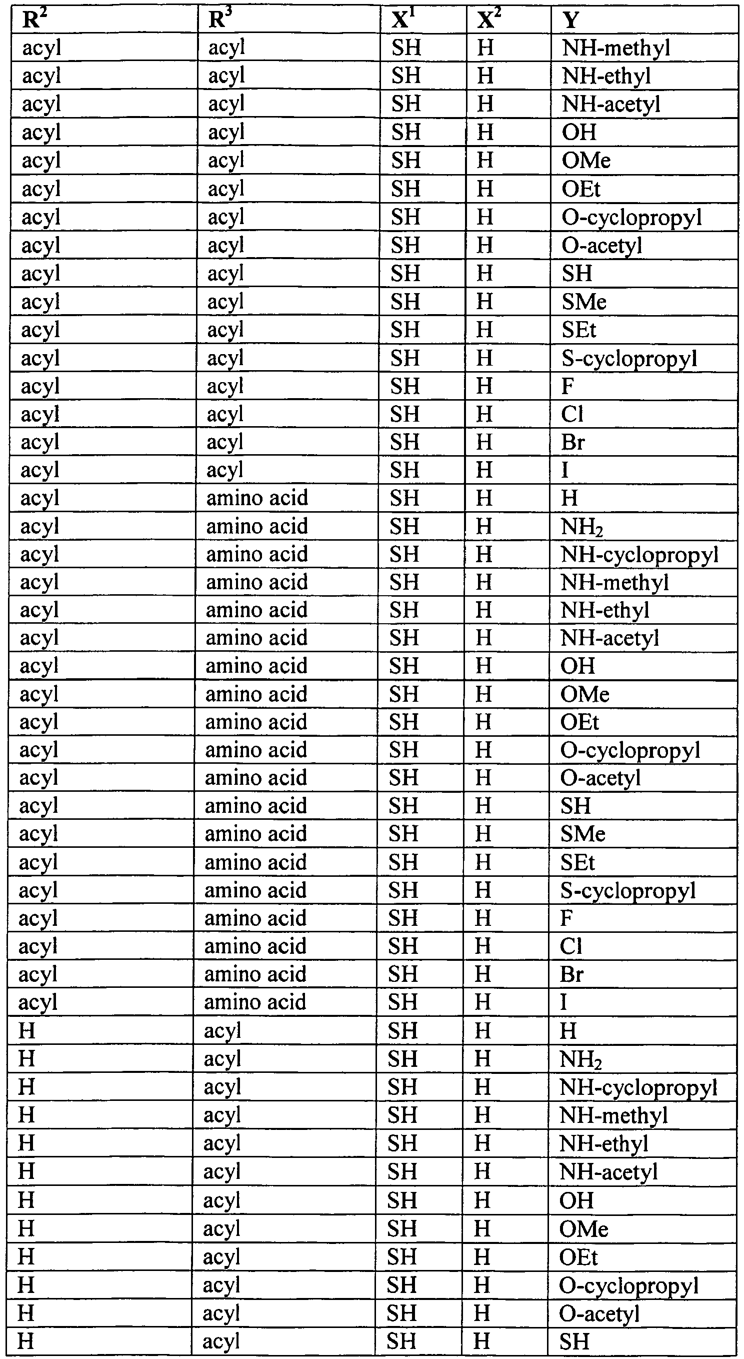

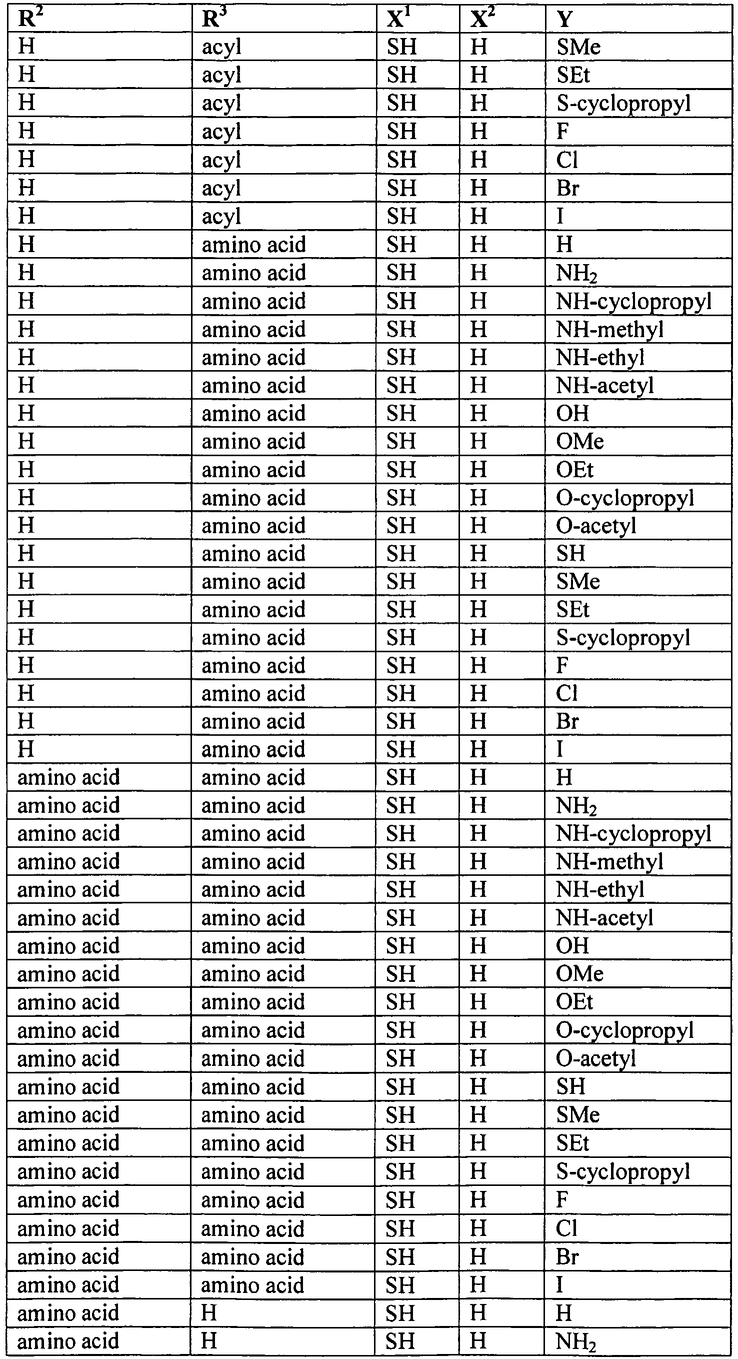

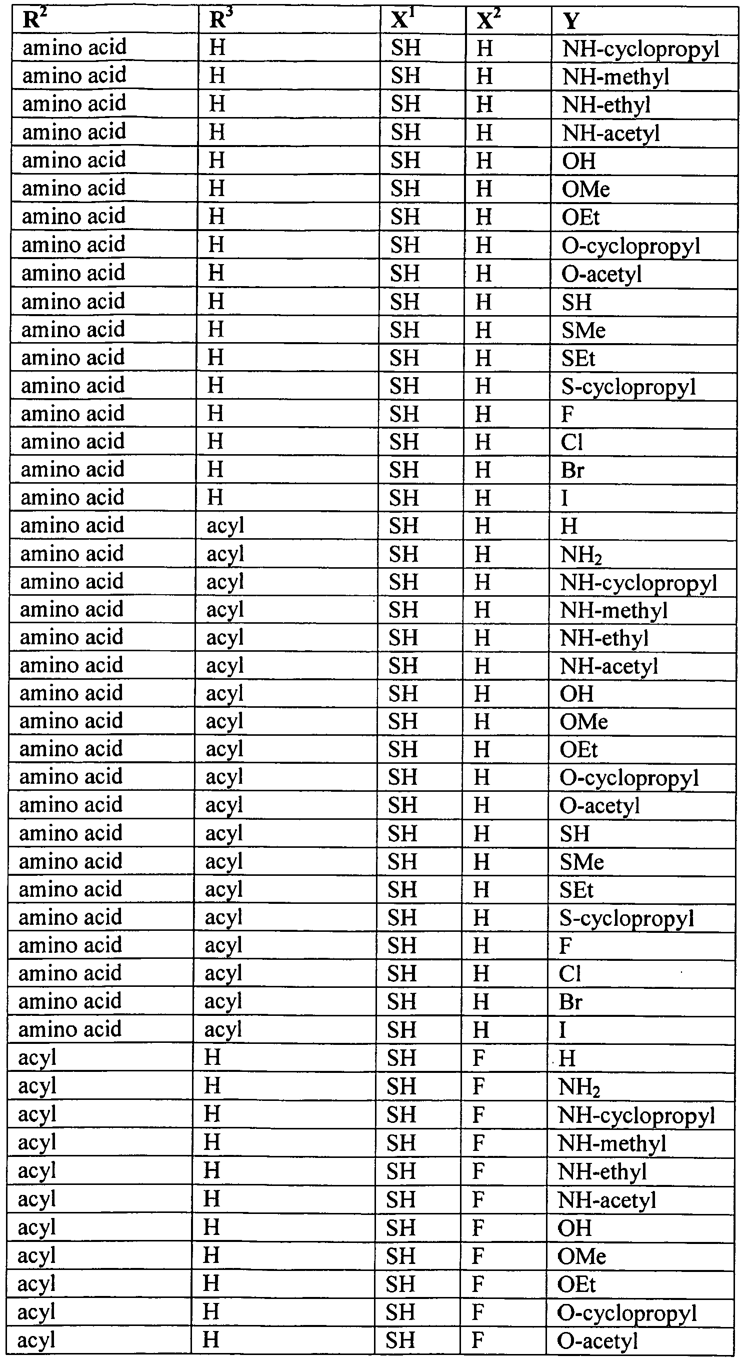

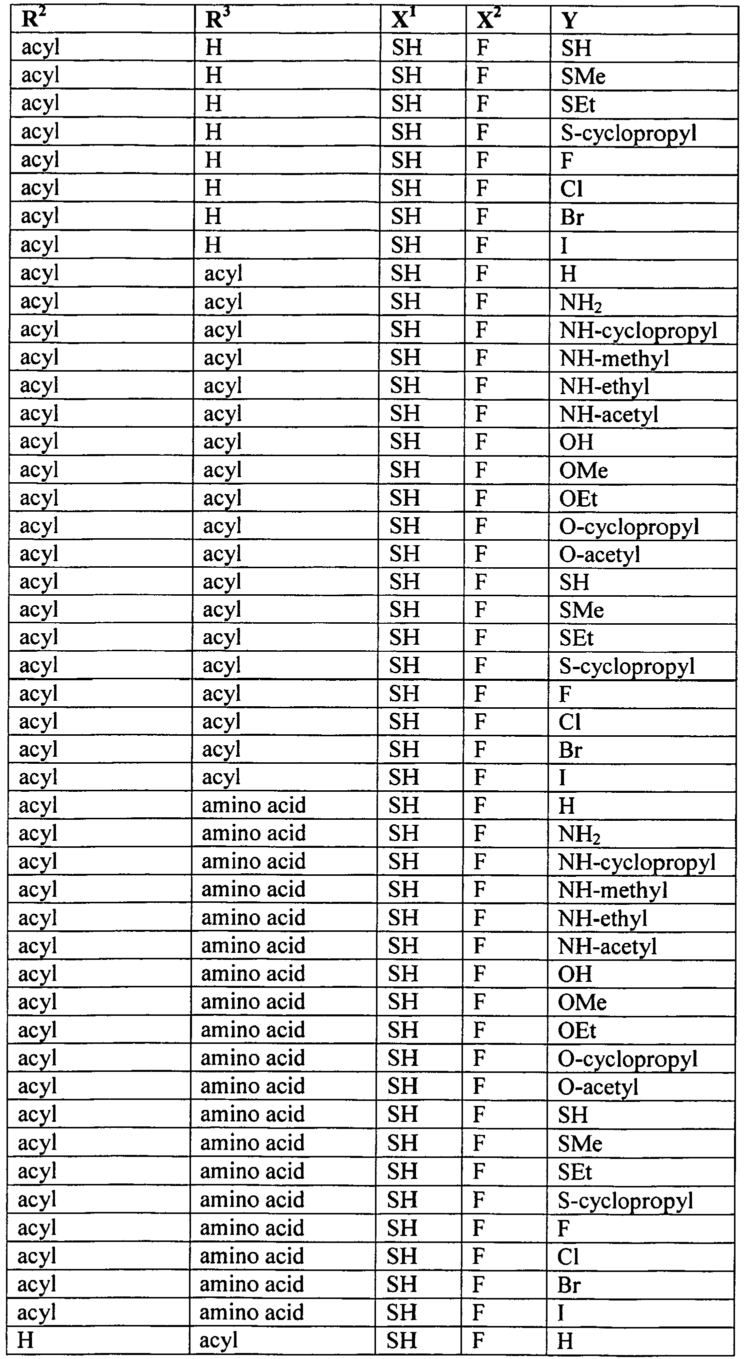

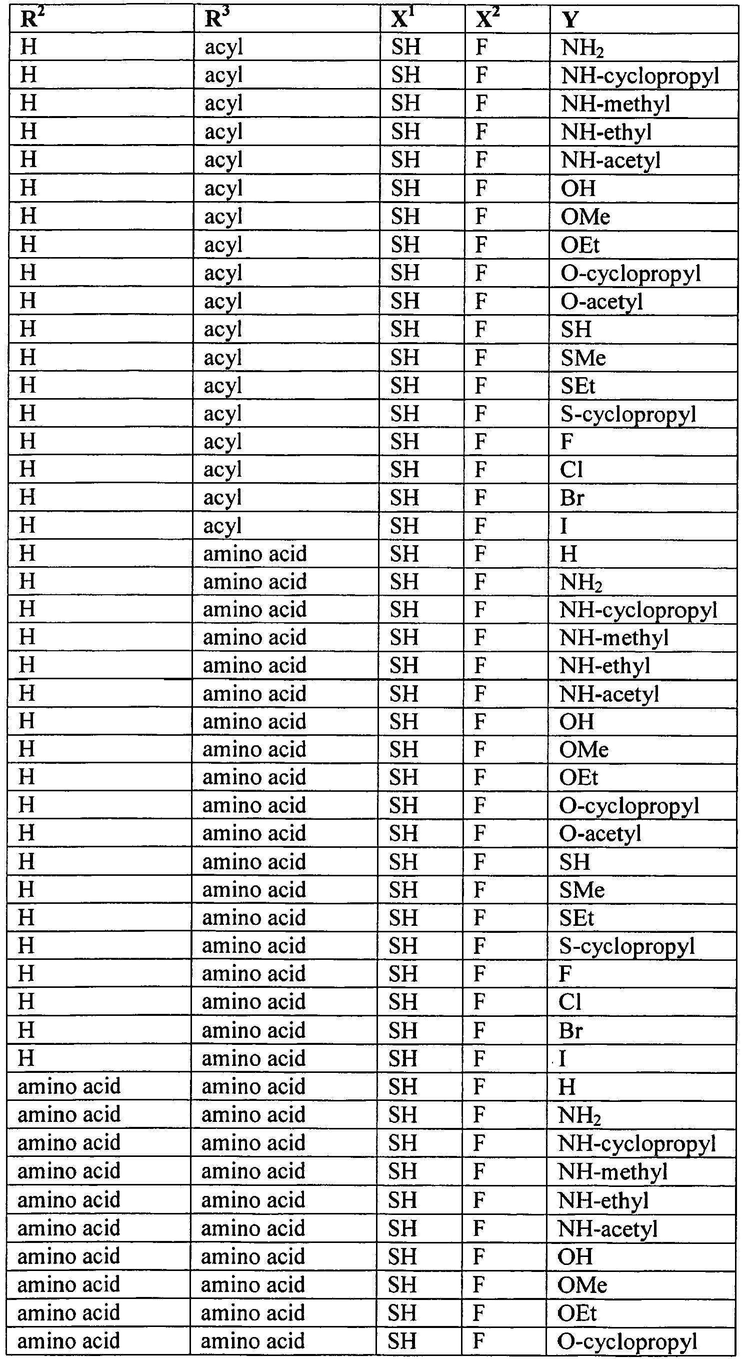

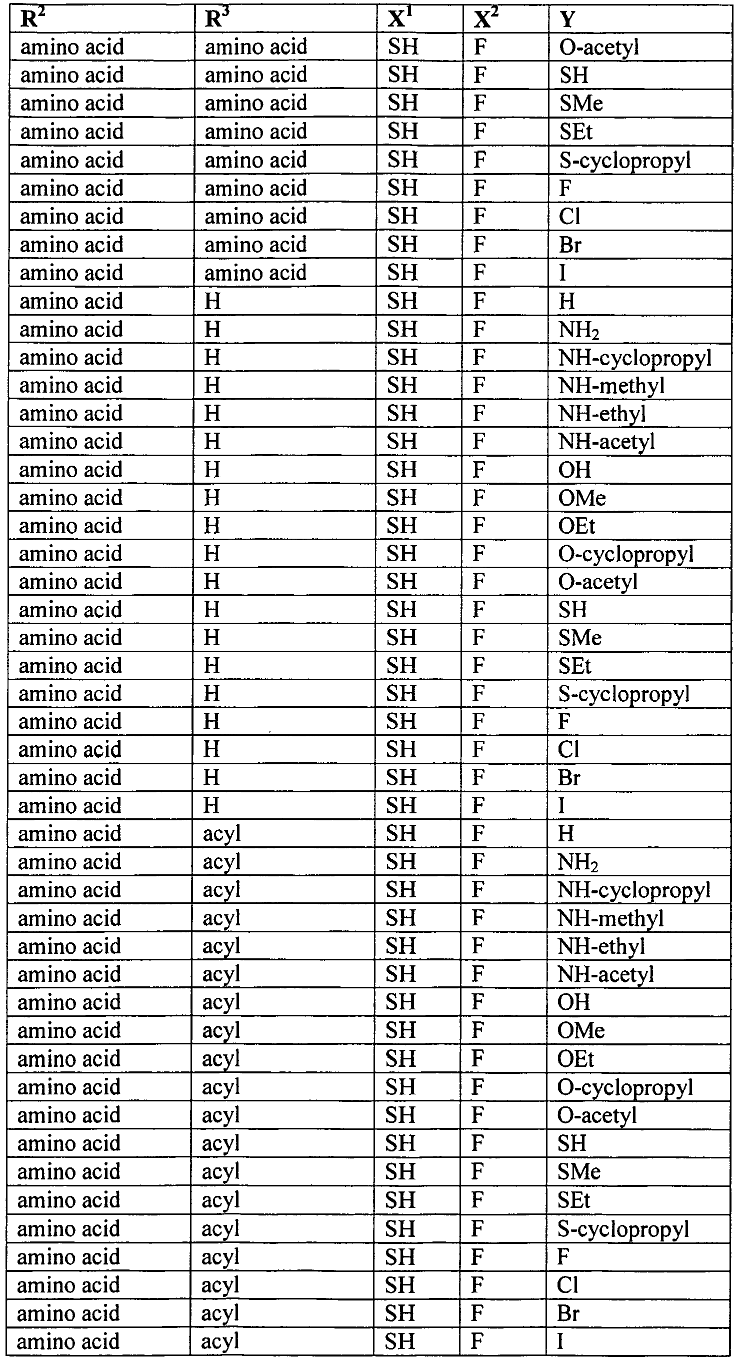

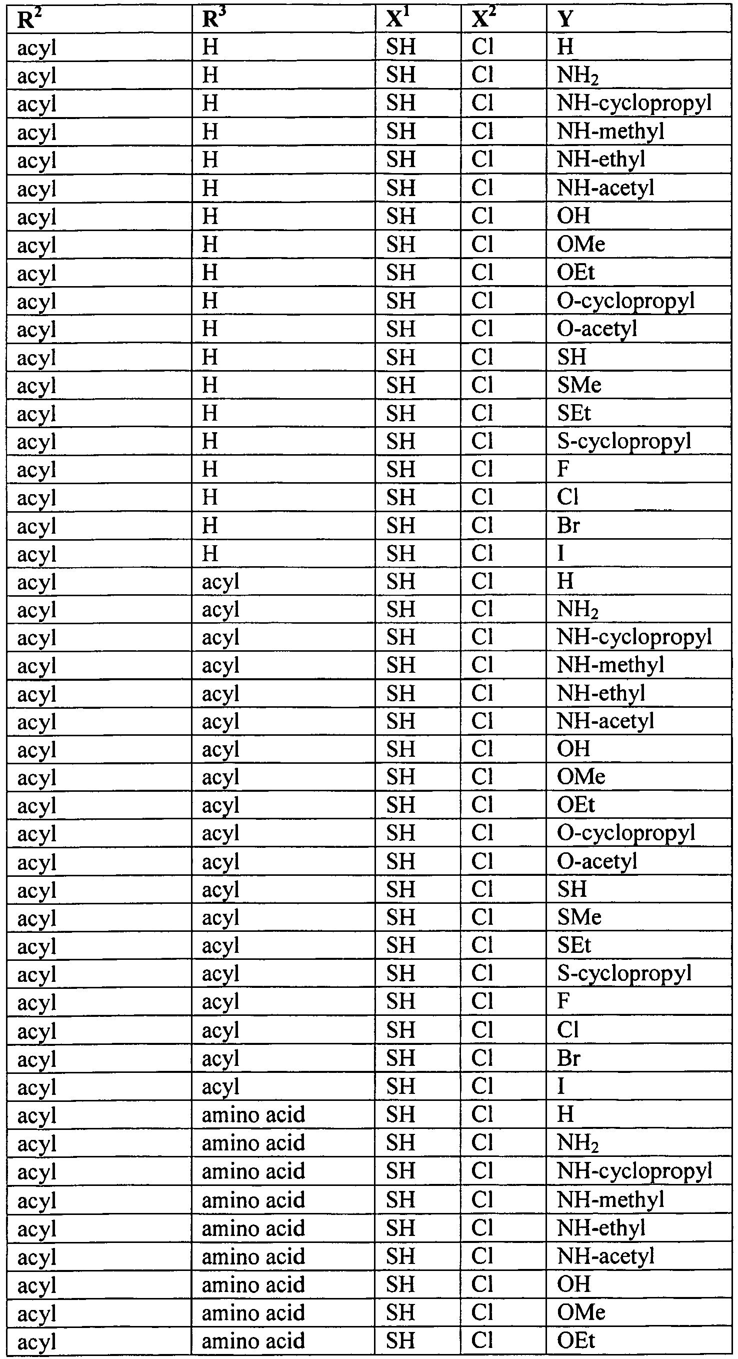

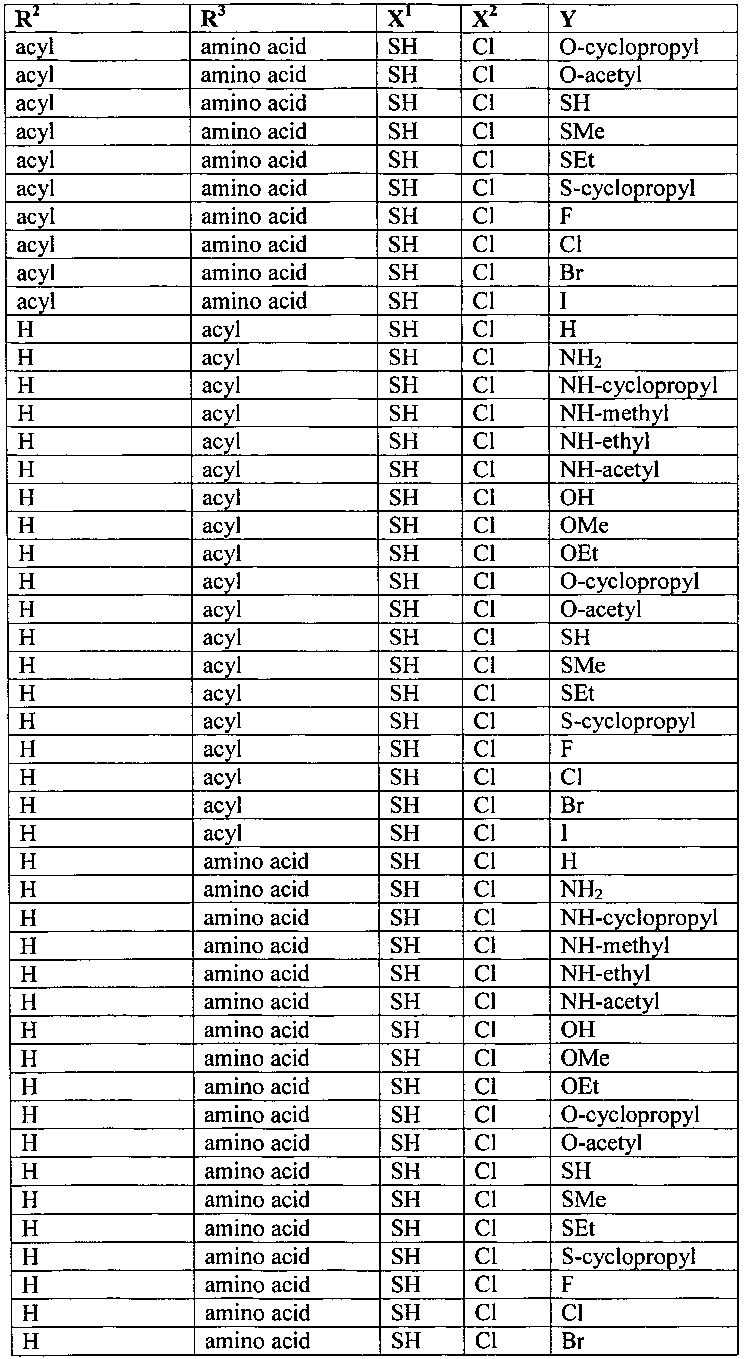

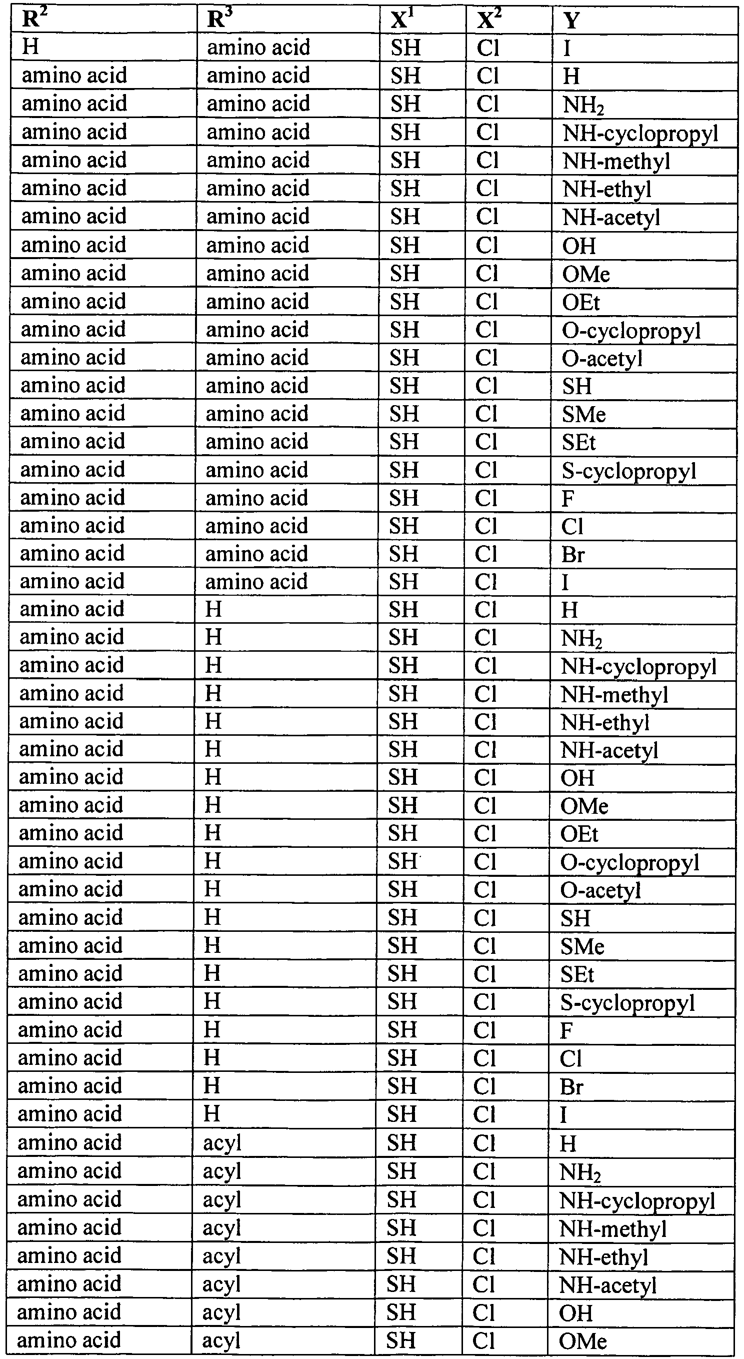

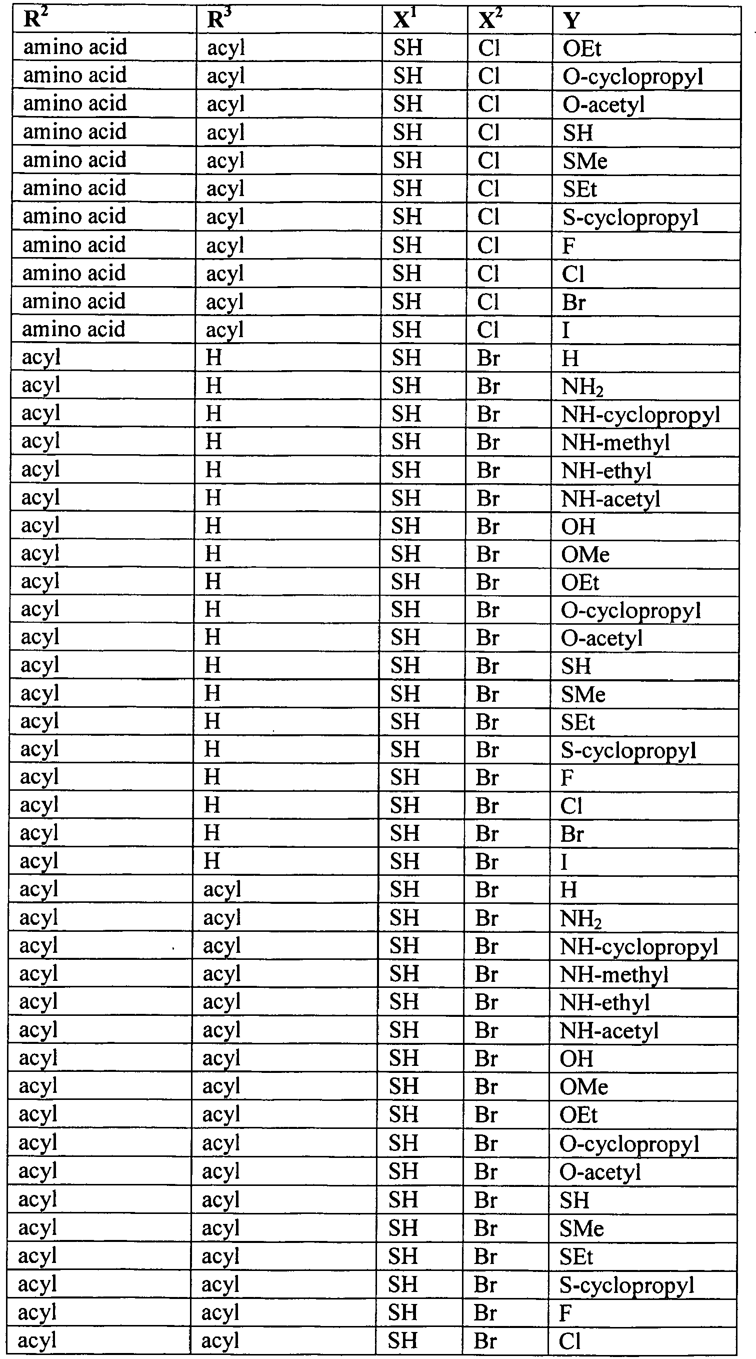

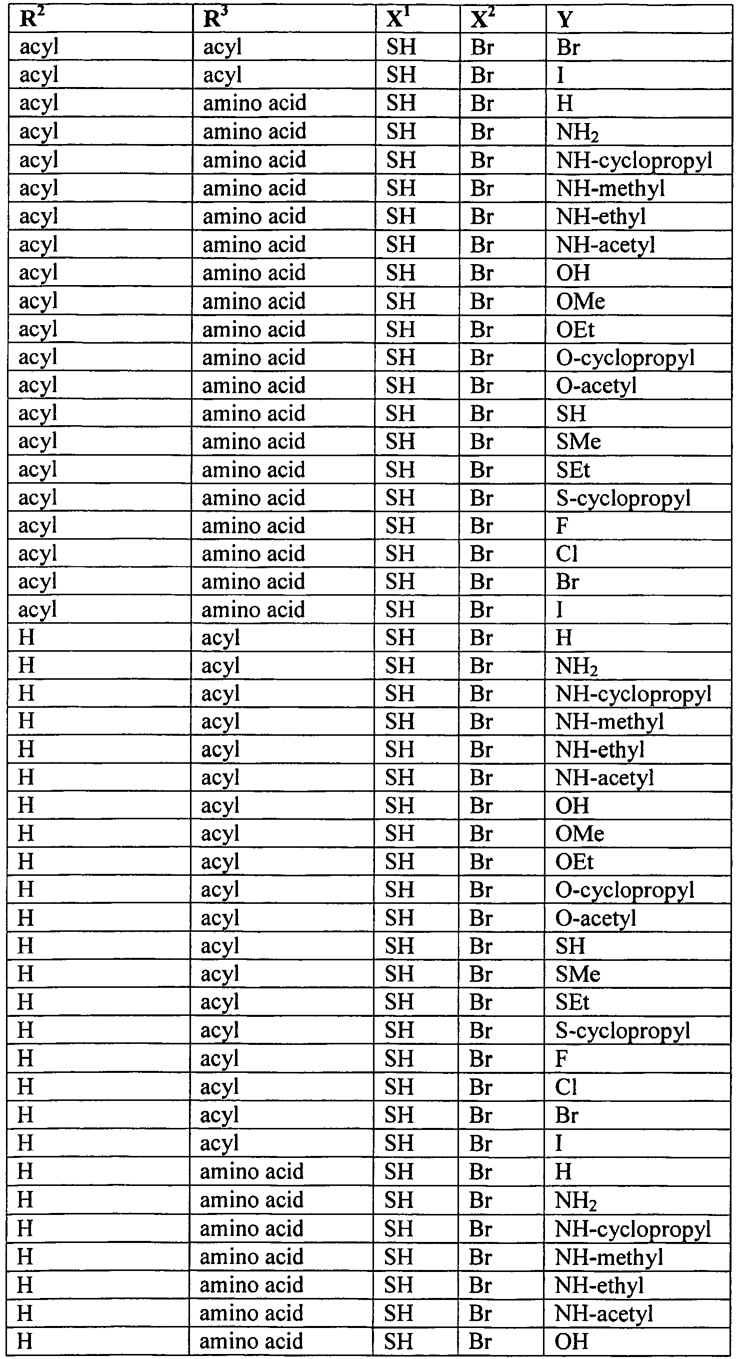

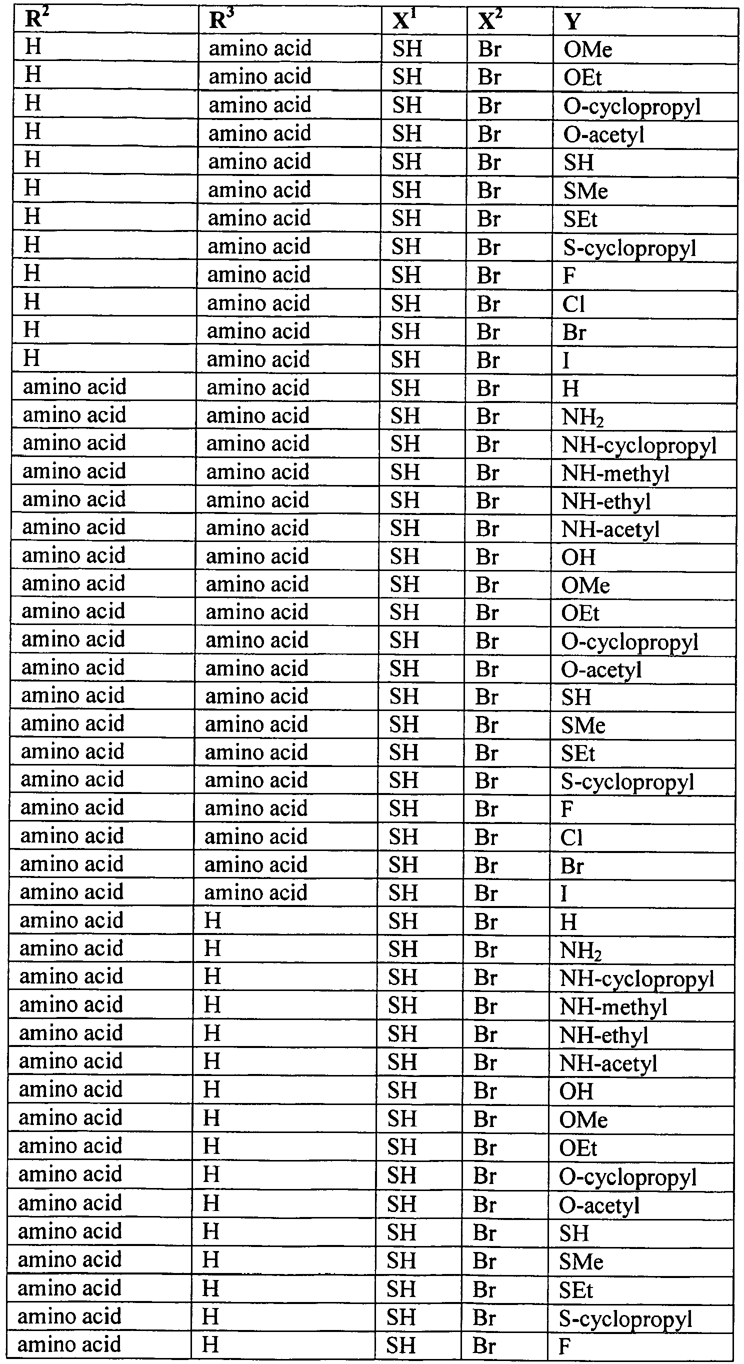

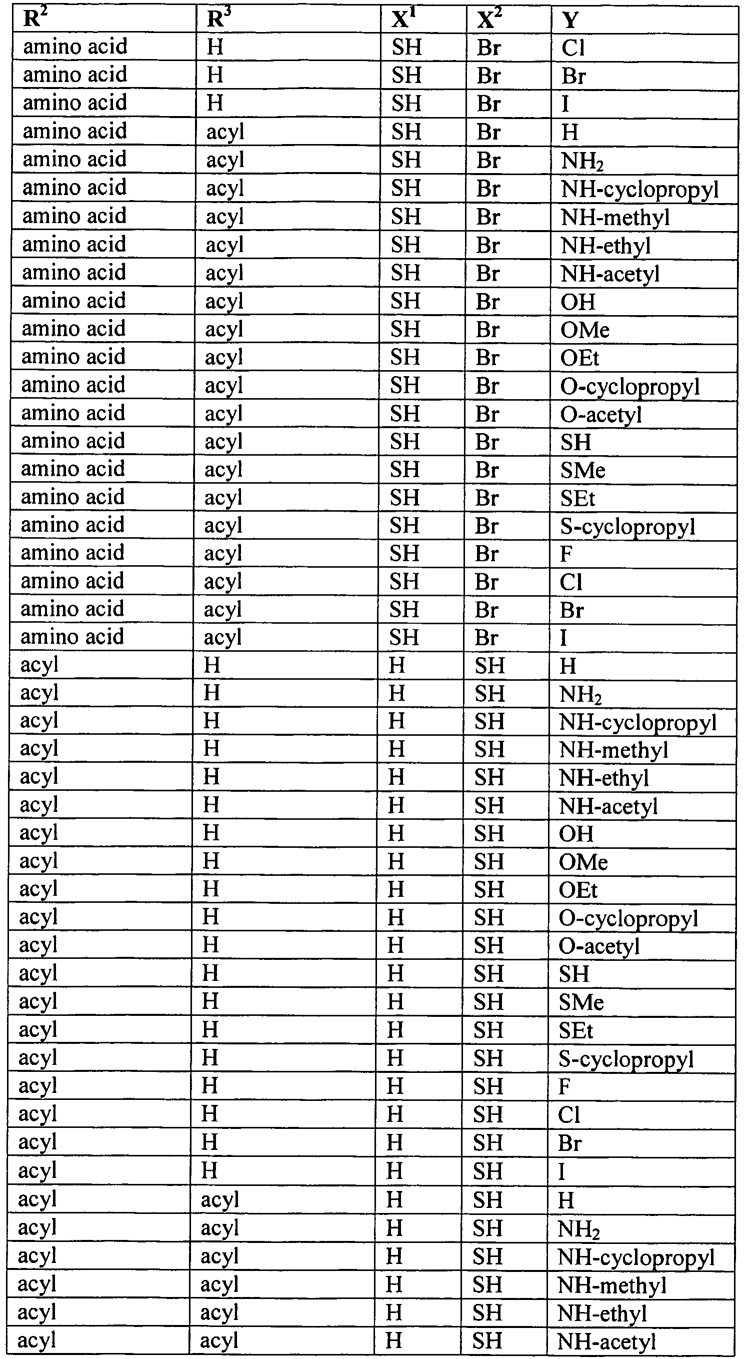

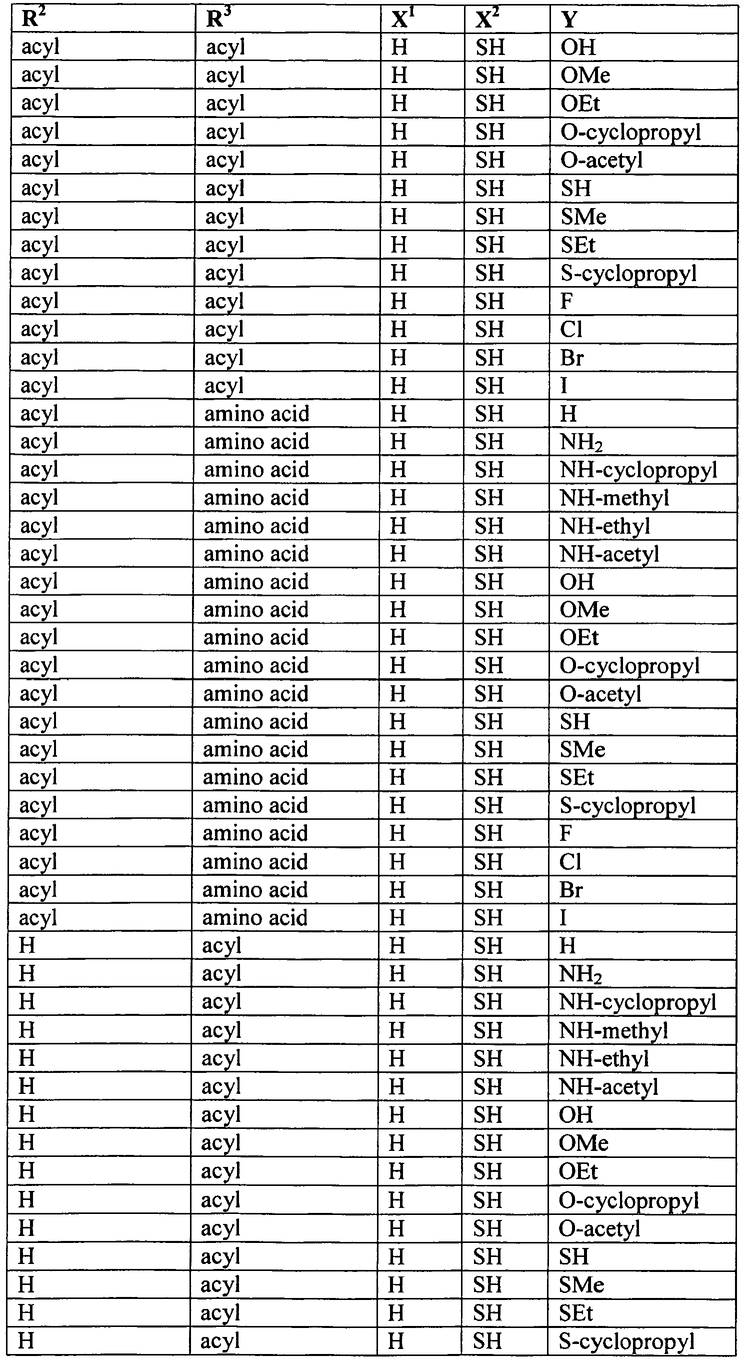

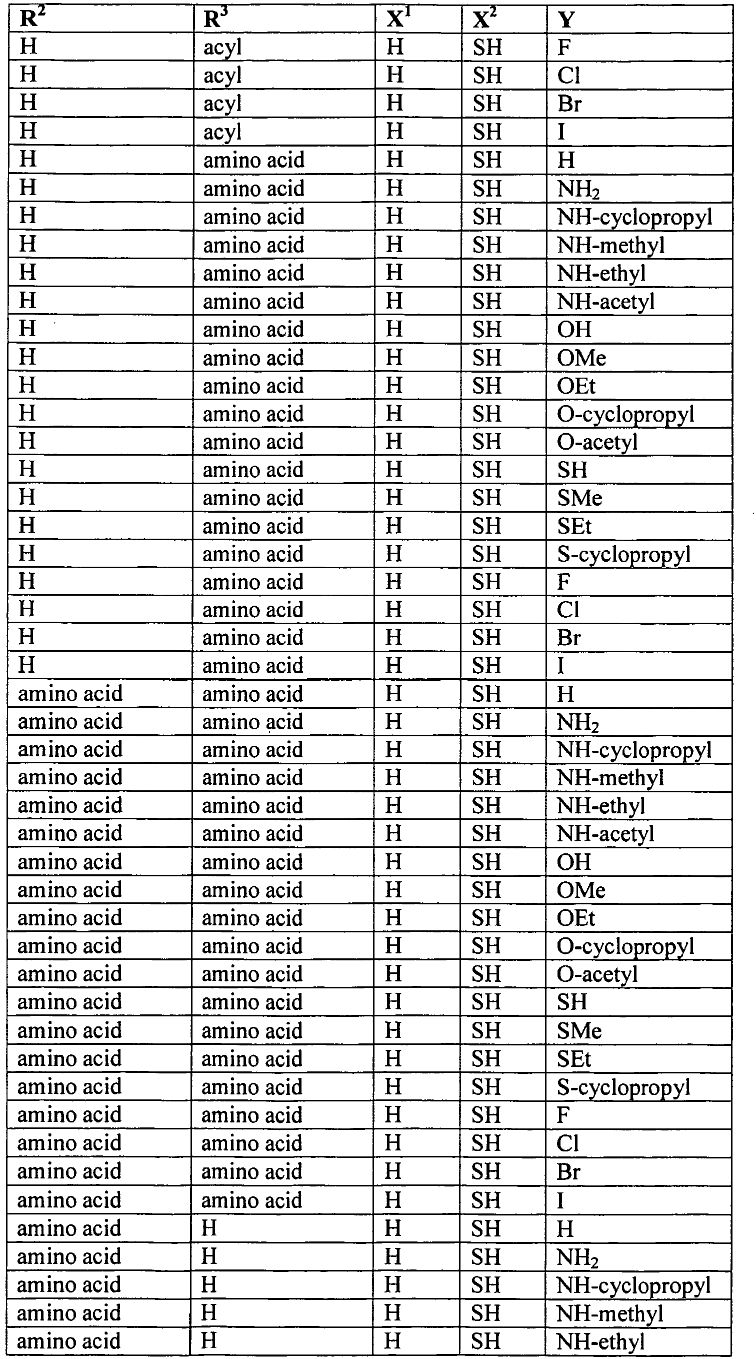

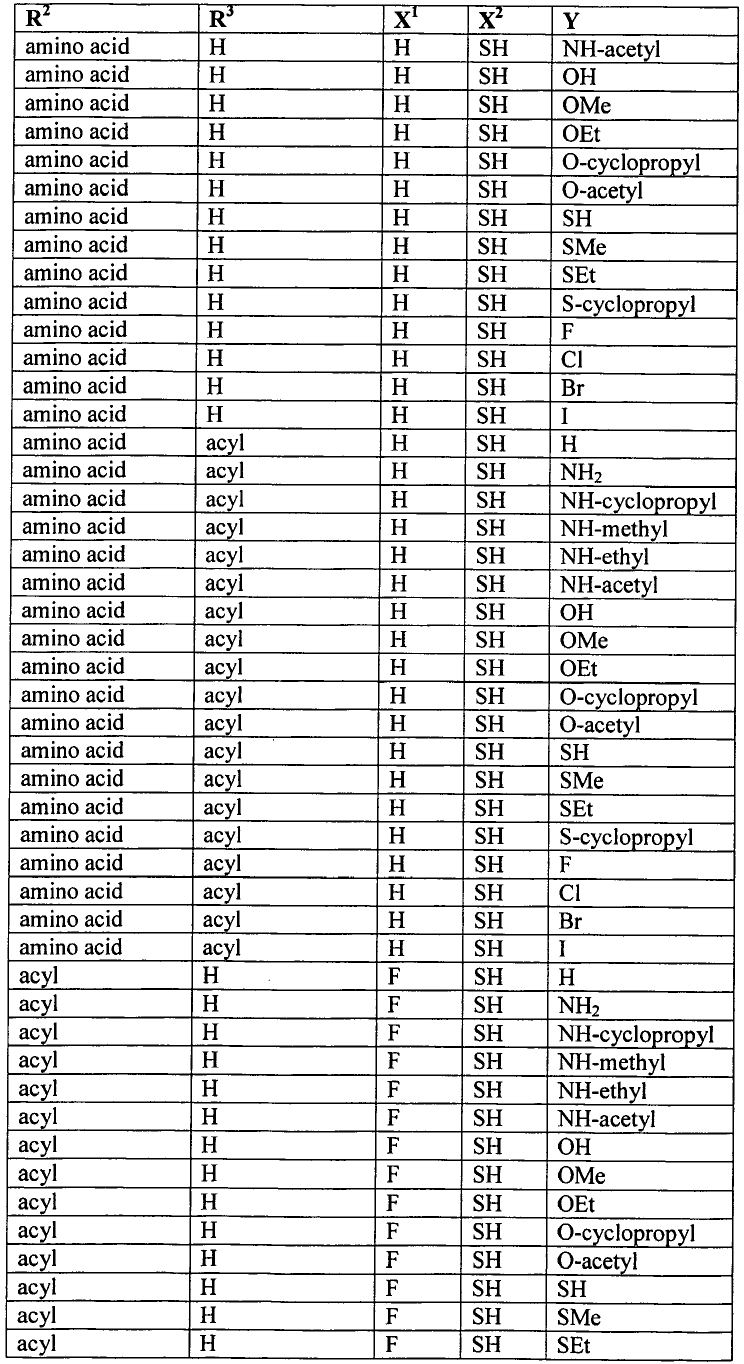

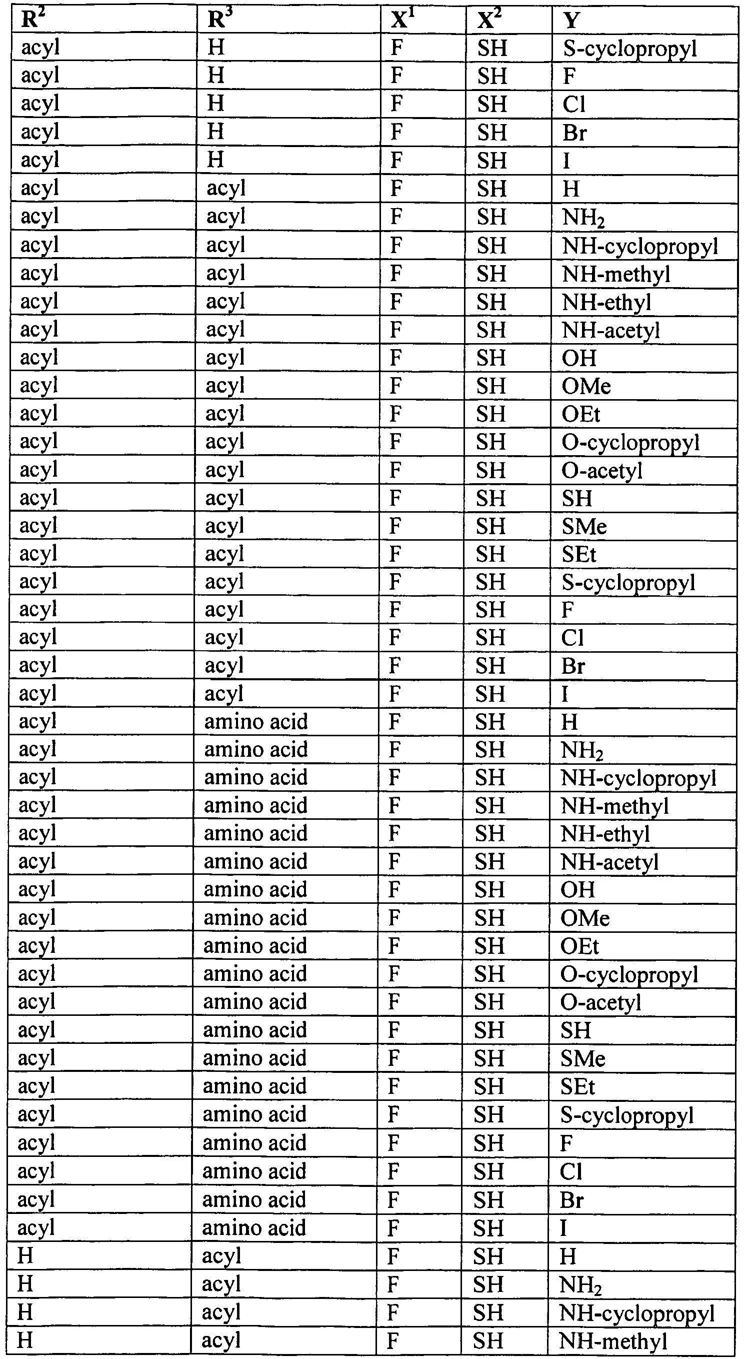

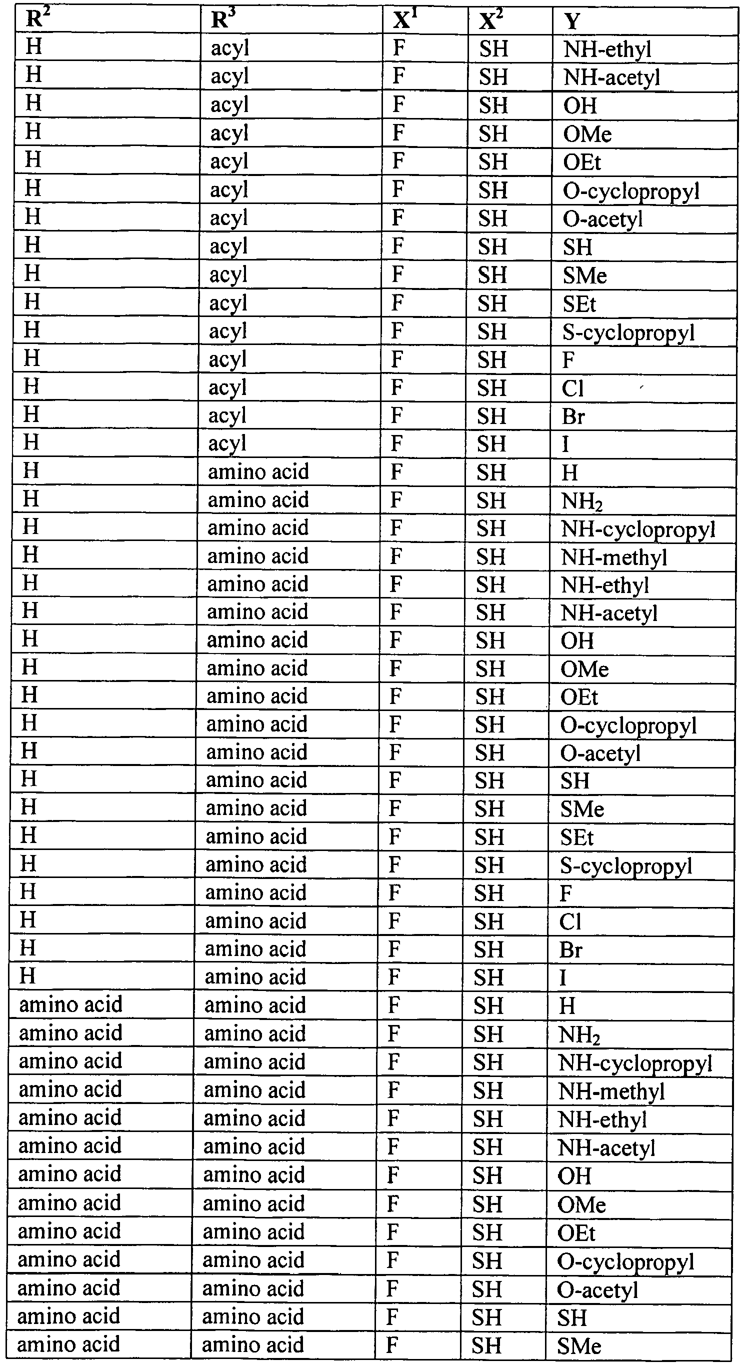

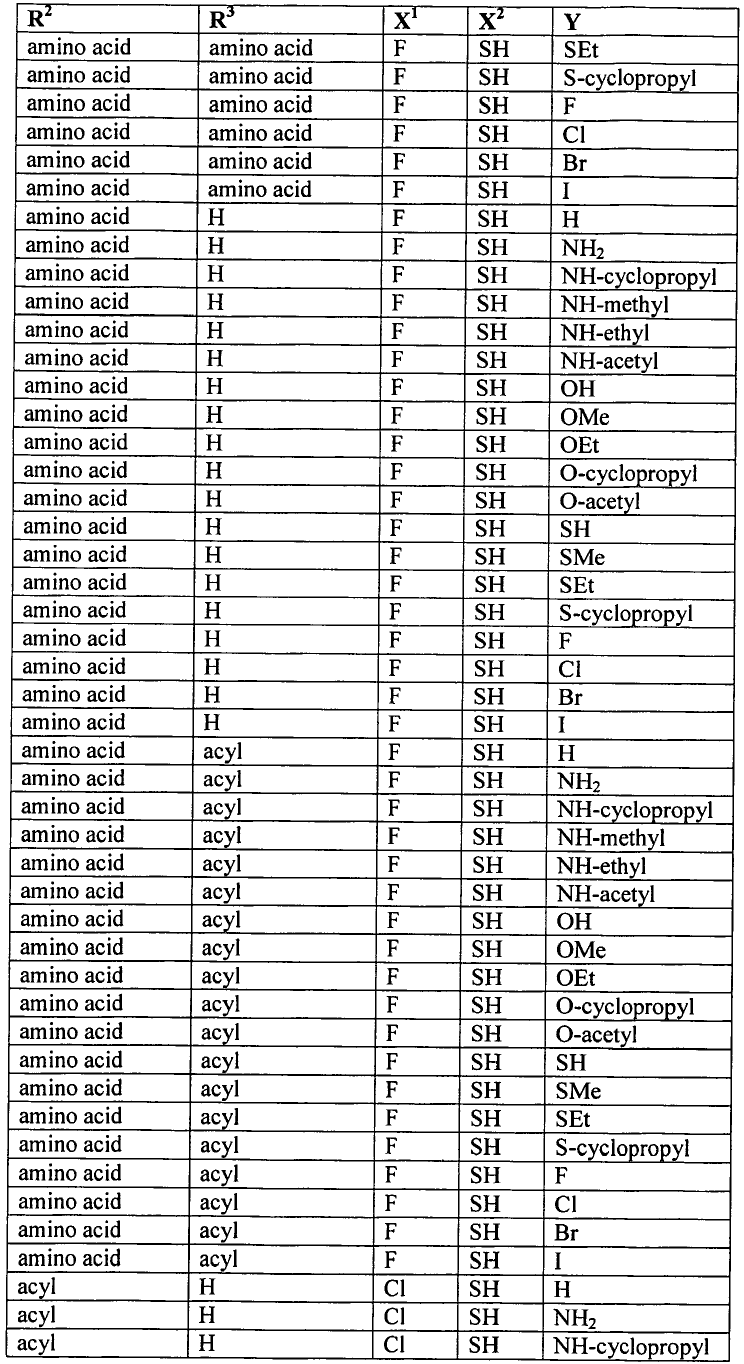

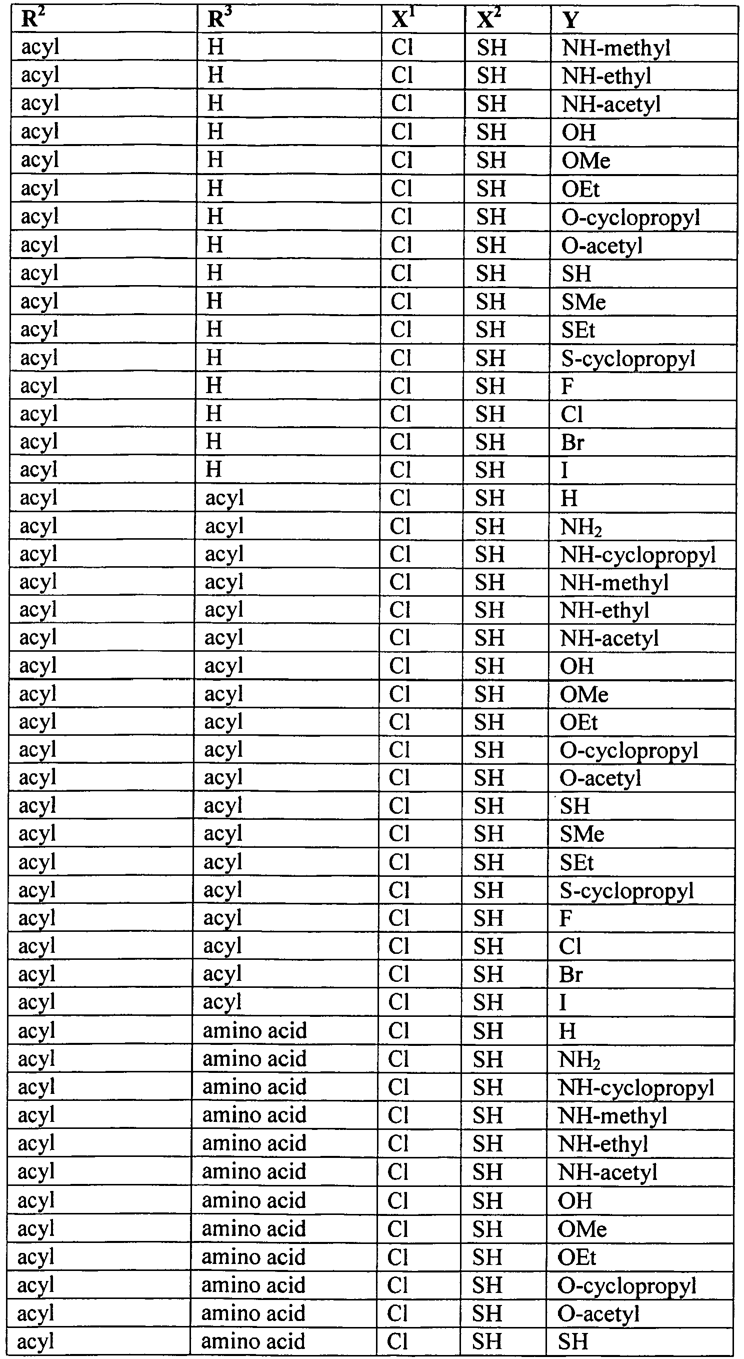

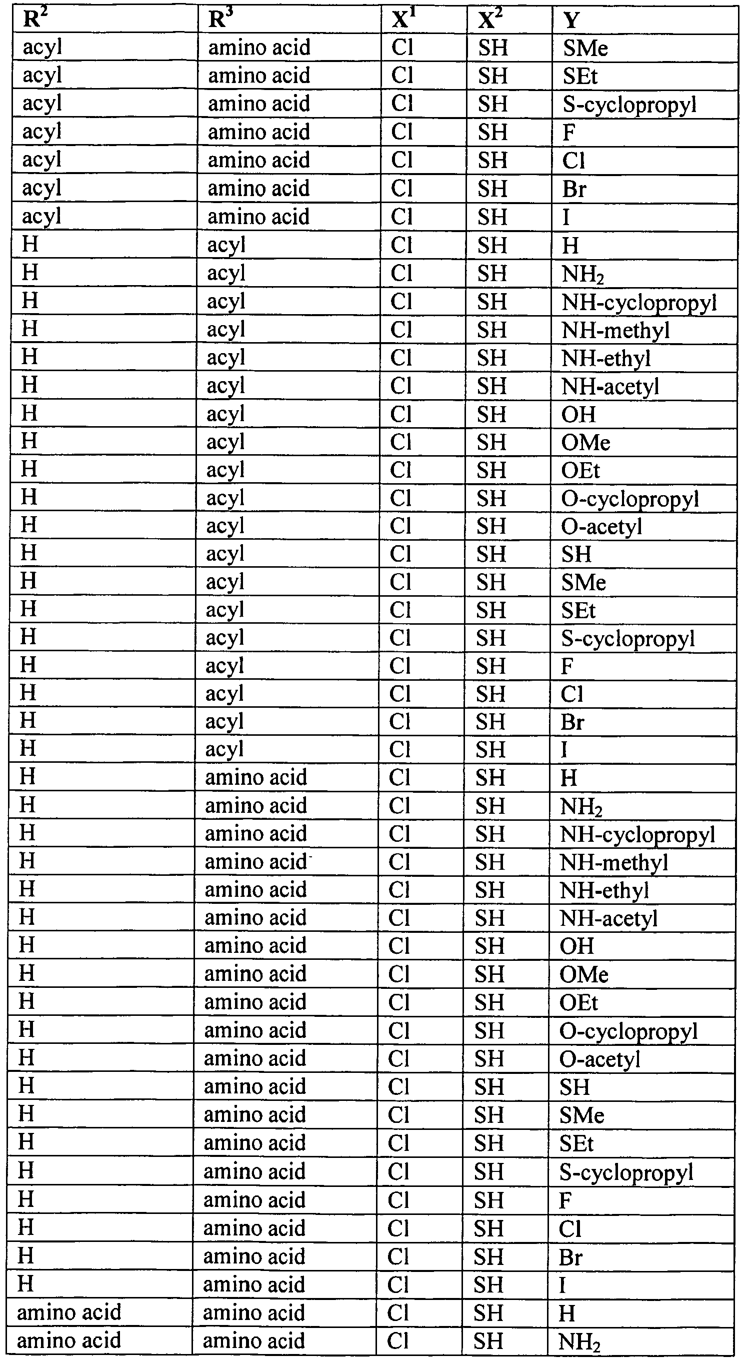

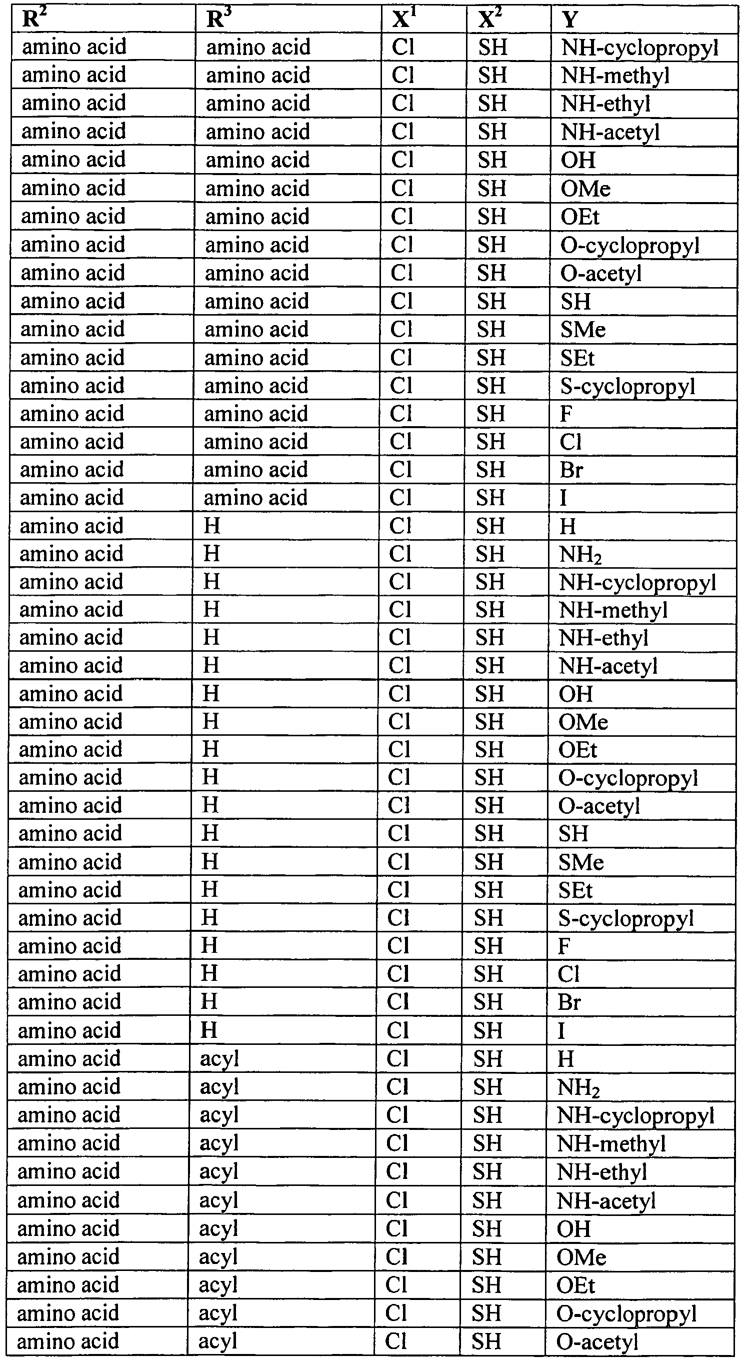

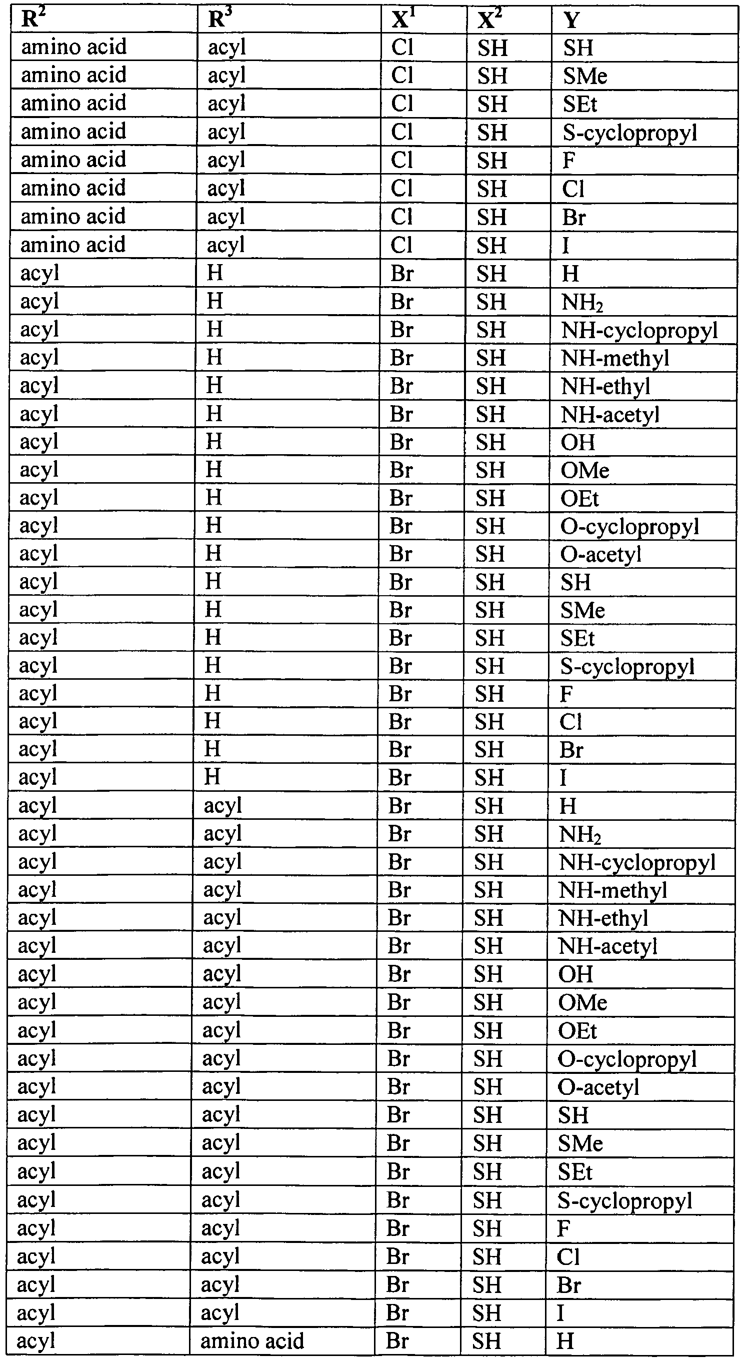

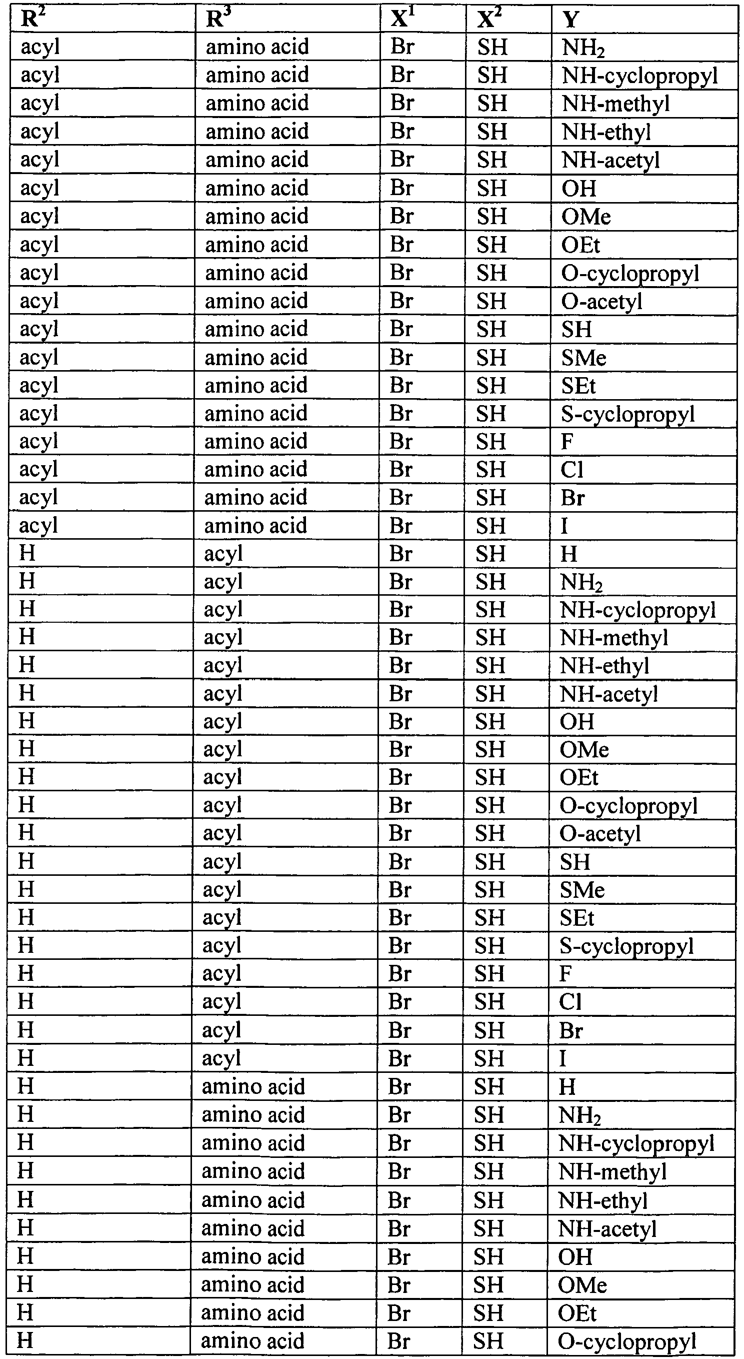

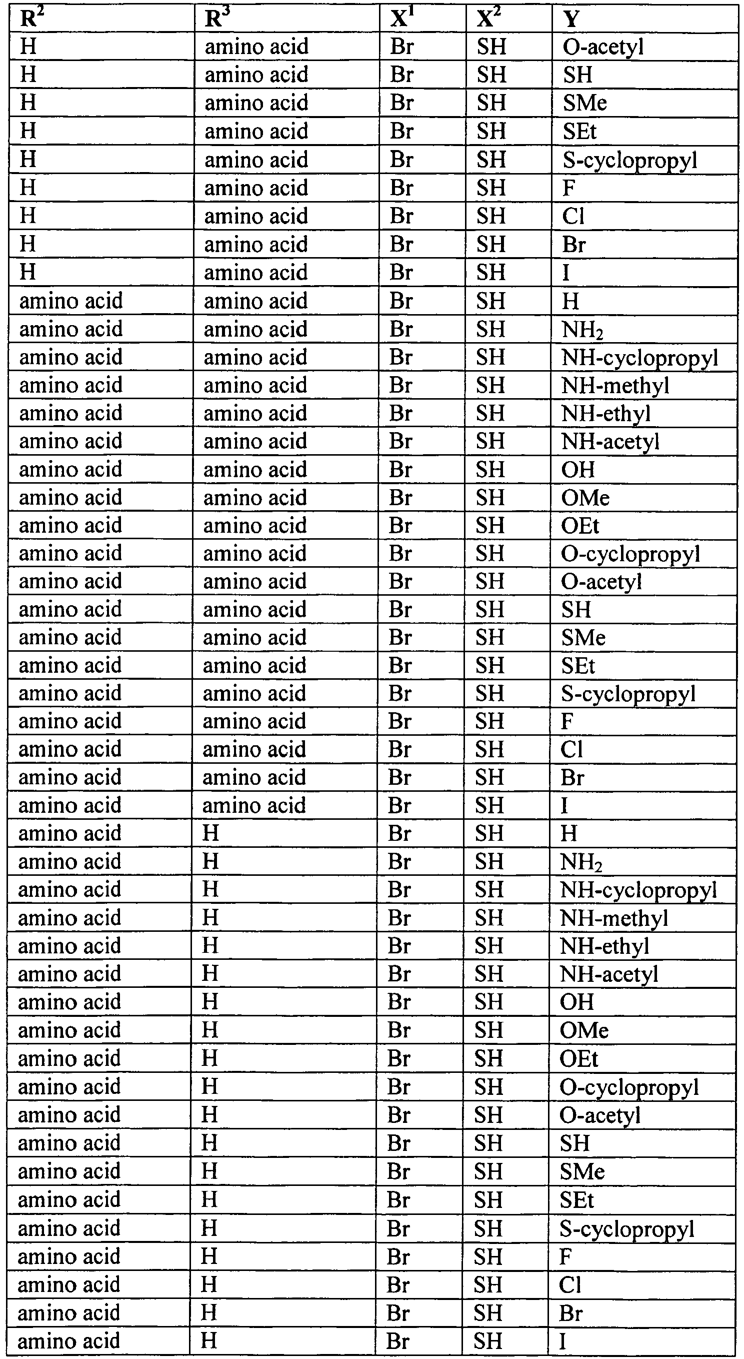

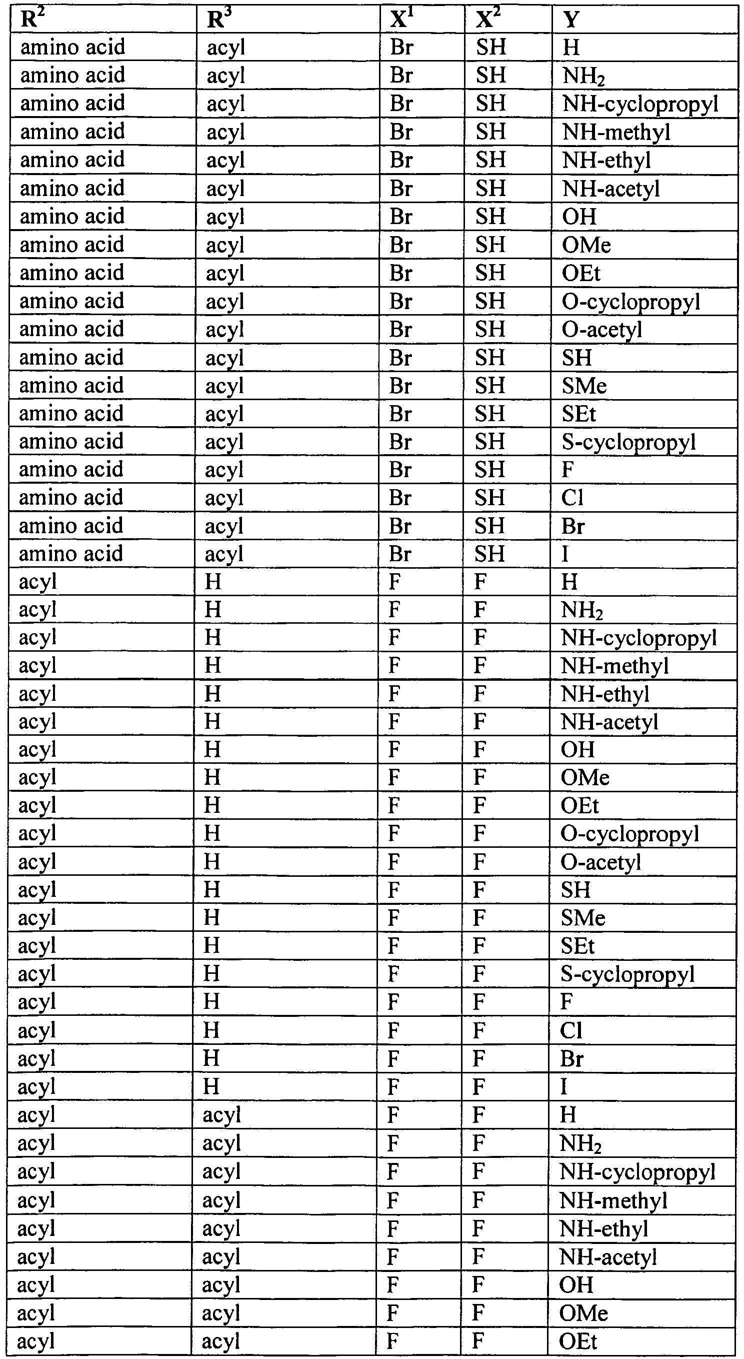

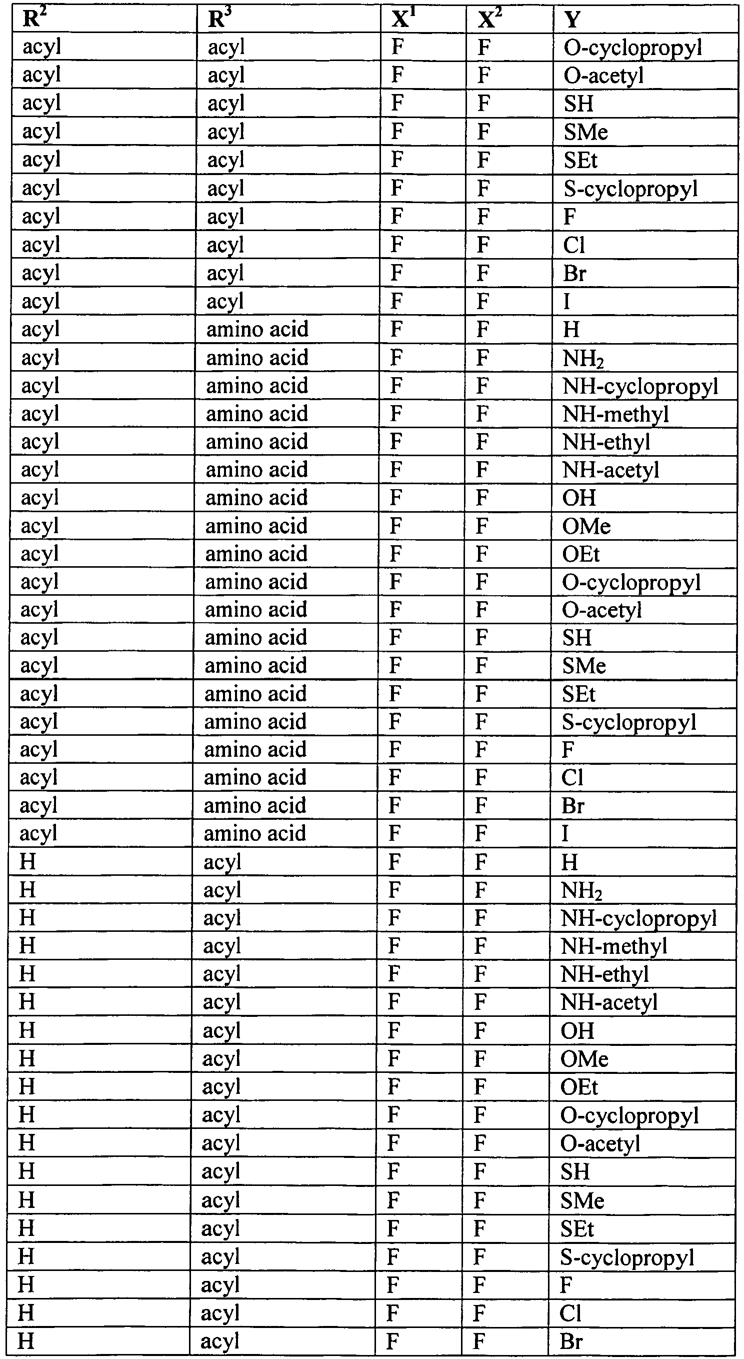

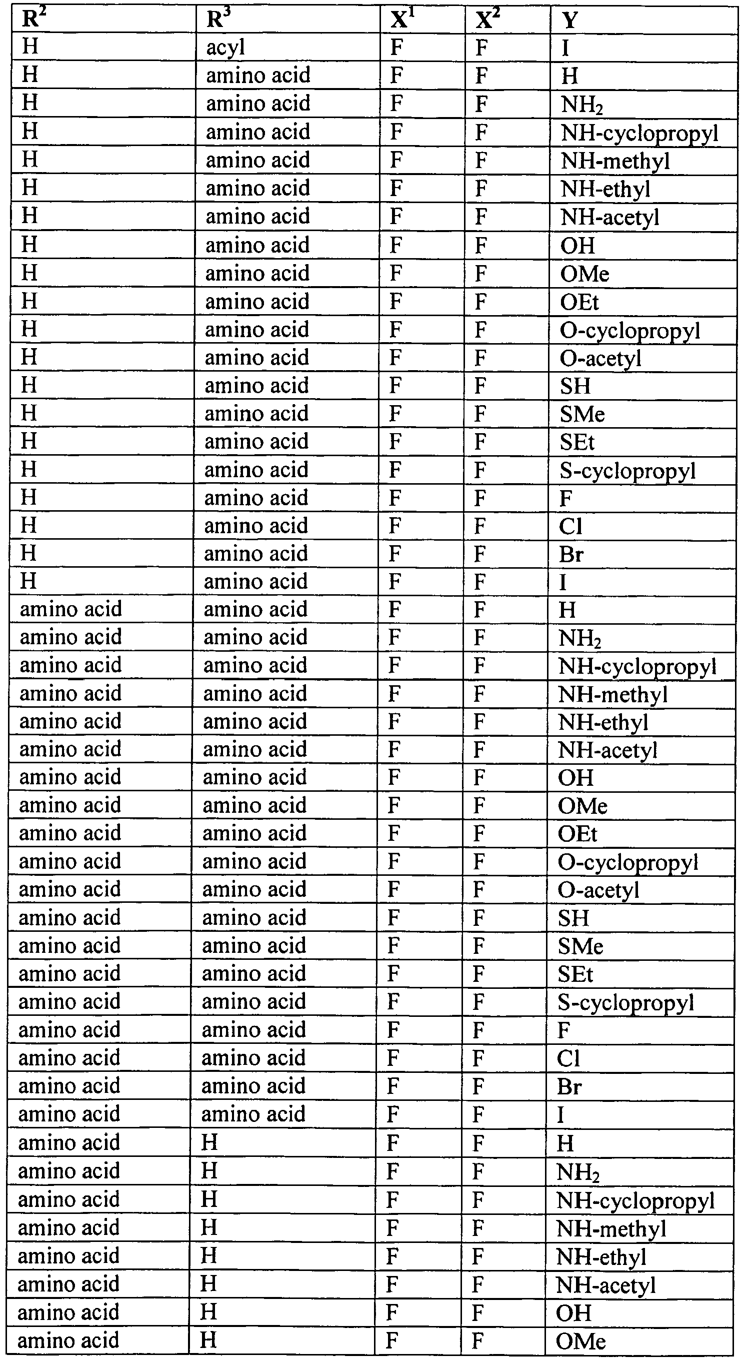

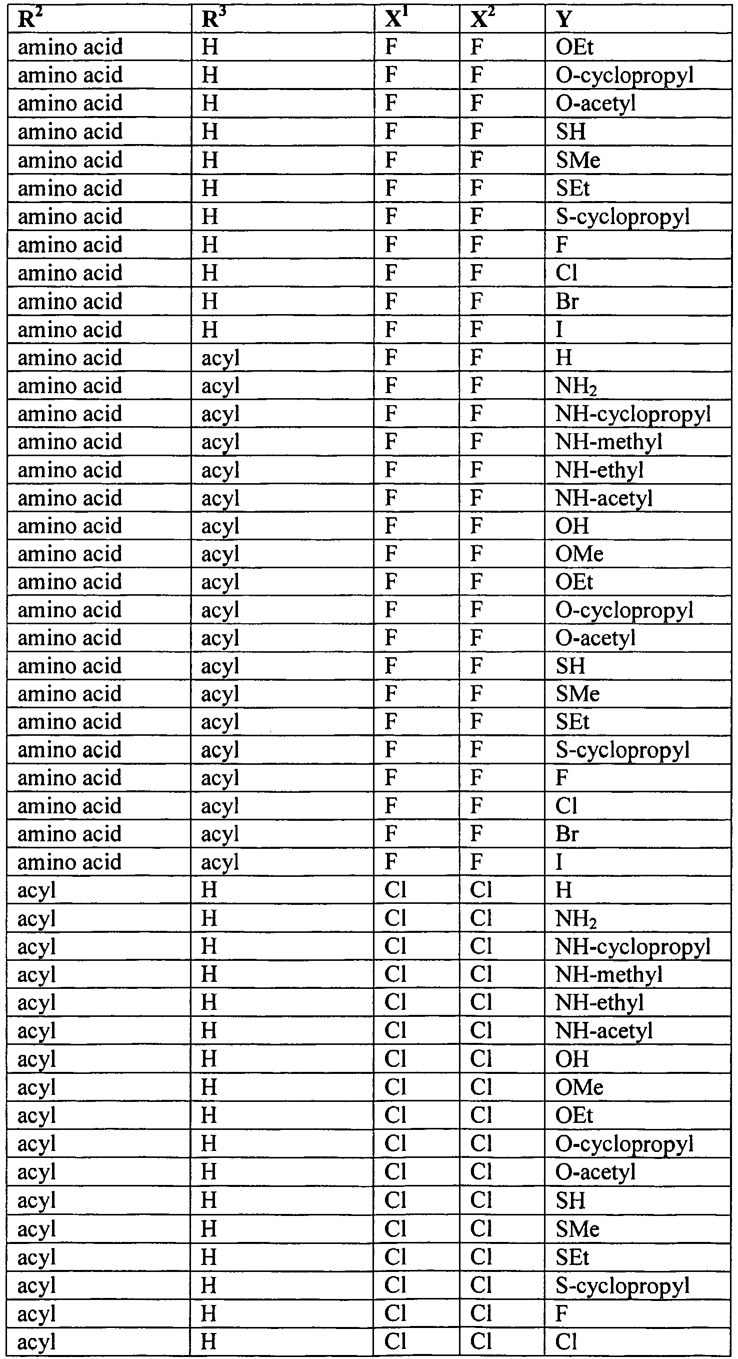

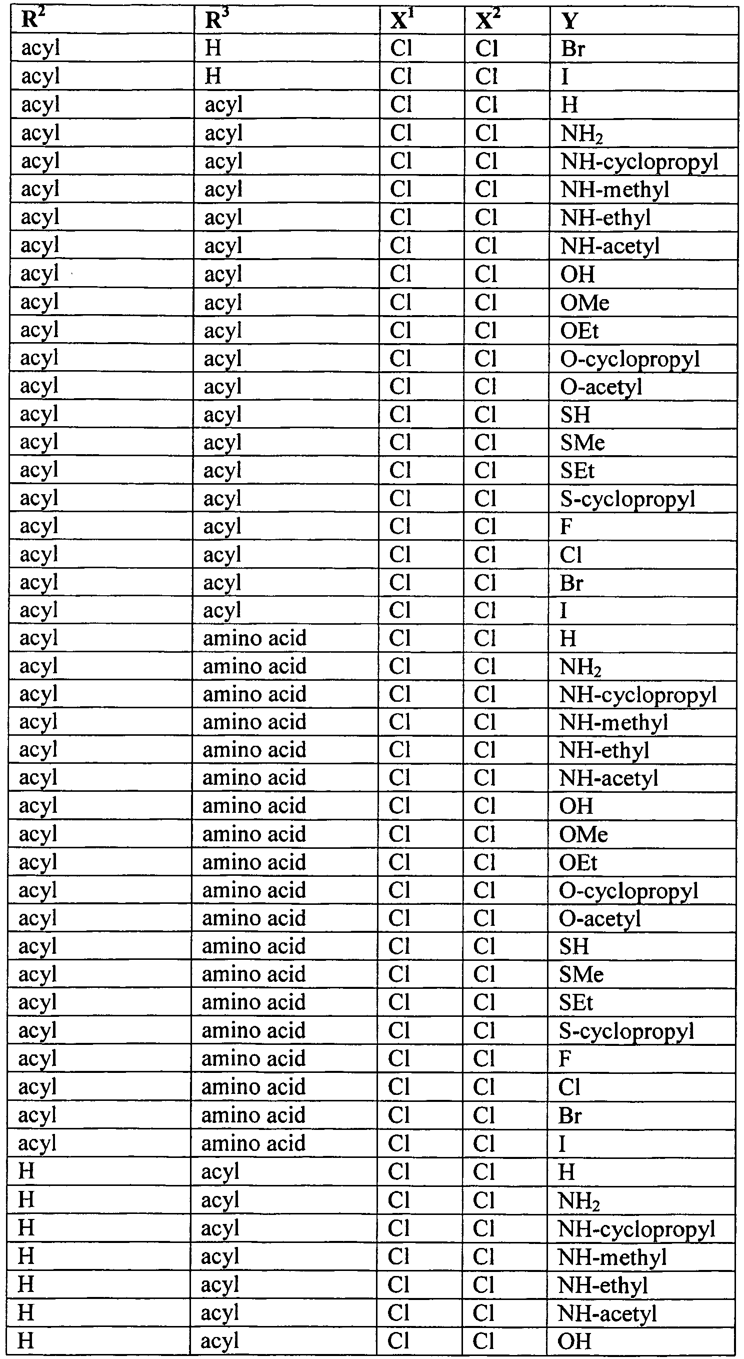

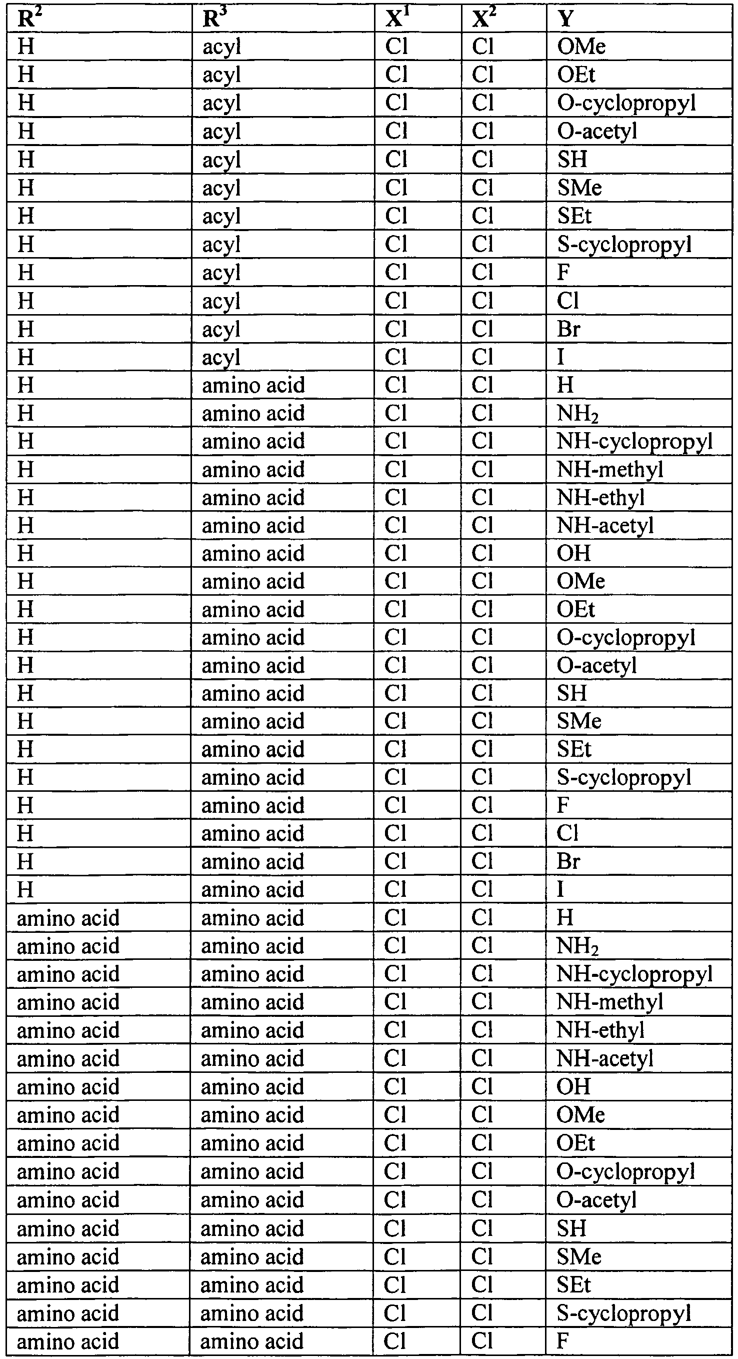

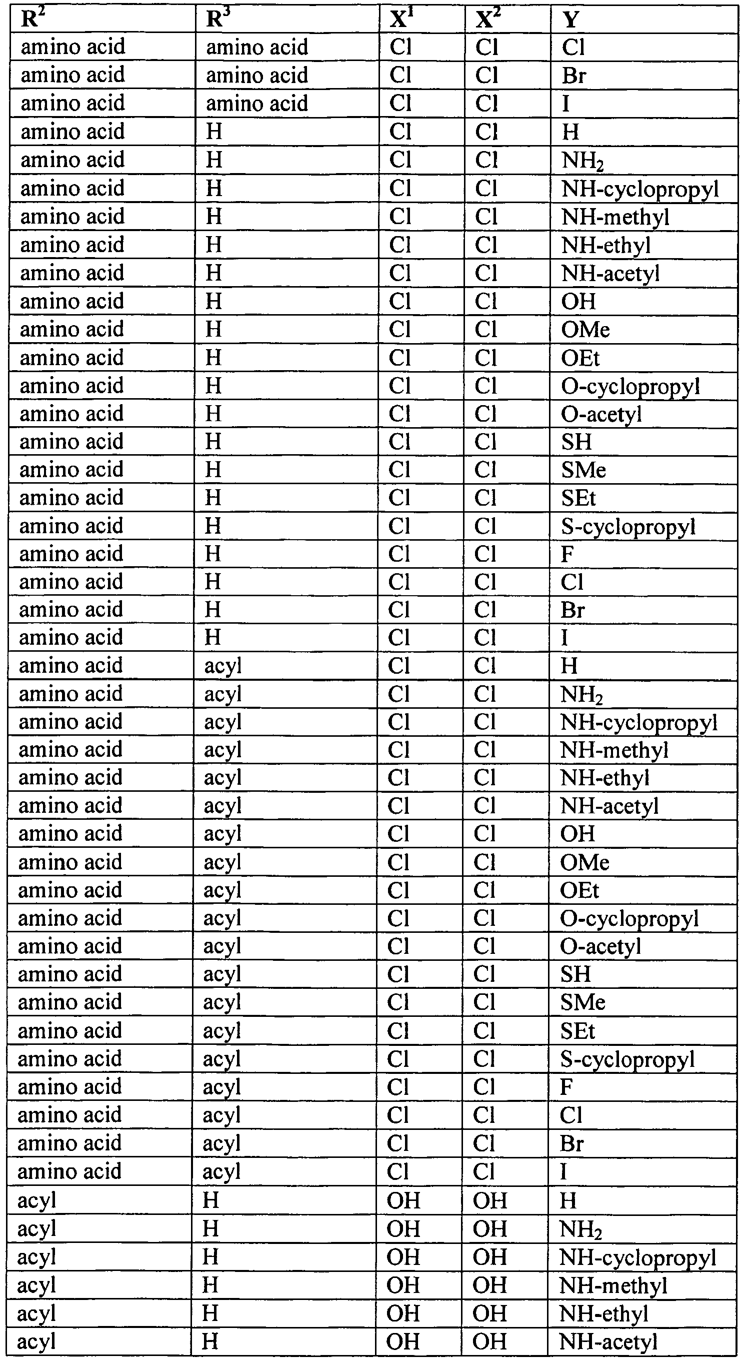

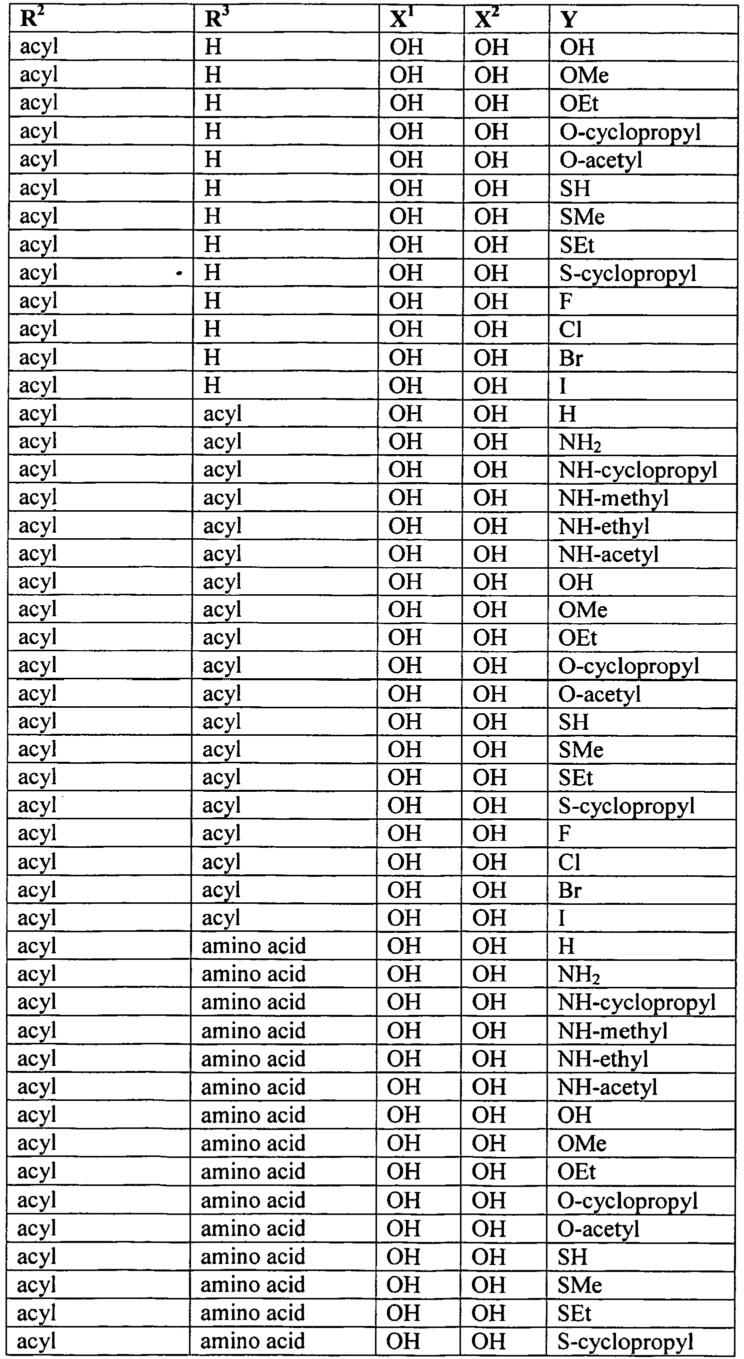

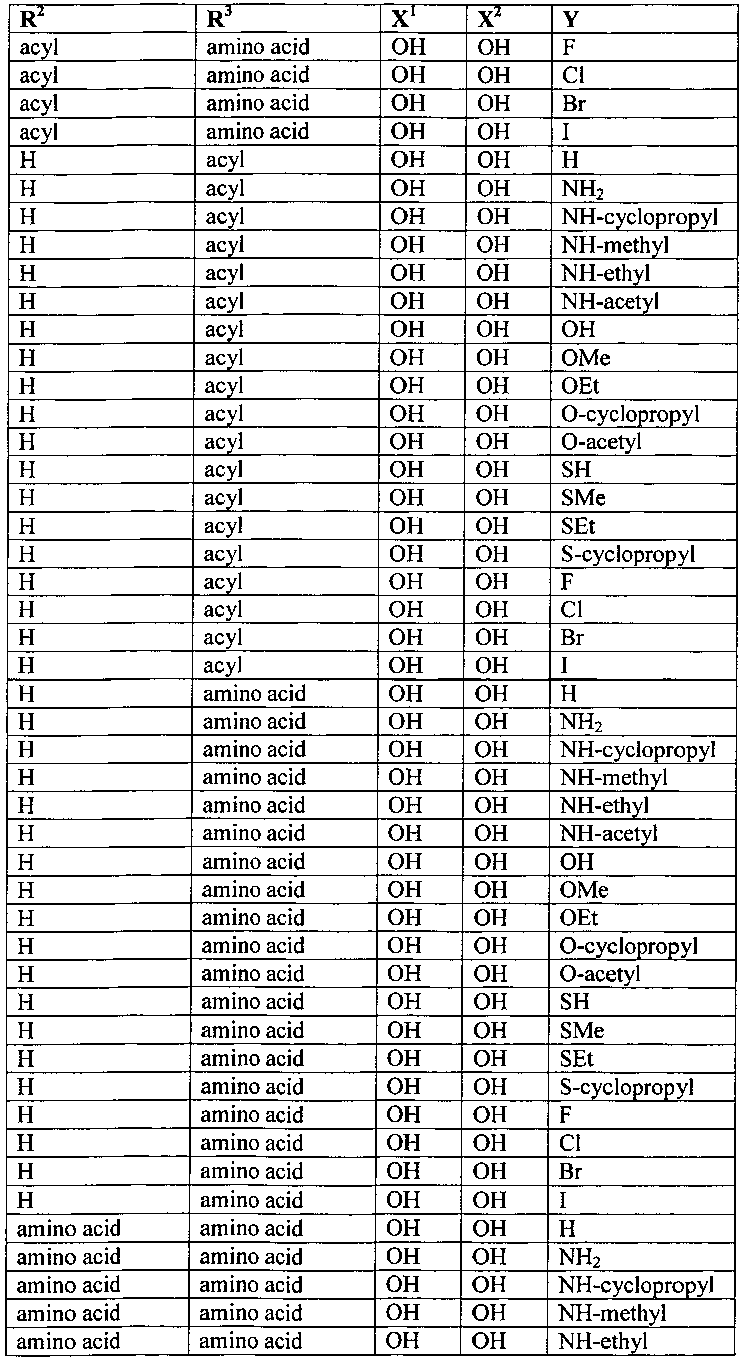

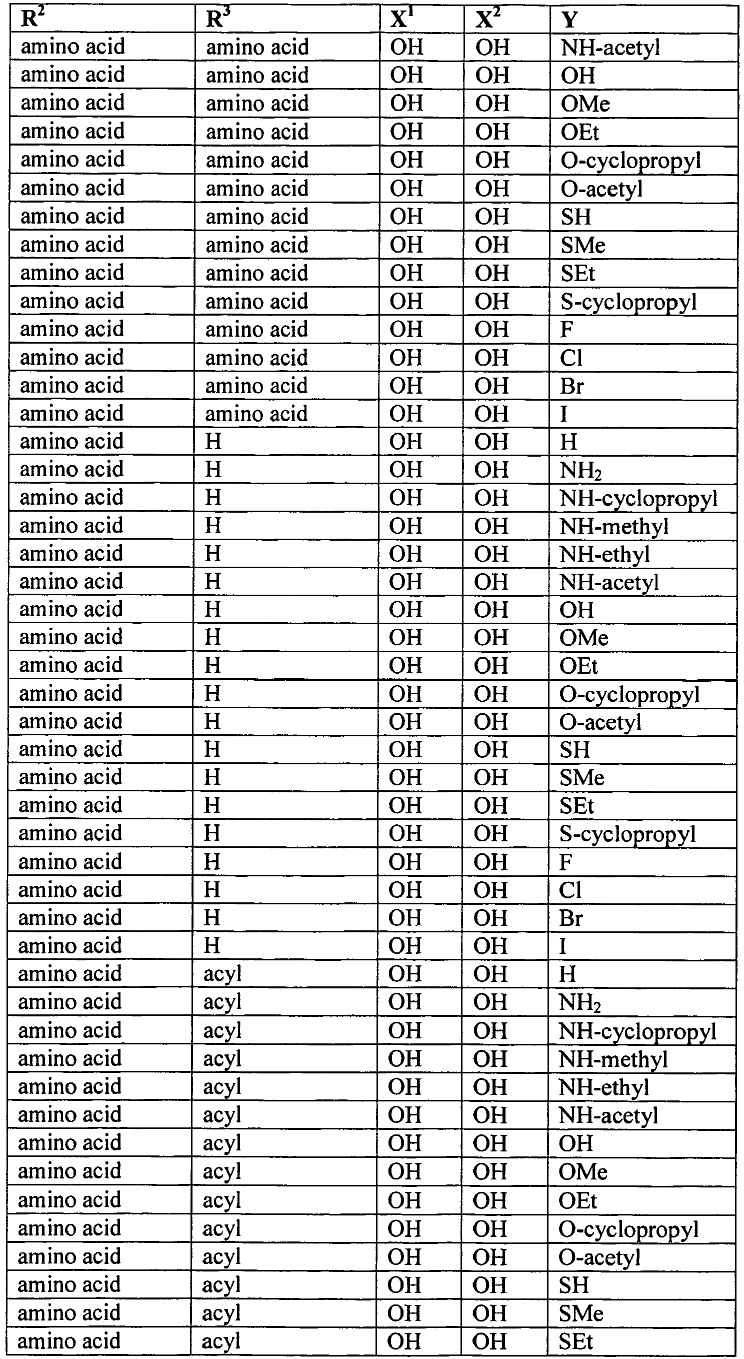

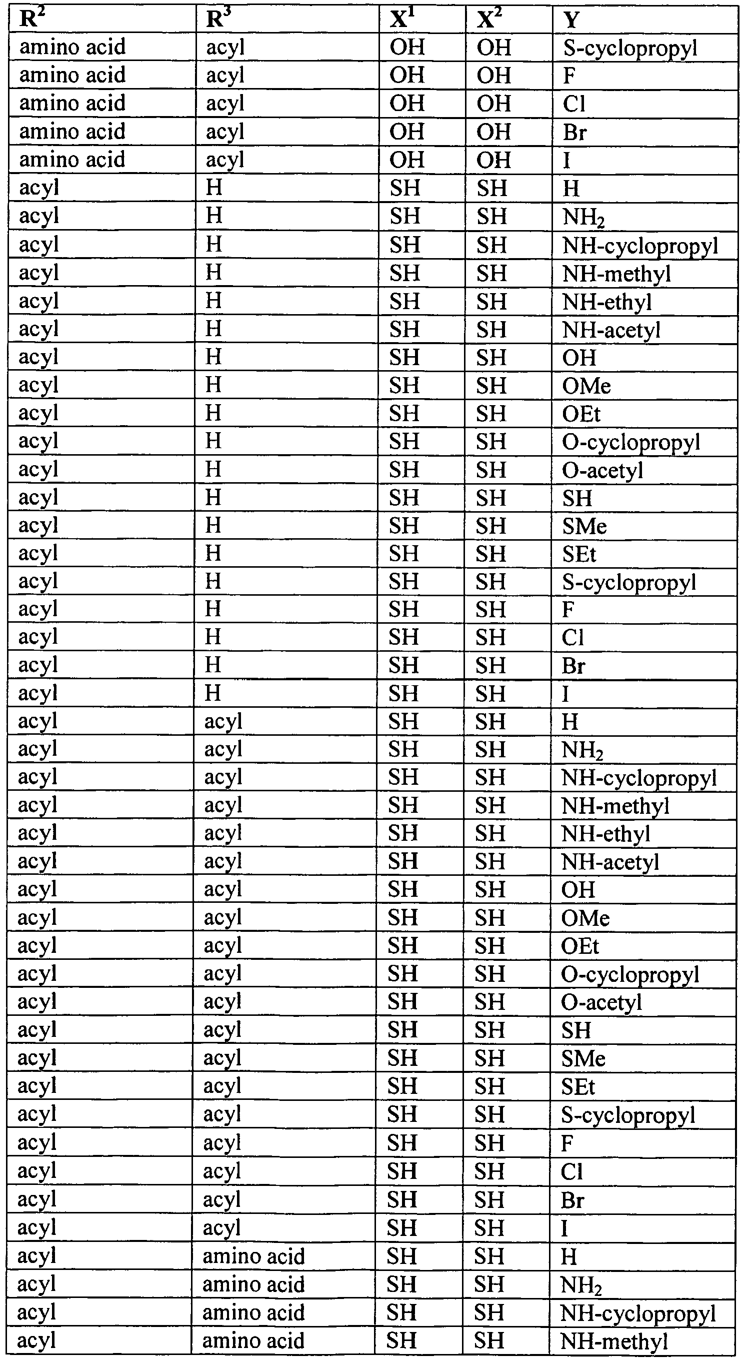

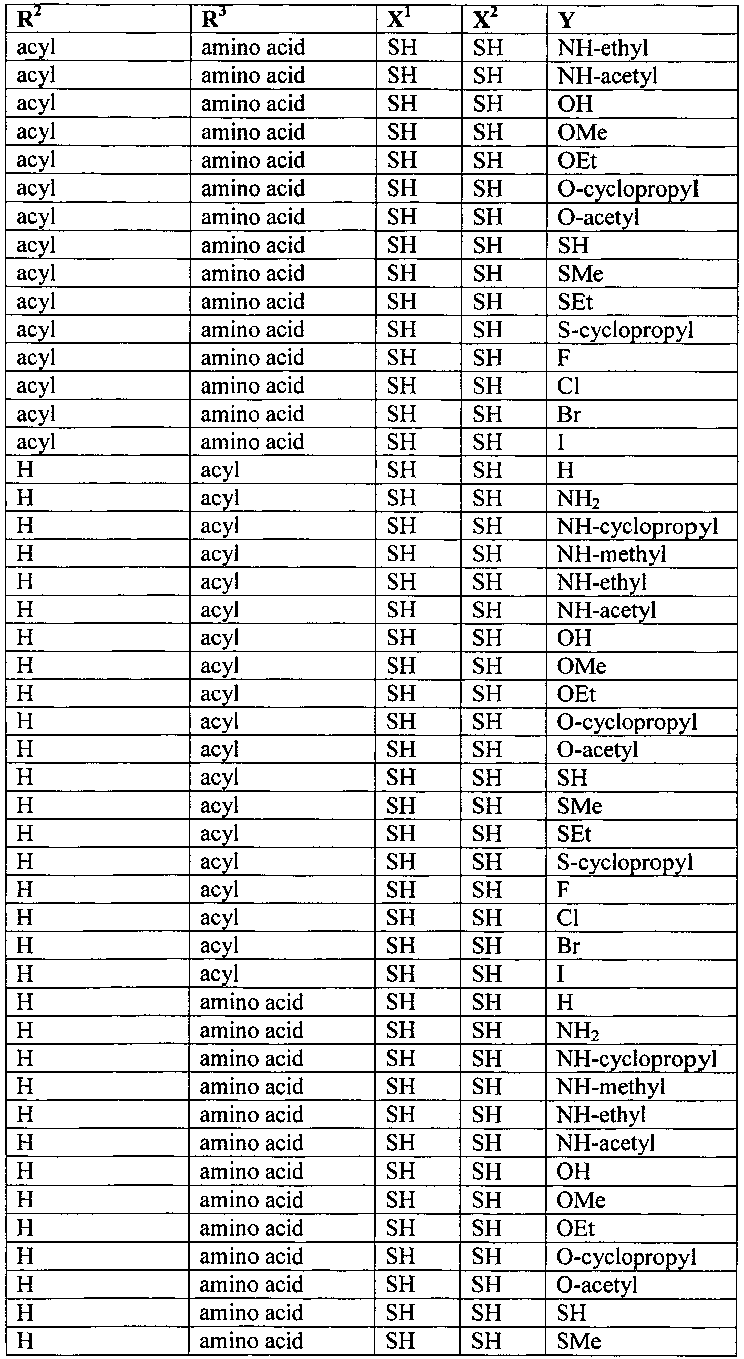

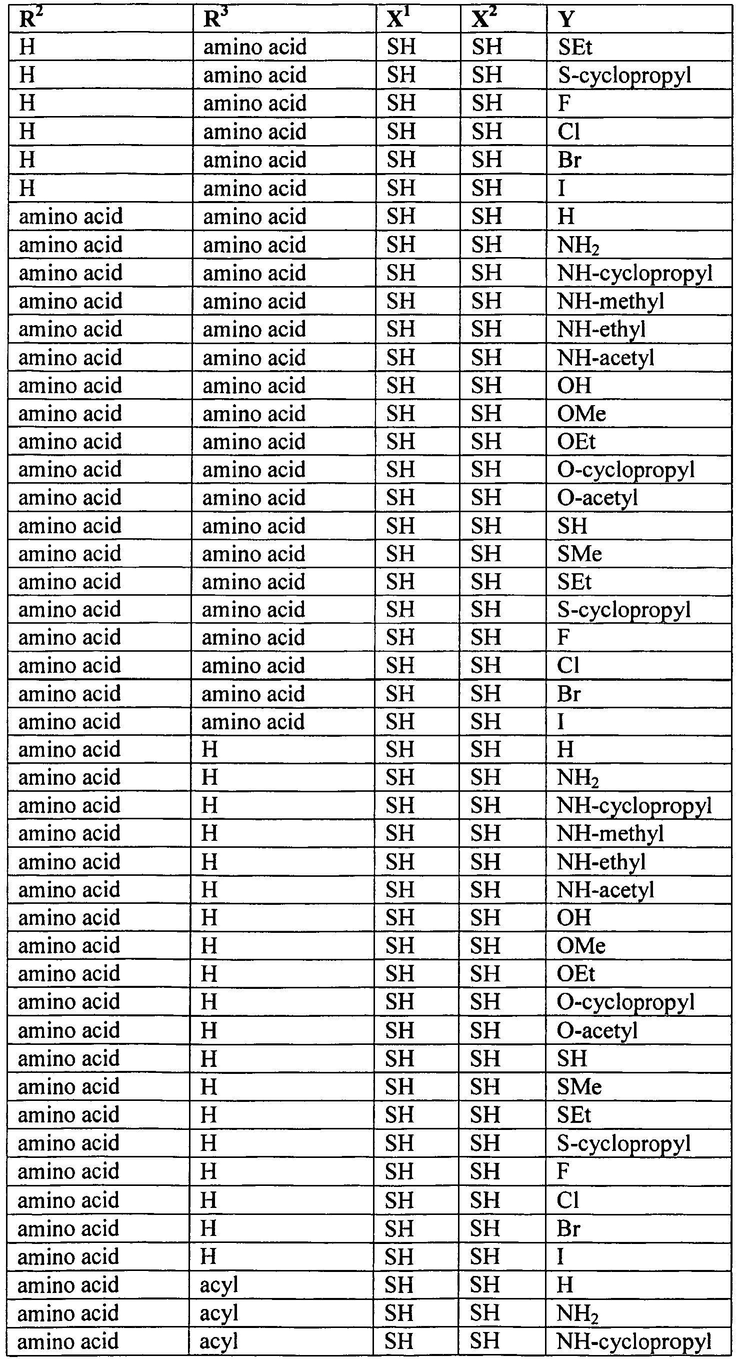

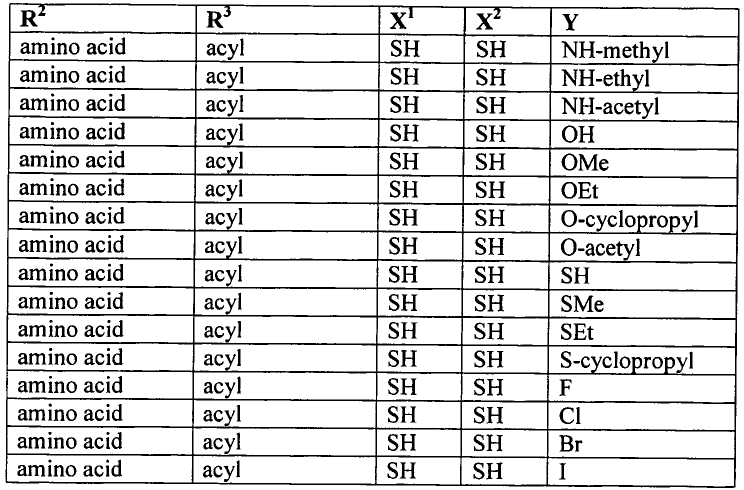

**Table 8**

| Insofar as the compounds in this table are encompassed by the compounds referred to in the enclosed claims, the present invention relates to such compounds. | | | |
|---|---|---|---|
| **R²** | **R³** | **X¹** | **Y** |
| acyl | H | H | H |
| acyl | H | H | NH₂ |
| acyl | H | H | NH-cyclopropyl |
| acyl | H | H | NH-methyl |
| acyl | H | H | NH-ethyl |
| acyl | H | H | NH-acetyl |
| acyl | H | H | OH |
| acyl | H | H | OMe |
| acyl | H | H | OEt |
| acyl | H | H | O-cyclopropyl |
| acyl | H | H | O-acetyl |
| acyl | H | H | SH |
| acyl | H | H | SMe |
| acyl | H | H | SEt |
| acyl | H | H | S-cyclopropyl |
| acyl | H | H | F |
| acyl | H | H | Cl |
| acyl | H | H | Br |
| acyl | H | H | I |
| acyl | acyl | H | H |
| acyl | acyl | H | NH₂ |
| acyl | acyl | H | NH-cyclopropyl |
| acyl | acyl | H | NH-methyl |
| acyl | acyl | H | NH-ethyl |
| acyl | acyl | H | NH-acetyl |
| acyl | acyl | H | OH |
| acyl | acyl | H | OMe |
| acyl | acyl | H | OEt |
| acyl | acyl | H | O-cyclopropyl |
| acyl | acyl | H | O-acetyt |
| acyl | acyl | H | SH |
| acyl | acyl | H | SMe |
| acyl | acyl | H | SEt |
| acyl | acyl | H | S-cyclopropyl |
| acyl | acyl | H | F |
| acyl | acyl | H | Cl |
| acyl | acyl | H | Br |
| acyl | acyl | H | I |
| acyl | amino acid | H | H |
| acyl | amino acid | H | NH₂ |
| acyl | amino acid | H | NH-cyclopropyl |
| acyl | amino acid | H | NH-methyl |
| acyl | amino acid | H | NH-ethyl |
| acyl | amino acid | H | NH-acetyl |
| acyl | amino acid | H | OH |
| acyl | amino acid | H | OMe |
| acyl | amino acid | H | OEt |
| acyl | amino acid | H | O-cyclopropyl |
| acyl | amino acid | H | O-acetyl |
| acyl | amino acid | H | SH |
| acyl | amino acid | H | SMe |
| acyl | amino acid | H | SEt |
| acyl | amino acid | H | S-cyclopropyl |
| acyl | amino acid | H | F |
| acyl | amino acid | H | Cl |
| acyl | amino acid | H | Br |
| acyl | amino acid | H | I |
| H | acyl | H | H |
| H | acyl | H | NH₂ |
| H | acyl | H | NH-cyclopropyl |
| H | acyl | H | NH-methyl |
| H | acyl | H | NH-ethyl |
| H | acyl | H | NH-acetyl |
| H | acyl | H | OH |
| H | acyl | H | OMe |
| H | acyl | H | OEt |
| H | acyl | H | O-cyclopropyl |
| H | acyl | H | O-acetyl |
| H | acyl | H | SH |
| H | acyl | H | SMe |
| H | acyl | H | SEt |
| H | acyl | H | S-cyclopropyl |
| H | acyl | H | F |
| H | acyl | H | Cl |
| H | acyl | H | Br |
| H | acyl | H | I |
| H | amino acid | H | H |
| H | amino acid | H | NH₂ |
| H | amino acid | H | NH-cyclopropyl |
| H | amino acid | H | NH-methyl |
| H | amino acid | H | NH-ethyl |
| H | amino acid | H | NH-acetyl |
| H | amino acid | H | OH |
| H | amino acid | H | OMe |
| H | amino acid | H | OEt |
| H | amino acid | H | O-cyclopropyl |
| H | amino acid | H | O-acetyl |
| H | amino acid | H | SH |
| H | amino acid | H | SMe |
| H | amino acid | H | SEt |
| H | amino acid | H | S-cyclopropyl |
| H | amino acid | H | F |

| **R²** | **R³** | **X¹** | **Y** |
|---|---|---|---|
| H | amino acid | H | Cl |
| H | amino acid | H | Br |
| H | amino acid | H | I |
| amino acid | amino acid | H | H |
| amino acid | amino acid | H | NH₂ |
| amino acid | amino acid | H | NH-cyclopropyl |
| amino acid | amino acid | H | NH-methyl |
| amino acid | amino acid | H | NH-ethyl |
| amino acid | amino acid | H | NH-acetyl |
| amino acid | amino acid | H | OH |
| amino acid | amino acid | H | OMe |
| amino acid | amino acid | H | OEt |
| amino acid | amino acid | H | O-cyclopropyl |
| amino acid | amino acid | H | O-acetyl |
| amino acid | amino acid | H | SH |
| amino acid | amino acid | H | SMe |
| amino acid | amino acid | H | SEt |
| amino acid | amino acid | H | S-cyclopropyl |
| amino acid | amino acid | H | F |
| amino acid | amino acid | H | Cl |
| amino acid | amino acid | H | Br |
| amino acid | amino acid | H | I |
| amino acid | H | H | H |
| amino acid | H | H | NH₂ |
| amino acid | H | H | NH-cyclopropyl |
| amino acid | H | H | NH-methyl |
| amino acid | H | H | NH-ethyl |
| amino acid | H | H | NH-acetyl |
| amino acid | H | H | OH |
| amino acid | H | H | OMe |
| amino acid | H | H | OEt |
| amino acid | H | H | O-cyclopropyl |
| amino acid | H | H | O-acetyl |
| amino acid | H | H | SH |
| amino acid | H | H | SMe |
| amino acid | H | H | SEt |
| amino acid | H | H | S-cyclopropyl |
| amino acid | H | H | F |
| amino acid | H | H | Cl |
| amino acid | H | H | Br |
| amino acid | H | H | I |
| amino acid | acyl | H | H |
| amino acid | acyl | H | NH₂ |
| amino acid | acyl | H | NH-cyclopropyl |
| amino acid | acyl | H | NH-methyl |
| amino acid | acyl | H | NH-ethyl |
| amino acid | acyl | H | NH-acetyl |
| amino acid | acyl | H | OH |
| amino acid | acyl | H | OMe |
| amino acid | acyl | H | OEt |
| amino acid | acyl | H | O-cyclopropyl |
| amino acid | acyl | H | O-acetyl |
| amino acid | acyl | H | SH |
| amino acid | acyl | H | SMe |
| amino acid | acyl | H | SEt |
| amino acid | acyl | H | S-cyclopropyl |
| amino acid | acyl | H | F |
| amino acid | acyl | H | Cl |
| amino acid | acyl | H | Br |
| amino acid | acyl | H | I |
| acyl | H | SH | H |
| acyl | H | SH | NH₂ |
| acyl | H | SH | NH-cyclopropyl |
| acyl | H | SH | NH-methyl |
| acyl | H | SH | NH-ethyl |
| acyl | H | SH | NH-acetyl |
| acyl | H | SH | OH |
| acyl | H | SH | OMe |
| acyl | H | SH | OEt |
| acyl | H | SH | O-cyclopropyl |
| acyl | H | SH | O-acetyl |
| acyl | H | SH | SH |
| acyl | H | SH | SMe |
| acyl | H | SH | SEt |
| acyl | H | SH | S-cyclopropyl |
| acyl | H | SH | F |
| acyl | H | SH | Cl |
| acyl | H | SH | Br |
| acyl | H | SH | I |
| acyl | acyl | SH | H |
| acyl | acyl | SH | NH₂ |
| acyl | acyl | SH | NH-cyclopropyl |
| acyl | acyl | SH | NH-methyl |
| acyl | acyl | SH | NH-ethyl |
| acyl | acyl | SH | NH-acetyl |
| acyl | acyl | SH | OH |
| acyl | acyl | SH | OMe |
| acyl | acyl | SH | OEt |
| acyl | acyl | SH | O-cyclopropyl |
| acyl | acyl | SH | O-acetyl |
| acyl | acyl | SH | SH |
| acyl | acyl | SH | SMe |
| acyl | acyl | SH | SEt |
| acyl | acyl | SH | S-cyclopropyl |
| acyl | acyl | SH | F |
| acyl | acyl | SH | Cl |
| acyl | acyl | SH | Br |
| acyl | acyl | SH | I |
| acyl | amino acid | SH | H |
| acyl | amino acid | SH | NH₂ |
| acyl | amino acid | SH | NH-cyclopropyl |
| acyl | amino acid | SH | NH-methyl |
| acyl | amino acid | SH | NH-ethyl |
| acyl | amino acid | SH | NH-acetyl |
| acyl | amino acid | SH | OH |
| acyl | amino acid | SH | OMe |
| acyl | amino acid | SH | OEt |
| acyl | amino acid | SH | O-cyclopropyl |
| acyl | amino acid | SH | O-acetyl |
| acyl | amino acid | SH | SH |
| acyl | amino acid | SH | SMe |
| acyl | amino acid | SH | SEt |
| acyl | amino acid | SH | S-cyclopropyl |
| acyl | amino acid | SH | F |
| acyl | amino acid | SH | Cl |
| acyl | amino acid | SH | Br |
| acyl | amino acid | SH | I |
| H | acyl | SH | H |
| H | acyl | SH | NH₂ |
| H | acyl | SH | NH-cyclopropyl |
| H | acyl | SH | NH-methyl |
| H | acyl | SH | NH-ethyl |
| H | acyl | SH | NH-acetyl |
| H | acyl | SH | OH |
| H | acyl | SH | OMe |
| H | acyl | SH | OEt |
| H | acyl | SH | O-cyclopropyl |
| H | acyl | SH | O-acetyl |
| H | acyl | SH | SH |
| H | acyl | SH | SMe |
| H | acyl | SH | SEt |
| H | acyl | SH | S-cyclopropyl |
| H | acyl | SH | F |
| H | acyl | SH | Cl |
| H | acyl | SH | Br |
| H | acyl | SH | I |
| H | amino acid | SH | H |
| H | amino acid | SH | NH₂ |
| H | amino acid | SH | NH-cyclopropyl |
| H | amino acid | SH | NH-methyl |
| H | amino acid | SH | NH-ethyl |
| H | amino acid | SH | NH-acetyl |
| H | amino acid | SH | OH |
| H | amino acid | SH | OMe |
| H | amino acid | SH | OEt |
| H | amino acid | SH | O-cyclopropyl |
| H | amino acid | SH | O-acetyl |
| H | amino acid | SH | SH |
| H | amino acid | SH | SMe |
| H | amino acid | SH | SEt |
| H | amino acid | SH | S-cyclopropyl |
| H | amino acid | SH | F |
| H | amino acid | SH | Cl |
| H | amino acid | SH | Br |
| H | amino acid | SH | I |
| amino acid | amino acid | SH | H |
| amino acid | amino acid | SH | NH₂ |
| amino acid | amino acid | SH | NH-cyclopropyl |
| amino acid | amino acid | SH | NH-methyl |
| amino acid | amino acid | SH | NH-ethyl |
| amino acid | amino acid | SH | NH-acetyl |
| amino acid | amino acid | SH | OH |
| amino acid | amino acid | SH | OMe |
| amino acid | amino acid | SH | OEt |
| amino acid | amino acid | SH | O-cyclopropyl |
| amino acid | amino acid | SH | O-acetyl |
| amino acid | amino acid | SH | SH |
| amino acid | amino acid | SH | SMe |
| amino acid | amino acid | SH | SEt |
| amino acid | amino acid | SH | S-cyclopropyl |
| amino acid | amino acid | SH | F |
| amino acid | amino acid | SH | Cl |
| amino acid | amino acid | SH | Br |
| amino acid | amino acid | SH | I |
| amino acid | H | SH | H |
| amino acid | H | SH | NH₂ |
| amino acid | H | SH | NH-cyclopropyl |
| amino acid | H | SH | NH-methyl |
| amino acid | H | SH | NH-ethyl |
| amino acid | H | SH | NH-acetyl |
| amino acid | H | SH | OH |
| amino acid | H | SH | OMe |
| amino acid | H | SH | OEt |
| amino acid | H | SH | O-cyclopropyl |
| amino acid | H | SH | O-acetyl |
| amino acid | H | SH | SH |
| amino acid | H | SH | SMe |
| amino acid | H | SH | SEt |
| amino acid | H | SH | S-cyclopropyl |
| amino acid | H | SH | F |
| amino acid | H | SH | Cl |
| amino acid | H | SH | Br |
| amino acid | H | SH | I |
| amino acid | acyl | SH | H |
| amino acid | acyl | SH | NH₂ |
| amino acid | acyl | SH | NH-cyclopropyl |
| amino acid | acyl | SH | NH-methyl |
| amino acid | acyl | SH | NH-ethyl |
| amino acid | acyl | SH | NH-acetyl |
| amino acid | acyl | SH | OH |
| amino acid | acyl | SH | OMe |
| amino acid | acyl | SH | OEt |
| amino acid | acyl | SH | O-cyclopropyl |
| amino acid | acyl | SH | O-acetyl |
| amino acid | acyl | SH | SH |
| amino acid | acyl | SH | SMe |
| amino acid | acyl | SH | SEt |
| amino acid | acyl | SH | S-cyclopropyl |
| amino acid | acyl | SH | F |
| amino acid | acyl | SH | Cl |
| amino acid | acyl | SH | Br |
| amino acid | acyl | SH | I |
| acyl | H | Cl | H |
| acyl | H | Cl | NH₂ |
| acyl | H | Cl | NH-cyclopropyl |
| acyl | H | Cl | NH-methyl |
| acyl | H | Cl | NH-ethyl |
| acyl | H | Cl | NH-acetyl |
| acyl | H | Cl | OH |
| acyl | H | Cl | OMe |
| acyl | H | Cl | OEt |
| acyl | H | Cl | O-cyclopropyl |
| acyl | H | Cl | O-acetyl |
| acyl | H | Cl | SH |
| acyl | H | Cl | SMe |
| acyl | H | Cl | SEt |
| acyl | H | Cl | S-cyclopropyl |
| acyl | H | Cl | F |
| acyl | H | Cl | Cl |
| acyl | H | Cl | Br |
| acyl | H | Cl | I |
| acyl | acyl | Cl | H |
| acyl | acyl | Cl | NH₂ |
| acyl | acyl | Cl | NH-cyclopropyl |
| acyl | acyl | Cl | NH-methyl |
| acyl | acyl | Cl | NH-ethyl |
| acyl | acyl | Cl | NH-acetyl |
| acyl | acyl | Cl | OH |
| acyl | acyl | Cl | OMe |
| acyl | acyl | Cl | OEt |
| acyl | acyl | Cl | O-cyclopropyl |
| acyl | acyl | Cl | O-acetyl |
| acyl | acyl | Cl | SH |
| acyl | acyl | Cl | SMe |
| acyl | acyl | Cl | SEt |
| acyl | acyl | Cl | S-cyclopropyl |
| acyl | acyl | Cl | F |
| acyl | acyl | Cl | Cl |
| acyl | acyl | Cl | Br |
| acyl | acyl | Cl | I |
| acyl | amino acid | Cl | H |
| acyl | amino acid | Cl | NH₂ |
| acyl | amino acid | Cl | NH-cyclopropyl |
| acyl | amino acid | Cl | NH-methyl |
| acyl | amino acid | Cl | NH-ethyl |
| acyl | amino acid | Cl | NH-acetyl |
| acyl | amino acid | Cl | OH |
| acyl | amino acid | Cl | OMe |
| acyl | amino acid | Cl | OEt |
| acyl | amino acid | Cl | O-cyclopropyl |
| acyl | amino acid | Cl | O-acetyl |
| acyl | amino acid | Cl | SH |
| acyl | amino acid | Cl | SMe |
| acyl | amino acid | Cl | SEt |
| acyl | amino acid | Cl | S-cyclopropyl |
| acyl | amino acid | Cl | F |
| acyl | amino acid | Cl | Cl |
| acyl | amino acid | Cl | Br |
| acyl | amino acid | Cl | I |
| H | acyl | Cl | H |
| H | acyl | Cl | NH₂ |
| H | acyl | Cl | NH-cyclopropyl |
| H | acyl | Cl | NH-methyl |
| H | acyl | Cl | NH-ethyl |
| H | acyl | Cl | NH-acetyl |
| H | acyl | Cl | OH |
| H | acyl | Cl | OMe |
| H | acyl | Cl | OEt |
| H | acyl | Cl | O-cyclopropyl |
| H | acyl | Cl | O-acetyl |
| H | acyl | Cl | SH |
| H | acyl | Cl | SMe |
| H | acyl | Cl | SEt |
| H | acyl | Cl | S-cyclopropyl |
| H | acyl | Cl | F |
| H | acyl | Cl | Cl |
| H | acyl | Cl | Br |
| H | acyl | Cl | I |
| H | amino acid | Cl | H |
| H | amino acid | Cl | NH₂ |
| H | amino acid | Cl | NH-cyclopropyl |
| H | amino acid | Cl | NH-methyl |
| H | amino acid | Cl | NH-ethyl |
| H | amino acid | Cl | NH-acetyl |
| H | amino acid | Cl | OH |
| H | amino acid | Cl | OMe |
| H | amino acid | Cl | OEt |
| H | amino acid | Cl | O-cyclopropyl |
| H | amino acid | Cl | O-acetyl |
| H | amino acid | Cl | SH |
| H | amino acid | Cl | SMe |
| H | amino acid | Cl | SEt |
| H | amino acid | Cl | S-cyclopropyl |
| H | amino acid | Cl | F |
| H | amino acid | Cl | Cl |
| H | amino acid | Cl | Br |
| H | amino acid | Cl | I |
| amino acid | amino acid | Cl | H |
| amino acid | amino acid | Cl | NH₂ |
| amino acid | amino acid | Cl | NH-cyclopropyl |
| amino acid | amino acid | Cl | NH-methyl |
| amino acid | amino acid | Cl | NH-ethyl |
| amino acid | amino acid | Cl | NH-acetyl |
| amino acid | amino acid | Cl | OH |
| amino acid | amino acid | Cl | OMe |
| amino acid | amino acid | Cl | OEt |
| amino acid | amino acid | Cl | O-cyclopropyl |
| amino acid | amino acid | Cl | O-acetyl |
| amino acid | amino acid | Cl | SH |
| amino acid | amino acid | Cl | SMe |
| amino acid | amino acid | Cl | SEt |
| amino acid | amino acid | Cl | S-cyclopropyl |
| amino acid | amino acid | Cl | F |
| amino acid | amino acid | Cl | Cl |
| amino acid | amino acid | Cl | Br |
| amino acid | amino acid | Cl | I |
| amino acid | H | Cl | H |
| amino acid | H | Cl | NH₂ |
| amino acid | H | Cl | NH-cyclopropyl |
| amino acid | H | Cl | NH-methyl |
| amino acid | H | Cl | NH-methyl |
| amino acid | H | Cl | NH-acetyl |
| amino acid | H | Cl | OH |
| amino acid | H | Cl | OMe |
| amino acid | H | Cl | OEt |
| amino acid | H | Cl | O-cyclopropyl |
| amino acid | H | Cl | O-acetyl |
| amino acid | H | Cl | SH |
| amino acid | H | Cl | SMe |
| amino acid | H | Cl | SEt |
| amino acid | H | Cl | S-cyclopropyl |
| amino acid | H | Cl | F |
| amino acid | H | Cl | Cl |
| amino acid | H | Cl | Br |
| amino acid | H | Cl | I |
| amino acid | acyl | Cl | H |
| amino acid | acyl | Cl | NH₂ |
| amino acid | acyl | Cl | NH-cyclopropyl |
| amino acid | acyl | Cl | NH-methyl |
| amino acid | acyl | Cl | NH-ethyl |
| amino acid | acyl | Cl | NH-acetyl |
| amino acid | acyl | Cl | OH |
| amino acid | acyl | Cl | OMe |
| amino acid | acyl | Cl | OEt |
| amino acid | acyl | Cl | O-cyclopropyl |
| amino acid | acyl | Cl | O-acetyl |
| amino acid | acyl | Cl | SH |
| amino acid | acyl | Cl | SMe |
| amino acid | acyl | Cl | SEt |
| amino acid | acyl | Cl | S-cyclopropyl |
| amino acid | acyl | Cl | F |
| amino acid | acyl | Cl | Cl |
| amino acid | acyl | Cl | Br |
| amino acid | acyl | Cl | I |
| acyl | H | Br | H |
| acyl | H | Br | NH₂ |
| acyl | H | Br | NH-cyclopropyl |
| acyl | H | Br | NH-methyl |
| acyl | H | Br | NH-ethyl |
| acyl | H | Br | NH-acetyl |
| acyl | H | Br | OH |
| acyl | H | Br | OMe |
| acyl | H | Br | OEt |
| acyl | H | Br | O-cyclopropyl |
| acyl | H | Br | O-acetyl |
| acyl | H | Br | SH |
| acyl | H | Br | SMe |
| acyl | H | Br | SEt |
| acyl | H | Br | S-cyclopropyl |
| acyl | H | Br | F |
| acyl | H | Br | Cl |
| acyl | H | Br | Br |
| acyl | H | Br | I |
| acyl | acyl | Br | H |
| acyl | acyl | Br | NH₂ |
| acyl | acyl | Br | NH-cyclopropyl |
| acyl | acyl | Br | NH-methyl |
| acyl | acyl | Br | NH-ethyl |
| acyl | acyl | Br | NH-acetyl |
| acyl | acyl | Br | OH |
| acyl | acyl | Br | OMe |
| acyl | acyl | Br | OEt |
| acyl | acyl | Br | O-cyclopropyl |
| acyl | acyl | Br | O-acetyl |
| acyl | acyl | Br | SH |
| acyl | acyl | Br | SMe |
| acyl | acyl | Br | SEt |
| acyl | acyl | Br | S-cyclopropyl |
| acyl | acyl | Br | F |
| acyl | acyl | Br | Cl |
| acyl | acyl | Br | Br |
| acyl | acyl | Br | I |
| acyl | amino acid | Br | H |
| acyl | amino acid | Br | NH₂ |
| acyl | amino acid | Br | NH-cyclopropyl |
| acyl | amino acid | Br | NH-methyl |
| acyl | amino acid | Br | NH-ethyl |
| acyl | amino acid | Br | NH-acetyl |
| acyl | amino acid | Br | OH |
| acyl | amino acid | Br | OMe |
| acyl | amino acid | Br | OEt |
| acyl | amino acid | Br | O-cyclopropyl |
| acyl | amino acid | Br | O-acetyl |
| acyl | amino acid | Br | SH |
| acyl | amino acid | Br | SMe |
| acyl | amino acid | Br | SEt |
| acyl | amino acid | Br | S-cyclopropyl |
| acyl | amino acid | Br | F |
| acyl | amino acid | Br | Cl |
| acyl | amino acid | Br | Br |
| acyl | amino acid | Br | I |
| H | acyl | Br | H |
| H | acyl | Br | NH₂ |
| H | acyl | Br | NH-cyclopropyl |
| H | acyl | Br | NH-methyl |
| H | acyl | Br | NH-ethyl |
| H | acyl | Br | NH-acetyl |
| H | acyl | Br | OH |
| H | acyl | Br | OMe |
| H | acyl | Br | OEt |
| H | acyl | Br | O-cyclopropyl |
| H | acyl | Br | O-acetyl |
| H | acyl | Br | SH |
| H | acyl | Br | SMe |
| H | acyl | Br | SEt |
| H | acyl | Br | S-cyclopropyl |
| H | acyl | Br | F |
| H | acyl | Br | Cl |
| H | acyl | Br | Br |
| H | acyl | Br | I |
| H | amino acid | Br | H |
| H | amino acid | Br | NH₂ |
| H | amino acid | Br | NH-cyclopropyl |
| H | amino acid | Br | NH-methyl |
| H | amino acid | Br | NH-ethyl |
| H | amino acid | Br | NH-acetyl |
| H | amino acid | Br | OH |
| H | amino acid | Br | OMe |
| H | amino acid | Br | OEt |
| H | amino acid | Br | O-cyclopropyl |
| H | amino acid | Br | O-acetyl |
| H | amino acid | Br | SH |
| H | amino acid | Br | SMe |
| H | amino acid | Br | SEt |
| H | amino acid | Br | S-cyclopropyl |
| H | amino acid | Br | F |
| H | amino acid | Br | Cl |
| H | amino acid | Br | Br |
| H | amino acid | Br | I |
| amino acid | amino acid | Br | H |
| amino acid | amino acid | Br | NH₂ |
| amino acid | amino acid | Br | NH-cyclopropyl |
| amino acid | amino acid | Br | NH-methyl |
| amino acid | amino acid | Br | NH-ethyl |
| amino acid | amino acid | Br | NH-acetyl |
| amino acid | amino acid | Br | OH |
| amino acid | amino acid | Br | OMe |
| amino acid | amino acid | Br | OEt |
| amino acid | amino acid | Br | O-cyclopropyl |
| amino acid | amino acid | Br | O-acetyl |
| amino acid | amino acid | Br | SH |
| amino acid | amino acid | Br | SMe |
| amino acid | amino acid | Br | SEt |
| amino acid | amino acid | Br | S-cyclopropyl |
| amino acid | amino acid | Br | F |
| amino acid | amino acid | Br | Cl |
| amino acid | amino acid | Br | Br |
| amino acid | amino acid | Br | I |
| amino acid | H | Br | H |
| amino acid | H | Br | NH₂ |
| amino acid | H | Br | NH-cyclopropyl |
| amino acid | H | Br | NH-methyl |
| amino acid | H | Br | NH-ethyl |
| amino acid | H | Br | NH-acetyl |
| amino acid | H | Br | OH |
| amino acid | H | Br | OMe |
| amino acid | H | Br | OEt |
| amino acid | H | Br | O-cyclopropyl |
| amino acid | H | Br | O-acetyl |
| amino acid | H | Br | SH |
| amino acid | H | Br | SMe |
| amino acid | H | Br | SEt |
| amino acid | H | Br | S-cyclopropyl |
| amino acid | H | Br | F |
| amino acid | H | Br | Cl |
| amino acid | H | Br | Br |
| amino acid | H | Br | I |
| amino acid | acyl | Br | H |
| amino acid | acyl | Br | NH₂ |
| amino acid | acyl | Br | NH-cyclopropyl |
| amino acid | acyl | Br | NH-methyl |
| amino acid | acyl | Br | NH-ethyl |
| amino acid | acyl | Br | NH-acetyl |
| amino acid | acyl | Br | OH |
| amino acid | acyl | Br | OMe |
| amino acid | acyl | Br | OEt |
| amino acid | acyl | Br | O-cyclopropyl |
| amino acid | acyl | Br | O-acetyl |
| amino acid | acyl | Br | SH |
| amino acid | acyl | Br | SMe |
| amino acid | acyl | Br | SEt |
| amino acid | acyl | Br | S-cyclopropyl |
| amino acid | acyl | Br | F |
| amino acid | acyl | Br | Cl |
| amino acid | acyl | Br | Br |
| amino acid | acyl | Br | I |
| acyl | H | NH₂ | H |
| acyl | H | NH₂ | NH₂ |
| acyl | H | NH₂ | NH-cyclopropyl |
| acyl | H | NH₂ | NH-methyl |
| acyl | H | NH₂ | NH-ethyl |
| acyl | H | NH₂ | NH-acetyl |
| acyl | H | NH₂ | OH |
| acyl | H | NH₂ | OMe |
| acyl | H | NH₂ | OEt |
| acyl | H | NH₂ | O-cyclopropyl |
| acyl | H | NH₂ | O-acetyl |
| acyl | H | NH₂ | SH |
| acyl | H | NH₂ | SMe |
| acyl | H | NH₂ | SEt |
| acyl | H | NH₂ | S-cyclopropyl |
| acyl | H | NH₂ | F |
| acyl | H | NH₂ | Cl |
| acyl | H | NH₂ | Br |
| acyl | H | NH₂ | I |
| acyl | acyl | NH₂ | H |
| acyl | acyl | NH₂ | NH₂ |
| acyl | acyl | NH₂ | NH-cyclopropyl |
| acyl | acyl | NH₂ | NH-methyl |
| acyl | acyl | NH₂ | NH-ethyl |
| acyl | acyl | NH₂ | NH-acetyl |
| acyl | acyl | NH₂ | OH |
| acyl | acyl | NH₂ | OMe |
| acyl | acyl | NH₂ | OEt |
| acyl | acyl | NH₂ | O-cyclopropyl |
| acyl | acyl | NH₂ | O-acetyl |
| acyl | acyl | NH₂ | SH |
| acyl | acyl | NH₂ | SMe |
| acyl | acyl | NH₂ | SEt |
| acyl | acyl | NH₂ | S-cyclopropyl |
| acyl | acyl | NH₂ | F |
| acyl | acyl | NH₂ | Cl |
| acyl | acyl | NH₂ | Br |
| acyl | acyl | NH₂ | I |
| acyl | amino acid | NH₂ | H |
| acyl | amino acid | NH₂ | NH₂ |
| acyl | amino acid | NH₂ | NH-cyclopropyl |
| acyl | amino acid | NH₂ | NH-methyl |
| acyl | amino acid | NH₂ | NH-ethyl |
| acyl | amino acid | NH₂ | NH-acetyl |
| acyl | amino acid | NH₂ | OH |
| acyl | amino acid | NH₂ | OMe |
| acyl | amino acid | NH₂ | OEt |
| acyl | amino acid | NH₂ | O-cyclopropyl |
| acyl | amino acid | NH₂ | O-acetyl |
| acyl | amino acid | NH₂ | SH |
| acyl | amino acid | NH₂ | SMe |
| acyl | amino acid | NH₂ | SEt |
| acyl | amino acid | NH₂ | S-cyclopropyl |
| acyl | amino acid | NH₂ | F |
| acyl | amino acid | NH₂ | Cl |
| acyl | amino acid | NH₂ | Br |
| acyl | amino acid | NH₂ | I |
| H | acyl | NH₂ | H |
| H | acyl | NH₂ | NH₂ |
| H | acyl | NH₂ | NH-cyclopropyl |
| H | acyl | NH₂ | NH-methyl |
| H | acyl | NH₂ | NH-ethyl |
| H | acyl | NH₂ | NH-acetyl |
| H | acyl | NH₂ | OH |
| H | acyl | NH₂ | OMe |
| H | acyl | NH₂ | OEt |
| H | acyl | NH₂ | O-cyclopropyl |
| H | acyl | NH₂ | O-acetyl |
| H | acyl | NH₂ | SH |
| H | acyl | NH₂ | SMe |
| H | acyl | NH₂ | SEt |
| H | acyl | NH₂ | S-cyclopropyl |
| H | acyl | NH₂ | F |
| H | acyl | NH₂ | Cl |
| H | acyl | NH₂ | Br |
| H | acyl | NH₂ | I |
| H | amino acid | NH₂ | H |
| H | amino acid | NH₂ | NH₂ |
| H | amino acid | NH₂ | NH-cyclopropyl |
| H | amino acid | NH₂ | NH-methyl |
| H | amino acid | NH₂ | NH-ethyl |
| H | amino acid | NH₂ | NH-acetyl |
| H | amino acid | NH₂ | OH |
| H | amino acid | NH₂ | OMe |
| H | amino acid | NH₂ | OEt |
| H | amino acid | NH₂ | O-cyclopropyl |
| H | amino acid | NH₂ | O-acetyl |
| H | amino acid | NH₂ | SH |
| H | amino acid | NH₂ | SMe |
| H | amino acid | NH₂ | SEt |
| H | amino acid | NH₂ | S-cyclopropyl |
| H | amino acid | NH₂ | F |
| H | amino acid | NH₂ | Cl |
| H | amino acid | NH₂ | Br |
| H | amino acid | NH₂ | I |
| amino acid | amino acid | NH₂ | H |
| amino acid | amino acid | NH₂ | NH₂ |
| amino acid | amino acid | NH₂ | NH-cyclopropyl |
| amino acid | amino acid | NH₂ | NH-methyl |
| amino acid | amino acid | NH₂ | NH-ethyl |
| amino acid | amino acid | NH₂ | NH-acetyl |
| amino acid | amino acid | NH₂ | OH |
| amino acid | amino acid | NH₂ | OMe |
| amino acid | amino acid | NH₂ | OEt |
| amino acid | amino acid | NH₂ | O-cyclopropyl |
| amino acid | amino acid | NH₂ | O-acetyl |
| amino acid | amino acid | NH₂ | SH |
| amino acid | amino acid | NH₂ | SMe |
| amino acid | amino acid | NH₂ | SEt |
| amino acid | amino acid | NH₂ | S-cyclopropyl |
| amino acid | amino acid | NH₂ | F |
| amino acid | amino acid | NH₂ | Cl |
| amino acid | amino acid | NH₂ | Br |
| amino acid | amino acid | NH₂ | I |
| amino acid | H | NH₂ | H |
| amino acid | H | NH₂ | NH₂ |
| amino acid | H | NH₂ | NH-cyclopropyl |
| amino acid | H | NH₂ | NH-methyl |
| amino acid | H | NH₂ | NH-ethyl |
| amino acid | H | NH₂ | NH-acetyl |
| amino acid | H | NH₂ | OH |
| amino acid | H | NH₂ | OMe |
| amino acid | H | NH₂ | OEt |
| amino acid | H | NH₂ | O-cyclopropyl |
| amino acid | H | NH₂ | O-acetyl |
| amino acid | H | NH₂ | SH |
| amino acid | H | NH₂ | SMe |
| amino acid | H | NH₂ | SEt |
| amino acid | H | NH₂ | S-cyclopropyl |
| amino acid | H | NH₂ | F |
| amino acid | H | NH₂ | Cl |
| amino acid | H | NH₂ | Br |
| amino acid | H | NH₂ | I |
| amino acid | acyl | NH₂ | H |
| amino acid | acyl | NH₂ | NH₂ |
| amino acid | acyl | NH₂ | NH-cyclopropyl |
| amino acid | acyl | NH₂ | NH-methyl |
| amino acid | acyl | NH₂ | NH-ethyl |
| amino acid | acyl | NH₂ | NH-acetyl |
| amino acid | acyl | NH₂ | OH |
| amino acid | acyl | NH₂ | OMe |
| amino acid | acyl | NH₂ | OEt |
| amino acid | acyl | NH₂ | O-cyclopropyl |
| amino acid | acyl | NH₂ | O-acetyl |
| amino acid | acyl | NH₂ | SH |
| amino acid | acyl | NH₂ | SMe |
| amino acid | acyl | NH₂ | SEt |
| amino acid | acyl | NH₂ | S-cyclopropyl |
| amino acid | acyl | NH₂ | F |
| amino acid | acyl | NH₂ | Cl |
| amino acid | acyl | NH₂ | Br |
| amino acid | acyl | NH₂ | I |
| acyl | H | OH | H |
| acyl | H | OH | NH₂ |
| acyl | H | OH | NH-cyclopropyl |
| acyl | H | OH | NH-methyl |
| acyl | H | OH | NH-ethyl |
| acyl | H | OH | NH-acetyl |
| acyl | H | OH | OH |
| acyl | H | OH | OMe |
| acyl | H | OH | OEt |
| acyl | H | OH | O-cyclopropyl |
| acyl | H | OH | O-acetyl |
| acyl | H | OH | SH |
| acyl | H | OH | SMe |
| acyl | H | OH | SEt |
| acyl | H | OH | S-cyclopropyl |
| acyl | H | OH | F |
| acyl | H | OH | Cl |
| acyl | H | OH | Br |
| acyl | H | OH | I |
| acyl | acyl | OH | H |
| acyl | acyl | OH | NH₂ |
| acyl | acyl | OH | NH-cyclopropyl |
| acyl | acyl | OH | NH-methyl |
| acyl | acyl | OH | NH-ethyl |
| acyl | acyl | OH | NH-acetyl |
| acyl | acyl | OH | OH |
| acyl | acyl | OH | OMe |
| acyl | acyl | OH | OEt |
| acyl | acyl | OH | O-cyclopropyl |
| acyl | acyl | OH | O-acetyl |
| acyl | acyl | OH | SH |
| acyl | acyl | OH | SMe |
| acyl | acyl | OH | SEt |
| acyl | acyl | OH | S-cyclopropyl |
| acyl | acyl | OH | F |
| acyl | acyl | OH | Cl |
| acyl | acyl | OH | Br |
| acyl | acyl | OH | I |
| acyl | amino acid | OH | H |
| acyl | amino acid | OH | NH₂ |
| acyl | amino acid | OH | NH-cyclopropyl |
| acyl | amino acid | OH | NH-methyl |
| acyl | amino acid | OH | NH-ethyl |
| acyl | amino acid | OH | NH-acetyl |
| acyl | amino acid | OH | OH |
| acyl | amino acid | OH | OMe |
| acyl | amino acid | OH | OEt |
| acyl | amino acid | OH | O-cyclopropyl |
| acyl | amino acid | OH | O-acetyl |
| acyl | amino acid | OH | SH |
| acyl | amino acid | OH | SMe |
| acyl | amino acid | OH | SEt |
| acyl | amino acid | OH | S-cyclopropyl |
| acyl | amino acid | OH | F |
| acyl | amino acid | OH | Cl |
| acyl | amino acid | OH | Br |
| acyl | amino acid | OH | I |
| H | acyl | OH | H |
| H | acyl | OH | NH₂ |
| H | acyl | OH | NH-cyclopropyl |
| H | acyl | OH | NH-methyl |
| H | acyl | OH | NH-ethyl |
| H | acyl | OH | NH-acetyl |
| H | acyl | OH | OH |
| H | acyl | OH | OMe |
| H | acyl | OH | OEt |
| H | acyl | OH | O-cyclopropyl |
| H | acyl | OH | O-acetyl |
| H | acyl | OH | SH |
| H | acyl | OH | SMe |
| H | acyl | OH | SEt |
| H | acyl | OH | S-cyclopropyl |
| H | acyl | OH | F |
| H | acyl | OH | Cl |
| H | acyl | OH | Br |
| H | acyl | OH | I |
| H | amino acid | OH | H |
| H | amino acid | OH | NH₂ |
| H | amino acid | OH | NH-cyclopropyl |
| H | amino acid | OH | NH-methyl |
| H | amino acid | OH | NH-ethyl |
| H | amino acid | OH | NH-acetyl |
| H | amino acid | OH | OH |
| H | amino acid | OH | OMe |
| H | amino acid | OH | OEt |
| H | amino acid | OH | O-cyclopropyl |
| H | amino acid | OH | O-acetyl |
| H | amino acid | OH | SH |
| H | amino acid | OH | SMe |
| H | amino acid | OH | SEt |
| H | amino acid | OH | S-cyclopropyl |
| H | amino acid | OH | F |
| H | amino acid | OH | Cl |
| H | amino acid | OH | Br |
| H | amino acid | OH | I |
| amino acid | amino acid | OH | H |
| amino acid | amino acid | OH | NH₂ |
| amino acid | amino acid | OH | NH-cyclopropyl |
| amino acid | amino acid | OH | NH-methyl |
| amino acid | amino acid | OH | NH-ethyl |
| amino acid | amino acid | OH | NH-acetyl |
| amino acid | amino acid | OH | OH |
| amino acid | amino acid | OH | OMe |
| amino acid | amino acid | OH | OEt |
| amino acid | amino acid | OH | O-cyclopropyl |
| amino acid | amino acid | OH | O-acetyl |
| amino acid | amino acid | OH | SH |
| amino acid | amino acid | OH | SMe |
| amino acid | amino acid | OH | SEt |
| amino acid | amino acid | OH | S-cyclopropyl |
| amino acid | amino acid | OH | F |
| amino acid | amino acid | OH | Cl |
| amino acid | amino acid | OH | Br |
| amino acid | amino acid | OH | I |
| amino acid | H | OH | H |
| amino acid | H | OH | NH₂ |
| amino acid | H | OH | NH-cyclopropyl |
| amino acid | H | OH | NH-methyl |
| amino acid | H | OH | NH-ethyl |
| amino acid | H | OH | NH-acetyl |
| amino acid | H | OH | OH |
| amino acid | H | OH | OMe |
| amino acid | H | OH | OEt |
| amino acid | H | OH | O-cyclopropyl |
| amino acid | H | OH | O-acetyl |
| amino acid | H | OH | SH |
| amino acid | H | OH | SMe |
| amino acid | H | OH | SEt |
| amino acid | H | OH | S-cyclopropyl |
| amino acid | H | OH | F |
| amino acid | H | OH | Cl |
| amino acid | H | OH | Br |
| amino acid | H | OH | I |
| amino acid | acyl | OH | H |
| amino acid | acyl | OH | NH₂ |
| amino acid | acyl | OH | NH-cyclopropyl |
| amino acid | acyl | OH | NH-methyl |
| amino acid | acyl | OH | NH-ethyl |
| amino acid | acyl | OH | NH-acetyl |
| amino acid | acyl | OH | OH |
| amino acid | acyl | OH | OMe |
| amino acid | acyl | OH | OEt |
| amino acid | acyl | OH | O-cyclopropyl |
| amino acid | acyl | OH | O-acetyl |
| amino acid | acyl | OH | SH |
| amino acid | acyl | OH | SMe |
| amino acid | acyl | OH | SEt |
| amino acid | acyl | OH | S-cyclopropyl |
| amino acid | acyl | OH | F |
| amino acid | acyl | OH | Cl |
| amino acid | acyl | OH | Br |
| amino acid | acyl | OH | I |
| acyl | H | F | H |
| acyl | H | F | NH₂ |
| acyl | H | F | NH-cyclopropyl |
| acyl | H | F | NH-methyl |
| acyl | H | F | NH-ethyl |
| acyl | H | F | NH-acetyl |
| acyl | H | F | OH |
| acyl | H | F | OMe |
| acyl | H | F | OEt |
| acyl | H | F | O-cyclopropyl |
| acyl | H | F | O-acetyl |
| acyl | H | F | SH |
| acyl | H | F | SMe |
| acyl | H | F | SEt |
| acyl | H | F | S-cyclopropyl |
| acyl | H | F | F |
| acyl | H | F | Cl |
| acyl | H | F | Br |
| acyl | H | F | I |
| acyl | acyl | F | H |
| acyl | acyl | F | NH₂ |
| acyl | acyl | F | NH-cyclopropyl |
| acyl | acyl | F | NH-methyl |
| acyl | acyl | F | NH-ethyl |
| acyl | acyl | F | NH-acetyl |
| acyl | acyl | F | OH |
| acyl | acyl | F | OMe |
| acyl | acyl | F | OEt |
| acyl | acyl | F | O-cyclopropyl |
| acyl | acyl | F | O-acetyl |
| acyl | acyl | F | SH |
| acyl | acyl | F | SMe |
| acyl | acyl | F | SEt |
| acyl | acyl | F | S-cyclopropyl |
| acyl | acyl | F | F |
| acyl | acyl | F | Cl |
| acyl | acyl | F | Br |
| acyl | acyl | F | I |
| acyl | amino acid | F | H |
| acyl | amino acid | F | NH₂ |
| acyl | amino acid | F | NH-cyclopropyl |
| acyl | amino acid | F | NH-methyl |
| acyl | amino acid | F | NH-ethyl |
| acyl | amino acid | F | NH-acetyl |
| acyl | amino acid | F | OH |
| acyl | amino acid | F | OMe |
| acyl | amino acid | F | OEt |
| acyl | amino acid | F | O-cyclopropyl |
| acyl | amino acid | F | O-acetyl |
| acyl | amino acid | F | SH |
| acyl | amino acid | F | SMe |
| acyl | amino acid | F | SEt |
| acyl | amino acid | F | S-cyclopropyl |
| acyl | amino acid | F | F |
| acyl | amino acid | F | Cl |
| acyl | amino acid | F | Br |
| acyl | amino acid | F | I |
| H | acyl | F | H |
| H | acyl | F | NH₂ |
| H | acyl | F | NH-cyclopropyl |
| H | acyl | F | NH-methyl |
| H | acyl | F | NH-ethyl |
| H | acyl | F | NH-acetyl |
| H | acyl | F | OH |
| H | acyl | F | OMe |
| H | acyl | F | OEt |
| H | acyl | F | O-cyclopropyl |
| H | acyl | F | O-acetyl |
| H | acyl | F | SH |
| H | acyl | F | SMe |
| H | acyl | F | SEt |
| H | acyl | F | S-cyclopropyl |
| H | acyl | F | F |
| H | acyl | F | Cl |
| H | acyl | F | Br |
| H | acyl | F | I |
| H | amino acid | F | H |
| H | amino acid | F | NH₂ |
| H | amino acid | F | NH-cyclopropyl |
| H | amino acid | F | NH-methyl |
| H | amino acid | F | NH-ethyl |
| H | amino acid | F | NH-acetyl |
| H | amino acid | F | OH |
| H | amino acid | F | OMe |
| H | amino acid | F | OEt |
| H | amino acid | F | O-cyclopropyl |
| H | amino acid | F | O-acetyl |
| H | amino acid | F | SH |
| H | amino acid | F | SMe |
| H | amino acid | F | SEt |
| H | amino acid | F | S-cyclopropyl |
| H | amino acid | F | F |
| H | amino acid | F | Cl |
| H | amino acid | F | Br |
| H | amino acid | F | I |
| amino acid | amino acid | F | H |
| amino acid | amino acid | F | NH₂ |
| amino acid | amino acid | F | NH-cyclopropyl |
| amino acid | amino acid | F | NH-methyl |
| amino acid | amino acid | F | NH-ethyl |
| amino acid | amino acid | F | NH-acetyl |
| amino acid | amino acid | F | OH |
| amino acid | amino acid | F | OMe |
| amino acid | amino acid | F | OEt |
| amino acid | amino acid | F | O-cyclopropyl |
| amino acid | amino acid | F | O-acetyl |
| amino acid | amino acid | F | SH |
| amino acid | amino acid | F | SMe |
| amino acid | amino acid | F | SEt |
| amino acid | amino acid | F | S-cyclopropyl |
| amino acid | amino acid | F | F |
| amino acid | amino acid | F | Cl |
| amino acid | amino acid | F | Br |
| amino acid | amino acid | F | I |
| amino acid | H | F | H |
| amino acid | H | F | NH₂ |
| amino acid | H | F | NH-cyclopropyl |
| amino acid | H | F | NH-methyl |
| amino acid | H | F | NH-ethyl |
| amino acid | H | F | NH-acetyl |
| amino acid | H | F | OH |
| amino acid | H | F | OMe |
| amino acid | H | F | OEt |
| amino acid | H | F | O-cyclopropyl |
| amino acid | H | F | O-acetyl |
| amino acid | H | F | SH |
| amino acid | H | F | SMe |
| amino acid | H | F | SEt |
| amino acid | H | F | S-cyclopropyl |
| amino acid | H | F | F |
| amino acid | H | F | Cl |
| amino acid | H | F | Br |
| amino acid | H | F | I |
| amino acid | acyl | F | H |
| amino acid | acyl | F | NH₂ |
| amino acid | acyl | F | NH-cyclopropyl |
| amino acid | acyl | F | NH-methyl |
| amino acid | acyl | F | NH-ethyl |
| amino acid | acyl | F | NH-acetyl |
| amino acid | acyl | F | OH |
| amino acid | acyl | F | OMe |
| amino acid | acyl | F | OEt |
| amino acid | acyl | F | O-cyclopropyl |
| amino acid | acyl | F | O-acetyl |
| amino acid | acyl | F | SH |
| amino acid | acyl | F | SMe |
| amino acid | acyl | F | SEt |
| amino acid | acyl | F | S-cyclopropyl |
| amino acid | acyl | F | F |
| amino acid | acyl | F | Cl |
| amino acid | acyl | F | Br |
| amino acid | acyl | F | I |
| acyl | H | I | H |
| acyl | H | I | NH₂ |
| acyl | H | I | NH-cyclopropyl |
| acyl | H | I | NH-methyl |
| acyl | H | I | NH-ethyl |
| acyl | H | I | NH-acetyl |
| acyl | H | I | OH |
| acyl | H | I | OMe |
| acyl | H | I | OEt |
| acyl | H | I | O-cyclopropyl |
| acyl | H | I | O-acetyl |
| acyl | H | I | SH |
| acyl | H | I | SMe |
| acyl | H | I | SEt |
| acyl | H | I | S-cyclopropyl |
| acyl | H | I | F |
| acyl | H | I | Cl |
| acyl | H | I | Br |
| acyl | H | I | I |
| acyl | acyl | I | H |
| acyl | acyl | I | NH₂ |
| acyl | acyl | I | NH-cyclopropyl |
| acyl | acyl | I | NH-methyl |
| acyl | acyl | I | NH-ethyl |
| acyl | acyl | I | NH-acetyl |
| acyl | acyl | I | OH |
| acyl | acyl | I | OMe |
| acyl | acyl | I | OEt |
| acyl | acyl | I | O-cyclopropyl |
| acyl | acyl | I | O-acetyl |
| acyl | acyl | I | SH |
| acyl | acyl | I | SMe |
| acyl | acyl | I | SEt |
| acyl | acyl | I | S-cyclopropyl |
| acyl | acyl | I | F |
| acyl | acyl | I | Cl |
| acyl | acyl | I | Br |
| acyl | acyl | I | I |
| acyl | amino acid | I | H |
| acyl | amino acid | I | NH₂ |
| acyl | amino acid | I | NH-cyclopropyl |
| acyl | amino acid | I | NH-methyl |
| acyl | amino acid | I | NH-ethyl |
| acyl | amino acid | I | NH-acetyl |
| acyl | amino acid | I | OH |
| acyl | amino acid | I | OMe |
| acyl | amino acid | I | OEt |
| acyl | amino acid | I | O-cyclopropyl |
| acyl | amino acid | I | O-acetyl |
| acyl | amino acid | I | SH |
| acyl | amino acid | I | SMe |
| acyl | amino acid | I | SEt |
| acyl | amino acid | I | S-cyclopropyl |
| acyl | amino acid | I | F |
| acyl | amino acid | I | Cl |
| acyl | amino acid | I | Br |
| acyl | amino acid | I | I |
| H | acyl | I | H |
| H | acyl | I | NH₂ |
| H | acyl | I | NH-cyclopropyl |
| H | acyl | I | NH-methyl |
| H | acyl | I | NH-ethyl |
| H | acyl | I | NH-acetyl |
| H | acyl | I | OH |
| H | acyl | I | OMe |
| H | acyl | I | OEt |
| H | acyl | I | O-cyclopropyl |
| H | acyl | I | O-acetyl |
| H | acyl | I | SH |
| H | acyl | I | SMe |
| H | acyl | I | SEt |
| H | acyl | I | S-cyclopropyl |
| H | acyl | I | F |
| H | acyl | I | Cl |
| H | acyl | I | Br |
| H | acyl | I | I |
| H | amino acid | I | H |
| H | amino acid | I | NH₂ |
| H | amino acid | I | NH-cyclopropyl |
| H | amino acid | I | NH-methyl |
| H | amino acid | I | NH-ethyl |
| H | amino acid | I | NH-acetyl |
| H | amino acid | I | OH |
| H | amino acid | I | OMe |
| H | amino acid | I | OEt |
| H | amino acid | I | O-cyclopropyl |
| H | amino acid | I | O-acetyl |
| H | amino acid | I | SH |
| H | amino acid | I | SMe |
| H | amino acid | I | SEt |
| H | amino acid | I | S-cyclopropyl |
| H | amino acid | I | F |
| H | amino acid | I | Cl |
| H | amino acid | I | Br |
| H | amino acid | I | I |
| amino acid | amino acid | I | H |
| amino acid | amino acid | I | NH₂ |
| amino acid | amino acid | I | NH-cyclopropyl |
| amino acid | amino acid | I | NH-methyl |
| amino acid | amino acid | I | NH-ethyl |
| amino acid | amino acid | I | NH-acetyl |
| amino acid | amino acid | I | OH |
| amino acid | amino acid | I | OMe |
| amino acid | amino acid | I | OEt |
| amino acid | amino acid | I | O-cyclopropyl |
| amino acid | amino acid | I | O-acetyl |
| amino acid | amino acid | I | SH |
| amino acid | amino acid | I | SMe |
| amino acid | amino acid | I | SEt |
| amino acid | amino acid | I | S-cyclopropyl |
| amino acid | amino acid | I | F |
| amino acid | amino acid | I | Cl |
| amino acid | amino acid | I | Br |
| amino acid | amino acid | I | I |
| amino acid | H | I | H |
| amino acid | H | I | NH₂ |
| amino acid | H | I | NH-cyclopropyl |
| amino acid | H | I | NH-methyl |
| amino acid | H | I | NH-ethyl |
| amino acid | H | I | NH-acetyl |
| amino acid | H | I | OH |
| amino acid | H | I | OMe |
| amino acid | H | I | OEt |
| amino acid | H | I | O-cyclopropyl |
| amino acid | H | I | O-acetyl |
| amino acid | H | I | SH |
| amino acid | H | I | SMe |
| amino acid | H | I | SEt |
| amino acid | H | I | S-cyclopropyl |
| amino acid | H | I | F |
| amino acid | H | I | Cl |
| amino acid | H | I | Br |
| amino acid | H | I | I |
| amino acid | acyl | I | H |
| amino acid | acyl | I | NH₂ |
| amino acid | acyl | I | NH-cyclopropyl |
| amino acid | acyl | I | NH-methyl |
| amino acid | acyl | I | NH-ethyl |
| amino acid | acyl | I | NH-acetyl |
| amino acid | acyl | I | OH |
| amino acid | acyl | I | OMe |
| amino acid | acyl | I | OEt |
| amino acid | acyl | I | O-cyclopropyl |
| amino acid | acyl | I | O-acetyl |
| amino acid | acyl | I | SH |
| amino acid | acyl | I | SMe |
| amino acid | acyl | I | SEt |
| amino acid | acyl | I | S-cyclopropyl |
| amino acid | acyl | I | F |
| amino acid | acyl | I | Cl |
| amino acid | acyl | I | Br |
| amino acid | acyl | I | I |

**Table 9**

| Insofar as the compounds in this table are encompassed by the compounds referred to in the enclosed claims, the present invention relates to such compounds. | | | | |
|---|---|---|---|---|
| **R²** | **R³** | **R⁶** | **X** | **Base** |
| acyl | H | CH₃ | O | Thymine |
| acyl | H | CH₃ | O | Uracil |
| acyl | H | CH₃ | O | Guanine |
| acyl | H | CH₃ | O | Cytosine |
| acyl | H | CH₃ | O | Adenine |
| acyl | H | CH₃ | O | Hypoxanthine |
| acyl | H | CH₃ | O | 5-Fluorouracil |
| acyl | H | CH₃ | O | 8-Fluoroguanine |
| acyl | H | CH₃ | O | 5-Fluorocytosine |
| acyl | H | CH₃ | O | 8-Fluoroadenine |
| acyl | H | CH₃ | O | 2-Fluoroadenine |
| acyl | H | CH₃ | O | 2,8-Difluoroadenine |
| acyl | H | CH₃ | O | 2-Fluorohypoxanthine |
| acyl | H | CH₃ | O | 8-Fluorohypoxanthine |
| acyl | H | CH₃ | O | 2,8-Difluorohypoxanthine |
| acyl | H | CH₃ | O | 2-Aminoadenine |
| acyl | H | CH₃ | O | 2-Amino-8-fluoroadenine |
| acyl | H | CH₃ | O | 2-Amino-8-fluorohypoxanthine |
| acyl | H | CH₃ | O | 2-Aminohypoxanthine |
| acyl | H | CH₃ | O | 2 N-acetylguanine |
| acyl | H | CH₃ | O | 4-N-acetylcytosine |
| acyl | H | CH₃ | O | 6-N-acetyladenine |
| acyl | H | CH₃ | O | 2-N-acetyl-8-fluoroguanine |
| acyl | H | CH₃ | O | 4-N-acetyl-5-fluorocytosine |
| acyl | H | CH₃ | O | 6-N-acetyl-2-fluoroadenine |
| acyl | H | CH₃ | O | 6-N-acetyl-2,8-difluoroadenine |
| acyl | H | CH₃ | O | 6-N-acetyl-2-aminoadenine |
| acyl | H | CH₃ | O | 6-N-acetyl-2-amino-8-fluoroadenine |
| acyl | H | CH₃ | O | 2-N-acetylaminoadenine |
| acyl | H | CH₃ | O | 2-N-acetylamino-8-fluoroadenine |
| acyl | H | CH₃ | O | 2-N-acetylaraino-8-fluorohypoxanthine |
| acyl | H | CH₃ | O | 2-N-acetylaminohypoxanthine |
| acyl | acyl | CH₃ | O | Thymine |
| acyl | acyl | CH₃ | O | Uracil |
| acyl | acyl | CH₃ | O | Guanine |
| acyl | acyl | CH₃ | O | Cytosine |
| acyl | acyl | CH₃ | O | Adenine |
| acyl | acyl | CH₃ | O | Hypoxanthine |
| acyl | acyl | CH₃ | O | 5-Fluorouracil |
| acyl | acyl | CH₃ | O | 8-Fluoroguanine |
| acyl | acyl | CH₃ | O | 5-Fluorocytosine |
| acyl | acyl | CH₃ | O | 8-Fluoroadenine |
| acyl | acyl | CH₃ | O | 2-Fluoroadenine |
| acyl | acyl | CH₃ | O | 2,8-Difluoroadenine |
| acyl | acyl | CH₃ | O | 2-Fluorohypoxanthine |
| acyl | acyl | CH₃ | O | 8-Fluorohypoxanthine |
| acyl | acyl | CH₃ | O | 2,8-Difluorohypoxanthine |
| acyl | acyl | CH₃ | O | 2-Aminoadenine |
| acyl | acyl | CH₃ | O | 2-Amino-8-fluoroadenine |
| acyl | acyl | CH₃ | O | 2-Amino-8-fluorohypoxanthine |
| acyl | acyl | CH₃ | O | 2-Aminohypoxanthine |
| acyl | acyl | CH₃ | O | 2-N-acetylguanine |
| acyl | acyl | CH₃ | O | 4-N-acetylcytosine |
| acyl | acyl | CH₃ | O | 6-N-acetyladenine |
| acyl | acyl | CH₃ | O | 2-N-acetyl-8-fluoroguanine |
| acyl | acyl | CH₃ | O | 4-N-acetyl-5-fluorocytosine |
| acyl | acyl | CH₃ | O | 6-N-acetyl-2-fluoroadenine |
| acyl | acyl | CH₃ | O | 6-N-acetyl-2,8-difluoroadenine |
| acyl | acyl | CH₃ | O | 6-N-acetyl-2-aminoadenine |
| acyl | acyl | CH₃ | O | 6-N-acetyl-2-amino-8-fluoroadenine |
| acyl | acyl | CH₃ | O | 2-N-acetylaminoadenine |
| acyl | acyl | CH₃ | O | 2-N-acetylamino-8-fluoroadenine |
| acyl | acyl | CH₃ | O | 2-N-acetylamino-8-fluorohypoxanthine |
| acyl | acyl | CH₃ | O | 2-N-acetylaminohypoxanthine |
| acyl | amino acid | CH₃ | O | Thymine |
| acyl | amino acid | CH₃ | O | Uracil |
| acyl | amino acid | CH₃ | O | Guanine |
| acyl | amino acid | CH₃ | O | Cytosine |
| acyl | amino acid | CH₃ | O | Adenine |
| acyl | amino acid | CH₃ | O | Hypoxanthine |
| acyl | amino acid | CH₃ | O | 5-Fluorouracil |
| acyl | amino acid | CH₃ | O | 8-Fluoroguanine |
| acyl | amino acid | CH₃ | O | 5-Fluorocytosine |
| acyl | amino acid | CH₃ | O | 8-Fluoroadenine |
| acyl | amino acid | CH₃ | O | 2-Fluoroadenine |
| acyl | amino acid | CH₃ | O | 2,8-Difluoroadenine |
| acyl | amino acid | CH₃ | O | 2-Fluorohypoxanthine |
| acyl | amino acid | CH₃ | O | 8-Fluorohypoxanthine |
| acyl | amino acid | CH₃ | O | 2,8-Difluorohypoxanthine |
| acyl | amino acid | CH₃ | O | 2-Aminoadenine |
| acyl | amino acid | CH₃ | O | 2-Amino-8-fluoroadenine |
| acyl | amino acid | CH₃ | O | 2-Amino-8-fluorohypoxanthine |
| acyl | amino acid | CH₃ | O | 2-Aminohypoxanthine |
| acyl | amino acid | CH₃ | O | 2-N-acetylguanine |
| acyl | amino acid | CH₃ | O | 4-N-acetylcytosine |
| acyl | amino acid | CH₃ | O | 6-N-acetyladenine |
| acyl | amino acid | CH₃ | O | 2-N-acetyl-8-fluoroguanine |
| acyl | amino acid | CH₃ | O | 4-N-acetyl-5-fluorocytosine |
| acyl | amino acid | CH₃ | O | 6-N-acetyl-2-fluoroadenine |
| acyl | amino acid | CH₃ | O | 6-N-acetyl-2,8-difluoroadenine |
| acyl | amino acid | CH₃ | O | 6-N-acetyl-2-aminoadenine |
| acyl | amino acid | CH₃ | O | 6-N-acetyl-2-amino-8-fluoroadenine |
| acyl | amino acid | CH₃ | O | 2-N-acetylaminoadenine |
| acyl | amino acid | CH₃ | O | 2-N-acetylamino-8-fluoroadenine |
| acyl | amino acid | CH₃ | O | 2-N-acetylamino-8-fluorohypoxanthine |
| acyl | amino acid | CH₃ | O | 2-N-acetylaminohypoxanthine |
| H | acyl | CH₃ | O | Thymine |
| H | acyl | CH₃ | O | Uracil |
| H | acyl | CH₃ | O | Guanine |
| H | acyl | CH₃ | O | Cytosine |
| H | acyl | CH₃ | O | Adenine |
| H | acyl | CH₃ | O | Hypoxanthine |
| H | acyl | CH₃ | O | 5-Fluorouracil |
| H | acyl | CH₃ | O | 8-Fluoroguanine |
| H | acyl | CH₃ | O | 5-Fluorocytosine |
| H | acyl | CH₃ | O | 8-Fluoroadenine |
| H | acyl | CH₃ | O | 2-Fluoroadenine |
| H | acyl | CH₃ | O | 2,8-Difluoroadenine |
| H | acyl | CH₃ | O | 2-Fluorohypoxanthine |
| H | acyl | CH₃ | O | 8-Fluorohypoxanthine |
| H | acyl | CH₃ | O | 2,8-Difluorohypoxanthine |
| H | acyl | CH₃ | O | 2-Aminoadenine |
| H | acyl | CH₃ | O | 2-Amino-8-fluoroadenine |
| H | acyl | CH₃ | O | 2-Amino-8-fluorohypoxanthine |
| H | acyl | CH₃ | O | 2-Aminohypoxanthine |
| H | acyl | CH₃ | O | 2-N-acetylguanine |
| H | acyl | CH₃ | O | 4-N-acetylcytosine |
| H | acyl | CH₃ | O | 6-N-acetyladenine |
| H | acyl | CH₃ | O | 2-N-acetyl-8-fluoroguanine |
| H | acyl | CH₃ | O | 4-N-acetyl-5-fluorocytosine |
| H | acyl | CH₃ | O | 6-N-acetyl-2-fluoroadenine |
| H | acyl | CH₃ | O | 6-N-acetyl-2,8-difluoroadenine |
| H | acyl | CH₃ | O | 6-N-acetyl-2-aminoadenine |
| H | acyl | CH₃ | O | 6-N-acetyl-2-amino-8-fluoroadenine |
| H | acyl | CH₃ | O | 2-N-acetylaminoadenine |
| H | acyl | CH₃ | O | 2-N-acetylamino-8-fluoroadenine |
| H | acyl | CH₃ | O | 2-N-acetylamino-8-fluorohypoxanthine |
| H | acyl | CH₃ | O | 2-N-acetylaminohypoxanthine |
| H | amino acid | CH₃ | O | Thymine |
| H | amino acid | CH₃ | O | Uracil |
| H | amino acid | CH₃ | O | Guanine |
| H | amino acid | CH₃ | O | Cytosine |
| H | amino acid | CH₃ | O | Adenine |
| H | amino acid | CH₃ | O | Hypoxanthine |
| H | amino acid | CH₃ | O | 5-Fluorouracil |
| H | amino acid | CH₃ | O | 8-Fluoroguanine |
| H | amino acid | CH₃ | O | 5-Fluorocytosine |
| H | amino acid | CH₃ | O | 8-Fluoroadenine |
| H | amino acid | CH₃ | O | 2-Fluoroadenine |
| H | amino acid | CH₃ | O | 2,8-Difluoroadenine |
| H | amino acid | CH₃ | O | 2-Fluorohypoxanthine |
| H | amino acid | CH₃ | O | 8-Fluorohypoxanthine |
| H | amino acid | CH₃ | O | 2,8-Difluorohypoxanthine |
| H | amino acid | CH₃ | O | 2-Aminoadenine |
| H | amino acid | CH₃ | O | 2-Amino-8-fluoroadenine |
| H | amino acid | CH₃ | O | 2-Amino-8-fluorohypoxanthine |
| H | amino acid | CH₃ | O | 2-Aminohypoxanthine |
| H | amino acid | CH₃ | O | 2-N-acetylguanine |
| H | amino acid | CH₃ | O | 4-N-acetylcytosine |
| H | amino acid | CH₃ | O | 6-N-acetyladenine |
| H | amino acid | CH₃ | O | 2-N-acetyl-8-fluoroguanine |
| H | amino acid | CH₃ | O | 4-N-acetyl-5-fluorocytosine |
| H | amino acid | CH₃ | O | 6-N-acetyl-2-fluoroadenine |
| H | amino acid | CH₃ | O | 6-N-acetyl-2,8-difluoroadenine |
| H | amino acid | CH₃ | O | 6-N-acetyl-2-aminoadenine |
| H | amino acid | CH₃ | O | 6-N-acetyl-2-amino-8-fluoroadenine |
| H | amino acid | CH₃ | O | 2-N-acetylaminoadenine |
| H | amino acid | CH₃ | O | 2-N-acetylamino-8-fluoroadenine |
| H | amino acid | CH₃ | O | 2-N-acetylamino-8-fluorohypoxanthine |
| H | amino acid | CH₃ | O | 2-N-acetylaminohypoxanthine |
| amino acid | amino acid | CH₃ | O | Thymine |
| amino acid | amino acid | CH₃ | O | Uracil |
| amino acid | amino acid | CH₃ | O | Guanine |
| amino acid | amino acid | CH₃ | O | Cytosine |
| amino acid | amino acid | CH₃ | O | Adenine |
| amino acid | amino acid | CH₃ | O | Hypoxanthine |
| amino acid | amino acid | CH₃ | O | 5-Fluorouracil |
| amino acid | amino acid | CH₃ | O | 8-Fluoroguanine |
| amino acid | amino acid | CH₃ | O | 5-Fluorocytosine |
| amino acid | amino acid | CH₃ | O | 8-Fluoroadenine |
| amino acid | amino acid | CH₃ | O | 2-Fluoroadenine |
| amino acid | amino acid | CH₃ | O | 2,8-Difluoroadenine |
| amino acid | amino acid | CH₃ | O | 2-Fluorohypoxanthine |
| amino acid | amino acid | CH₃ | O | 8-Fluorohypoxanthine |
| amino acid | amino acid | CH₃ | O | 2,8-Difluorohypoxanthine |
| amino acid | amino acid | CH₃ | O | 2-Aminoadenine |
| amino acid | amino acid | CH₃ | O | 2-Amino-8-fluoroadenine |
| amino acid | amino acid | CH₃ | O | 2-Amino-8-fluorohypoxanthine |
| amino acid | amino acid | CH₃ | O | 2-Aminohypoxanthine |
| amino acid | amino acid | CH₃ | O | 2-N-acetylguanine |
| amino acid | amino acid | CH₃ | O | 4-N-acetylcytosine |
| amino acid | amino acid | CH₃ | O | 6-N-acetyladenine |
| amino acid | amino acid | CH₃ | O | 2-N-acetyl-8-fluoroguanine |
| amino acid | amino acid | CH₃ | O | 4-N-acetyl-5-fluorocytosine |
| amino acid | amino acid | CH₃ | O | 6-N-acetyl-2-fluoroadenine |
| amino acid | amino acid | CH₃ | O | 6-N-acetyl-2,8-difluoroadenine |
| amino acid | amino acid | CH₃ | O | 6-N-acetyl-2-aminoadenine |
| amino acid | amino acid | CH₃ | O | 6-N-acetyl-2-amino-8-fluoroadenine |
| amino acid | amino acid | CH₃ | O | 2-N-acetylaminoadenine |
| amino acid | amino acid | CH₃ | O | 2-N-acetylamino-8-fluoroadenine |
| amino acid | amino acid | CH₃ | O | 2-N-acetylamino-8-fluorohypoxanthine |
| amino acid | amino acid | CH₃ | O | 2-N-acetylaminohypoxanthine |
| amino acid | H | CH₃ | O | Thymine |
| amino acid | H | CH₃ | O | Uracil |
| amino acid | H | CH₃ | O | Guanine |
| amino acid | H | CH₃ | O | Cytosine |
| amino acid | H | CH₃ | O | Adenine |
| amino acid | H | CH₃ | O | Hypoxanthine |
| amino acid | H | CH₃ | O | 5-Fluorouracil |
| amino acid | H | CH₃ | O | 8-Fluoroguanine |
| amino acid | H | CH₃ | O | 5-Fluorocytosine |
| amino acid | H | CH₃ | O | 8-Fluoroadenine |
| amino acid | H | CH₃ | O | 2-Fluoroadenine |
| amino acid | H | CH₃ | O | 2,8-Difluoroadenine |
| amino acid | H | CH₃ | O | 2-Fluorohypoxanthine |
| amino acid | H | CH₃ | O | 8-Fluorohypoxanthine |
| amino acid | H | CH₃ | O | 2,8-Difluorohypoxanthine |
| amino acid | H | CH₃ | O | 2-Aminoadenine |
| amino acid | H | CH₃ | O | 2-Amino-8-fluoroadenine |
| amino acid | H | CH₃ | O | 2-Amino-8-fluorohypoxanthine |
| amino acid | H | CH₃ | O | 2-Aminohypoxanthine |
| amino acid | H | CH₃ | O | 2-N-acetylguanine |
| amino acid | H | CH₃ | O | 4-N-acetylcytosine |
| amino acid | H | CH₃ | O | 6-N-acetyladenine |
| amino acid | H | CH₃ | O | 2-N-acetyl-8-fluoroguanine |
| amino acid | H | CH₃ | O | 4-N-acetyl-5-fluorocytosine |
| amino acid | H | CH₃ | O | 6-N-acetyl-2-fluoroadenine |
| amino acid | H | CH₃ | O | 6-N-acetyl-2,8-difluoroadenine |
| amino acid | H | CH₃ | O | 6-N-acetyl-2-aminoadenine |
| amino acid | H | CH₃ | O | 6-N-acetyl-2-amino-8-fluoroadenine |
| amino acid | H | CH₃ | O | 2-N-acetylaminoadenine |
| amino acid | H | CH₃ | O | 2-N-acetylamino-8-fluoroadenine |
| amino acid | H | CH₃ | O | 2-N-acetylamino-8-fluorohypoxanthine |
| amino acid | H | CH₃ | O | 2-N-acetylaminohypoxanthine |
| amino acid | acyl | CH₃ | O | Thymine |
| amino acid | acyl | CH₃ | O | Uracil |
| amino acid | acyl | CH₃ | O | Guanine |
| amino acid | acyl | CH₃ | O | Cytosine |
| amino acid | acyl | CH₃ | O | Adenine |
| amino acid | acyl | CH₃ | O | Hypoxanthine |
| amino acid | acyl | CH₃ | O | 5-Fluorouracil |
| amino acid | acyl | CH₃ | O | 8-Fluoroguanine |
| amino acid | acyl | CH₃ | O | 5-Fluorocytosine |
| amino acid | acyl | CH₃ | O | 8-Fluoroadenine |
| amino acid | acyl | CH₃ | O | 2-Fluoroadenine |
| amino acid | acyl | CH₃ | O | 2,8-Difluoroadenine |
| amino acid | acyl | CH₃ | O | 2-Fluorohypoxanthine |
| amino acid | acyl | CH₃ | O | 8-Fluorohypoxanthine |
| amino acid | acyl | CH₃ | O | 2,8-Difluorohypoxanthine |
| amino acid | acyl | CH₃ | O | 2-Aminoadenine |
| amino acid | acyl | CH₃ | O | 2-Amino-8-fluoroadenine |
| amino acid | acyl | CH₃ | O | 2-Amino-8-fluorohypoxanthine |
| amino acid | acyl | CH₃ | O | 2-Aminohypoxanthine |
| amino acid | acyl | CH₃ | O | 2-N-acetylguanine |
| amino acid | acyl | CH₃ | O | 4-N-acetylcytosine |
| amino acid | acyl | CH₃ | O | 6-N-acetyladenine |
| amino acid | acyl | CH₃ | O | 2-N-acetyl-8-fluoroguanine |
| amino acid | acyl | CH₃ | O | 4-N-acetyl-5-fluorocytosine |
| amino acid | acyl | CH₃ | O | 6-N-acetyl-2-fluoroadenine |
| amino acid | acyl | CH₃ | O | 6-N-acetyl-2,8-difluoroadenine |
| amino acid | acyl | CH₃ | O | 6-N-acetyl-2-aminoadenine |
| amino acid | acyl | CH₃ | O | 6-N-acetyl-2-amino-8-fluoroadenine |
| amino acid | acyl | CH₃ | O | 2-N-acetylaminoadenine |
| amino acid | acyl | CH₃ | O | 2-N-acetylamino-8-fluoroadenine |
| amino acid | acyl | CH₃ | O | 2-N-acetylamino-8-fluorohypoxanthine |
| amino acid | acyl | CH₃ | O | 2-N-acetylaminohypoxanthine |
| acyl | H | CH₃ | S | Thymine |
| acyl | H | CH₃ | S | Uracil |
| acyl | H | CH₃ | S | Guanine |
| acyl | H | CH₃ | S | Cytosine |
| acyl | H | CH₃ | S | Adenine |
| acyl | H | CH₃ | S | Hypoxanthine |
| acyl | H | CH₃ | S | 5-Fluorouracil |
| acyl | H | CH₃ | S | 8-Fluoroguanine |
| acyl | H | CH₃ | S | 5-Fluorocytosine |
| acyl | H | CH₃ | S | 8-Fluoroadenine |
| acyl | H | CH₃ | S | 2-Fluoroadenine |
| acyl | H | CH₃ | S | 2,8-Difluoroadenine |
| acyl | H | CH₃ | S | 2-Fluorohypoxanthine |
| acyl | H | CH₃ | S | 8-Fluorohypoxanthine |
| acyl | H | CH₃ | S | 2,8-Difluorohypoxanthine |
| acyl | H | CH₃ | S | 2-Aminoadenine |
| acyl | H | CH₃ | S | 2-Amino-8-fluoroadenine |
| acyl | H | CH₃ | S | 2-Amino-8-fluorohypoxanthine |
| acyl | H | CH₃ | S | 2-Aminohypoxanthine |
| acyl | H | CH₃ | S | 2-N-acetylguanine |
| acyl | H | CH₃ | S | 4-N-acetylcytosine |
| acyl | H | CH₃ | S | 6-N-acetyladenine |
| acyl | H | CH₃ | S | 2-N-acetyl-8-fluoroguanine |
| acyl | H | CH₃ | S | 4-N-acetyl-5-fluorocytosine |
| acyl | H | CH₃ | S | 6-N-acetyl-2-fluoroadenine |
| acyl | H | CH₃ | S | 6-N-acetyl-2,8-difluoroadenine |
| acyl | H | CH₃ | S | 6-N-acetyl-2-aminoadenine |
| acyl | H | CH₃ | S | 6-N-acetyl-2-amino-8-fluoroadenine |
| acyl | H | CH₃ | S | 2-N-acetylaminoadenine |
| acyl | H | CH₃ | S | 2-N-acetylamino-8-fluoroadenine |
| acyl | H | CH₃ | S | 2-N-acetylamino-8-fluorohypoxanthine |
| acyl | H | CH₃ | S | 2-N-acetylaminohypoxanthine |
| acyl | acyl | CH₃ | S | Thymine |
| acyl | acyl | CH₃ | S | Uracil |
| acyl | acyl | CH₃ | S | Guanine |
| acyl | acyl | CH₃ | S | Cytosine |
| acyl | acyl | CH₃ | S | Adenine |
| acyl | acyl | CH₃ | S | Hypoxanthine |
| acyl | acyl | CH₃ | S | 5-Fluorouracil |
| acyl | acyl | CH₃ | S | 8-Fluoroguanine |
| acyl | acyl | CH₃ | S | 5-Fluorocytosine |
| acyl | acyl | CH₃ | S | 8-Fluoroadenine |
| acyl | acyl | CH₃ | S | 2-Fluoroadenine |
| acyl | acyl | CH₃ | S | 2,8-Difluoroadenine |
| acyl | acyl | CH₃ | S | 2-Fluorohypoxanthine |
| acyl | acyl | CH₃ | S | 8-Fluorohypoxanthine |
| acyl | acyl | CH₃ | S | 2,8-Difluorohypoxanthine |
| acyl | acyl | CH₃ | S | 2-Aminoadenine |
| acyl | acyl | CH₃ | S | 2-Amino-8-fluoroadenine |
| acyl | acyl | CH₃ | S | 2-Amino-8-fluorohypoxanthine |
| acyl | acyl | CH₃ | S | 2-Aminohypoxanthine |
| acyl | acyl | CH₃ | S | 2-N-acetylguanine |
| acyl | acyl | CH₃ | S | 4-N-acetylcytosine |
| acyl | acyl | CH₃ | S | 6-N-acetyladenine |
| acyl | acyl | CH₃ | S | 2-N-acetyl-8-fluoroguanine |
| acyl | acyl | CH₃ | S | 4-N-acetyl-5-fluorocytosine |
| acyl | acyl | CH₃ | S | 6-N-acetyl-2-fluoroadenine |
| acyl | acyl | CH₃ | S | 6-N-acetyl-2,8-difluoroadenine |
| acyl | acyl | CH₃ | S | 6-N-acetyl-2-aminoadenine |
| acyl | acyl | CH₃ | S | 6-N-acetyl-2-amino-8-fluoroadenine |
| acyl | acyl | CH₃ | S | 2-N-acetylaminoadenine |
| acyl | acyl | CH₃ | S | 2-N-acetylamino-8-fluoroadenine |
| acyl | acyl | CH₃ | S | 2-N-acetylamino-8-fluorohypoxanthine |
| acyl | acyl | CH₃ | S | 2-N-acetylaminohypoxanthine |
| acyl | amino acid | CH₃ | S | Thymine |
| acyl | amino acid | CH₃ | S | Uracil |
| acyl | amino acid | CH₃ | S | Guanine |
| acyl | amino acid | CH₃ | S | Cytosine |
| acyl | amino acid | CH₃ | S | Adenine |
| acyl | amino acid | CH₃ | S | Hypoxanthine |
| acyl | amino acid | CH₃ | S | 5-Fluorouracil |
| acyl | amino acid | CH₃ | S | 8-Fluoroguanine |
| acyl | amino acid | CH₃ | S | 5-Fluorocytosine |
| acyl | amino acid | CH₃ | S | 8-Fluoroadenine |
| acyl | amino acid | CH₃ | S | 2-Fluoroadenine |
| acyl | amino acid | CH₃ | S | 2,8-Difluoroadenine |
| acyl | amino acid | CH₃ | S | 2-Fluorohypoxanthine |
| acyl | amino acid | CH₃ | S | 8-Fluorohypoxanthine |
| acyl | amino acid | CH₃ | S | 2,8-Difluorohypoxanthine |
| acyl | amino acid | CH₃ | S | 2-Aminoadenine |
| acyl | amino acid | CH₃ | S | 2-Amino-8-fluoroadenine |
| acyl | amino acid | CH₃ | S | 2-Amino-8-fluorohypoxanthine |
| acyl | amino acid | CH₃ | S | 2-Aminohypoxanthine |
| acyl | amino acid | CH₃ | S | 2-N-acetylguanine |
| acyl | amino acid | CH₃ | S | 4-N-acetylcytosine |
| acyl | amino acid | CH₃ | S | 6-N-acetyladenine |
| acyl | amino acid | CH₃ | S | 2-N-acetyl-8-fluoroguanine |
| acyl | amino acid | CH₃ | S | 4-N-acetyl-5-fluorocytosine |
| acyl | amino acid | CH₃ | S | 6-N-acetyl-2-fluoroadenine |
| acyl | amino acid | CH₃ | S | 6-N-acetyl-2,8-difluoroadenine |
| acyl | amino acid | CH₃ | S | 6-N-acetyl-2-aminoadenine |
| acyl | amino acid | CH₃ | S | 6-N-acetyl-2-amino-8-fluoroadenine |
| acyl | amino acid | CH₃ | S | 2-N-acetylaminoadenine |
| acyl | amino acid | CH₃ | S | 2-N-acetylamino-8-fluoroadenine |
| acyl | amino acid | CH₃ | S | 2-N-acetylamino-8-fluorohypoxanthine |
| acyl | amino acid | CH₃ | S | 2-N-acetylaminohypoxanthine |
| H | acyl | CH₃ | S | Thymine |
| H | acyl | CH₃ | S | Uracil |
| H | acyl | CH₃ | S | Guanine |
| H | acyl | CH₃ | S | Cytosine |
| H | acyl | CH₃ | S | Adenine |
| H | acyl | CH₃ | S | Hypoxanthine |
| H | acyl | CH₃ | S | 5-Fluorouracil |
| H | acyl | CH₃ | S | 8-Fluoroguanine |
| H | acyl | CH₃ | S | 5-Fluorocytosine |
| H | acyl | CH₃ | S | 8-Fluoroadenine |
| H | acyl | CH₃ | S | 2-Fluoroadenine |
| H | acyl | CH₃ | S | 2,8-Difluoroadenine |
| H | acyl | CH₃ | S | 2-Fluorohypoxanthine |
| H | acyl | CH₃ | S | 8-Fluorohypoxanthine |
| H | acyl | CH₃ | S | 2,8-Difluorohypoxanthine |
| H | acyl | CH₃ | S | 2-Aminoadenine |
| H | acyl | CH₃ | S | 2-Amino-8-fluoroadenine |
| H | acyl | CH₃ | S | 2-Amino-8-fluorohypoxanthine |
| H | acyl | CH₃ | S | 2-Aminohypoxanthine |
| H | acyl | CH₃ | S | 2-N-acetylguanine |
| H | acyl | CH₃ | S | 4-N-acetylcytosine |
| H | acyl | CH₃ | S | 6-N-acetyladenine |
| H | acyl | CH₃ | S | 2-N-acetyl-8-fluoroguanine |
| H | acyl | CH₃ | S | 4-N-acetyl-5-fluorocytosine |
| H | acyl | CH₃ | S | 6-N-acetyl-2-fluoroadenine |
| H | acyl | CH₃ | S | 6-N-acetyl-2,8-difluoroadenine |
| H | acyl | CH₃ | S | 6-N-acetyl-2-aminoadenine |
| H | acyl | CH₃ | S | 6-N-acetyl-2-amino-8-fluoroadenine |
| H | acyl | CH₃ | S | 2-N-acetylaminoadenine |
| H | acyl | CH₃ | S | 2-N-acetylamino-8-fluoroadenine |
| H | acyl | CH₃ | S | 2-N-acetylamino-8-fluorohypoxanthine |
| H | acyl | CH₃ | S | 2-N-acetylaminohypoxanthine |
| H | amino acid | CH₃ | S | Thymine |
| H | amino acid | CH₃ | S | Uracil |
| H | amino acid | CH₃ | S | Guanine |
| H | amino acid | CH₃ | S | Cytosine |
| H | amino acid | CH₃ | S | Adenine |
| H | amino acid | CH₃ | S | Hypoxanthine |
| H | amino acid | CH₃ | S | 5-Fluorouracil |
| H | amino acid | CH₃ | S | 8-Fluoroguanine |
| H | amino acid | CH₃ | S | 5-Fluorocytosine |
| H | amino acid | CH₃ | S | 8-Fluoroadenine |
| H | amino acid | CH₃ | S | 2-Fluoroadenine |
| H | amino acid | CH₃ | S | 2,8-Difluoroadenine |
| H | amino acid | CH₃ | S | 2-Fluorohypoxanthine |
| H | amino acid | CH₃ | S | 8-Fluorohypoxanthine |
| H | amino acid | CH₃ | S | 2,8-Difluorohypoxanthine |
| H | amino acid | CH₃ | S | 2-Aminoadenine |
| H | amino acid | CH₃ | S | 2-Amino-8-fluoroadenine |
| H | amino acid | CH₃ | S | 2-Amino-8-fluorohypoxanthine |
| H | amino acid | CH₃ | S | 2-Aminohypoxanthine |
| H | amino acid | CH₃ | S | 2-N-acetylguanine |
| H | amino acid | CH₃ | S | 4-N-acetylcytosine |
| H | amino acid | CH₃ | S | 6-N-acetyladenine |
| H | amino acid | CH₃ | S | 2-N-acetyl-8-fluoroguanine |
| H | amino acid | CH₃ | S | 4-N-acetyl-5-fluorocytosine |
| H | amino acid | CH₃ | S | 6-N-acetyl-2-fluoroadenine |
| H | amino acid | CH₃ | S | 6-N-acetyl-2,8-difluoroadenine |
| H | amino acid | CH₃ | S | 6-N-acetyl-2-aminoadenine |
| H | amino acid | CH₃ | S | 6-N-acetyl-2-amino-8-fluoroadenine |
| H | amino acid | CH₃ | S | 2-N-acetylaminoadenine |
| H | amino acid | CH₃ | S | 2-N-acetylamino-8-fluoroadenine |
| H | amino acid | CH₃ | S | 2-N-acetylamino-8-fluorohypoxanthine |
| H | amino acid | CH₃ | S | 2-N-acetylaminohypoxanthine |
| amino acid | amino acid | CH₃ | S | Thymine |
| amino acid | amino acid | CH₃ | S | Uracil |
| amino acid | amino acid | CH₃ | S | Guanine |
| amino acid | amino acid | CH₃ | S | Cytosine |
| amino acid | amino acid | CH₃ | S | Adenine |
| amino acid | amino acid | CH₃ | S | Hypoxanthine |
| amino acid | amino acid | CH₃ | S | 5-Fluorouracil |
| amino acid | amino acid | CH₃ | S | 8-Fluoroguanine |
| amino acid | amino acid | CH₃ | S | 5-Fluorocytosine |
| amino acid | amino acid | CH₃ | S | 8-Fluoroadenine |
| amino acid | amino acid | CH₃ | S | 2-Fluoroadenine |
| amino acid | amino acid | CH₃ | S | 2,8-Difluoroadenine |
| amino acid | amino acid | CH₃ | S | 2-Fluorohypoxanthine |
| amino acid | amino acid | CH₃ | S | 8-Fluorohypoxanthine |
| amino acid | amino acid | CH₃ | S | 2,8-Difluorohypoxanthine |
| amino acid | amino acid | CH₃ | S | 2-Aminoadenine |
| amino acid | amino acid | CH₃ | S | 2-Amino-8-fluoroadenine |
| amino acid | amino acid | CH₃ | S | 2-Amino-8-fluorohypoxanthine |
| amino acid | amino acid | CH₃ | S | 2-Aminohypoxanthine |
| amino acid | amino acid | CH₃ | S | 2-N-acetylguanine |
| amino acid | amino acid | CH₃ | S | 4-N-acetylcytosine |
| amino acid | amino acid | CH₃ | S | 6-N-acetyladenine |
| amino acid | amino acid | CH₃ | S | 2-N-acetyl-8-fluoroguanine |
| amino acid | amino acid | CH₃ | S | 4-N-acetyl-5-fluorocytosine |
| amino acid | amino acid | CH₃ | S | 6-N-acetyl-2-fluoroadenine |
| amino acid | amino acid | CH₃ | S | 6-N-acetyl-2,8-difluoroadenine |
| amino acid | amino acid | CH₃ | S | 6-N-acetyl-2-aminoadenine |
| amino acid | amino acid | CH₃ | S | 6-N-acetyl-2-amino-8-fluoroadenine |
| amino acid | amino acid | CH₃ | S | 2-N-acetylaminoadenine |
| amino acid | amino acid | CH₃ | S | 2-N-acetylamino-8-fluoroadenine |
| amino acid | amino acid | CH₃ | S | 2-N-acetylamino-8-fluorohypoxanthine |
| amino acid | amino acid | CH₃ | S | 2-N-acetylaminohypoxanthine |
| amino acid | H | CH₃ | S | Thymine |
| amino acid | H | CH₃ | S | Uracil |
| amino acid | H | CH₃ | S | Guanine |
| amino acid | H | CH₃ | S | Cytosine |
| amino acid | H | CH₃ | S | Adenine |
| amino acid | H | CH₃ | S | Hypoxanthine |
| amino acid | H | CH₃ | S | 5-Fluorouracil |
| amino acid | H | CH₃ | S | 8-Fluoroguanine |
| amino acid | H | CH₃ | S | 5-Fluorocytosine |
| amino acid | H | CH₃ | S | 8-Fluoroadenine |
| amino acid | H | CH₃ | S | 2-Fluoroadenine |
| amino acid | H | CH₃ | S | 2,8-Difluoroadenine |
| amino acid | H | CH₃ | S | 2-Fluorohypoxanthine |
| amino acid | H | CH₃ | S | 8-Fluorohypoxanthine |
| amino acid | H | CH₃ | S | 2,8-Difluorohypoxanthine |
| amino acid | H | CH₃ | S | 2-Aminoadenine |
| amino acid | H | CH₃ | S | 2-Amino-8-fluoroadenine |
| amino acid | H | CH₃ | S | 2-Am ino-8-fluorohypoxanthine |
| amino acid | H | CH₃ | S | 2-Aminohypoxanthine |
| amino acid | H | CH₃ | S | 2-N-acetylguanine |
| amino acid | H | CH₃ | S | 4-N-acetylcytosine |
| amino acid | H | CH₃ | S | 6-N-acetyladenine |
| amino acid | H | CH₃ | S | 2-N-acetyl-8-fluoroguanine |
| amino acid | H | CH₃ | S | 4-N-acetyl-5-fluorocytosine |
| amino acid | H | CH₃ | S | 6-N-acetyl-2-fluoroadenine |
| amino acid | H | CH₃ | S | 6-N-acetyl-2,8-difluoroadenine |
| amino acid | H | CH₃ | S | 6-N-acetyl-2-aminoadenine |
| amino acid | H | CH₃ | S | 6-N-acetyl-2-amino-8-fluoroadenine |
| amino acid | H | CH₃ | S | 2-N-acetylaminoadenine |
| amino acid | H | CH₃ | S | 2-N-acetylamino-8-fluoroadenine |
| amino acid | H | CH₃ | S | 2-N-acetylamino-8-fluorohypoxanthine |
| amino acid | H | CH₃ | S | 2-N-acetylaminohypoxanthine |
| amino acid | acyl | CH₃ | S | Thymine |
| amino acid | acyl | CH₃ | S | Uracil |
| amino acid | acyl | CH₃ | S | Guanine |
| amino acid | acyl | CH₃ | S | Cytosine |
| amino acid | acyl | CH₃ | S | Adenine |
| amino acid | acyl | CH₃ | S | Hypoxanthine |
| amino acid | acyl | CH₃ | S | 5-Fluorouracil |
| amino acid | acyl | CH₃ | S | 8-Fluoroguanine |
| amino acid | acyl | CH₃ | S | 5-Fluorocytosine |
| amino acid | acyl | CH₃ | S | 8-Fluoroadenine |
| amino acid | acyl | CH₃ | S | 2-Fluoroadenine |
| amino acid | acyl | CH₃ | S | 2,8-Difluoroadenine |
| amino acid | acyl | CH₃ | S | 2-Fluorohypoxanthine |
| amino acid | acyl | CH₃ | S | 8-Fluorohypoxanthine |
| amino acid | acyl | CH₃ | S | 2,8-Difluorohypoxanthine |
| amino acid | acyl | CH₃ | S | 2-Aminoadenine |
| amino acid | acyl | CH₃ | S | 2-Amino-8-fluoroadenine |
| amino acid | acyl | CH₃ | S | 2-Amino-8-fluorohypoxanthine |
| amino acid | acyl | CH₃ | S | 2-Aminohypoxanthine |
| amino acid | acyl | CH₃ | S | 2-N-acetylguanine |
| amino acid | acyl | CH₃ | S | 4-N-acetylcytosine |
| amino acid | acyl | CH₃ | S | 6-N-acetyladenine |
| amino acid | acyl | CH₃ | S | 2-N-acetyl-8-fluoroguanine |
| amino acid | acyl | CH₃ | S | 4-N-acetyl-5-fluorocytosine |
| amino acid | acyl | CH₃ | S | 6-N-acetyl-2-fluoroadenine |
| amino acid | acyl | CH₃ | S | 6-N-acetyl-2,8-difluoroadenine |
| amino acid | acyl | CH₃ | S | 6-N-acetyl-2-aminoadenine |
| amino acid | acyl | CH₃ | S | 6-N-acetyl-2-amino-8-fluoroadenine |
| amino acid | acyl | CH₃ | S | 2-N-acetylaminoadenine |
| amino acid | acyl | CH₃ | S | 2-N-acetylamino-8-fluoroadenine |
| amino acid | acyl | CH₃ | S | 2-N-acetylamino-8-fluorohypoxanthine |
| amino acid | acyl | CH₃ | S | 2-N-acetylaminohypoxanthine |
| acyl | H | CF₃ | S | Thymine |
| acyl | H | CF₃ | S | Uracil |
| acyl | H | CF₃ | S | Guanine |
| acyl | H | CF₃ | S | Cytosine |
| acyl | H | CF₃ | S | Adenine |
| acyl | H | CF₃ | S | Hypoxanthine |
| acyl | H | CF₃ | S | 5-Fluorouracil |
| acyl | H | CF₃ | S | 8-Fluoroguanine |
| acyl | H | CF₃ | S | 5-Fluorocytosine |
| acyl | H | CF₃ | S | 8-Fluoroadenine |
| acyl | H | CF₃ | S | 2-Fluoroadenine |
| acyl | H | CF₃ | S | 2,8-Difluoroadenine |
| acyl | H | CF₃ | S | 2-Fluorohypoxanthine |
| acyl | H | CF₃ | S | 8-Fluorohypoxanthine |
| acyl | H | CF₃ | S | 2,8-Difluorohypoxanthine |
| acyl | H | CF₃ | S | 2-Aminoadenine |
| acyl | H | CF₃ | S | 2-Amino-8-fluoroadenine |
| acyl | H | CF₃ | S | 2-Amino-8-fluorohypoxanthine |
| acyl | H | CF₃ | S | 2-Aminohypoxanthine |
| acyl | H | CF₃ | S | 2-N-acetylguanine |
| acyl | H | CF₃ | S | 4-N-acetylcytosine |
| acyl | H | CF₃ | S | 6-N-acetyladenine |
| acyl | H | CF₃ | S | 2-N-acetyl-8-fluoroguanine |
| acyl | H | CF₃ | S | 4-N-acetyl-5-fluorocytosine |
| acyl | H | CF₃ | S | 6-N-acetyl-2-fluoroaden ine |
| acyl | H | CF₃ | S | 6-N-acetyl-2,8-difluoroadenine |
| acyl | H | CF₃ | S | 6-N-acetyl-2-aminoadenine |
| acyl | H | CF₃ | S | 6-N-acetyl-2-amino-8-fluoroadenine |
| acyl | H | CF₃ | S | 2-N-acetylaminoadenine |
| acyl | H | CF₃ | S | 2-N-acetylamino-8-fluoroadenine |
| acyl | H | CF₃ | S | 2-N-acetylamino-8-fluorohypoxanthine |
| acyl | H | CF₃ | S | 2-N-acetylaminohypoxanthine |
| acyl | acyl | CF₃ | S | Thymine |
| acyl | acyl | CF₃ | S | Uracil |
| acyl | acyl | CF₃ | S | Guanine |
| acyl | acyl | CF₃ | S | Cytosine |
| acyl | acyl | CF₃ | S | Adenine |
| acyl | acyl | CF₃ | S | Hypoxanthine |
| acyl | acyl | CF₃ | S | 5-Fluorouracil |
| acyl | acyl | CF₃ | S | 8-Fluoroguanine |
| acyl | acyl | CF₃ | S | 5-Fluorocytosine |
| acyl | acyl | CF₃ | S | 8-Fluoroadenine |
| acyl | acyl | CF₃ | S | 2-Fluoroadenine |
| acyl | acyl | CF₃ | S | 2,8-Difluoroadenine |
| acyl | acyl | CF₃ | S | 2-Fluorohypoxanthine |
| acyl | acyl | CF₃ | S | 8-Fluorohypoxanthine |
| acyl | acyl | CF₃ | S | 2,8-Difluorohypoxanthine |
| acyl | acyl | CF₃ | S | 2-Aminoadenine |
| acyl | acyl | CF₃ | S | 2-Amino-8-fluoroadenine |
| acyl | acyl | CF₃ | S | 2-Amino-8-fluorohypoxanthine |
| acyl | acyl | CF₃ | S | 2-Aminohypoxanthine |
| acyl | acyl | CF₃ | S | 2-N-acetylguanine |
| acyl | acyl | CF₃ | S | 4-N-acetylcytosine |
| acyl | acyl | CF₃ | S | 6-N-acetyladenine |
| acyl | acyl | CF₃ | S | 2-N-acetyl-8-fluoroguanine |
| acyl | acyl | CF₃ | S | 4-N-acetyl-5-fluorocytosine |
| acyl | acyl | CF₃ | S | 6-N-acetyl-2-fluoroadenine |
| acyl | acyl | CF₃ | S | 6-N-acetyl-2,8-difluoroadenine |
| acyl | acyl | CF₃ | S | 6-N-acetyl-2-aminoadenine |
| acyl | acyl | CF₃ | S | 6-N-acetyl-2-amino-8-fluoroadenine |
| acyl | acyl | CF₃ | S | 2-N-acetylaminoadenine |
| acyl | acyl | CF₃ | S | 2-N-acetylamino-8-fluoroadenine |
| acyl | acyl | CF₃ | S | 2-N-acetylamino-8-fluorohypoxanthine |
| acyl | acyl | CF₃ | S | 2-N-acetylaminohypoxanthine |
| acyl | amino acid | CF₃ | S | Thymine |
| acyl | amino acid | CF₃ | S | Uracil |
| acyl | amino acid | CF₃ | S | Guanine |
| acyl | amino acid | CF₃ | S | Cytosine |
| acyl | amino acid | CF₃ | S | Adenine |
| acyl | amino acid | CF₃ | S | Hypoxanthine |
| acyl | amino acid | CF₃ | S | 5-Fluorouracil |
| acyl | amino acid | CF₃ | S | 8-Fluoroguanine |
| acyl | amino acid | CF₃ | S | 5-Fluorocytosine |
| acyl | amino acid | CF₃ | S | 8-Fluoroadenine |
| acyl | amino acid | CF₃ | S | 2-Fluoroadenine |
| acyl | amino acid | CF₃ | S | 2,8-Difluoroadenine |
| acyl | amino acid | CF₃ | S | 2-Fluorohypoxanthine |
| acyl | amino acid | CF₃ | S | 8-Fluorohypoxanthine |
| acyl | amino acid | CF₃ | S | 2,8-Difluorohypoxanthine |
| acyl | amino acid | CF₃ | S | 2-Aminoadenine |
| acyl | amino acid | CF₃ | S | 2-Amino-8-fluoroadenine |
| acyl | amino acid | CF₃ | S | 2-Amino-8-fluorohypoxanthine |
| acyl | amino acid | CF₃ | S | 2-Aminohypoxanthine |
| acyl | amino acid | CF₃ | S | 2-N-acetylguanine |
| acyl | amino acid | CF₃ | S | 4-N-acetylcytosine |
| acyl | amino acid | CF₃ | S | 6-N-acetyladenine |
| acyl | amino acid | CF₃ | S | 2-N-acetyl-8-fluoroguanine |
| acyl | amino acid | CF₃ | S | 4-N-acetyl-5-fluorocytosine |
| acyl | amino acid | CF₃ | S | 6-N-acetyl-2-fluoroadenine |
| acyl | amino acid | CF₃ | S | 6-N-acetyl-2,8-difluoroadenine |
| acyl | amino acid | CF₃ | S | 6-N-acetyl-2-aminoadenine |
| acyl | amino acid | CF₃ | S | 6-N-acetyl-2-amino-8-fluoroadenine |
| acyl | amino acid | CF₃ | S | 2-N-acetylaminoadenine |
| acyl | amino acid | CF₃ | S | 2-N-acetylamino-8-fluoroadenine |
| acyl | amino acid | CF₃ | S | 2-N-acetylamino-8-fluorohypoxanthine |
| acyl | amino acid | CF₃ | S | 2-N-acetylaminohypoxanthine |
| H | acyl | CF₃ | S | Thymine |
| H | acyl | CF₃ | S | Uracil |
| H | acyl | CF₃ | S | Guanine |
| H | acyl | CF₃ | S | Cytosine |
| H | acyl | CF₃ | S | Adenine |
| H | acyl | CF₃ | S | Hypoxanthine |
| H | acyl | CF₃ | S | 5-Fluorouracil |
| H | acyl | CF₃ | S | 8-Fluoroguanine |
| H | acyl | CF₃ | S | 5-Fluorocytosine |
| H | acyl | CF₃ | S | 8-Fluoroadenine |
| H | acyl | CF₃ | S | 2-Fluoroadenine |
| H | acyl | CF₃ | S | 2,8-Difluoroadenine |
| H | acyl | CF₃ | S | 2-Fluorohypoxanthine |
| H | acyl | CF₃ | S | 8-Fluorohypoxanthine |
| H | acyl | CF₃ | S | 2,8-Difluorohypoxanthine |
| H | acyl | CF₃ | S | 2-Aminoadenine |
| H | acyl | CF₃ | S | 2-Amino-8-fluoroadenine |
| H | acyl | CF₃ | S | 2-Amino-8-fluorohypoxanthine |
| H | acyl | CF₃ | S | 2-Aminohypoxanthine |
| H | acyl | CF₃ | S | 2-N-acetylguanine |
| H | acyl | CF₃ | S | 4-N-acetylcytosine |
| H | acyl | CF₃ | S | 6-N-acetyladenine |
| H | acyl | CF₃ | S | 2-N-acetyl-8-fluoroguanine |
| H | acyl | CF₃ | S | 4-N-acetyl-5-fluorocytosine |
| H | acyl | CF₃ | S | 6-N-acetyl-2-fluoroadenine |
| H | acyl | CF₃ | S | 6-N-acetyl-2,8-difluoroadenine |
| H | acyl | CF₃ | S | 6-N-acetyl-2-aminoadenine |
| H | acyl | CF₃ | S | 6-N-acetyl-2-amino-8-fluoroadenine |
| H | acyl | CF₃ | S | 2-N-acetylaminoadenine |
| H | acyl | CF₃ | S | 2-N-acetylamino-8-fluoroadenine |
| H | acyl | CF₃ | S | 2-N-acetylamino-8-fluorohypoxanthine |
| H | acyl | CF₃ | S | 2-N-acetylaminohypoxanthine |
| H | amino acid | CF₃ | S | Thymine |
| H | amino acid | CF₃ | S | Uracil |
| H | amino acid | CF₃ | S | Guanine |
| H | amino acid | CF₃ | S | Cytosine |
| H | amino acid | CF₃ | S | Adenine |
| H | amino acid | CF₃ | S | Hypoxanthine |
| H | amino acid | CF₃ | S | 5-Fluorouracil |
| H | amino acid | CF₃ | S | 8-Fluoroguanine |
| H | amino acid | CF₃ | S | 5-Fluorocytosine |
| H | amino acid | CF₃ | S | 8-Fluoroadenine |
| H | amino acid | CF₃ | S | 2-Fluoroadenine |
| H | amino acid | CF₃ | S | 2,8-Difluoroadenine |
| H | amino acid | CF₃ | S | 2-Fluorohypoxanthine |
| H | amino acid | CF₃ | S | 8-Fluorohypoxanthine |
| H | amino acid | CF₃ | S | 2,8-Difluorohypoxanthine |
| H | amino acid | CF₃ | S | 2-Aminoadenine |
| H | amino acid | CF₃ | S | 2-Amino-8-fluoroadenine |
| H | amino acid | CF₃ | S | 2-Amino-8-fluorohypoxanthine |
| H | amino acid | CF₃ | S | 2-Aminohypoxanthine |
| H | amino acid | CF₃ | S | 2-N-acetylguanine |
| H | amino acid | CF₃ | S | 4-N-acetylcytosine |
| H | amino acid | CF₃ | S | 6-N-acetyladenine |
| H | amino acid | CF₃ | S | 2-N-acetyl-8-fluoroguanine |
| H | amino acid | CF₃ | S | 4-N-acetyl-5-fluorocytosine |
| H | amino acid | CF₃ | S | 6-N-acetyl-2-fluoroadenine |
| H | amino acid | CF₃ | S | 6-N-acetyl-2,8-difluoroadenine |
| H | amino acid | CF₃ | S | 6-N-acetyl-2-aminoadenine |
| H | amino acid | CF₃ | S | 6-N-acetyl-2-amino-8-fluoroadenine |
| H | amino acid | CF₃ | S | 2-N-acetylaminoadenine |
| H | amino acid | CF₃ | S | 2-N-acetylamino-8-fluoroadenine |
| H | amino acid | CF₃ | S | 2-N-acetylamino-8-fluorohypoxanthine |
| H | amino acid | CF₃ | S | 2-N-acetylaminohypoxanthine |
| amino acid | amino acid | CF₃ | S | Thymine |
| amino acid | amino acid | CF₃ | S | Uracil |
| amino acid | amino acid | CF₃ | S | Guanine |
| amino acid | amino acid | CF₃ | S | Cytosine |
| amino acid | amino acid | CF₃ | S | Adenine |
| amino acid | amino acid | CF₃ | S | Hypoxanthine |
| amino acid | amino acid | CF₃ | S | 5-Fluorouracil |
| amino acid | amino acid | CF₃ | S | 8-Fluoroguanine |
| amino acid | amino acid | CF₃ | S | 5-Fluorocytosine |
| amino acid | amino acid | CF₃ | S | 8-Fluoroadenine |
| amino acid | amino acid | CF₃ | S | 2-Fluoroadenine |
| amino acid | amino acid | CF₃ | S | 2,8-Difluoroadenine |
| amino acid | amino acid | CF₃ | S | 2-Fluorohypoxanthine |
| amino acid | amino acid | CF₃ | S | 8-Fluorohypoxanthine |
| amino acid | amino acid | CF₃ | S | 2,8-Difluorohypoxanthine |
| amino acid | amino acid | CF₃ | S | 2-Aminoadenine |
| amino acid | amino acid | CF₃ | S | 2-Amino-8-fluoroadenine |
| amino acid | amino acid | CF₃ | S | 2-Amino-8-fluorohypoxanthine |
| amino acid | amino acid | CF₃ | S | 2-Aminohypoxanthine |
| amino acid | amino acid | CF₃ | S | 2-N-acetylguanine |
| amino acid | amino acid | CF₃ | S | 4-N-acetylcytosine |
| amino acid | amino acid | CF₃ | S | 6-N-acetyladenine |
| amino acid | amino acid | CF₃ | S | 2-N-acetyl-8-fluoroguanine |
| amino acid | amino acid | CF₃ | S | 4-N-acetyl-5-fluorocytosine |
| amino acid | amino acid | CF₃ | S | 6-N-acetyl-2-fluoroadenine |
| amino acid | amino acid | CF₃ | S | 6-N-acetyl-2,8-difluoroadenine |
| amino acid | amino acid | CF₃ | S | 6-N-acetyl-2-aminoadenine |
| amino acid | amino acid | CF₃ | S | 6-N-acetyl-2-amino-8-fluoroadenine |
| amino acid | amino acid | CF₃ | S | 2-N-acetylaminoadenine |
| amino acid | amino acid | CF₃ | S | 2-N-acetylamino-8-fluoroadenine |
| amino acid | amino acid | CF₃ | S | 2-N-acetylamino-8-fluorohypoxanthine |
| amino acid | amino acid | CF₃ | S | 2-N-acetylaminohypoxanthine |
| amino acid | H | CF₃ | S | Thymine |
| amino acid | H | CF₃ | S | Uracil |
| amino acid | H | CF₃ | S | Guanine |
| amino acid | H | CF₃ | S | Cytosine |
| amino acid | H | CF₃ | S | Adenine |
| amino acid | H | CF₃ | S | Hypoxanthine |
| amino acid | H | CF₃ | S | 5-Fluorouracil |
| amino acid | H | CF₃ | S | 8-Fluoroguanine |
| amino acid | H | CF₃ | S | 5-Fluorocytosine |
| amino acid | H | CF₃ | S | 8-Fluoroadenine |
| amino acid | H | CF₃ | S | 2-Fluoroadenine |
| amino acid | H | CF₃ | S | 2,8-Difluoroadenine |
| amino acid | H | CF₃ | S | 2-Fluorohypoxanthine |
| amino acid | H | CF₃ | S | 8-Fluorohypoxanthine |
| amino acid | H | CF₃ | S | 2,8-Difluorohypoxanthine |
| amino acid | H | CF₃ | S | 2-Aminoadenine |
| amino acid | H | CF₃ | S | 2-Amino-8-fluoroadenine |
| amino acid | H | CF₃ | S | 2-Amino-8-fluorohypoxanthine |
| amino acid | H | CF₃ | S | 2-Aminohypoxanthine |
| amino acid | H | CF₃ | S | 2-N-acetylguanine |
| amino acid | H | CF₃ | S | 4-N-acetylcytosine |
| amino acid | H | CF₃ | S | 6-N-acetyladenine |
| amino acid | H | CF₃ | S | 2-N-acetyl-8-fluoroguanine |
| amino acid | H | CF₃ | S | 4-N-acetyl-5-fluorocytosine |
| amino acid | H | CF₃ | S | 6-N-acetyl-2-fluoroadenine |
| amino acid | H | CF₃ | S | 6-N-acetyl-2,8-difluoroadenine |
| amino acid | H | CF₃ | S | 6-N-acetyl-2-aminoadenine |
| amino acid | H | CF₃ | S | 6-N-acetyl-2-amino-8-fluoroadenine |
| amino acid | H | CF₃ | S | 2-N-acetylaminoadenine |
| amino acid | H | CF₃ | S | 2-N-acetylamino-8-fluoroadenine |
| amino acid | H | CF₃ | S | 2-N-acetylamino-8-fluorohypoxanthine |
| amino acid | H | CF₃ | S | 2-N-acetylaminohypoxanthine |
| amino acid | acyl | CF₃ | S | Thymine |
| amino acid | acyl | CF₃ | S | Uracil |
| amino acid | acyl | CF₃ | S | Guanine |
| amino acid | acyl | CF₃ | S | Cytosine |
| amino acid | acyl | CF₃ | S | Adenine |
| amino acid | acyl | CF₃ | S | Hypoxanthine |
| amino acid | acyl | CF₃ | S | 5-Fluorouracil |
| amino acid | acyl | CF₃ | S | 8-Fluoroguanine |
| amino acid | acyl | CF₃ | S | 5-Fluorocytosine |
| amino acid | acyl | CF₃ | S | 8-Fluoroadenine |
| amino acid | acyl | CF₃ | S | 2-Fluoroadenine |
| amino acid | acyl | CF₃ | S | 2,8-Difluoroadenine |
| amino acid | acyl | CF₃ | S | 2-Fluorohypoxanthine |
| amino acid | acyl | CF₃ | S | 8-Fluorohypoxanthine |
| amino acid | acyl | CF₃ | S | 2,8-Difluorohypoxanthine |
| amino acid | acyl | CF₃ | S | 2-Aminoadenine |
| amino acid | acyl | CF₃ | S | 2-Amino-8-fluoroadenine |
| amino acid | acyl | CF₃ | S | 2-Amino-8-fluorohypoxanthine |
| amino acid | acyl | CF₃ | S | 2-Aminohypoxanthine |
| amino acid | acyl | CF₃ | S | 2-N-acetylguanine |
| amino acid | acyl | CF₃ | S | 4-N-acetylcytosine |
| amino acid | acyl | CF₃ | S | 6-N-acetyladenine |
| amino acid | acyl | CF₃ | S | 2-N-acetyl-8-fluoroguanine |
| amino acid | acyl | CF₃ | S | 4-N-acetyl-5-fluorocytosine |
| amino acid | acyl | CF₃ | S | 6-N-acetyl-2-fluoroadenine |
| amino acid | acyl | CF₃ | S | 6-N-acetyl-2,8-difluoroadenine |
| amino acid | acyl | CF₃ | S | 6-N-acetyl-2-aminoadenine |
| amino acid | acyl | CF₃ | S | 6-N-acetyl-2-amino-8-fluoroadenine |
| amino acid | acyl | CF₃ | S | 2-N-acetylaminoadenine |
| amino acid | acyl | CF₃ | S | 2-N-acetylamino-8-fluoroadenine |
| amino acid | acyl | CF₃ | s | 2-N-acetylamino-8-fluorohypoxanthine |
| amino acid | acyl | CF₃ | S | 2-N-acetylaminohypoxanthine |
| acyl | H | CF₃ | O | Thymine |
| acyl | H | CF₃ | O | Uracil |
| acyl | H | CF₃ | O | Guanine |
| acyl | H | CF₃ | O | Cytosine |
| acyl | H | CF₃ | O | Adenine |
| acyl | H | CF₃ | O | Hypoxanthine |
| acyl | H | CF₃ | O | 5-Fluorouracil |
| acyl | H | CF₃ | O | 8-Fluoroguanine |
| acyl | H | CF₃ | O | 5-Fluorocytosine |
| acyl | H | CF₃ | O | 8-Fluoroadenine |
| acyl | H | CF₃ | O | 2-Fluoroadenine |
| acyl | H | CF₃ | O | 2,8-Difluoroadenine |
| acyl | H | CF₃ | O | 2-Fluorohypoxanthine |
| acyl | H | CF₃ | O | 8-Fluorohypoxanthine |
| acyl | H | CF₃ | O | 2,8-Difluorohypoxanthine |
| acyl | H | CF₃ | O | 2-Aminoadenine |
| acyl | H | CF₃ | O | 2-Amino-8-fluoroadenine |
| acyl | H | CF₃ | O | 2-Amino-8-fluorohypoxanthine |
| acyl | H | CF₃ | O | 2-Aminohypoxanthine |
| acyl | H | CF₃ | O | 2-N-acetylguanine |
| acyl | H | CF₃ | O | 4-N-acetylcytosine |
| acyl | H | CF₃ | O | 6-N-acetyladenine |
| acyl | H | CF₃ | O | 2-N-acetyl-8-fluoroguanine |
| acyl | H | CF₃ | O | 4-N-acetyl-5-fluorocytosine |
| acyl | H | CF₃ | O | 6-N-acetyl-2-fluoroadenine |
| acyl | H | CF₃ | O | 6-N-acetyl-2,8-difluoroadenine |
| acyl | H | CF₃ | O | 6-N-acetyl-2-aminoadenine |
| acyl | H | CF₃ | O | 6-N-acetyl-2-amino-8-fluoroadenine |
| acyl | H | CF₃ | O | 2-N-acetylaminoadenine |
| acyl | H | CF₃ | O | 2-N-acetylamino-8-fluoroadenine |
| acyl | H | CF₃ | O | 2-N-acetylamino-8-fluorohypoxanthine |
| acyl | H | CF₃ | O | 2-N-acetylaminohypoxanthine |
| acyl | acyl | CF₃ | O | Thymine |
| acyl | acyl | CF₃ | O | Uracil |
| acyl | acyl | CF₃ | O | Guanine |
| acyl | acyl | CF₃ | O | Cytosine |
| acyl | acyl | CF₃ | O | Adenine |
| acyl | acyl | CF₃ | O | Hypoxanthine |
| acyl | acyl | CF₃ | O | 5-Fluorouracil |
| acyl | acyl | CF₃ | O | 8-Fluoroguanine |
| acyl | acyl | CF₃ | O | 5-Fluorocytosine |
| acyl | acyl | CF₃ | O | 8-Fluoroadenine |
| acyl | acyl | CF₃ | O | 2-Fluoroadenine |
| acyl | acyl | CF₃ | O | 2,8-Difluoroadenine |
| acyl | acyl | CF₃ | O | 2-Fluorohypoxanthine |
| acyl | acyl | CF₃ | O | 8-Fluorohypoxanthine |
| acyl | acyl | CF₃ | O | 2,8-Difluorohypoxanthine |
| acyl | acyl | CF₃ | O | 2-Aminoadenine |
| acyl | acyl | CF₃ | O | 2-Amino-8-fluoroadenine |
| acyl | acyl | CF₃ | O | 2-Amino-8-fluorohypoxanthine |
| acyl | acyl | CF₃ | O | 2-Aminohypoxanthine |
| acyl | acyl | CF₃ | O | 2-N-acetylguanine |
| acyl | acyl | CF₃ | O | 4-N-acetylcytosine |
| acyl | acyl | CF₃ | O | 6-N-acetyladenine |
| acyl | acyl | CF₃ | O | 2-N-acetyl-8-fluoroguanine |
| acyl | acyl | CF₃ | O | 4-N-acetyl-5-fluorocytosine |
| acyl | acyl | CF₃ | O | 6-N-acetyl-2-fluoroadenine |
| acyl | acyl | CF₃ | O | 6-N-acetyl-2,8-difluoroadenine |
| acyl | acyl | CF₃ | O | 6-N-acetyl-2-aminoadenine |
| acyl | acyl | CF₃ | O | 6-N-acetyl-2-amino-8-fluoroadenine |
| acyl | acyl | CF₃ | O | 2-N-acetylaminoadenine |
| acyl | acyl | CF₃ | O | 2-N-acetylamino-8-fluoroadenine |
| acyl | acyl | CF₃ | O | 2-N-acetylamino-8-fluorohypoxanthine |
| acyl | acyl | CF₃ | O | 2-N-acetylaminohypoxanthine |
| acyl | amino acid | CF₃ | O | Thymine |
| acyl | amino acid | CF₃ | O | Uracil |
| acyl | amino acid | CF₃ | O | Guanine |
| acyl | amino acid | CF₃ | O | Cytosine |
| acyl | amino acid | CF₃ | O | Adenine |
| acyl | amino acid | CF₃ | O | Hypoxanthine |
| acyl | amino acid | CF₃ | O | 5-Fluorouracil |
| acyl | amino acid | CF₃ | O | 8-Fluoroguanine |
| acyl | amino acid | CF₃ | O | 5-Fluorocytosine |
| acyl | amino acid | CF₃ | O | 8-Fluoroadenine |
| acyl | amino acid | CF₃ | O | 2-Fluoroadenine |
| acyl | amino acid | CF₃ | O | 2,8-Difluoroadenine |
| acyl | amino acid | CF₃ | O | 2-Fluorohypoxanthine |
| acyl | amino acid | CF₃ | O | 8-Fluorohypoxanthine |
| acyl | amino acid | CF₃ | O | 2,8-Difluorohypoxanthine |
| acyl | amino acid | CF₃ | O | 2-Aminoadenine |
| acyl | amino acid | CF₃ | O | 2-Amino-8-fluoroadenine |
| acyl | amino acid | CF₃ | O | 2-Amino-8-fluorohypoxanthine |
| acyl | amino acid | CF₃ | O | 2-Aminohypoxanthine |
| acyl | amino acid | CF₃ | O | 2-N-acetylguanine |
| acyl | amino acid | CF₃ | O | 4-N-acetylcytosine |
| acyl | amino acid | CF₃ | O | 6-N-acetyladenine |
| acyl | amino acid | CF₃ | O | 2-N-acetyl-8-fluoroguanine |
| acyl | amino acid | CF₃ | O | 4-N-acetyl-5-fluorocytosine |
| acyl | amino acid | CF₃ | O | 6-N-acetyl-2-fluoroadenine |
| acyl | amino acid | CF₃ | O | 6-N-acetyl-2,8-difluoroadenine |
| acyl | amino acid | CF₃ | O | 6-N-acetyl-2-aminoadenine |
| acyl | amino acid | CF₃ | O | 6-N-acetyl-2-amino-8-fluoroadenine |
| acyl | amino acid | CF₃ | O | 2-N-acetylaminoadenine |
| acyl | amino acid | CF₃ | O | 2-N-acetylamino-8-fluoroadenine |
| acyl | amino acid | CF₃ | O | 2-N-acetylamino-8-fluorohypoxanthine |
| acyl | amino acid | CF₃ | O | 2-N-acetylaminohypoxanthine |
| H | acyl | CF₃ | O | Thymine |
| H | acyl | CF₃ | O | Uracil |
| H | acyl | CF₃ | O | Guanine |
| H | acyl | CF₃ | O | Cytosine |
| H | acyl | CF₃ | O | Adenine |
| H | acyl | CF₃ | O | Hypoxanthine |
| H | acyl | CF₃ | O | 5-Fluorouracil |
| H | acyl | CF₃ | O | 8-Fluoroguanine |
| H | acyl | CF₃ | O | 5-Fluorocytosine |
| H | acyl | CF₃ | O | 8-Fluoroadenine |
| H | acyl | CF₃ | O | 2-Fluoroadenine |
| H | acyl | CF₃ | O | 2,8-Difluoroadenine |
| H | acyl | CF₃ | O | 2-Fluorohypoxanthine |
| H | acyl | CF₃ | O | 8-Fluorohypoxanthine |
| H | acyl | CF₃ | O | 2,8-Difluorohypoxanthine |
| H | acyl | CF₃ | O | 2-Aminoadenine |
| H | acyl | CF₃ | O | 2-Amino-8-fluoroadenine |
| H | acyl | CF₃ | O | 2-Amino-8-fluorohypoxanthine |
| H | acyl | CF₃ | O | 2-Aminohypoxanthine |
| H | acyl | CF₃ | O | 2-N-acetylguanine |
| H | acyl | CF₃ | O | 4-N-acetylcytosine |
| H | acyl | CF₃ | O | 6-N-acetyladenine |
| H | acyl | CF₃ | O | 2-N-acetyl-8-fluoroguanine |
| H | acyl | CF₃ | O | 4-N-acetyl-5-fluorocytosine |
| H | acyl | CF₃ | O | 6-N-acetyl-2-fluoroadenine |
| H | acyl | CF₃ | O | 6-N-acetyl-2,8-difluoroadenine |
| H | acyl | CF₃ | O | 6-N-acetyl-2-aminoadenine |
| H | acyl | CF₃ | O | 6-N-acetyl-2-amino-8-fluoroadenine |
| H | acyl | CF₃ | O | 2-N-acetylaminoadenine |
| H | acyl | CF₃ | O | 2-N-acetylamino-8-fluoroadenine |
| H | acyl | CF₃ | O | 2-N-acetylamino-8-fluorohypoxanthine |
| H | acyl | CF₃ | O | 2-N-acetylaminohypoxanthine |
| H | amino acid | CF₃ | O | Thymine |
| H | amino acid | CF₃ | O | Uracil |
| H | amino acid | CF₃ | O | Guanine |
| H | amino acid | CF₃ | O | Cytosine |
| H | amino acid | CF₃ | O | Adenine |
| H | amino acid | CF₃ | O | Hypoxanthine |
| H | amino acid | CF₃ | O | 5-Fluorouracil |
| H | amino acid | CF₃ | O | 8-Fluoroguanine |
| H | amino acid | CF₃ | O | 5-Fluorocytosine |
| H | amino acid | CF₃ | O | 8-Fluoroadenine |
| H | amino acid | CF₃ | O | 2-Fluoroadenine |
| H | amino acid | CF₃ | O | 2,8-Difluoroadenine |
| H | amino acid | CF₃ | O | 2-Fluorohypoxanthine |
| H | amino acid | CF₃ | O | 8-Fluorohypoxanthine |
| H | amino acid | CF₃ | O | 2,8-Difluorohypoxanthine |
| H | amino acid | CF₃ | O | 2-Aminoadenine |
| H | amino acid | CF₃ | O | 2-Amino-8-fluoroadenine |
| H | amino acid | CF₃ | O | 2-Amino-8-fluorohypoxanthine |
| H | amino acid | CF₃ | O | 2-Aminohypoxanthine |
| H | amino acid | CF₃ | O | 2-N-acetylguanine |
| H | amino acid | CF₃ | O | 4-N-acetylcytosine |
| H | amino acid | CF₃ | O | 6-N-acetyladenine |
| H | amino acid | CF₃ | O | 2-N-acetyl-8-fluoroguanine |
| H | amino acid | CF₃ | O | 4-N-acetyl-5-fluorocytosine |
| H | amino acid | CF₃ | O | 6-N-acetyl-2-fluoroadenine |
| H | amino acid | CF₃ | O | 6-N-acetyl-2,8-difluoroadenine |
| H | amino acid | CF₃ | O | 6-N-acetyl-2-aminoadenine |
| H | amino acid | CF₃ | O | 6-N-acetyl-2-amino-8-fluoroadenine |
| H | amino acid | CF₃ | O | 2-N-acetylaminoadenine |
| H | amino acid | CF₃ | O | 2-N-acetylamino-8-fluoroadenine |
| H | amino acid | CF₃ | O | 2-N-acetylamino-8-fluorohypoxanthine |
| H | amino acid | CF₃ | O | 2-N-acetylaminohypoxanthine |
| amino acid | amino acid | CF₃ | O | Thymine |
| amino acid | amino acid | CF₃ | O | Uracil |
| amino acid | amino acid | CF₃ | O | Guanine |
| amino acid | amino acid | CF₃ | O | Cytosine |
| amino acid | amino acid | CF₃ | O | Adenine |
| amino acid | amino acid | CF₃ | O | Hypoxanthine |
| amino acid | amino acid | CF₃ | O | 5-Fluorouracil |
| amino acid | amino acid | CF₃ | O | 8-Fluoroguanine |
| amino acid | amino acid | CF₃ | O | 5-Fluorocytosine |
| amino acid | amino acid | CF₃ | O | 8-Fluoroadenine |
| amino acid | amino acid | CF₃ | O | 2-Fluoroadenine |
| amino acid | amino acid | CF₃ | O | 2,8-Difluoroadenine |
| amino acid | amino acid | CF₃ | O | 2-Fluorohypoxanthine |
| amino acid | amino acid | CF₃ | O | 8-Fluorohypoxanthine |
| amino acid | amino acid | CF₃ | O | 2,8-Difluorohypoxanthine |
| amino acid | amino acid | CF₃ | O | 2-Aminoadenine |
| amino acid | amino acid | CF₃ | O | 2-Amino-8-fluoroadenine |
| amino acid | amino acid | CF₃ | O | 2-Amino-8-fluorohypoxanthine |
| amino acid | amino acid | CF₃ | O | 2-Aminohypoxanthine |
| amino acid | amino acid | CF₃ | O | 2-N-acetylguanine |
| amino acid | amino acid | CF₃ | O | 4-N-acetylcytosine |
| amino acid | amino acid | CF₃ | O | 6-N-acetyladenine |
| amino acid | amino acid | CF₃ | O | 2-N-acetyl-8-fluoroguanine |
| amino acid | amino acid | CF₃ | O | 4-N-acetyl-5-fluorocytosine |
| amino acid | amino acid | CF₃ | O | 6-N-acetyl-2-fluoroadenine |
| amino acid | amino acid | CF₃ | O | 6-N-acetyl-2,8-difluoroadenine |
| amino acid | amino acid | CF₃ | O | 6-N-acetyl-2-aminoadenine |
| amino acid | amino acid | CF₃ | O | 6-N-acetyl-2-amino-8-fluoroadenine |
| amino acid | amino acid | CF₃ | O | 2-N-acetylaminoadenine |
| amino acid | amino acid | CF₃ | O | 2-N-acetylamino-8-fluoroadenine |
| amino acid | amino acid | CF₃ | O | 2-N-acetylamino-8-fluorohypoxanthine |
| amino acid | amino acid | CF₃ | O | 2-N-acetylaminohypoxanthine |
| amino acid | H | CF₃ | O | Thymine |
| amino acid | H | CF₃ | O | Uracil |
| amino acid | H | CF₃ | O | Guanine |
| amino acid | H | CF₃ | O | Cytosine |
| amino acid | H | CF₃ | O | Adenine |
| amino acid | H | CF₃ | O | Hypoxanthine |
| amino acid | H | CF₃ | O | 5-Fluorouracil |
| amino acid | H | CF₃ | O | 8-Fluoroguanine |
| amino acid | H | CF₃ | O | 5-Fluorocytosine |
| amino acid | H | CF₃ | O | 8-Fluoroadenine |
| amino acid | H | CF₃ | O | 2-Fluoroadenine |
| amino acid | H | CF₃ | O | 2,8-Difluoroadenine |
| amino acid | H | CF₃ | O | 2-Fluorohypoxanthine |
| amino acid | H | CF₃ | O | 8-Fluorohypoxanthine |
| amino acid | H | CF₃ | O | 2,8-Difluorohypoxanthine |
| amino acid | H | CF₃ | O | 2-Aminoadenine |
| amino acid | H | CF₃ | O | 2-Amino-8-fluoroadenine |
| amino acid | H | CF₃ | O | 2-Amino-8-fluorohypoxanthine |
| amino acid | H | CF₃ | O | 2-Aminohypoxanthine |
| amino acid | H | CF₃ | O | 2-N-acetylguanine |
| amino acid | H | CF₃ | O | 4-N-acetylcytosine |
| amino acid | H | CF₃ | O | 6-N-acetyladenine |
| amino acid | H | CF₃ | O | 2-N-acetyl-8-fluoroguanine |
| amino acid | H | CF₃ | O | 4-N-acetyl-5-fluorocytosine |
| amino acid | H | CF₃ | O | 6-N-acetyl-2-fluoroadenine |
| amino acid | H | CF₃ | O | 6-N-acetyl-2,8-difluoroadenine |
| amino acid | H | CF₃ | O | 6-N-acetyl-2-aminoadenine |
| amino acid | H | CF₃ | O | 6-N-acetyl-2-amino-8-fluoroadenine |
| amino acid | H | CF₃ | O | 2-N-acetylaminoadenine |
| amino acid | H | CF₃ | O | 2-N-acetylamino-8-fluoroadenine |
| amino acid | H | CF₃ | O | 2-N-acetylamino-8-fluorohypoxanthine |
| amino acid | H | CF₃ | O | 2-N-acetylaminohypoxanthine |
| amino acid | acyl | CF₃ | O | Thymine |
| amino acid | acyl | CF₃ | O | Uracil |
| amino acid | acyl | CF₃ | O | Guanine |
| amino acid | acyl | CF₃ | O | Cytosine |
| amino acid | acyl | CF₃ | O | Adenine |
| amino acid | acyl | CF₃ | O | Hypoxanthine |
| amino acid | acyl | CF₃ | O | 5-Fluorouracil |
| amino acid | acyl | CF₃ | O | 8-Fluoroguanine |
| amino acid | acyl | CF₃ | O | 5-Fluorocytosine |
| amino acid | acyl | CF₃ | O | 8-Fluoroadenine |
| amino acid | acyl | CF₃ | O | 2-Fluoroadenine |
| amino acid | acyl | CF₃ | O | 2,8-Difluoroadenine |
| amino acid | acyl | CF₃ | O | 2-Fluorohypoxanthine |
| amino acid | acyl | CF₃ | O | 8-Fluorohypoxanthine |
| amino acid | acyl | CF₃ | O | 2,8-Difluorohypoxanthine |
| amino acid | acyl | CF₃ | O | 2-Aminoadenine |
| amino acid | acyl | CF₃ | O | 2-Amino-8-fluoroadenine |
| amino acid | acyl | CF₃ | O | 2-Amino-8-fluorohypoxanthine |
| amino acid | acyl | CF₃ | O | 2-Aminohypoxanthine |
| amino acid | acyl | CF₃ | O | 2-N-acetylguanine |
| amino acid | acyl | CF₃ | O | 4-N-acetylcytosine |
| amino acid | acyl | CF₃ | O | 6-N-acetyladenine |
| amino acid | acyl | CF₃ | O | 2-N-acetyl-8-fluoroguanine |
| amino acid | acyl | CF₃ | O | 4-N-acetyl-5-fluorocytosine |
| amino acid | acyl | CF₃ | O | 6-N-acetyl-2-fluoroadenine |
| amino acid | acyl | CF₃ | O | 6-N-acetyl-2,8-difluoroadenine |
| amino acid | acyl | CF₃ | O | 6-N-acetyl-2-aminoadenine |
| amino acid | acyl | CF₃ | O | 6-N-acetyl-2-amino-8-fluoroadenine |
| amino acid | acyl | CF₃ | O | 2-N-acetylaminoadenine |
| amino acid | acyl | CF₃ | O | 2-N-acetylamino-8-fluoroadenine |
| amino acid | acyl | CF₃ | O | 2-N-acetylamino-8-fluorohypoxanthine |
| amino acid | acyl | CF₃ | O | 2-N-acetylaminohypoxanthine |

**Table 10**

| Insofar as the compounds in this table are encompassed by the compounds referred to in the enclosed claims, the present invention relates to such compounds. | | | | |
|---|---|---|---|---|
| **R²** | **R⁷** | **R⁶** | **X** | **Base** |
| acyl | H | CH₃ | O | Thymine |
| acyl | H | CH₃ | O | Uracil |
| acyl | H | CH₃ | O | Guanine |
| acyl | H | CH₃ | O | Cytosine |
| acyl | H | CH₃ | O | Adenine |
| acyl | H | CH₃ | O | Hypoxanthine |
| acyl | H | CH₃ | O | 5-Fluorouracil |
| acyl | H | CH₃ | O | 8-Fluoroguanine |
| acyl | H | CH₃ | O | 5-Fluorocytosine |
| acyl | H | CH₃ | O | 8-Fluoroadenine |
| acyl | H | CH₃ | O | 2-Fluoroadenine |
| acyl | H | CH₃ | O | 2,8-Difluoroadenine |
| acyl | H | CH₃ | O | 2-Fluorohypoxanthine |
| acyl | H | CH₃ | O | 8-Fluorohypoxanthine |
| acyl | H | CH₃ | O | 2,8-Difluorohypoxanthine |
| acyl | H | CH₃ | O | 2-Aminoadenine |
| acyl | H | CH₃ | O | 2-Amino-8-fluoroadenine |
| acyl | H | CH₃ | O | 2-Amino-8-fluorohypoxanthine |
| acyl | H | CH₃ | O | 2-Aminohypoxanthine |
| acyl | H | CH₃ | O | 2-N-acetylguanine |
| acyl | H | CH₃ | O | 4-N-acetvlcytosine |
| acyl | H | CH₃ | O | 6-N-acetyladenine |
| acyl | H | CH₃ | O | 2-N-acetyl-8-fluoroguanine |
| acyl | H | CH₃ | O | 4-N-acetyl-5-fluorocytosine |
| acyl | H | CH₃ | O | 6-N-acetyl-2-fluoroadenine |
| acyl | H | CH₃ | O | 6-N-acetyl-2,8-difluoroadenine |
| acyl | H | CH₃ | O | 6-N-acetyl-2-aminoadenine |
| acyl | H | CH₃ | O | 6-N-acetyl-2-amino-8-fluoroadenine |
| acyl | H | CH₃ | O | 2-N-acetylaminoadenine |
| acyl | H | CH₃ | O | 2-N-acetylamino-8-fluoroadenine |
| acyl | H | CH₃ | O | 2-N-acetylamino-8-fluorohypoxanthine |
| acyl | H | CH₃ | O | 2-N-acetylaminohypoxanthine |
| acyl | F | CH₃ | O | Thymine |
| acyl | F | CH₃ | O | Uracil |
| acyl | F | CH₃ | O | Guanine |
| acyl | F | CH₃ | O | Cytosine |
| acyl | F | CH₃ | O | Adenine |
| acyl | F | CH₃ | O | Hypoxanthine |
| acyl | F | CH₃ | O | 5-Fluorouracil |
| acyl | F | CH₃ | O | 8-Fluoroguanine |
| acyl | F | CH₃ | O | 5-Fluorocytosine |
| acyl | F | CH₃ | O | 8-Fluoroadenine |
| acyl | F | CH₃ | O | 2-Fluoroadenine |
| acyl | F | CH₃ | O | 2,8-Difluoroadenine |
| acyl | F | CH₃ | O | 2-Fluorohypoxanthine |
| acyl | F | CH₃ | O | 8-Fluorohypoxanthine |
| acyl | F | CH₃ | O | 2,8-Difluorohypoxanthine |
| acyl | F | CH₃ | O | 2-Aminoadenine |
| acyl | F | CH₃ | O | 2-Amino-8-fluoroadenine |
| acyl | F | CH₃ | O | 2-Amino-8-fluorohypoxanthine |
| acyl | F | CH₃ | O | 2-Aminohypoxanthine |
| acyl | F | CH₃ | O | 2-N-acetylguanine |
| acyl | F | CH₃ | O | 4-N-acetylcytosine |
| acyl | F | CH₃ | O | 6-N-acetyladenine |
| acyl | F | CH₃ | O | 2-N-acetyl-8-fluoroguanine |
| acyl | F | CH₃ | O | 4-N-acetyl-5-fluorocytosine |
| acyl | F | CH₃ | O | 6-N-acetyl-2-fluoroadenine |
| acyl | F | CH₃ | O | 6-N-acetyl-2,8-difluoroadenine |
| acyl | F | CH₃ | O | 6-N-acetyl-2-aminoadenine |
| acyl | F | CH₃ | O | 6-N-acetyl-2-amino-8-fluoroadenine |
| acyl | F | CH₃ | O | 2-N-acetylaminoadenine |
| acyl | F | CH₃ | O | 2-N-acetylamino-8-fluoroadenine |
| acyl | F | CH₃ | O | 2-N-acetylamino-8-fluorohypoxanthine |
| acyl | F | CH₃ | O | 2-N-acetylaminohypoxanthine |
| amino acid | H | CH₃ | O | Thymine |
| amino acid | H | CH₃ | O | Uracil |
| amino acid | H | CH₃ | O | Guanine |
| amino acid | H | CH₃ | O | Cytosine |
| amino acid | H | CH₃ | O | Adenine |
| amino acid | H | CH₃ | O | Hypoxanthine |
| amino acid | H | CH₃ | O | 5-Fluorouracil |
| amino acid | H | CH₃ | O | 8-Fluoroguanine |
| amino acid | H | CH₃ | O | 5-Fluorocytosine |
| amino acid | H | CH₃ | O | 8-Fluoroadenine |
| amino acid | H | CH₃ | O | 2-Fluoroadenine |
| amino acid | H | CH₃ | O | 2,8-Difluoroadenine |
| amino acid | H | CH₃ | O | 2-Fluorohypoxanthine |
| amino acid | H | CH₃ | O | 8-Fluorohypoxanthine |
| amino acid | H | CH₃ | O | 2,8-Difluorohypoxanthine |
| amino acid | H | CH₃ | O | 2-Aminoadenine |
| amino acid | H | CH₃ | O | 2-Amino-8-fluoroadenine |
| amino acid | H | CH₃ | O | 2-Amino-8-fluorohypoxanthine |
| amino acid | H | CH₃ | O | 2-Aminohypoxanthine |
| amino acid | H | CH₃ | O | 2-N-acetylguanine |
| amino acid | H | CH₃ | O | 4-N-acetylcytosine |
| amino acid | H | CH₃ | O | 6-N-acetyladenine |
| amino acid | H | CH₃ | O | 2-N-acetyl-8-fluoroguanine |
| amino acid | H | CH₃ | O | 4-N-acetyl-5-fluorocytosine |
| amino acid | H | CH₃ | O | 6-N-acetyl-2-fluoroadenine |
| amino acid | H | CH₃ | O | 6-N-acetyl-2,8-difluoroadenine |
| amino acid | H | CH₃ | O | 6-N-acetyl-2-aminoadenine |
| amino acid | H | CH₃ | O | 6-N-acetyl-2-amino-8-fluoroadenine |
| amino acid | H | CH₃ | O | 2-N-acetylaminoadenine |
| amino acid | H | CH₃ | O | 2-N-acetylamino-8-fluoroadenine |
| amino acid | H | CH₃ | O | 2-N-acetylamino-8-fluorohypoxanthine |
| amino acid | H | CH₃ | O | 2-N-acetylaminohypoxanthine |
| amino acid | F | CH₃ | O | Thymine |
| amino acid | F | CH₃ | O | Uracil |
| amino acid | F | CH₃ | O | Guanine |
| amino acid | F | CH₃ | O | Cytosine |
| amino acid | F | CH₃ | O | Adenine |
| amino acid | F | CH₃ | O | Hypoxanthine |
| amino acid | F | CH₃ | O | 5-Fluorouracil |
| amino acid | F | CH₃ | O | 8-Fluoroguanine |
| amino acid | F | CH₃ | O | 5-Fluorocytosine |
| amino acid | F | CH₃ | O | 8-Fluoroadenine |
| amino acid | F | CH₃ | O | 2-Fluoroadenine |
| amino acid | F | CH₃ | O | 2,8-Difluoroadenine |
| amino acid | F | CH₃ | O | 2-Fluorohypoxanthine |
| amino acid | F | CH₃ | O | 8-Fluorohypoxanthine |
| amino acid | F | CH₃ | O | 2,8-Difluorohypoxanthine |
| amino acid | F | CH₃ | O | 2-Aminoadenine |
| amino acid | F | CH₃ | O | 2-Amino-8-fluoroadenine |
| amino acid | F | CH₃ | O | 2-Amino-8-fluorohypoxanthine |
| amino acid | F | CH₃ | O | 2-Aminohypoxanthine |
| amino acid | F | CH₃ | O | 2-N-acetylguanine |
| amino acid | F | CH₃ | O | 4-N-acetylcytosine |
| amino acid | F | CH₃ | O | 6-N-acetyladenine |
| amino acid | F | CH₃ | O | 2-N-acetyl-8-fluoroguanine |
| amino acid | F | CH₃ | O | 4-N-acetyl-5-fluorocytosine |
| amino acid | F | CH₃ | O | 6-N-acetyl-2-fluoroadenine |
| amino acid | F | CH₃ | O | 6-N-acetyl-2,8-difluoroadenine |
| amino acid | F | CH₃ | O | 6-N-acetyl-2-aminoadenine |
| amino acid | F | CH₃ | O | 6-N-acetyl-2-amino-8-fluoroadenine |
| amino acid | F | CH₃ | O | 2-N-acetylaminoadenine |
| amino acid | F | CH₃ | O | 2-N-acetylamino-8-fluoroadenine |
| amino acid | F | CH₃ | O | 2-N-acetylamino-8-fluorohypoxanthine |
| amino acid | F | CH₃ | O | 2-N-acetylaminohypoxanthine |
| acyl | H | CH₃ | S | Thymine |
| acyl | H | CH₃ | S | Uracil |
| acyl | H | CH₃ | S | Guanine |
| acyl | H | CH₃ | S | Cytosine |
| acyl | H | CH₃ | S | Adenine |
| acyl | H | CH₃ | S | Hypoxanthine |
| acyl | H | CH₃ | S | 5-Fluorouracil |
| acyl | H | CH₃ | S | 8-Fluoroguanine |
| acyl | H | CH₃ | S | 5-Fluorocytosine |
| acyl | H | CH₃ | S | 8-Fluoroadenine |
| acyl | H | CH₃ | S | 2-Fluoroadenine |
| acyl | H | CH₃ | S | 2,8-Difluoroadenine |
| acyl | H | CH₃ | S | 2-Fluorohypoxanthine |
| acyl | H | CH₃ | S | 8-Fluorohypoxanthine |
| acyl | H | CH₃ | S | 2,8-Difluorohypoxanthine |
| acyl | H | CH₃ | S | 2-Aminoadenine |
| acyl | H | CH₃ | S | 2-Amino-8-fluoroadenine |
| acyl | H | CH₃ | S | 2-Amino-8-fluorohypoxanthine |
| acyl | H | CH₃ | S | 2-Aminohypoxanthine |
| acyl | H | CH₃ | S | 2-N-acetylguanine |
| acyl | H | CH₃ | S | 4-N-acetylcytosine |
| acyl | H | CH₃ | S | 6-N-acetyladenine |
| acyl | H | CH₃ | S | 2-N-acetyl-8-fluoroguanine |
| acyl | H | CH₃ | S | 4-N-acetyl-5-fluorocytosine |
| acyl | H | CH₃ | S | 6-N-acetyl-2-fluoroadenine |
| acyl | H | CH₃ | S | 6-N-acetyl-2,8-difluoroadenine |
| acyl | H | CH₃ | S | 6-N-acetyl-2-aminoadenine |
| acyl | H | CH₃ | S | 6-N-acetyl-2-amino-8-fluoroadenine |
| acyl | H | CH₃ | S | 2-N-acetylaminoadenine |
| acyl | H | CH₃ | S | 2-N-acetylamino-8-fluoroadenine |
| acyl | H | CH₃ | S | 2-N-acetylamino-8-fluorohypoxanthine |
| acyl | H | CH₃ | S | 2-N-acetylaminohypoxanthine |
| acyl | F | CH₃ | S | Thymine |
| acyl | F | CH₃ | S | Uracil |
| acyl | F | CH₃ | S | Guanine |
| acyl | F | CH₃ | S | Cytosine |
| acyl | F | CH₃ | S | Adenine |
| acyl | F | CH₃ | S | Hypoxanthine |
| acyl | F | CH₃ | S | 5-Fluorouracil |
| acyl | F | CH₃ | S | 8-Fluoroguanine |
| acyl | F | CH₃ | S | 5-Fluorocytosine |
| acyl | F | CH₃ | S | 8-Fluoroadenine |
| acyl | F | CH₃ | S | 2-Fluoroadenine |
| acyl | F | CH₃ | S | 2,8-Difluoroadenine |
| acyl | F | CH₃ | S | 2-Fluorohypoxanthine |
| acyl | F | CH₃ | S | 8-Fluorohypoxanthine |
| acyl | F | CH₃ | S | 2,8-Difluorohypoxanthine |
| acyl | F | CH₃ | S | 2-Aminoadenine |
| acyl | F | CH₃ | S | 2-Amino-8-fluoroadenine |
| acyl | F | CH₃ | S | 2-Amino-8-fluorohypoxanthine |
| acyl | F | CH₃ | S | 2-Aminohypoxanthine |
| acyl | F | CH₃ | S | 2-N-acetylguanine |
| acyl | F | CH₃ | S | 4-N-acetylcytosine |
| acyl | F | CH₃ | S | 6-N-acetyladenine |
| acyl | F | CH₃ | S | 2-N-acetyl-8-fluoroguanine |
| acyl | F | CH₃ | S | 4-N-acetyl-5-fluorocytosine |
| acyl | F | CH₃ | S | 6-N-acetyl-2-fluoroadenine |
| acyl | F | CH₃ | S | 6-N-acetyl-2,8-difluoroadenine |
| acyl | F | CH₃ | S | 6-N-acetyl-2-aminoadenine |
| acyl | F | CH₃ | S | 6-N-acetyl-2-amino-8-fluoroadenine |
| acyl | F | CH₃ | S | 2-N-acetylaminoadenine |
| acyl | F | CH₃ | S | 2-N-acetylamino-8-fluoroadenine |
| acyl | F | CH₃ | S | 2-N-acetylamino-8-fluorohypoxanthine |
| acyl | F | CH₃ | S | 2-N-acetylaminohypoxanthine |
| amino acid | F | CH₃ | S | Thymine |
| amino acid | F | CH₃ | S | Uracil |
| amino acid | F | CH₃ | S | Guanine |
| amino acid | F | CH₃ | S | Cytosine |
| amino acid | F | CH₃ | S | Adenine |
| amino acid | F | CH₃ | S | Hypoxanthine |
| amino acid | F | CH₃ | S | 5-Fluorouracil |
| amino acid | F | CH₃ | S | 8-Fluoroguanine |
| amino acid | F | CH₃ | S | 5-Fluorocytosine |
| amino acid | F | CH₃ | S | 8-Fluoroadenine |
| amino acid | F | CH₃ | S | 2-Fluoroadenine |
| amino acid | F | CH₃ | S | 2,8-Difluoroadenine |
| amino acid | F | CH₃ | S | 2-Fluorohypoxanthine |
| amino acid | F | CH₃ | S | 8-Fluorohypoxanthine |
| amino acid | F | CH₃ | S | 2,8-Difluorohypoxanthine |
| amino acid | F | CH₃ | S | 2-Aminoadenine |
| amino acid | F | CH₃ | S | 2-Amino-8-fluoroadenine |
| amino acid | F | CH₃ | S | 2-Amino-8-fluorohypoxanthine |
| amino acid | F | CH₃ | S | 2-Aminohypoxanthine |
| amino acid | F | CH₃ | S | 2-N-acetylguanine |
| amino acid | F | CH₃ | S | 4-N-acetylcytosine |
| amino acid | F | CH₃ | S | 6-N-acetyladenine |
| amino acid | F | CH₃ | S | 2-N-acetyl-8-fluoroguanine |
| amino acid | F | CH₃ | S | 4-N-acetyl-5-fluorocytosine |
| amino acid | F | CH₃ | S | 6-N-acetyl-2-fluoroadenine |
| amino acid | F | CH₃ | S | 6-N-acetyl-2,8-difluoroadenine |
| amino acid | F | CH₃ | S | 6-N-acetyl-2-aminoadenine |
| amino acid | F | CH₃ | S | 6-N-acetyl-2-amino-8-fluoroadenine |
| amino acid | F | CH₃ | S | 2-N-acetylaminoadenine |
| amino acid | F | CH₃ | S | 2-N-acetylamino-8-fluoroadenine |
| amino acid | F | CH₃ | S | 2-N-acetylamino-8-fluorohypoxanthine |
| amino acid | F | CH₃ | S | 2-N-acetylaminohypoxanthine |
| amino acid | H | CH₃ | S | Thymine |
| amino acid | H | CH₃ | S | Uracil |
| amino acid | H | CH₃ | S | Guanine |
| amino acid | H | CH₃ | S | Cytosine |
| amino acid | H | CH₃ | S | Adenine |
| amino acid | H | CH₃ | S | Hypoxanthine |
| amino acid | H | CH₃ | S | 5-Fluorouracil |
| amino acid | H | CH₃ | S | 8-Fluoroguanine |
| amino acid | H | CH₃ | S | 5-Fluorocytosine |
| amino acid | H | CH₃ | S | 8-Fluoroadenine |
| amino acid | H | CH₃ | S | 2-Fluoroadenine |
| amino acid | H | CH₃ | S | 2,8-Difluoroadenine |
| amino acid | H | CH₃ | S | 2-Fluorohypoxanthine |
| amino acid | H | CH₃ | S | 8-Fluorohypoxanthine |
| amino acid | H | CH₃ | S | 2,8-Difluorohypoxanthine |
| amino acid | H | CH₃ | S | 2-Aminoadenine |
| amino acid | H | CH₃ | S | 2-Amino-8-fluoroadenine |
| amino acid | H | CH₃ | S | 2-Amino-8-fluorohypoxanthine |
| amino acid | H | CH₃ | S | 2-Aminohypoxanthine |
| amino acid | H | CH₃ | S | 2-N-acetylguanine |
| amino acid | H | CH₃ | S | 4-N-acetylcytosine |
| amino acid | H | CH₃ | S | 6-N-acetyladenine |
| amino acid | H | CH₃ | S | 2-N-acetyl-8-fluoroguanine |
| amino acid | H | CH₃ | S | 4-N-acetyl-5-fluorocytosine |
| amino acid | H | CH₃ | S | 6-N-acetyl-2-fluoroadenine |
| amino acid | H | CH₃ | S | 6-N-acetyl-2,8-difluoroadenine |
| amino acid | H | CH₃ | S | 6-N-acetyl-2-aminoadenine |
| amino acid | H | CH₃ | S | 6-N-acetyl-2-amino-8-fluoroadenine |
| amino acid | H | CH₃ | S | 2-N-acetylaminoadenine |
| amino acid | H | CH₃ | S | 2-N-acetylamino-8-fluoroadenine |
| amino acid | H | CH₃ | S | 2-N-acetylamino-8-fluorohypoxanthine |
| amino acid | H | CH₃ | S | 2-N-acetylaminohypoxanthine |
| acyl | H | CF₃ | S | Thymine |
| acyl | H | CF₃ | S | Uracil |
| acyl | H | CF₃ | S | Guanine |
| acyl | H | CF₃ | S | Cytosine |
| acyl | H | CF₃ | S | Adenine |
| acyl | H | CF₃ | S | Hypoxanthine |
| acyl | H | CF₃ | S | 5-Fluorouracil |
| acyl | H | CF₃ | S | 8-Fluoroguanine |
| acyl | H | CF₃ | S | 5-Fluorocytosine |
| acyl | H | CF₃ | S | 8-Fluoroadenine |
| acyl | H | CF₃ | S | 2-Fluoroadenine |
| acyl | H | CF₃ | S | 2,8-Difluoroadenine |
| acyl | H | CF₃ | S | 2-Fluorohypoxanthine |
| acyl | H | CF₃ | S | 8-Fluorohypoxanthine |
| acyl | H | CF₃ | S | 2,8-Difluorohypoxanthine |
| acyl | H | CF₃ | S | 2-Aminoadenine |
| acyl | H | CF₃ | S | 2-Amino-8-fluoroadenine |
| acyl | H | CF₃ | S | 2-Amino-8-fluorohypoxanthine |
| acyl | H | CF₃ | S | 2-Aminohypoxanthine |
| acyl | H | CF₃ | S | 2-N-acetvlguanine |
| acyl | H | CF₃ | S | 4-N-acetylcytosine |
| acyl | H | CF₃ | S | 6-N-acetyladenine |
| acyl | H | CF₃ | S | 2-N-acetyl-8-fluoroguanine |
| acyl | H | CF₃ | S | 4-N-acetyl-5-fluorocytosine |
| acyl | H | CF₃ | S | 6-N-acetyl-2-fluoroadenine |
| acyl | H | CF₃ | S | 6-N-acetyl-2,8-difluoroadenine |
| acyl | H | CF₃ | S | 6-N-acetyl-2-aminoadenine |
| acyl | H | CF₃ | S | 6-N-acetyl-2-amino-8-fluoroadenine |
| acyl | H | CF₃ | S | 2-N-acetylaminoadenine |
| acyl | H | CF₃ | S | 2-N-acetylamino-8-fluoroadenine |
| acyl | H | CF₃ | S | 2-N-acetylamino-8-fluorohypoxanthine |
| acyl | H | CF₃ | S | 2-N-acetylaminohypoxanthine |
| acyl | F | CF₃ | S | Thymine |
| acyl | F | CF₃ | S | Uracil |
| acyl | F | CF₃ | S | Guanine |
| acyl | F | CF₃ | S | Cytosine |
| acyl | F | CF₃ | S | Adenine |
| acyl | F | CF₃ | S | Hypoxanthine |
| acyl | F | CF₃ | S | 5-Fluorouracil |
| acyl | F | CF₃ | S | 8-Fluoroguanine |
| acyl | F | CF₃ | S | 5-Fluorocytosine |
| acyl | F | CF₃ | S | 8-Fluoroadenine |
| acyl | F | CF₃ | S | 2-Fluoroadenine |
| acyl | F | CF₃ | S | 2,8-Difluoroadenine |
| acyl | F | CF₃ | S | 2-Fluorohypoxanthine |
| acyl | F | CF₃ | S | 8-Fluorohypoxanthine |
| acyl | F | CF₃ | S | 2,8-Difluorohypoxanthine |
| acyl | F | CF₃ | S | 2-Aminoadenine |
| acyl | F | CF₃ | S | 2-Amino-8-fluoroadenine |
| acyl | F | CF₃ | S | 2-Amino-8-fluorohypoxanthine |
| acyl | F | CF₃ | S | 2-Aminohypoxanthine |
| acyl | F | CF₃ | S | 2-N-acetylguanine |
| acyl | F | CF₃ | S | 4-N-acetylcytosine |
| acyl | F | CF₃ | S | 6-N-acetyladenine |
| acyl | F | CF₃ | S | 2-N-acetyl-8-fluoroguanine |
| acyl | F | CF₃ | S | 4-N-acetyl-5-fluorocytosine |
| acyl | F | CF₃ | S | 6-N-acetyl-2-fluoroadenine |
| acyl | F | CF₃ | S | 6-N-acetyl-2,8-difluoroadenine |
| acyl | F | CF₃ | S | 6-N-acetyl-2-aminoadenine |
| acyl | F | CF₃ | S | 6-N-acetyl-2-amino-8-fluoroadenine |
| acyl | F | CF₃ | S | 2-N-acetylaminoadenine |
| acyl | F | CF₃ | S | 2-N-acetylamino-8-fluoroadenine |
| acyl | F | CF₃ | S | 2-N-acetylamino-8-fluorohypoxanthine |
| acyl | F | CF₃ | S | 2-N-acetylaminohypoxanthine |
| amino acid | H | CF₃ | S | Thymine |
| amino acid | H | CF₃ | S | Uracil |
| amino acid | H | CF₃ | S | Guanine |
| amino acid | H | CF₃ | S | Cytosine |
| amino acid | H | CF₃ | S | Adenine |
| amino acid | H | CF₃ | S | Hypoxanthine |
| amino acid | H | CF₃ | S | 5-Fluorouracil |
| amino acid | H | CF₃ | S | 8-Fluoroguanine |
| amino acid | H | CF₃ | S | 5-Fluorocytosine |
| amino acid | H | CF₃ | S | 8-Fluoroadenine |
| amino acid | H | CF₃ | S | 2-Fluoroadenine |
| amino acid | H | CF₃ | S | 2,8-Difluoroadenine |
| amino acid | H | CF₃ | S | 2-Fluorohypoxanthine |
| amino acid | H | CF₃ | S | 8-Fluorohypoxanthine |
| amino acid | H | CF₃ | S | 2,8-Difluorohypoxanthine |
| amino acid | H | CF₃ | S | 2-Aminoadenine |
| amino acid | H | CF₃ | S | 2-Amino-8-fluoroadenine |
| amino acid | H | CF₃ | S | 2-Amino-8-fluorohypoxanthine |
| amino acid | H | CF₃ | S | 2-Aminohypoxanthine |
| amino acid | H | CF₃ | S | 2-N-acetylguanine |
| amino acid | H | CF₃ | S | 4-N-acetylcytosine |
| amino acid | H | CF₃ | S | 6-N-acetyladenine |
| amino acid | H | CF₃ | S | 2-N-acetyl-8-fluoroguanine |
| amino acid | H | CF₃ | S | 4-N-acetyl-5-fluorocytosine |
| amino acid | H | CF₃ | S | 6-N-acetyl-2-fluoroadenine |
| amino acid | H | CF₃ | S | 6-N-acetyl-2,8-difluoroadenine |
| amino acid | H | CF₃ | S | 6-N-acetyl-2-aminoadenine |
| amino acid | H | CF₃ | S | 6-N-acetyl-2-amino-8-fluoroadenine |
| amino acid | H | CF₃ | S | 2-N-acetylaminoadenine |
| amino acid | H | CF₃ | S | 2-N-acetylamino-8-fluoroadenine |
| amino acid | H | CF₃ | S | 2-N-acetylamino-8-fluorohypoxanthine |
| amino acid | H | CF₃ | S | 2-N-acetylaminohypoxanthine |
| amino acid | F | CF₃ | S | Thymine |
| amino acid | F | CF₃ | S | Uracil |
| amino acid | F | CF₃ | S | Guanine |
| amino acid | F | CF₃ | S | Cytosine |
| amino acid | F | CF₃ | S | Adenine |
| amino acid | F | CF₃ | S | Hypoxanthine |
| amino acid | F | CF₃ | S | 5-Fluorouracil |
| amino acid | F | CF₃ | S | 8-Fluoroguanine |
| amino acid | F | CF₃ | S | 5-Fluorocytosine |
| amino acid | F | CF₃ | S | 8-Fluoroadenine |
| amino acid | F | CF₃ | S | 2-Fluoroadenine |
| amino acid | F | CF₃ | S | 2,8-Difluoroadenine |
| amino acid | F | CF₃ | S | 2-Fluorohypoxanthine |
| amino acid | F | CF₃ | S | 8-Fluorohypoxanthine |
| amino acid | F | CF₃ | S | 2,8-Difluorohypoxanthine |
| amino acid | F | CF₃ | S | 2-Aminoadenine |
| amino acid | F | CF₃ | S | 2-Amino-8-fluoroadenine |
| amino acid | F | CF₃ | S | 2-Amino-8-fluorohypoxanthine |
| amino acid | F | CF₃ | S | 2-Aminohypoxanthine |
| amino acid | F | CF₃ | S | 2-N-acetylguanine |
| amino acid | F | CF₃ | S | 4-N-acetylcytosine |
| amino acid | F | CF₃ | S | 6-N-acetyladenine |
| amino acid | F | CF₃ | S | 2-N-acetyl-8-fluoroguanine |
| amino acid | F | CF₃ | S | 4-N-acetyl-5-fluorocytosine |
| amino acid | F | CF₃ | S | 6-N-acetyl-2-fluoroadenine |
| amino acid | F | CF₃ | S | 6-N-acetyl-2,8-difluoroadenine |
| amino acid | F | CF₃ | S | 6-N-acetyl-2-aminoadenine |
| amino acid | F | CF₃ | S | 6-N-acetyl-2-amino-8-fluoroadenine |
| amino acid | F | CF₃ | S | 2-N-acetylaminoadenine |
| amino acid | F | CF₃ | S | 2-N-acetylamino-8-fluoroadenine |
| amino acid | F | CF₃ | S | 2-N-acetylamino-8-fluorohypoxanthine |
| amino acid | F | CF₃ | S | 2-N-acetylaminohypoxanthine |
| acyl | H | CF₃ | O | Thymine |
| acyl | H | CF₃ | O | Uracil |
| acyl | H | CF₃ | O | Guanine |
| acyl | H | CF₃ | O | Cytosine |
| acyl | H | CF₃ | O | Adenine |
| acyl | H | CF₃ | O | Hypoxanthine |
| acyl | H | CF₃ | O | 5-Fluorouracil |
| acyl | H | CF₃ | O | 8-Fluoroguanine |
| acyl | H | CF₃ | O | 5-Fluorocytosine |
| acyl | H | CF₃ | O | 8-Fluoroadenine |
| acyl | H | CF₃ | O | 2-Fluoroadenine |
| acyl | H | CF₃ | O | 2,8-Difluoroadenine |
| acyl | H | CF₃ | O | 2-Fluorohypoxanthine |
| acyl | H | CF₃ | O | 8-Fluorohypoxanthine |
| acyl | H | CF₃ | O | 2,8-Difluorohypoxanthine |
| acyl | H | CF₃ | O | 2-Aminoadenine |
| acyl | H | CF₃ | O | 2-Amino-8-fluoroadenine |
| acyl | H | CF₃ | O | 2-Amino-8-fluorohypoxanthine |
| acyl | H | CF₃ | O | 2-Aminohypoxanthine |
| acyl | H | CF₃ | O | 2-N-acetylguanine |
| acyl | H | CF₃ | O | 4-N-acetylcytosine |
| acyl | H | CF₃ | O | 6-N-acetyladenine |
| acyl | H | CF₃ | O | 2-N-acetyl-8-fluoroguanine |
| acyl | H | CF₃ | O | 4-N-acetyl-5-fluorocytosine |
| acyl | H | CF₃ | O | 6-N-acetyl-2-fluoroadenine |
| acyl | H | CF₃ | O | 6-N-acetyl-2,8-difluoroadenine |
| acyl | H | CF₃ | O | 6-N-acetyl-2-aminoadenine |
| acyl | H | CF₃ | O | 6-N-acetyl-2-amino-8-fluoroadenine |
| acyl | H | CF₃ | O | 2-N-acetylaminoadenine |
| acyl | H | CF₃ | O | 2-N-acetylamino-8-fluoroadenine |
| acyl | H | CF₃ | O | 2-N-acetylamino-8-fluorohypoxanthine |
| acyl | H | CF₃ | O | 2-N-acetylaminohypoxanthine |
| acyl | F | CF₃ | O | Thymine |
| acyl | F | CF₃ | O | Uracil |
| acyl | F | CF₃ | O | Guanine |
| acyl | F | CF₃ | O | Cytosine |
| acyl | F | CF₃ | O | Adenine |
| acyl | F | CF₃ | O | Hypoxanthine |
| acyl | F | CF₃ | O | 5-Fluorouracil |
| acyl | F | CF₃ | O | 8-Fluoroguanine |
| acyl | F | CF₃ | O | 5-Fluorocytosine |
| acyl | F | CF₃ | O | 8-Fluoroadenine |
| acyl | F | CF₃ | O | 2-Fluoroadenine |
| acyl | F | CF₃ | O | 2,8-Difluoroadenine |
| acyl | F | CF₃ | O | 2-Fluorohypoxanthine |
| acyl | F | CF₃ | O | 8-Fluorohypoxanthine |
| acyl | F | CF₃ | O | 2,8-Difluorohypoxanthine |
| acyl | F | CF₃ | O | 2-Aminoadenine |
| acyl | F | CF₃ | O | 2-Amino-8-fluoroadenine |
| acyl | F | CF₃ | O | 2-Amino-8-fluorohypoxanthine |
| acyl | F | CF₃ | O | 2-Aminohypoxanthine |
| acyl | F | CF₃ | O | 2-N-acetylguanine |
| acyl | F | CF₃ | O | 4-N-acetylcytosine |
| acyl | F | CF₃ | O | 6-N-acetyladenine |
| acyl | F | CF₃ | O | 2-N-acetyl-8-fluoroguanine |
| acyl | F | CF₃ | O | 4-N-acetyl-5-fluorocytosine |
| acyl | F | CF₃ | O | 6-N-acetyl-2-fluoroadenine |
| acyl | F | CF₃ | O | 6-N-acetyl-2,8-difluoroadenine |
| acyl | F | CF₃ | O | 6-N-acetyl-2-aminoadenine |
| acyl | F | CF₃ | O | 6-N-acetyl-2-amino-8-fluoroadenine |
| acyl | F | CF₃ | O | 2-N-acetylaminoadenine |
| acyl | F | CF₃ | O | 2-N-acetylamino-8-fluoroadenine |
| acyl | F | CF₃ | O | 2-N-acetylamino-8-fluorohypoxanthine |
| acyl | F | CF₃ | O | 2-N-acetylaminohypoxanthine |
| amino acid | F | CF₃ | O | Thymine |
| amino acid | F | CF₃ | O | Uracil |
| amino acid | F | CF₃ | O | Guanine |
| amino acid | F | CF₃ | O | Cytosine |
| amino acid | F | CF₃ | O | Adenine |
| amino acid | F | CF₃ | O | Hypoxanthine |
| amino acid | F | CF₃ | O | 5-Fluorouracil |
| amino acid | F | CF₃ | O | 8-Fluoroguanine |
| amino acid | F | CF₃ | O | 5-Fluorocytosine |
| amino acid | F | CF₃ | O | 8-Fluoroadenine |
| amino acid | F | CF₃ | O | 2-Fluoroadenine |
| amino acid | F | CF₃ | O | 2,8-Difluoroadenine |
| amino acid | F | CF₃ | O | 2-Fluorohypoxanthine |
| amino acid | F | CF₃ | O | 8-Fluorohypoxanthine |
| amino acid | F | CF₃ | O | 2,8-Difluorohypoxanthine |
| amino acid | F | CF₃ | O | 2-Aminoadenine |
| amino acid | F | CF₃ | O | 2-Amino-8-fluoroadenine |
| amino acid | F | CF₃ | O | 2-Amino-8-fluorohypoxanthine |
| amino acid | F | CF₃ | O | 2-Aminohypoxanthine |
| amino acid | F | CF₃ | O | 2-N-acetylguanine |
| amino acid | F | CF₃ | O | 4-N-acetylcytosine |
| amino acid | F | CF₃ | O | 6-N-acetyladenine |
| amino acid | F | CF₃ | O | 2-N-acetyl-8-fluoroguanine |
| amino acid | F | CF₃ | O | 4-N-acetyl-5-fluorocytosine |
| amino acid | F | CF₃ | O | 6-N-acetyl-2-fluoroadenine |
| amino acid | F | CF₃ | O | 6-N-acetyl-2,8-difluoroadenine |
| amino acid | F | CF₃ | O | 6-N-acetyl-2-aminoadenine |
| amino acid | F | CF₃ | O | 6-N-acetyl-2-amino-8-fluoroadenine |
| amino acid | F | CF₃ | O | 2-N-acetylaminoadenine |
| amino acid | F | CF₃ | O | 2-N-acetylamino-8-fluoroadenine |
| amino acid | F | CF₃ | O | 2-N-acetylamino-8-fluorohypoxanthine |
| amino acid | F | CF₃ | O | 2-N-acetylaminohypoxanthine |
| amino acid | H | CF₃ | O | Thymine |
| amino acid | H | CF₃ | O | Uracil |
| amino acid | H | CF₃ | O | Guanine |
| amino acid | H | CF₃ | O | Cytosine |
| amino acid | H | CF₃ | O | Adenine |
| amino acid | H | CF₃ | O | Hypoxanthine |
| amino acid | H | CF₃ | O | 5-Fluorouracil |
| amino acid | H | CF₃ | O | 8-Fluoroguanine |
| amino acid | H | CF₃ | O | 5-Fluorocytosine |
| amino acid | H | CF₃ | O | 8-Fluoroadenine |
| amino acid | H | CF₃ | O | 2-Fluoroadenine |
| amino acid | H | CF₃ | O | 2,8-Difluoroadenine |
| amino acid | H | CF₃ | O | 2-Fluorohypoxanthine |
| amino acid | H | CF₃ | O | 8-Fluorohypoxanthine |
| amino acid | H | CF₃ | O | 2,8-Difluorohypoxanthine |
| amino acid | H | CF₃ | O | 2-Aminoadenine |
| amino acid | H | CF₃ | O | 2-Amino-8-fluoroadenine |
| amino acid | H | CF₃ | O | 2-Amino-8-fluorohypoxanthine |
| amino acid | H | CF₃ | O | 2-Aminohypoxanthine |
| amino acid | H | CF₃ | O | 2-N-acetylguanine |
| amino acid | H | CF₃ | O | 4-N-acetylcytosine |
| amino acid | H | CF₃ | O | 6-N-acetyladenine |
| amino acid | H | CF₃ | O | 2-N-acetyl-8-fluoroguanine |
| amino acid | H | CF₃ | O | 4-N-acetyl-5-fluorocytosine |
| amino acid | H | CF₃ | O | 6-N-acetyl-2-fluoroadenine |
| amino acid | H | CF₃ | O | 6-N-acetyl-2,8-difluoroadenine |
| amino acid | H | CF₃ | O | 6-N-acetyl-2-aminoadenine |
| amino acid | H | CF₃ | O | 6-N-acetyl-2-amino-8-fluoroadenine |
| amino acid | H | CF₃ | O | 2-N-acetylaminoadenine |
| amino acid | H | CF₃ | O | 2-N-acetylamino-8-fluoroadenine |
| amino acid | H | CF₃ | O | 2-N-acetylamino-8-fluorohypoxanthine |
| amino acid | H | CF₃ | O | 2-N-acetylaminohypoxanthine |
| acyl | OH | CH₃ | O | Thymine |
| acyl | OH | CH₃ | O | Uracil |
| acyl | OH | CH₃ | O | Guanine |
| acyl | OH | CH₃ | O | Cytosine |
| acyl | OH | CH₃ | O | Adenine |
| acyl | OH | CH₃ | O | Hypoxanthine |
| acyl | OH | CH₃ | O | 5-Fluorouracil |
| acyl | OH | CH₃ | O | 8-Fluoroguanine |
| acyl | OH | CH₃ | O | 5-Fluorocytosine |
| acyl | OH | CH₃ | O | 8-Fluoroadenine |
| acyl | OH | CH₃ | O | 2-Fluoroadenine |
| acyl | OH | CH₃ | O | 2,8-Difluoroadenine |
| acyl | OH | CH₃ | O | 2-Fluorohypoxanthine |
| acyl | OH | CH₃ | O | 8-Fluorohypoxanthine |
| acyl | OH | CH₃ | O | 2,8-Difluorohypoxanthine |
| acyl | OH | CH₃ | O | 2-Aminoadenine |
| acyl | OH | CH₃ | O | 2-Amino-8-fluoroadenine |
| acyl | OH | CH₃ | O | 2-Amino-8-fluorohypoxanthine |
| acyl | OH | CH₃ | O | 2-Aminohypoxanthine |
| acyl | OH | CH₃ | O | 2-N-acetylguanine |
| acyl | OH | CH₃ | O | 4-N-acetylcytosine |
| acyl | OH | CH₃ | O | 6-N-acetyladenine |
| acyl | OH | CH₃ | O | 2-N-acetyl-8-fluoroguanine |
| acyl | OH | CH₃ | O | 4-N-acetyl-5-fluorocytosine |
| acyl | OH | CH₃ | O | 6-N-acetyl-2-fluoroadenine |
| acyl | OH | CH₃ | O | 6-N-acetyl-2,8-difluoroadenine |
| acyl | OH | CH₃ | O | 6-N-acetyl-2-aminoadenine |
| acyl | OH | CH₃ | O | 6-N-acetyl-2-amino-8-fluoroadenine |
| acyl | OH | CH₃ | O | 2-N-acetylaminoadenine |
| acyl | OH | CH₃ | O | 2-N-acetylamino-8-fluoroadenine |
| acyl | OH | CH₃ | O | 2-N-acetylamino-8-fluorohypoxanthine |
| acyl | OH | CH₃ | O | 2-N-acetylaminohypoxanthine |
| acyl | OH | CH₃ | S | Thymine |
| acyl | OH | CH₃ | S | Uracil |
| acyl | OH | CH₃ | S | Guanine |
| acyl | OH | CH₃ | S | Cytosine |
| acyl | OH | CH₃ | S | Adenine |
| acyl | OH | CH₃ | S | Hypoxanthine |
| acyl | OH | CH₃ | S | 5-Fluorouracil |
| acyl | OH | CH₃ | S | 8-Fluoroguanine |
| acyl | OH | CH₃ | S | 5-Fluorocytosine |
| acyl | OH | CH₃ | S | 8-Fluoroadenine |
| acyl | OH | CH₃ | S | 2-Fluoroadenine |
| acyl | OH | CH₃ | S | 2,8-Difluoroadenine |
| acyl | OH | CH₃ | S | 2-Fluorohypoxanthine |
| acyl | OH | CH₃ | S | 8-Fluorohypoxanthine |
| acyl | OH | CH₃ | S | 2,8-Difluorohypoxanthine |
| acyl | OH | CH₃ | S | 2-Aminoadenine |
| acyl | OH | CH₃ | S | 2-Amino-8-fluoroadenine |
| acyl | OH | CH₃ | S | 2-Amino-8-fluorohypoxanthine |
| acyl | OH | CH₃ | S | 2-Aminohypoxanthine |
| acyl | OH | CH₃ | S | 2-N-acetylguanine |
| acyl | OH | CH₃ | S | 4-N-acetylcytosine |
| acyl | OH | CH₃ | S | 6-N-acetyladenine |
| acyl | OH | CH₃ | S | 2-N-acetyl-8-fluoroguanine |
| acyl | OH | CH₃ | S | 4-N-acetyl-5-fluorocytosine |
| acyl | OH | CH₃ | S | 6-N-acetyl-2-fluoroadenine |
| acyl | OH | CH₃ | S | 6-N-acetyl-2,8-difluoroadenine |
| acyl | OH | CH₃ | S | 6-N-acetyl-2-aminoadenine |
| acyl | OH | CH₃ | S | 6-N-acetyl-2-amino-8-fluoroadenine |
| acyl | OH | CH₃ | S | 2-N-acetylaminoadenine |
| acyl | OH | CH₃ | S | 2-N-acetylamino-8-fluoroadenine |
| acyl | OH | CH₃ | S | 2-N-acetylamino-8-fluorohypoxanthine |
| acyl | OH | CH₃ | S | 2-N-acetylaminohypoxanthine |
| acyl | OH | CF₃ | O | Thymine |
| acyl | OH | CF₃ | O | Uracil |
| acyl | OH | CF₃ | O | Guanine |
| acyl | OH | CF₃ | O | Cytosine |
| acyl | OH | CF₃ | O | Adenine |
| acyl | OH | CF₃ | O | Hypoxanthine |
| acyl | OH | CF₃ | O | 5-Fluorouracil |
| acyl | OH | CF₃ | O | 8-Fluoroguanine |
| acyl | OH | CF₃ | O | 5-Fluorocytosine |
| acyl | OH | CF₃ | O | 8-Fluoroadenine |
| acyl | OH | CF₃ | O | 2-Fluoroadenine |
| acyl | OH | CF₃ | O | 2,8-Difluoroadenine |
| acyl | OH | CF₃ | O | 2-Fluorohypoxanthine |
| acyl | OH | CF₃ | O | 8-Fluorohypoxanthine |
| acyl | OH | CF₃ | O | 2,8-Difluorohypoxanthine |
| acyl | OH | CF₃ | O | 2-Aminoadenine |
| acyl | OH | CF₃ | O | 2-Amino-8-fluoroadenine |
| acyl | OH | CF₃ | O | 2-Amino-8-fluorohypoxanthine |
| acyl | OH | CF₃ | O | 2-Aminohypoxanthine |
| acyl | OH | CF₃ | O | 2-N-acetylguanine |
| acyl | OH | CF₃ | O | 4-N-acetylcytosine |
| acyl | OH | CF₃ | O | 6-N-acetyladenine |
| acyl | OH | CF₃ | O | 2-N-acetyl-8-fluoroguanine |
| acyl | OH | CF₃ | O | 4-N-acetyl-5-fluorocytosine |
| acyl | OH | CF₃ | O | 6-N-acetyl-2-fluoroadenine |
| acyl | OH | CF₃ | O | 6-N-acetyl-2,8-difluoroadenine |
| acyl | OH | CF₃ | O | 6-N-acetyl-2-aminoadenine |
| acyl | OH | CF₃ | O | 6-N-acetyl-2-amino-8-fluoroadenine |
| acyl | OH | CF₃ | O | 2-N-acetylaminoadenine |
| acyl | OH | CF₃ | O | 2-N-acetylamino-8-fluoroadenine |
| acyl | OH | CF₃ | O | 2-N-acetylamino-8-fluorohypoxanthine |
| acyl | OH | CF₃ | O | 2-N-acetylaminohypoxanthine |
| acyl | OH | CF₃ | S | Thymine |
| acyl | OH | CF₃ | S | Uracil |
| acyl | OH | CF₃ | S | Guanine |
| acyl | OH | CF₃ | S | Cytosine |
| acyl | OH | CF₃ | S | Adenine |
| acyl | OH | CF₃ | S | Hypoxanthine |
| acyl | OH | CF₃ | S | 5-Fluorouracil |
| acyl | OH | CF₃ | S | 8-Fluoroguanine |
| acyl | OH | CF₃ | S | 5-Fluorocytosine |
| acyl | OH | CF₃ | S | 8-Fluoroadenine |
| acyl | OH | CF₃ | S | 2-Fluoroadenine |
| acyl | OH | CF₃ | S | 2,8-Difluoroadenine |
| acyl | OH | CF₃ | S | 2-Fluorohypoxanthine |
| acyl | OH | CF₃ | S | 8-Fluorohypoxanthine |
| acyl | OH | CF₃ | S | 2,8-Difluorohypoxanthine |
| acyl | OH | CF₃ | S | 2-Aminoadenine |
| acyl | OH | CF₃ | S | 2-Amino-8-fluoroadenine |
| acyl | OH | CF₃ | S | 2-Amino-8-fluorohypoxanthine |
| acyl | OH | CF₃ | S | 2-Aminohypoxanthine |
| acyl | OH | CF₃ | S | 2-N-acetylguanine |
| acyl | OH | CF₃ | s | 4-N-acetylcytosine |
| acyl | OH | CF₃ | S | 6-N-acetyladenine |
| acyl | OH | CF₃ | S | 2-N-acetyl-8-fluoroguanine |
| acyl | OH | CF₃ | S | 4-N-acetyl-5-fluorocytosine |
| acyl | OH | CF₃ | S | 6-N-acetyl-2-fluoroadenine |
| acyl | OH | CF₃ | S | 6-N-acetyl-2,8-difluoroadenine |
| acyl | OH | CF₃ | S | 6-N-acetyl-2-aminoadenine |
| acyl | OH | CF₃ | S | 6-N-acetyl-2-amino-8-fluoroadenine |
| acyl | OH | CF₃ | S | 2-N-acetylaminoadenine |
| acyl | OH | CF₃ | S | 2-N-acetylamino-8-fluoroadenine |
| acyl | OH | CF₃ | S | 2-N-acetylamino-8-fluorohypoxanthine |
| acyl | OH | CF₃ | S | 2-N-acetylaminohypoxanthine |
| amino acid | OH | CH₃ | O | Thymine |
| amino acid | OH | CH₃ | O | Uracil |
| amino acid | OH | CH₃ | O | Guanine |
| amino acid | OH | CH₃ | O | Cytosine |
| amino acid | OH | CH₃ | O | Adenine |
| amino acid | OH | CH₃ | O | Hypoxanthine |
| amino acid | OH | CH₃ | O | 5-Fluorouracil |
| amino acid | OH | CH₃ | O | 8-Fluoroguanine |
| amino acid | OH | CH₃ | O | 5-Fluorocytosine |
| amino acid | OH | CH₃ | O | 8-Fluoroadenine |
| amino acid | OH | CH₃ | O | 2-Fluoroadenine |
| amino acid | OH | CH₃ | O | 2,8-Difluoroadenine |
| amino acid | OH | CH₃ | O | 2-Fluorohypoxanthine |
| amino acid | OH | CH₃ | O | 8-Fluorohypoxanthine |
| amino acid | OH | CH₃ | O | 2,8-Difluorohypoxanthine |
| amino acid | OH | CH₃ | O | 2-Aminoadenine |
| amino acid | OH | CH₃ | O | 2-Amino-8-fluoroadenine |
| amino acid | OH | CH₃ | O | 2-Amino-8-fluorohypoxanthine |
| amino acid | OH | CH₃ | O | 2-Aminohypoxanthine |
| amino acid | OH | CH₃ | O | 2-N-acetylguanine |
| amino acid | OH | CH₃ | O | 4-N-acetylcytosine |
| amino acid | OH | CH₃ | O | 6-N-acetyladenine |
| amino acid | OH | CH₃ | O | 2-N-acetyl-8-fluoroguanine |
| amino acid | OH | CH₃ | O | 4-N-acetyl-5-fluorocytosine |
| amino acid | OH | CH₃ | O | 6-N-acetyl-2-fluoroadenine |
| amino acid | OH | CH₃ | O | 6-N-acetyl-2,8-difluoroadenine |
| amino acid | OH | CH₃ | O | 6-N-acetyl-2-aminoadenine |
| amino acid | OH | CH₃ | O | 6-N-acetyl-2-amino-8-fluoroadenine |
| amino acid | OH | CH₃ | O | 2-N-acetylaminoadenine |
| amino acid | OH | CH₃ | O | 2-N-acetylamino-8-fluoroadenine |
| amino acid | OH | CH₃ | O | 2-N-acetylamino-8-fluorohypoxanthine |
| amino acid | OH | CH₃ | O | 2-N-acetylaminohypoxanthine |
| amino acid | OH | CH₃ | S | Thymine |
| amino acid | OH | CH₃ | S | Uracil |
| amino acid | OH | CH₃ | S | Guanine |
| amino acid | OH | CH₃ | S | Cytosine |
| amino acid | OH | CH₃ | S | Adenine |
| amino acid | OH | CH₃ | S | Hypoxanthine |
| amino acid | OH | CH₃ | S | 5-Fluorouracil |
| amino acid | OH | CH₃ | S | 8-Fluoroguanine |
| amino acid | OH | CH₃ | S | 5-Fluorocytosine |
| amino acid | OH | CH₃ | S | 8-Fluoroadenine |
| amino acid | OH | CH₃ | S | 2-Fluoroadenine |
| amino acid | OH | CH₃ | S | 2,8-Difluoroadenine |
| amino acid | OH | CH₃ | S | 2-Fluorohypoxanthine |
| amino acid | OH | CH₃ | S | 8-Fluorohypoxanthine |
| amino acid | OH | CH₃ | S | 2,8-Difluorohypoxanthine |
| amino acid | OH | CH₃ | S | 2-Aminoadenine |
| amino acid | OH | CH₃ | S | 2-Amino-8-fluoroadenine |
| amino acid | OH | CH₃ | S | 2-Amino-8-fluorohypoxanthine |
| amino acid | OH | CH₃ | S | 2-Aminohypoxanthine |
| amino acid | OH | CH₃ | S | 2-N-acetylguanine |
| amino acid | OH | CH₃ | S | 4-N-acetylcytosine |
| amino acid | OH | CH₃ | S | 6-N-acetyladenine |
| amino acid | OH | CH₃ | S | 2-N-acetyl-8-fluoroguanine |
| amino acid | OH | CH₃ | S | 4-N-acetyl-5-fluorocytosine |
| amino acid | OH | CH₃ | S | 6-N-acetyl-2-fluoroadenine |
| amino acid | OH | CH₃ | S | 6-N-acetyl-2,8-difluoroadenine |
| amino acid | OH | CH₃ | S | 6-N-acetyl-2-aminoadenine |
| amino acid | OH | CH₃ | S | 6-N-acetyl-2-amino-8-fluoroadenine |
| amino acid | OH | CH₃ | S | 2-N-acetylaminoadenine |
| amino acid | OH | CH₃ | S | 2-N-acetylamino-8-fluoroadenine |
| amino acid | OH | CH₃ | S | 2-N-acetylamino-8-fluorohypoxanthine |
| amino acid | OH | CH₃ | S | 2-N-acetylaminohypoxanthine |
| amino acid | OH | CF₃ | O | Thymine |
| amino acid | OH | CF₃ | O | Uracil |
| amino acid | OH | CF₃ | O | Guanine |
| amino acid | OH | CF₃ | O | Cytosine |
| amino acid | OH | CF₃ | O | Adenine |
| amino acid | OH | CF₃ | O | Hypoxanthine |
| amino acid | OH | CF₃ | O | 5-Fluorouracil |
| amino acid | OH | CF₃ | O | 8-Fluoroguanine |
| amino acid | OH | CF₃ | O | 5-Fluorocytosine |
| amino acid | OH | CF₃ | O | 8-Fluoroadenine |
| amino acid | OH | CF₃ | O | 2-Fluoroadenine |
| amino acid | OH | CF₃ | O | 2,8-Difluoroadenine |
| amino acid | OH | CF₃ | O | 2-Fluorohypoxanthine |
| amino acid | OH | CF₃ | O | 8-Fluorohypoxanthine |
| amino acid | OH | CF₃ | O | 2,8-Difluorohypoxanthine |
| amino acid | OH | CF₃ | O | 2-Aminoadenine |
| amino acid | OH | CF₃ | O | 2-Amino-8-fluoroadenine |
| amino acid | OH | CF₃ | O | 2-Amino-8-fluorohypoxanthine |
| amino acid | OH | CF₃ | O | 2-Aminohypoxanthine |
| amino acid | OH | CF₃ | O | 2-N-acetylguanine |
| amino acid | OH | CF₃ | O | 4-N-acetylcytosine |
| amino acid | OH | CF₃ | O | 6-N-acetyladenine |
| amino acid | OH | CF₃ | O | 2-N-acetyl-8-fluoroguanine |
| amino acid | OH | CF₃ | O | 4-N-acetyl-5-fluorocytosine |
| amino acid | OH | CF₃ | O | 6-N-acetyl-2-fluoroadenine |
| amino acid | OH | CF₃ | O | 6-N-acetyl-2,8-difluoroadenine |
| amino acid | OH | CF₃ | O | 6-N-acetyl-2-aminoadenine |
| amino acid | OH | CF₃ | O | 6-N-acetyl-2-amino-8-fluoroadenine |
| amino acid | OH | CF₃ | O | 2-N-acetylaminoadenine |
| amino acid | OH | CF₃ | O | 2-N-acetylamino-8-fluoroadenine |
| amino acid | OH | CF₃ | O | 2-N-acetylamino-8-fluorohypoxanthine |
| amino acid | OH | CF₃ | O | 2-N-acetylaminohypoxanthine |
| amino acid | OH | CF₃ | S | Thymine |
| amino acid | OH | CF₃ | S | Uracil |
| amino acid | OH | CF₃ | S | Guanine |
| amino acid | OH | CF₃ | S | Cytosine |
| amino acid | OH | CF₃ | S | Adenine |
| amino acid | OH | CF₃ | S | Hypoxanthine |
| amino acid | OH | CF₃ | S | 5-Fluorouracil |
| amino acid | OH | CF₃ | S | 8-Fluoroguanine |
| amino acid | OH | CF₃ | S | 5-Fluorocytosine |
| amino acid | OH | CF₃ | S | 8-Fluoroadenine |
| amino acid | OH | CF₃ | S | 2-Fluoroadenine |
| amino acid | OH | CF₃ | S | 2,8-Difluoroadenine |
| amino acid | OH | CF₃ | S | 2-Fluorohypoxanthine |
| amino acid | OH | CF₃ | S | 8-Fluorohypoxanthine |
| amino acid | OH | CF₃ | S | 2,8-Difluorohypoxanthine |
| amino acid | OH | CF₃ | S | 2-Aminoadenine |
| amino acid | OH | CF₃ | S | 2-Amino-8-fluoroadenine |
| amino acid | OH | CF₃ | S | 2-Amino-8-fluorohypoxanthine |
| amino acid | OH | CF₃ | S | 2-Aminohypoxanthine |
| amino acid | OH | CF₃ | S | 2-N-acetylguanine |
| amino acid | OH | CF₃ | S | 4-N-acetylcytosine |
| amino acid | OH | CF₃ | S | 6-N-acetyladenine _{.} |
| amino acid | OH | CF₃ | S | 2-N-acetyl-8-fluoroguanine |
| amino acid | OH | CF₃ | S | 4-N-acetyl-5-fluorocytosine |
| amino acid | OH | CF₃ | s | 6-N-acetyl-2-fluoroadenine |
| amino acid | OH | CF₃ | S | 6-N-acetyl-2,8-difluoroadenine |
| amino acid | OH | CF₃ | S | 6-N-acetyl-2-aminoadenine |
| amino acid | OH | CF₃ | S | 6-N-acetyl-2-amino-8-fluoroadenine |
| amino acid | OH | CF₃ | S | 2-N-acetylaminoadenine |
| amino acid | OH | CF₃ | S | 2-N-acetylamino-8-fluoroadenine |
| amino acid | OH | CF₃ | S | 2-N-acetylamino-8-fluorohypoxanthine |
| amino acid | OH | CF₃ | S | 2-N-acetylaminohypoxanthine |

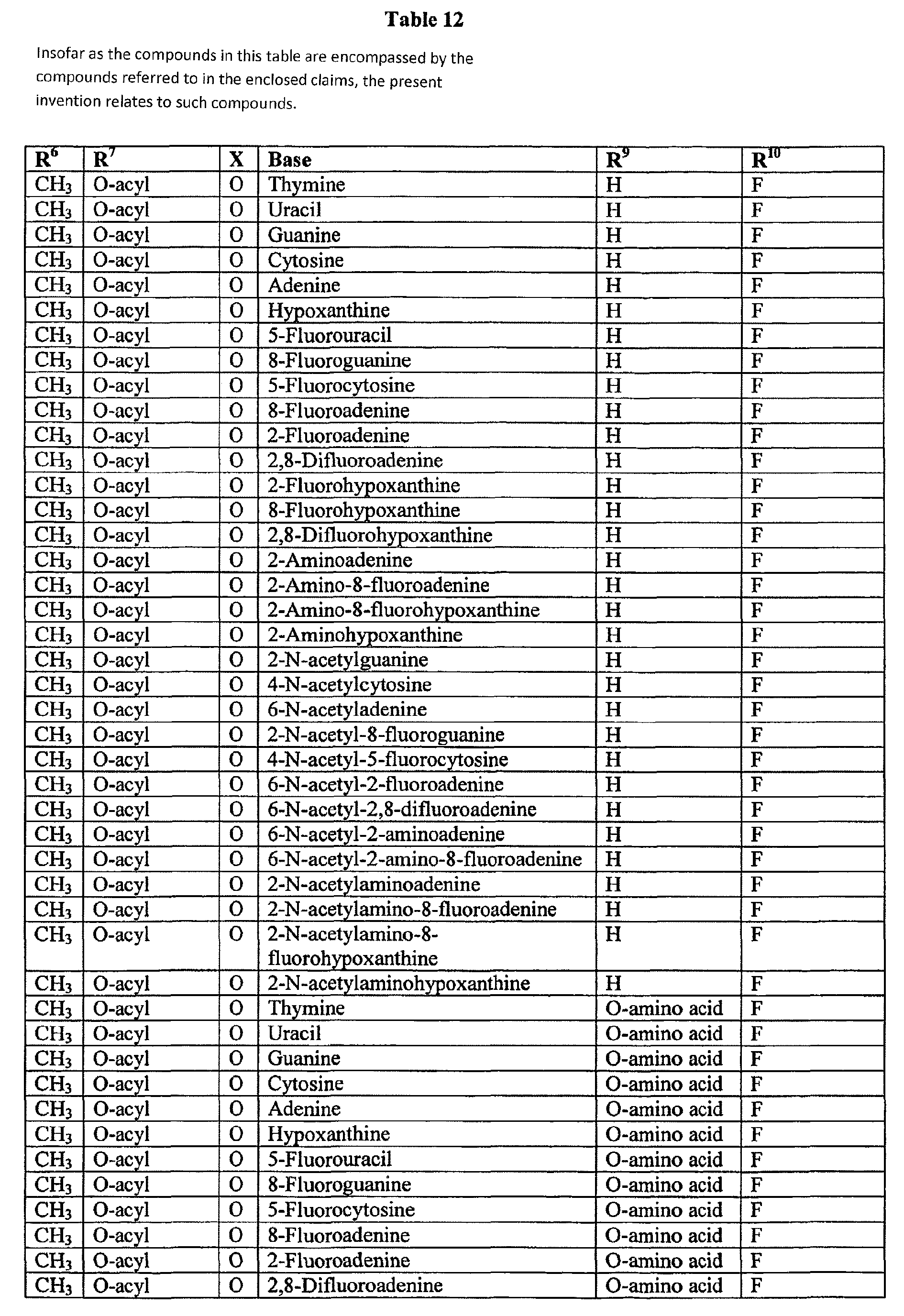

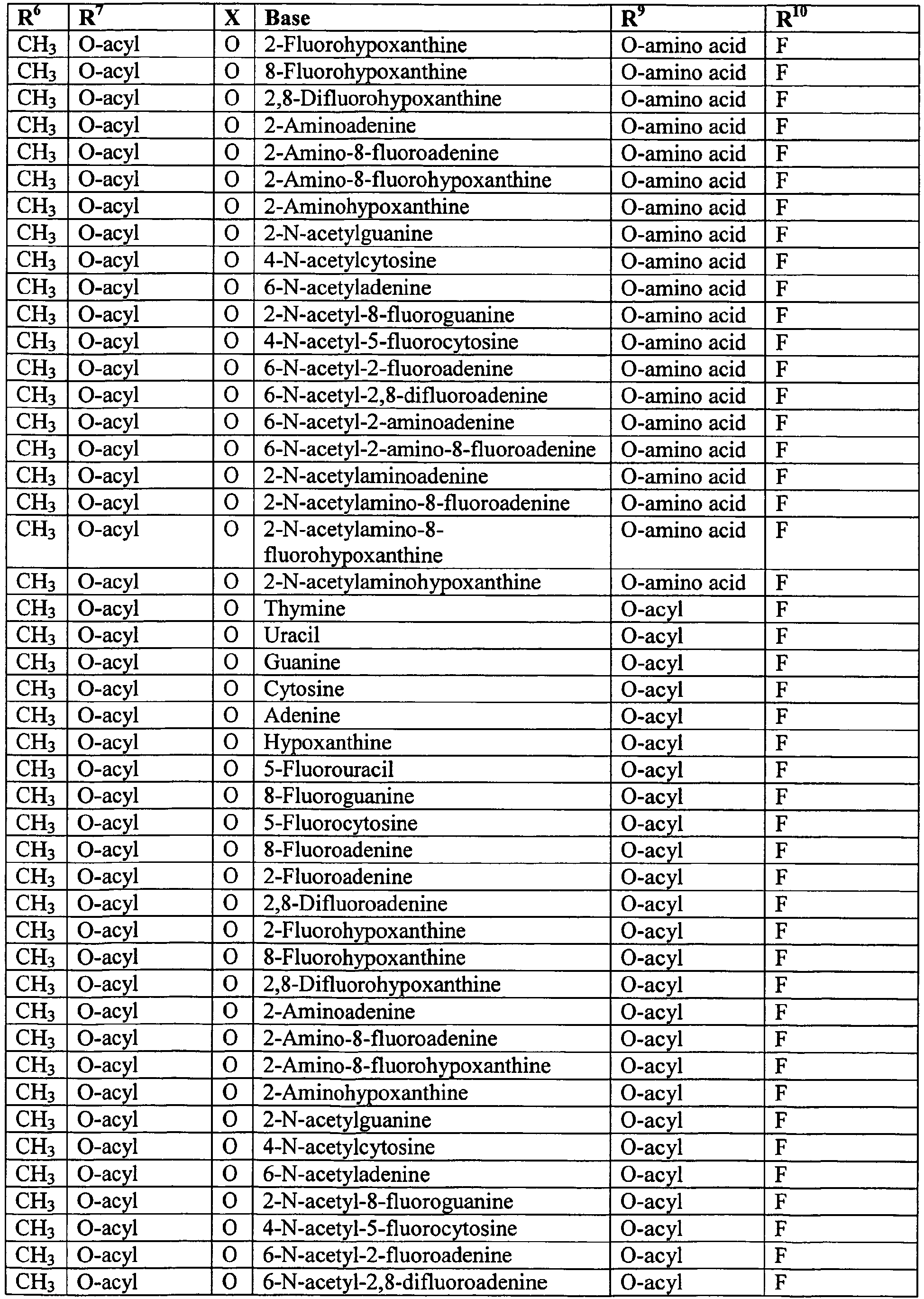

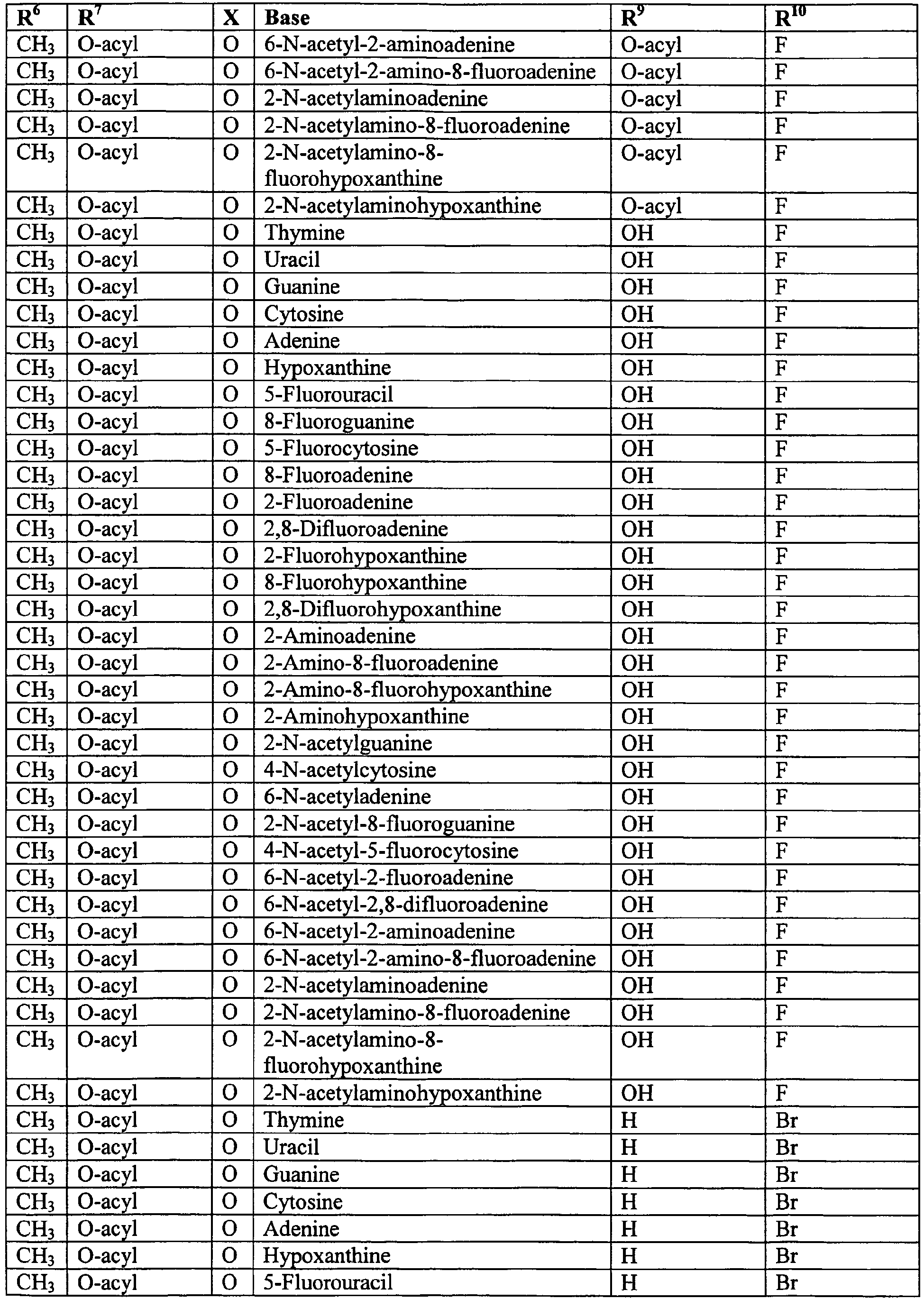

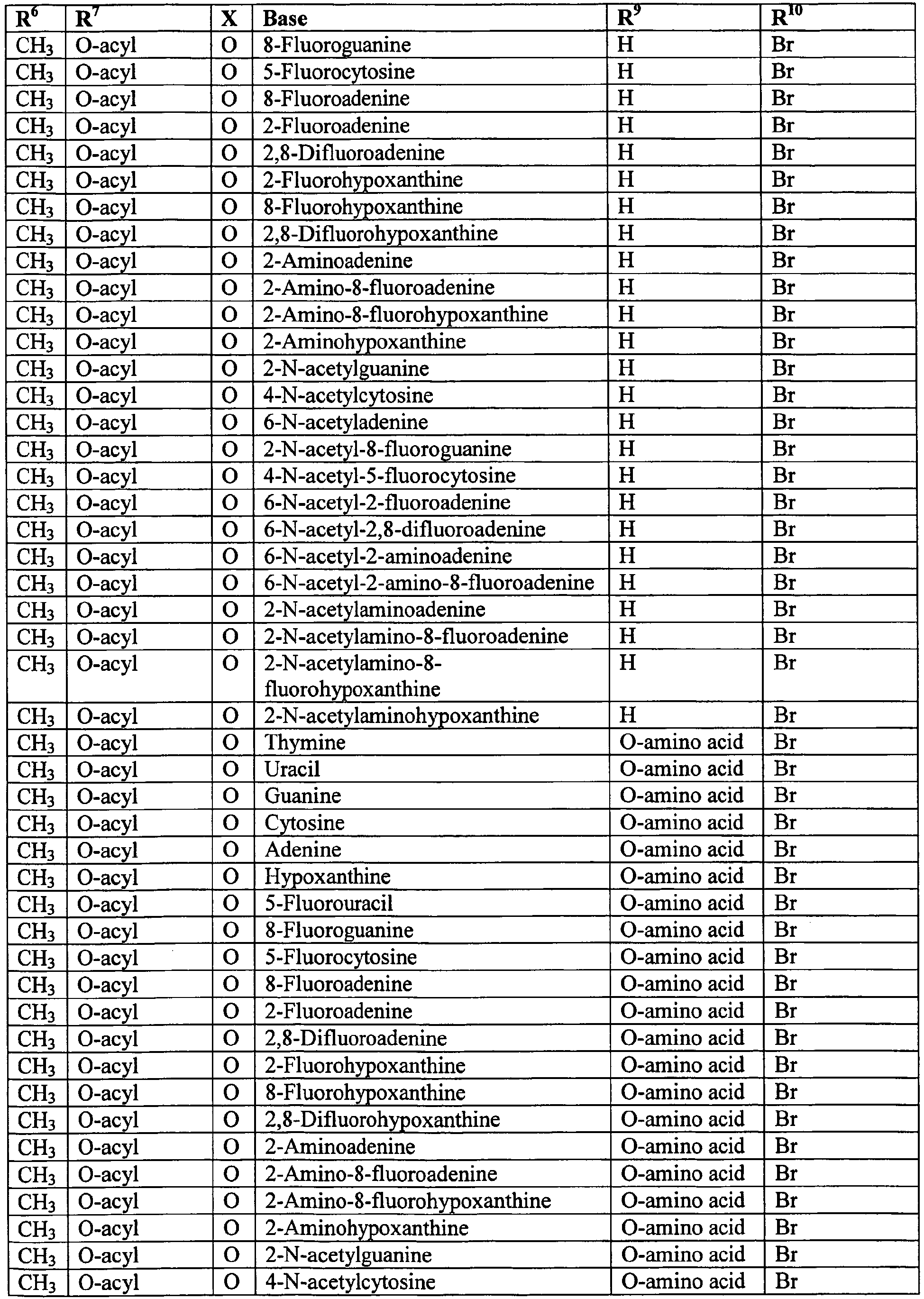

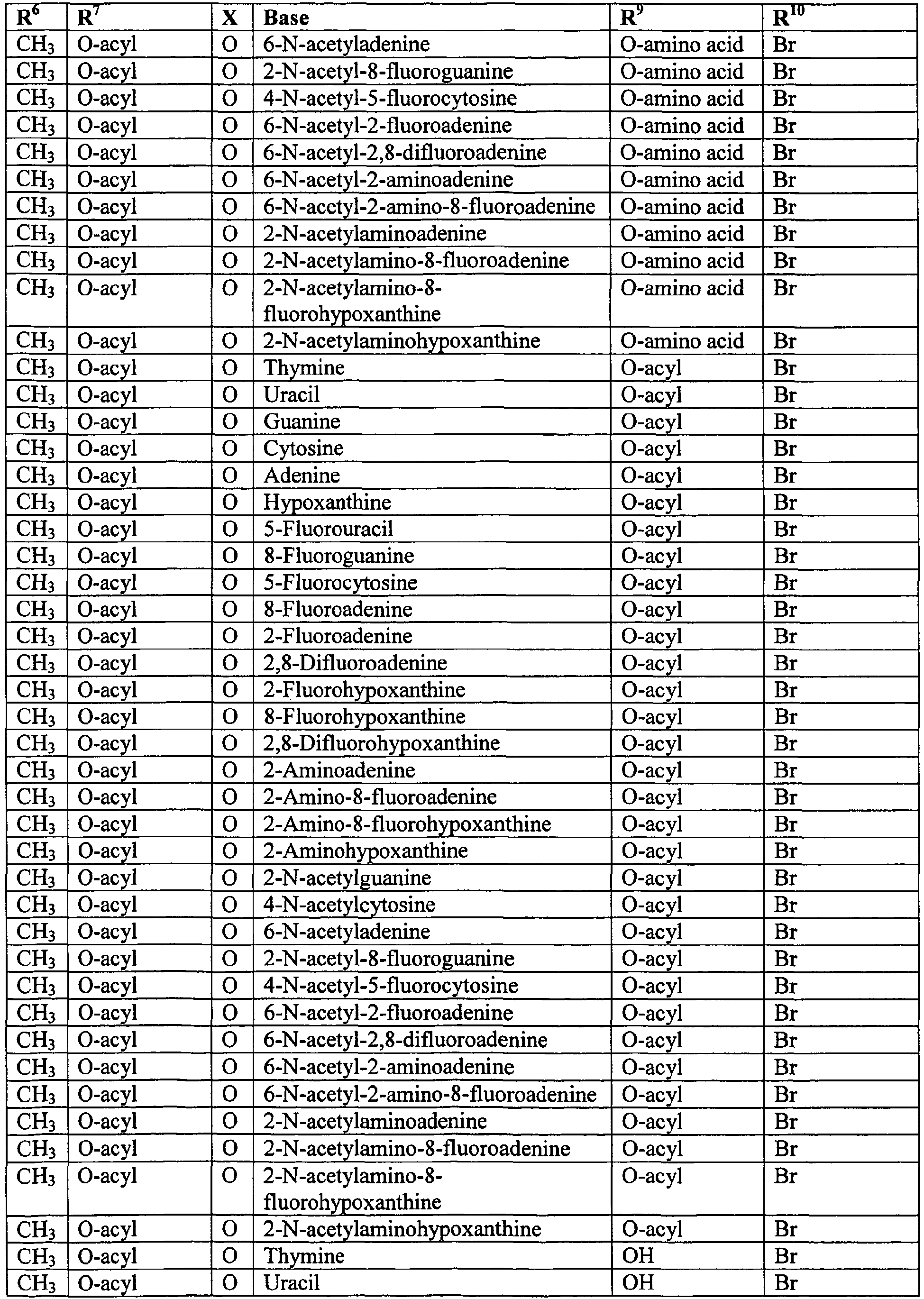

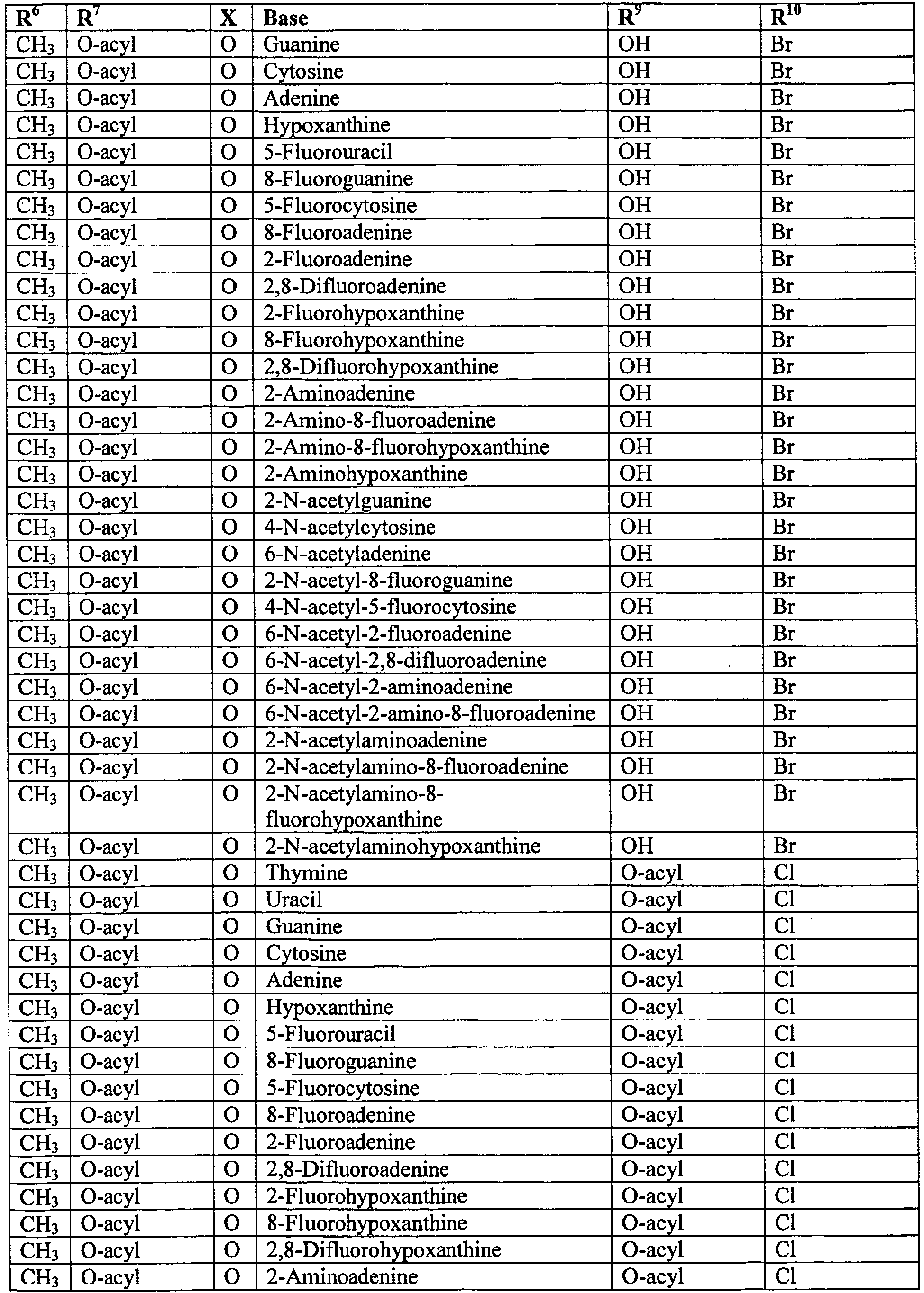

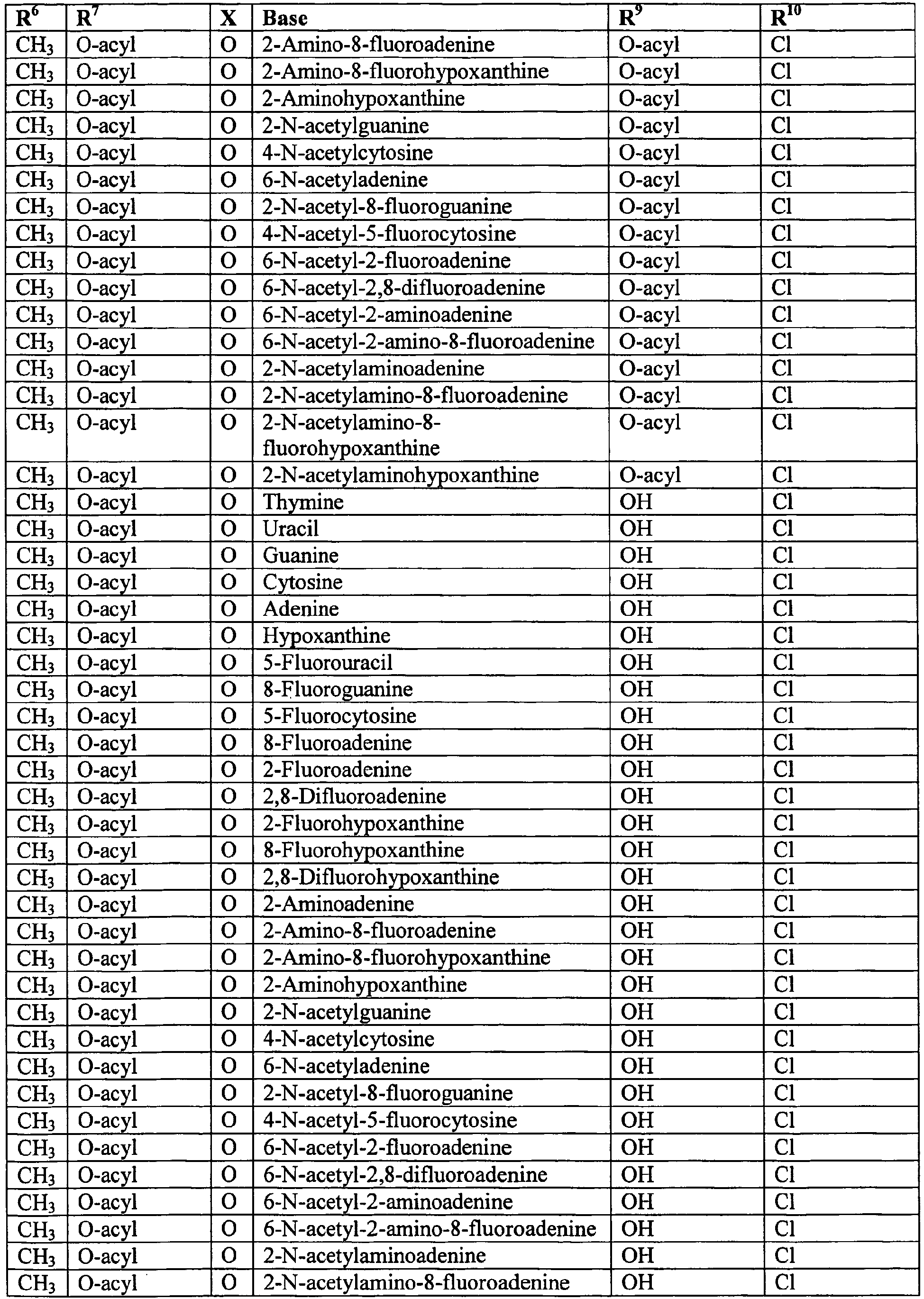

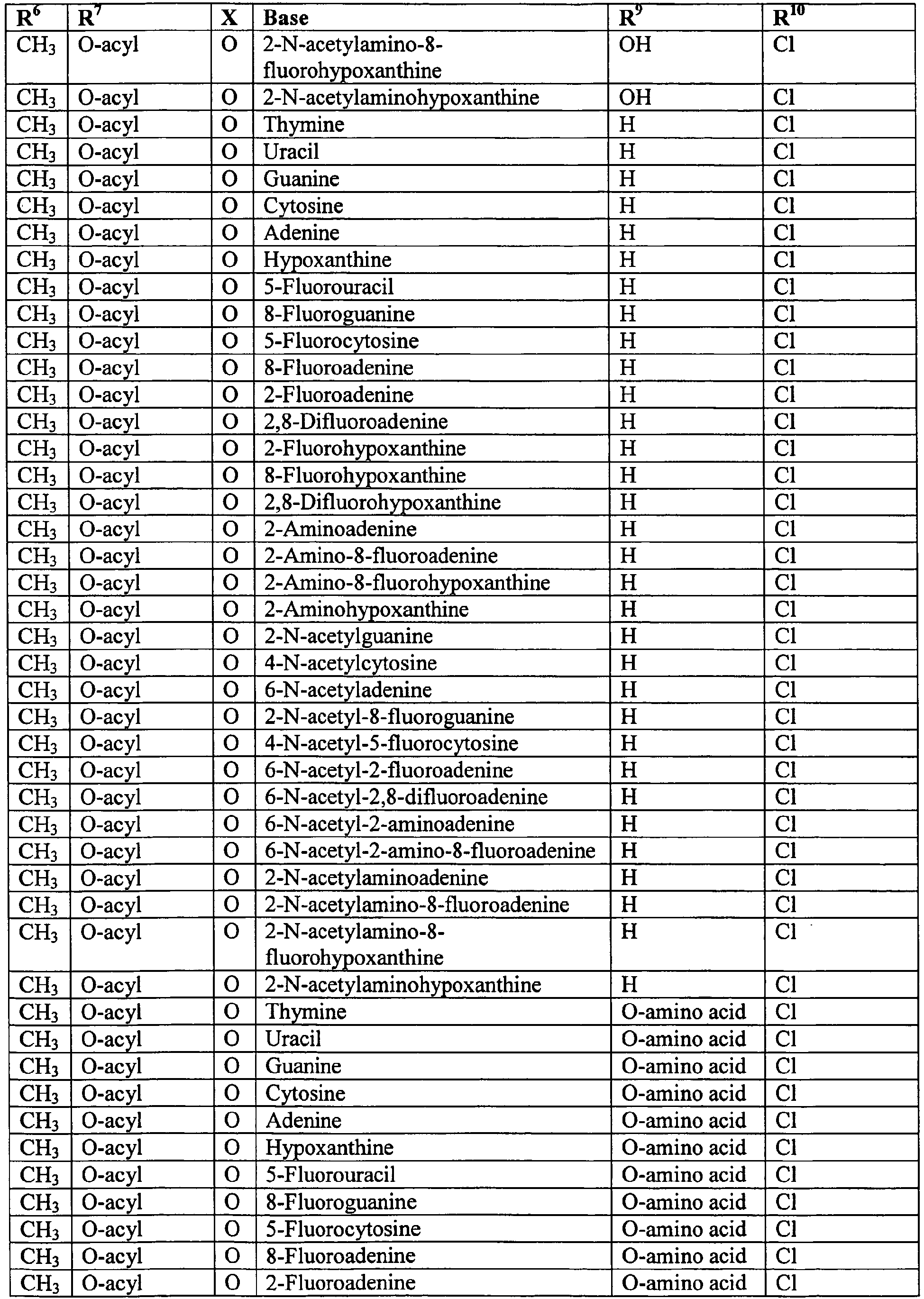

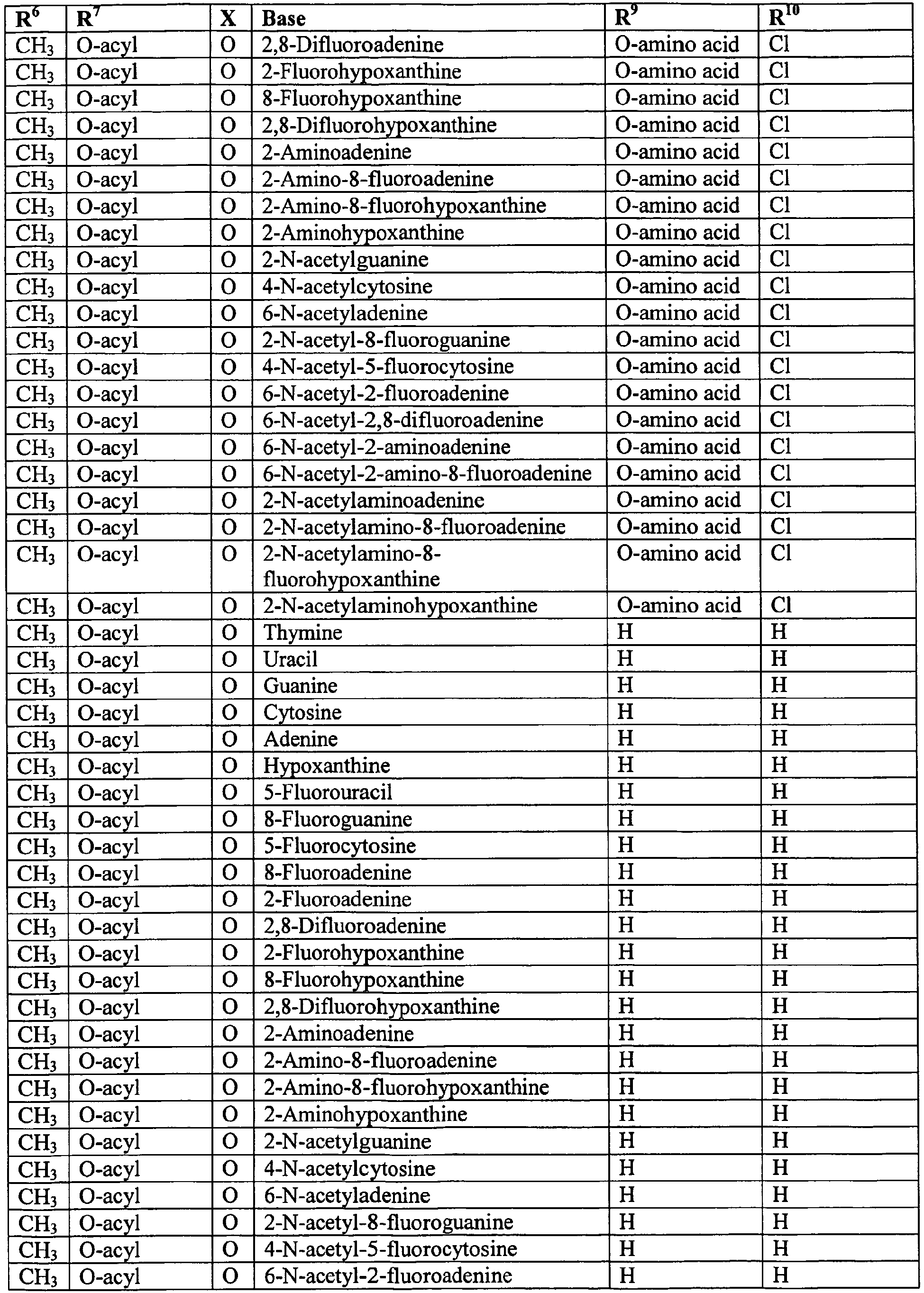

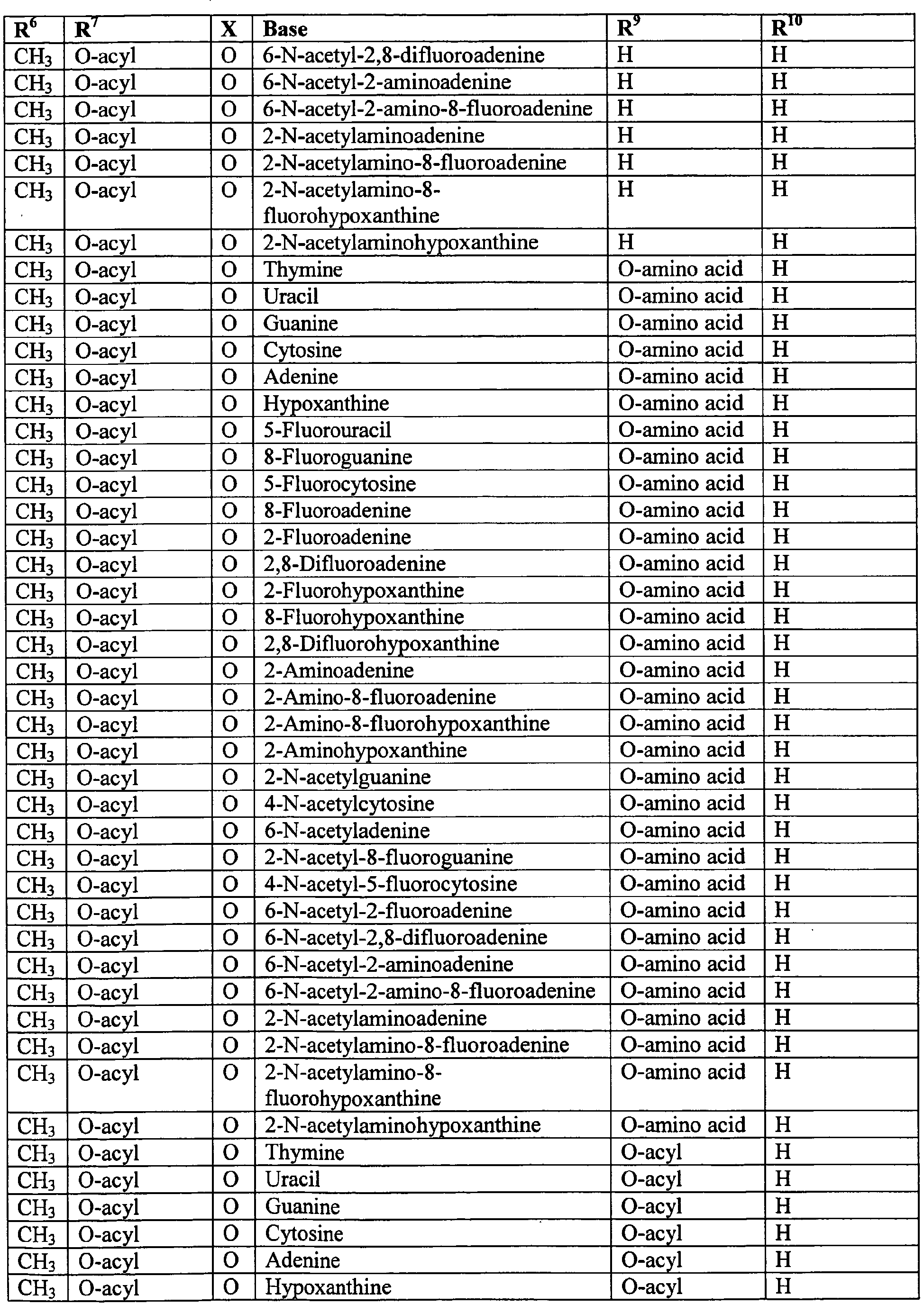

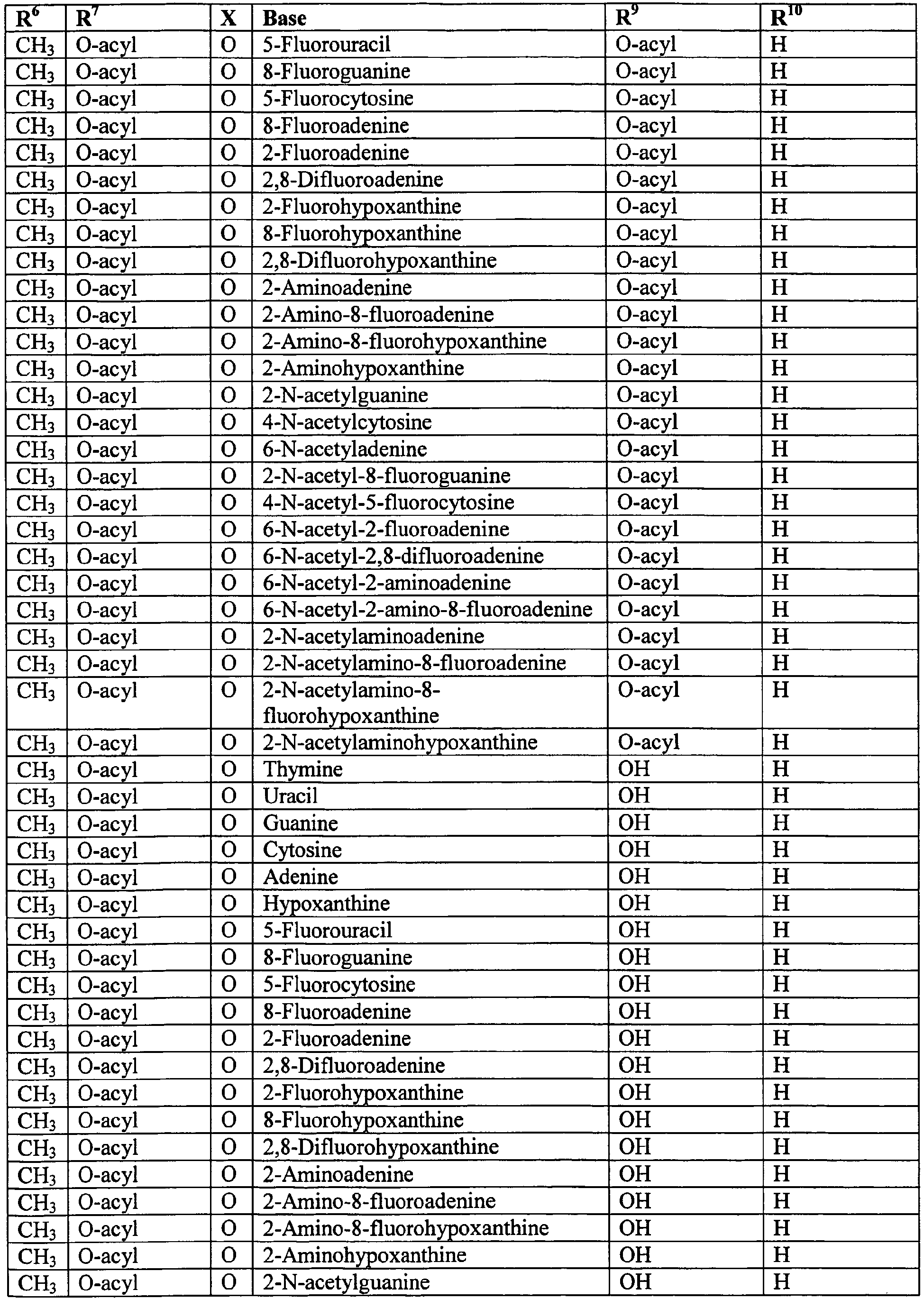

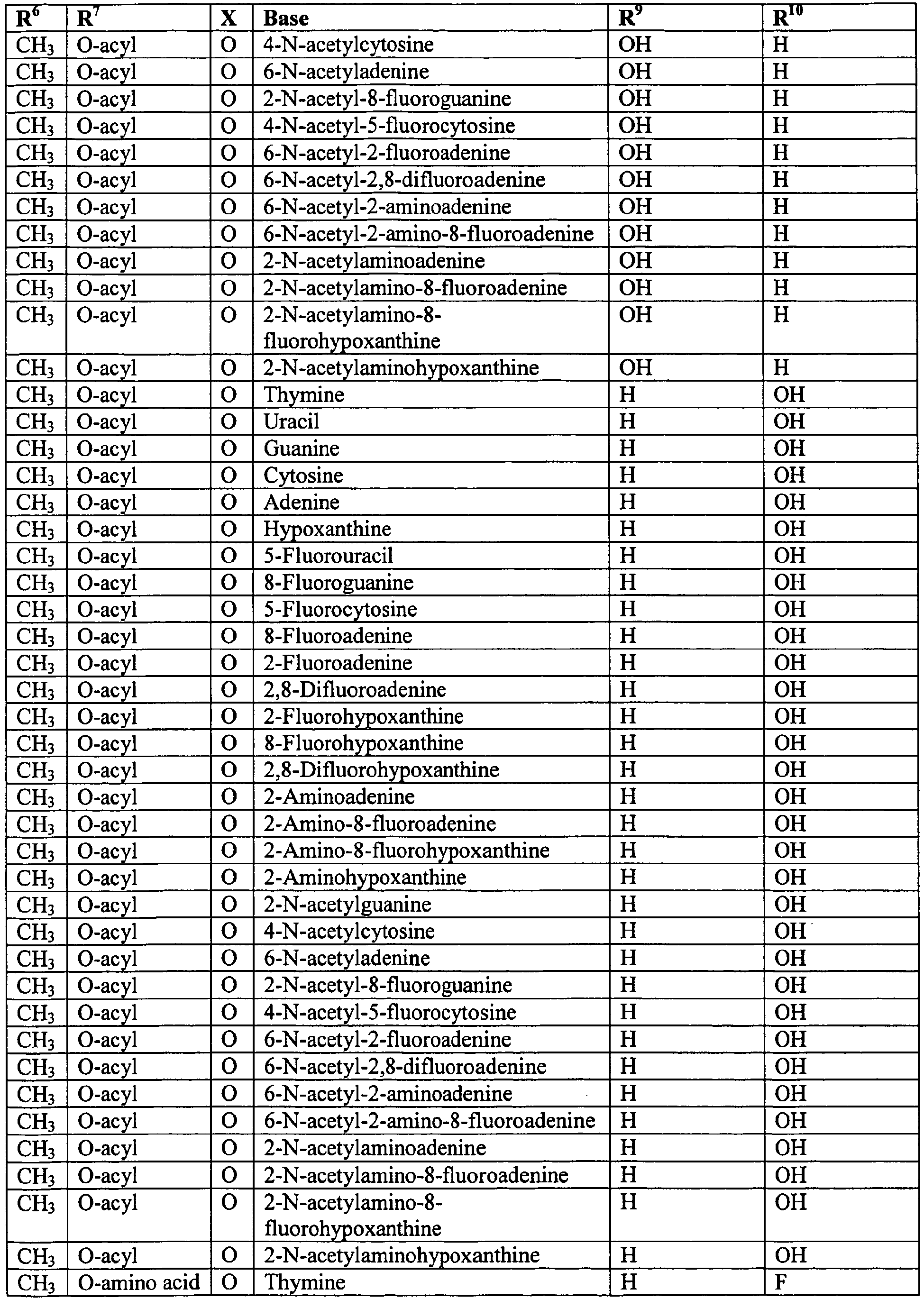

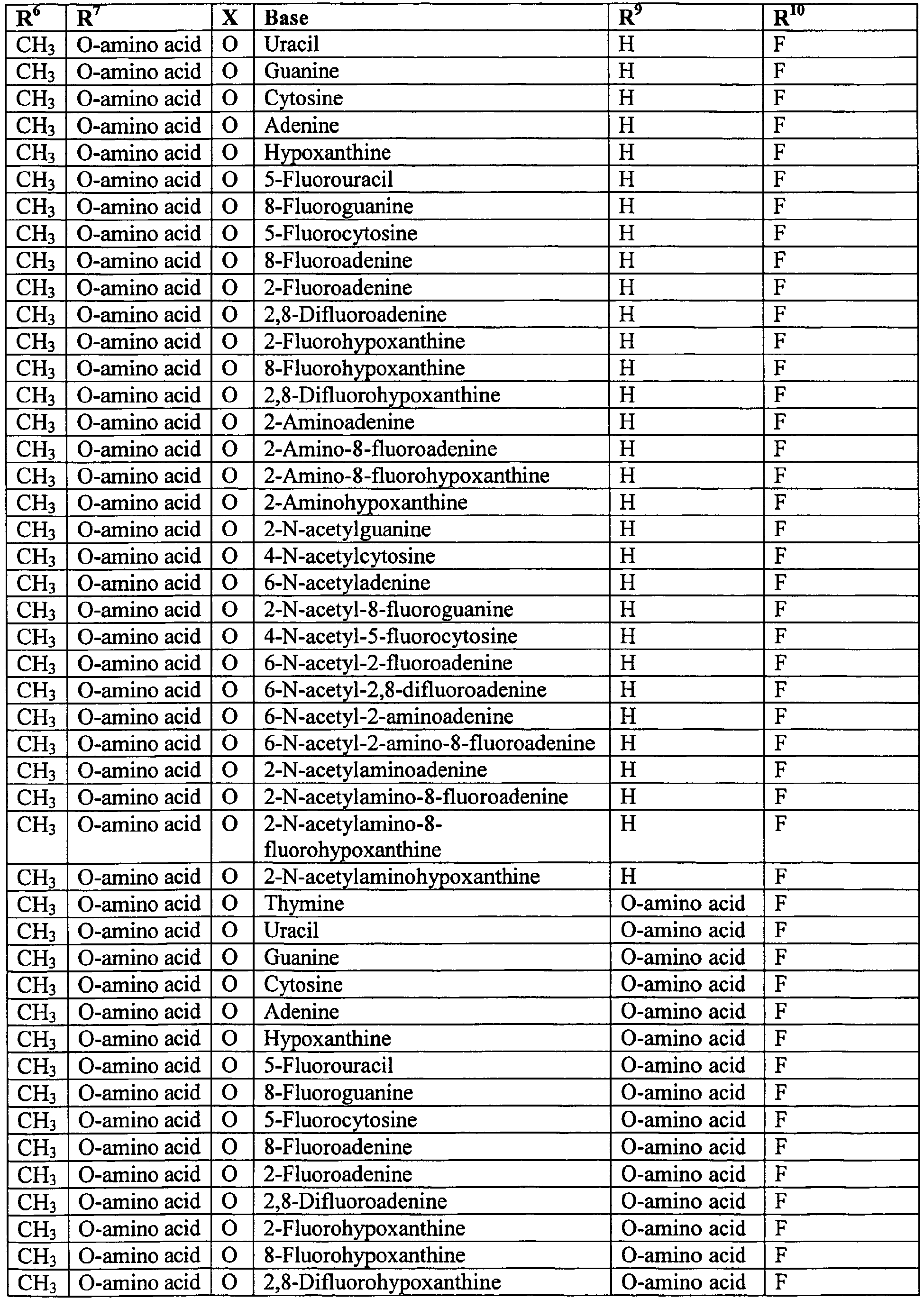

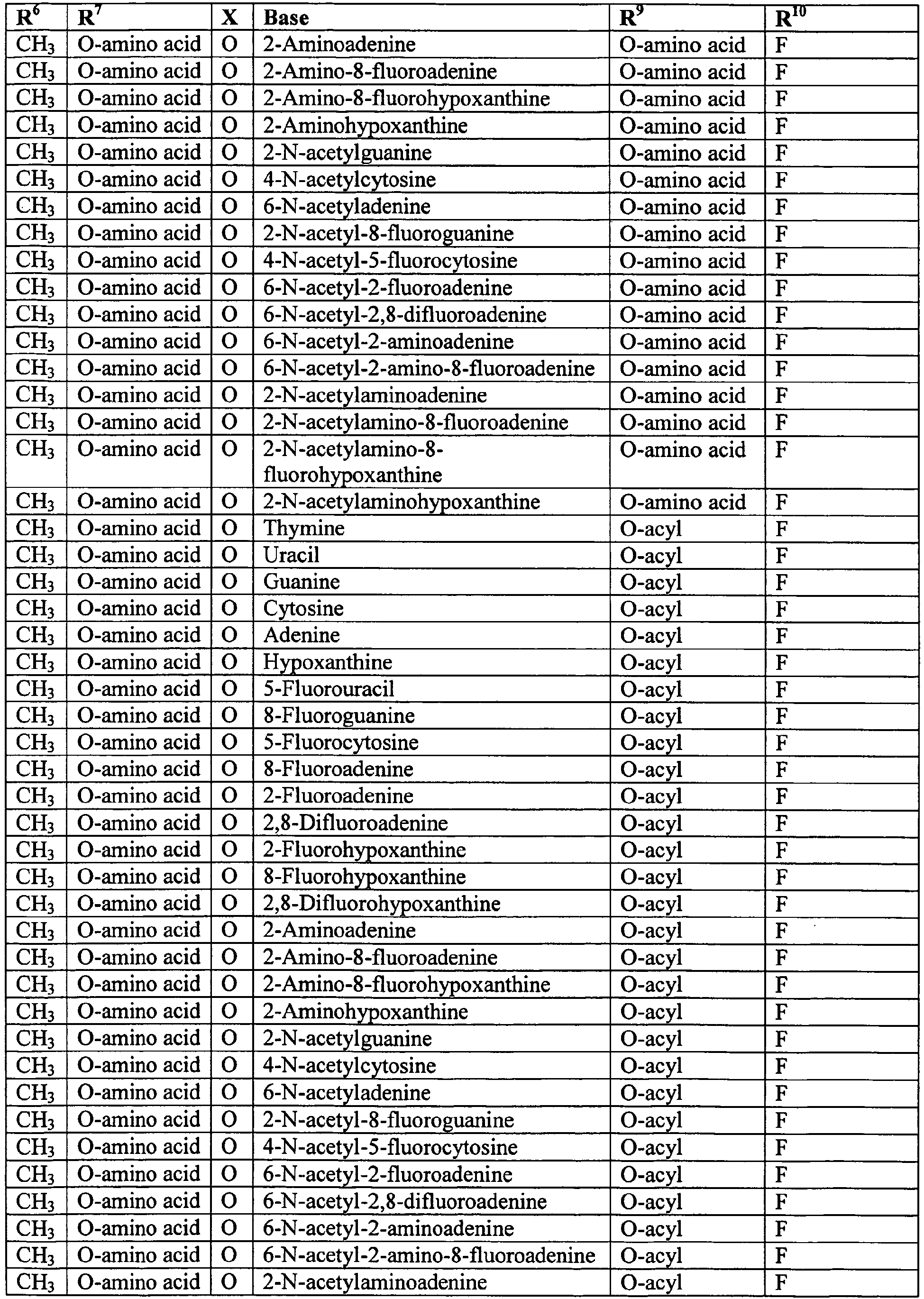

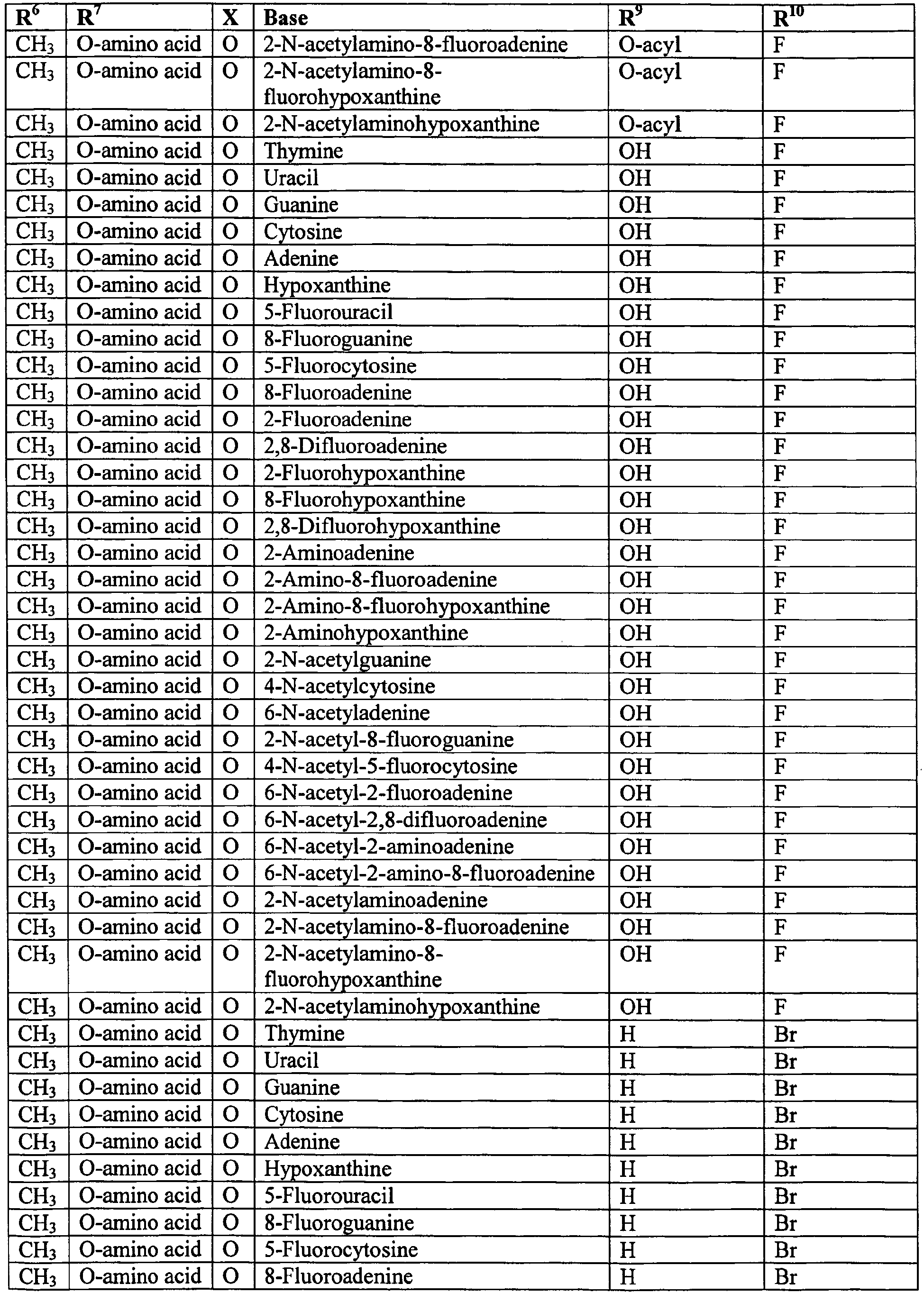

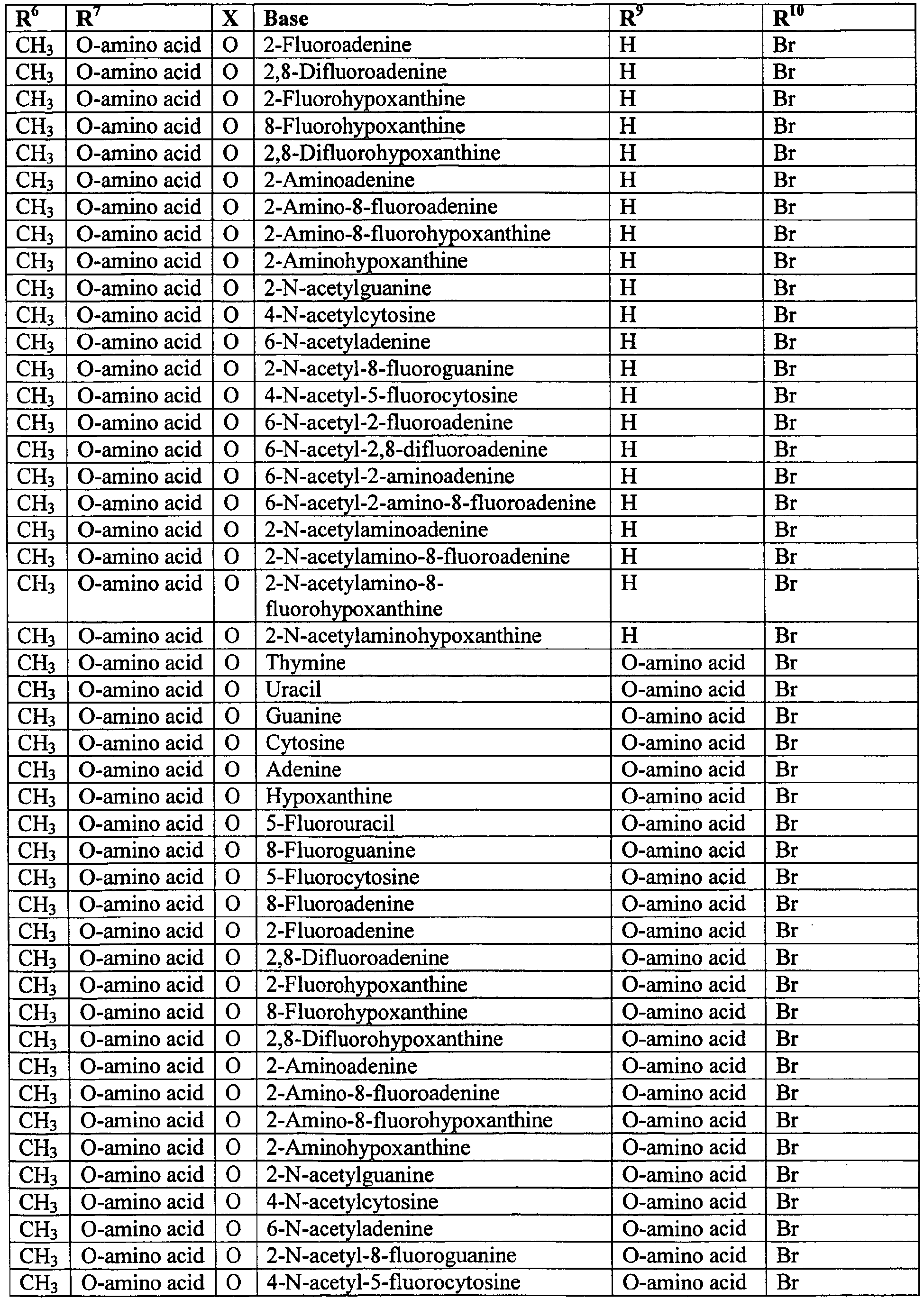

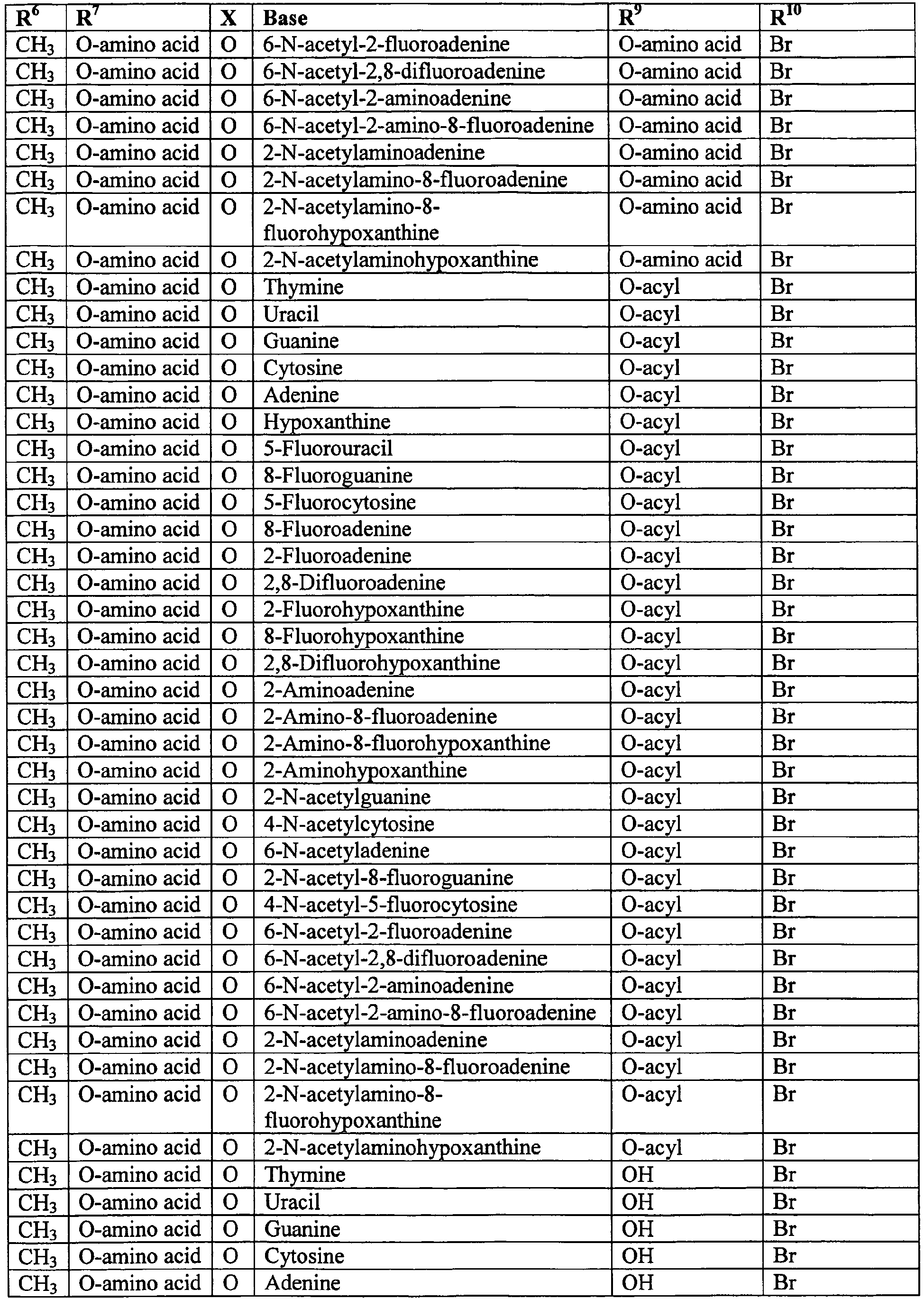

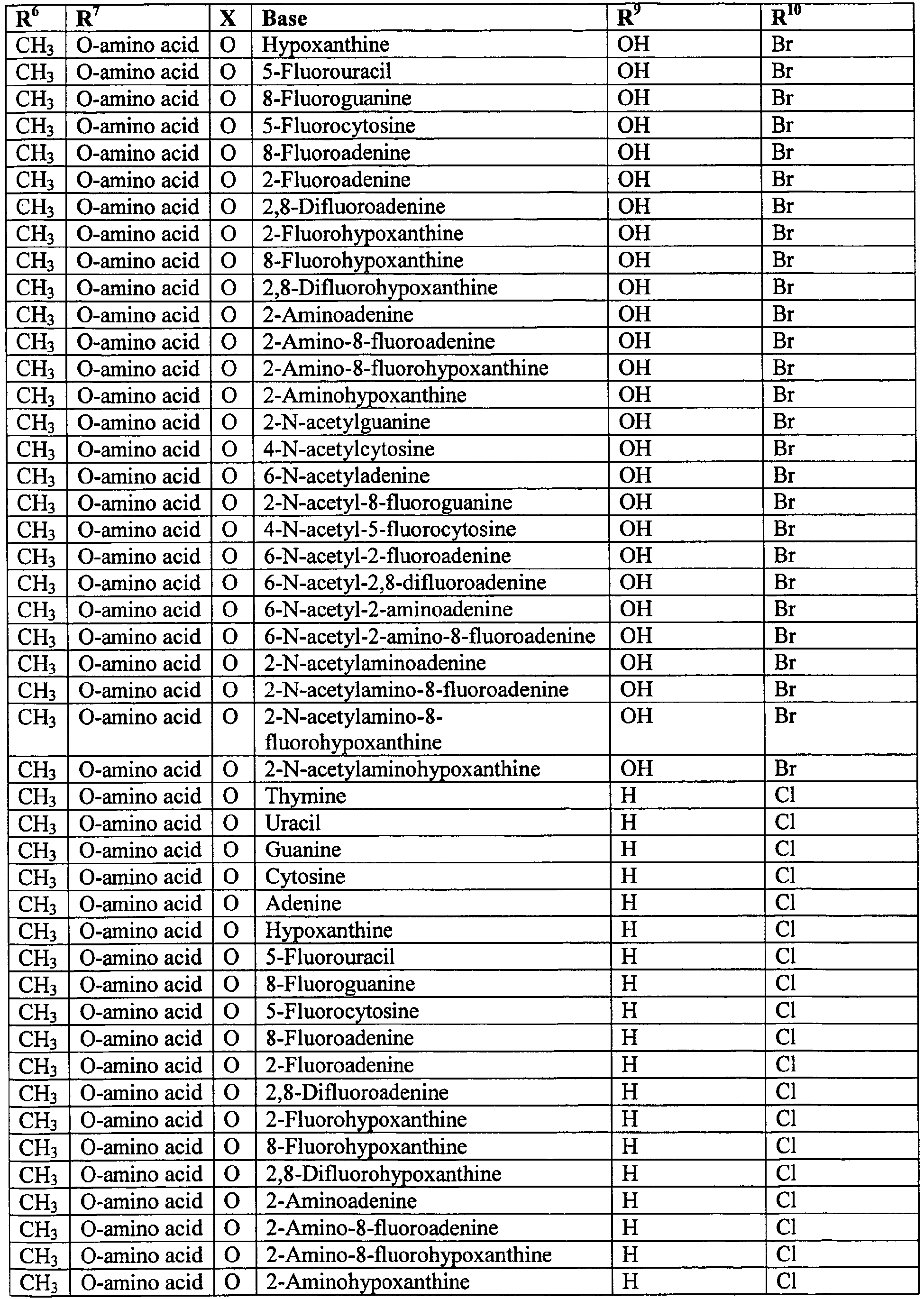

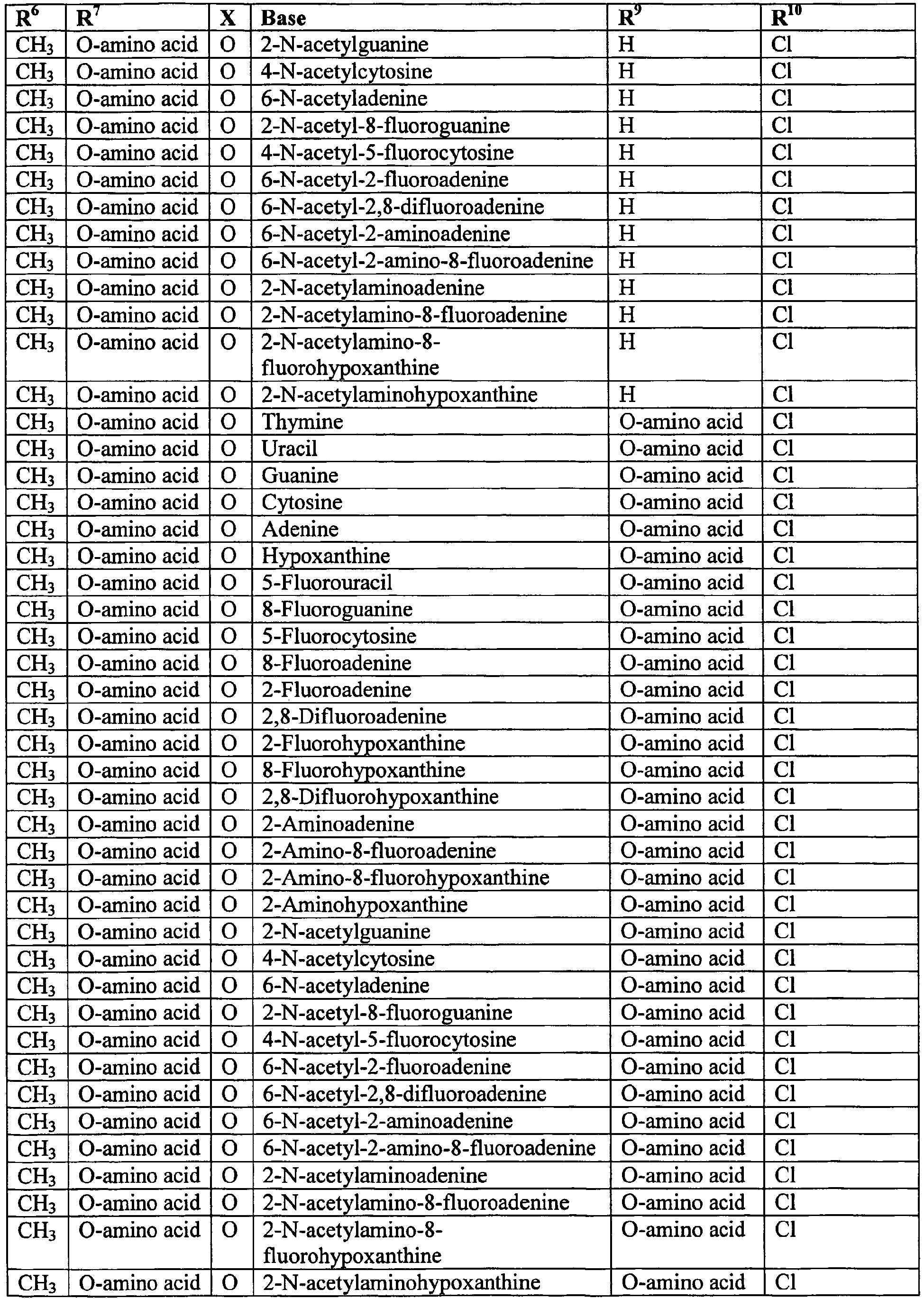

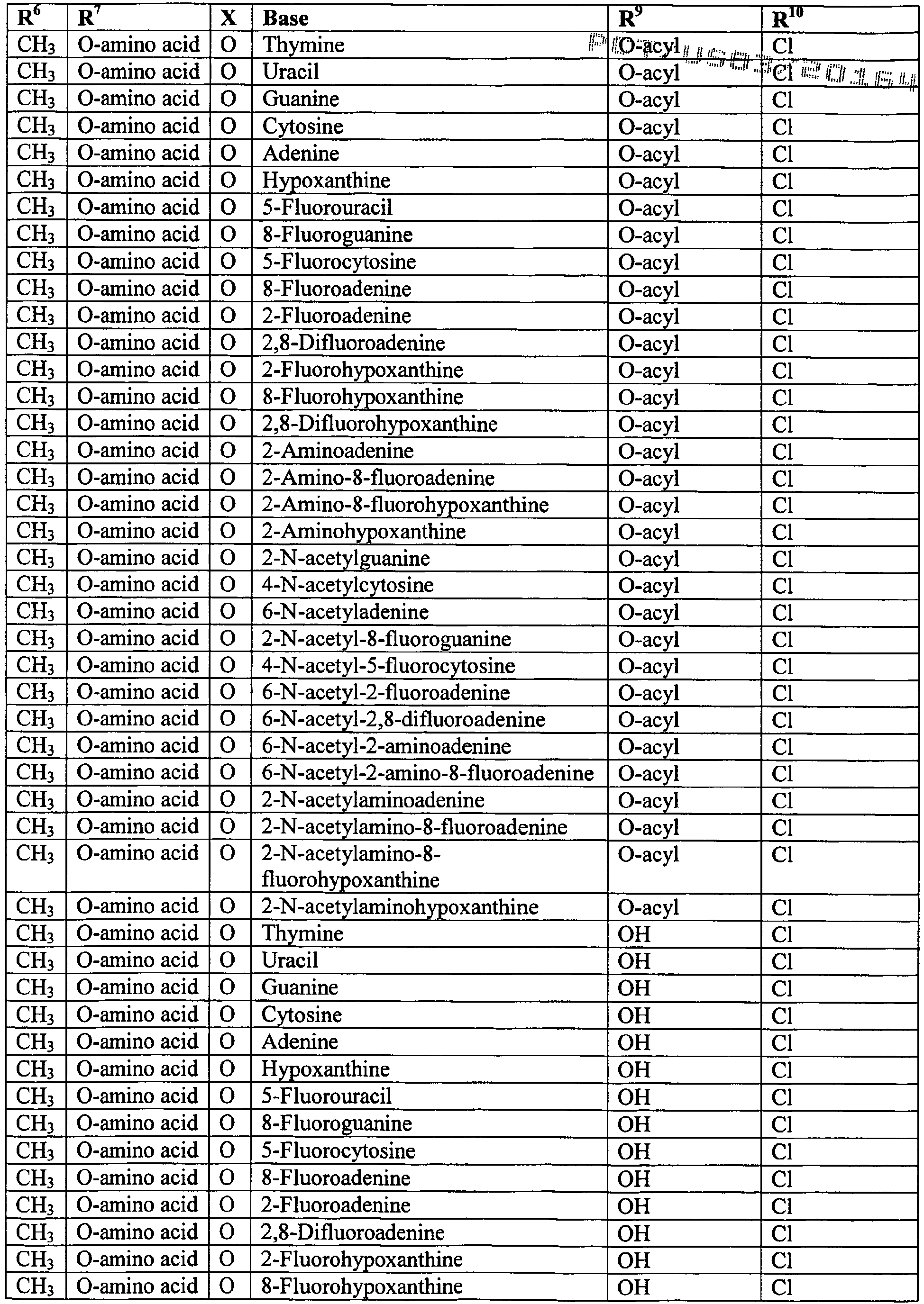

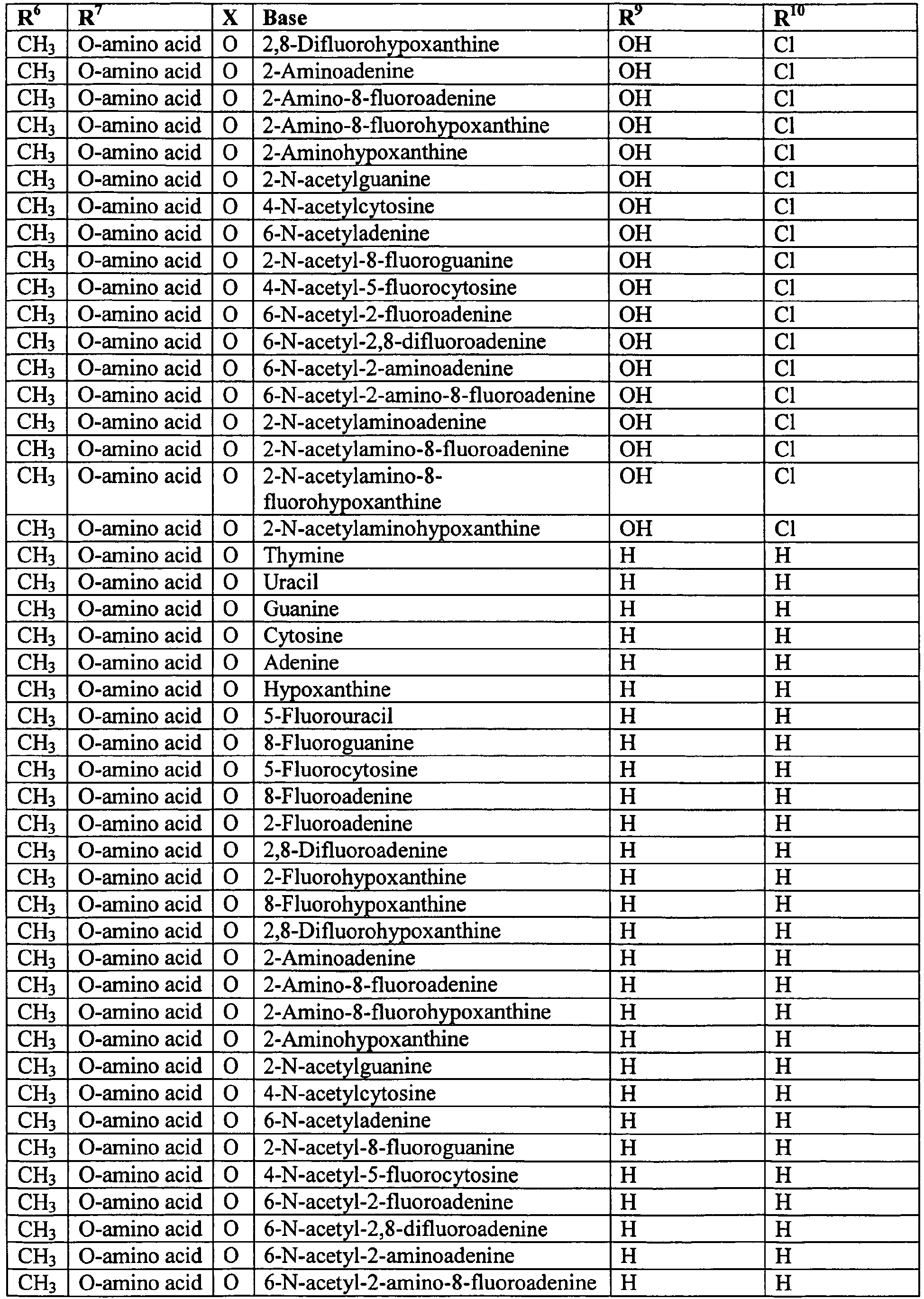

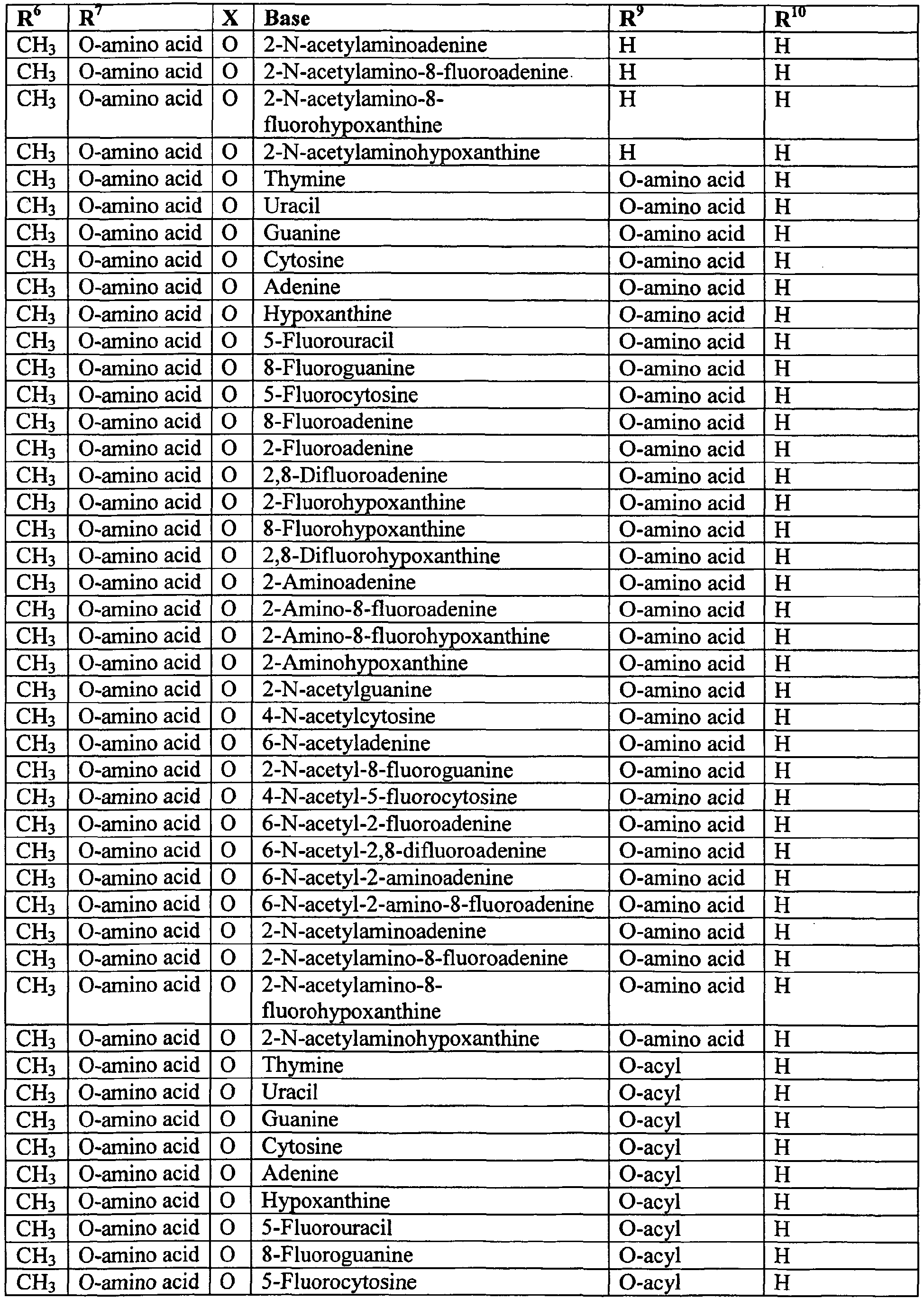

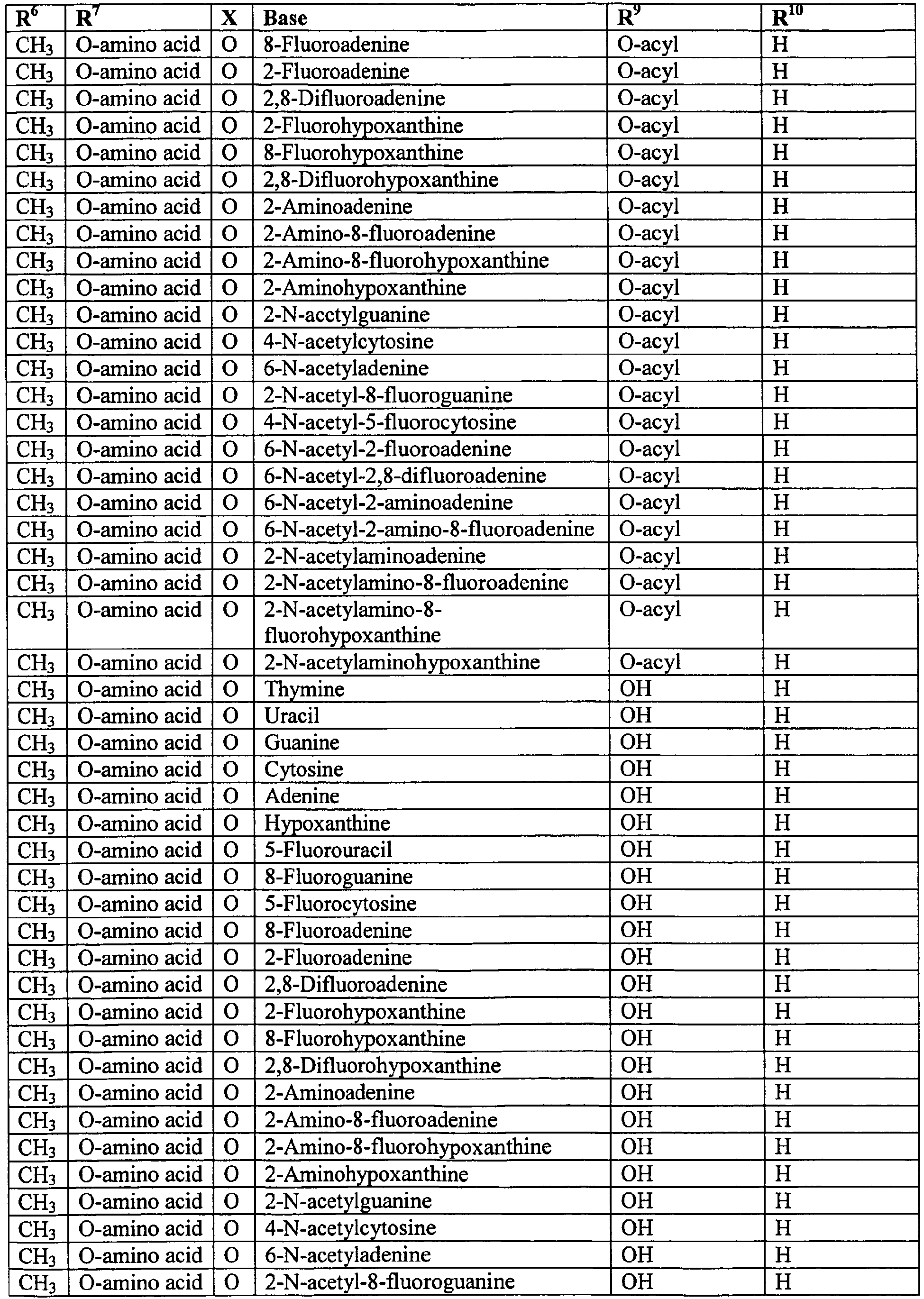

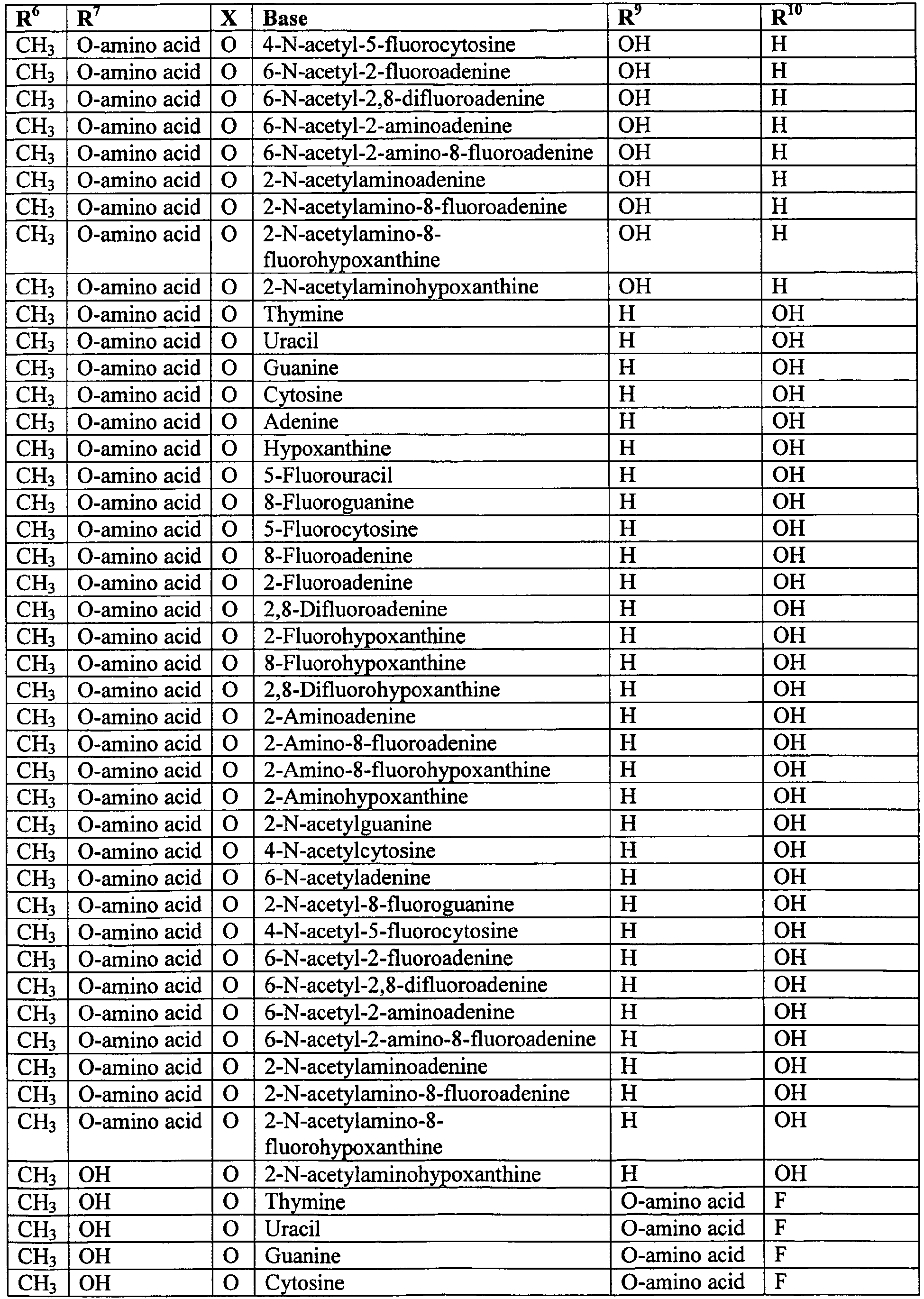

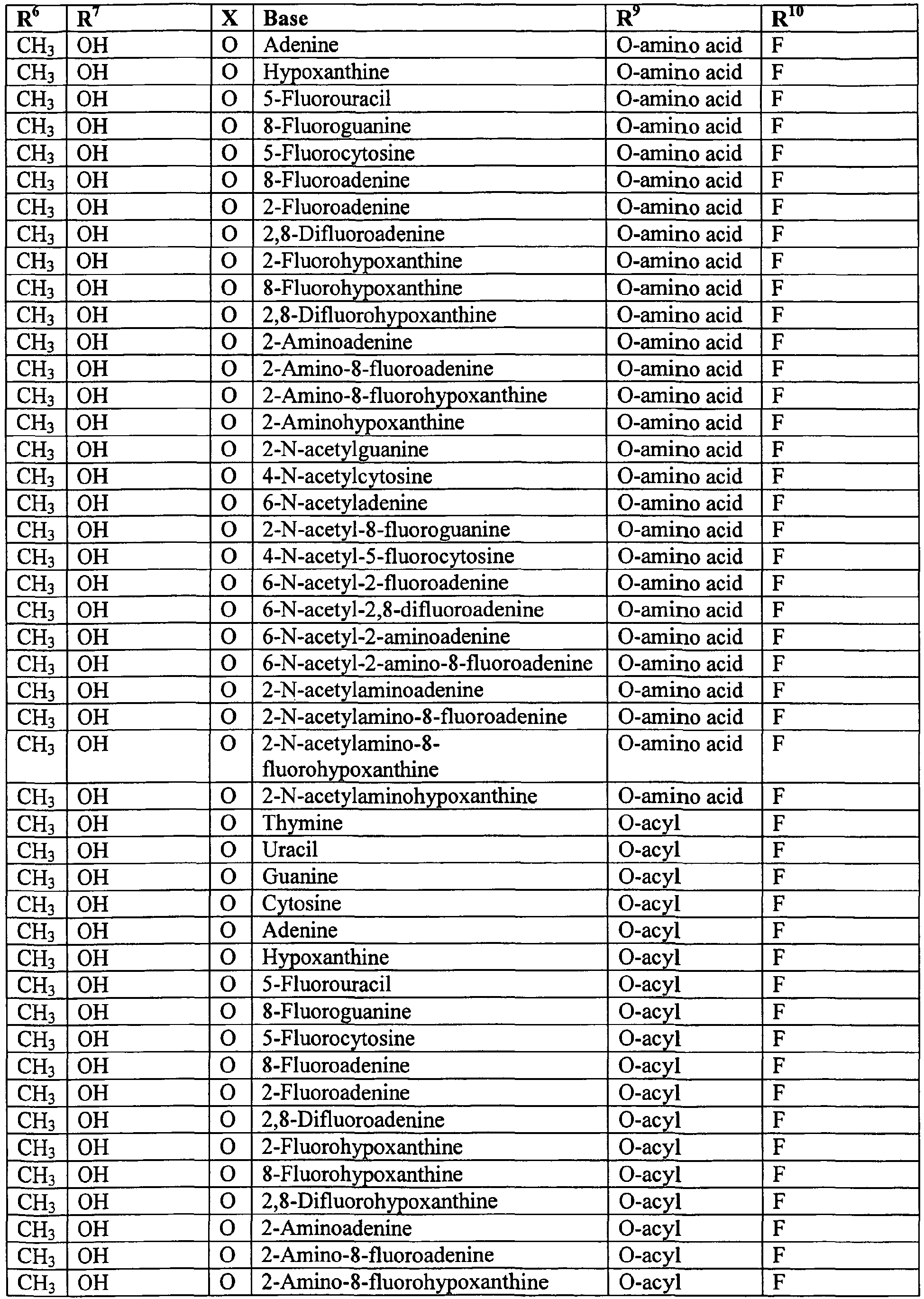

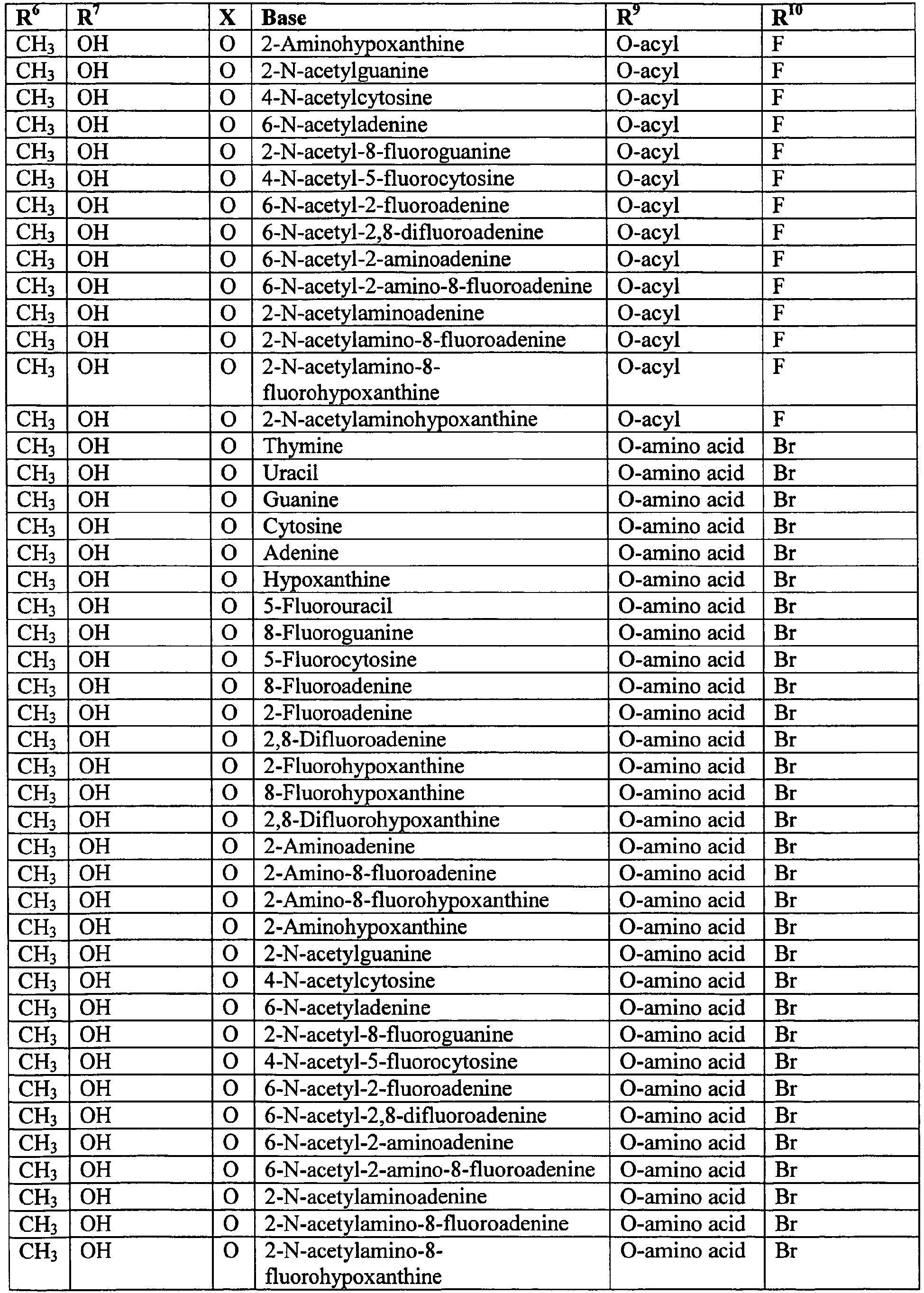

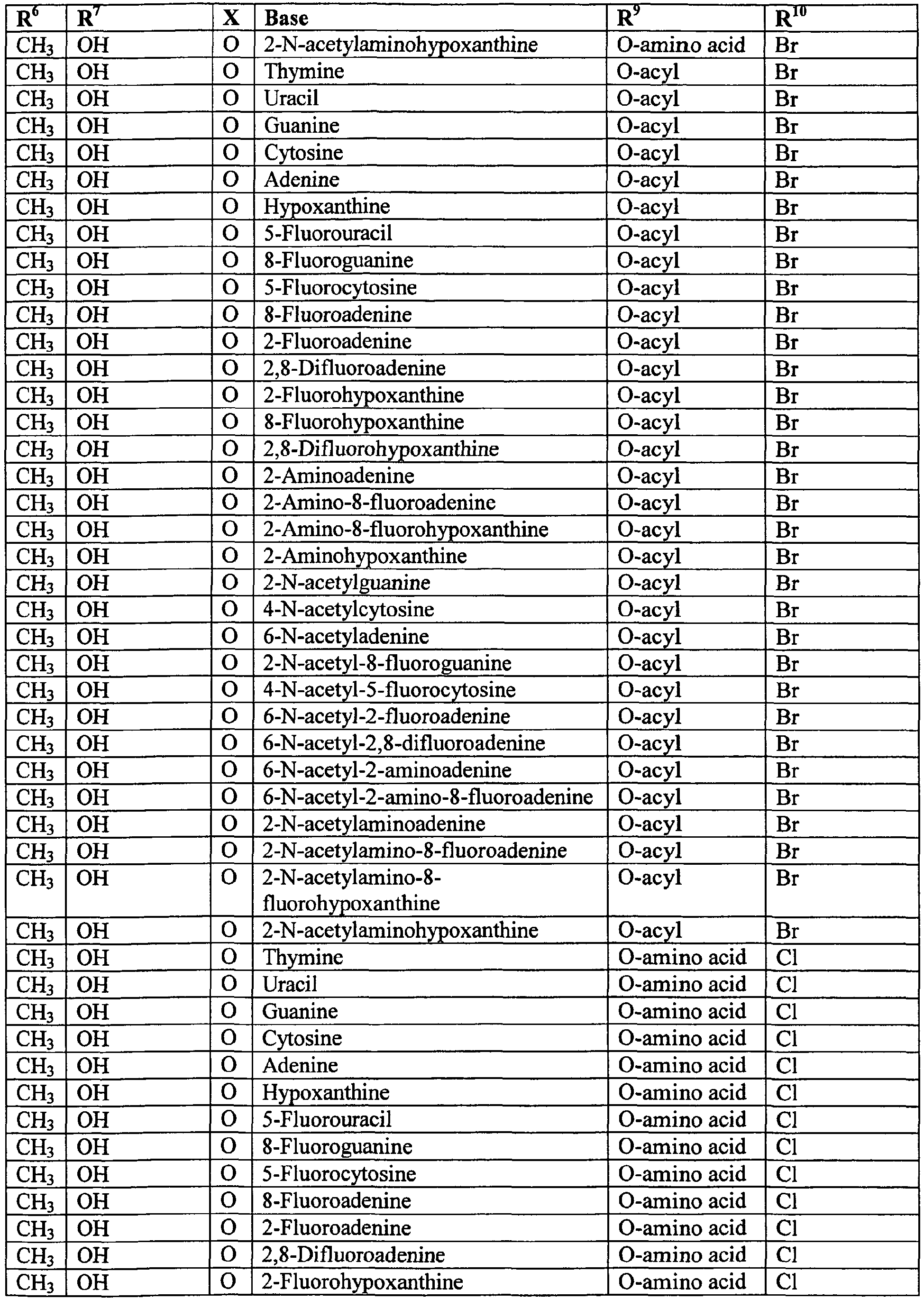

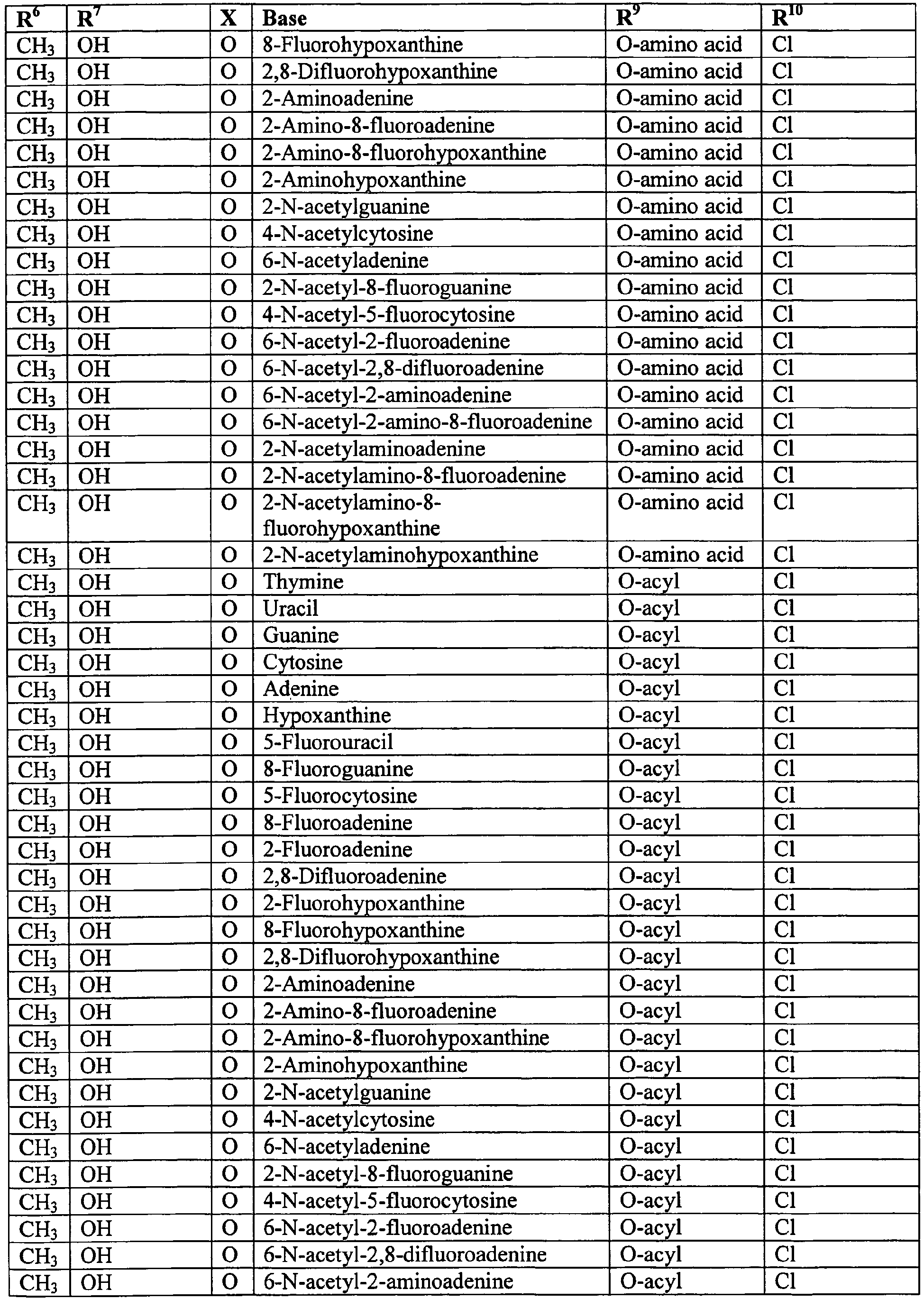

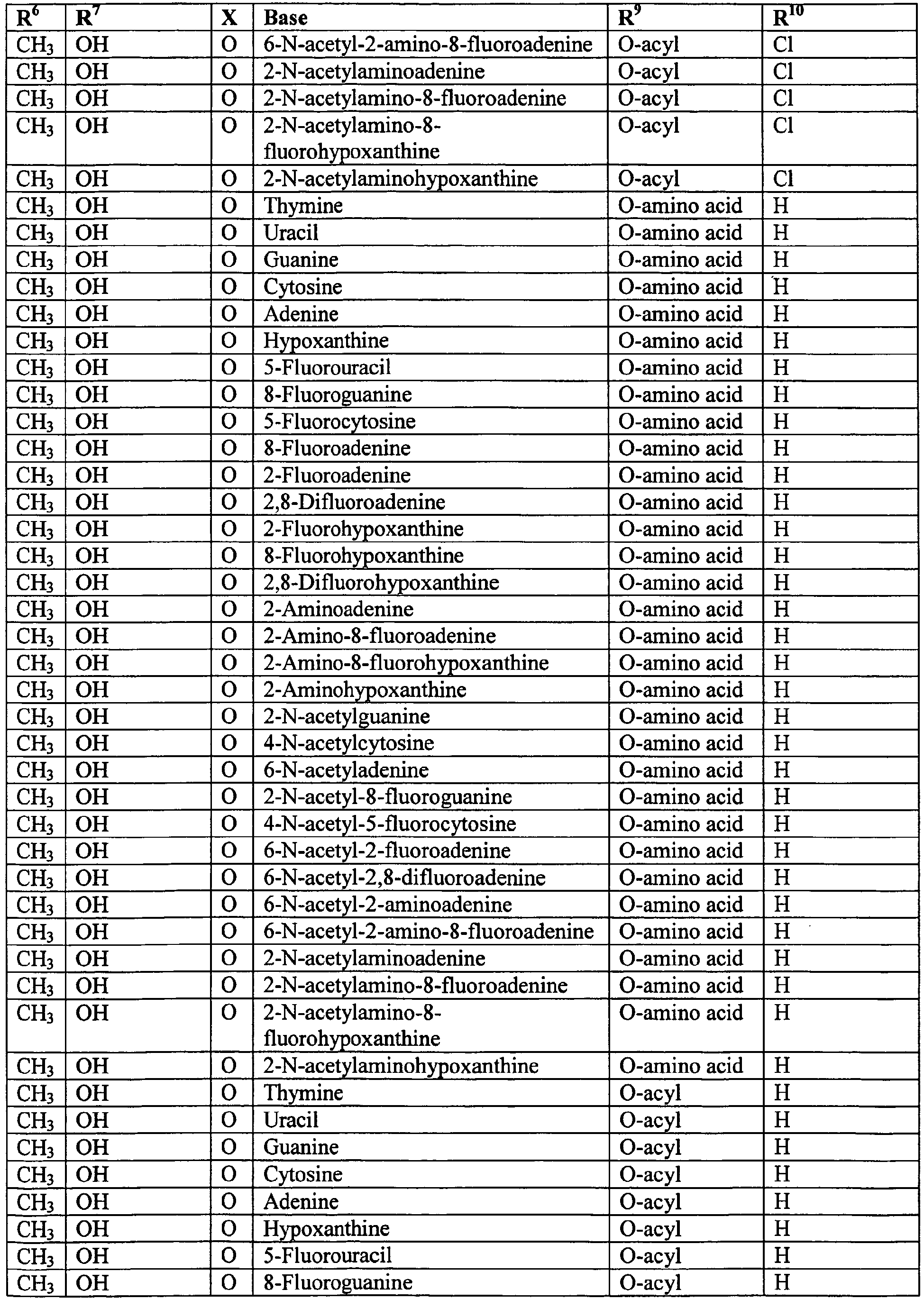

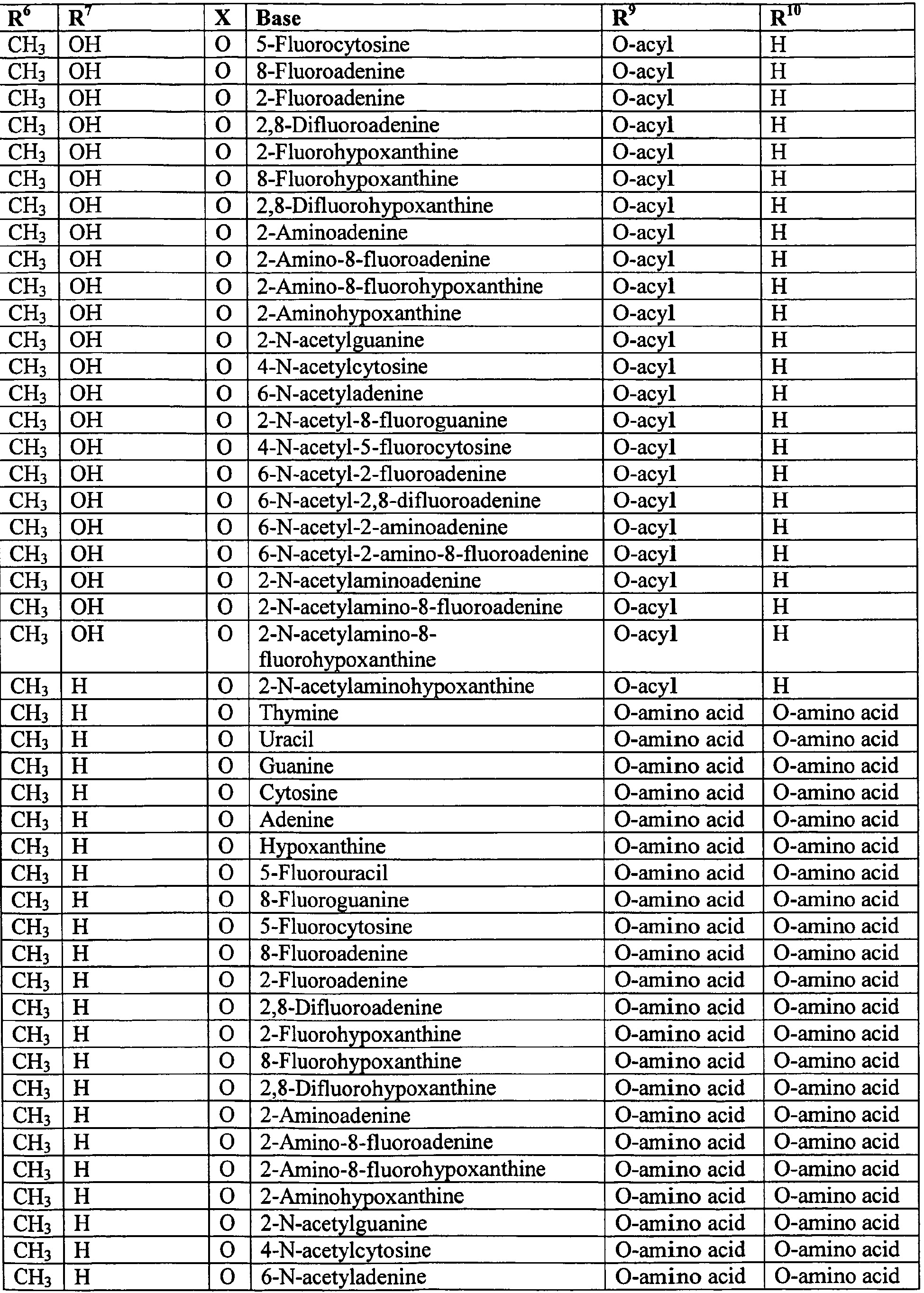

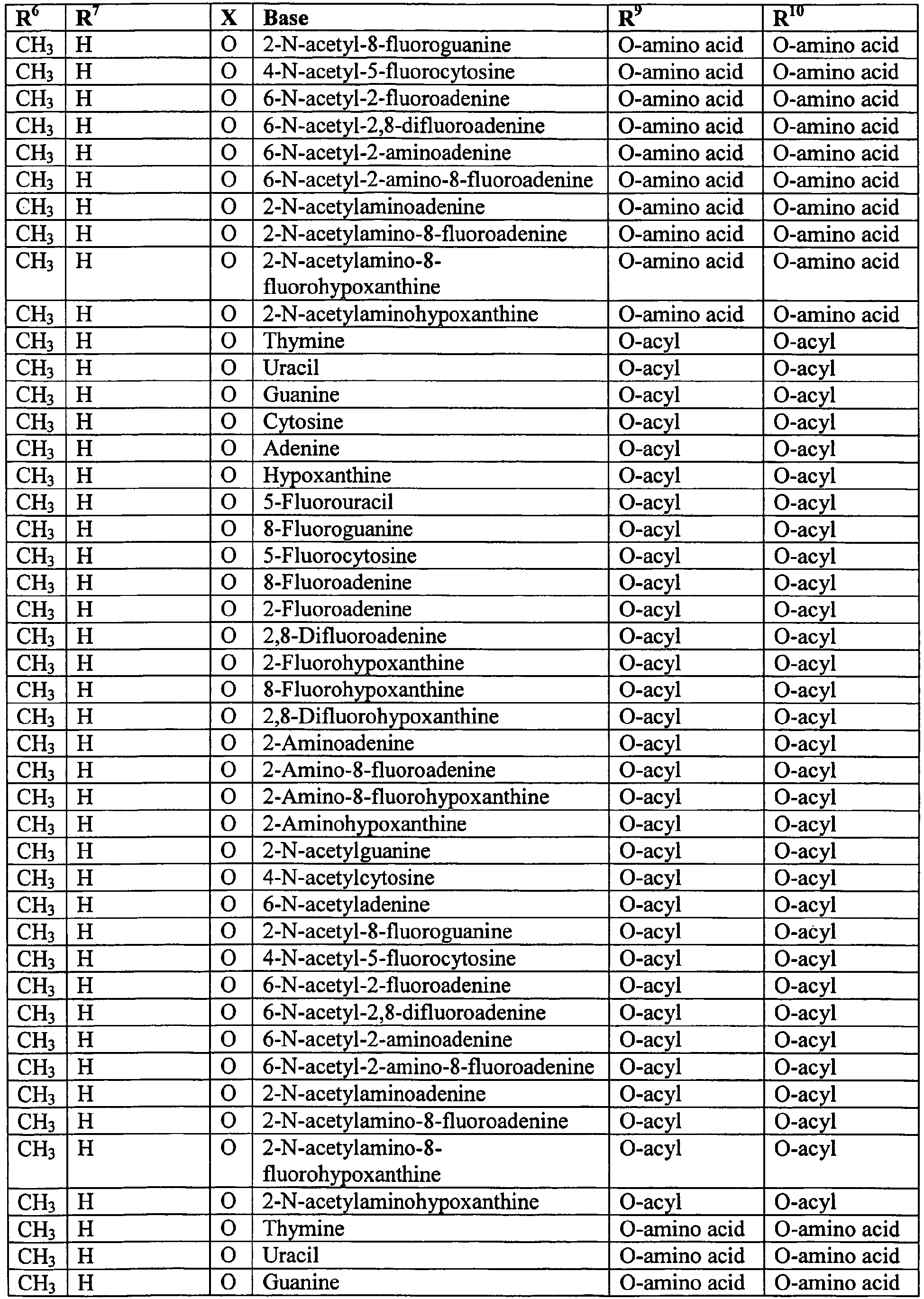

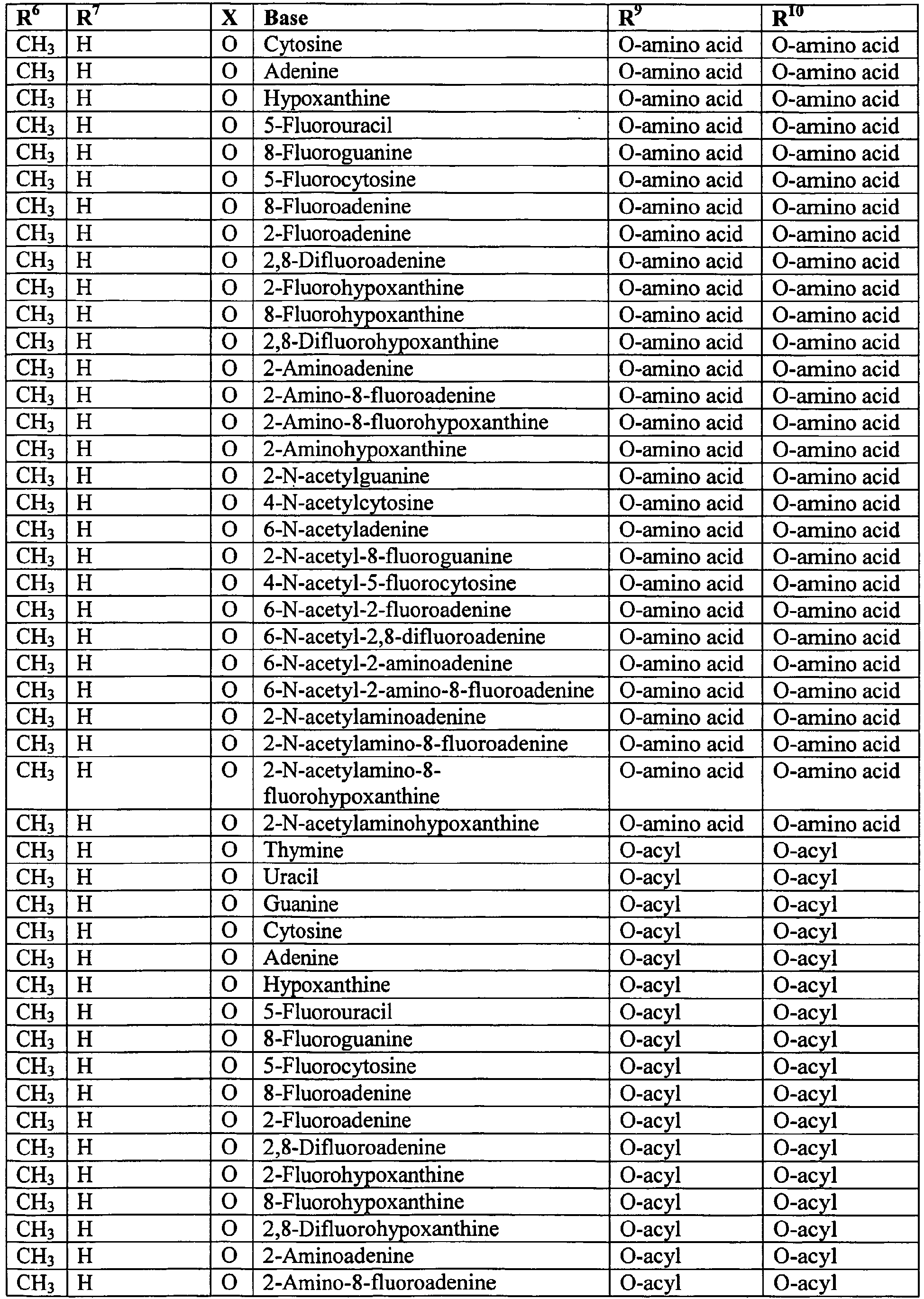

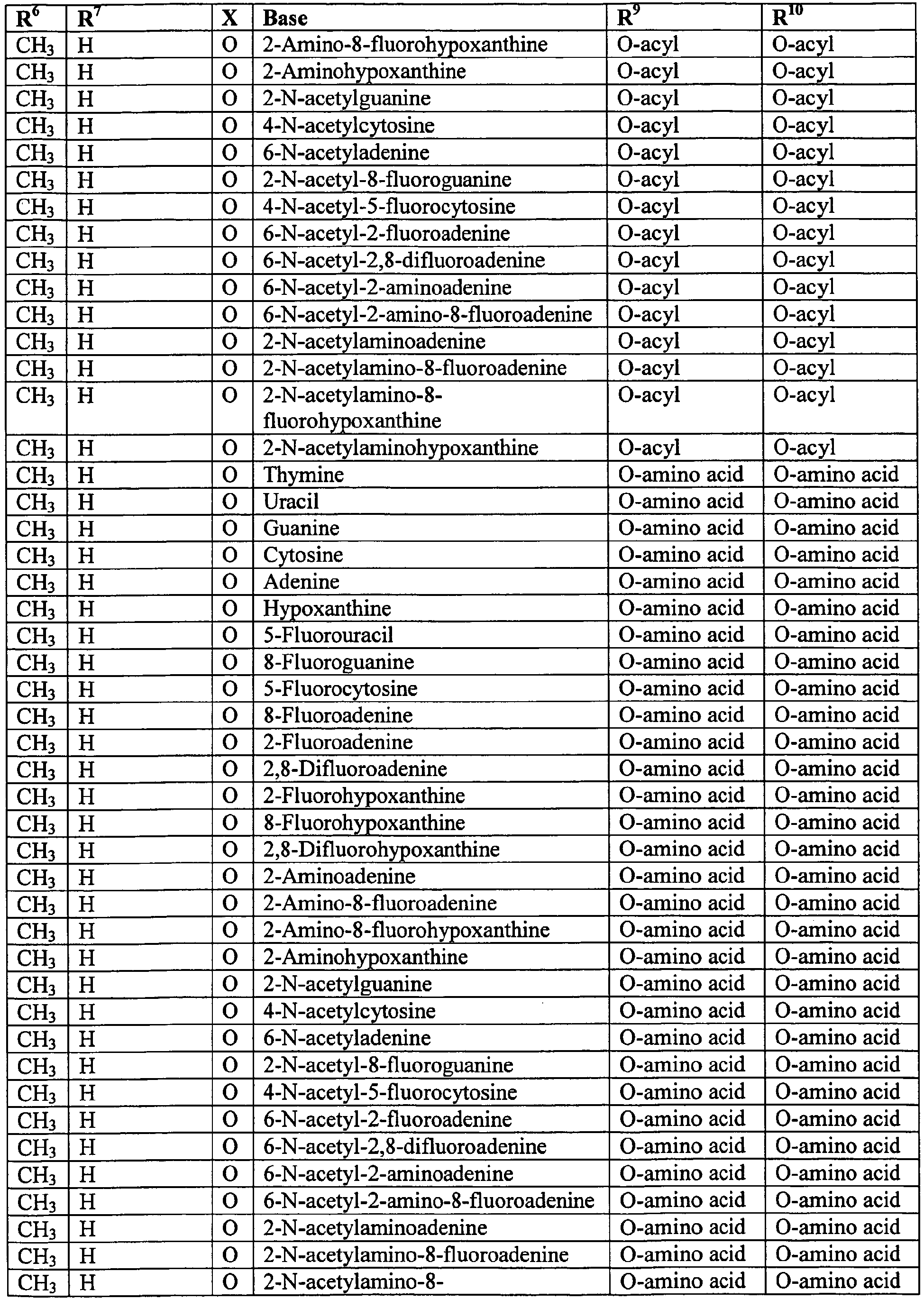

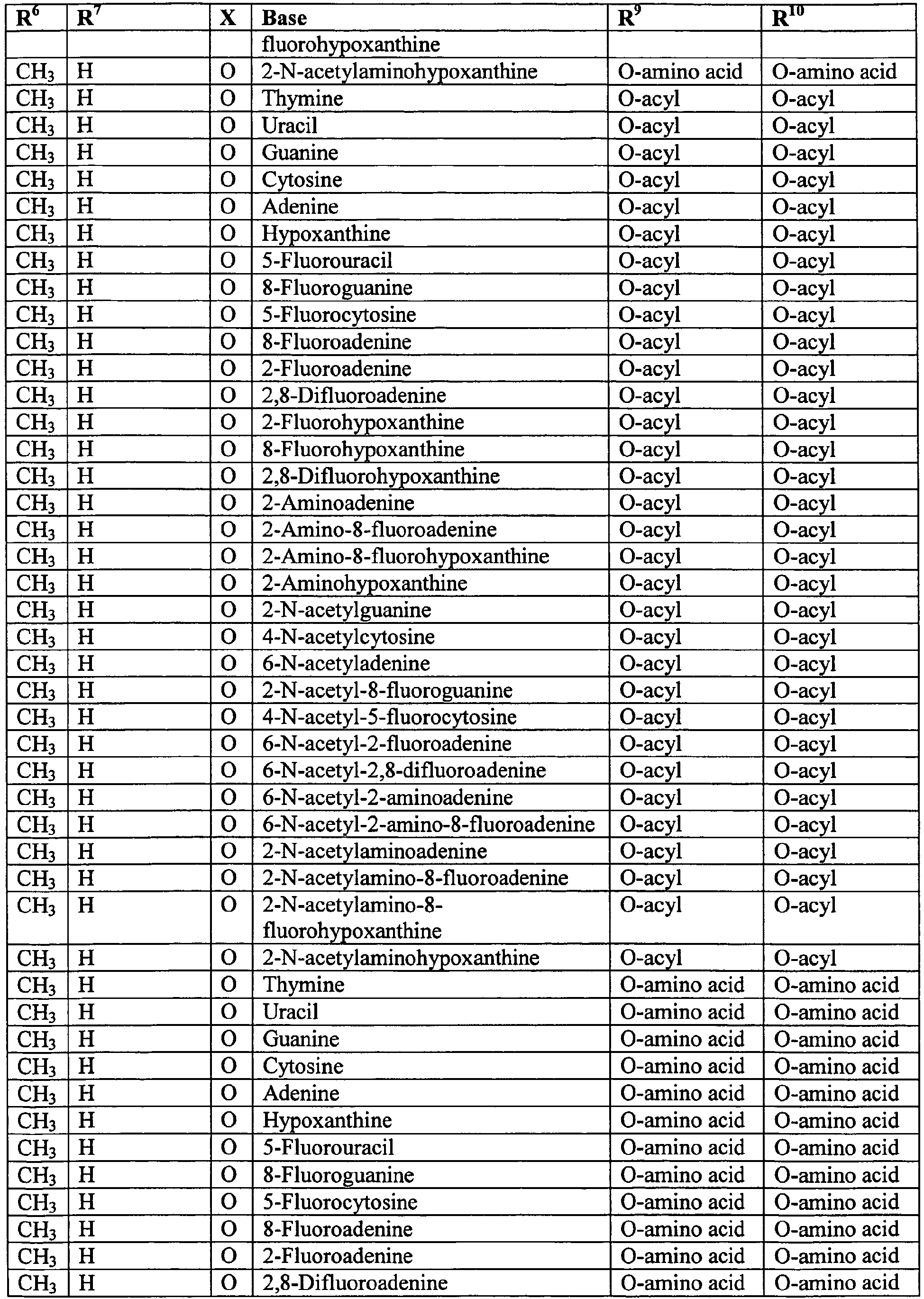

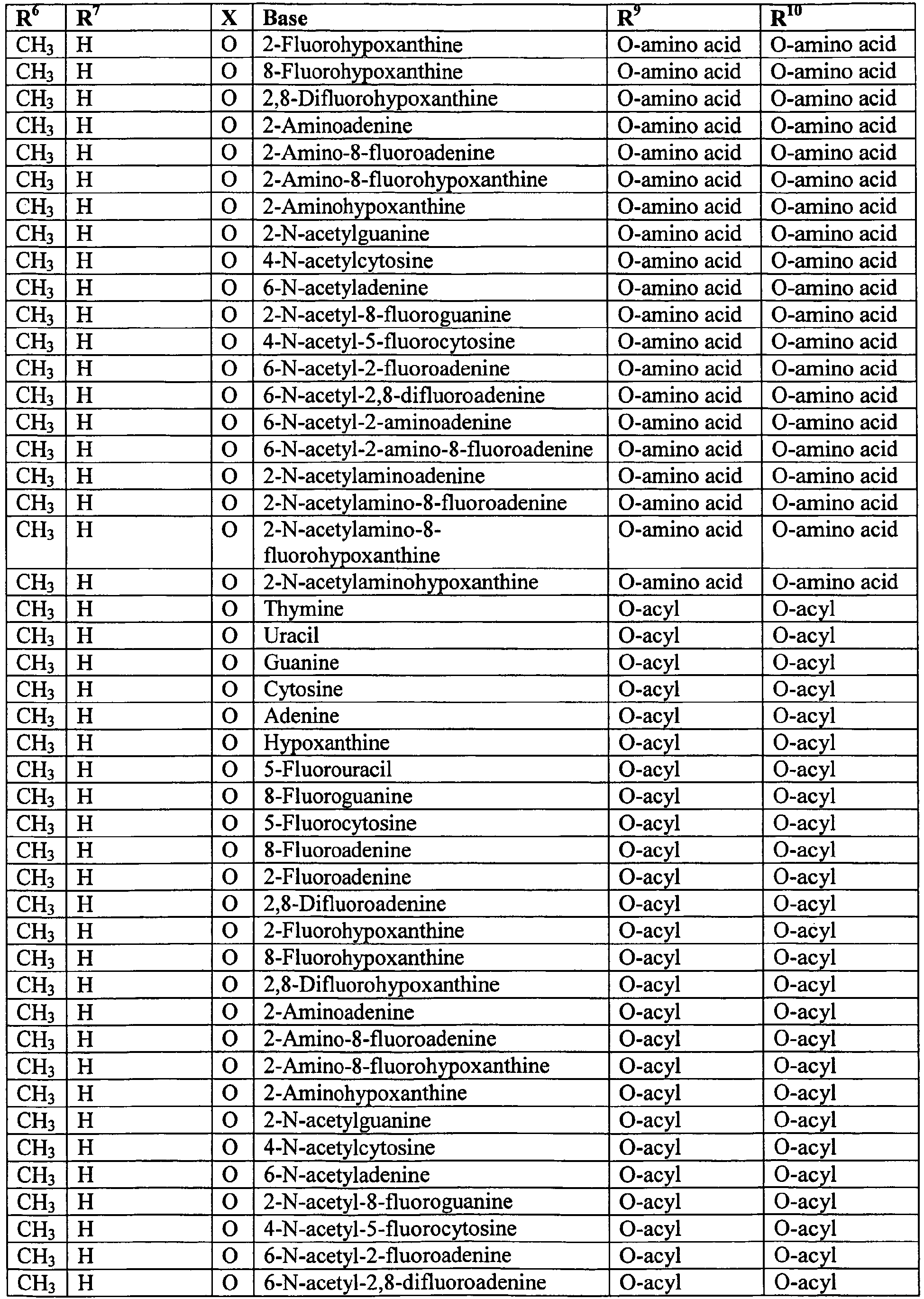

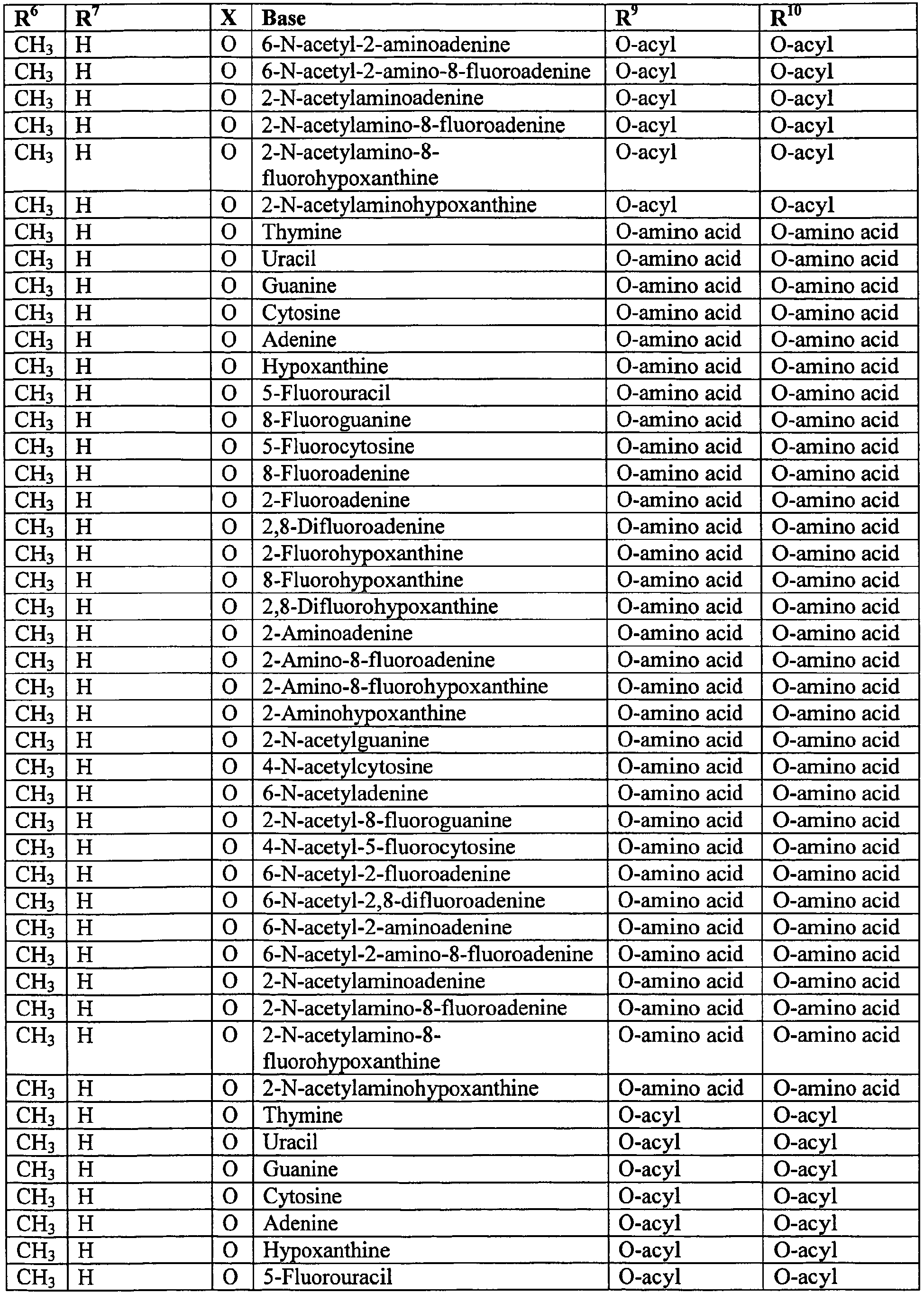

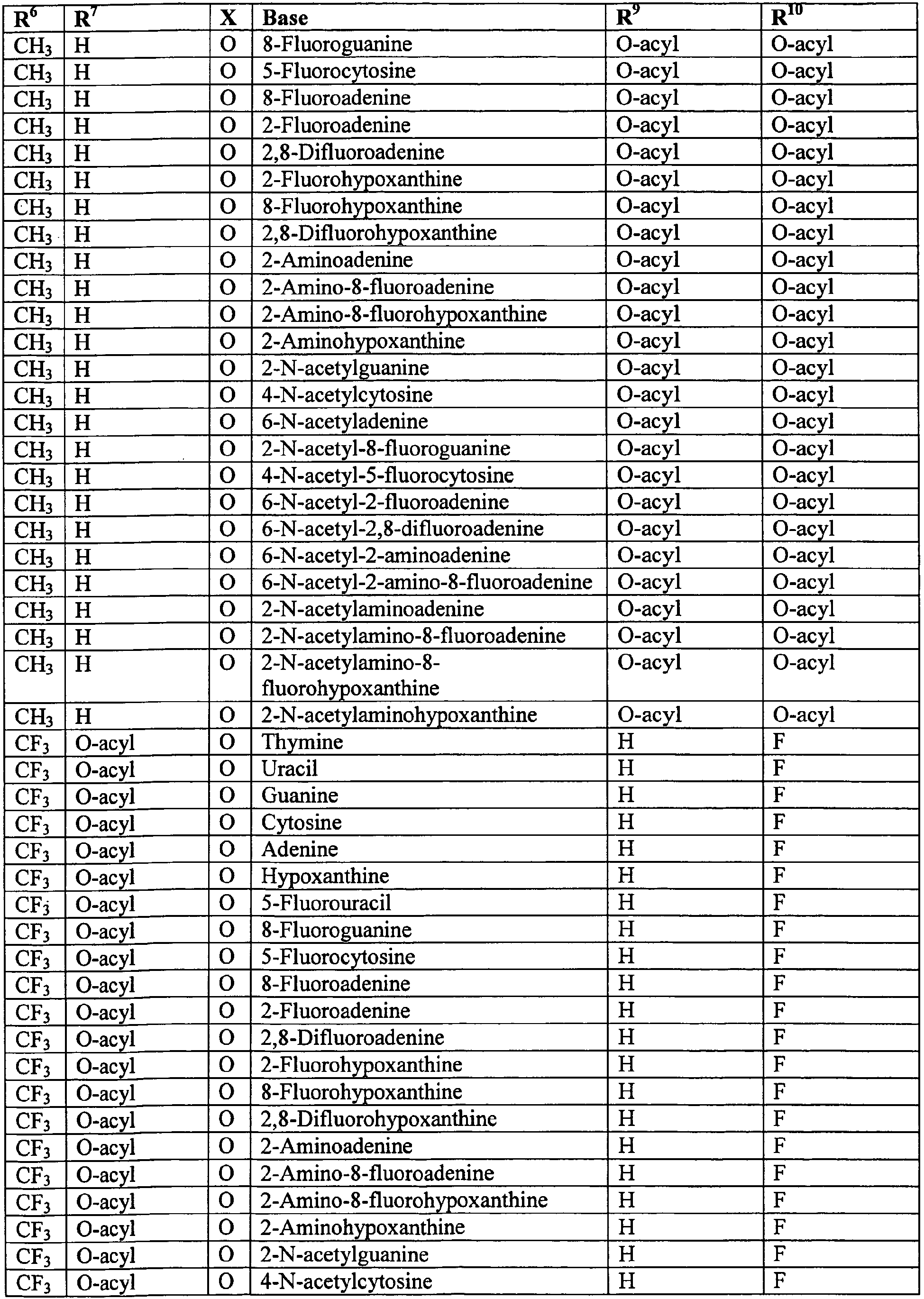

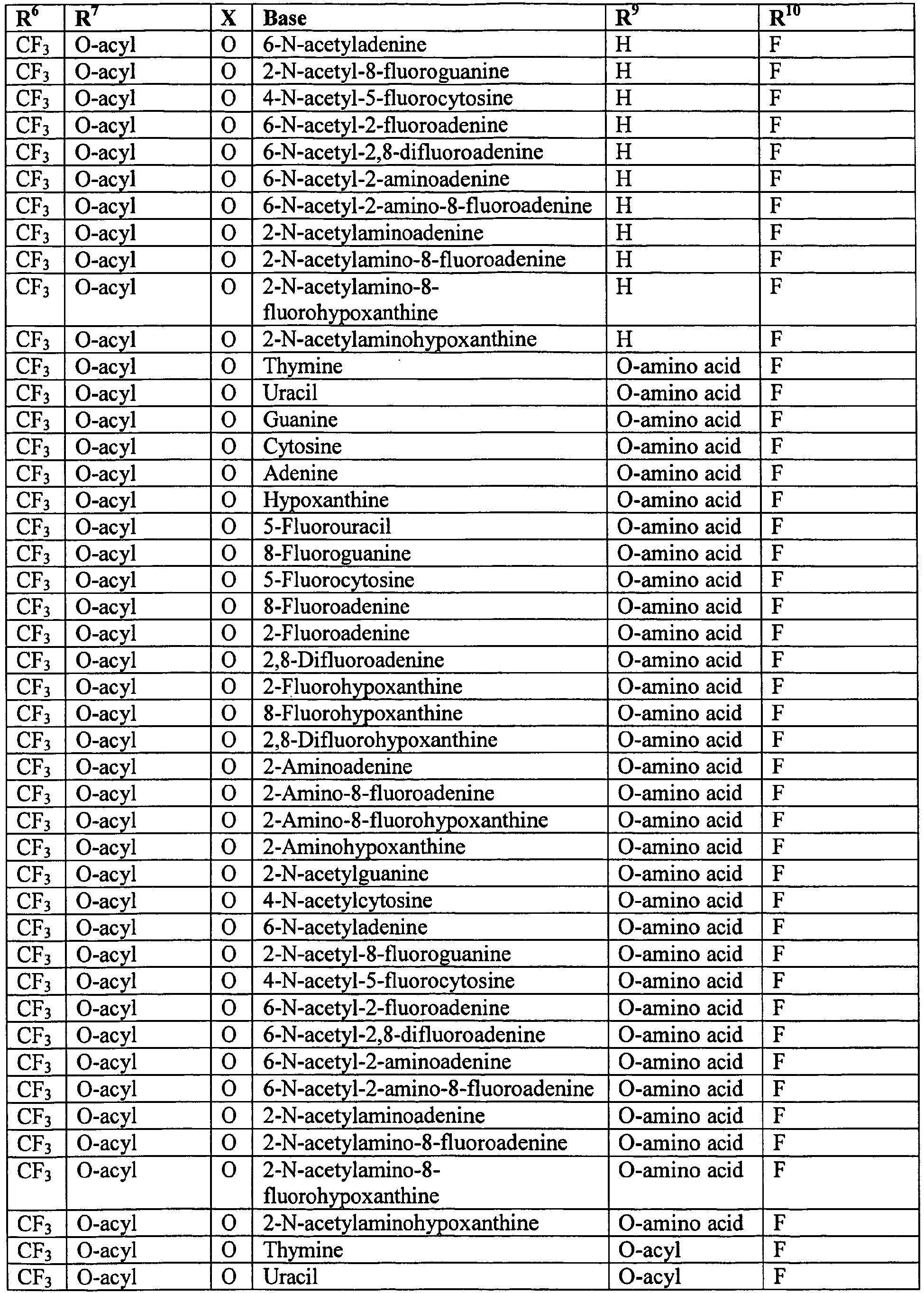

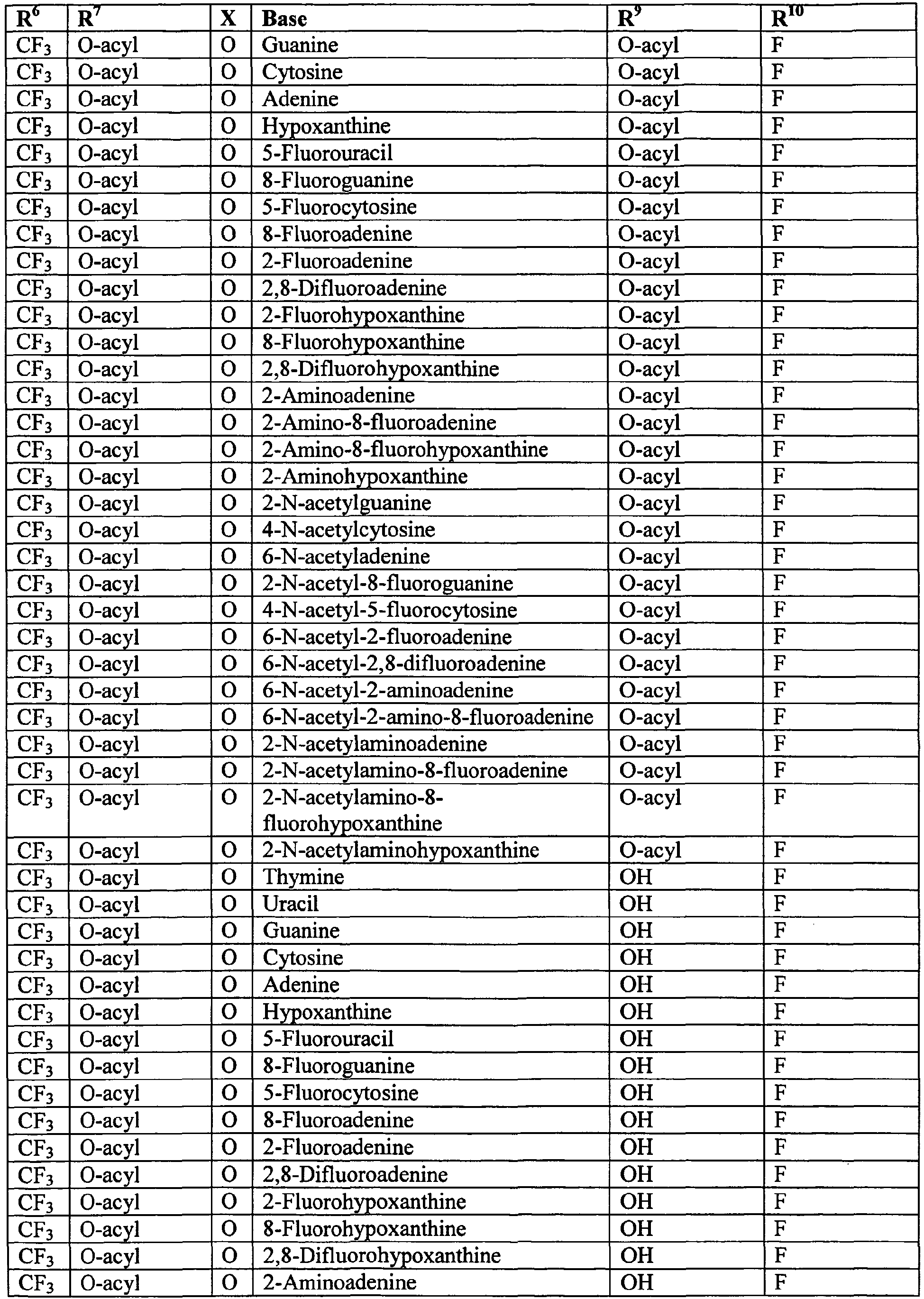

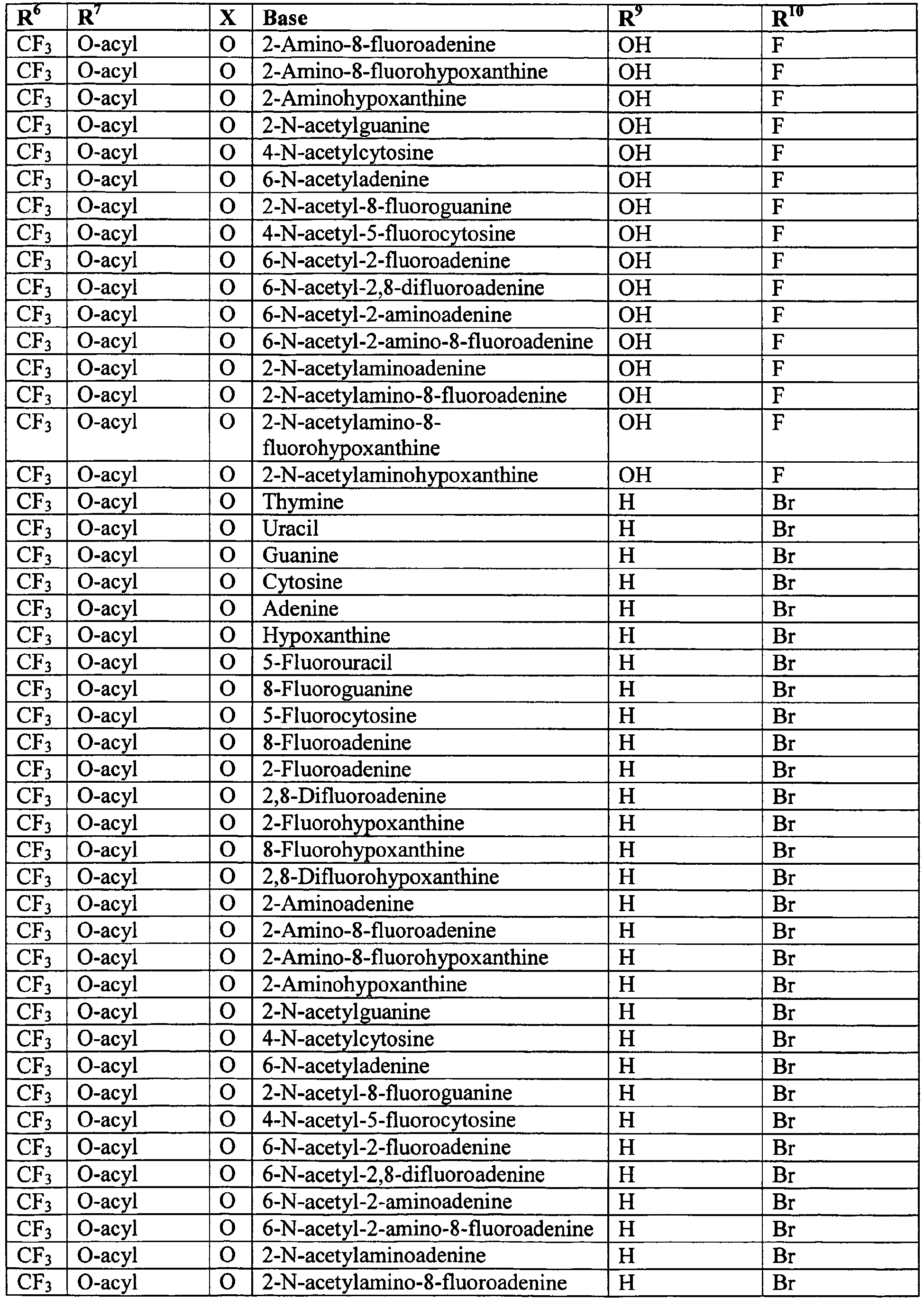

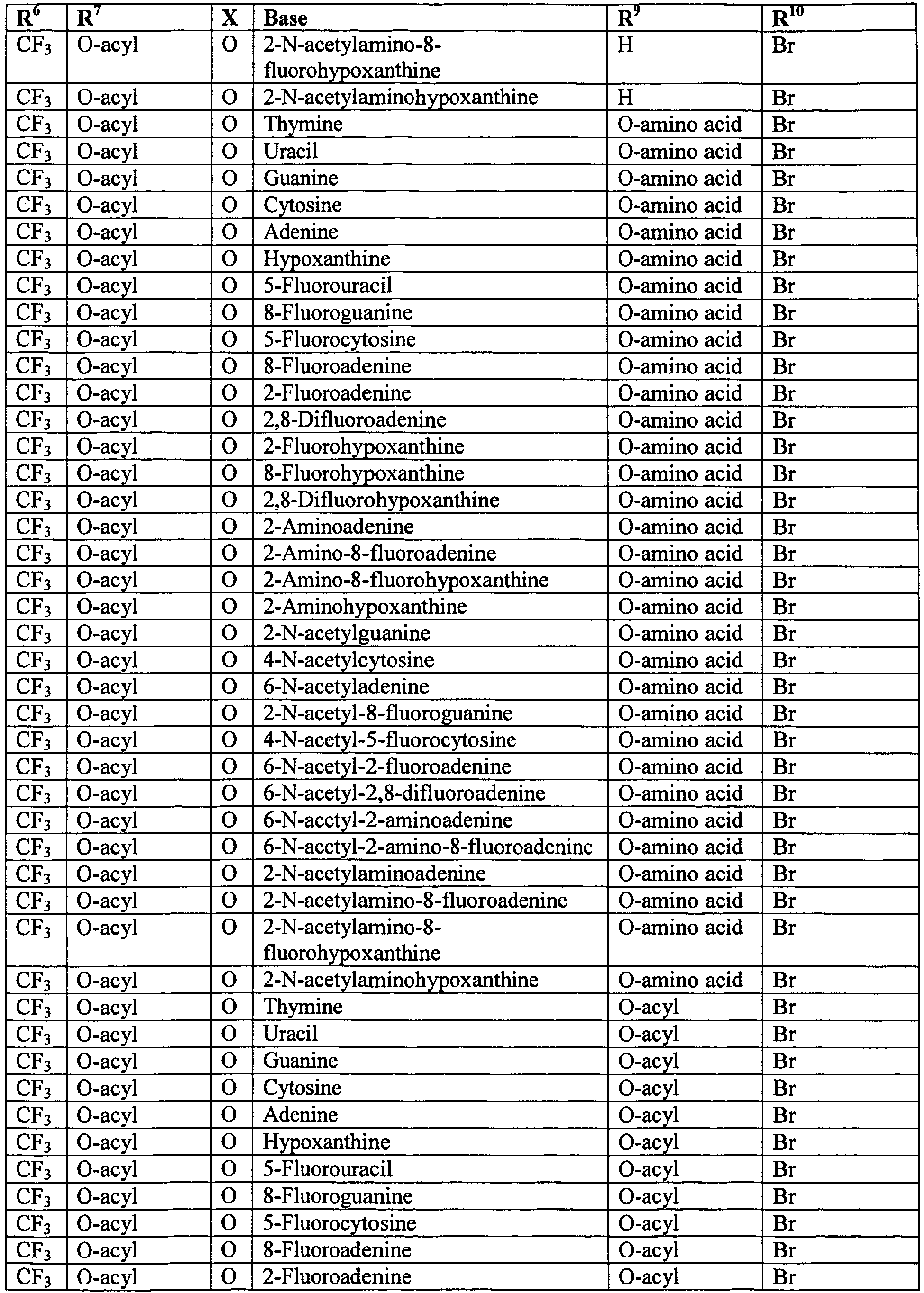

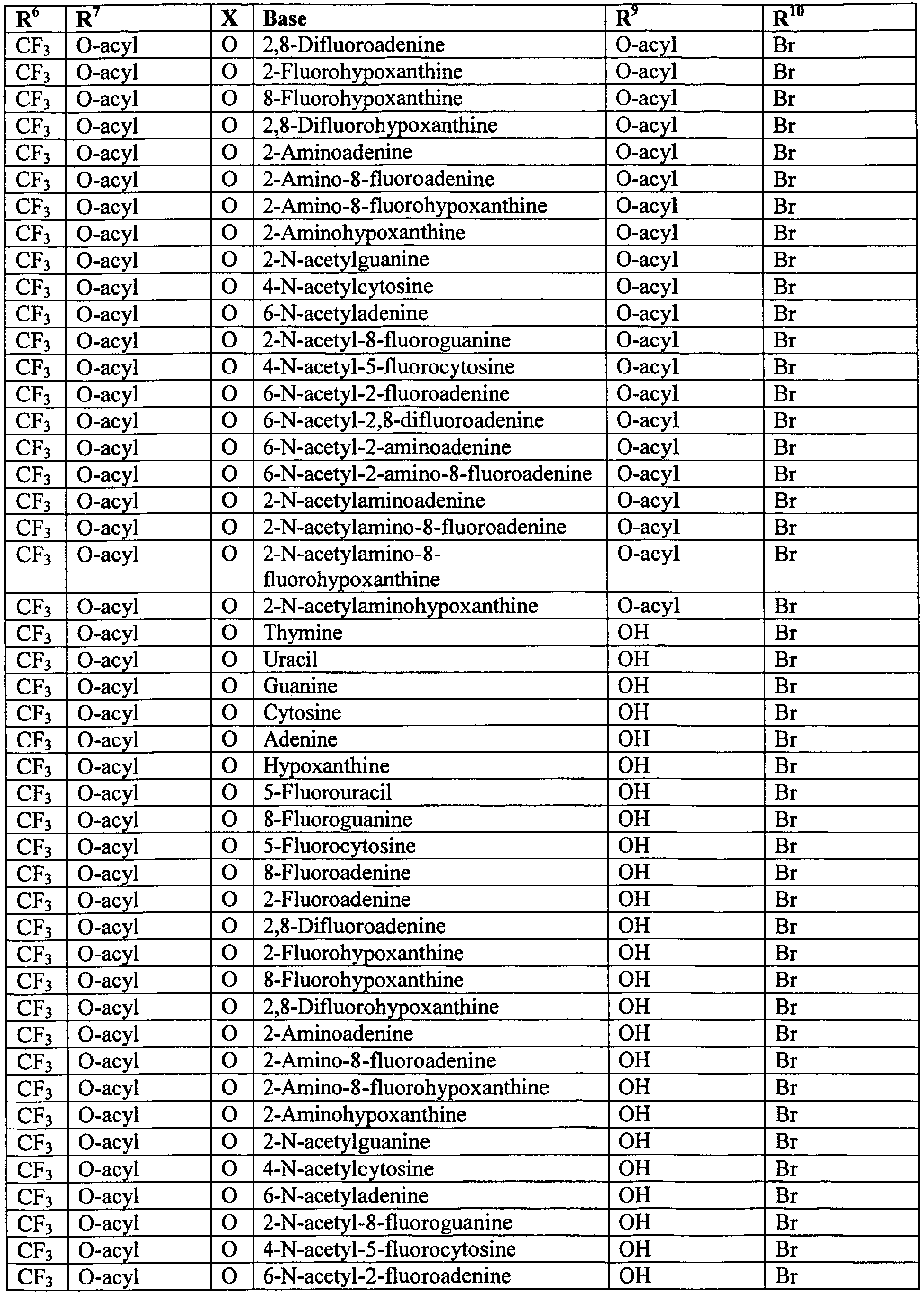

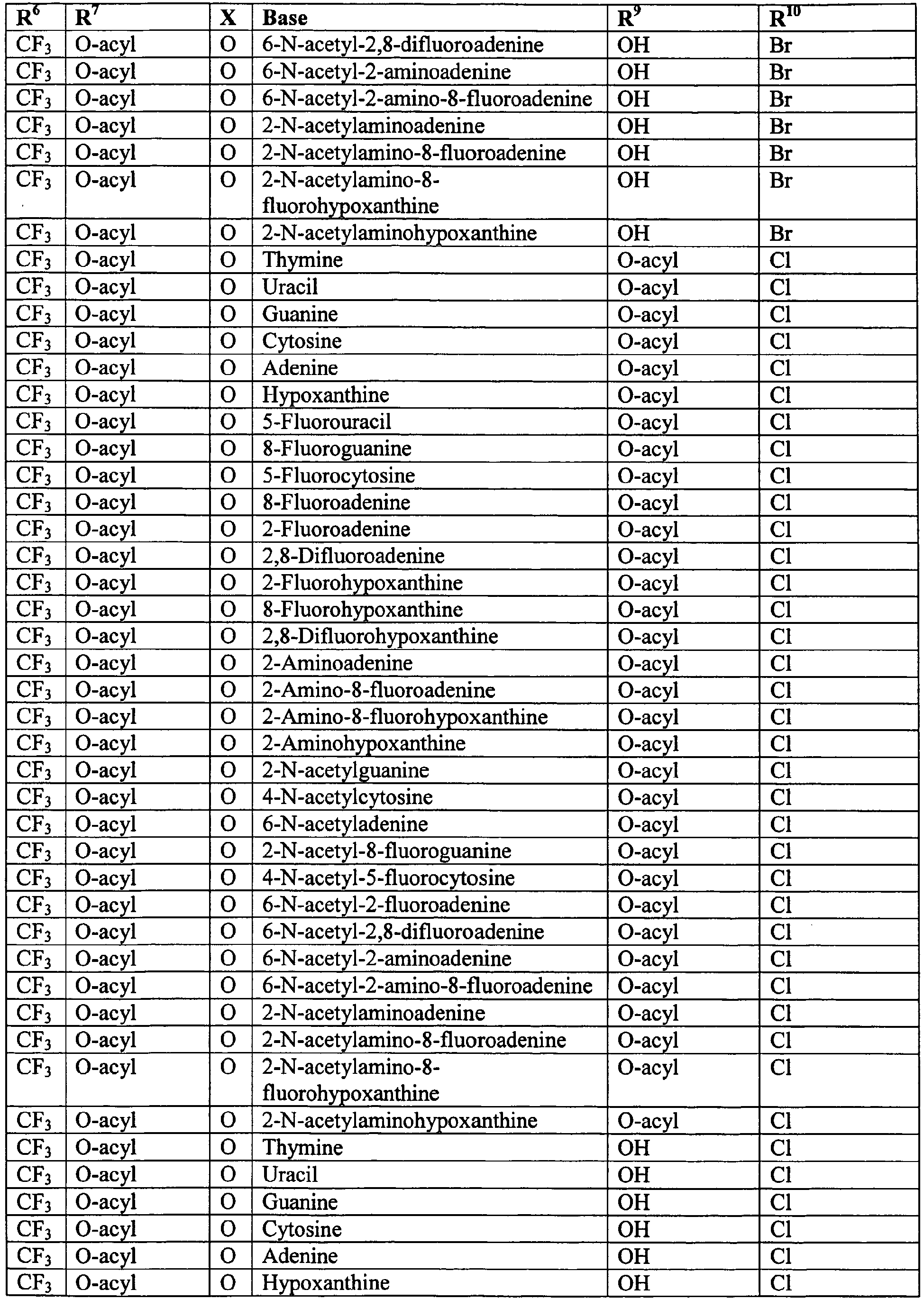

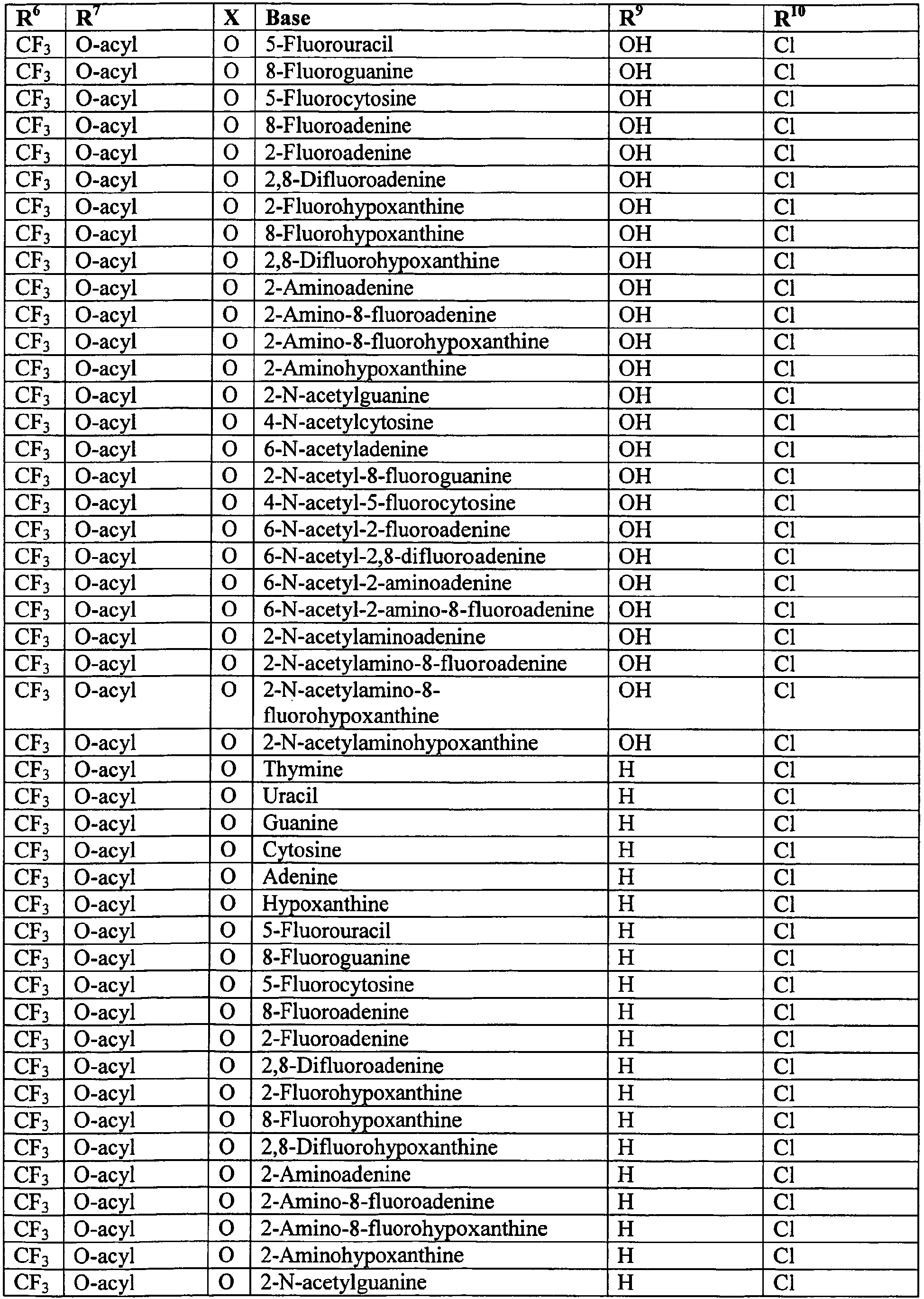

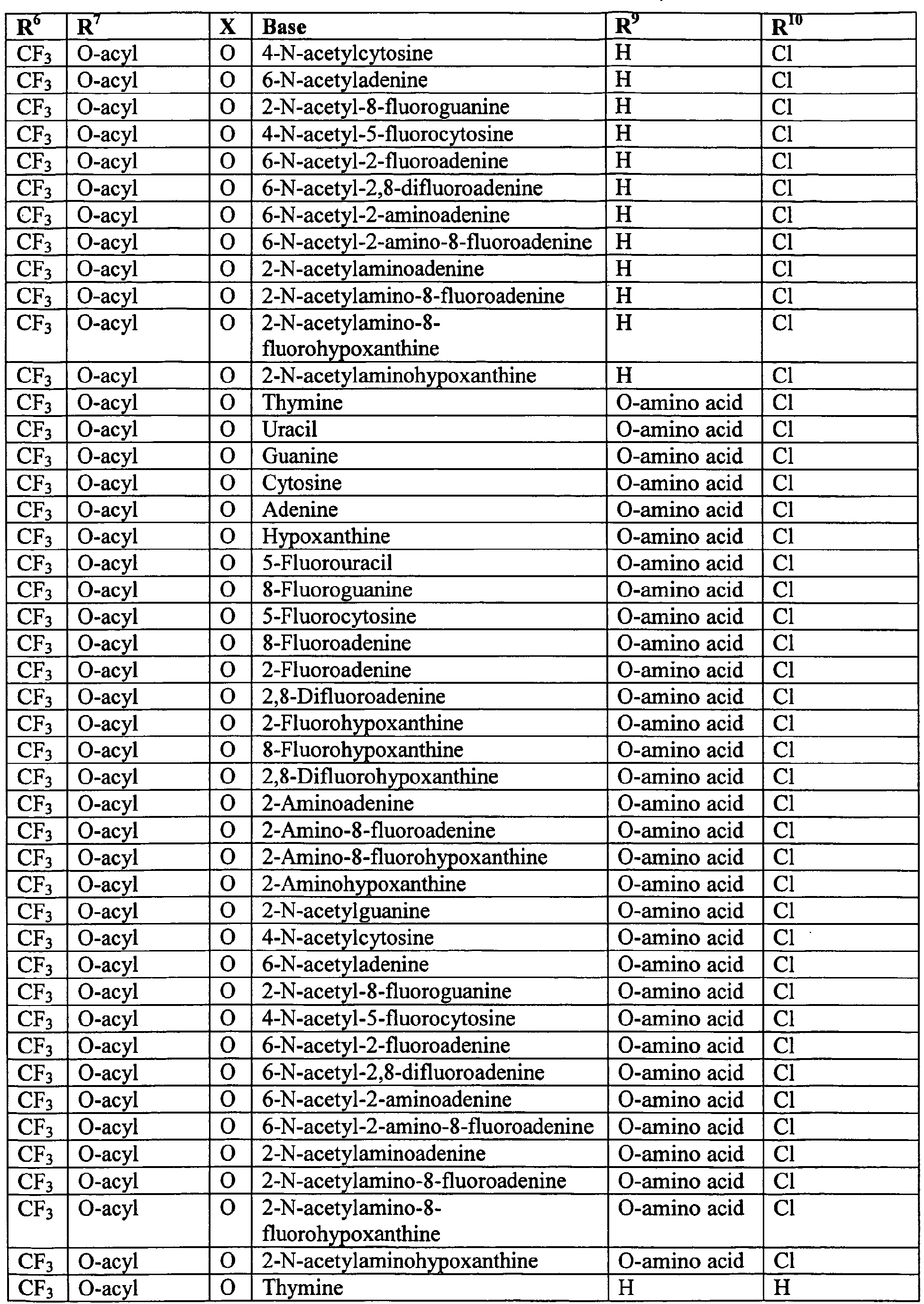

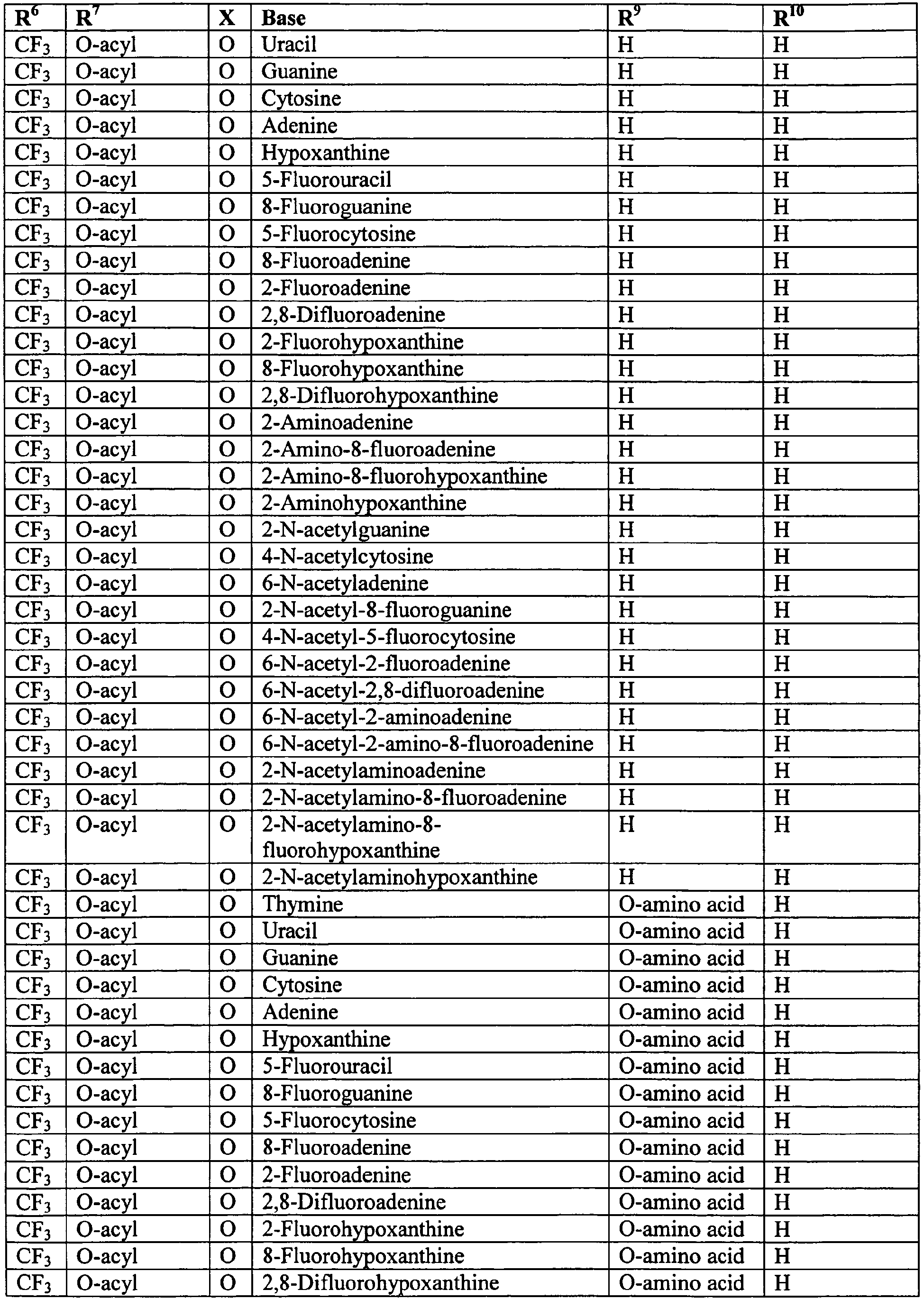

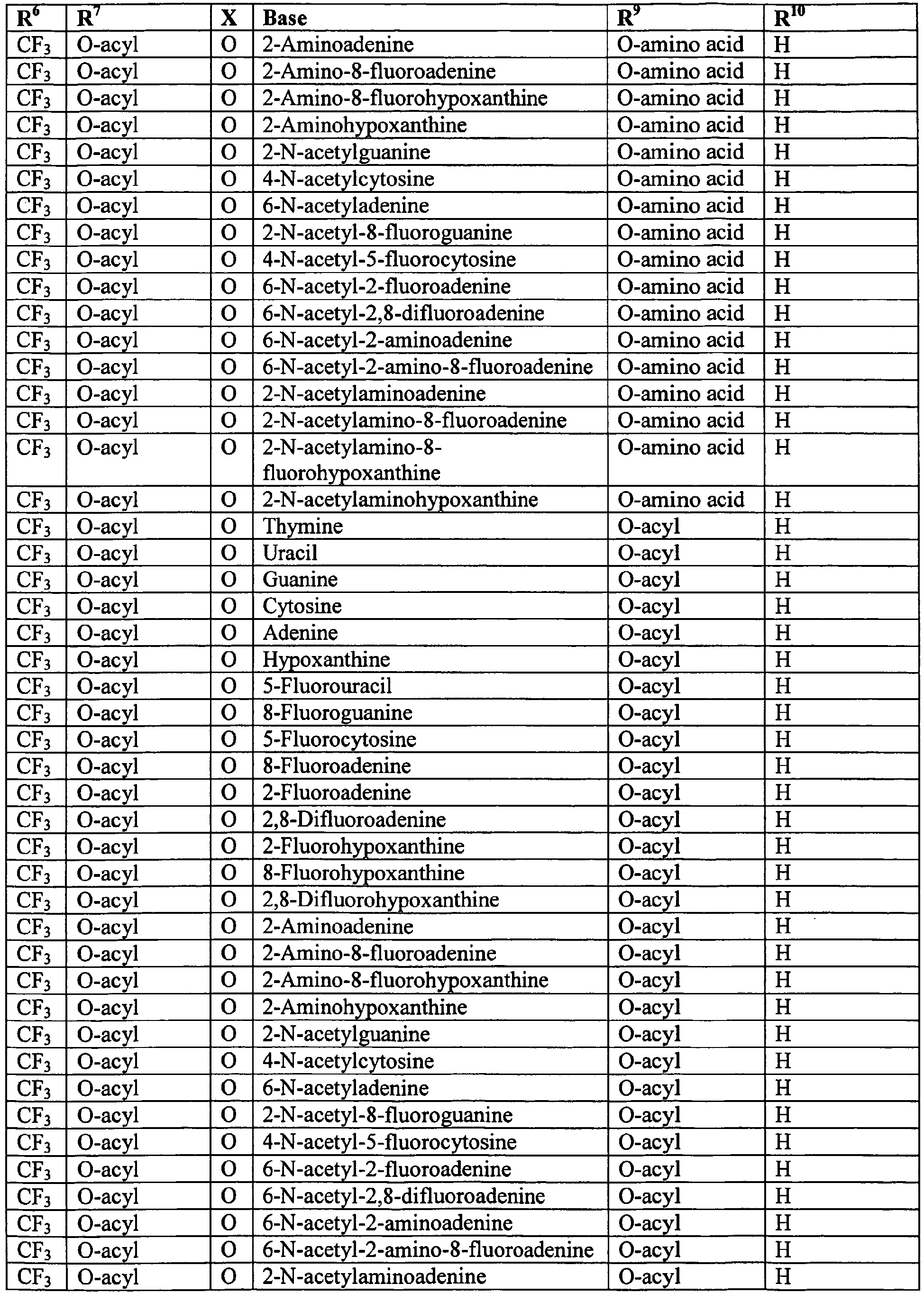

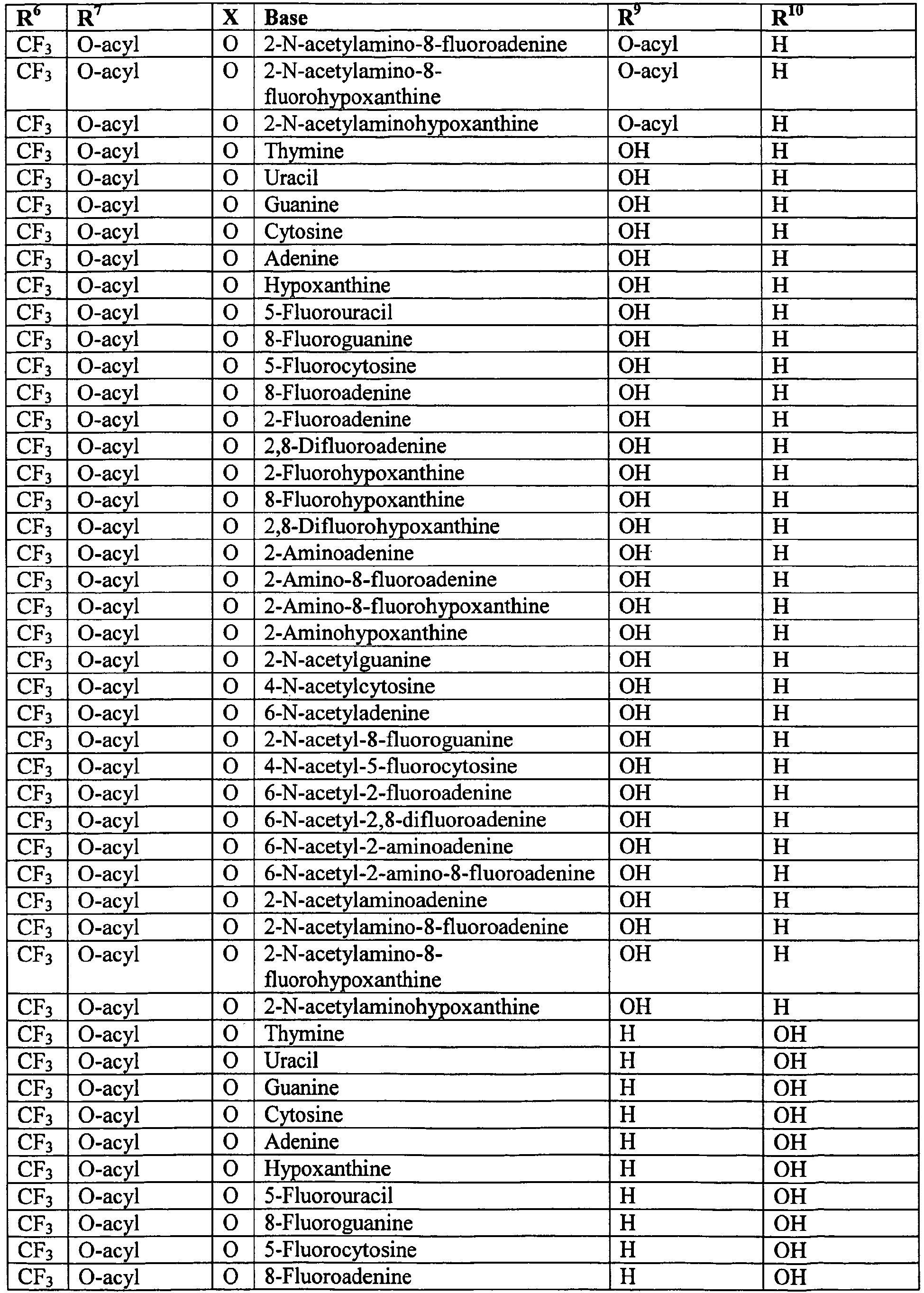

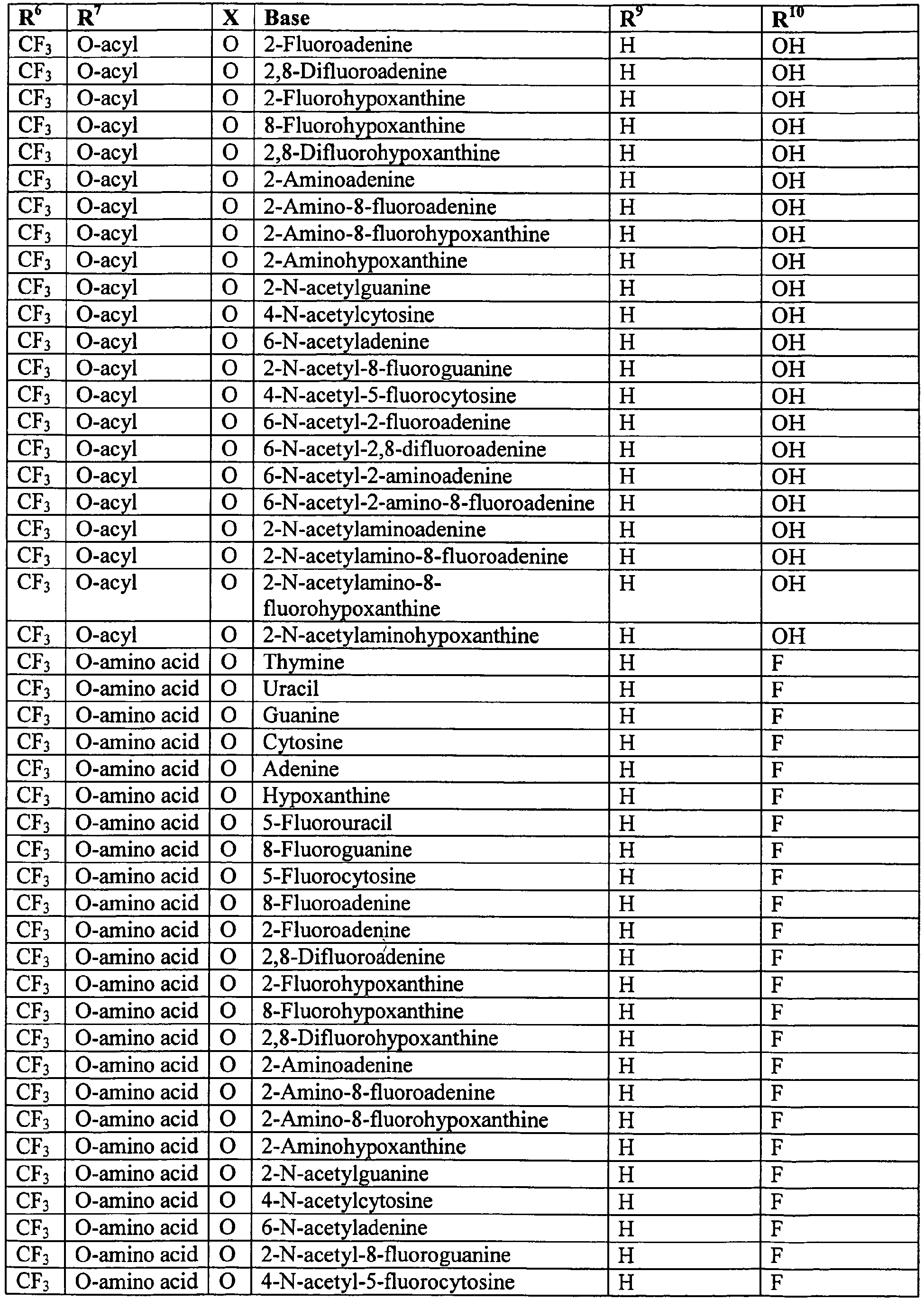

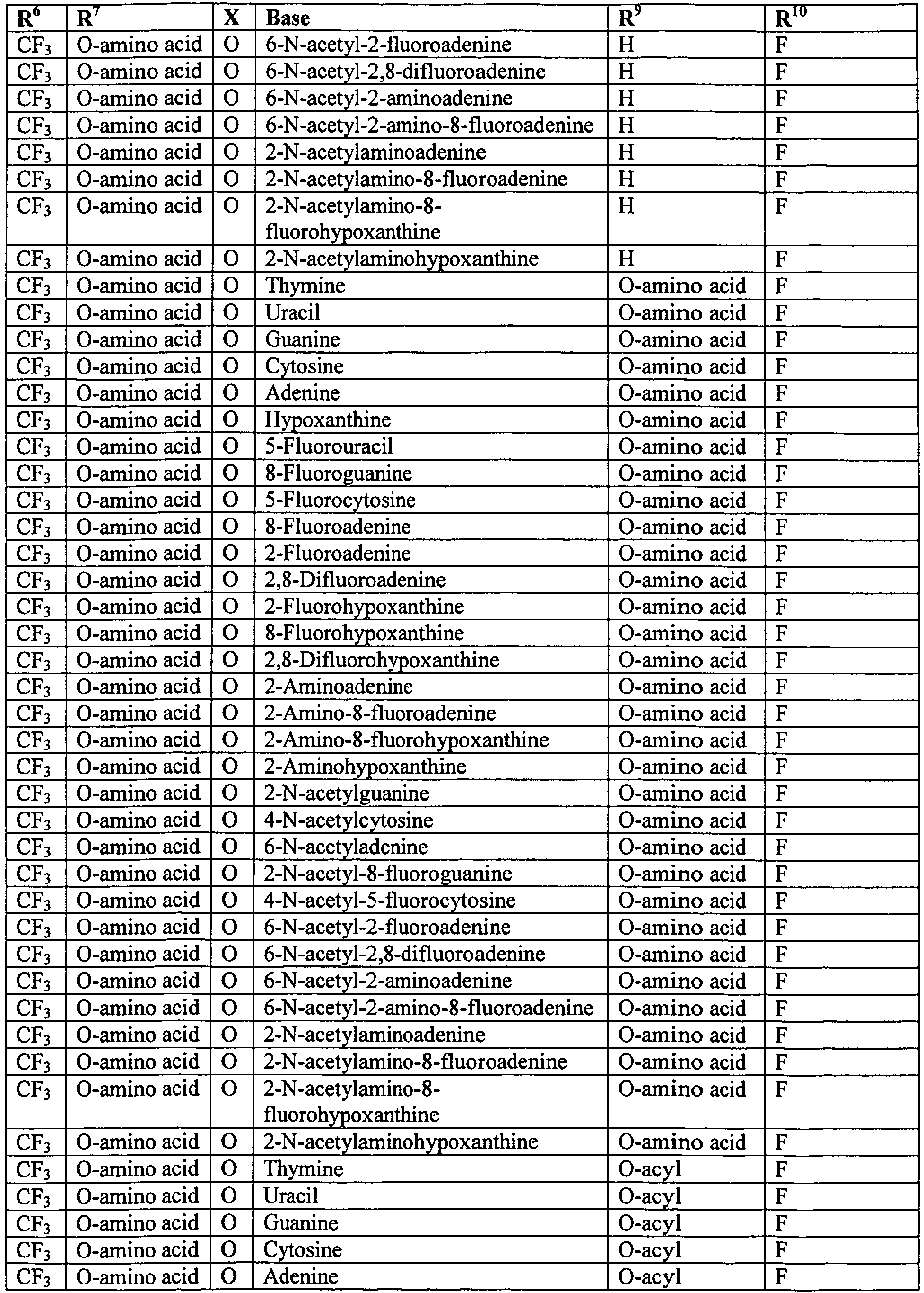

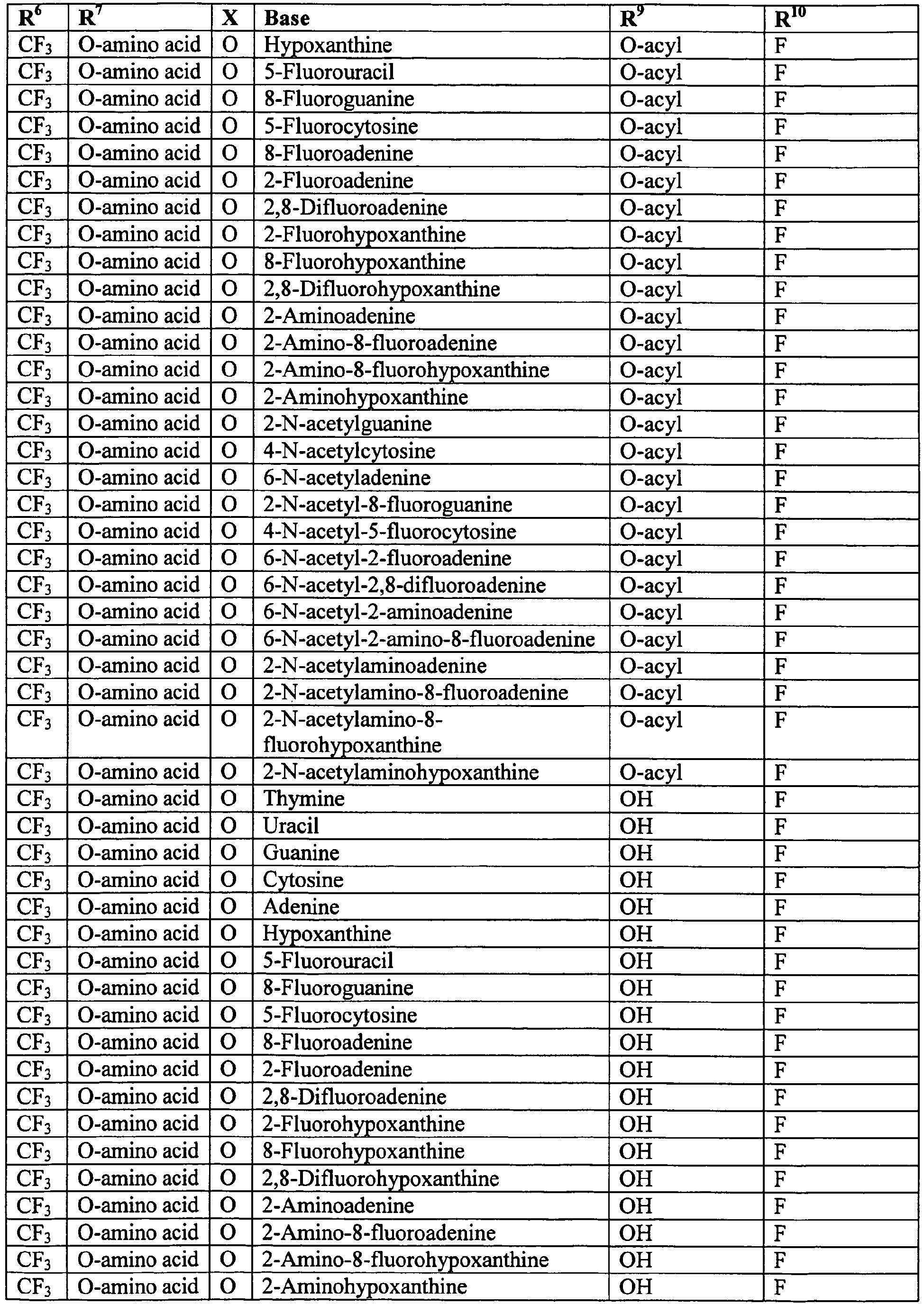

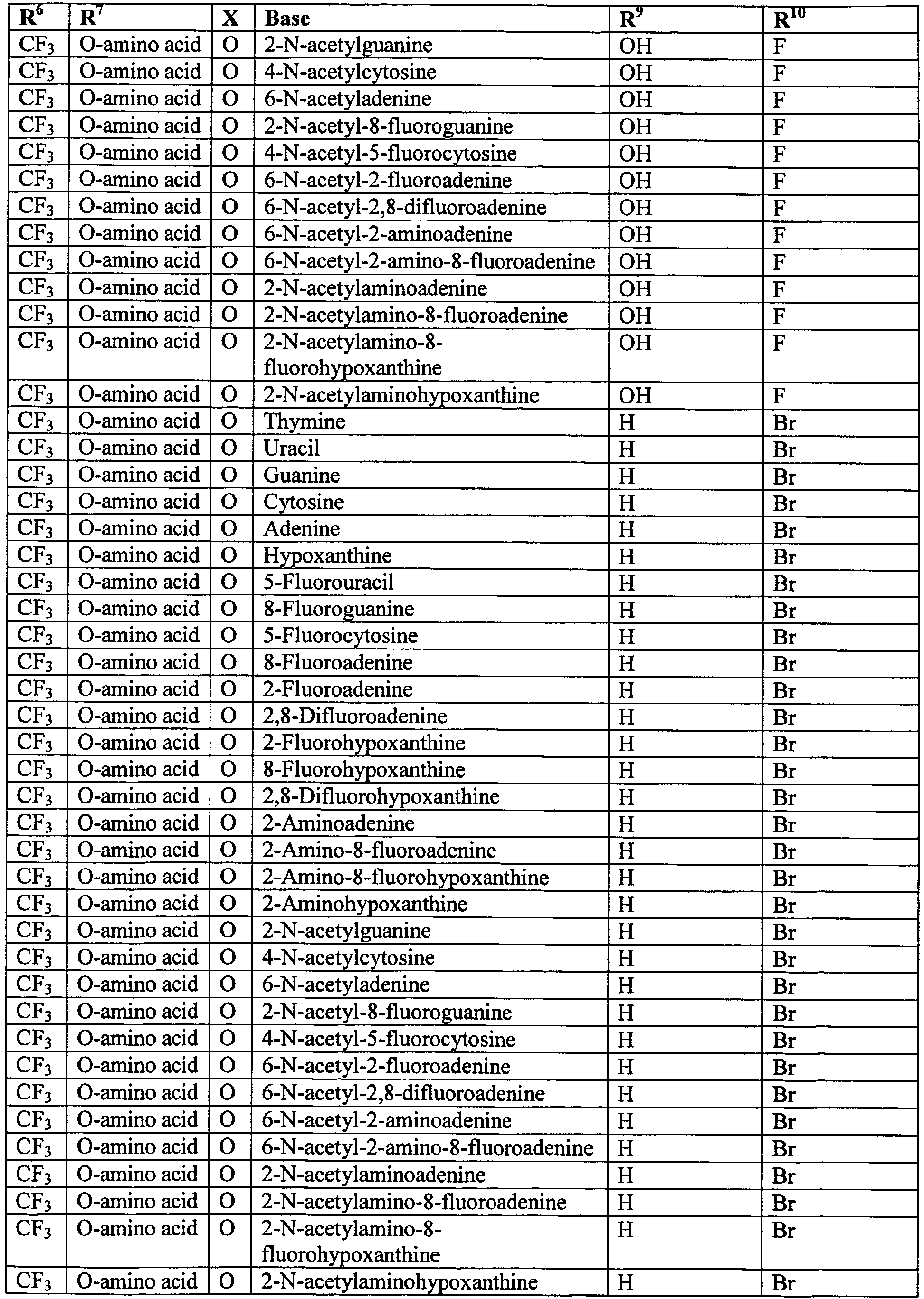

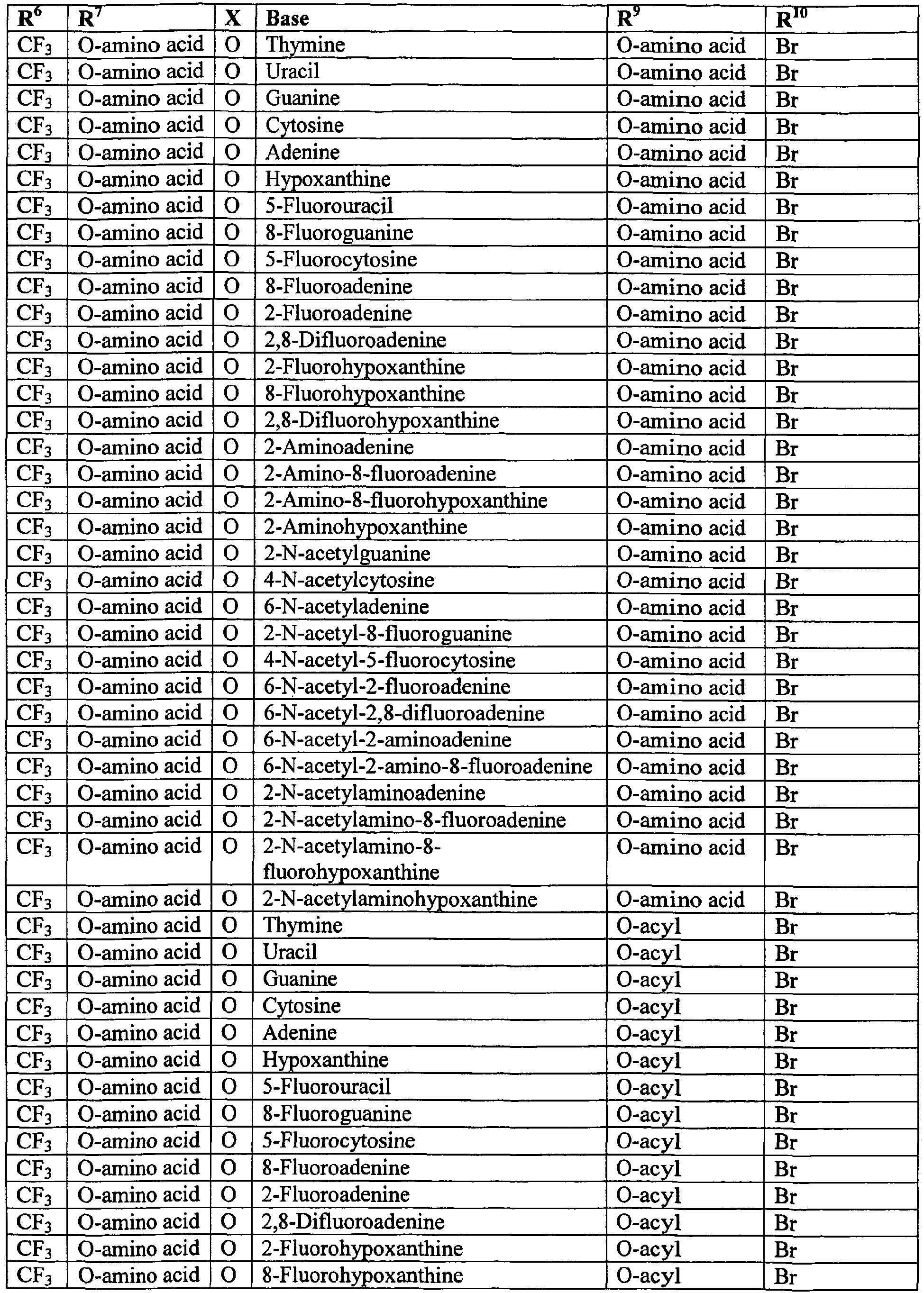

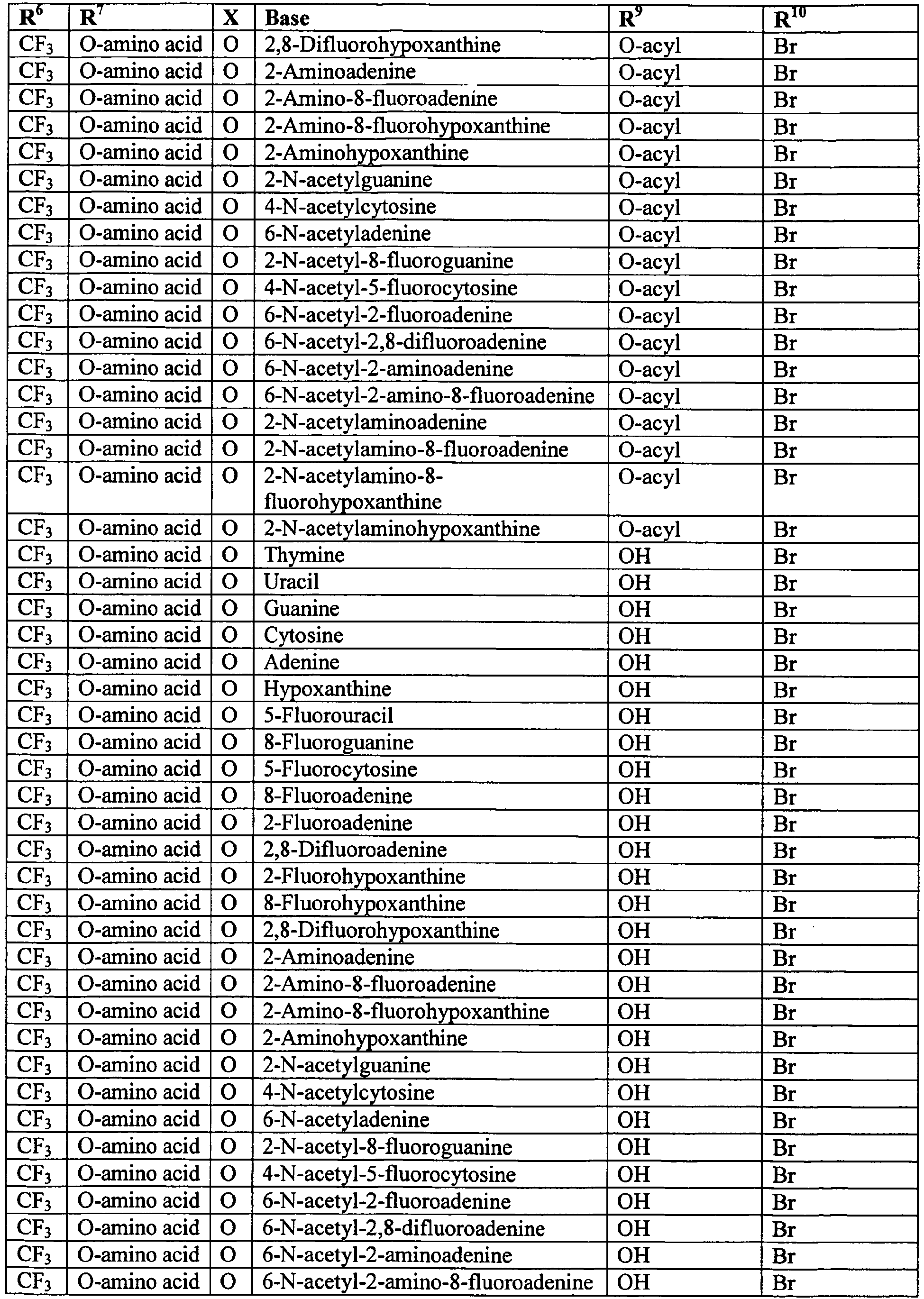

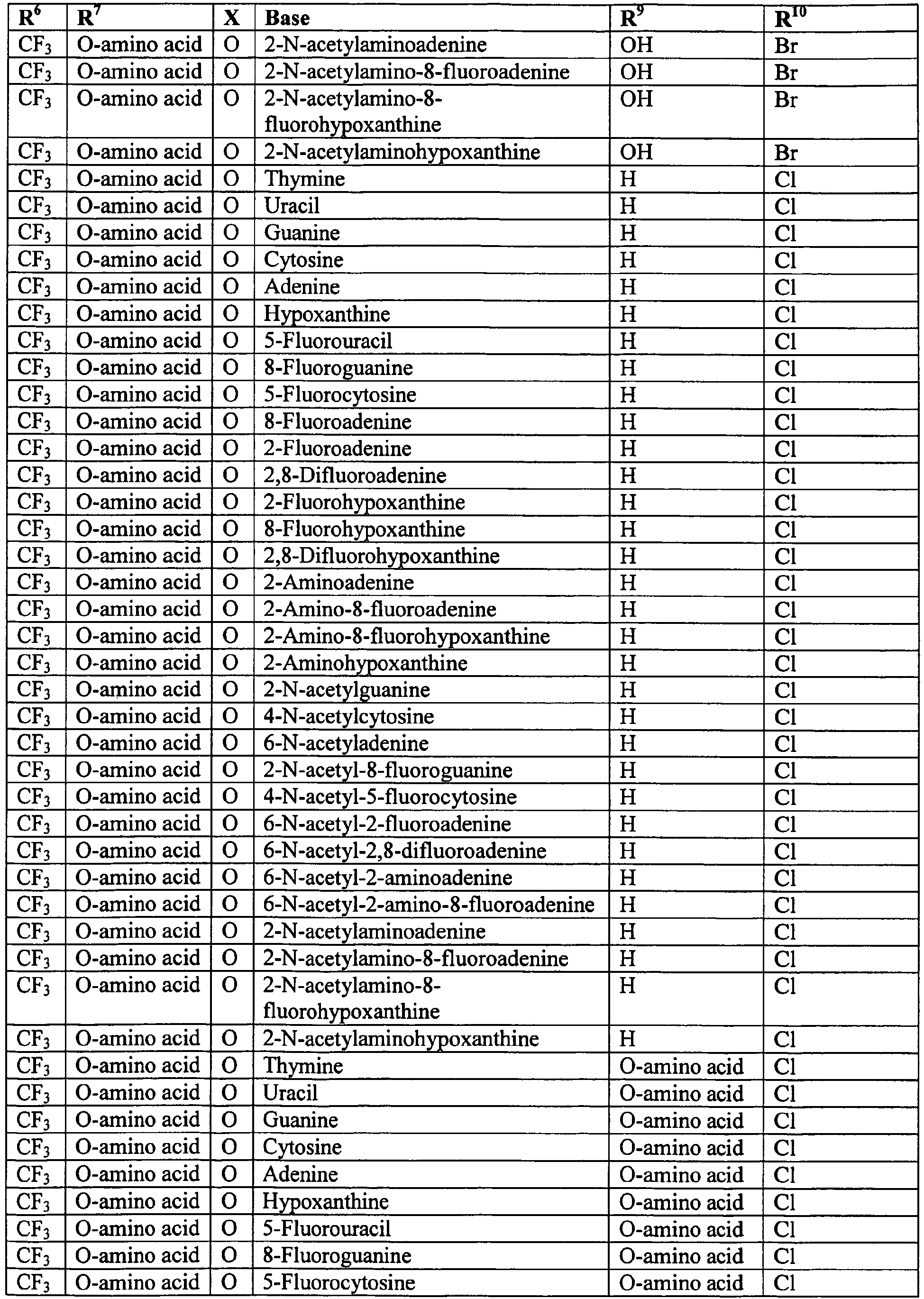

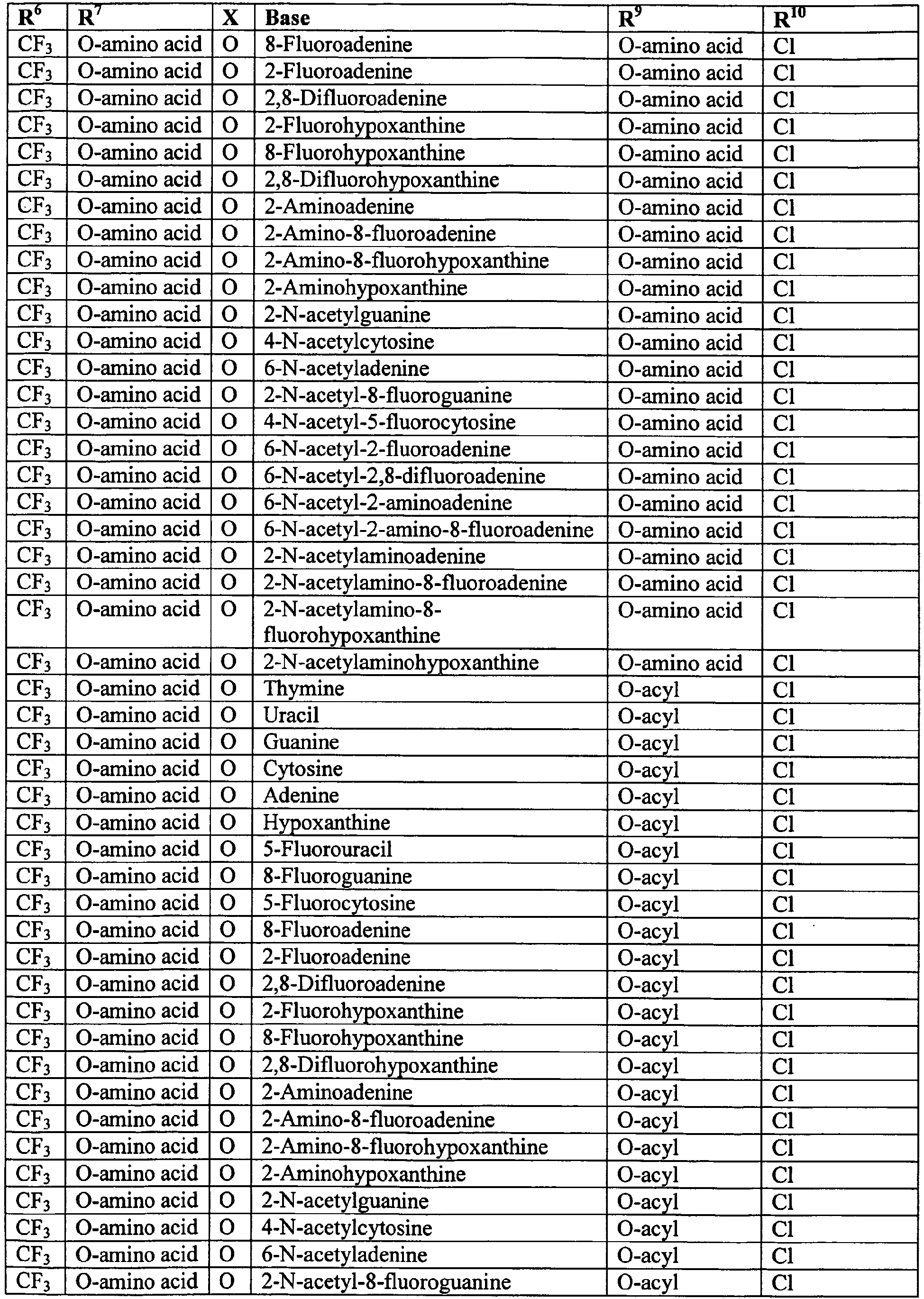

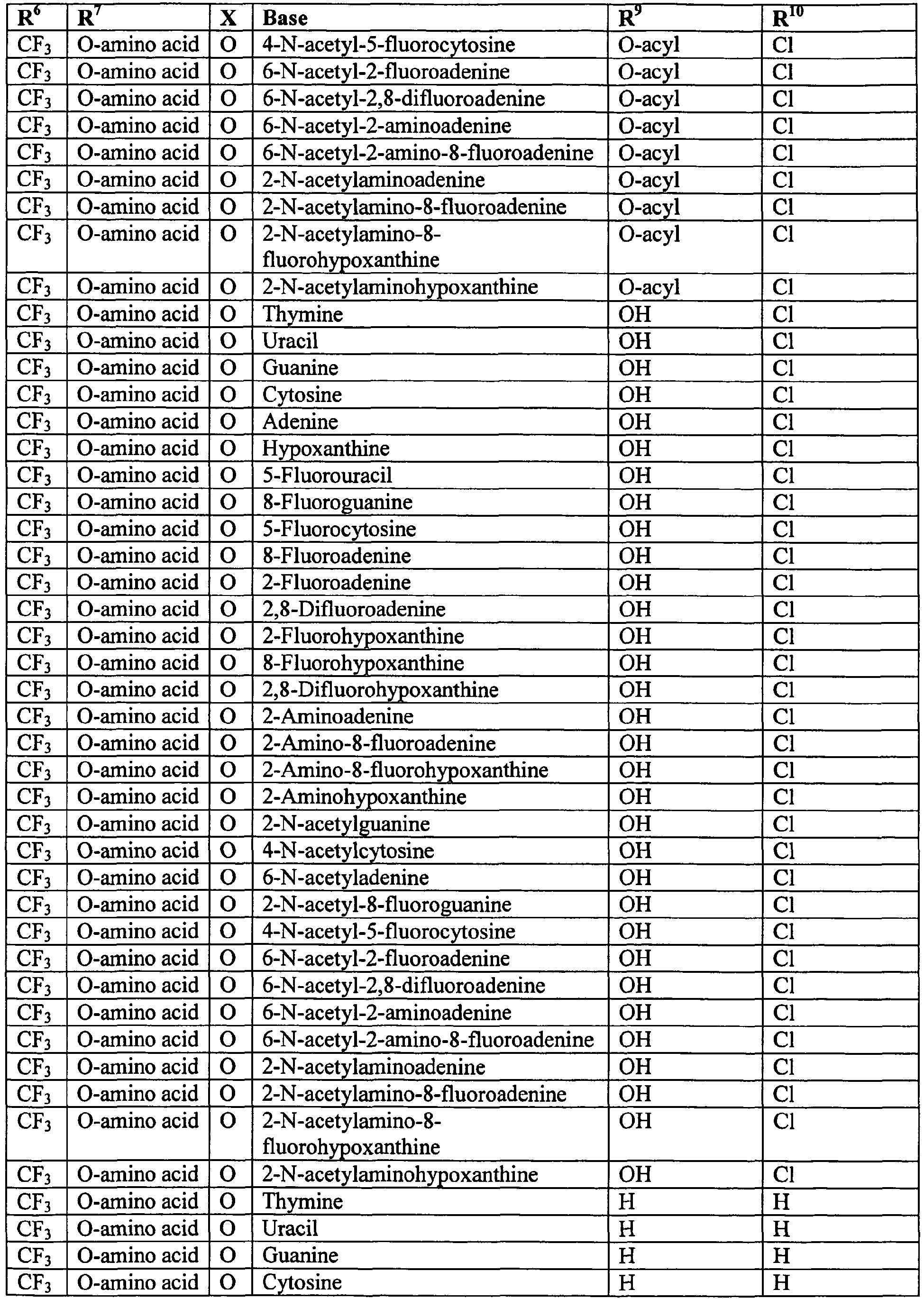

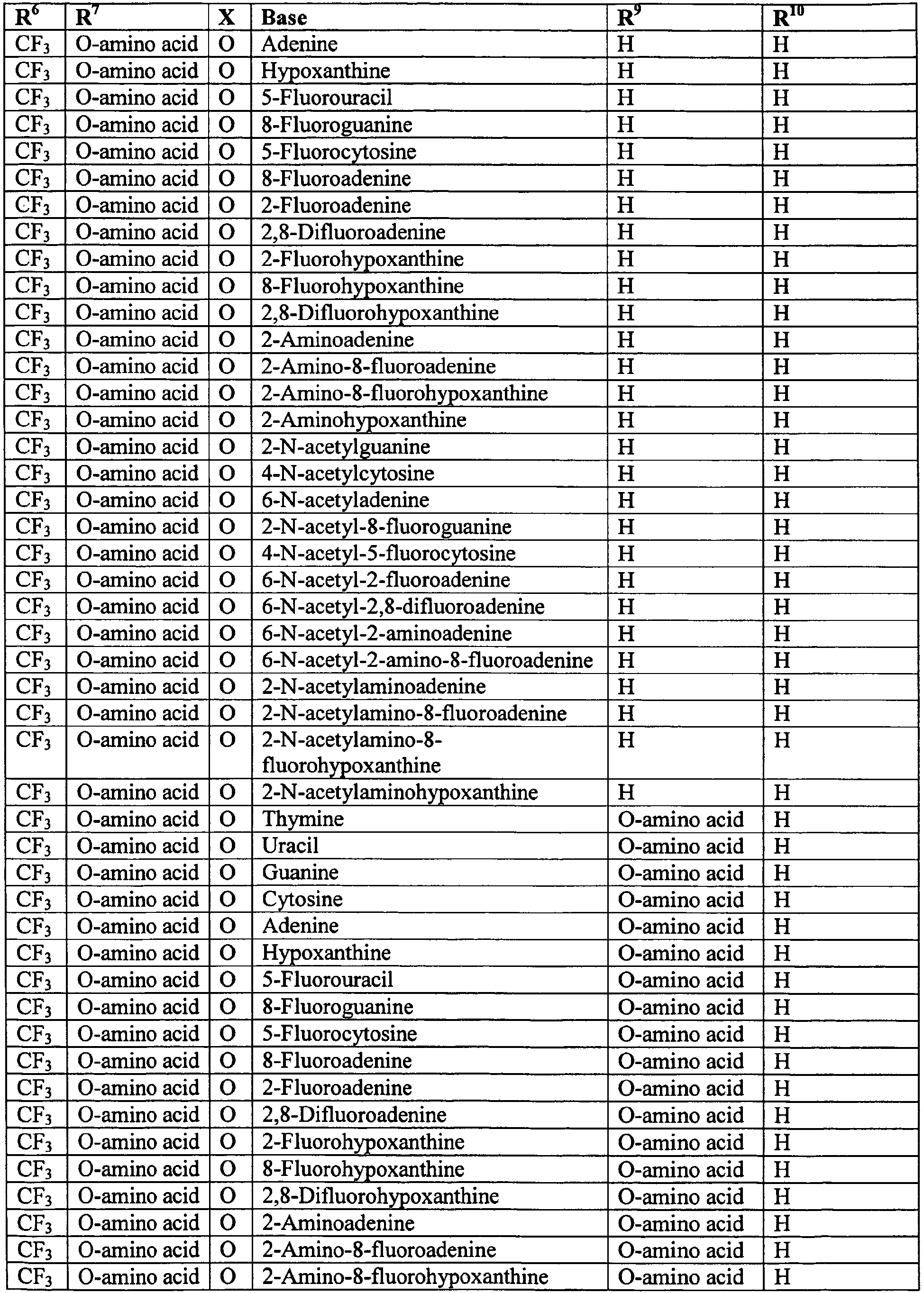

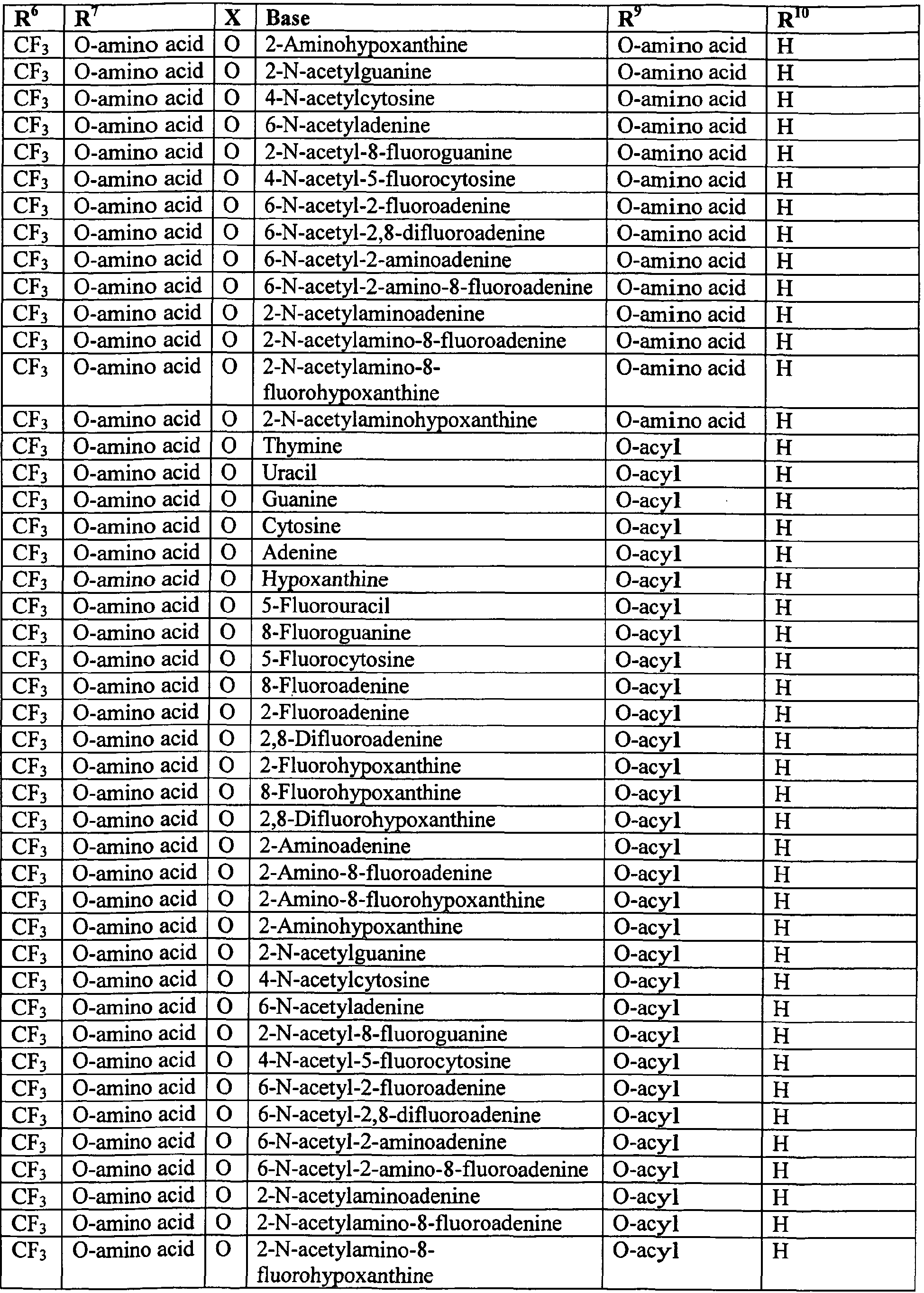

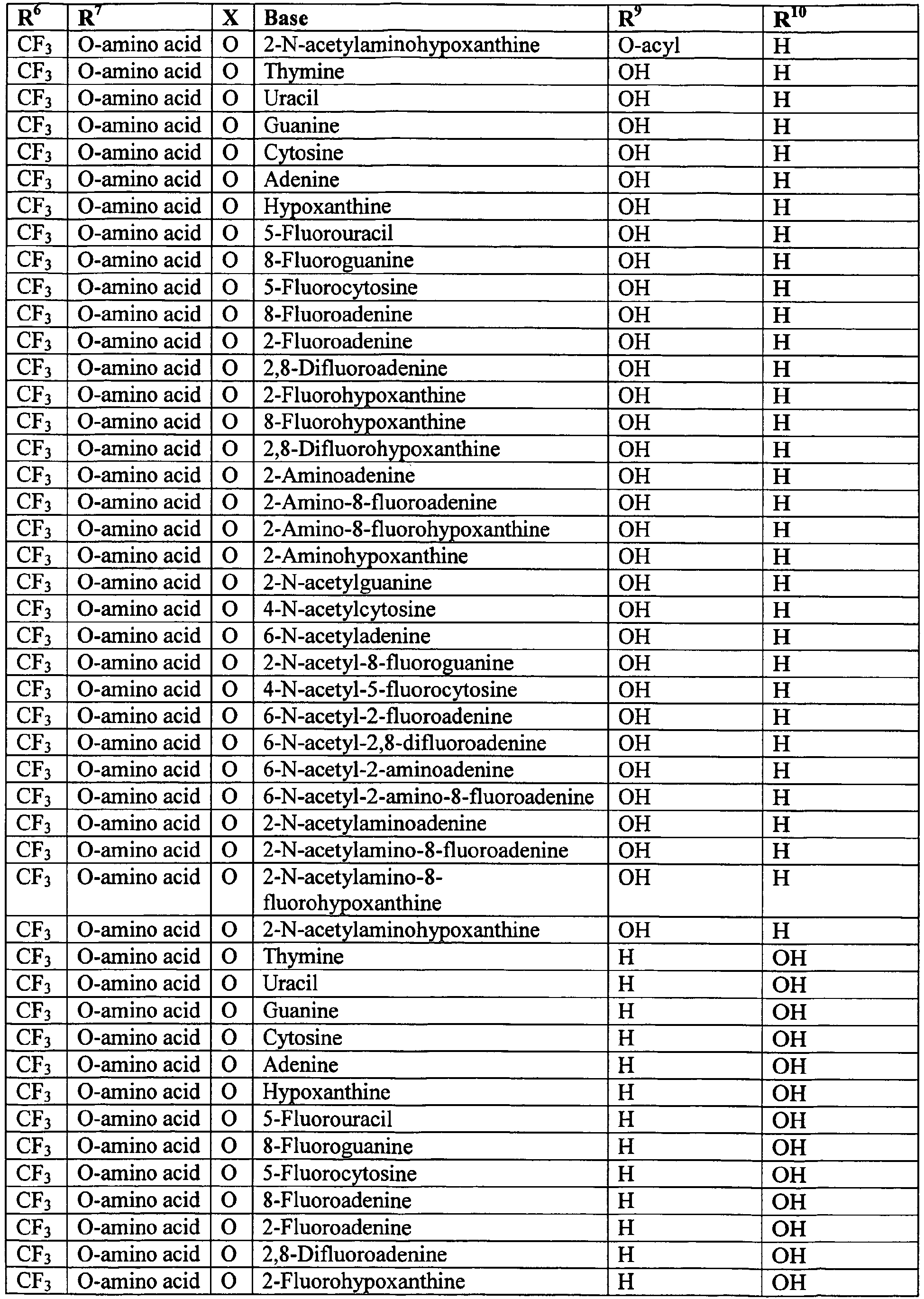

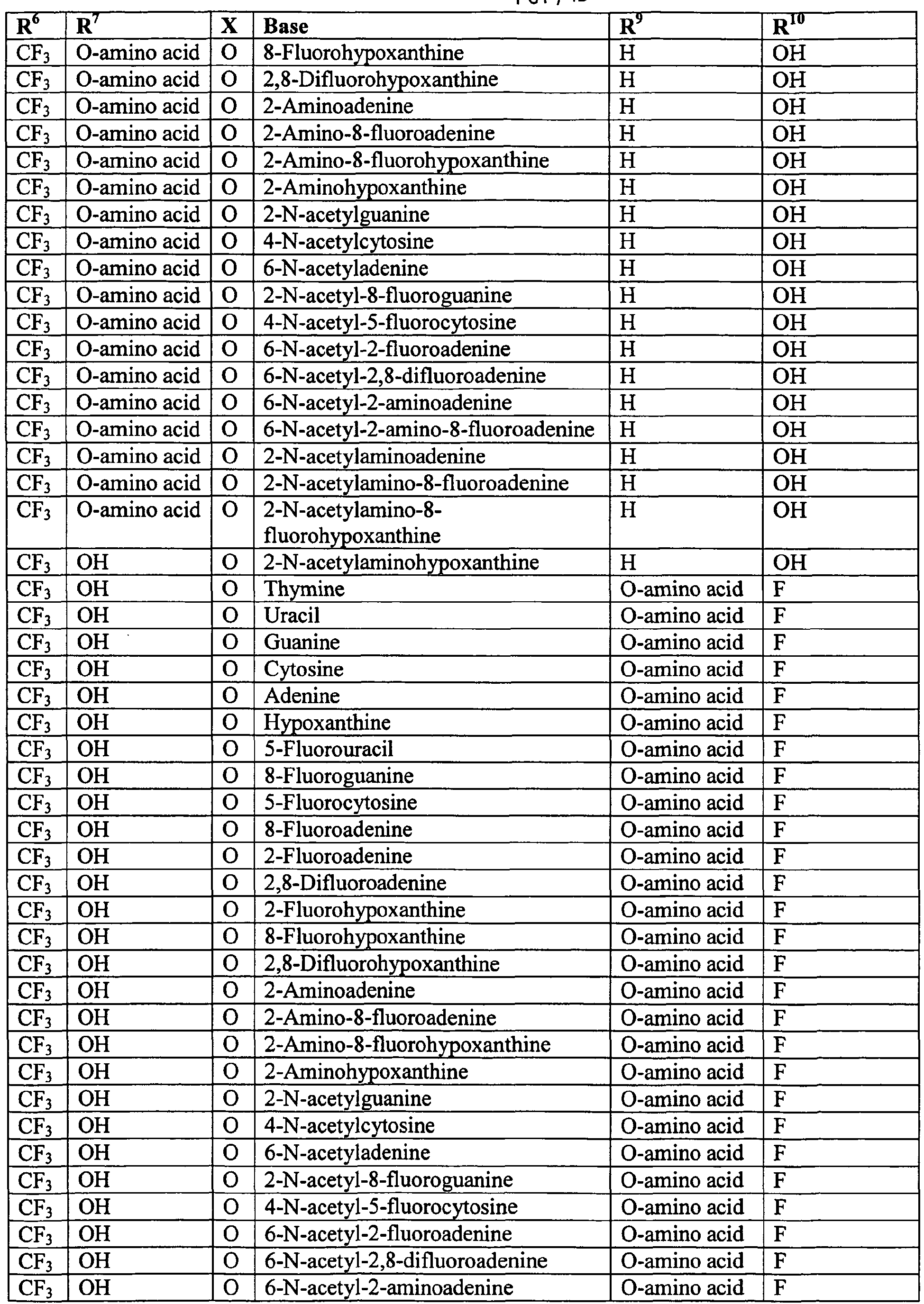

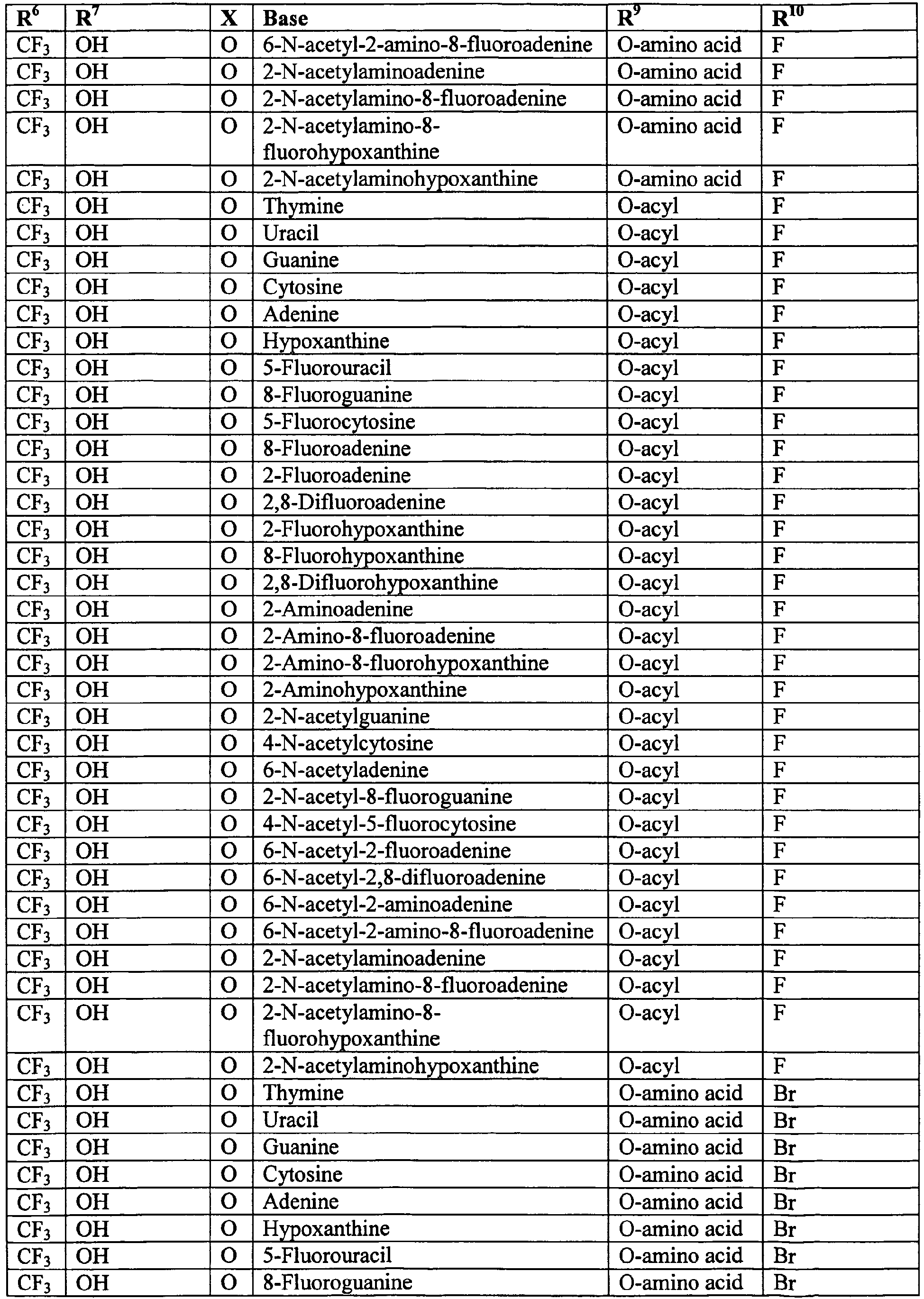

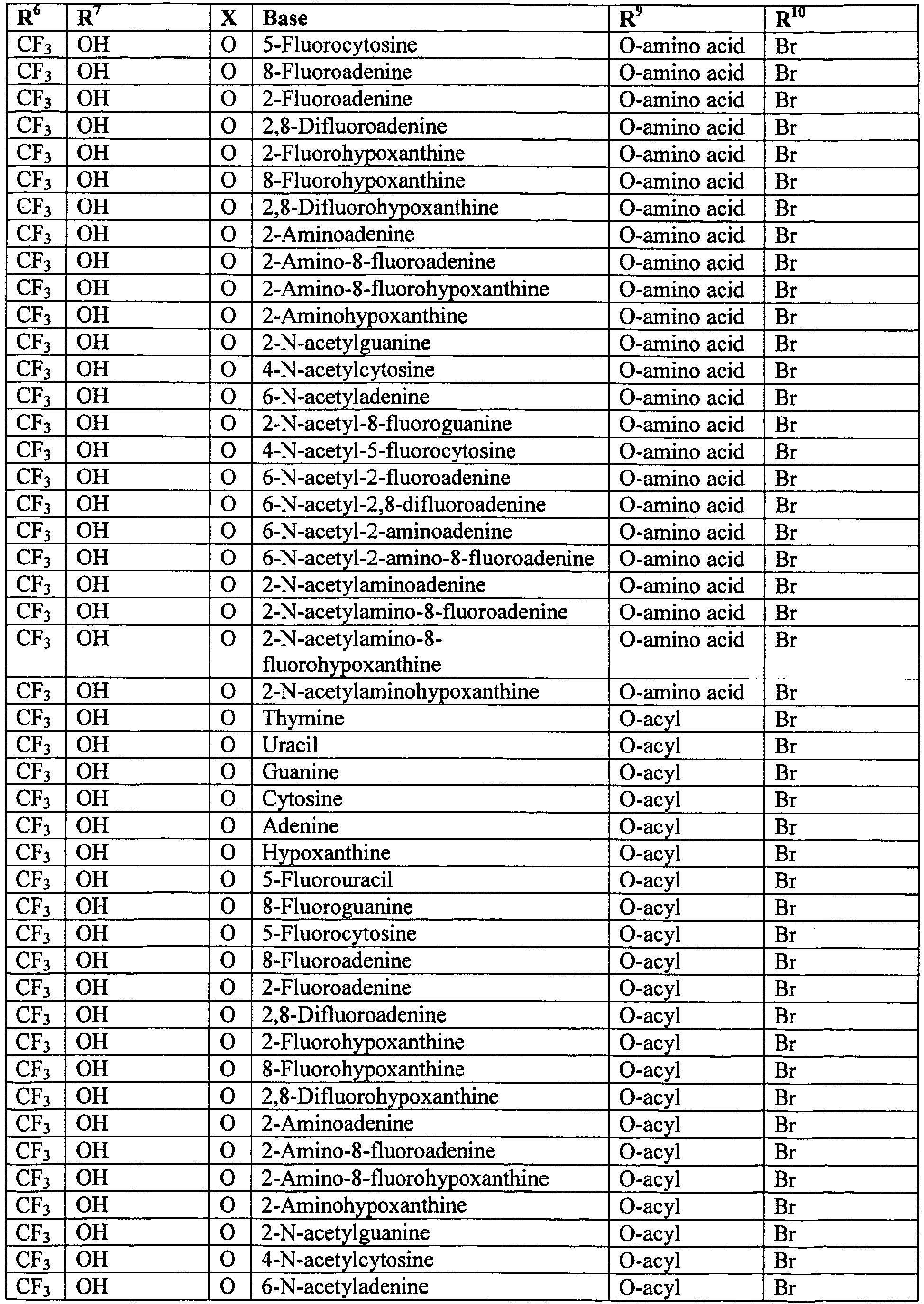

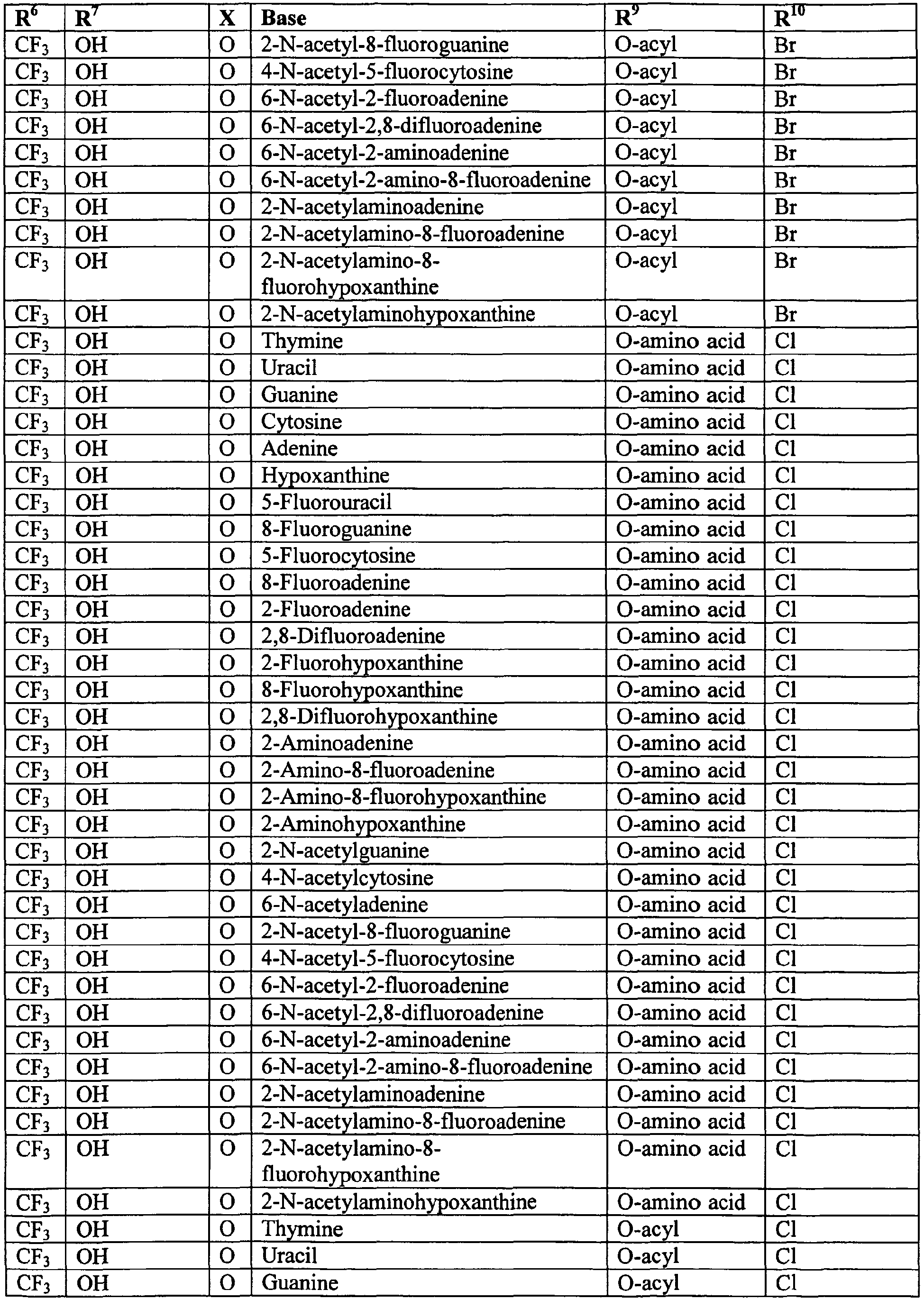

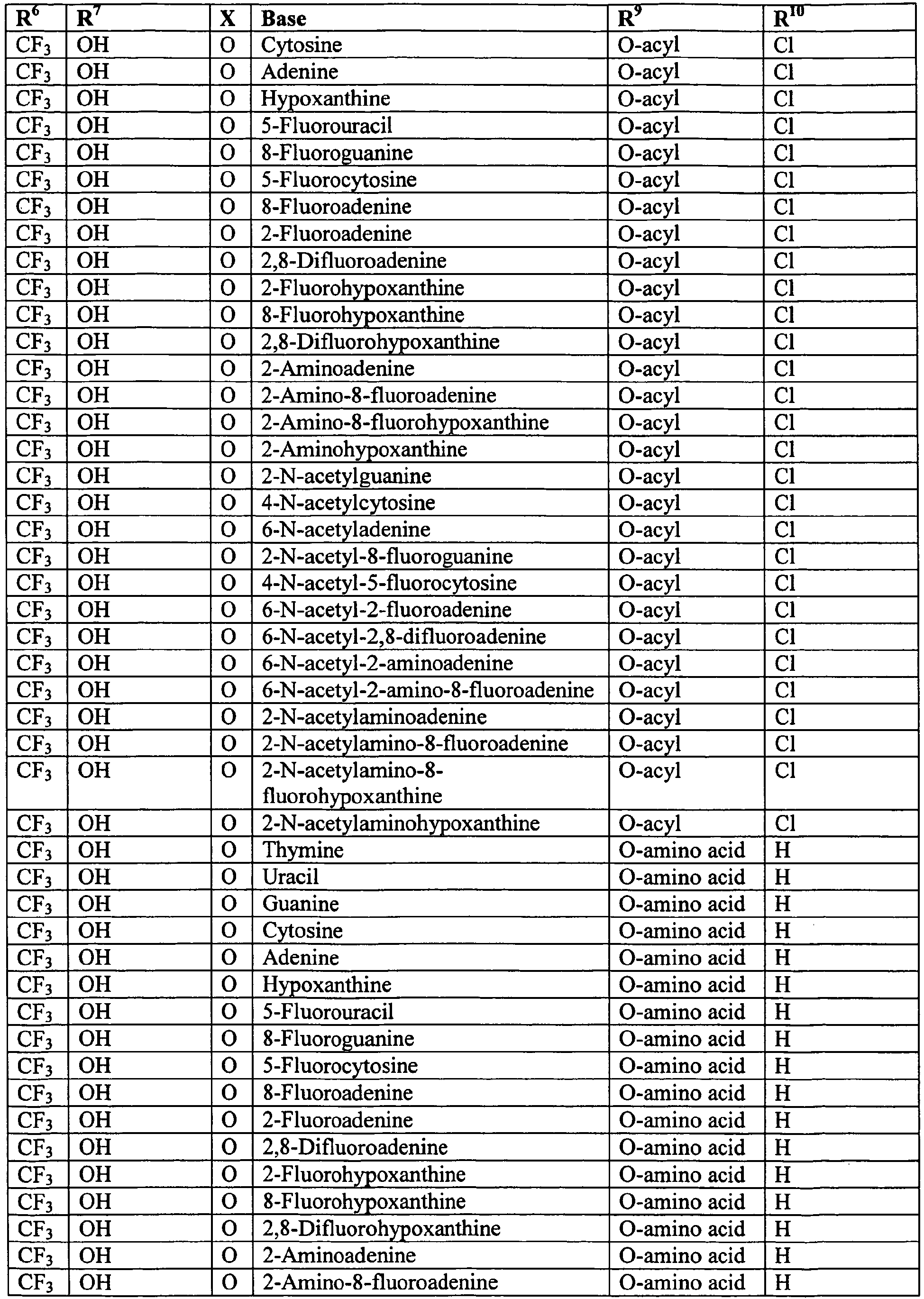

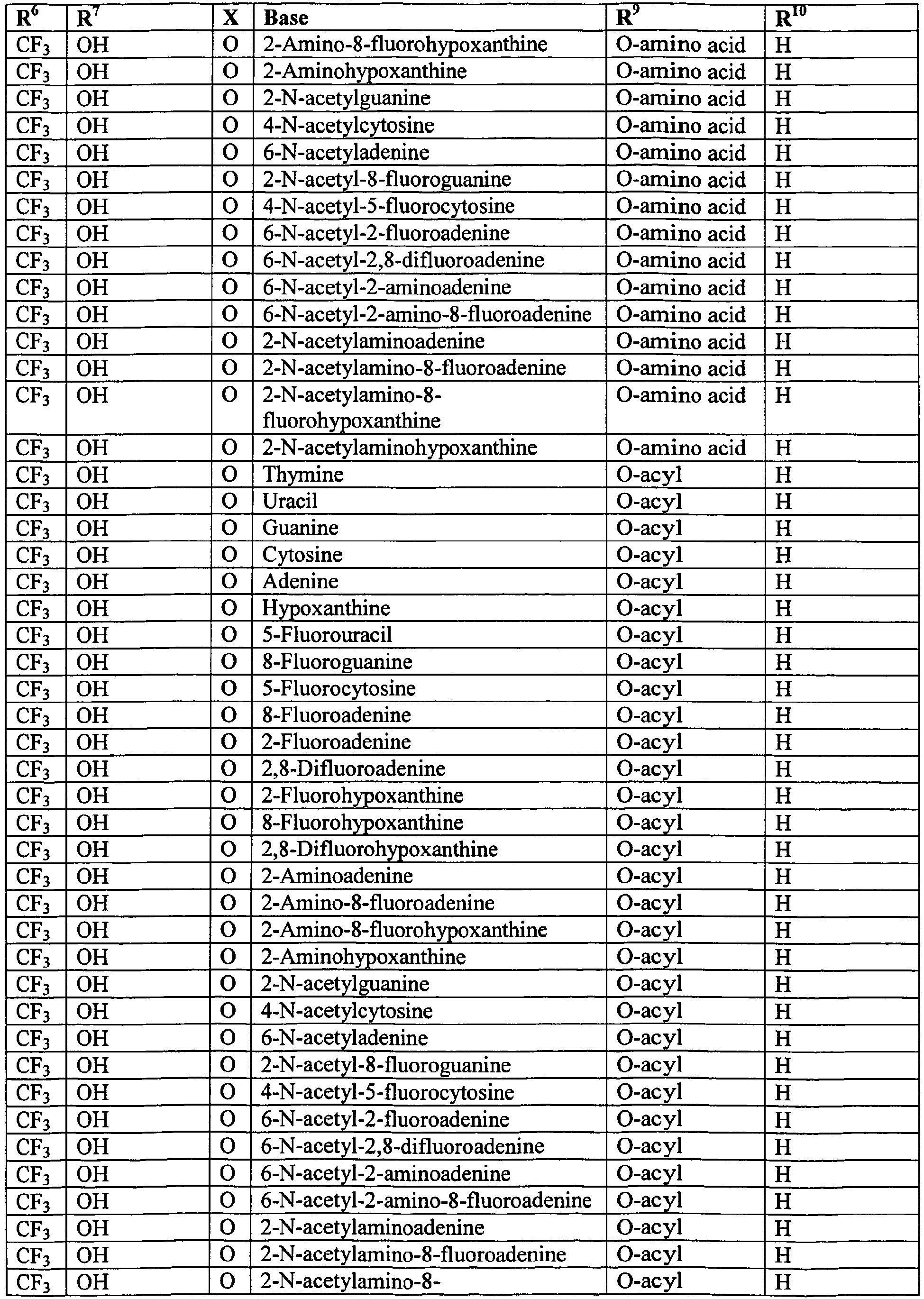

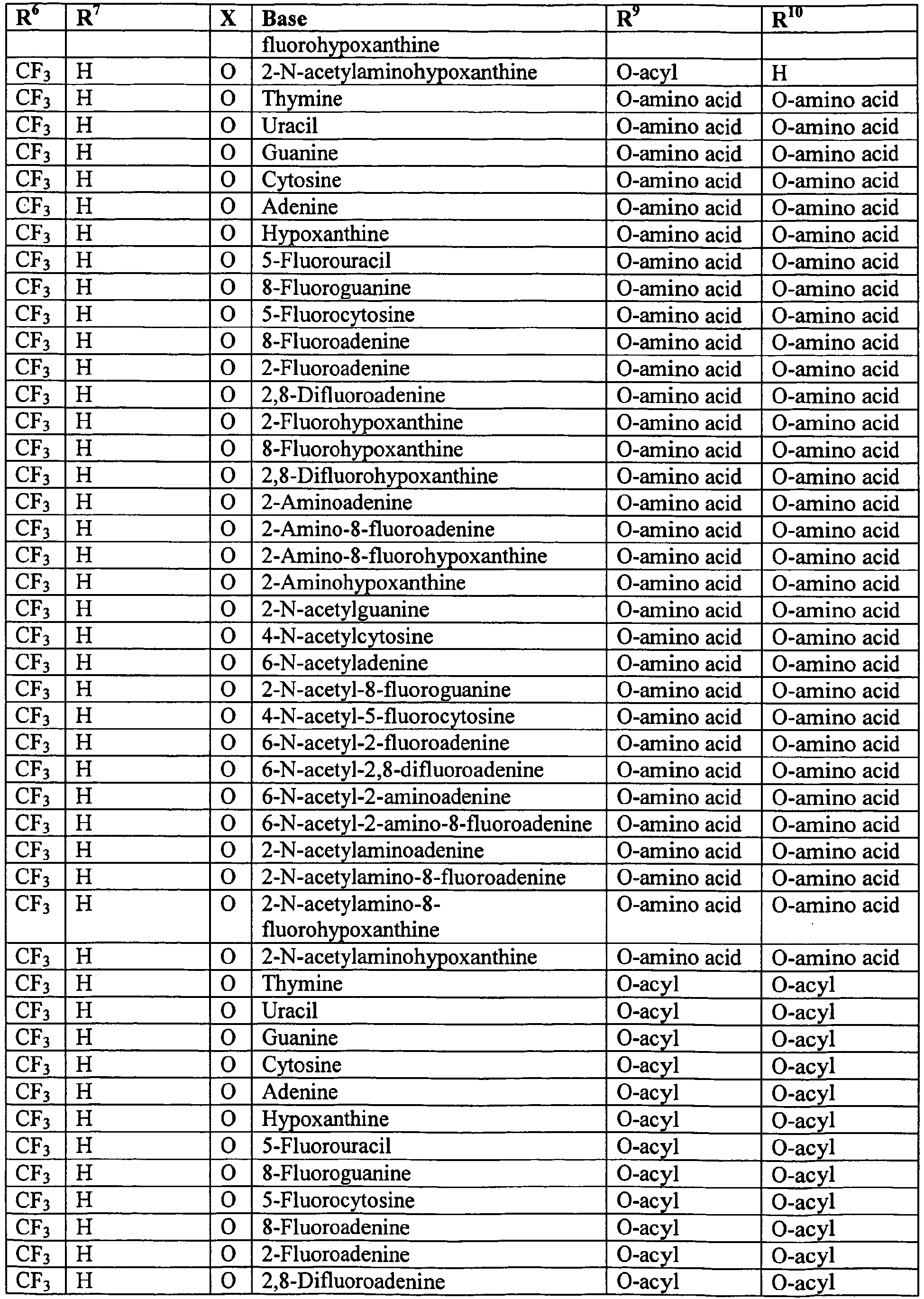

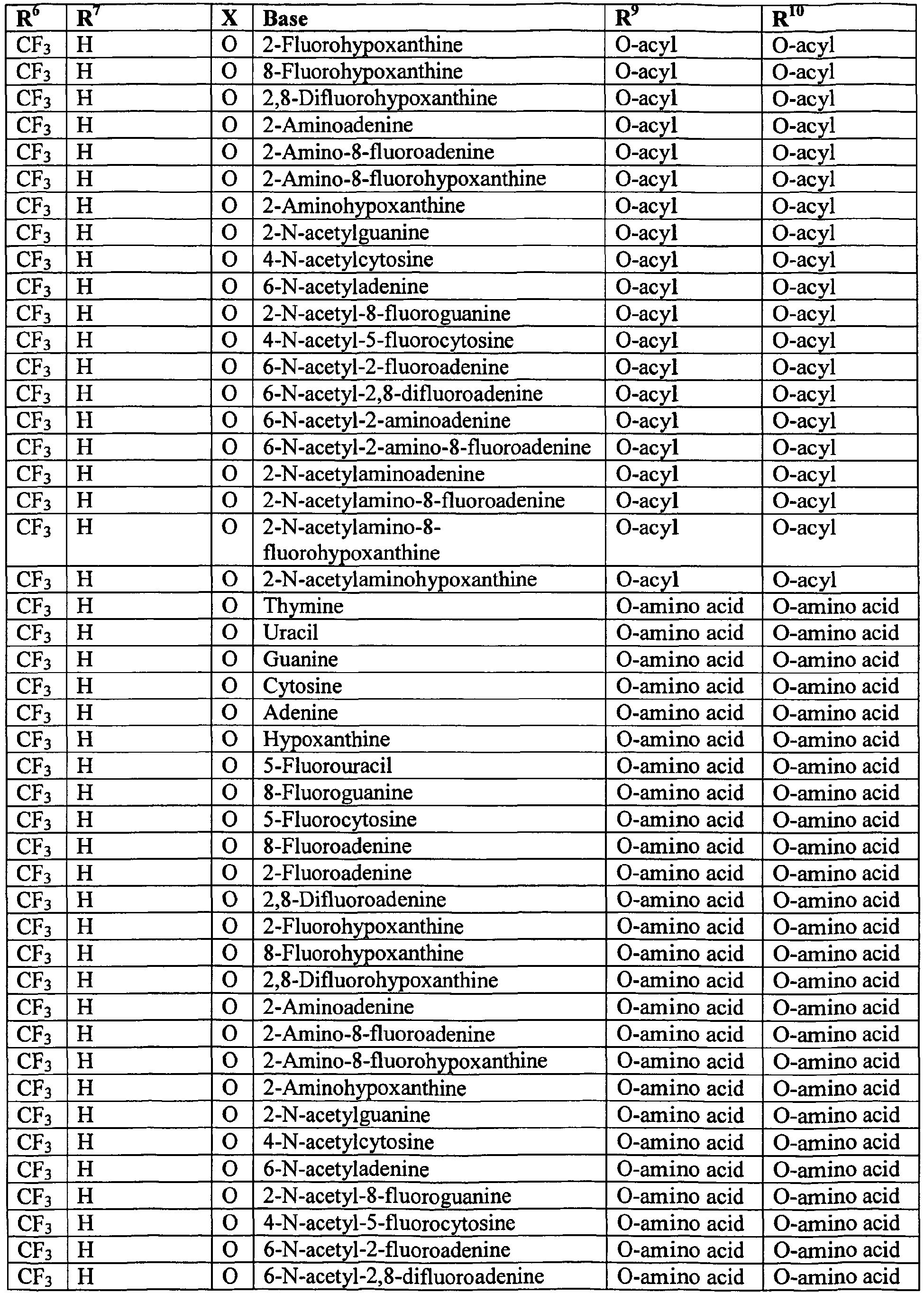

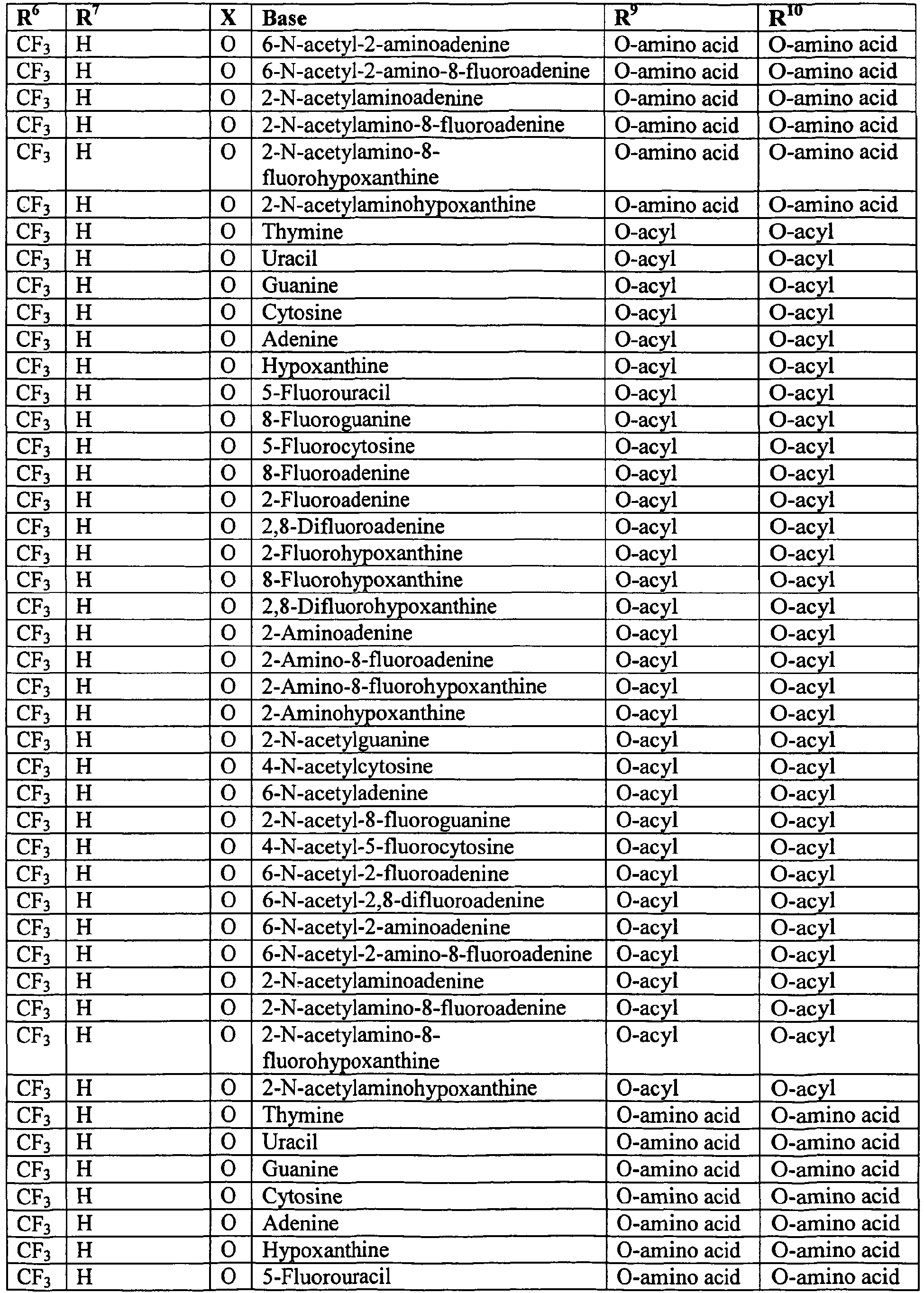

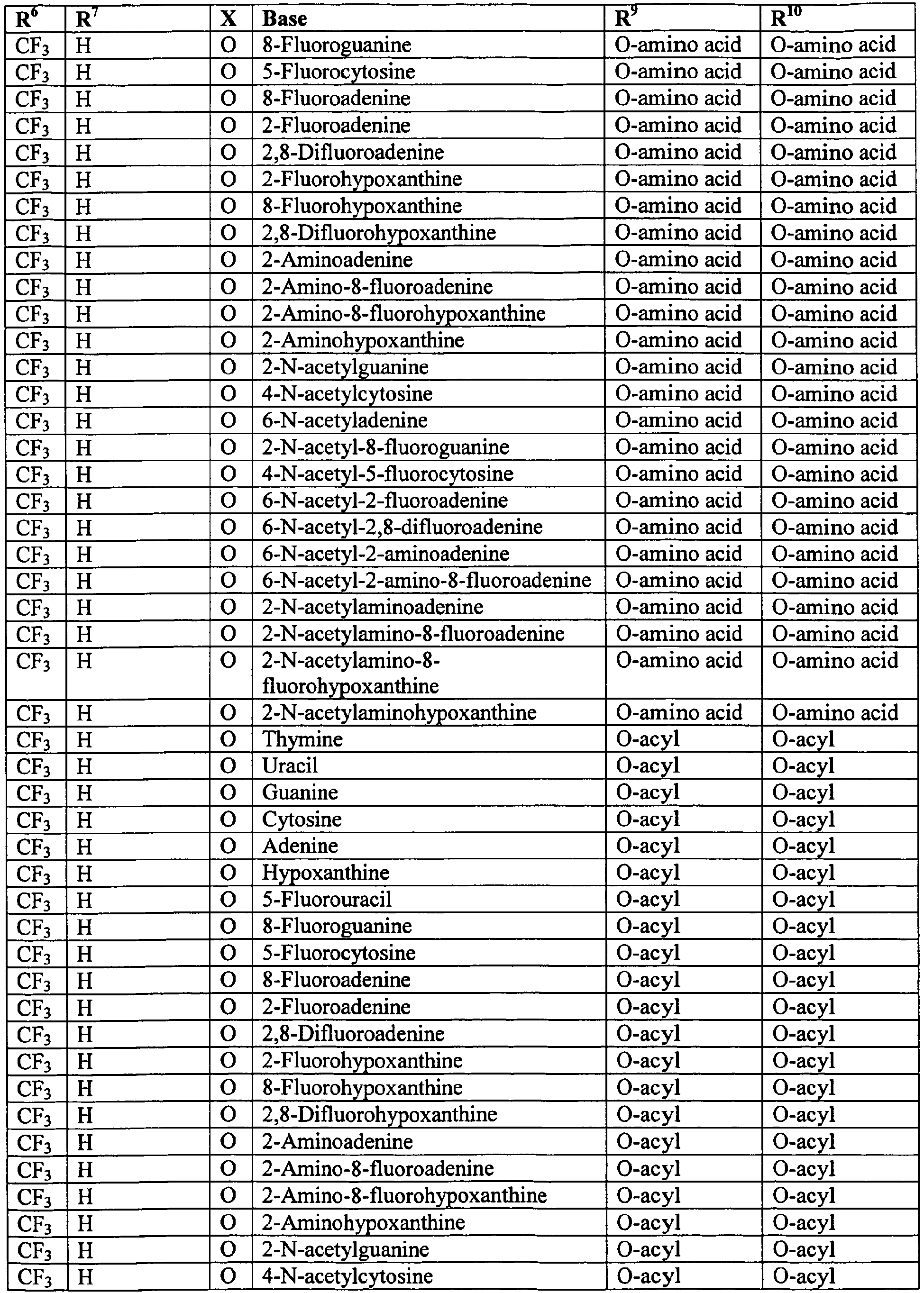

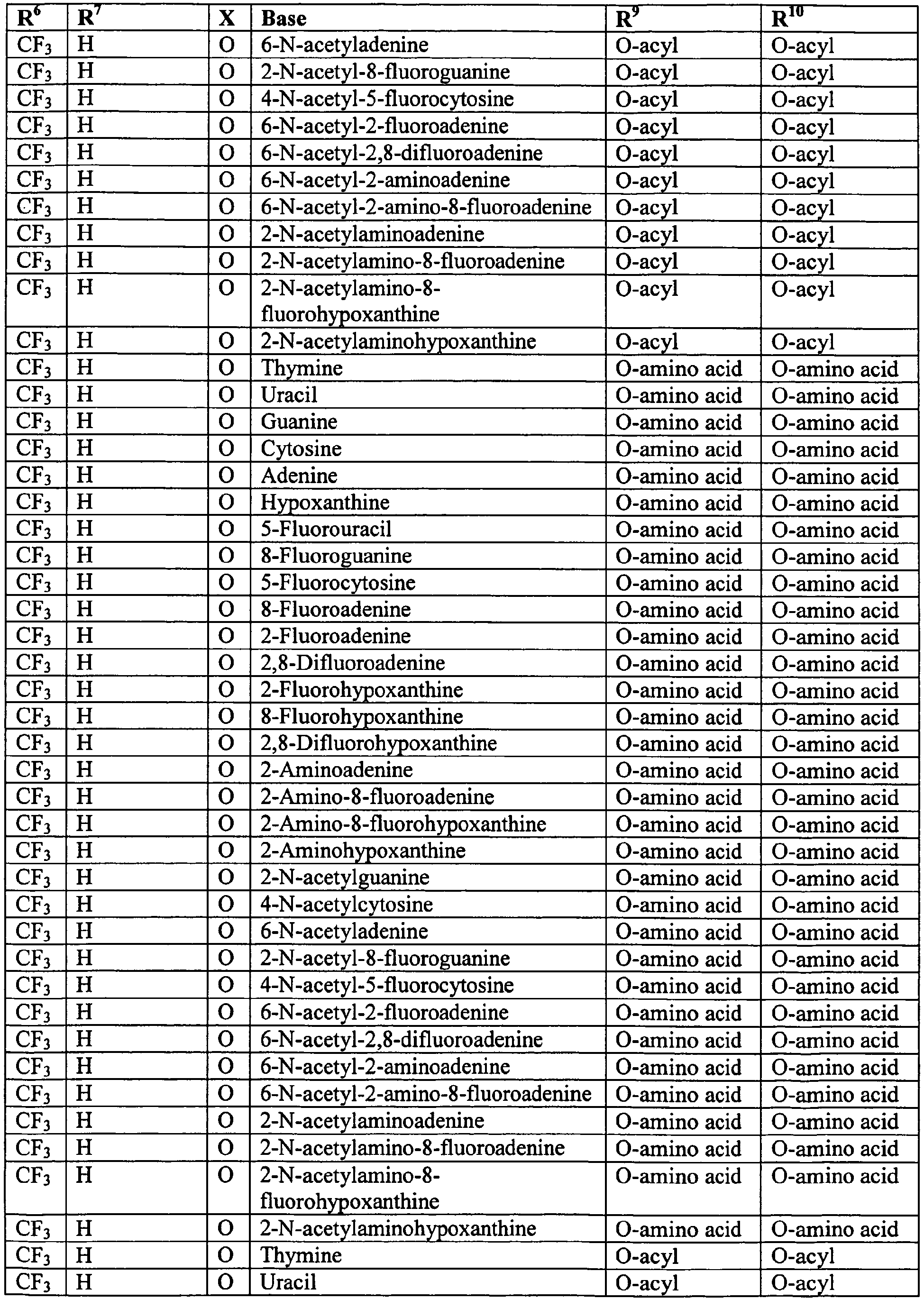

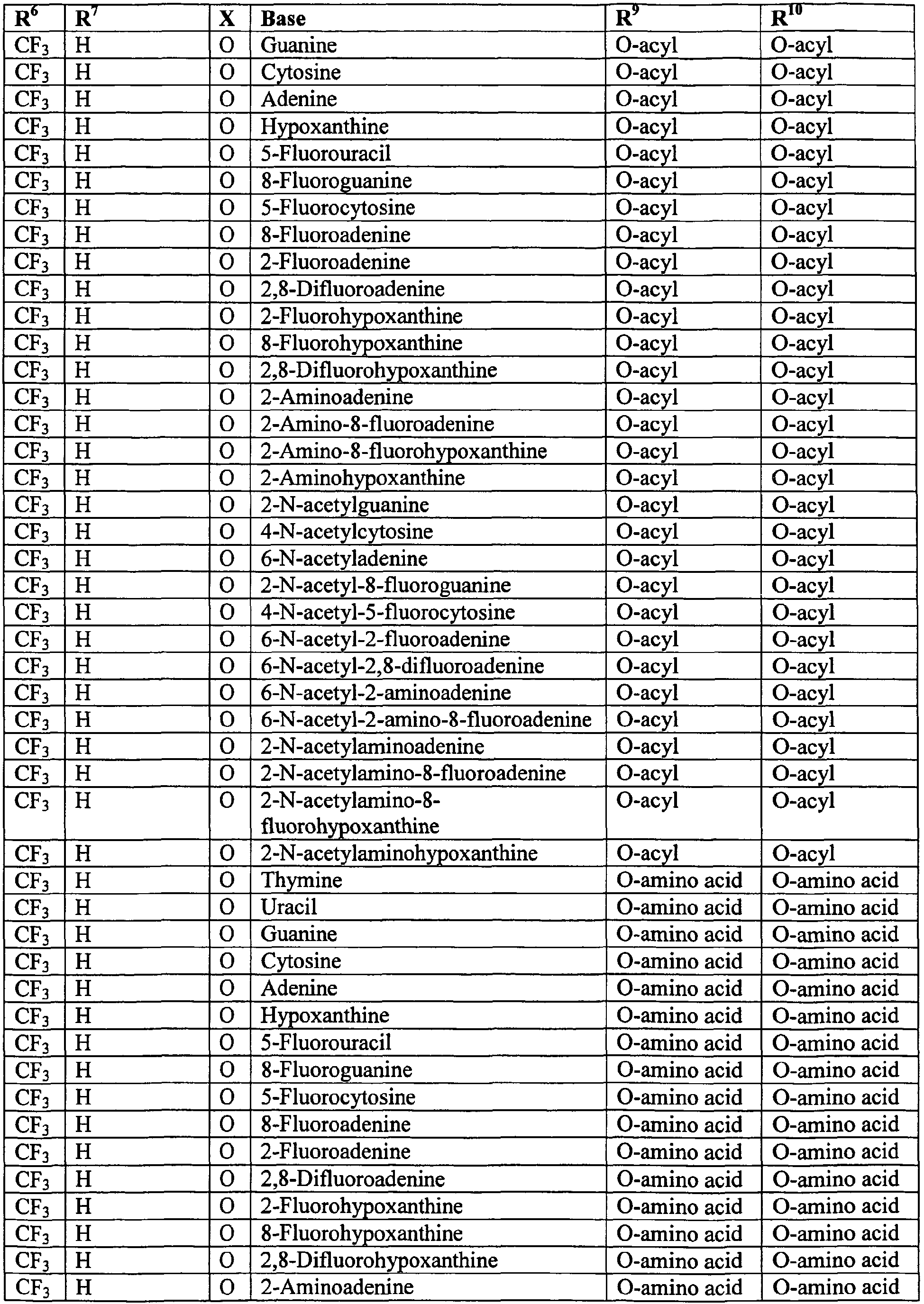

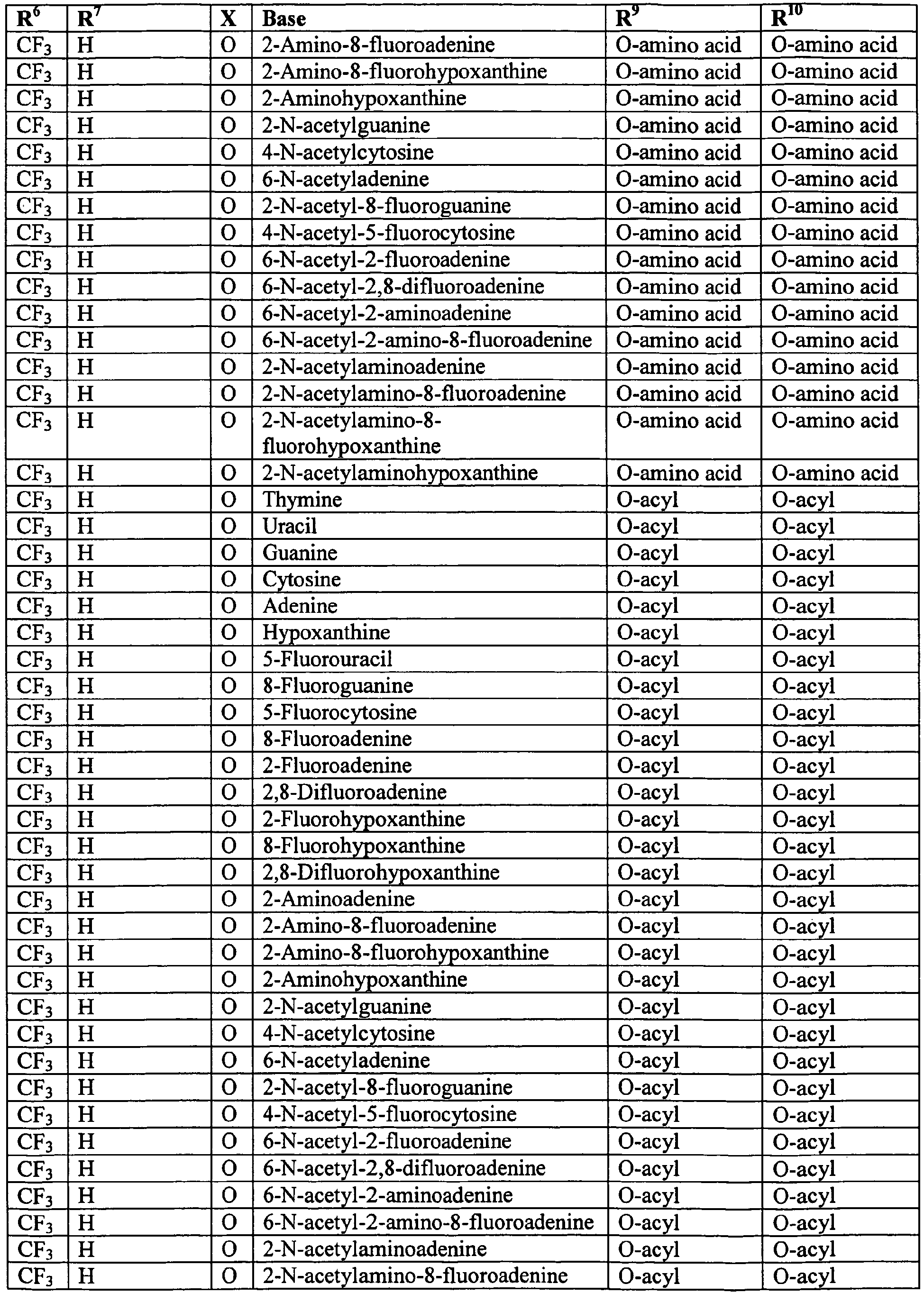

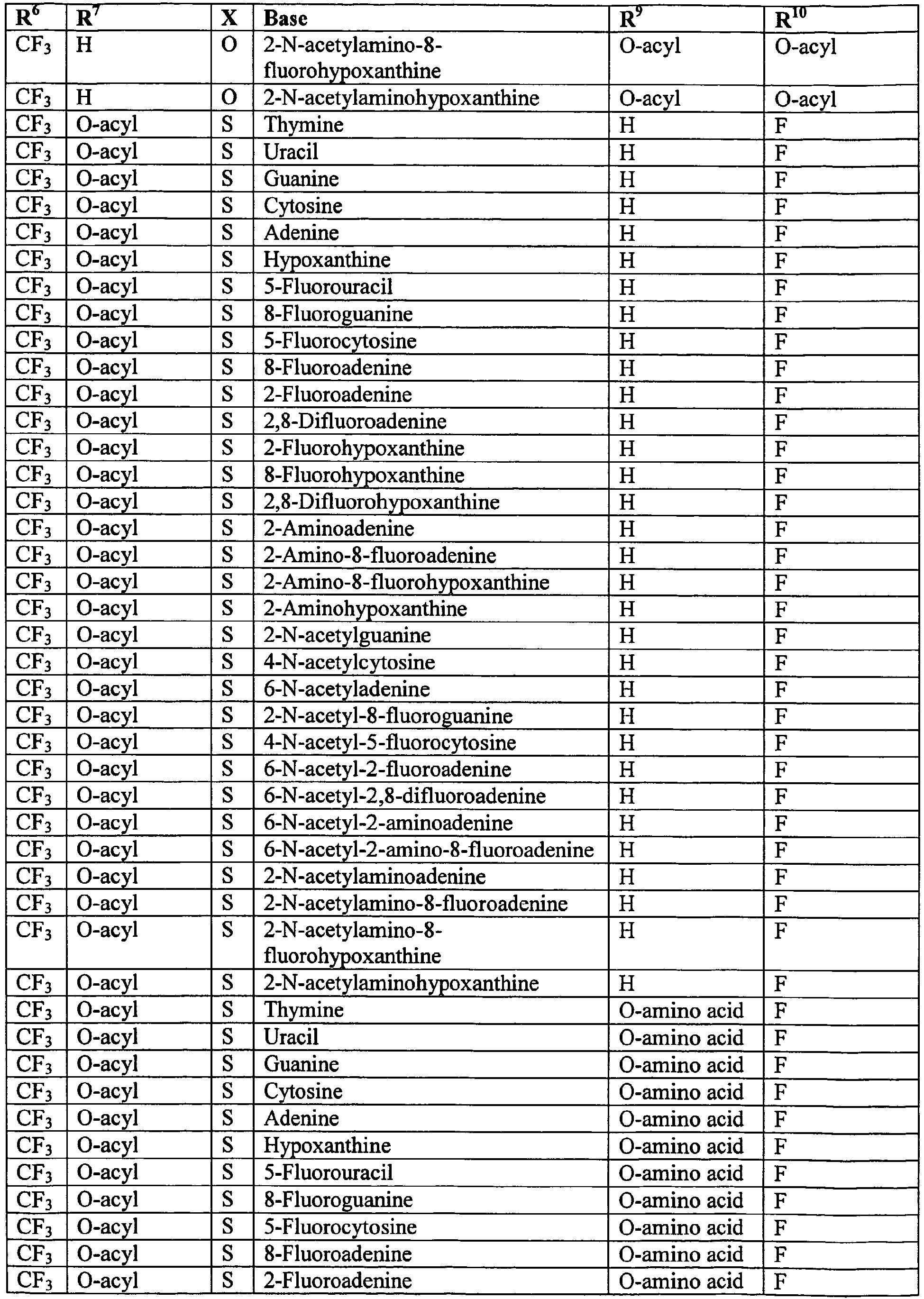

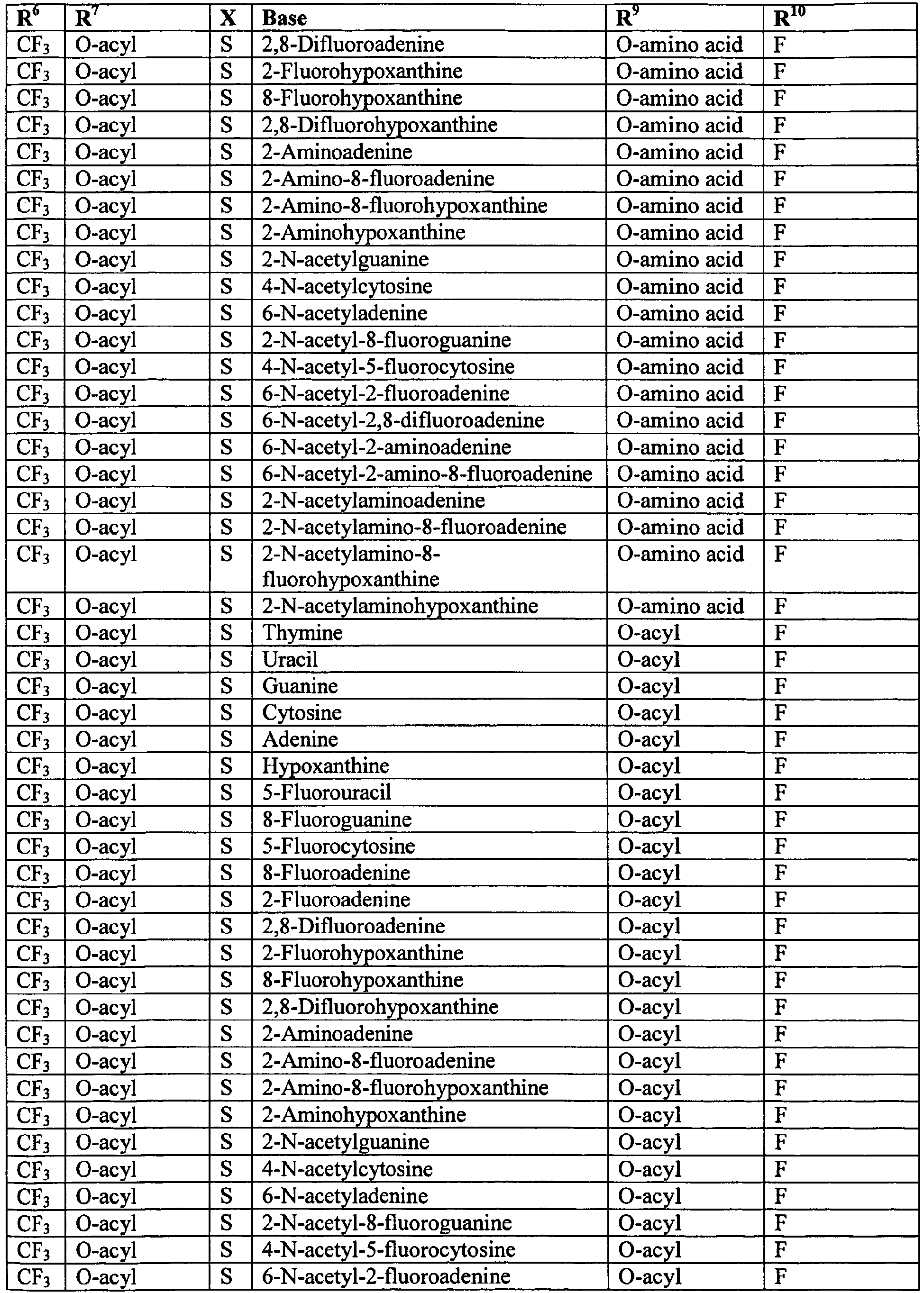

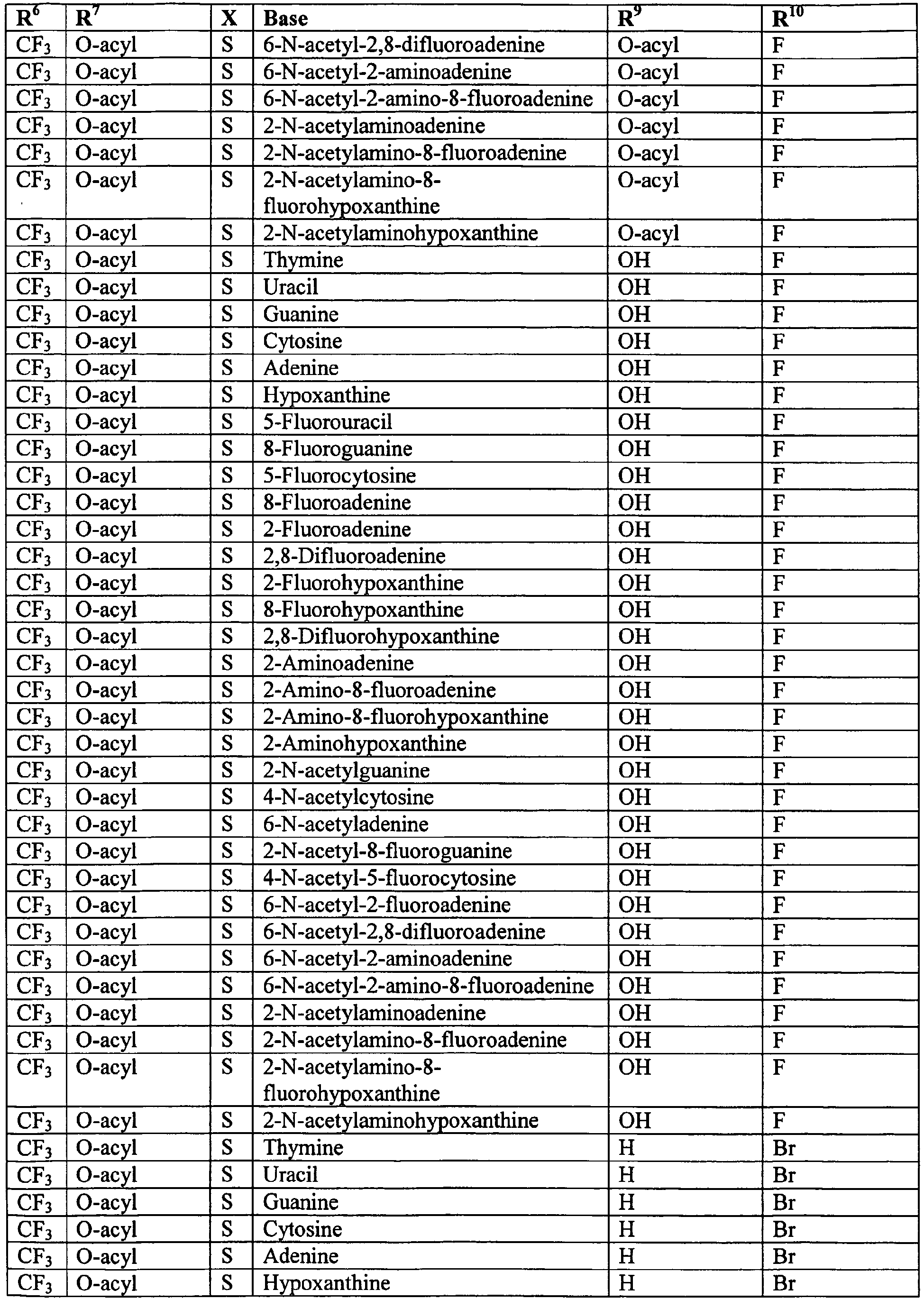

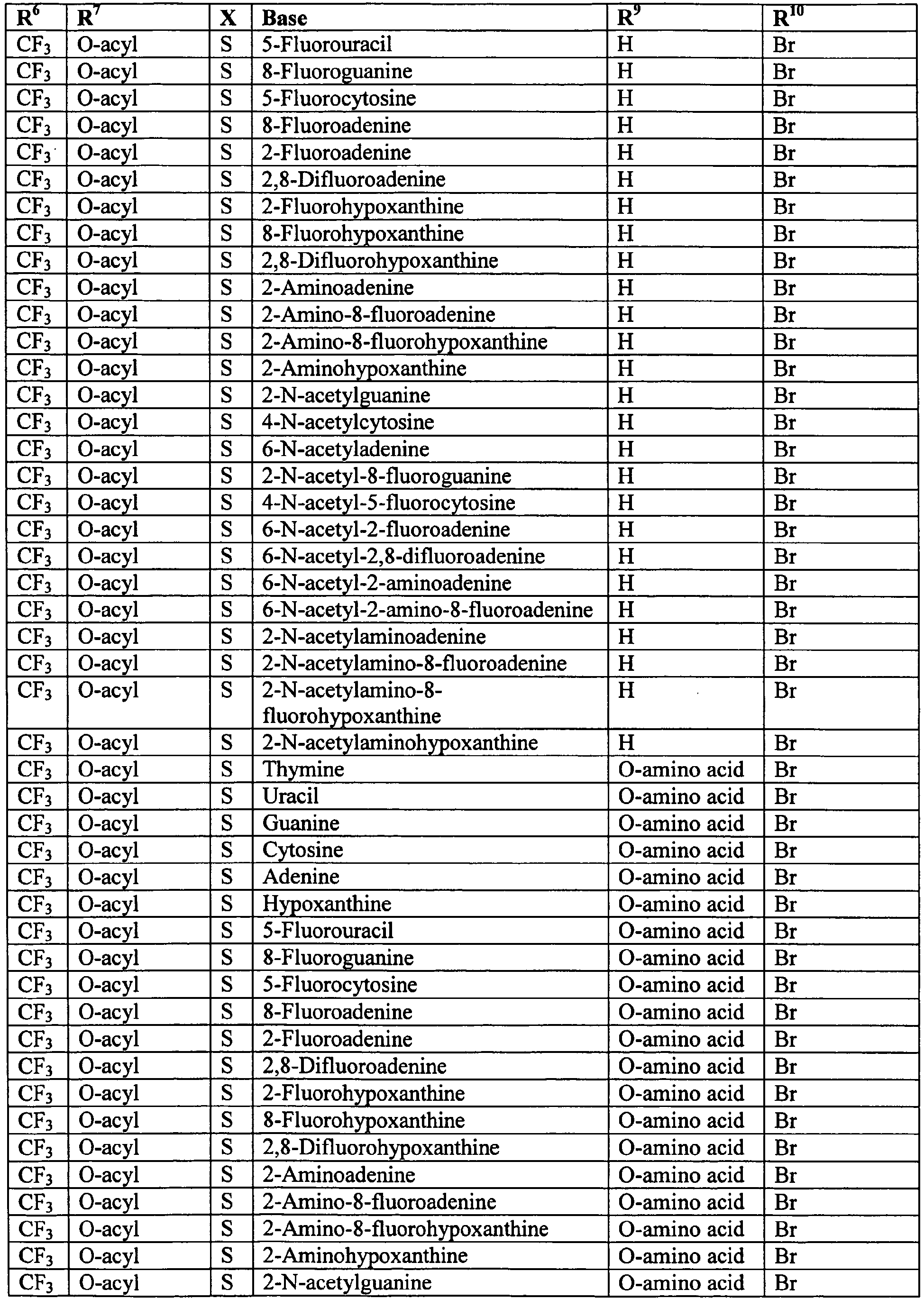

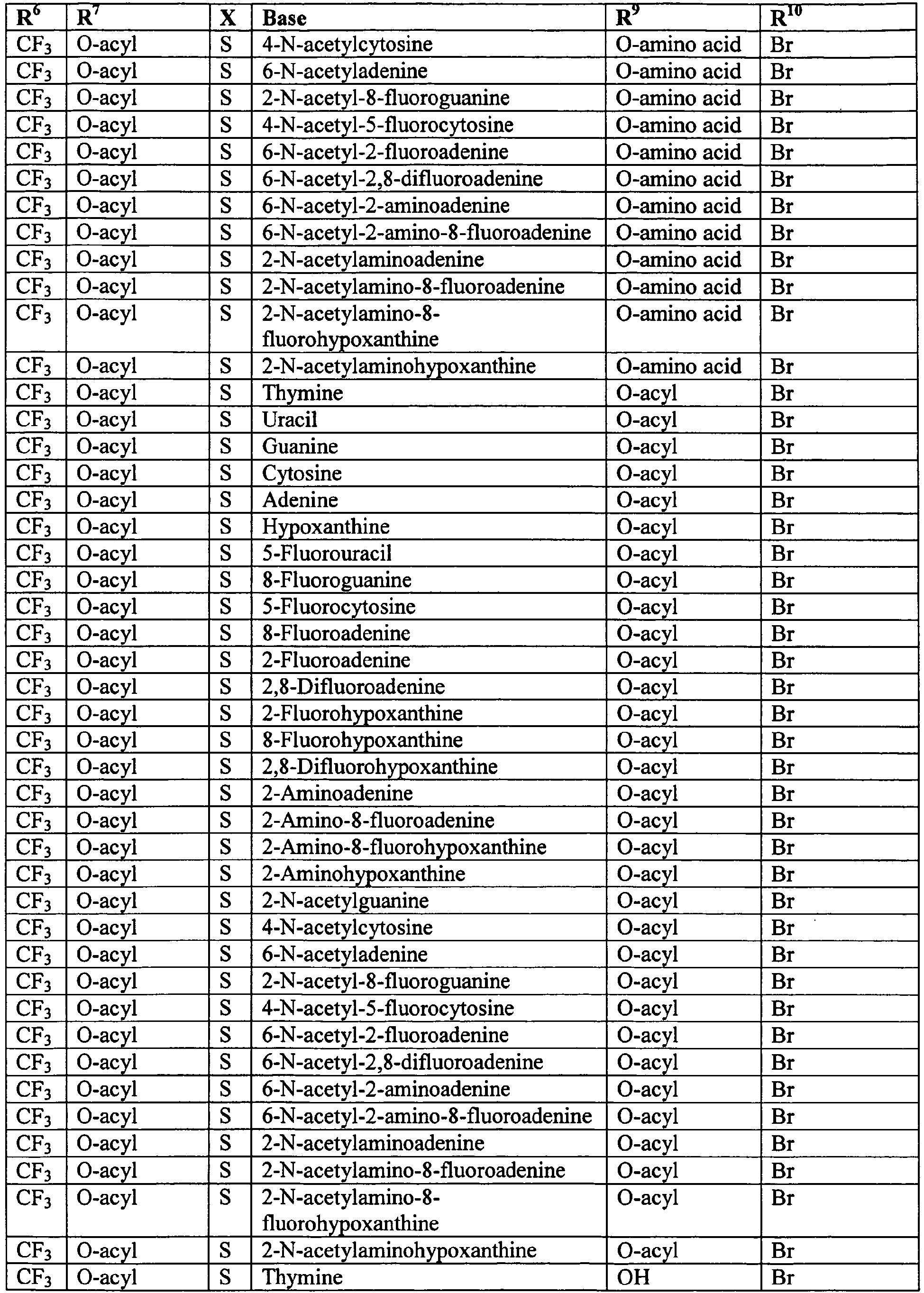

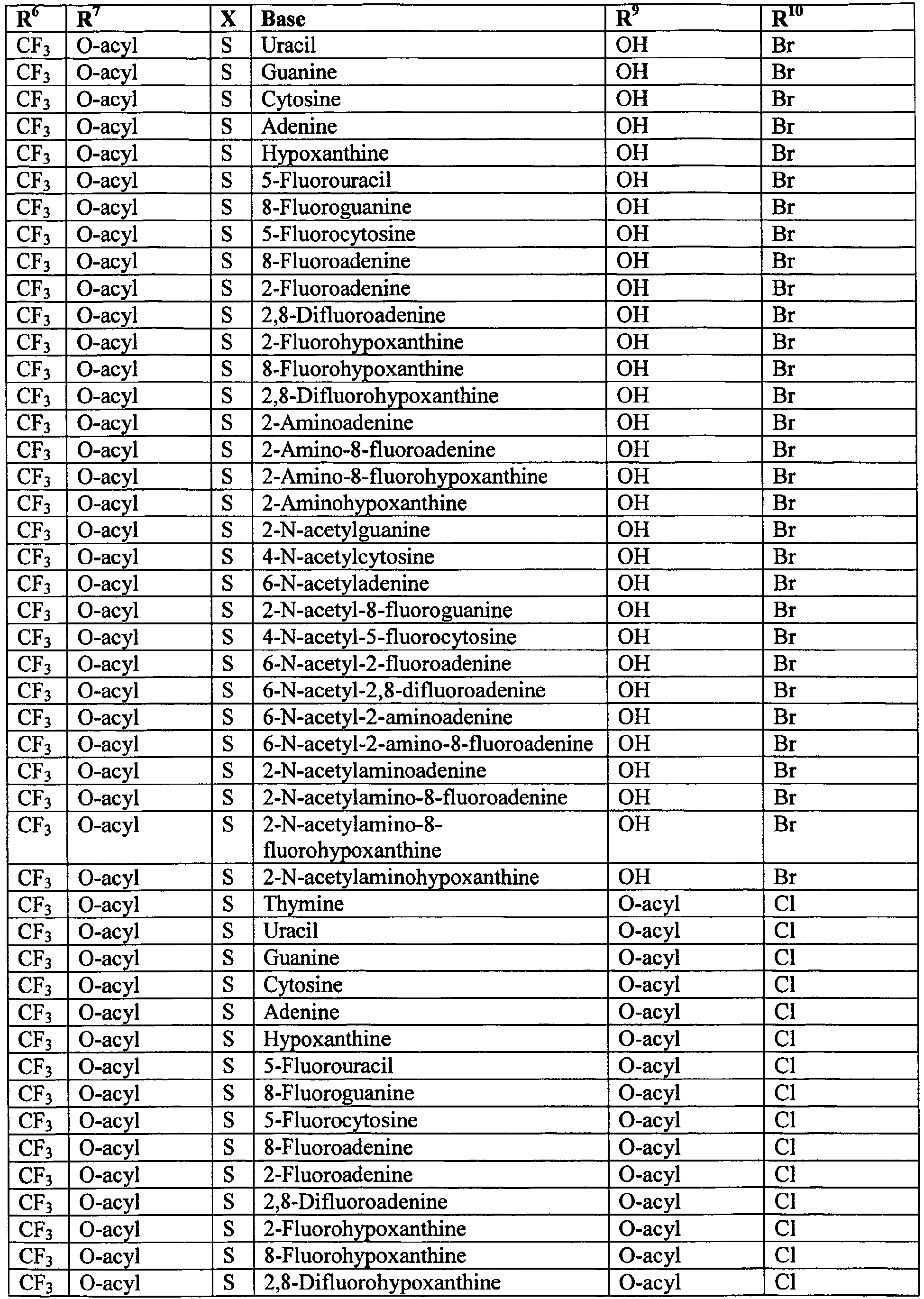

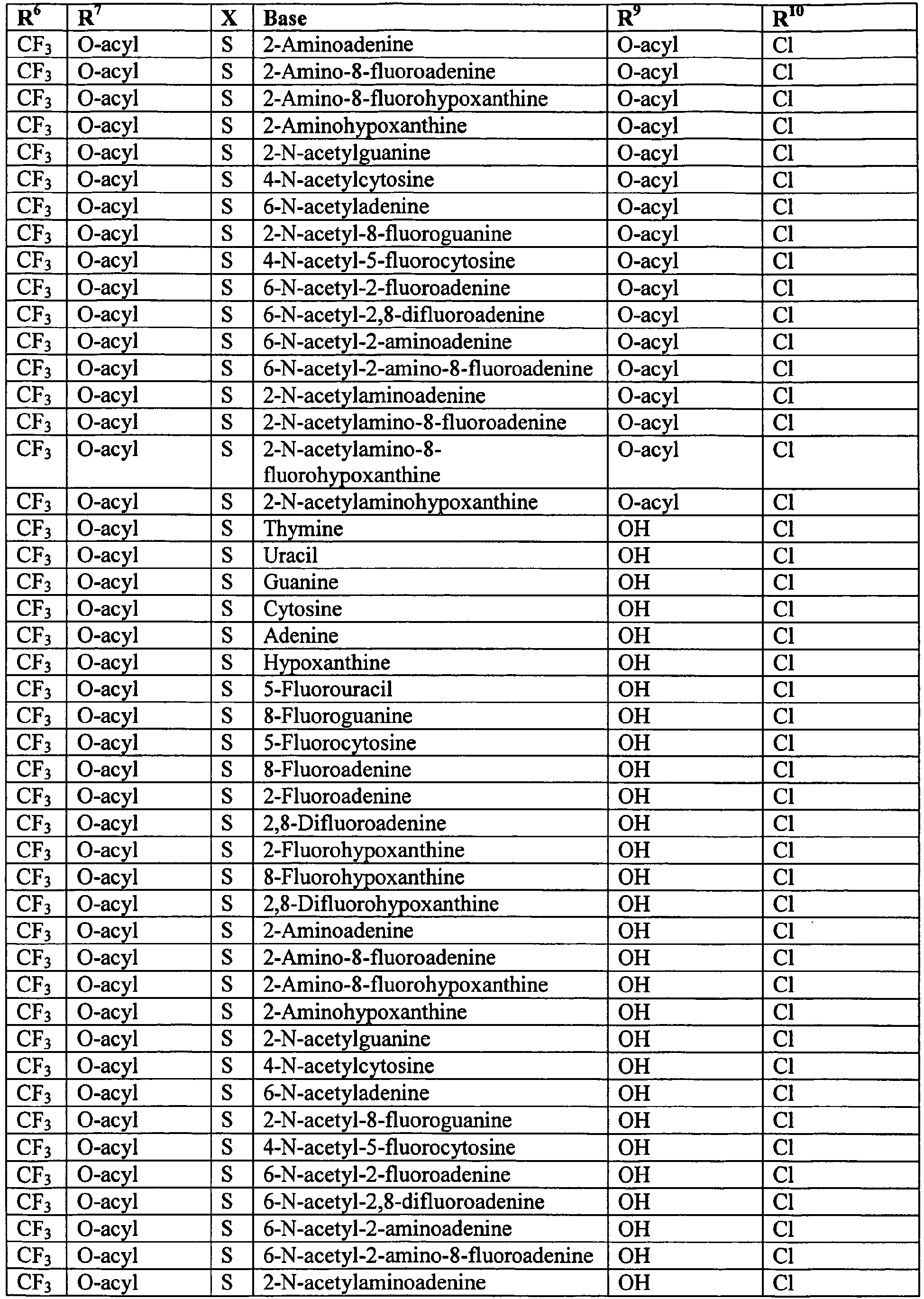

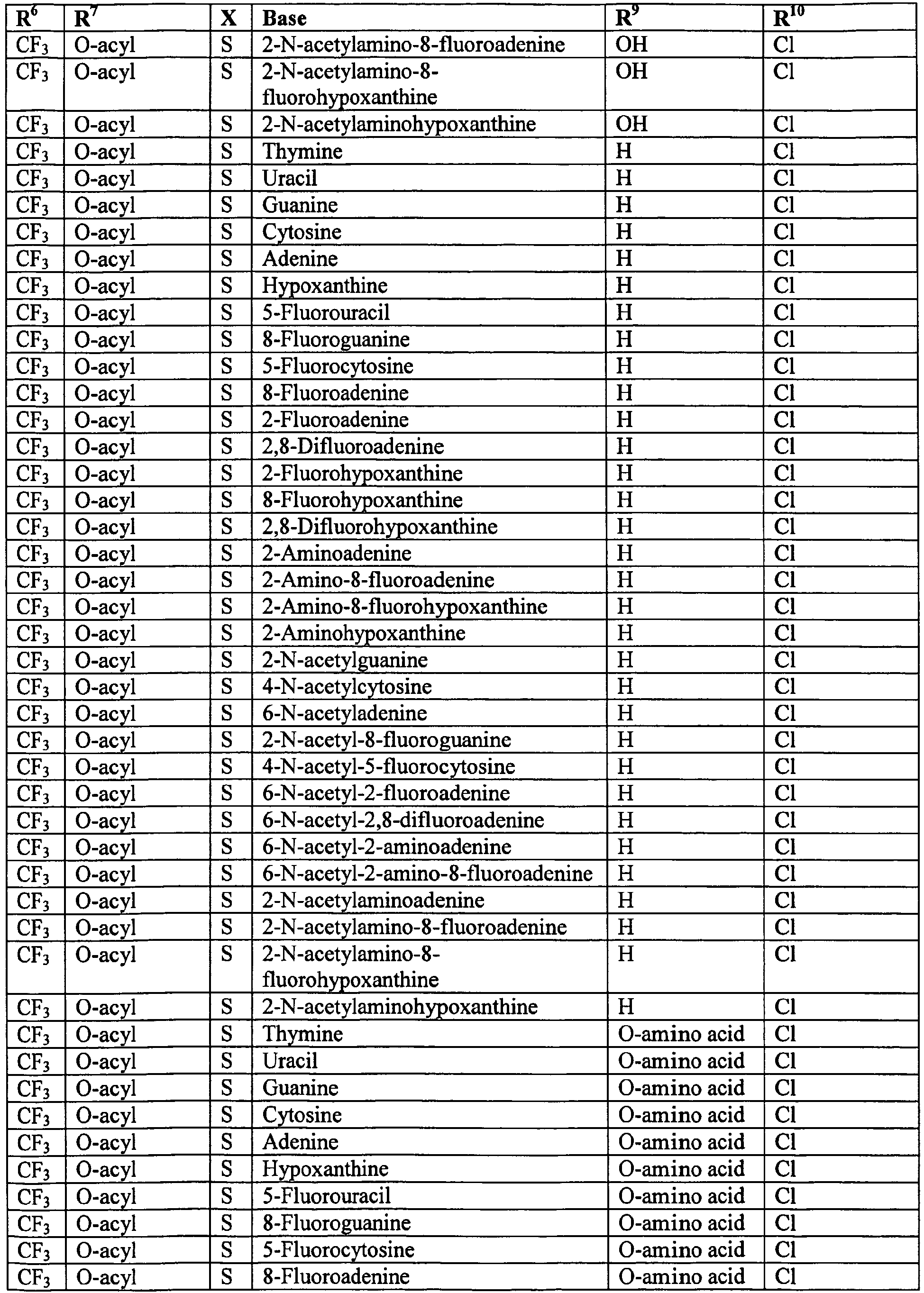

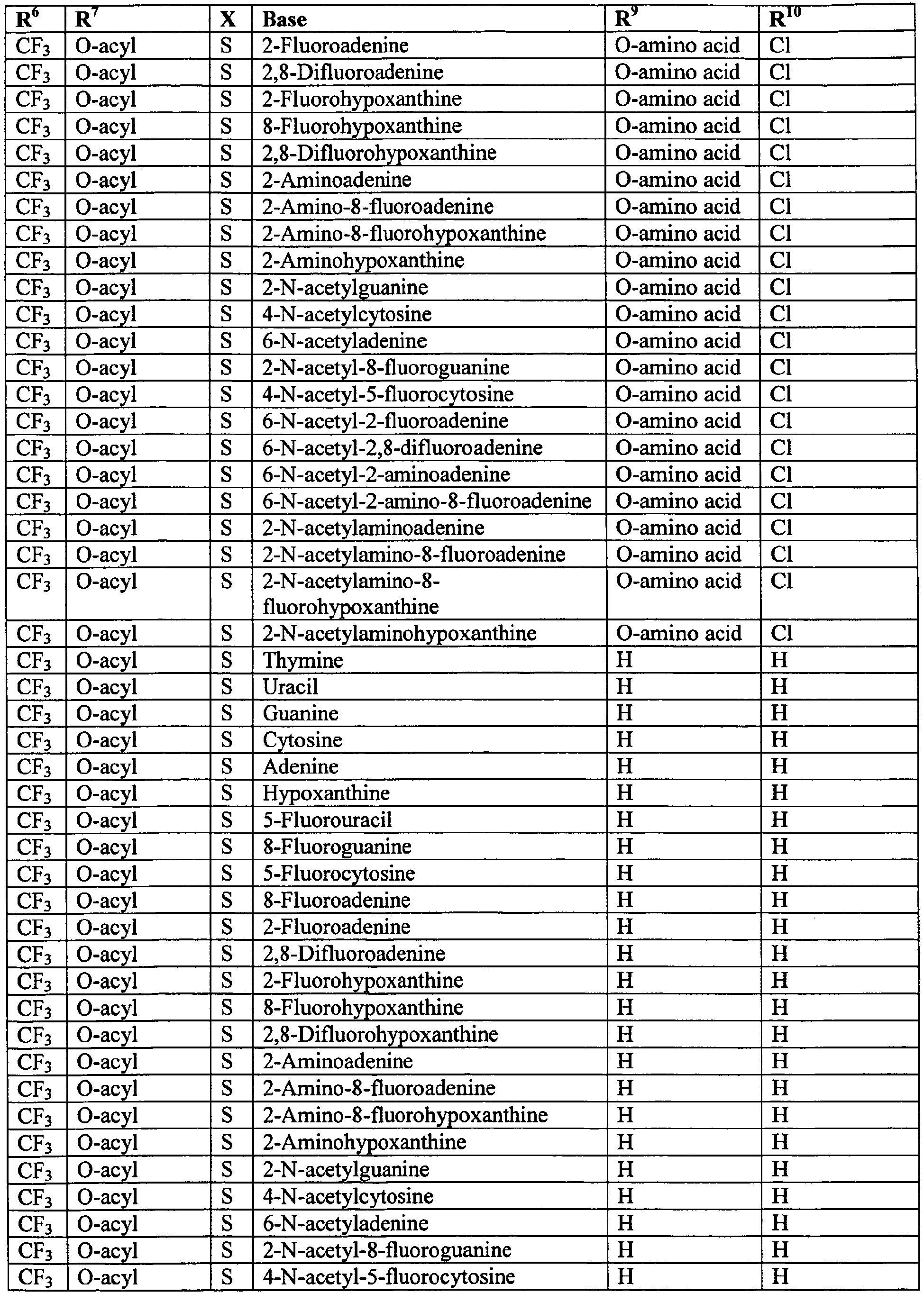

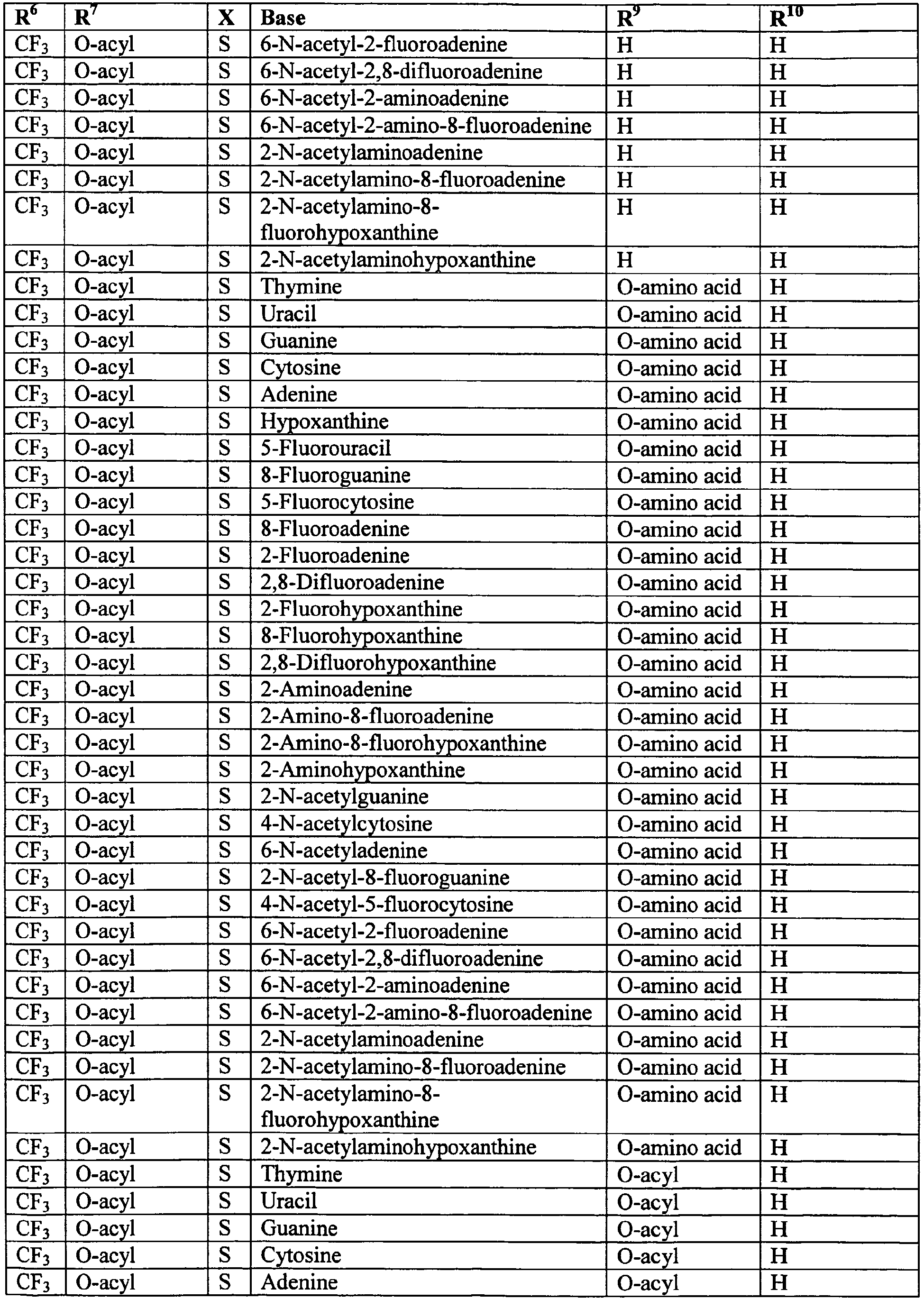

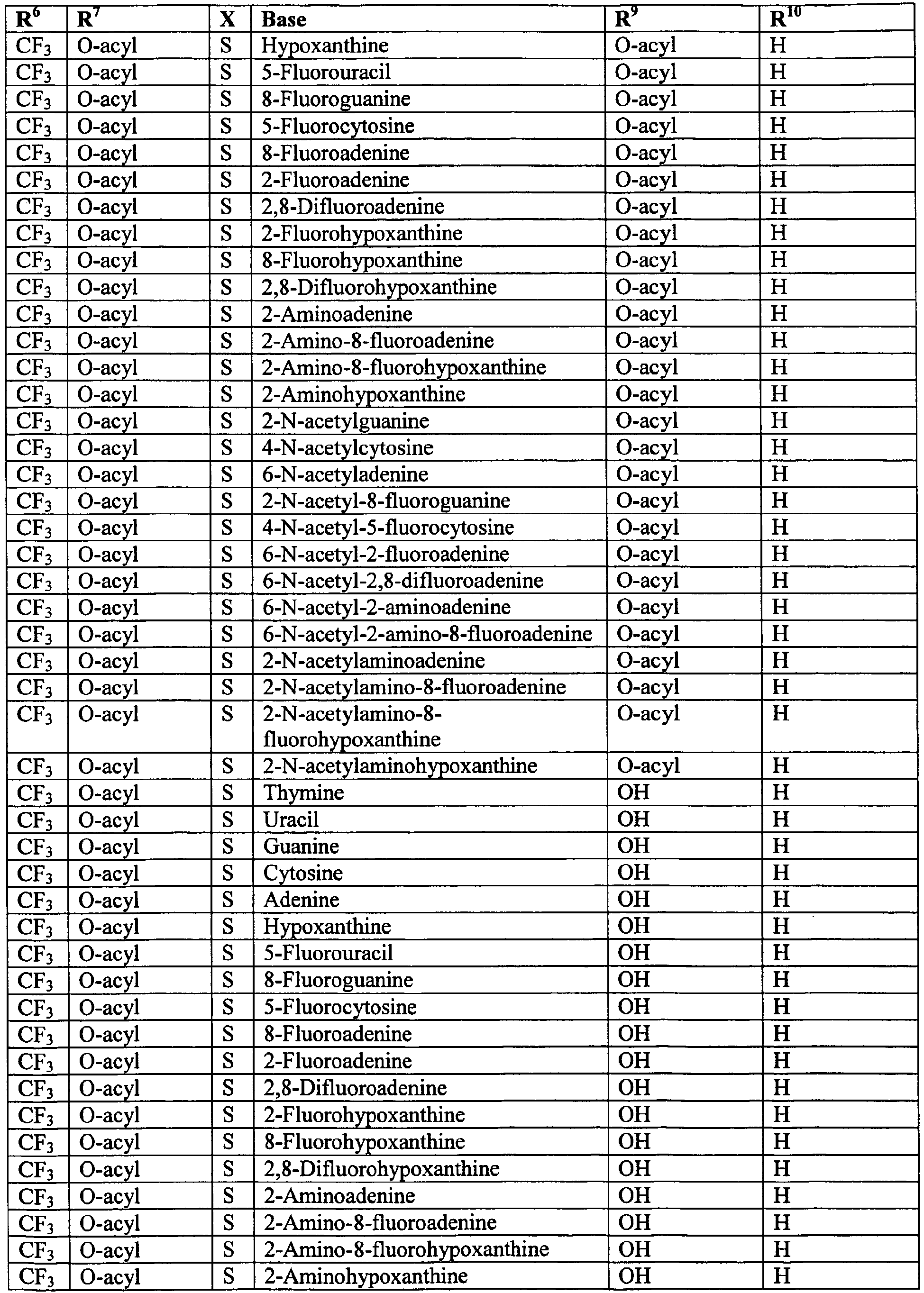

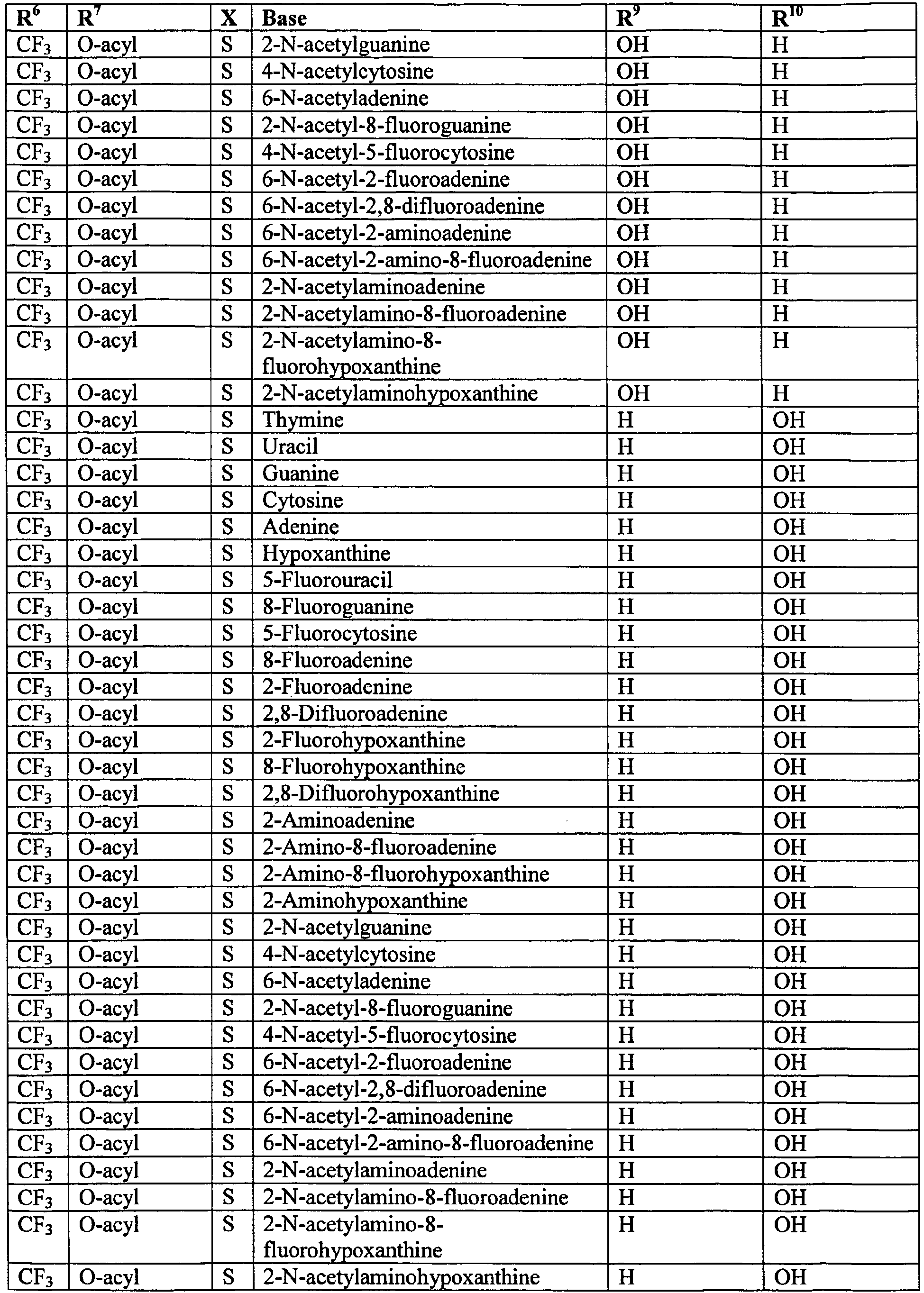

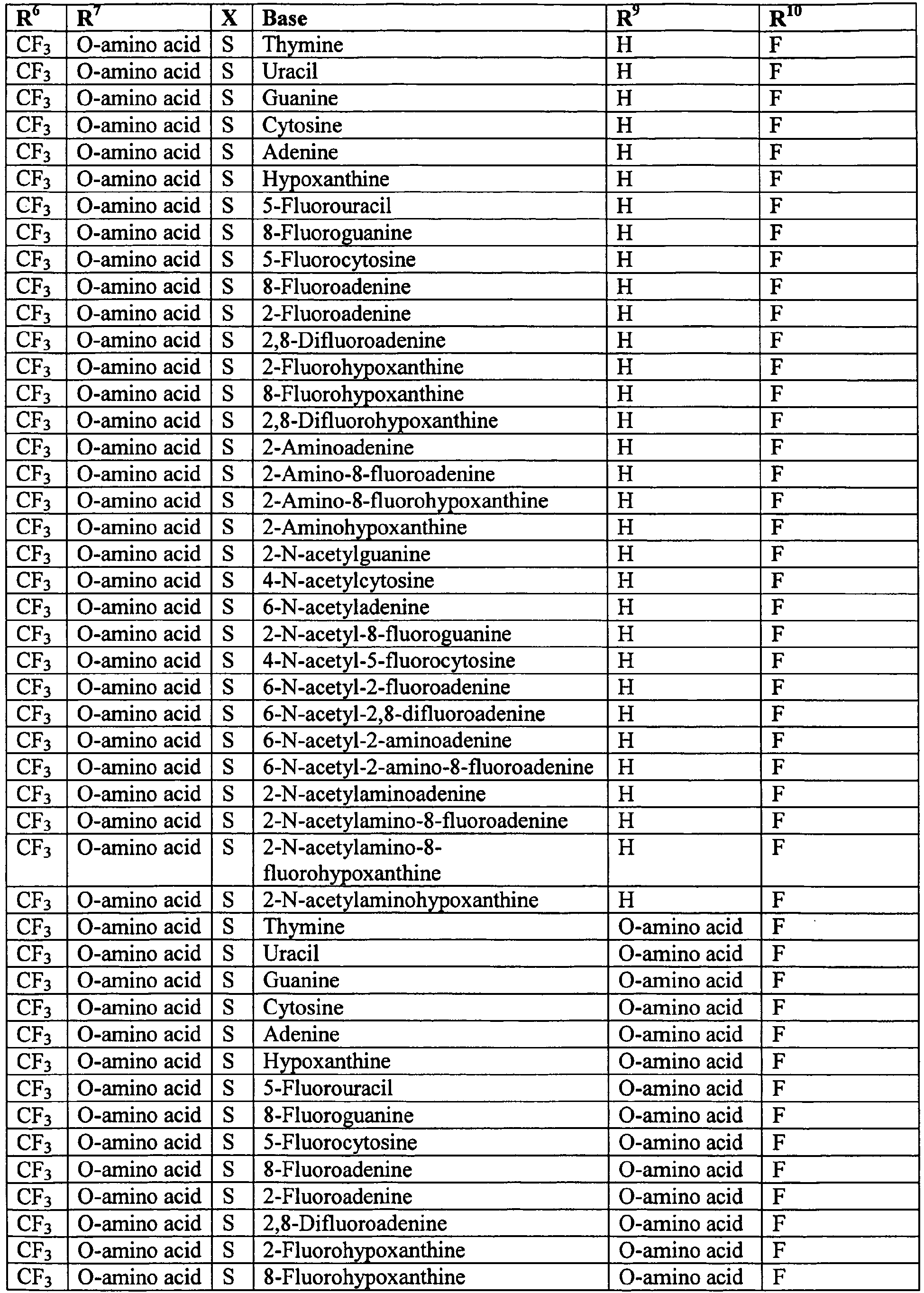

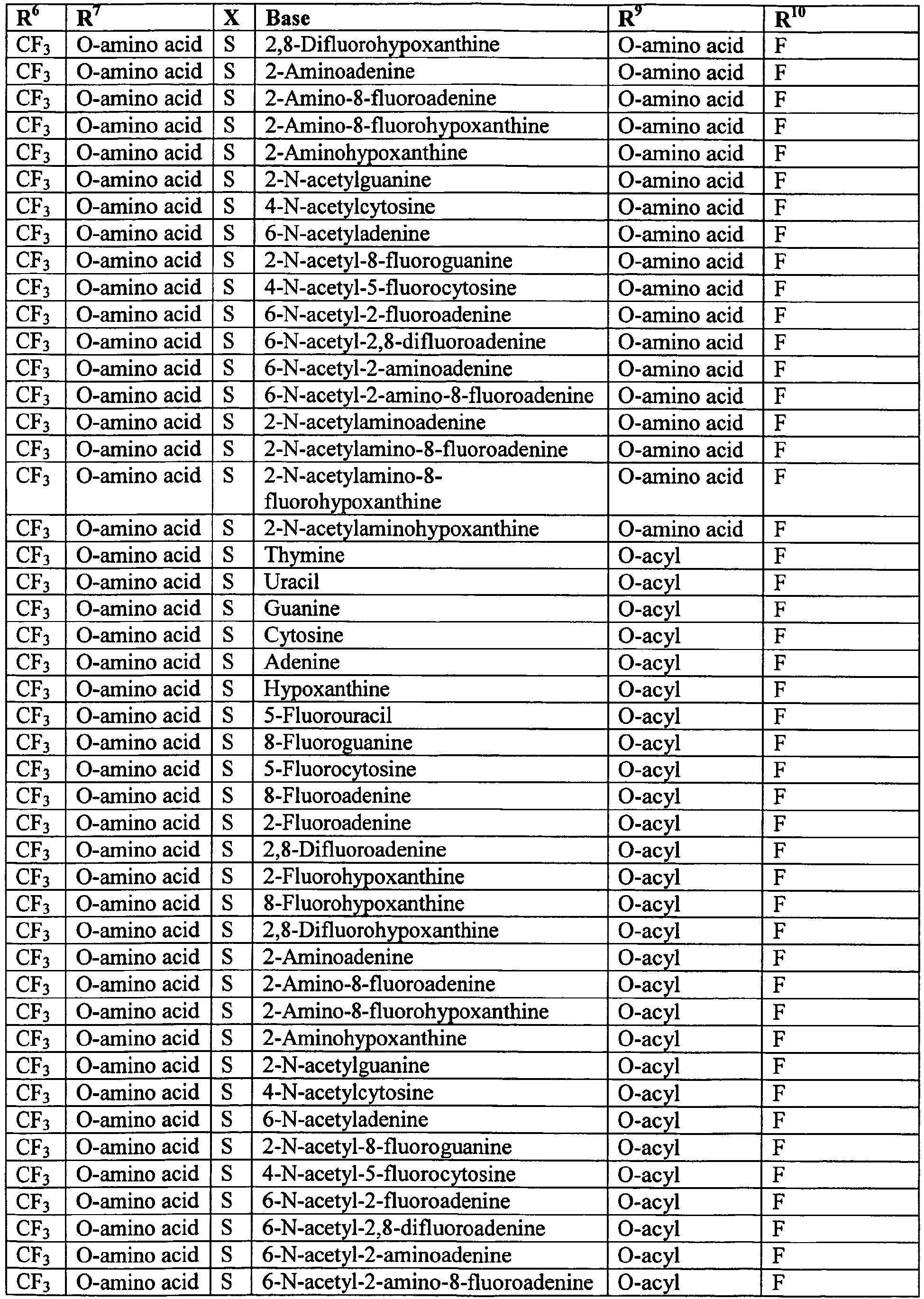

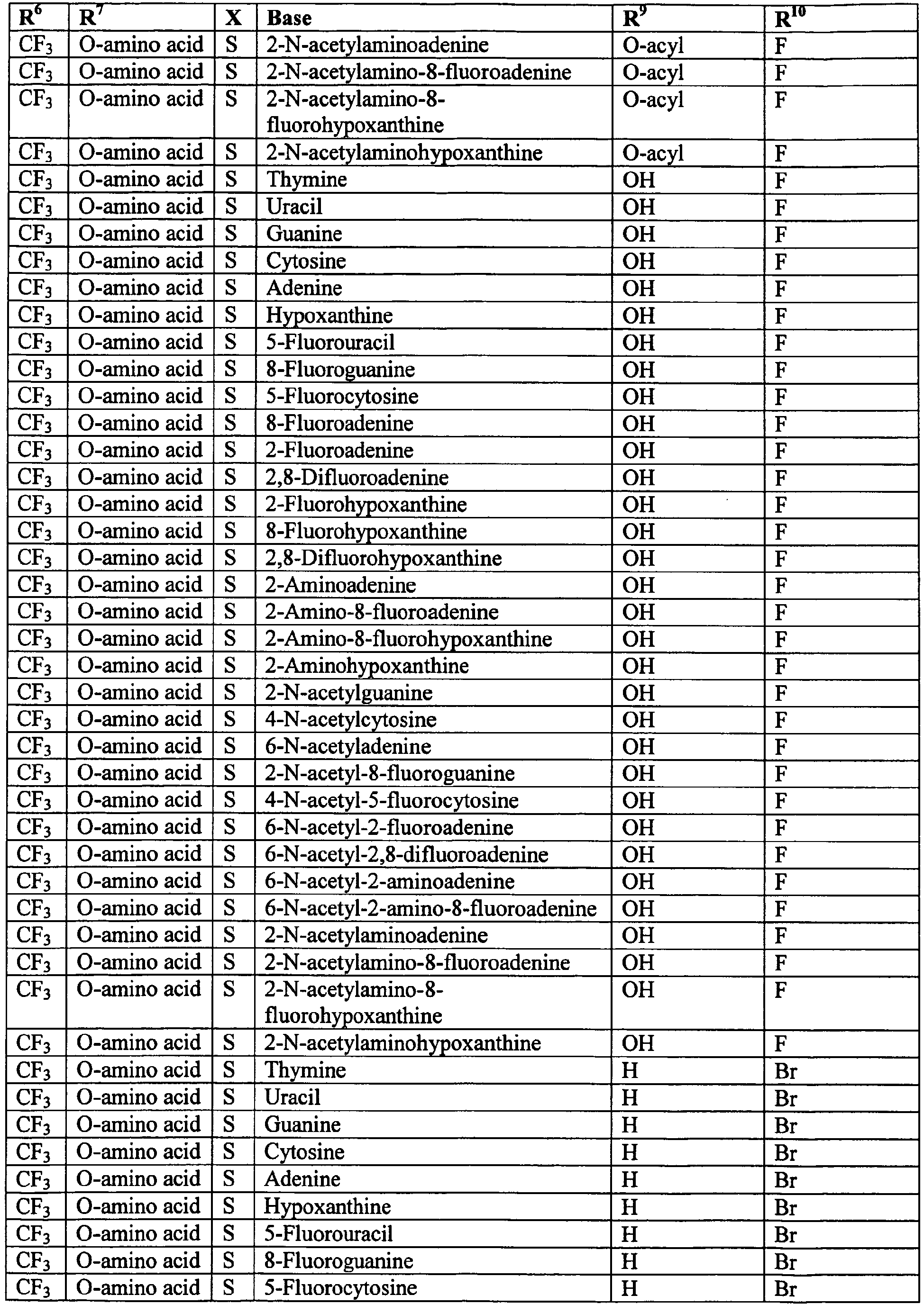

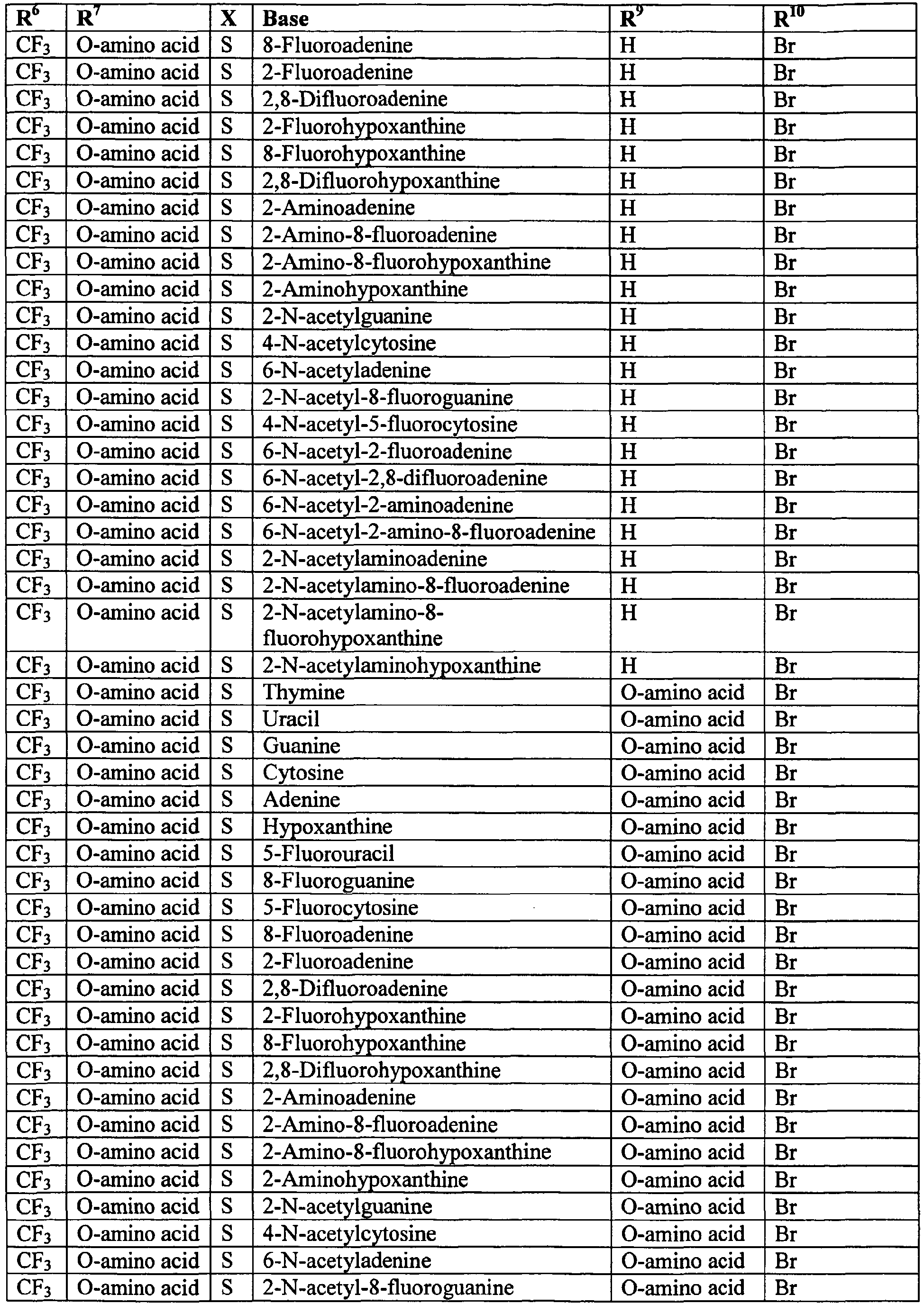

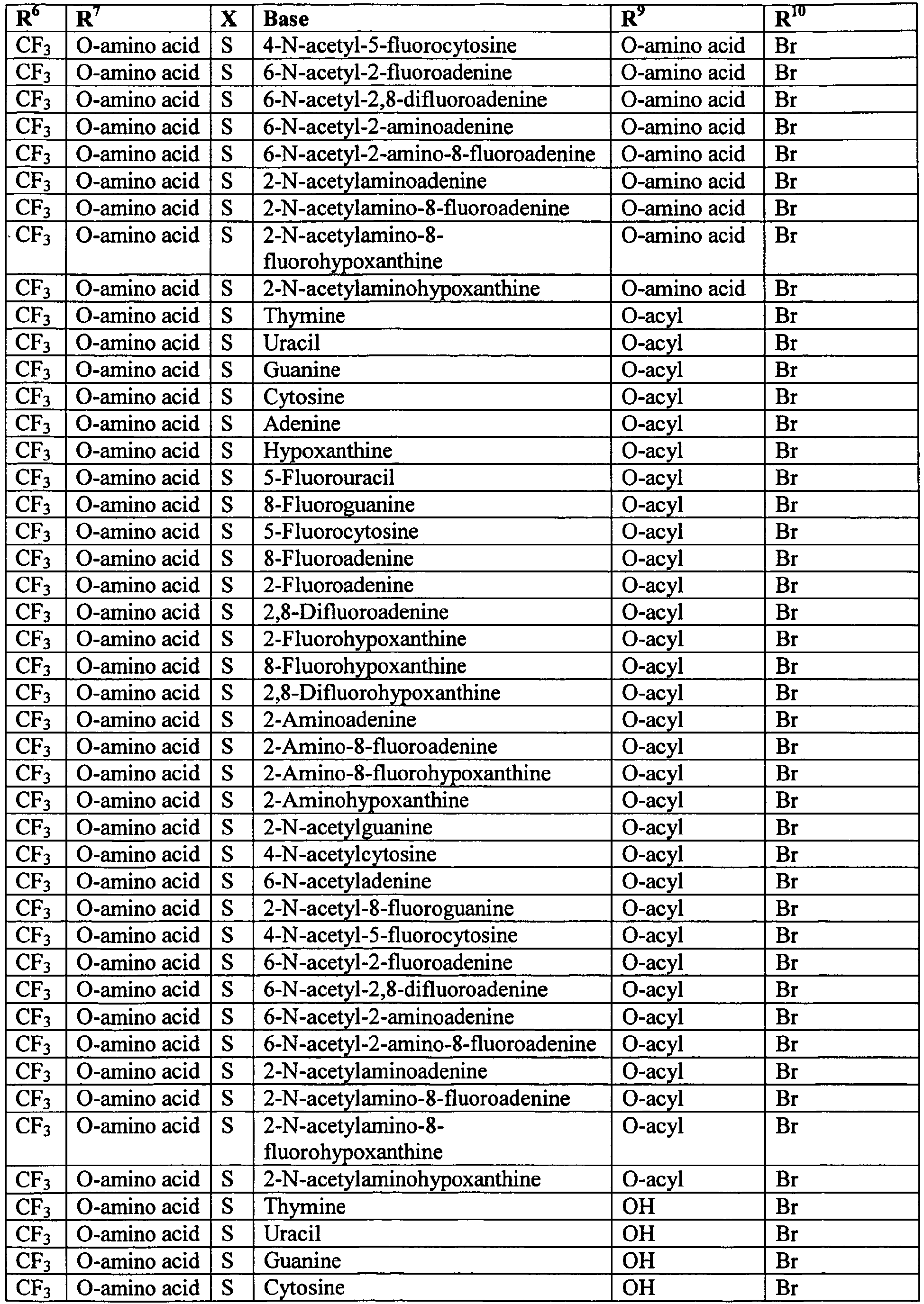

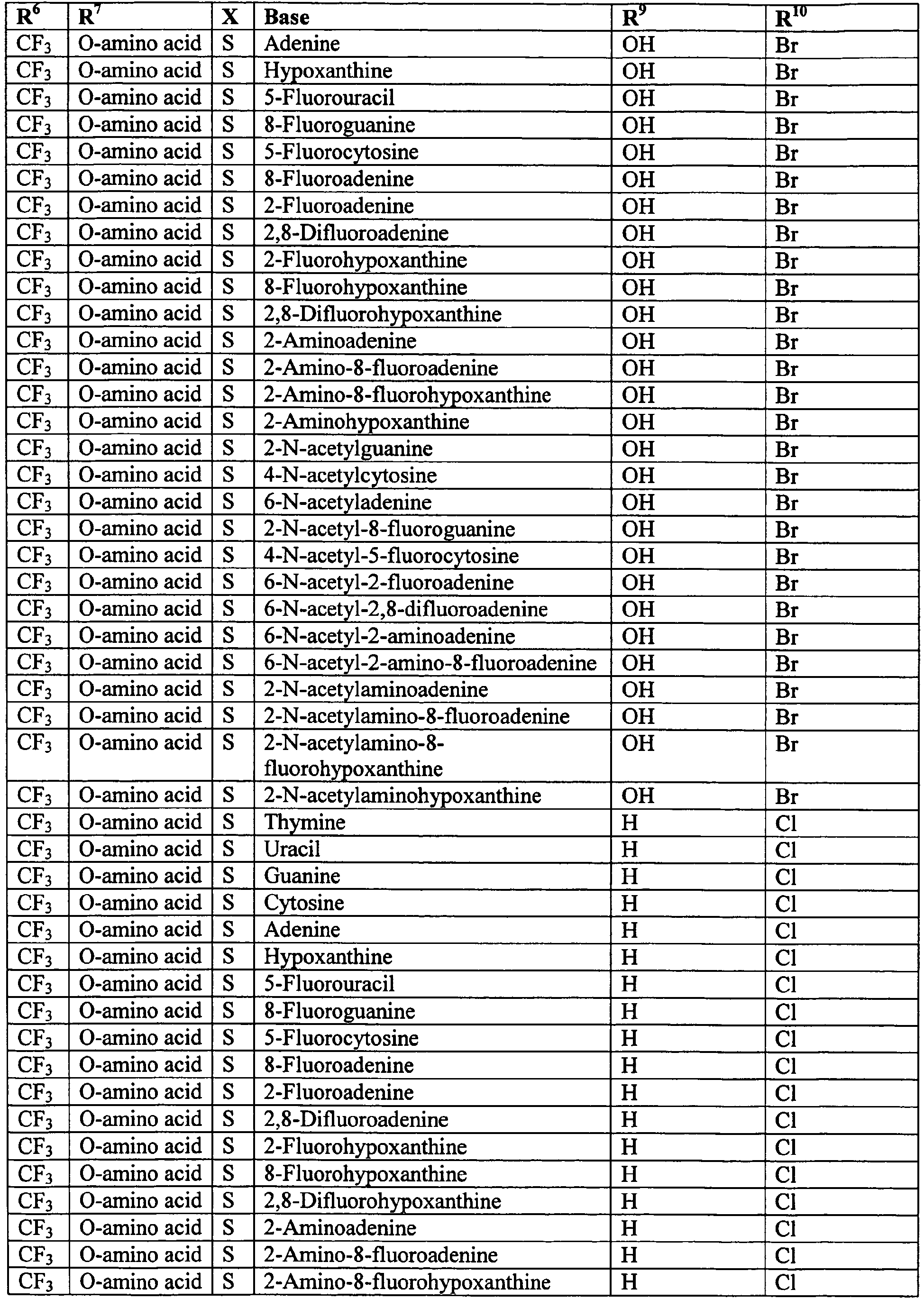

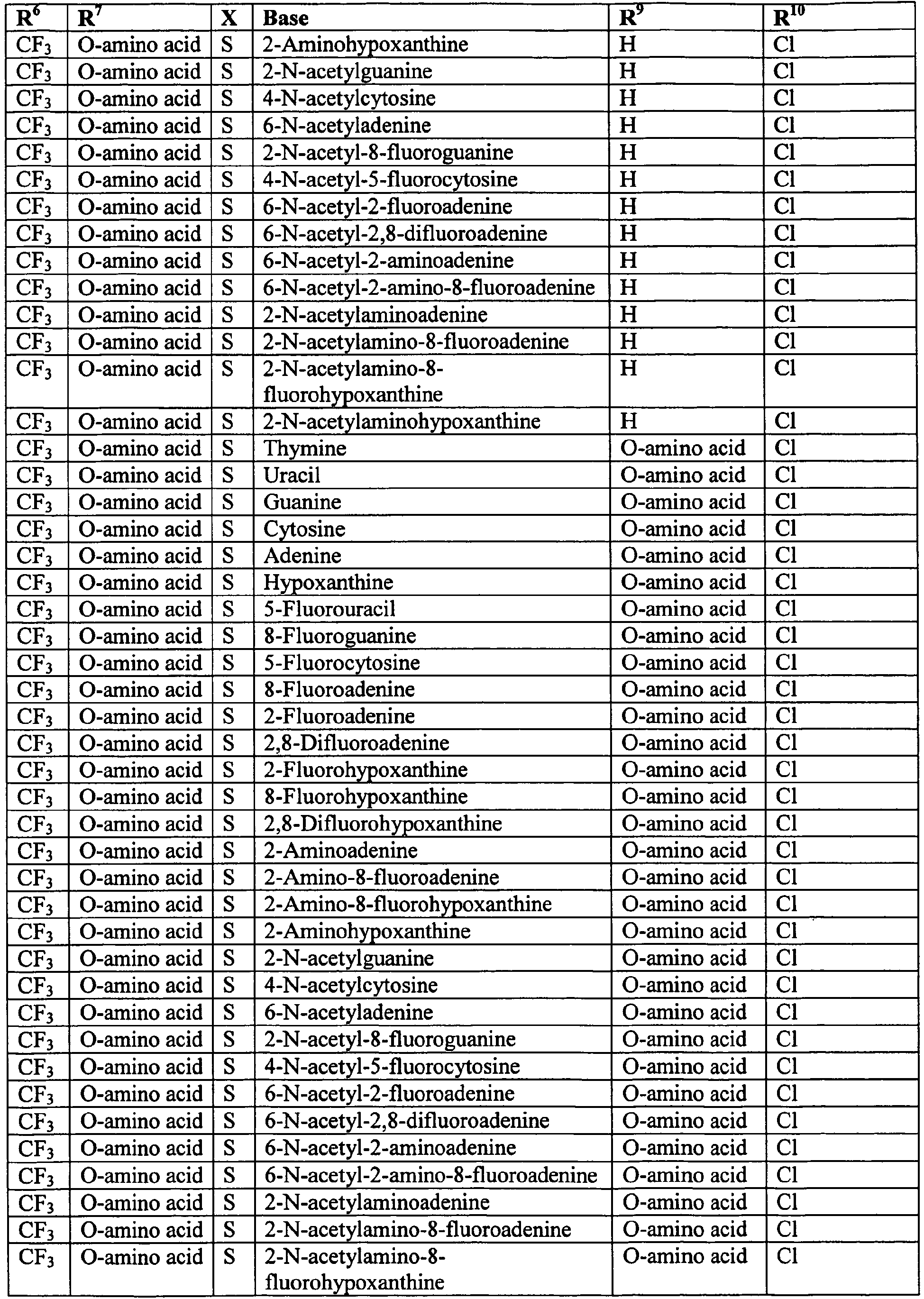

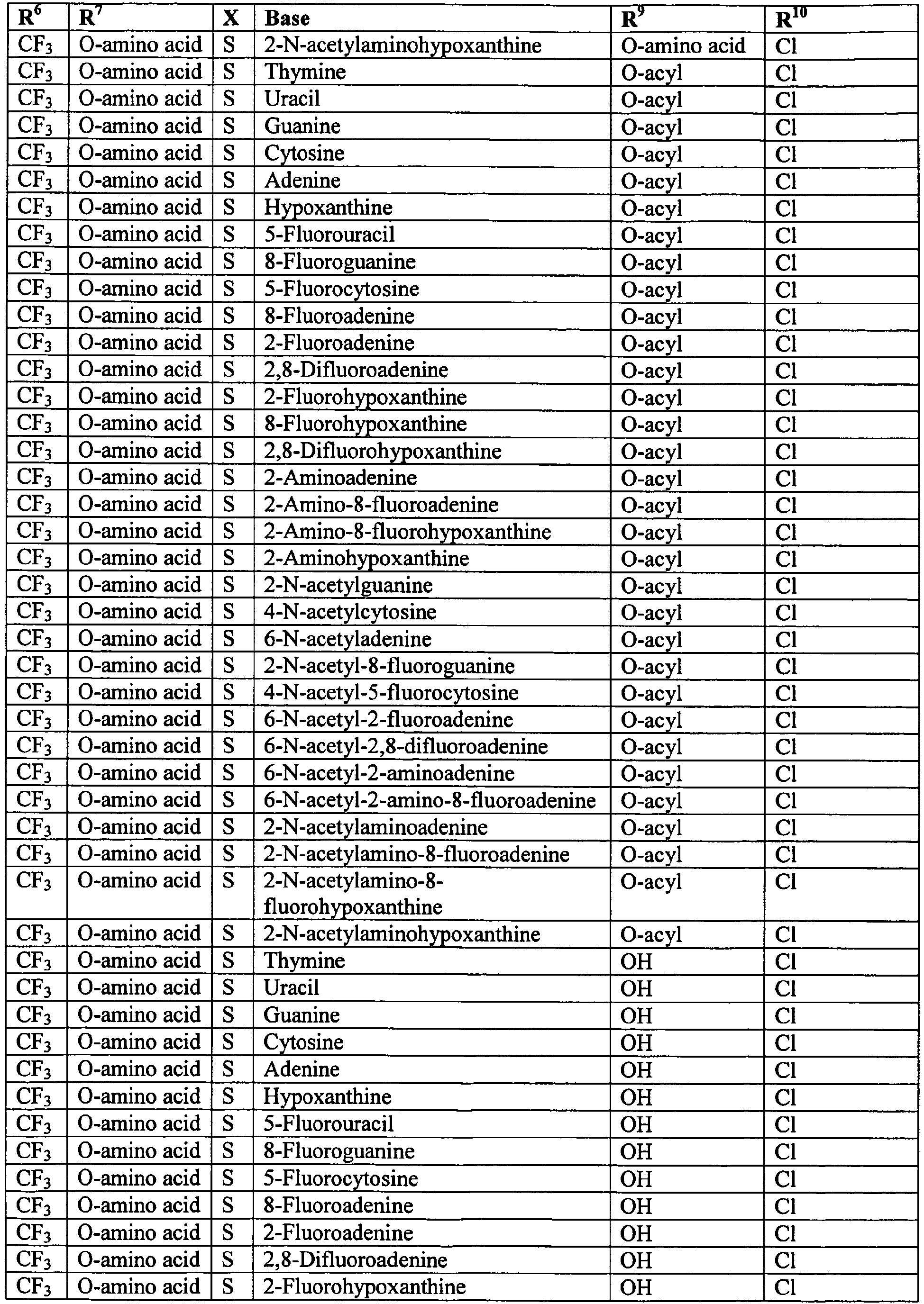

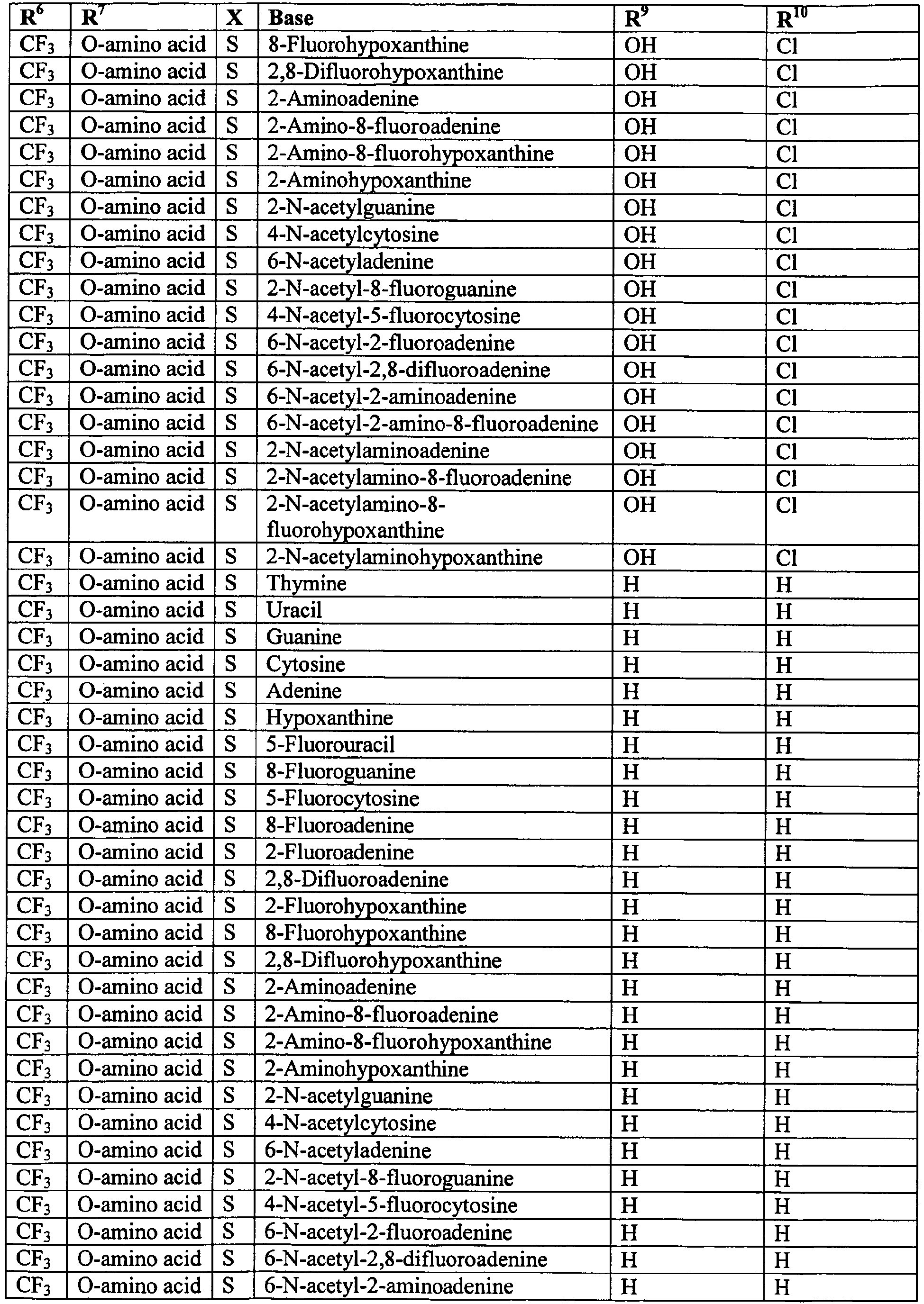

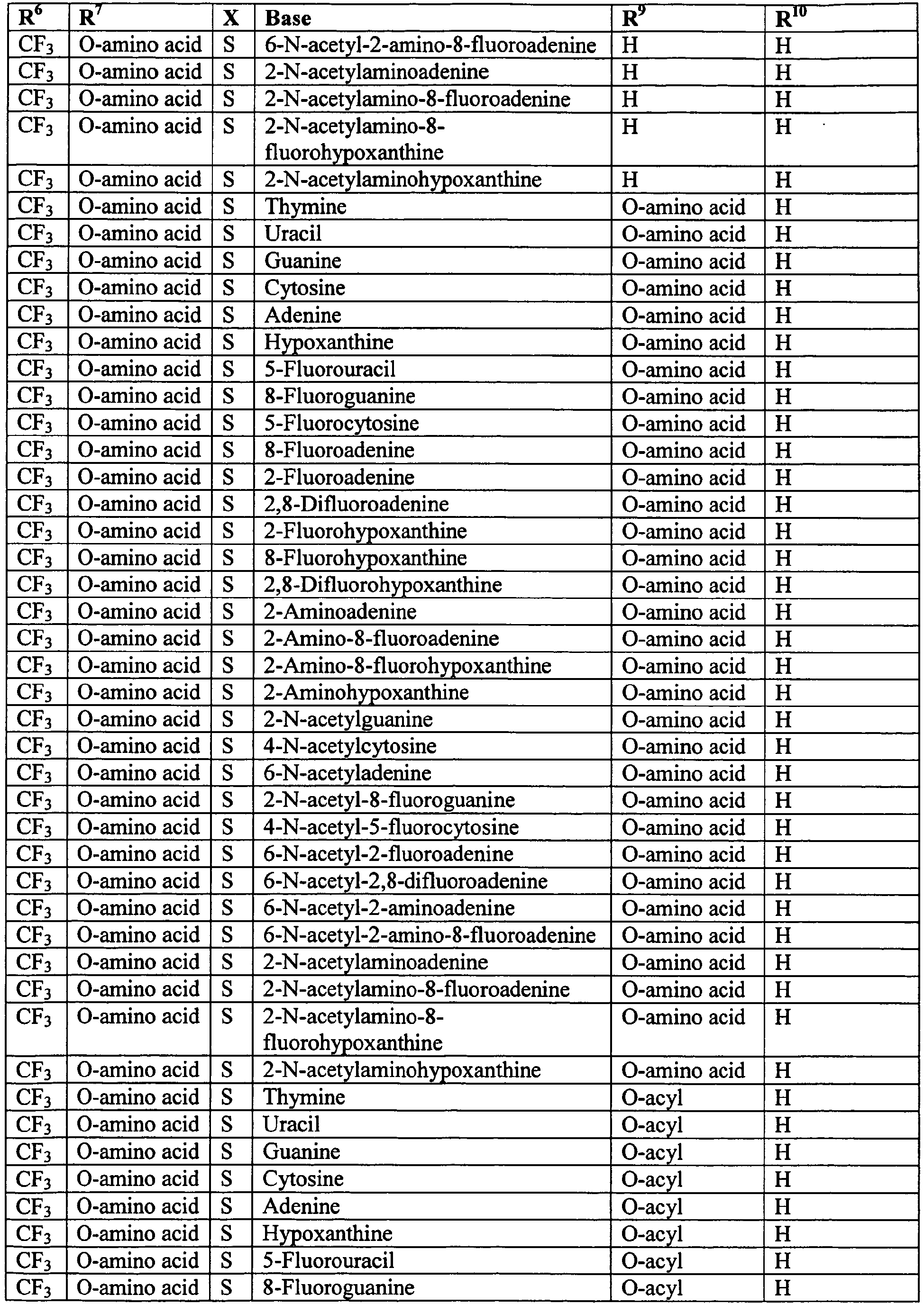

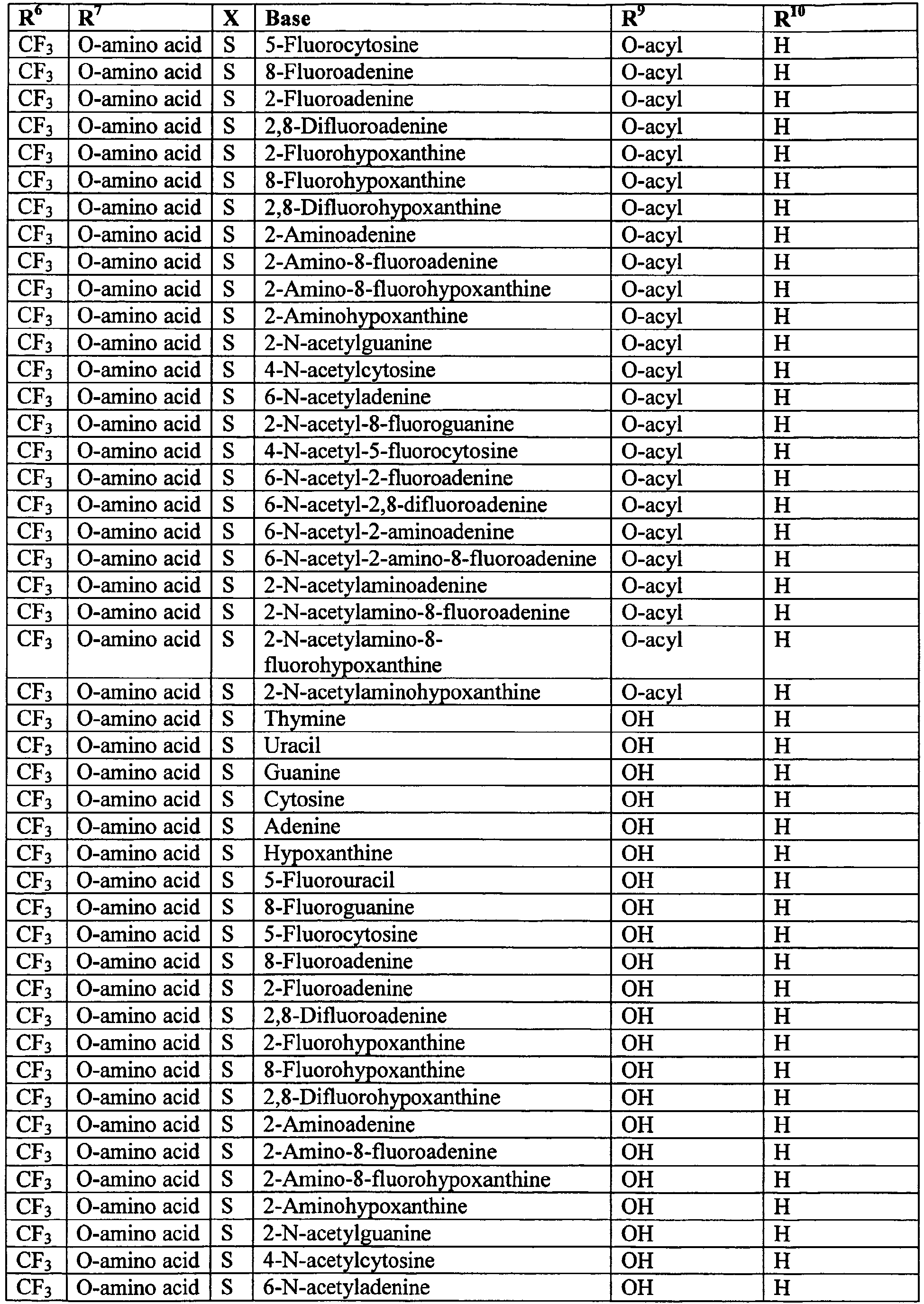

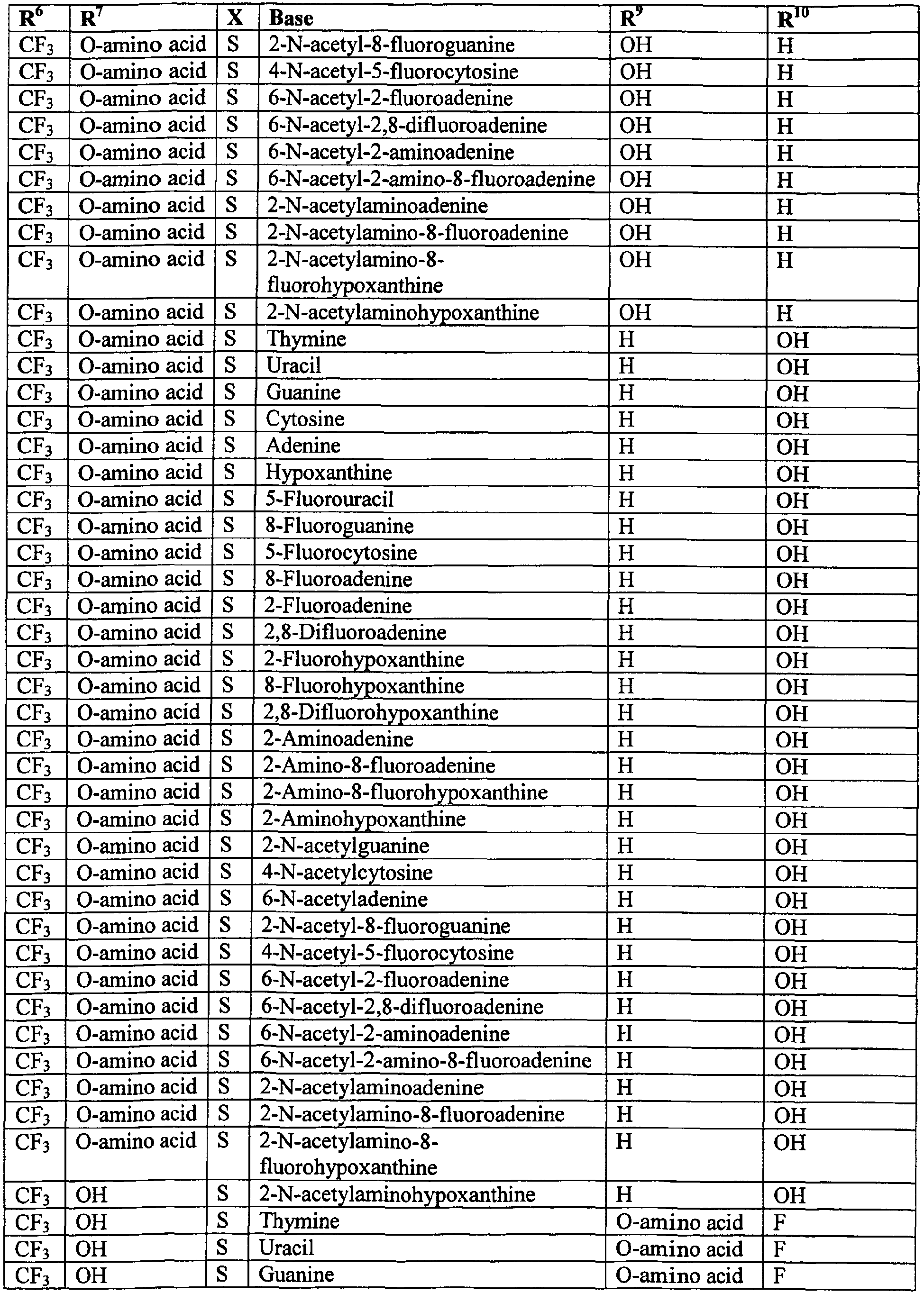

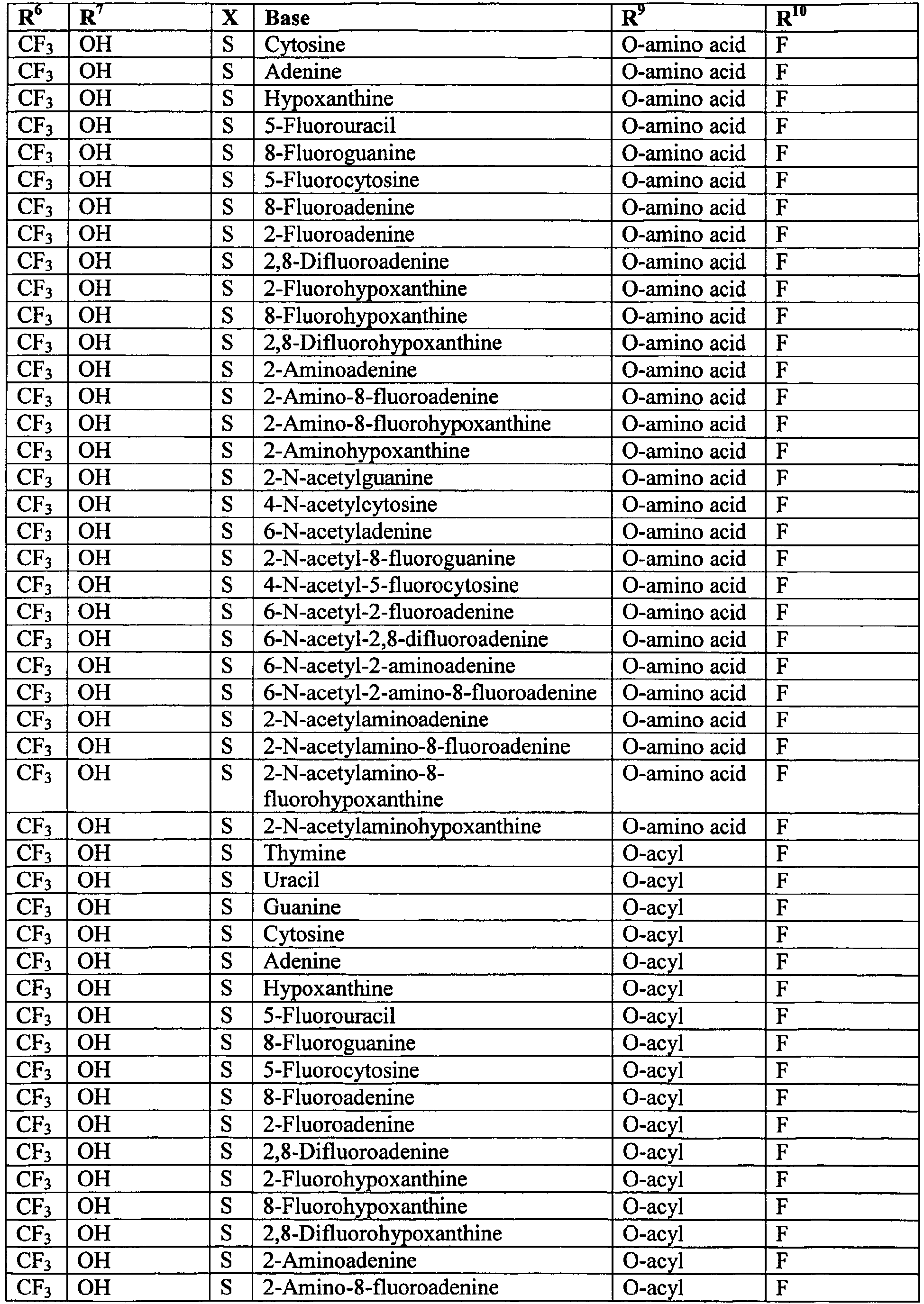

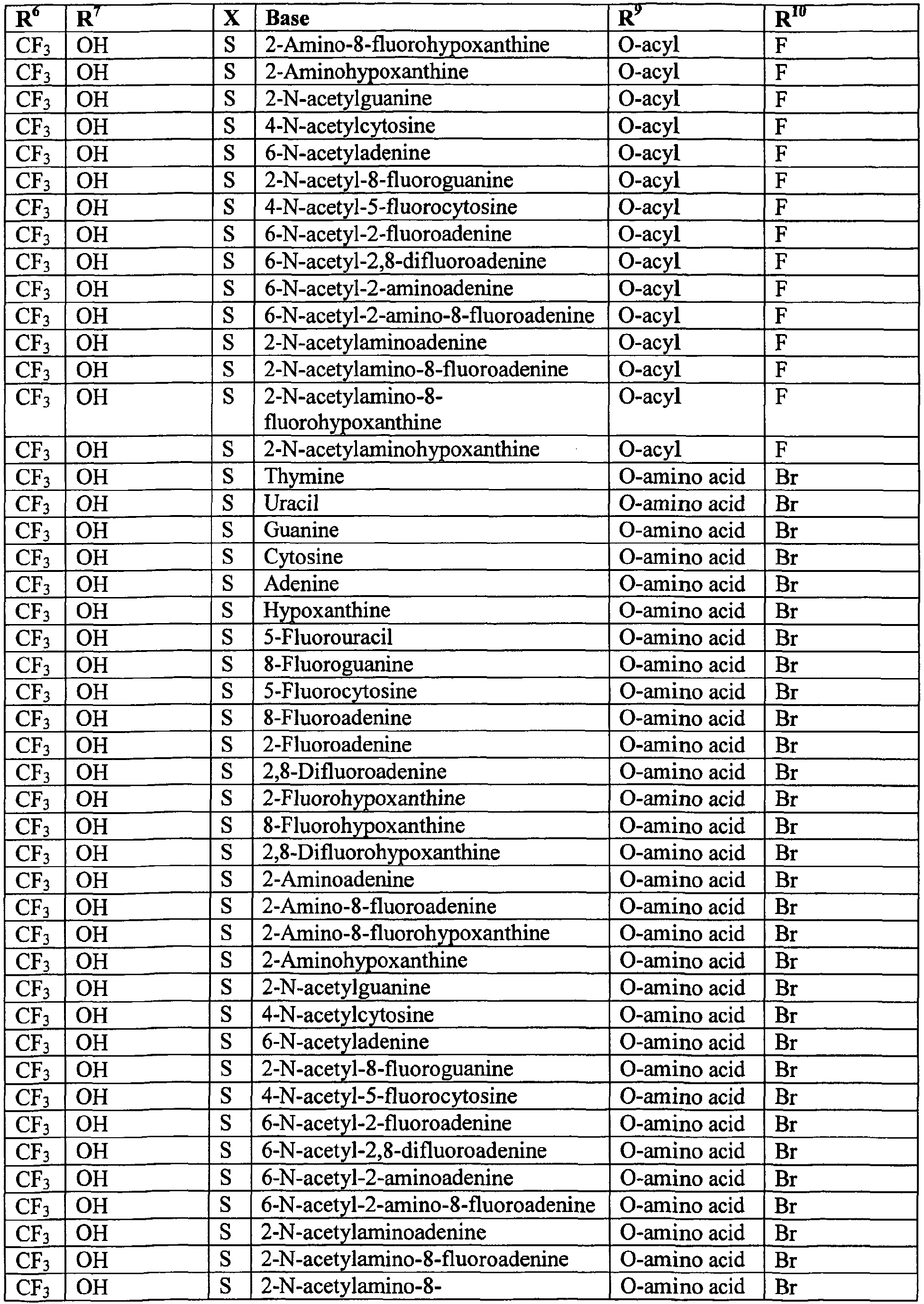

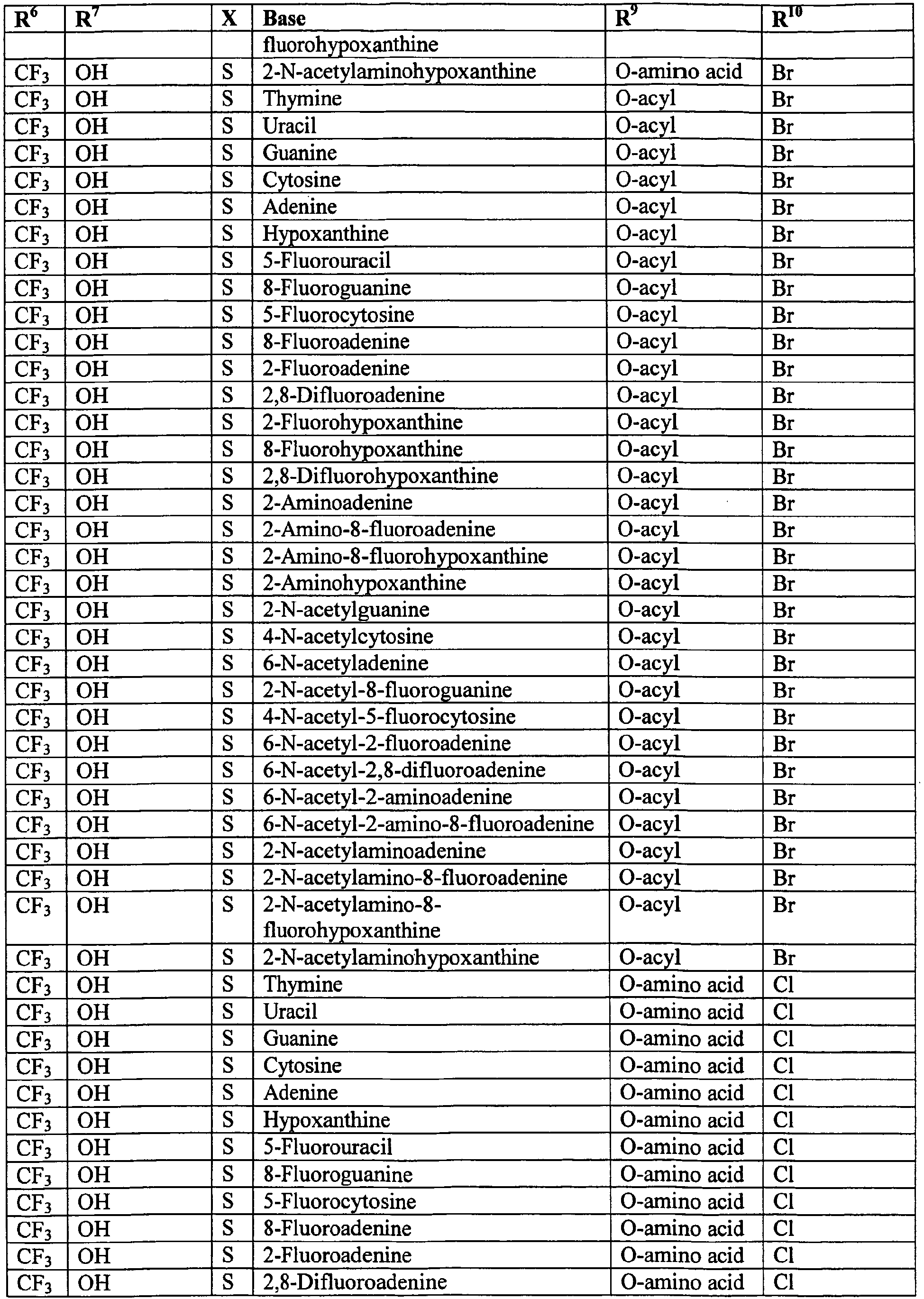

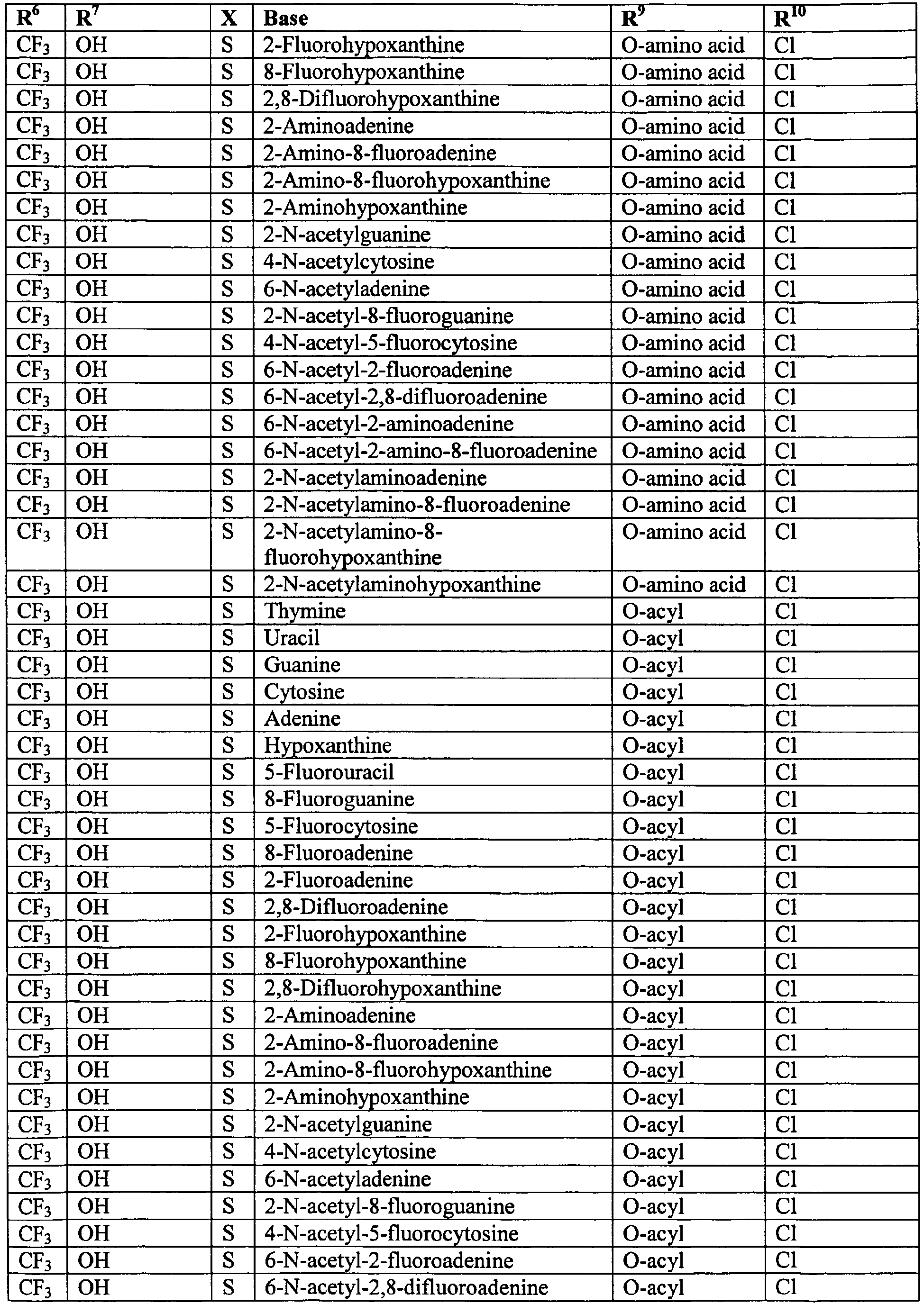

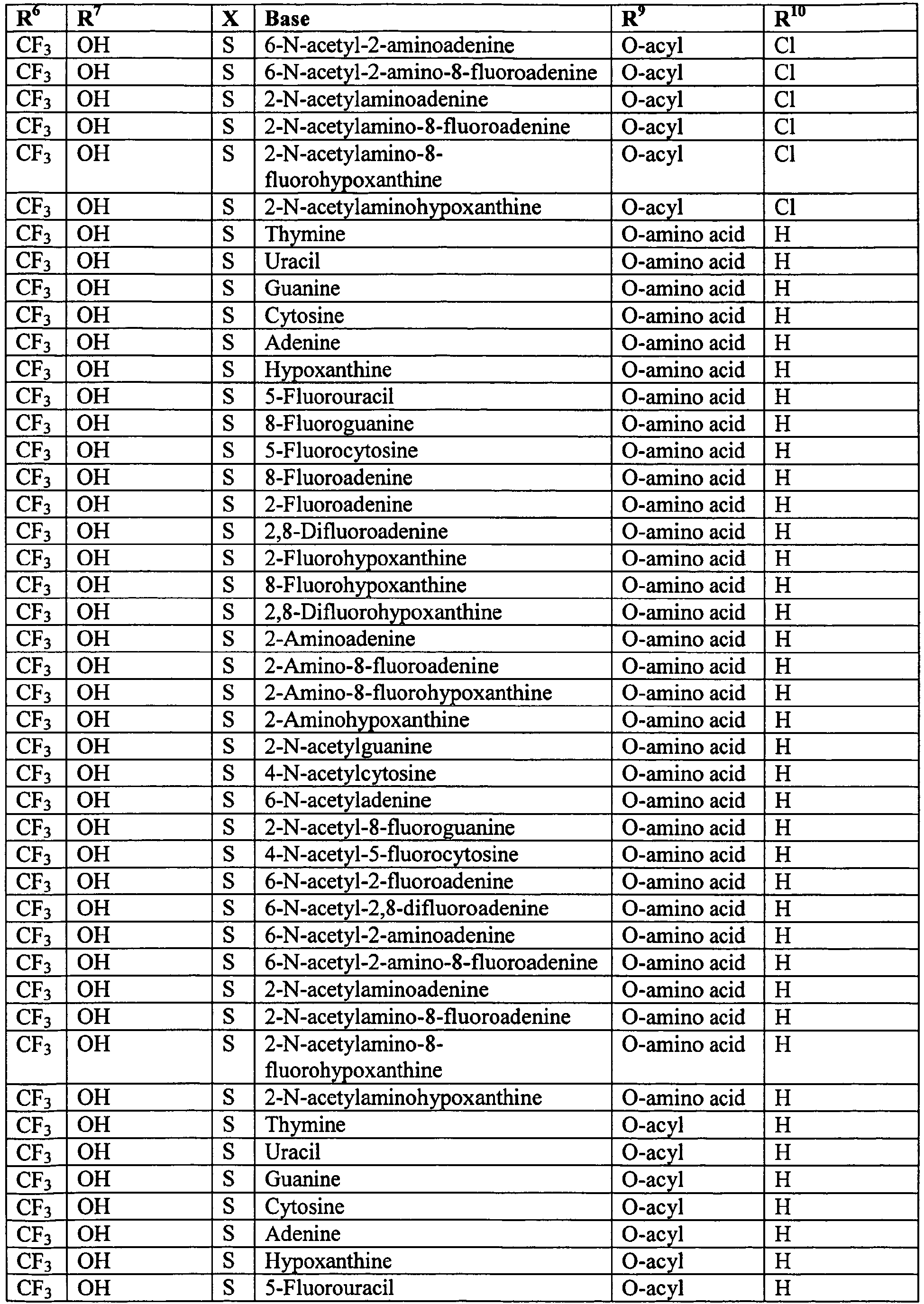

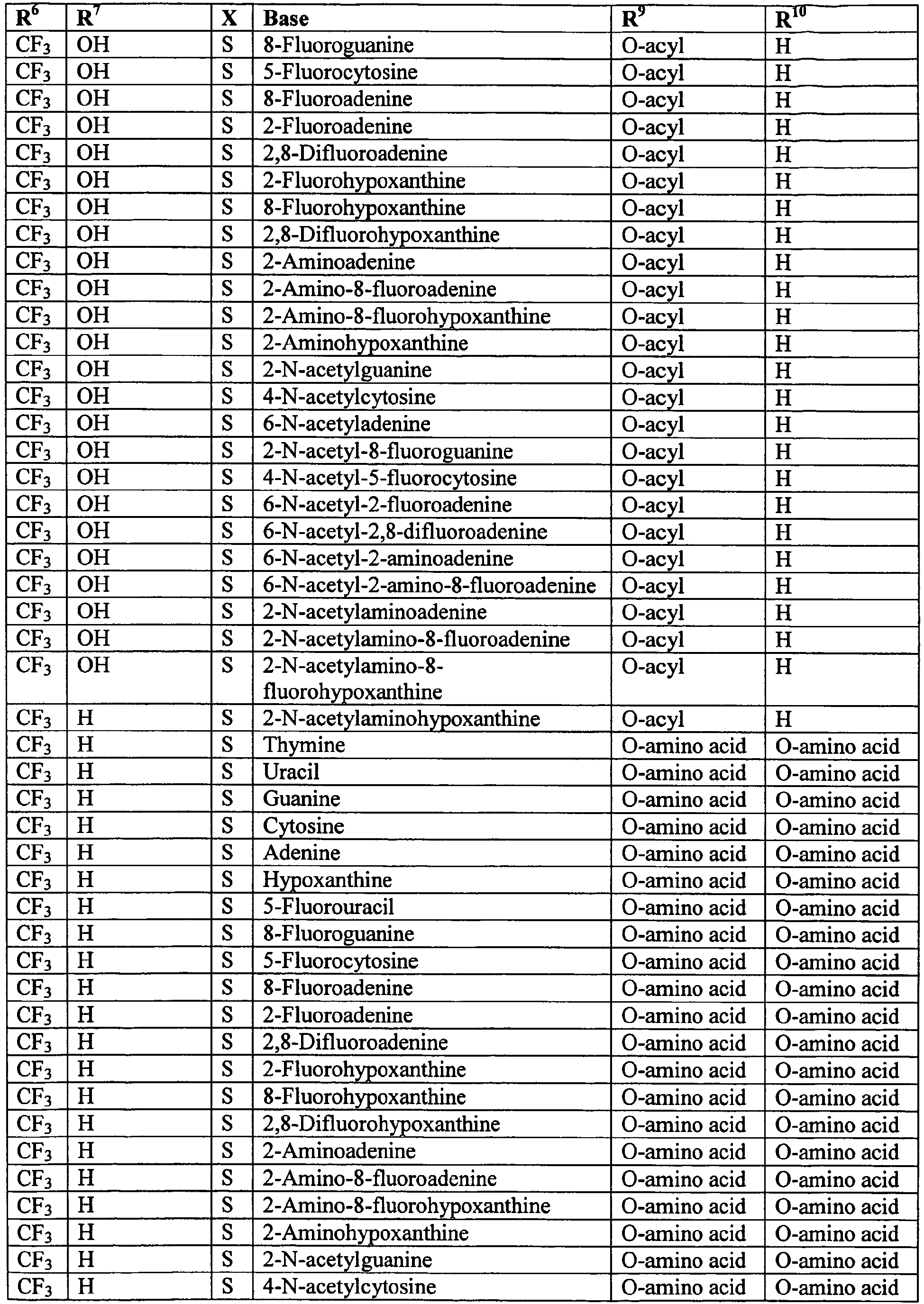

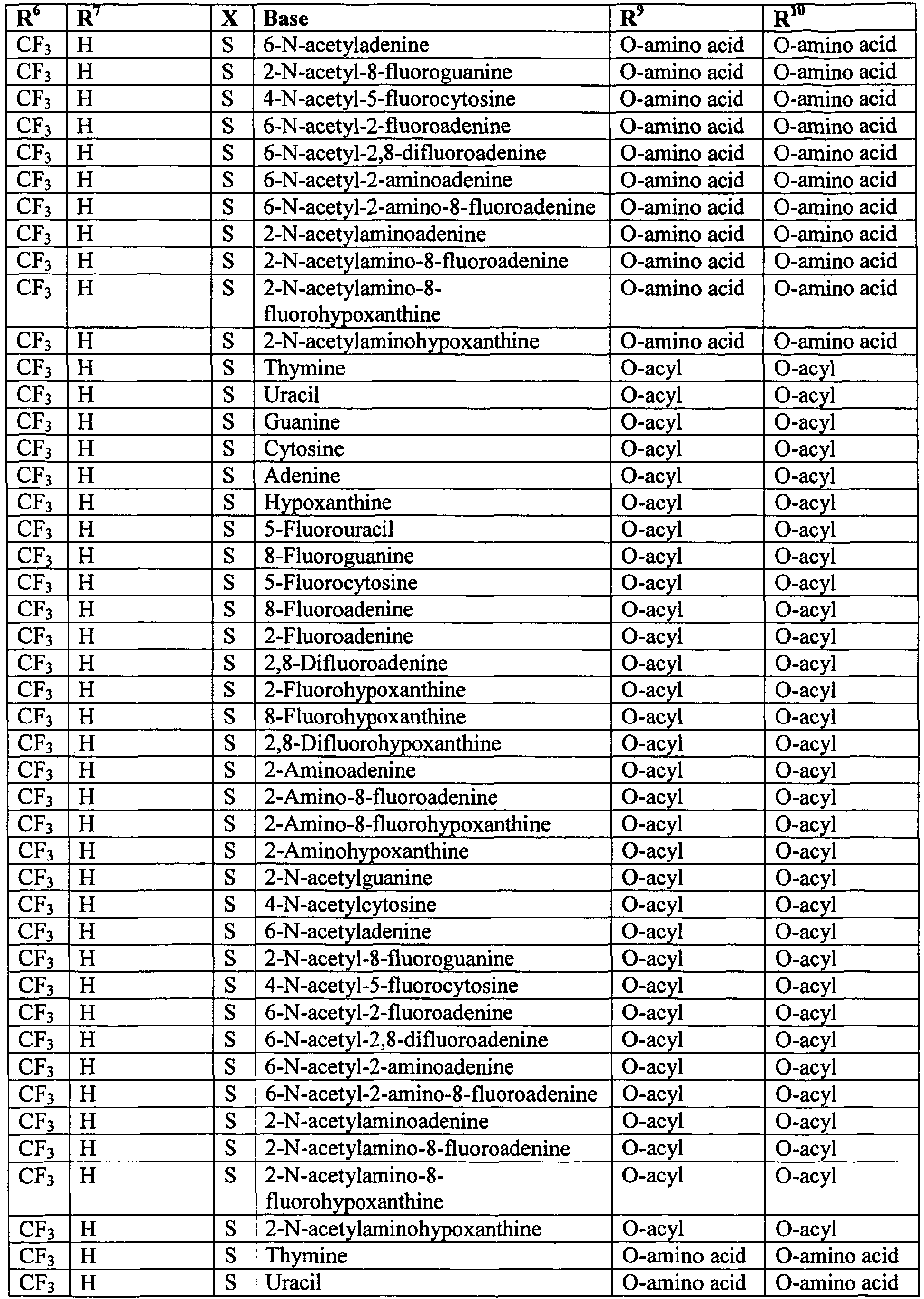

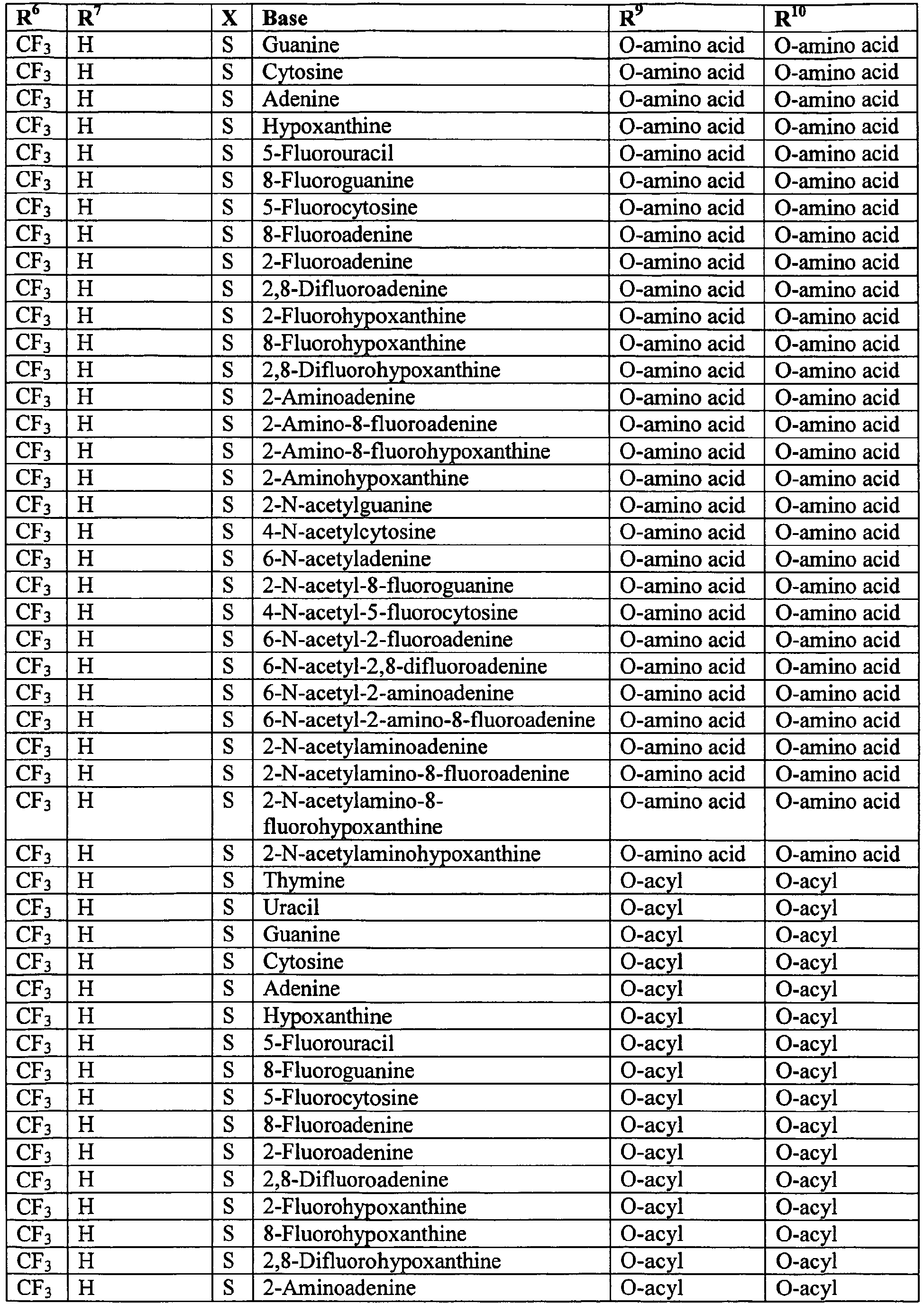

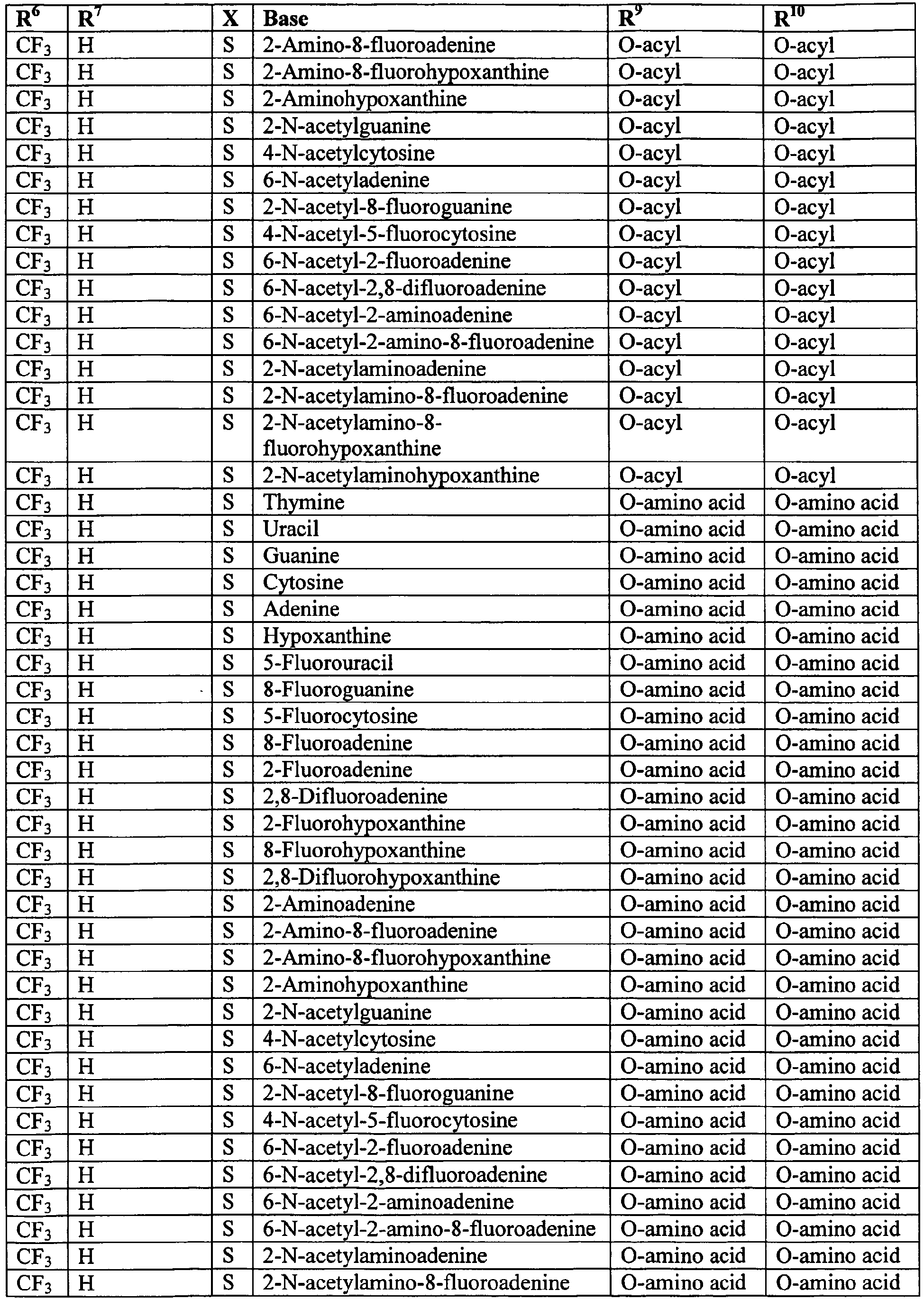

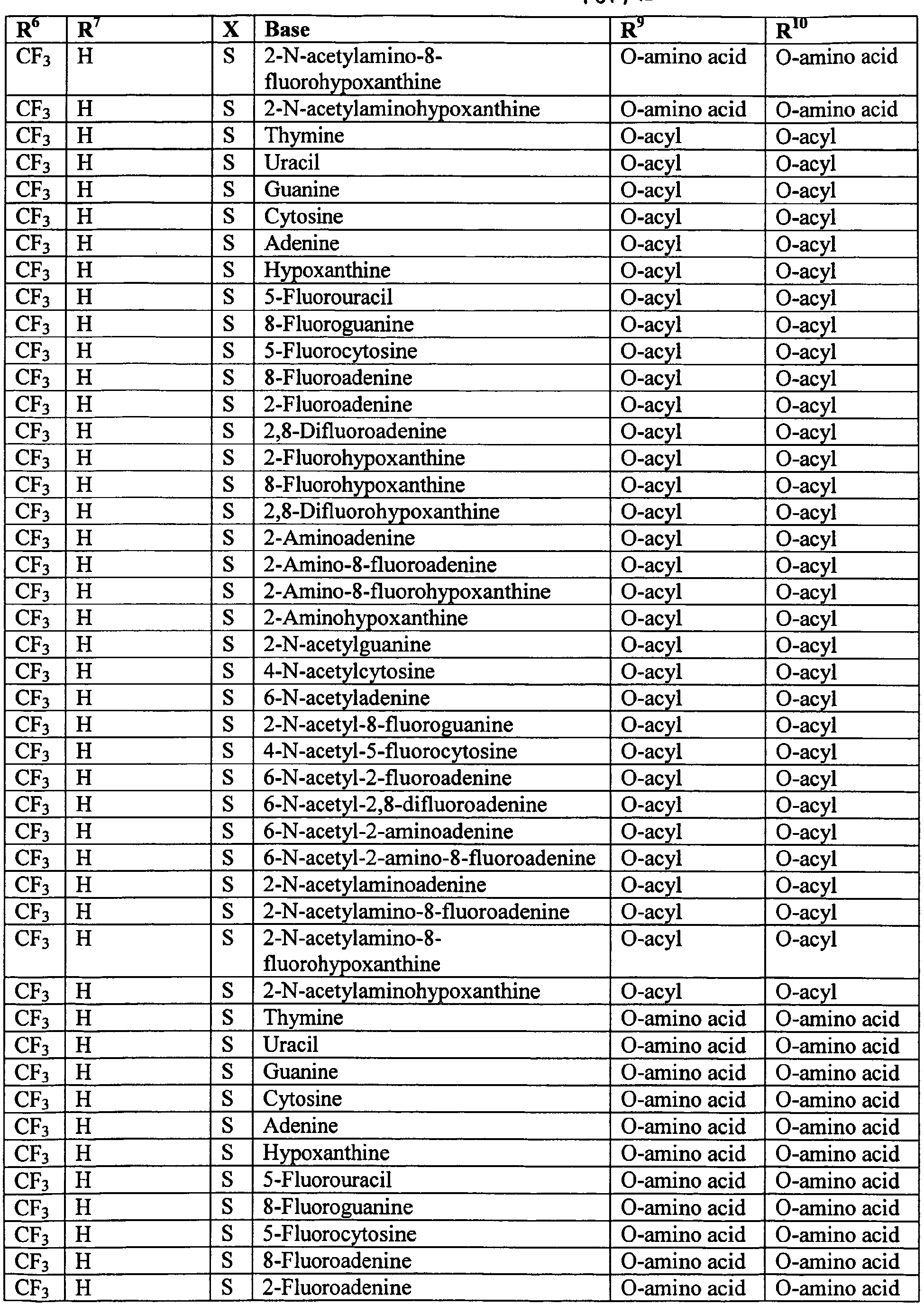

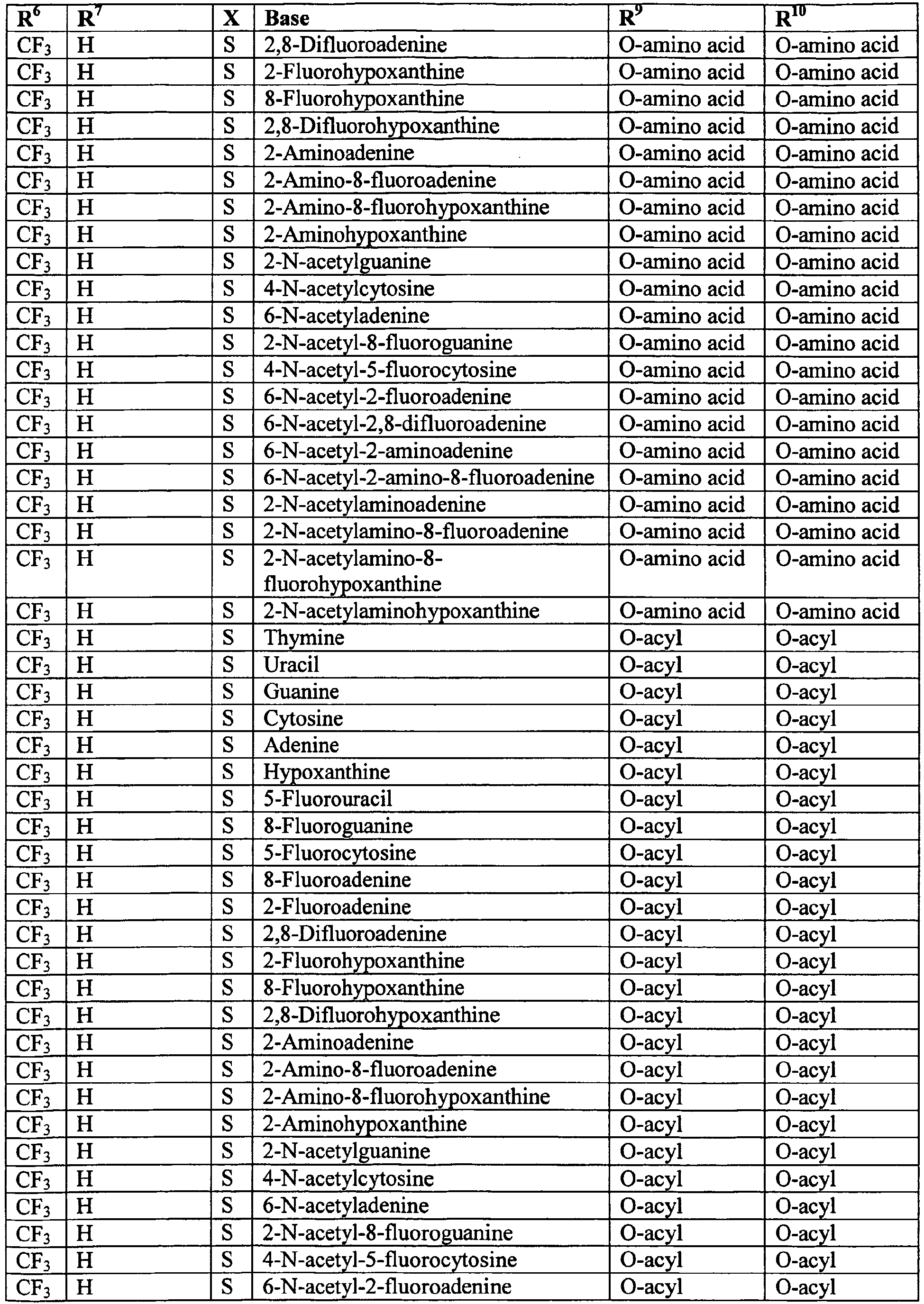

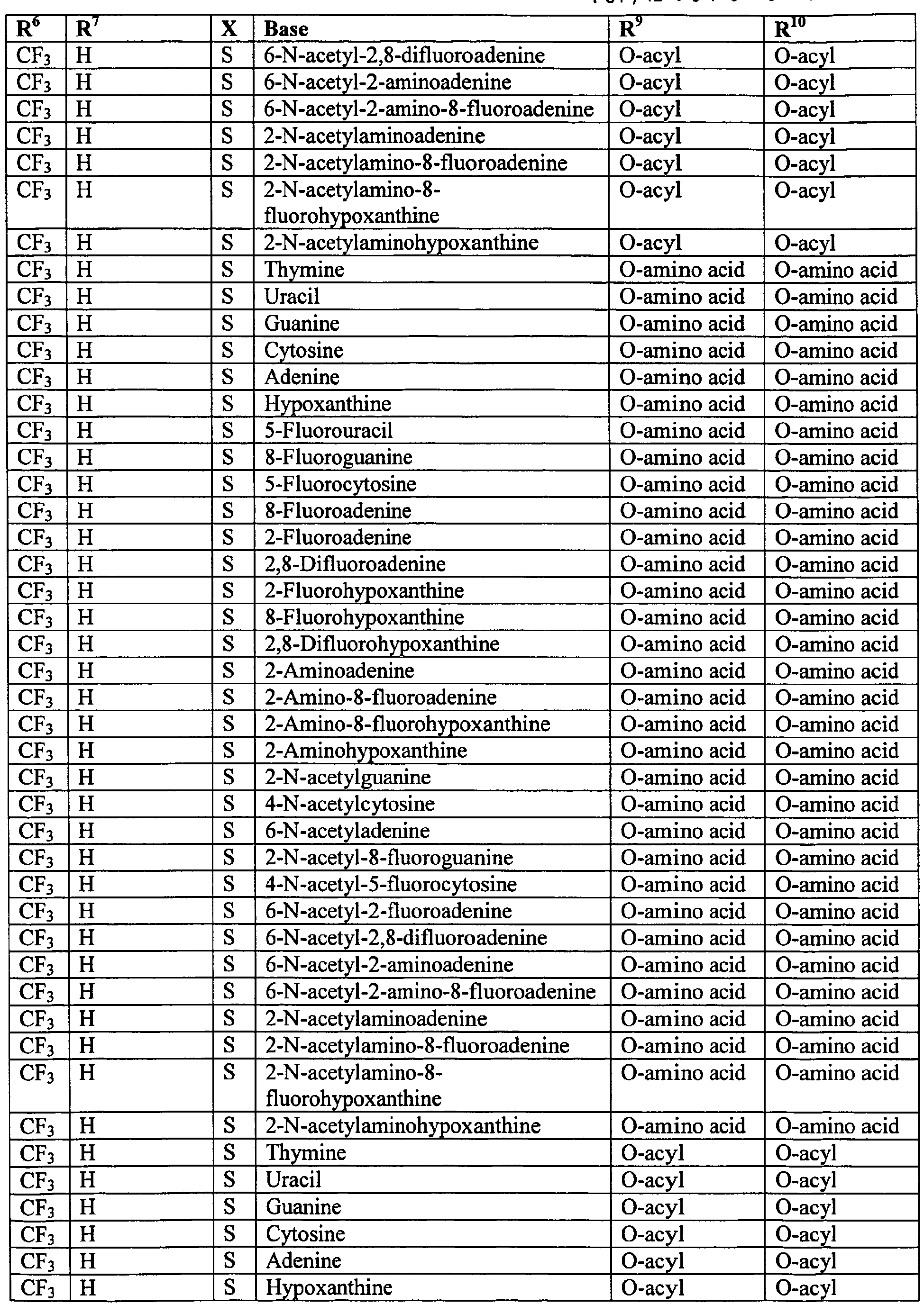

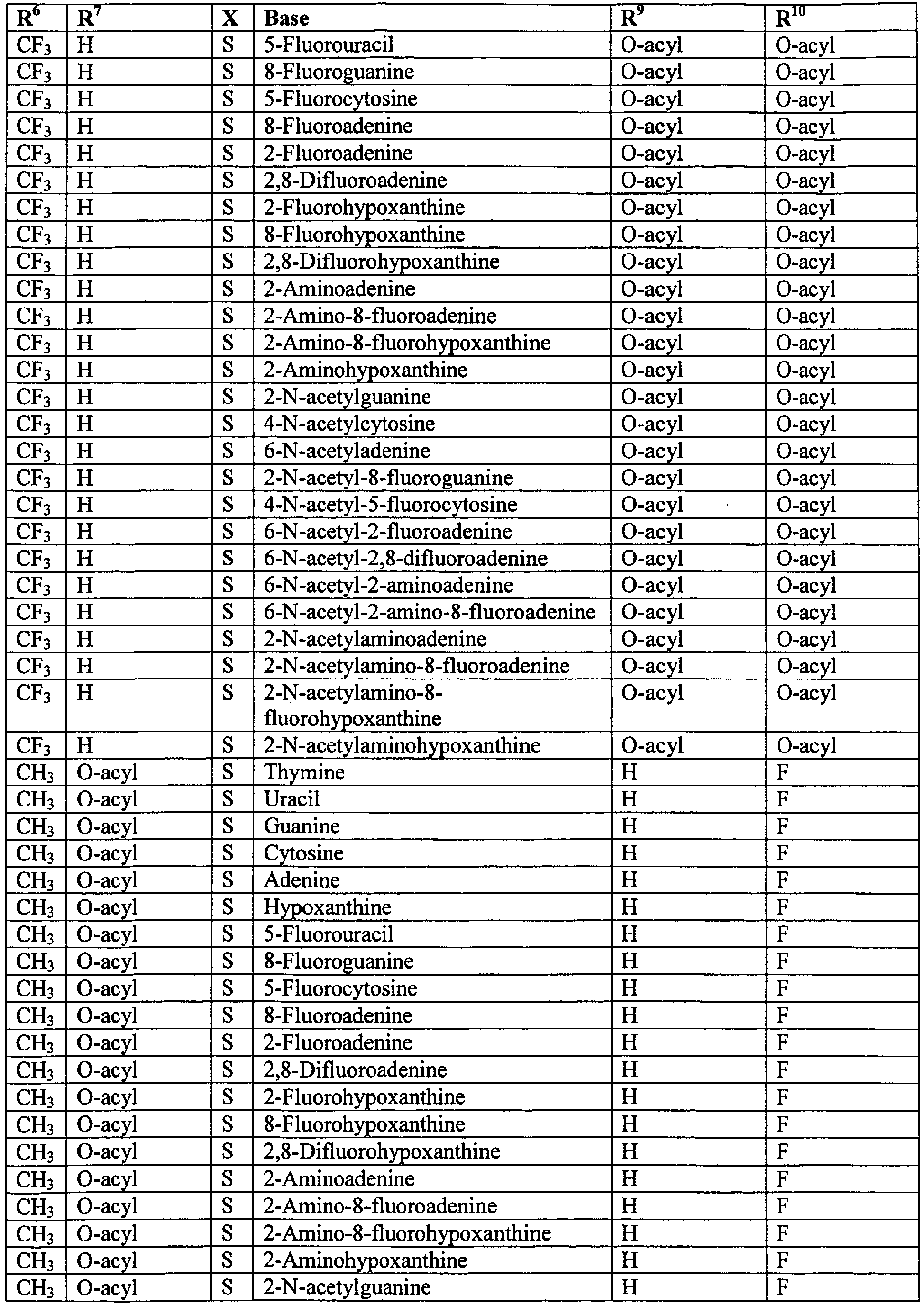

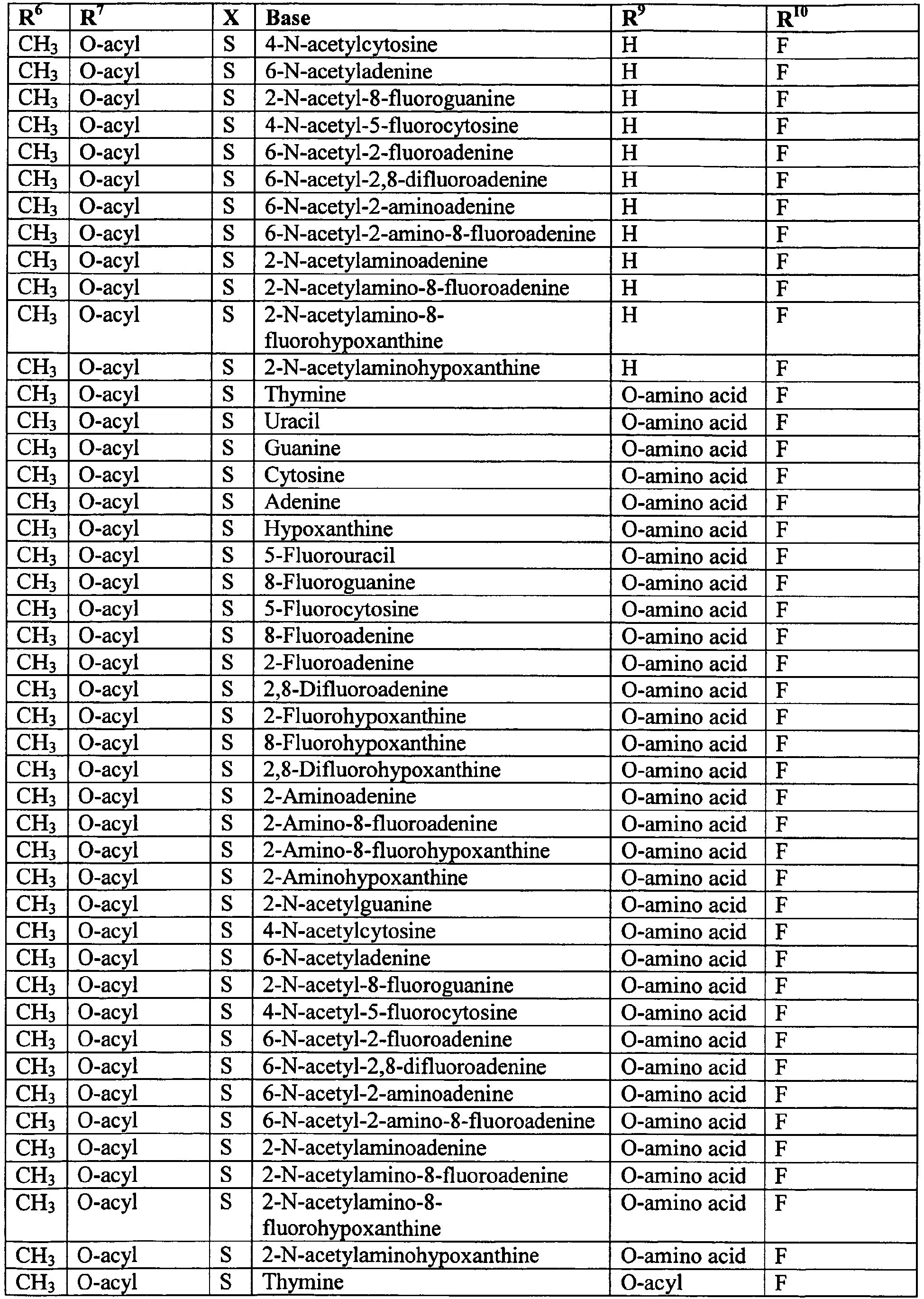

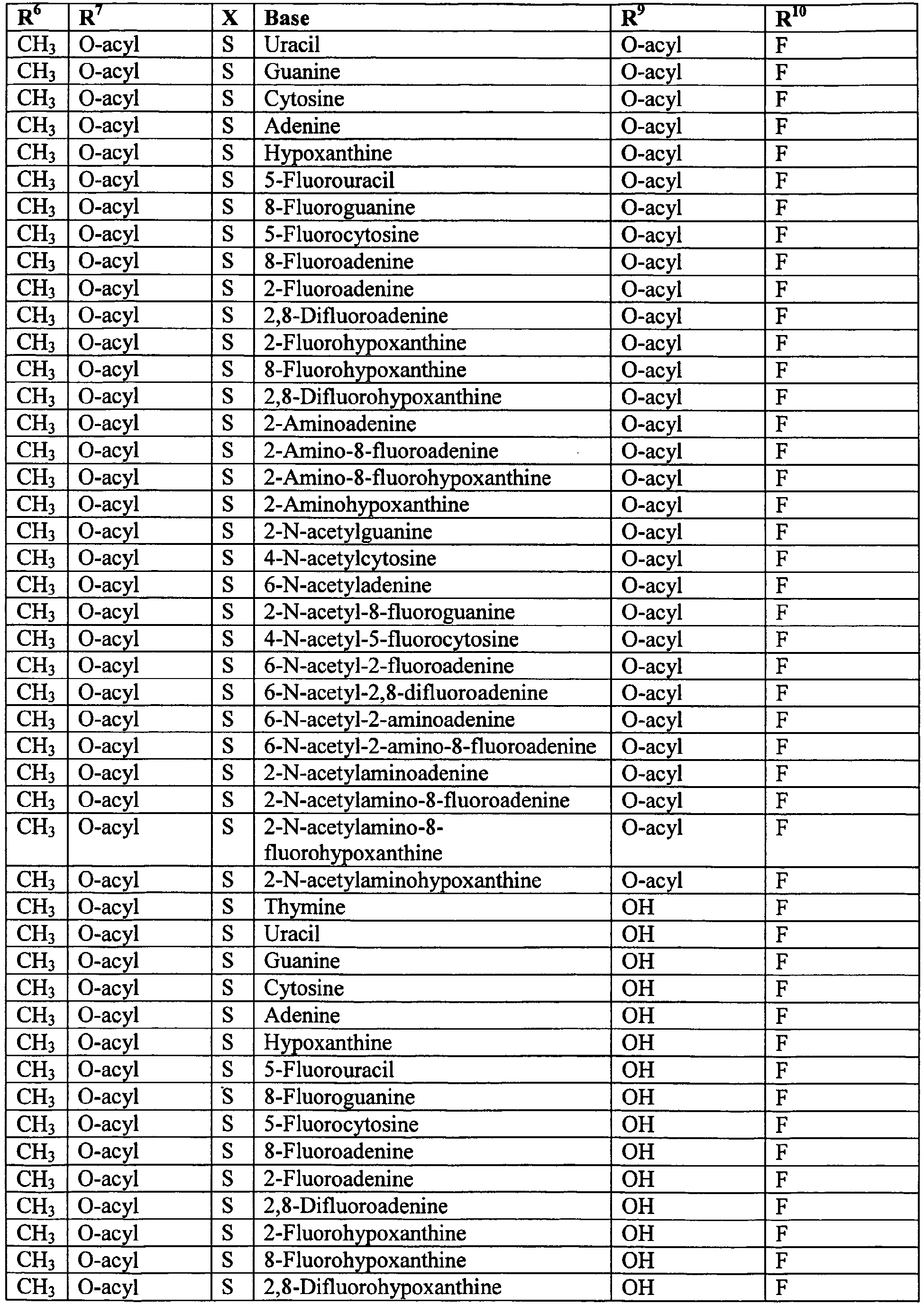

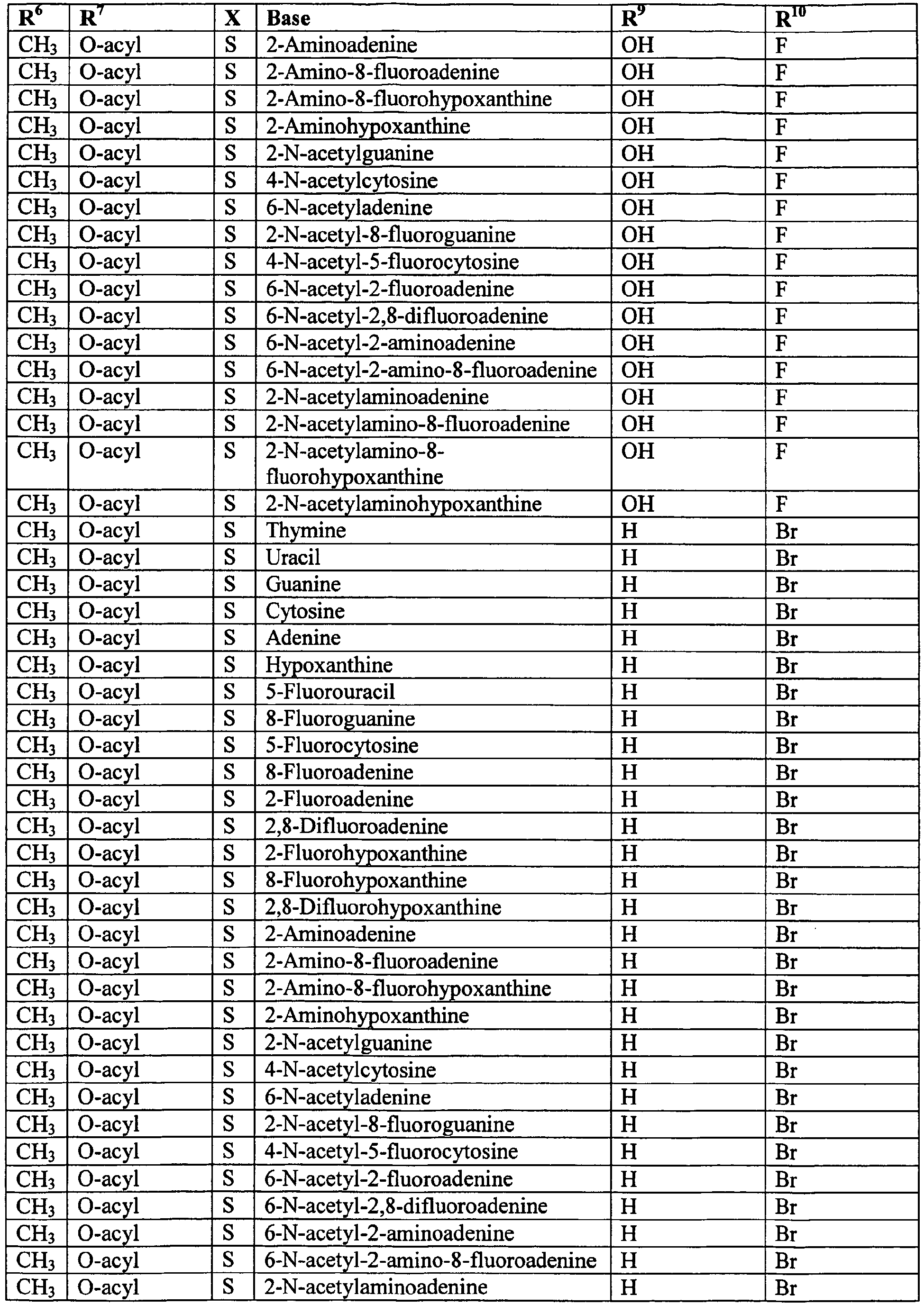

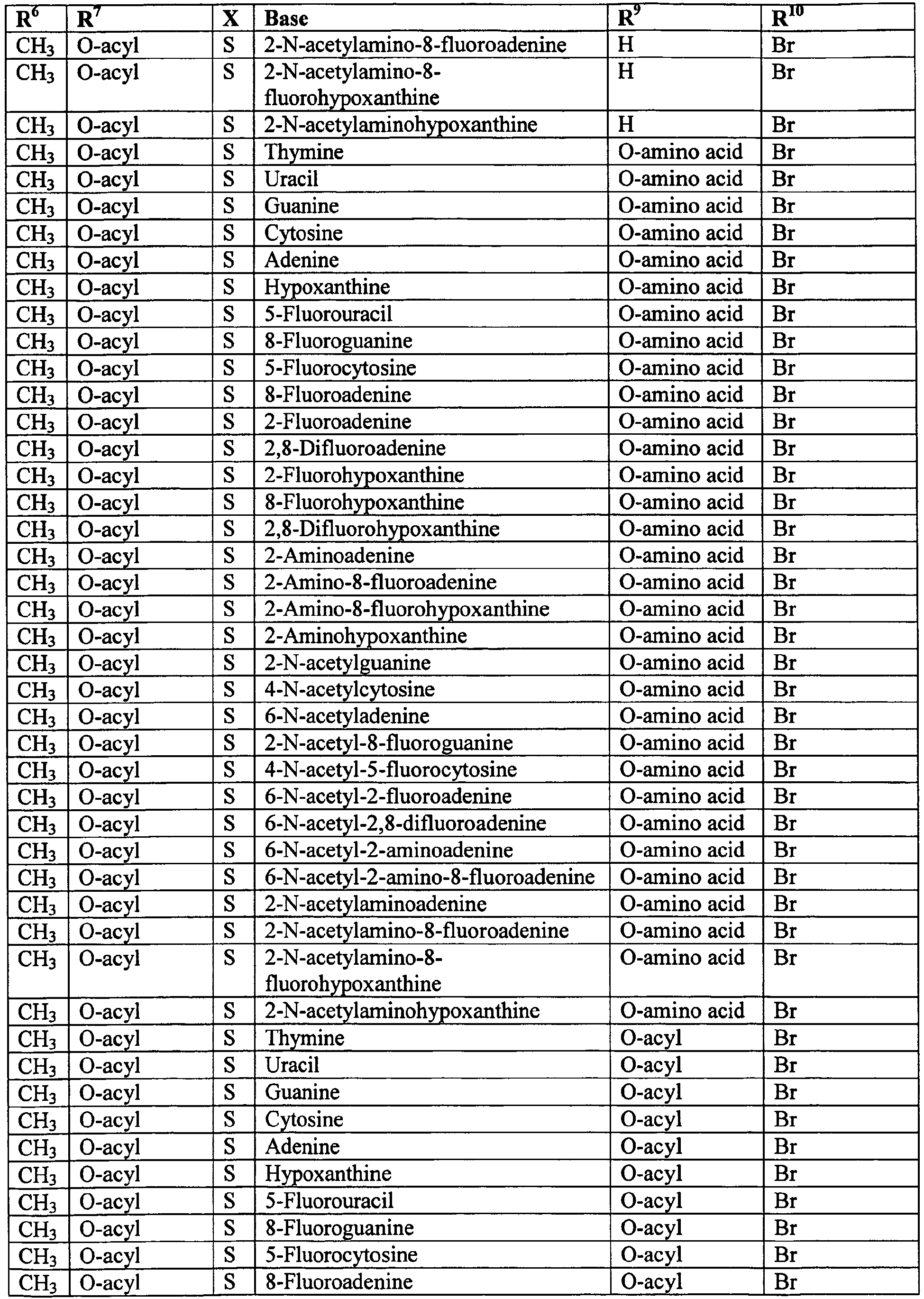

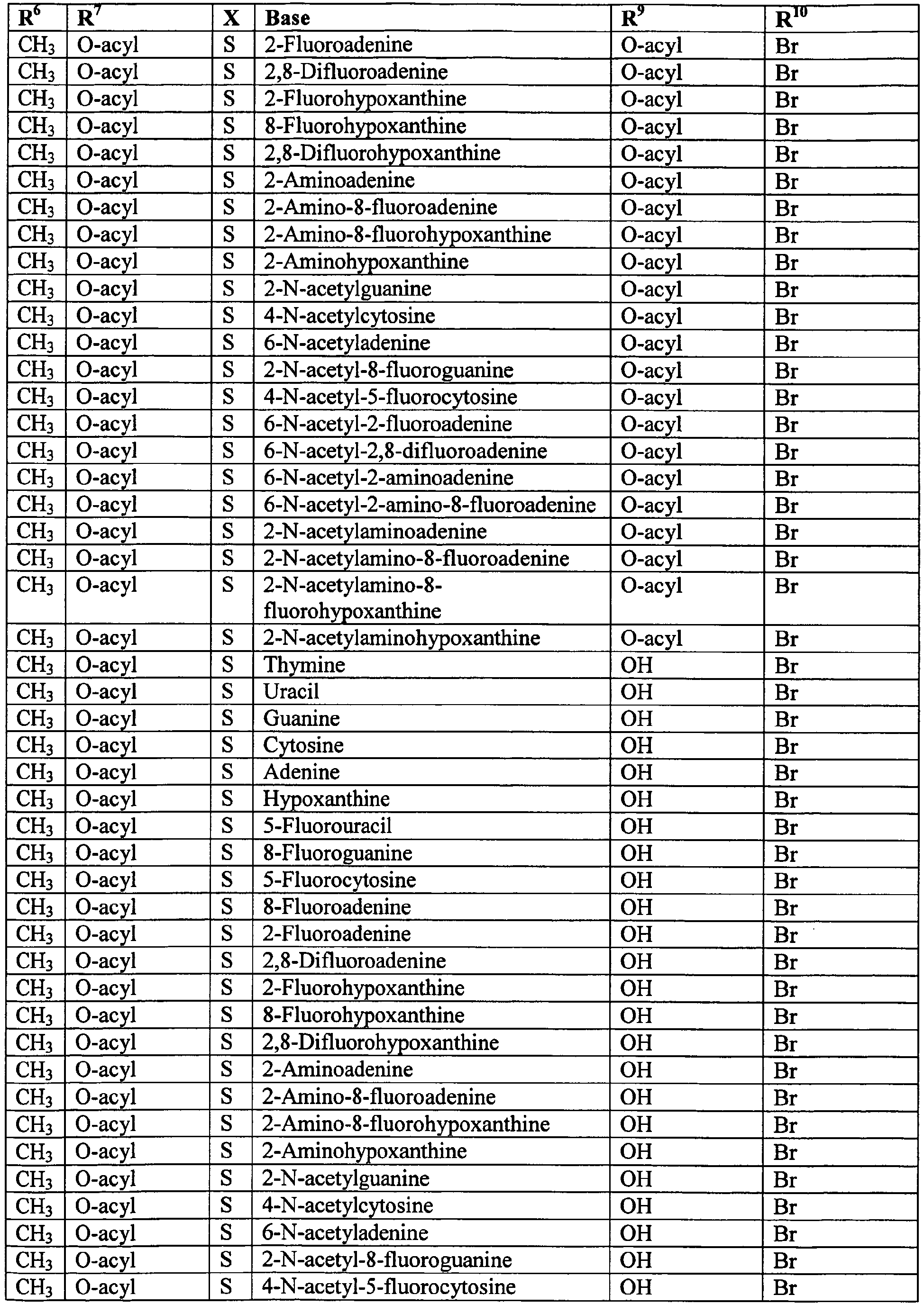

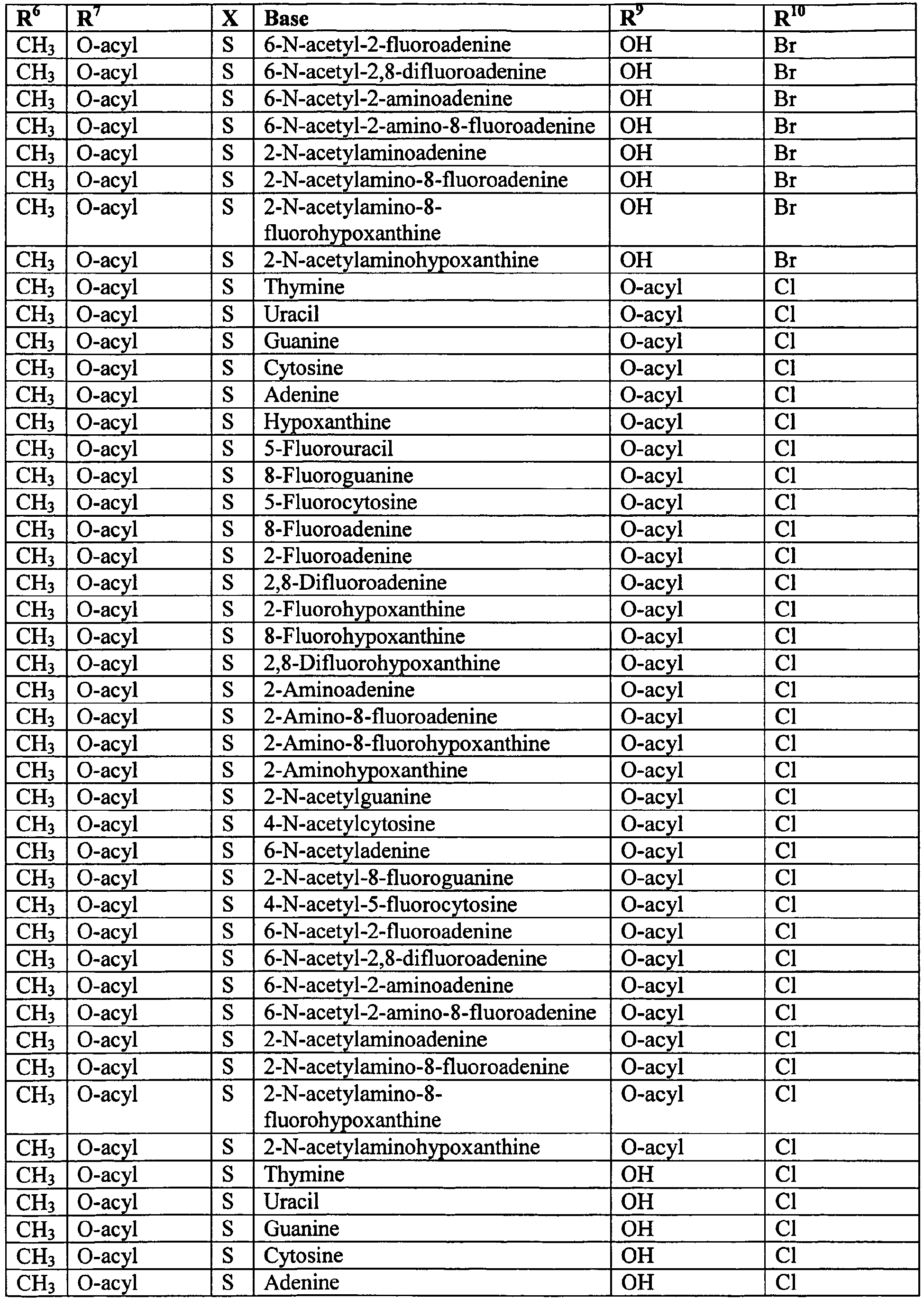

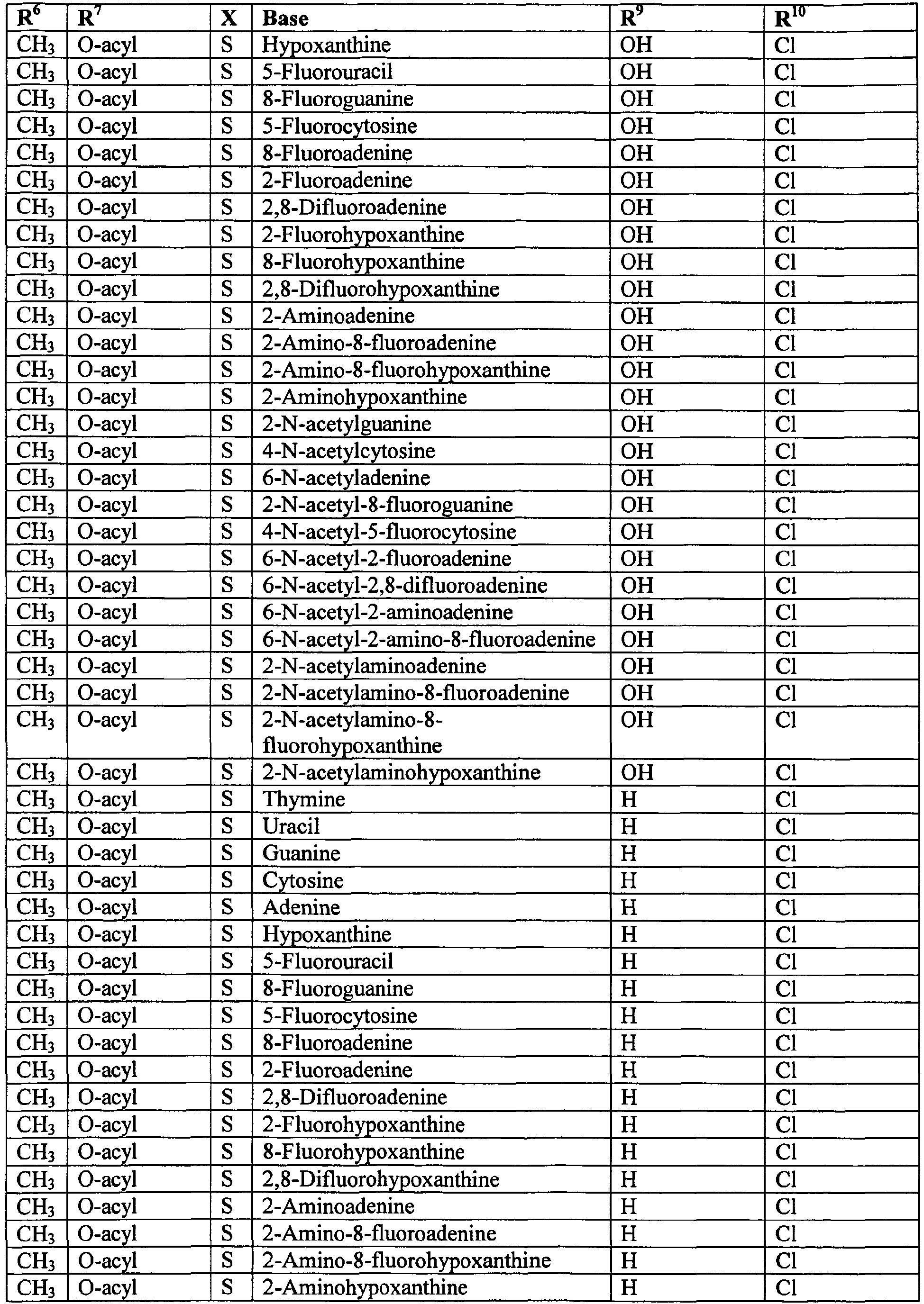

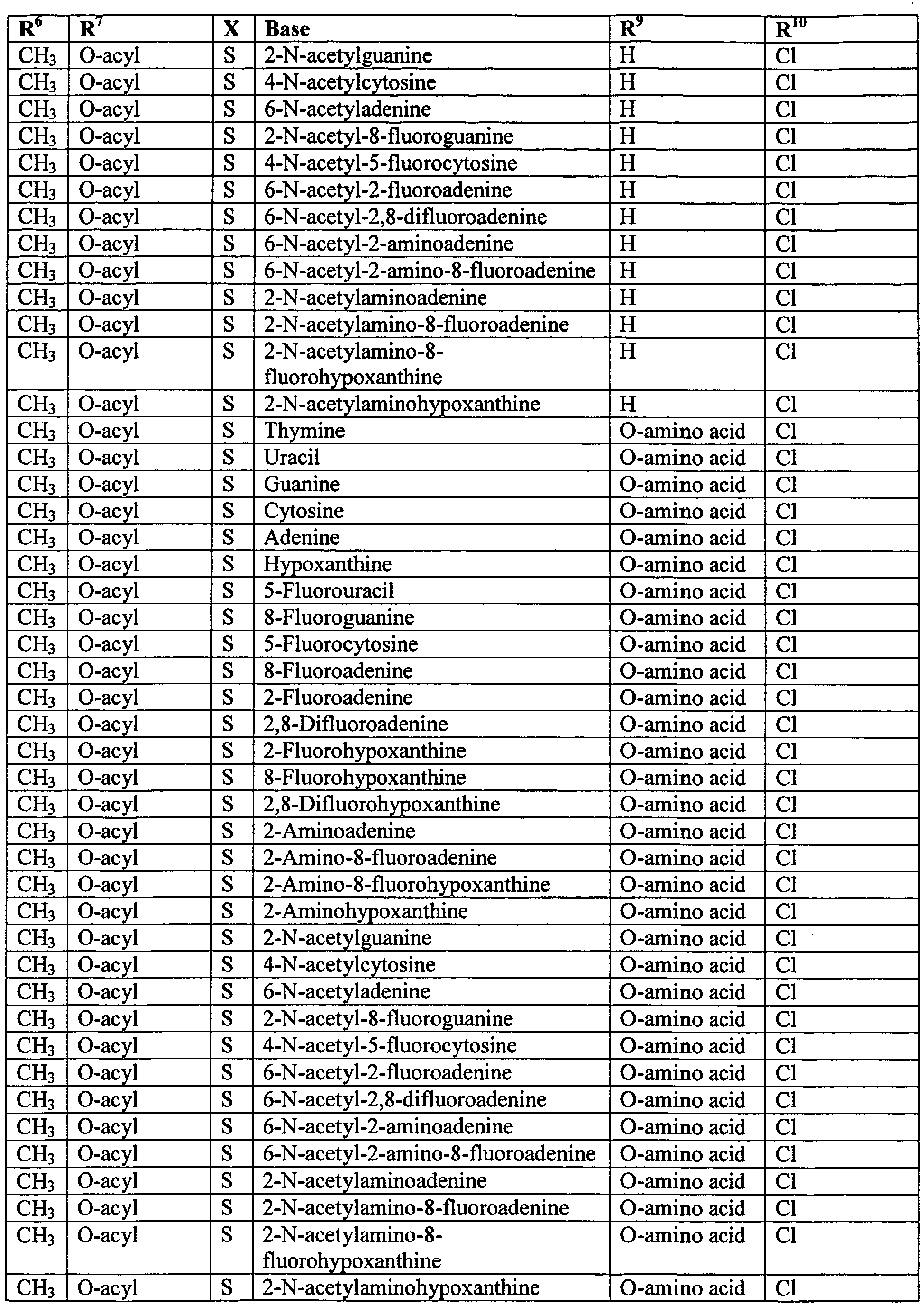

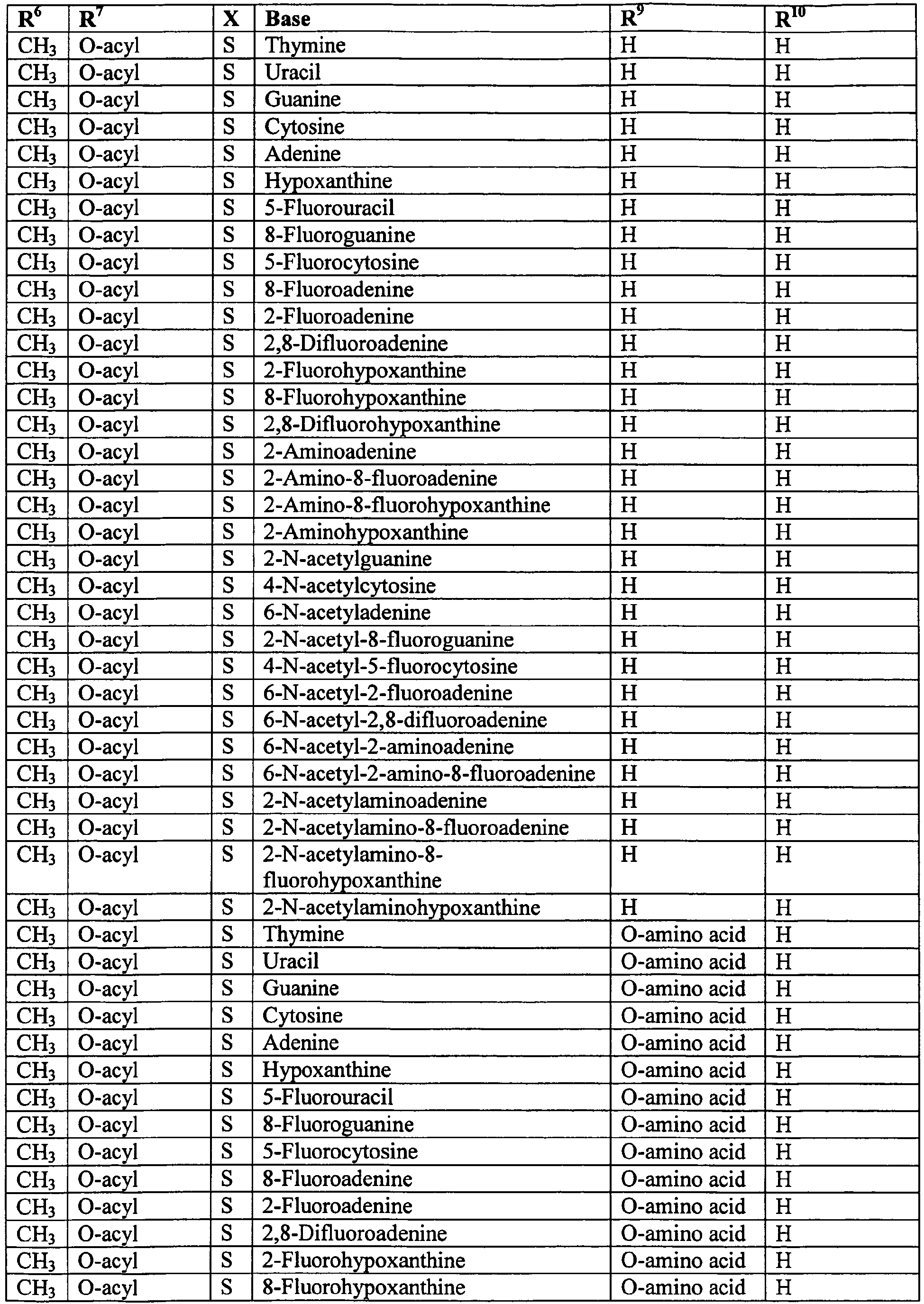

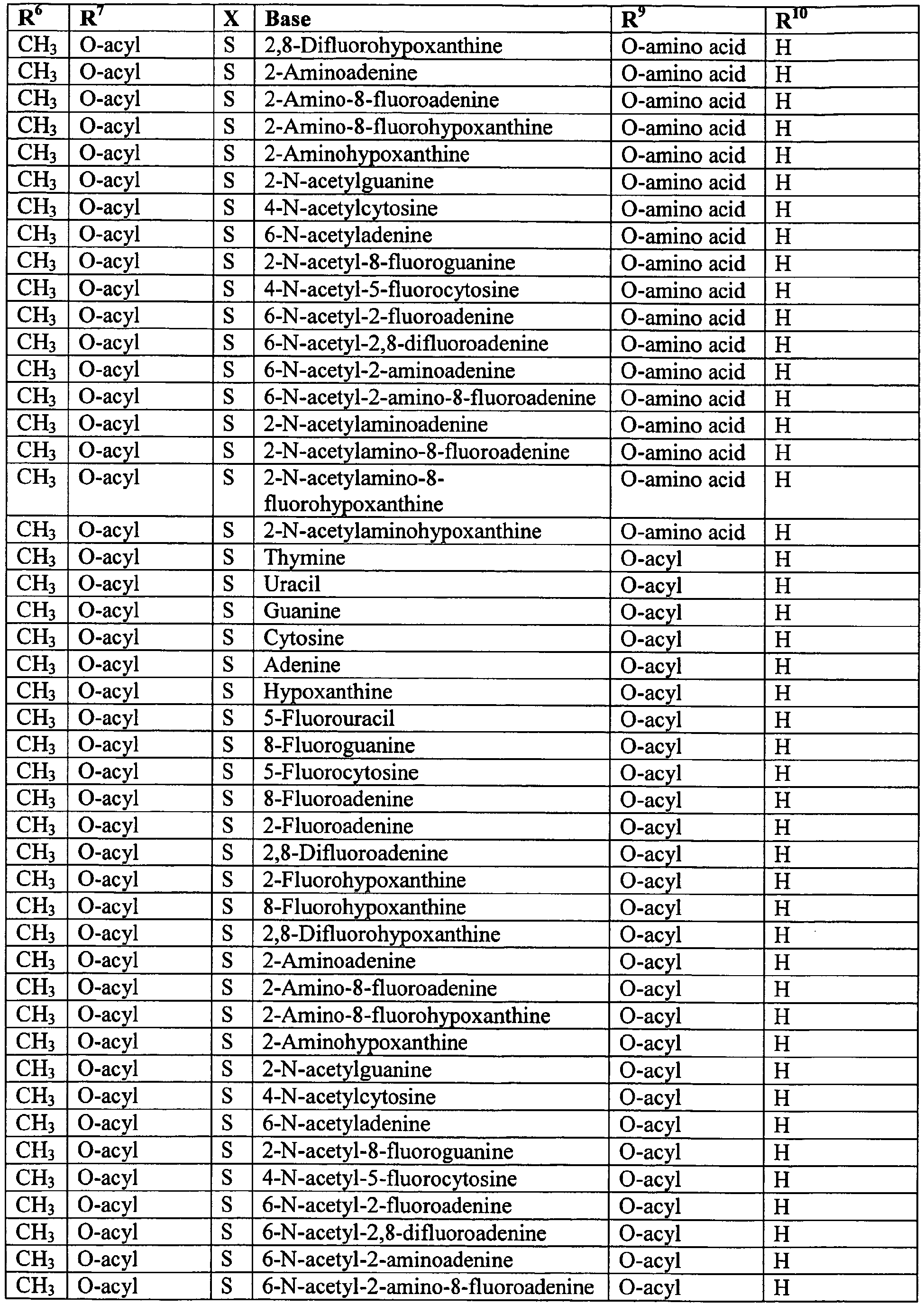

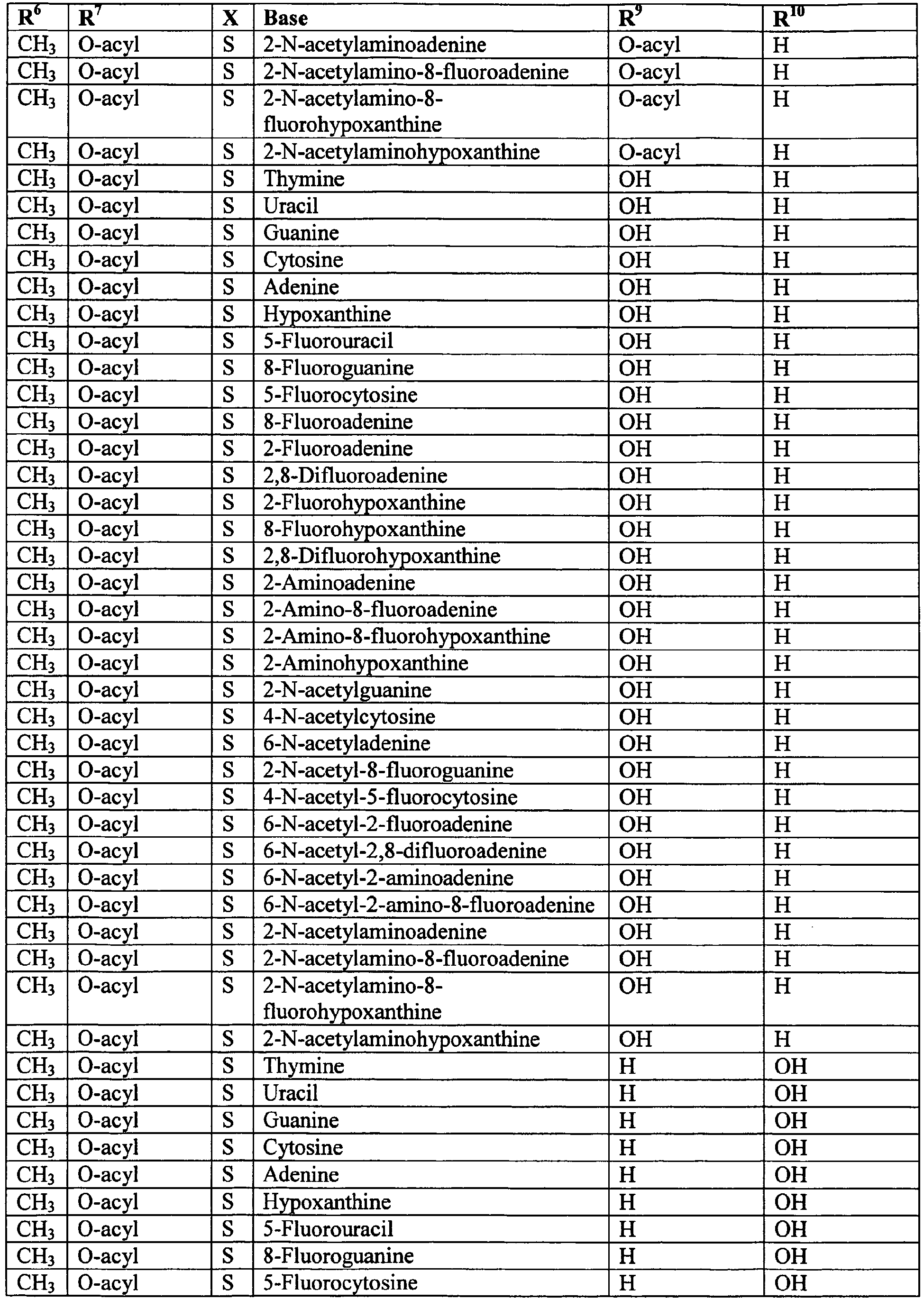

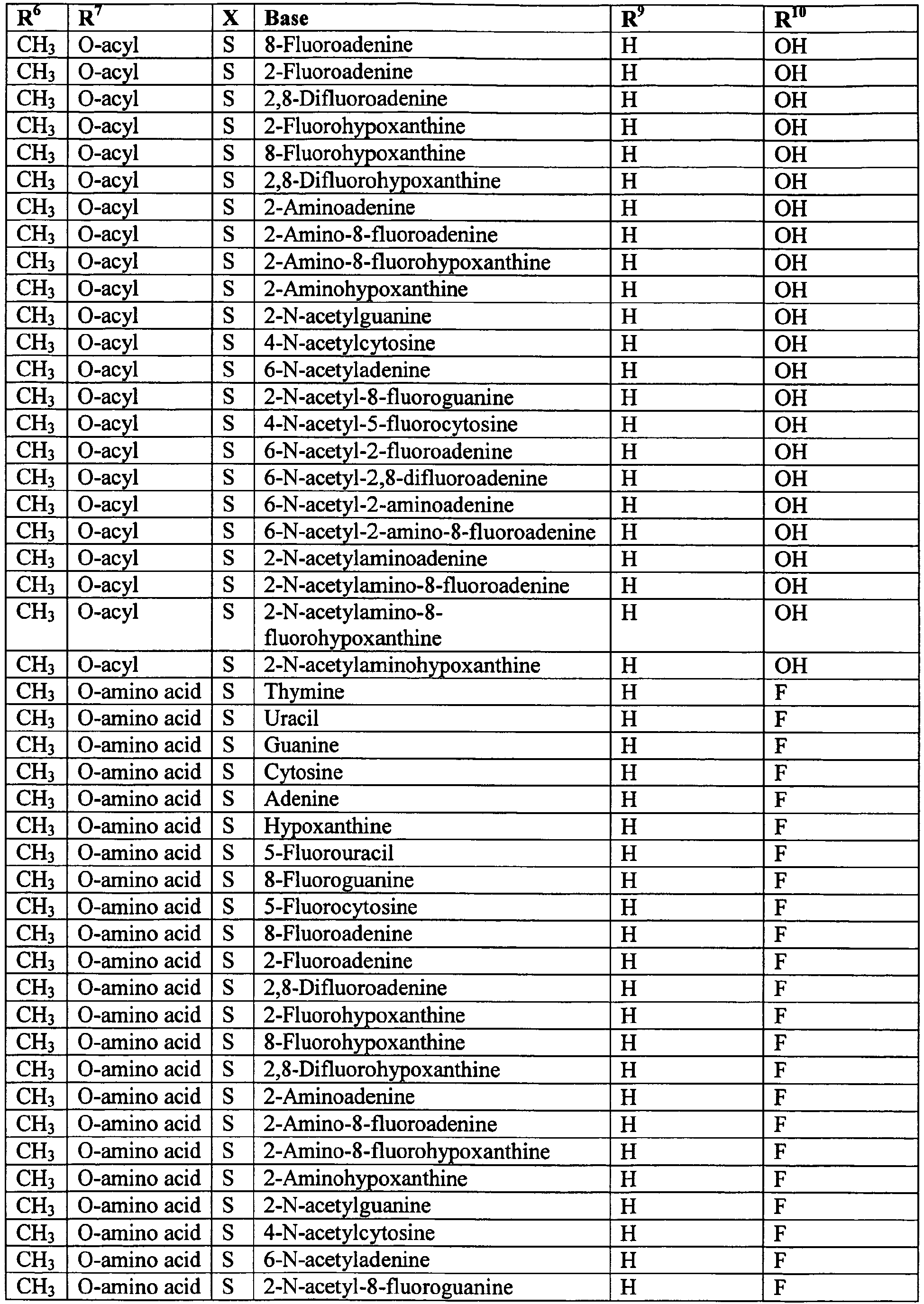

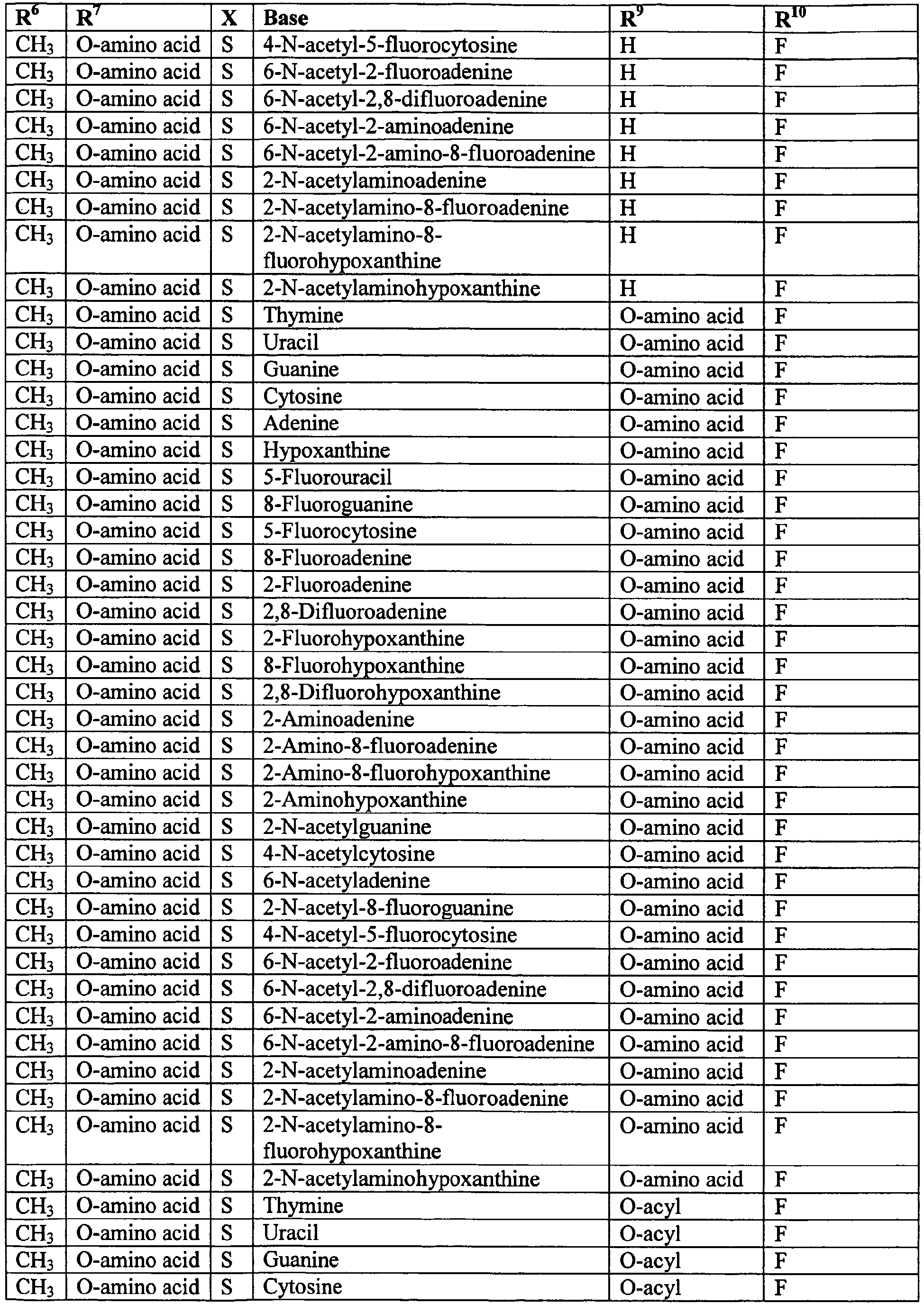

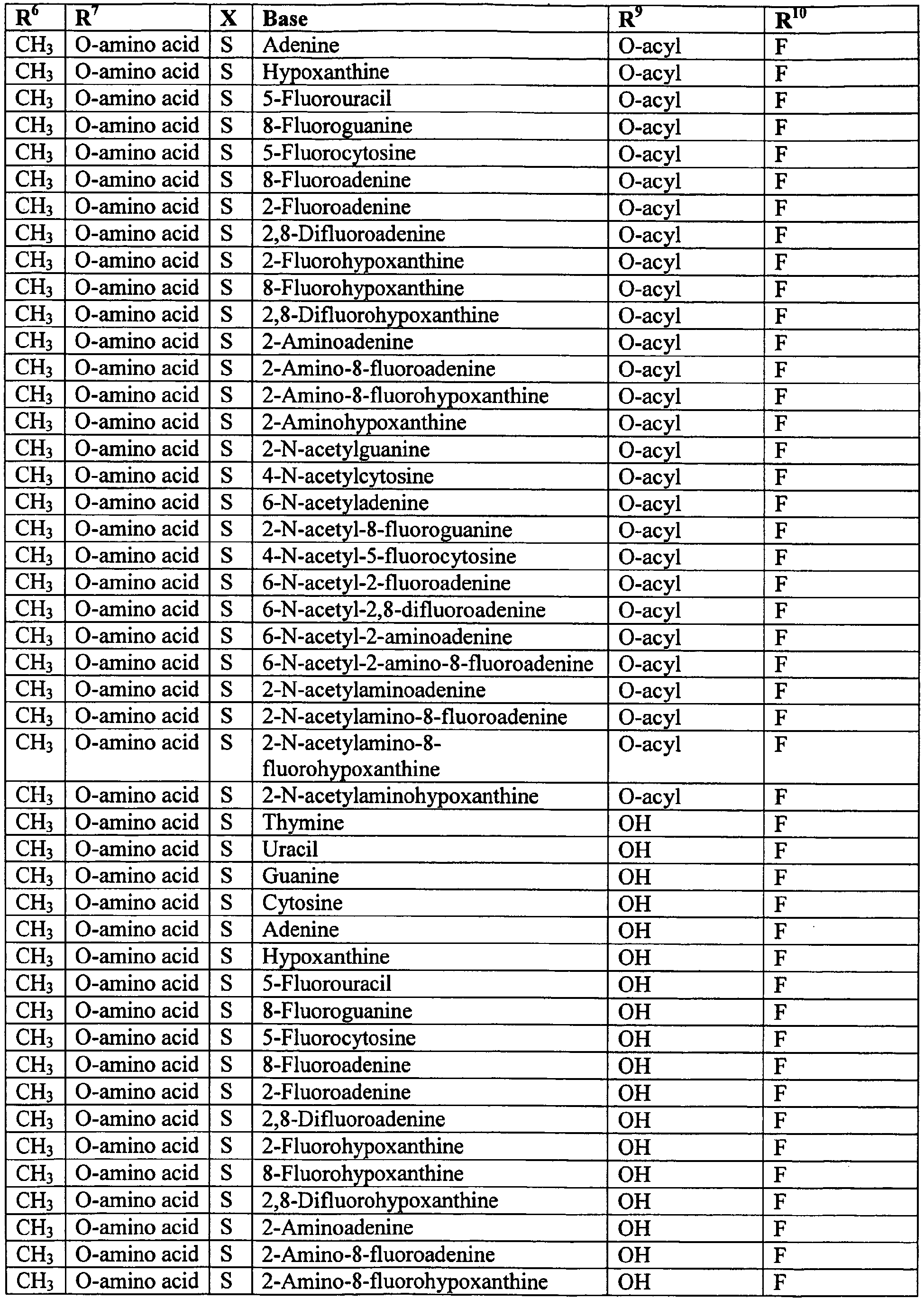

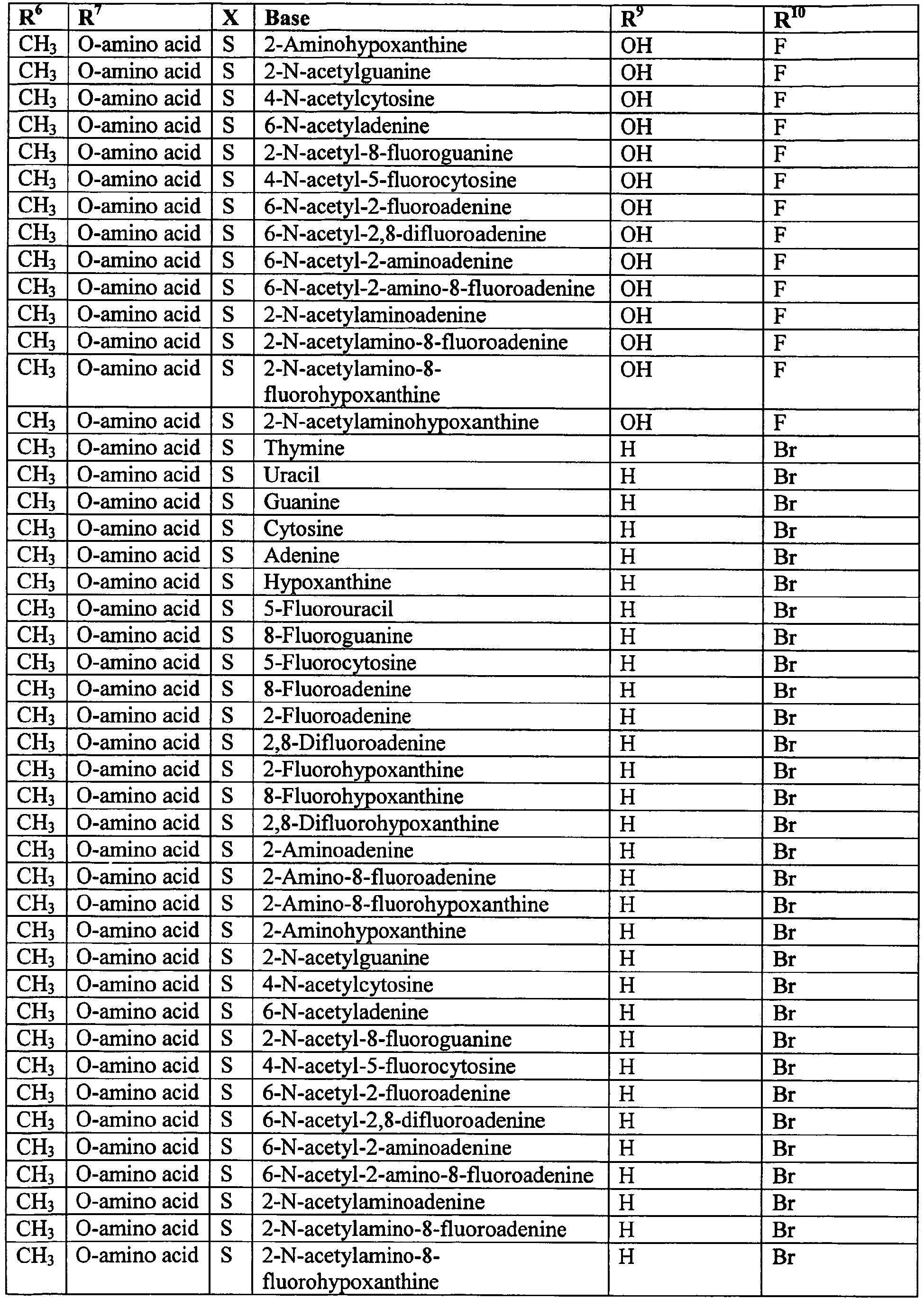

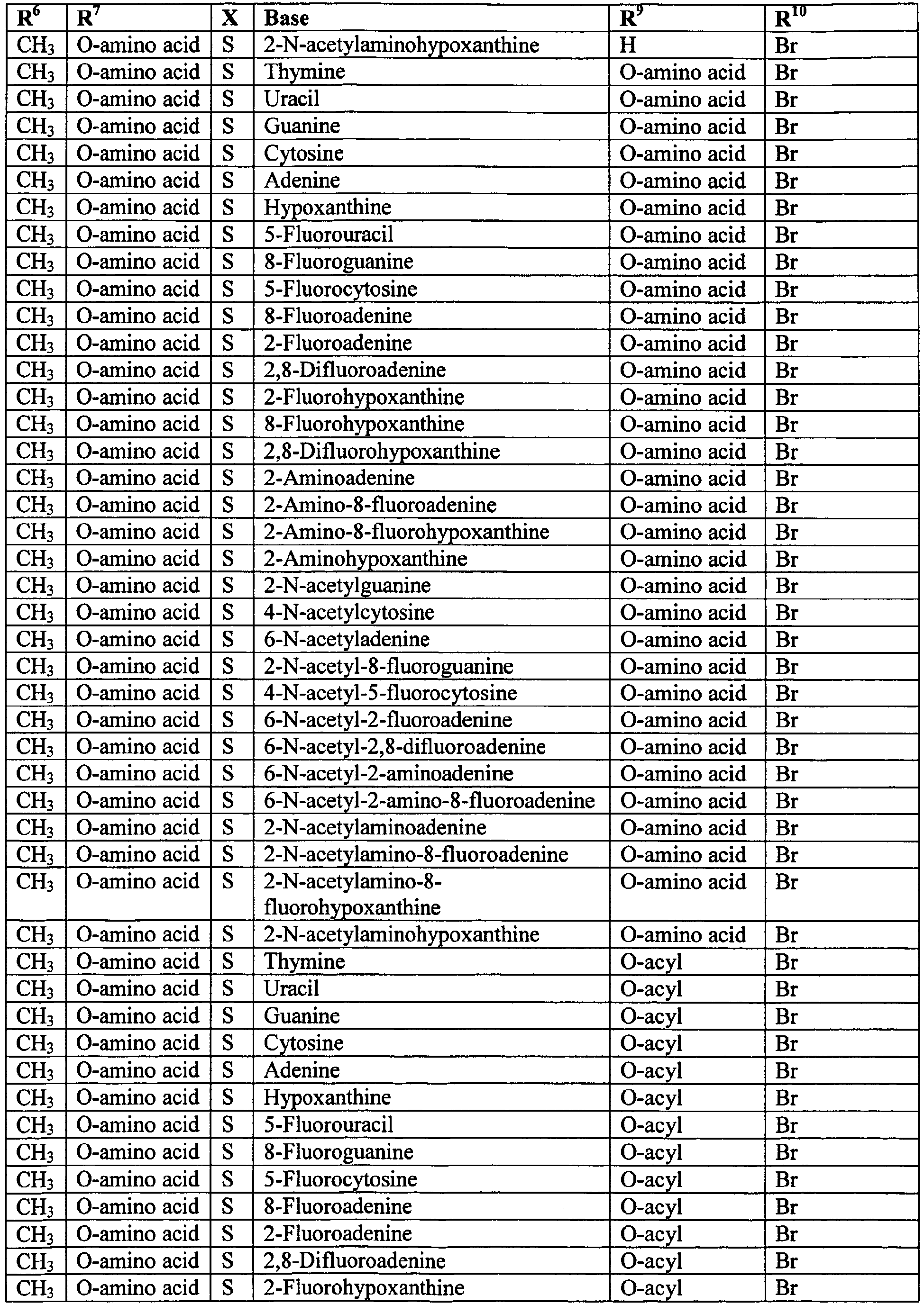

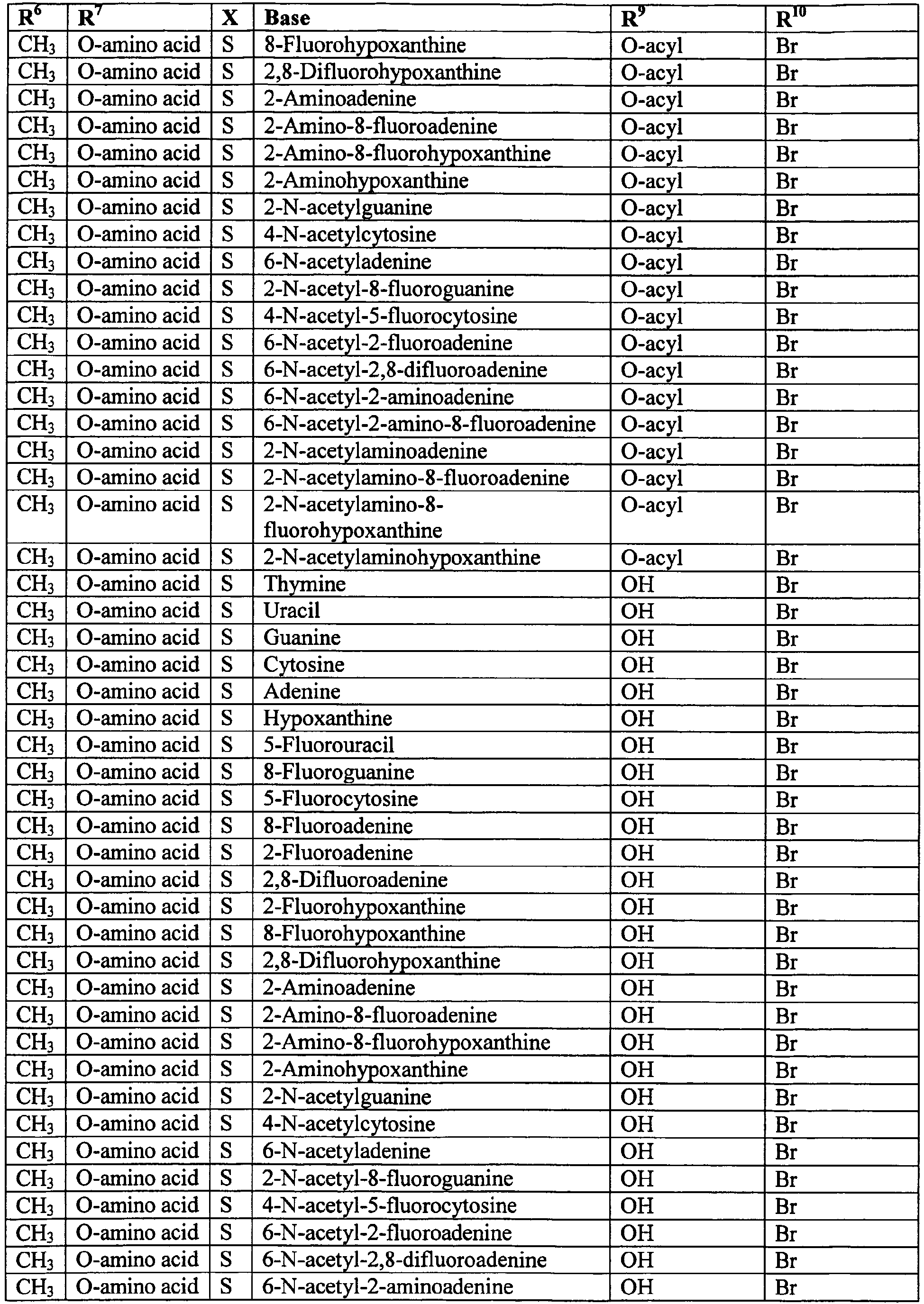

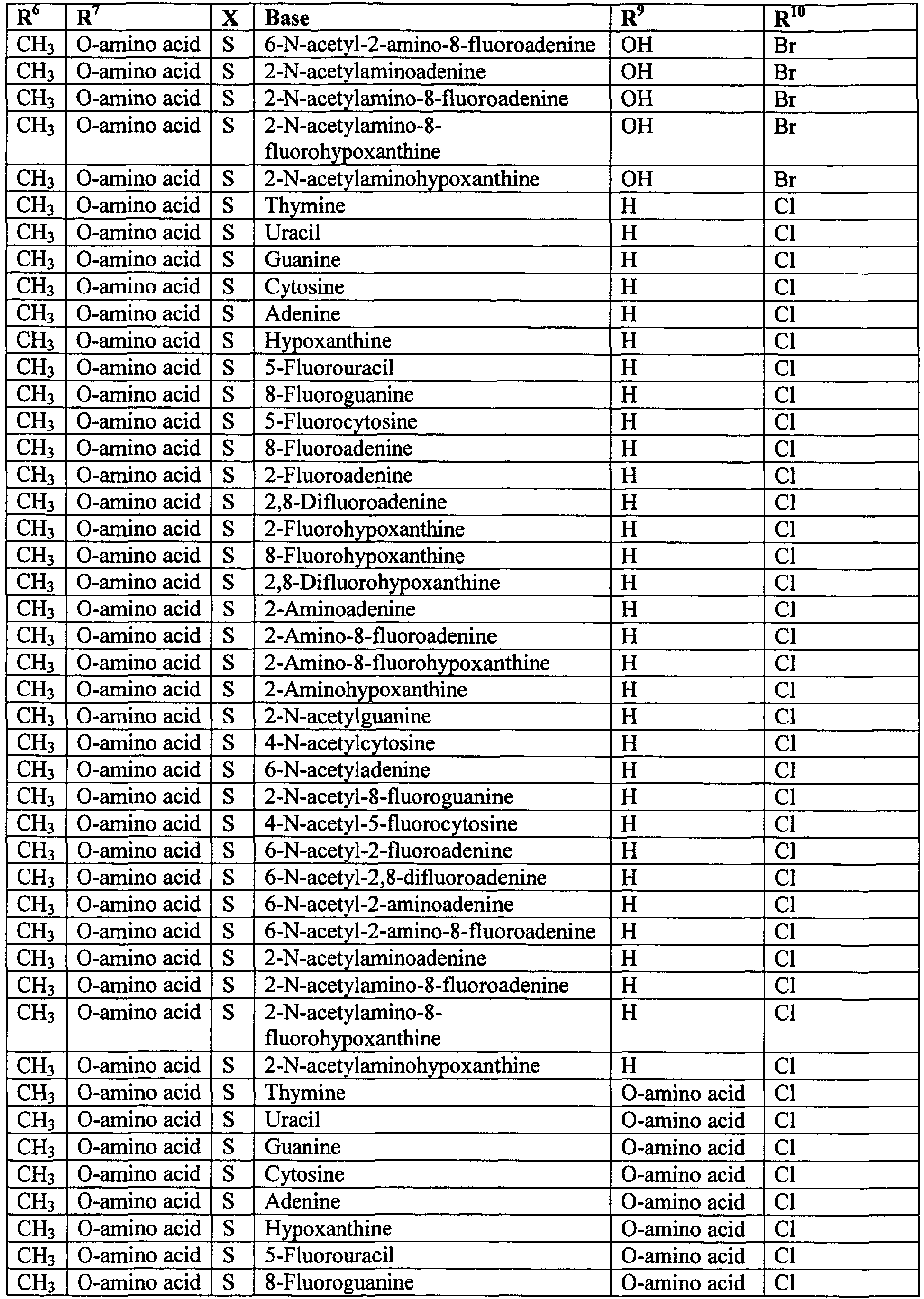

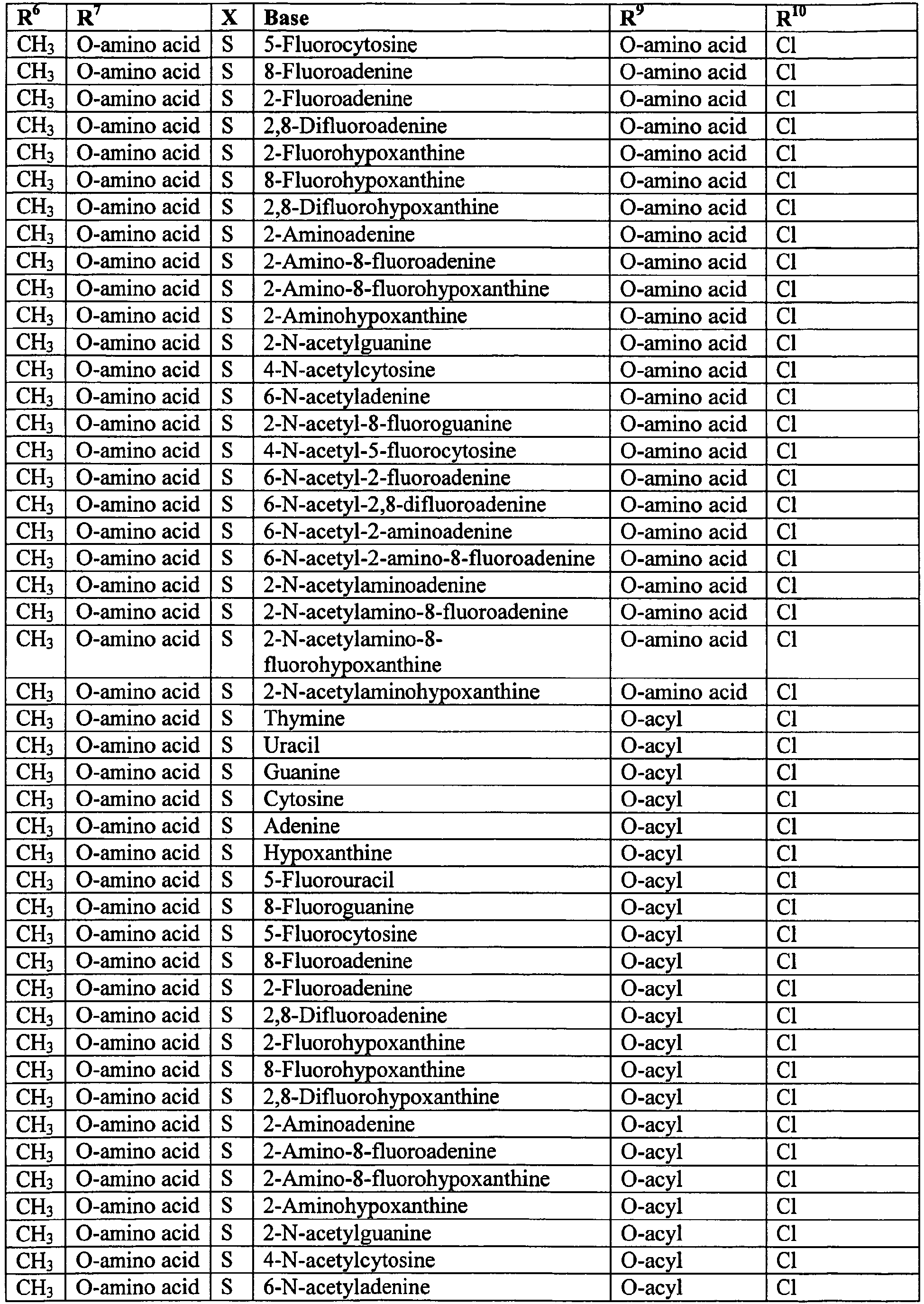

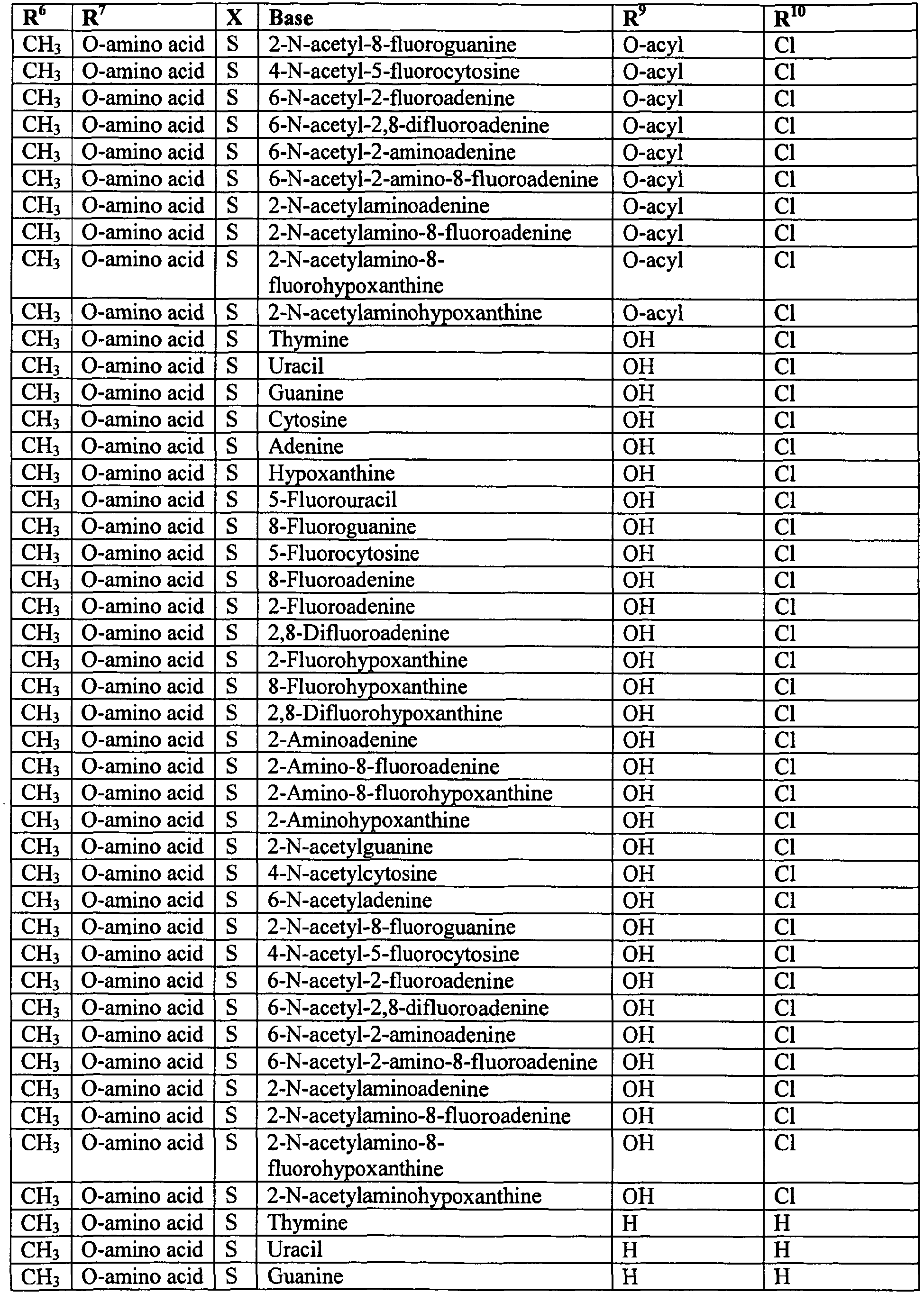

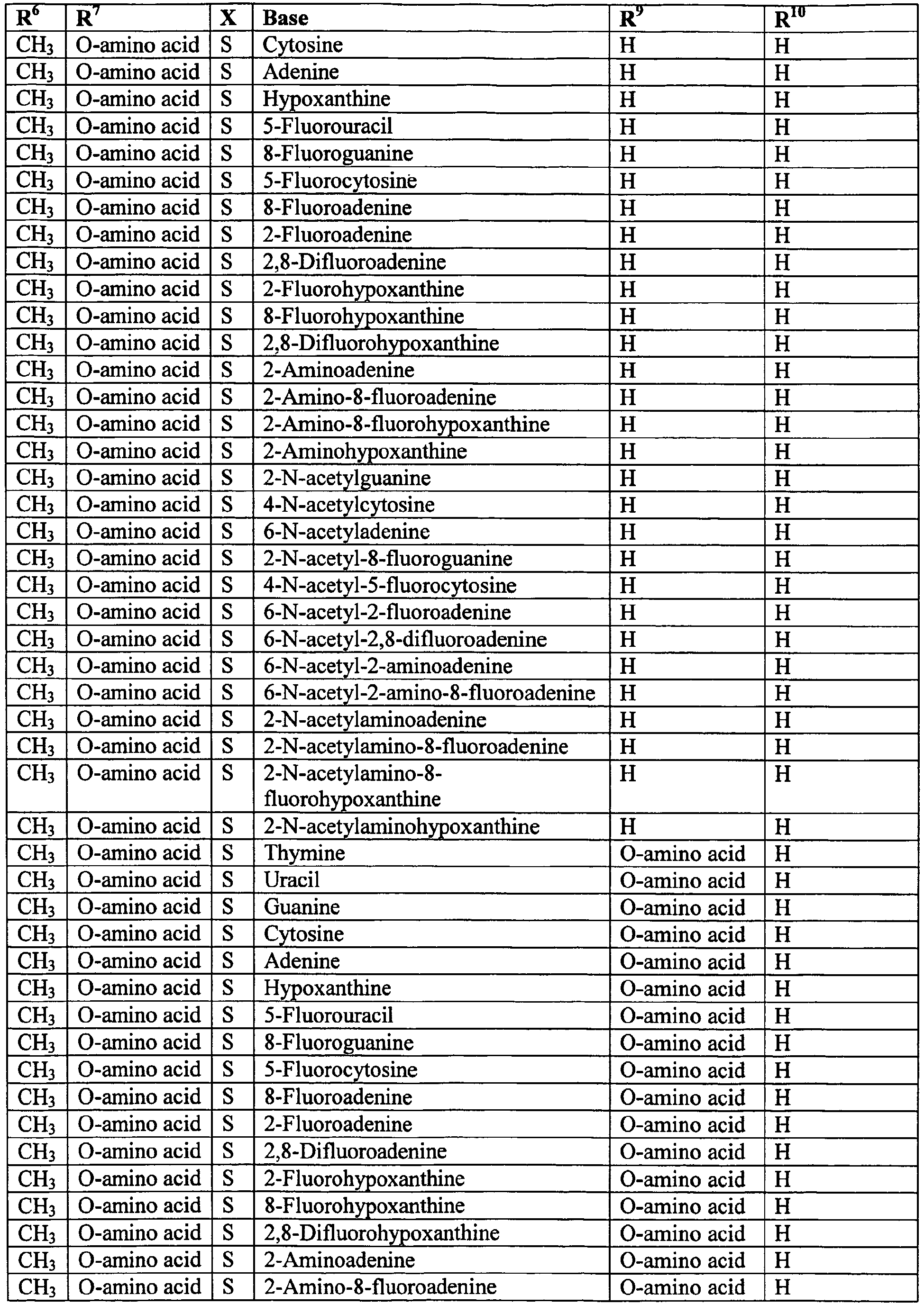

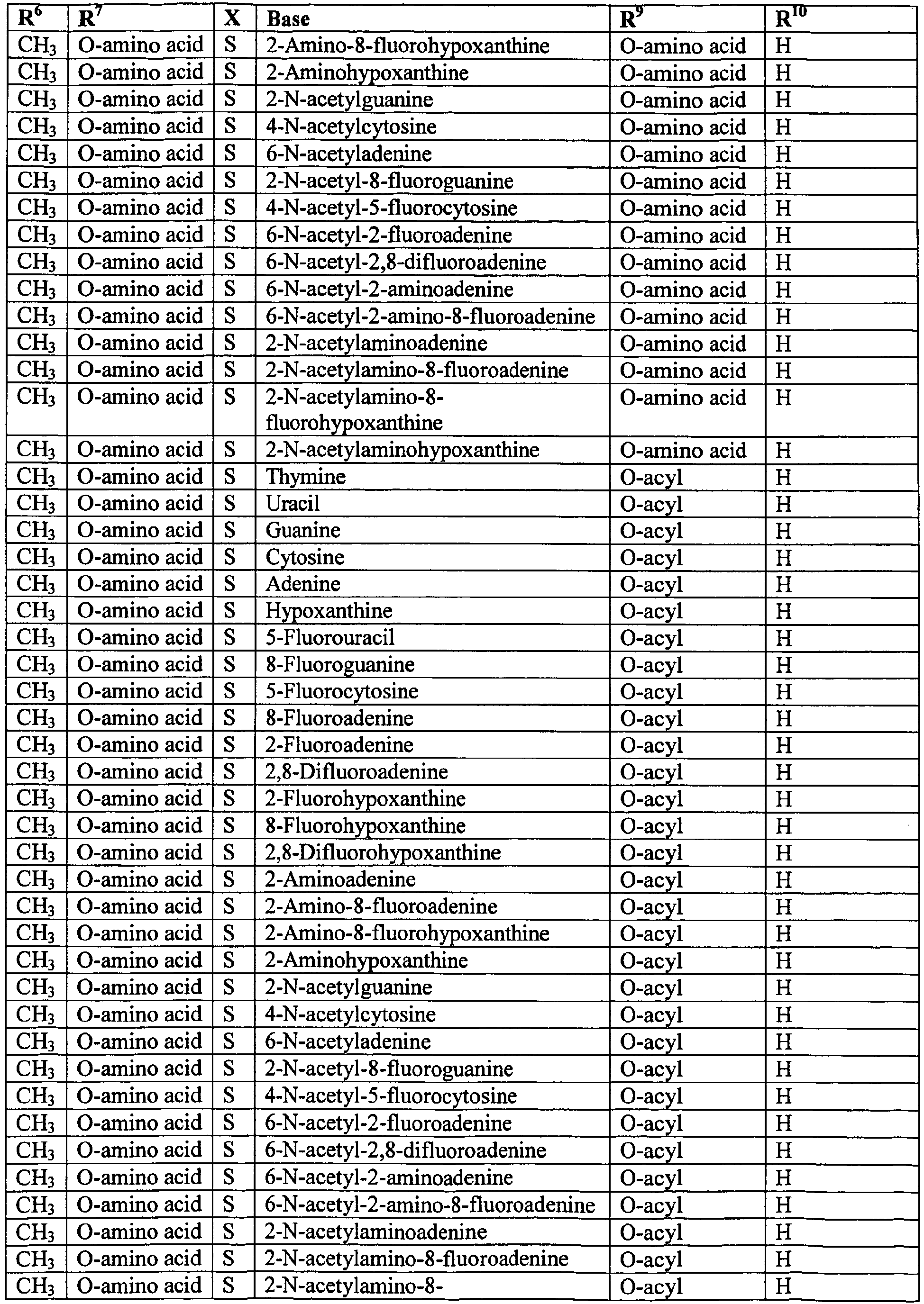

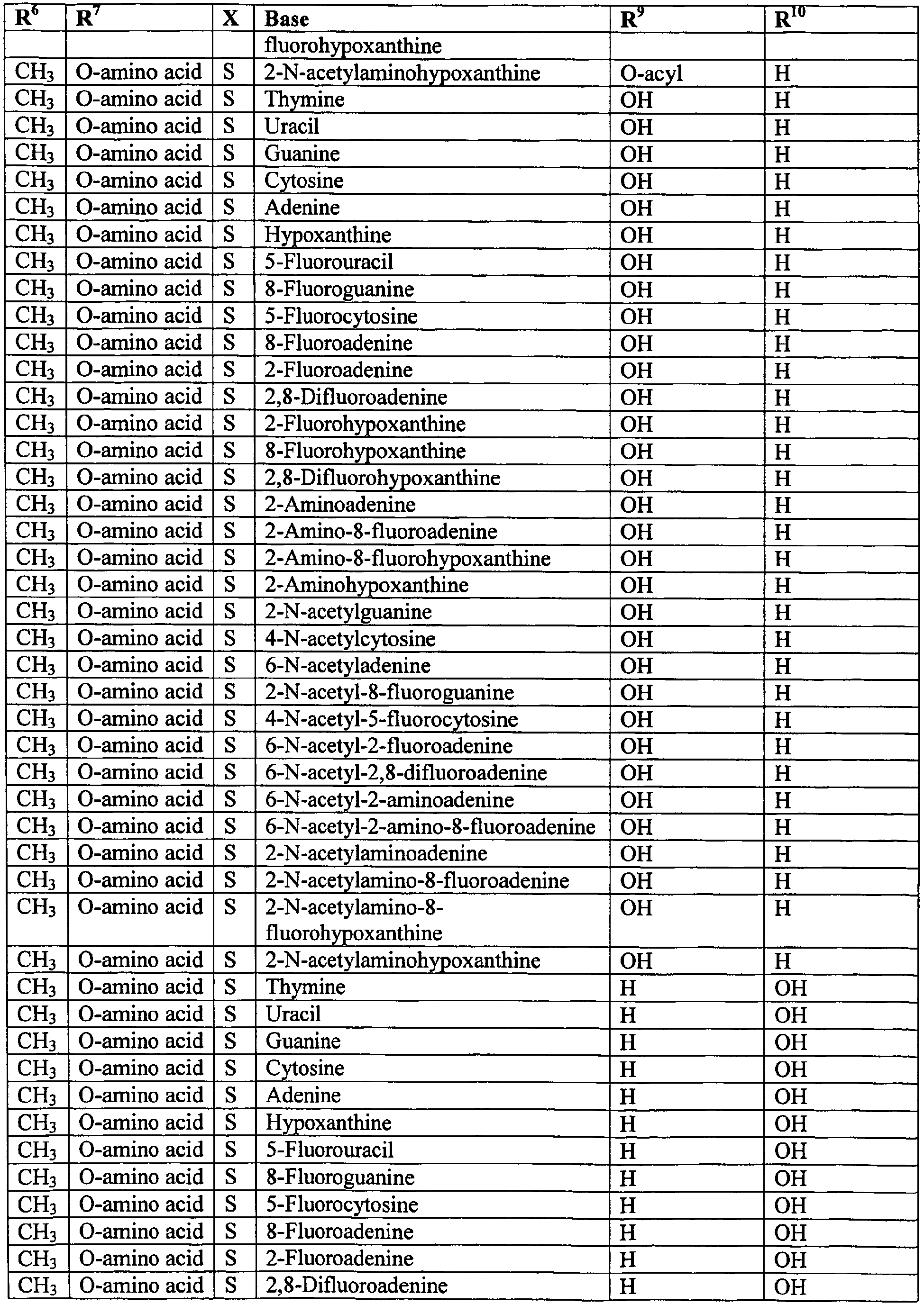

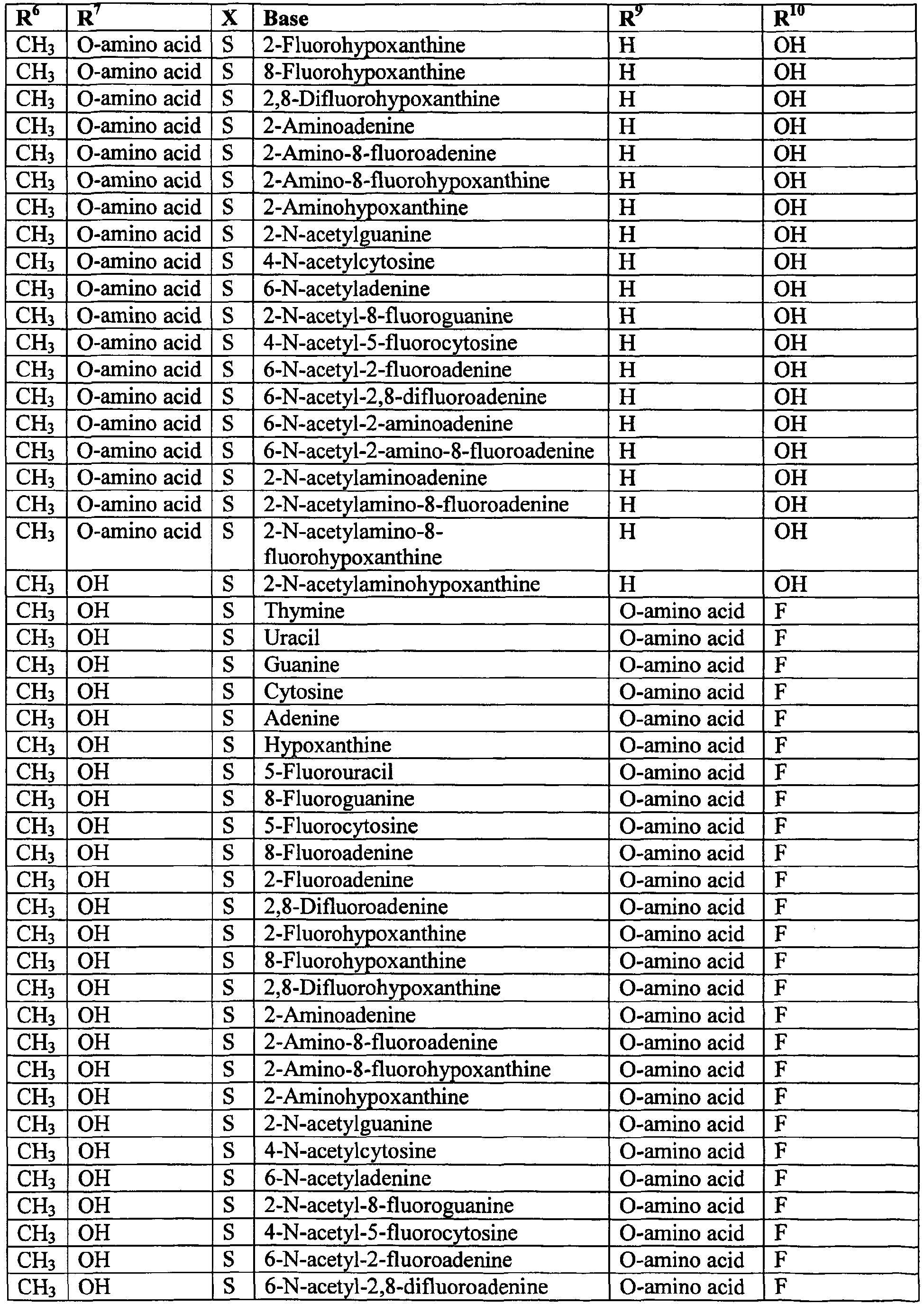

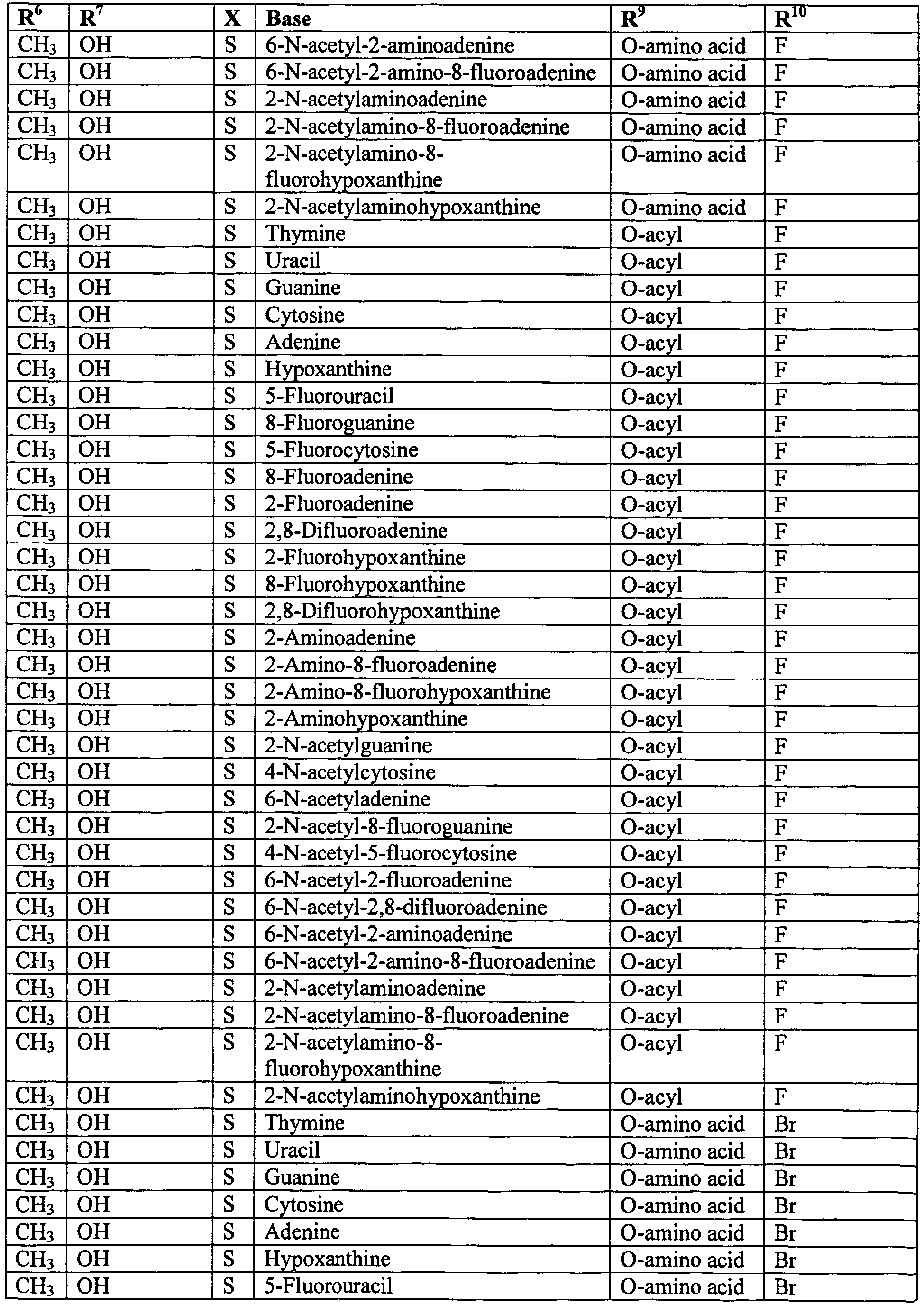

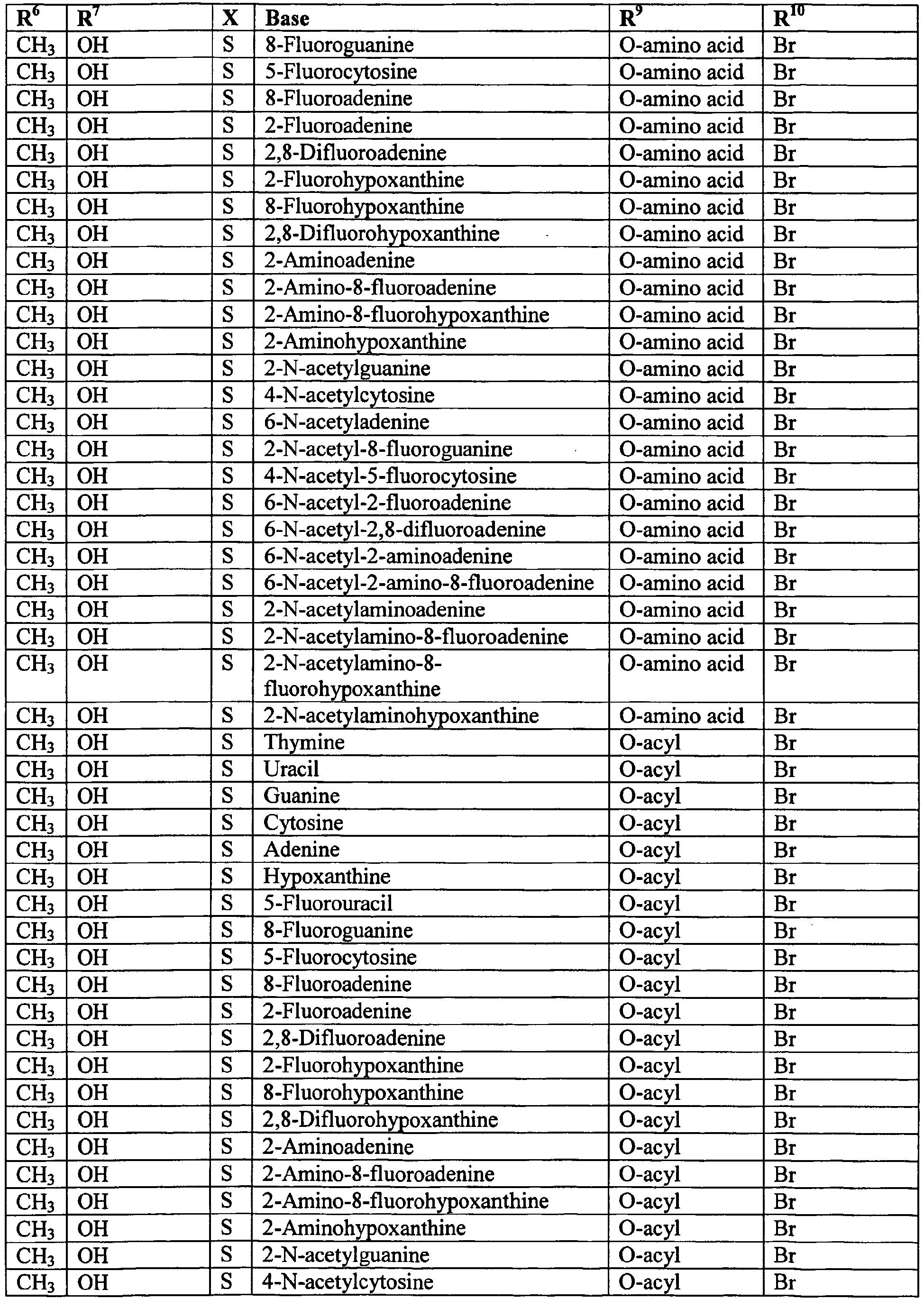

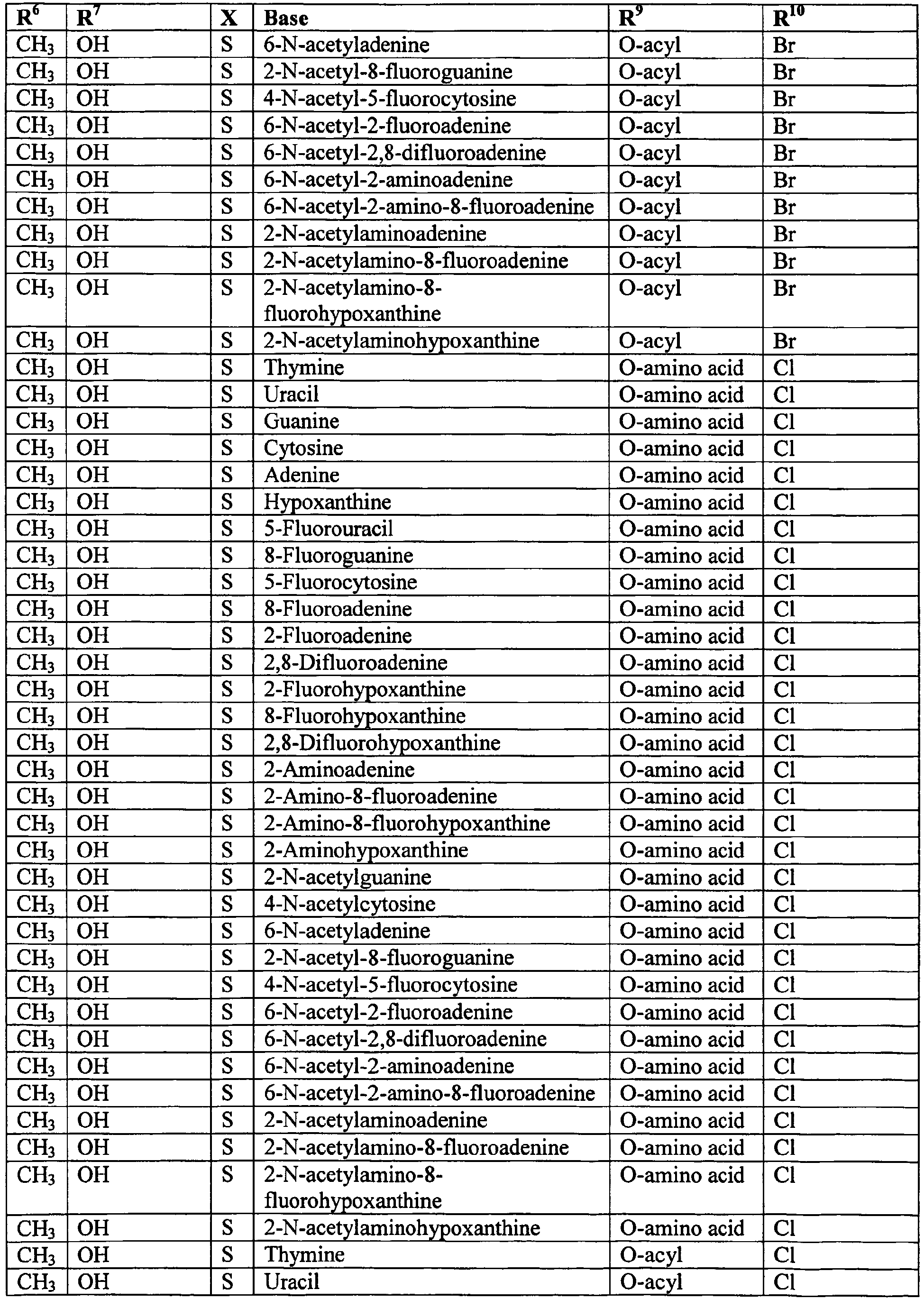

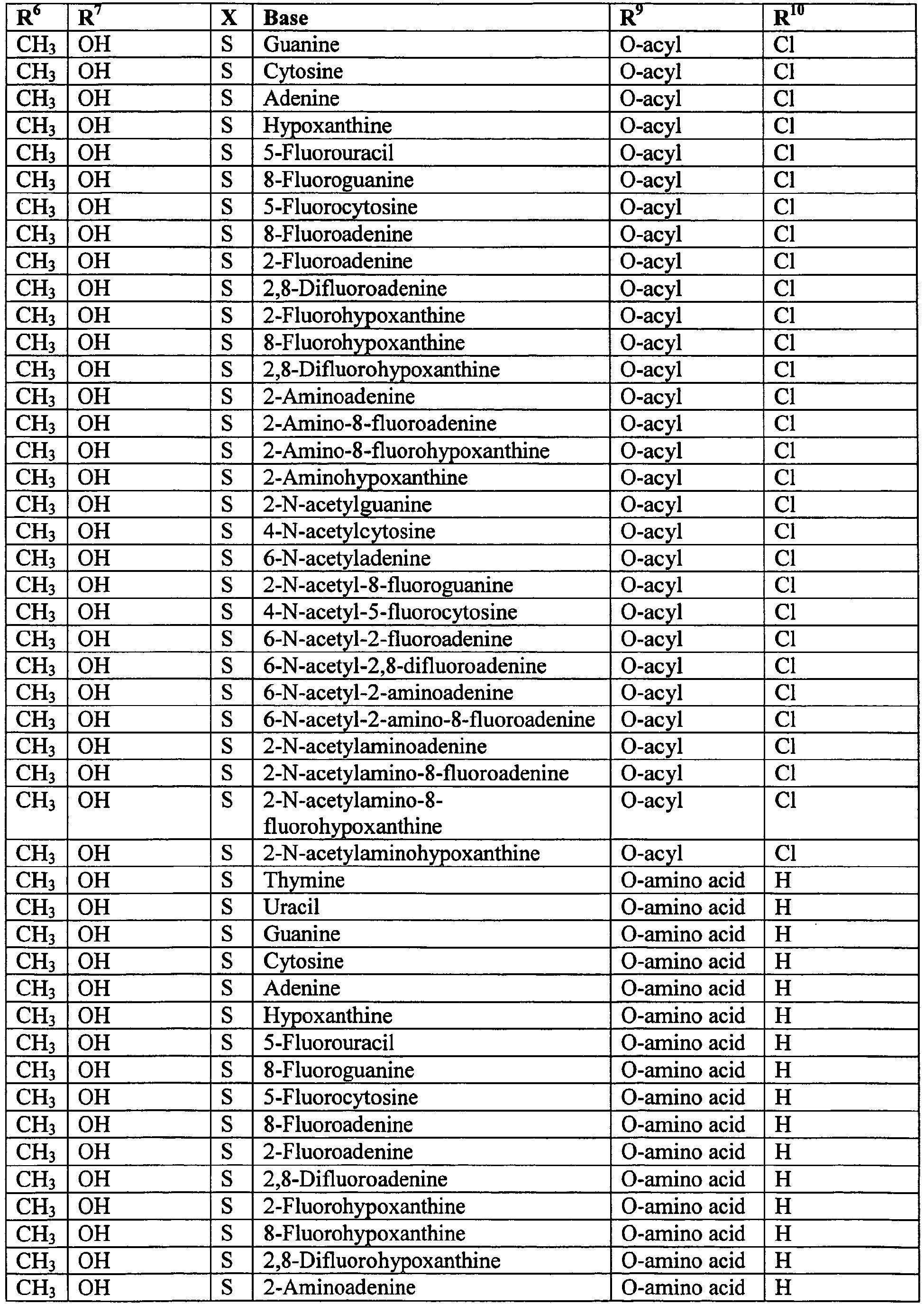

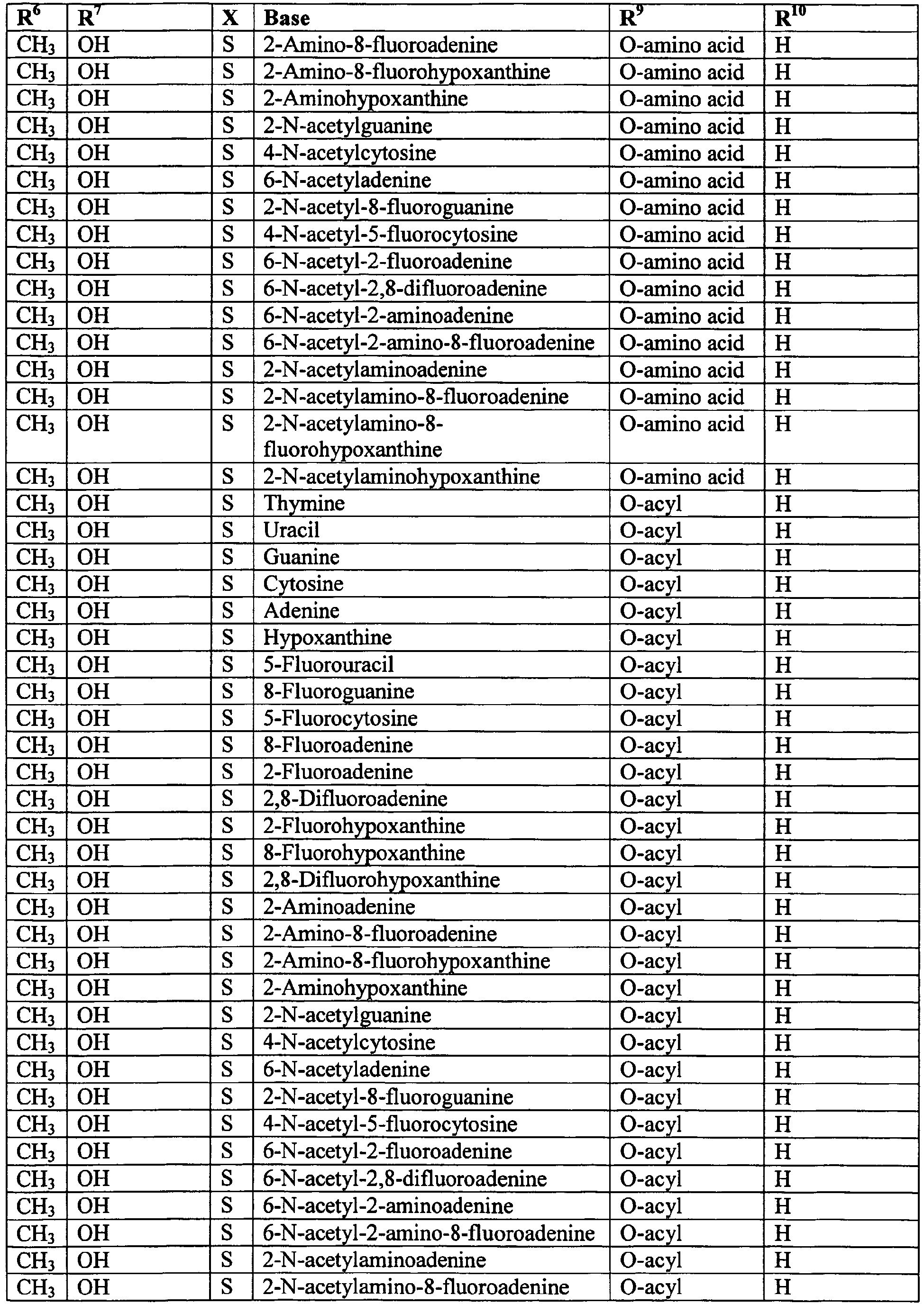

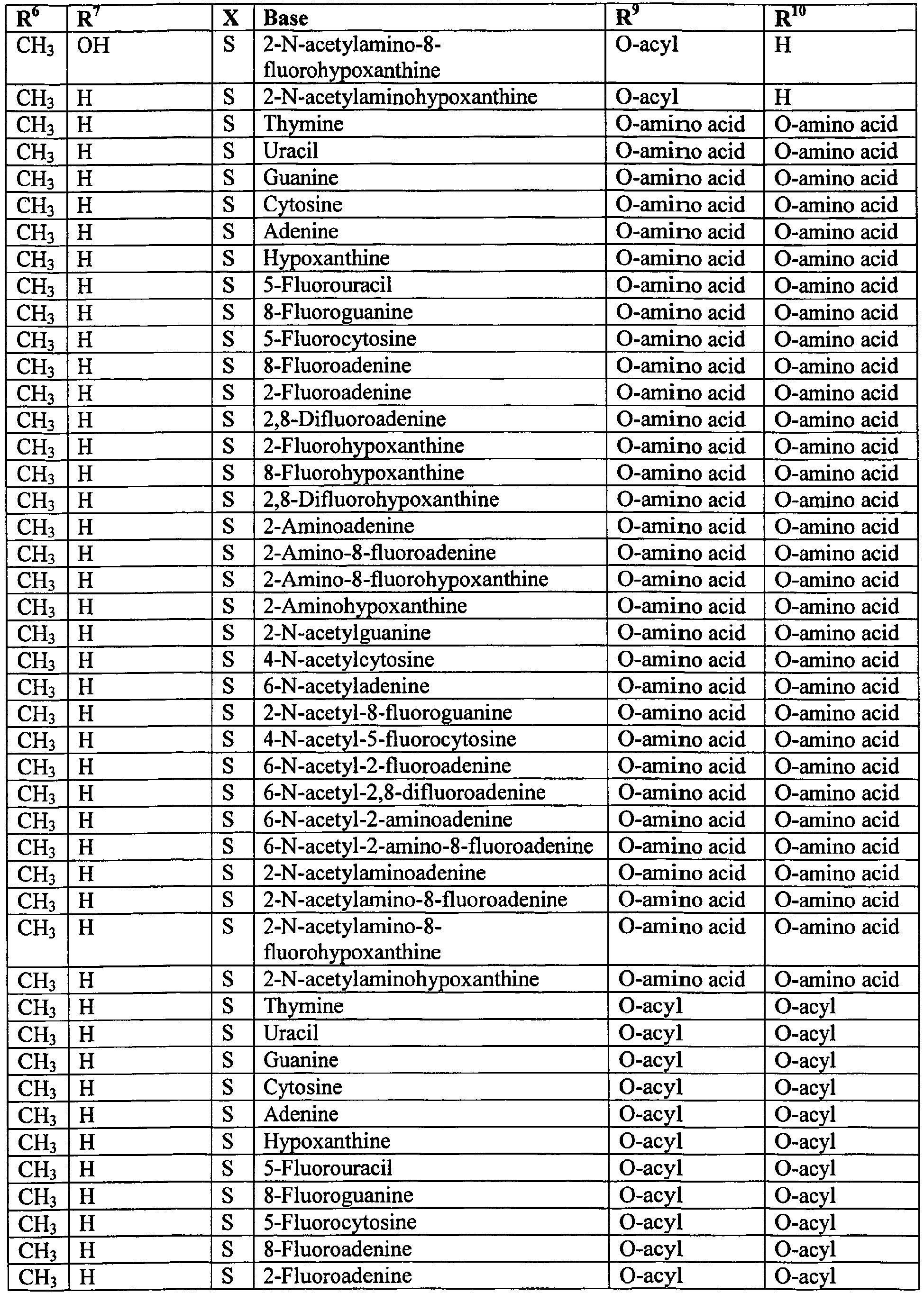

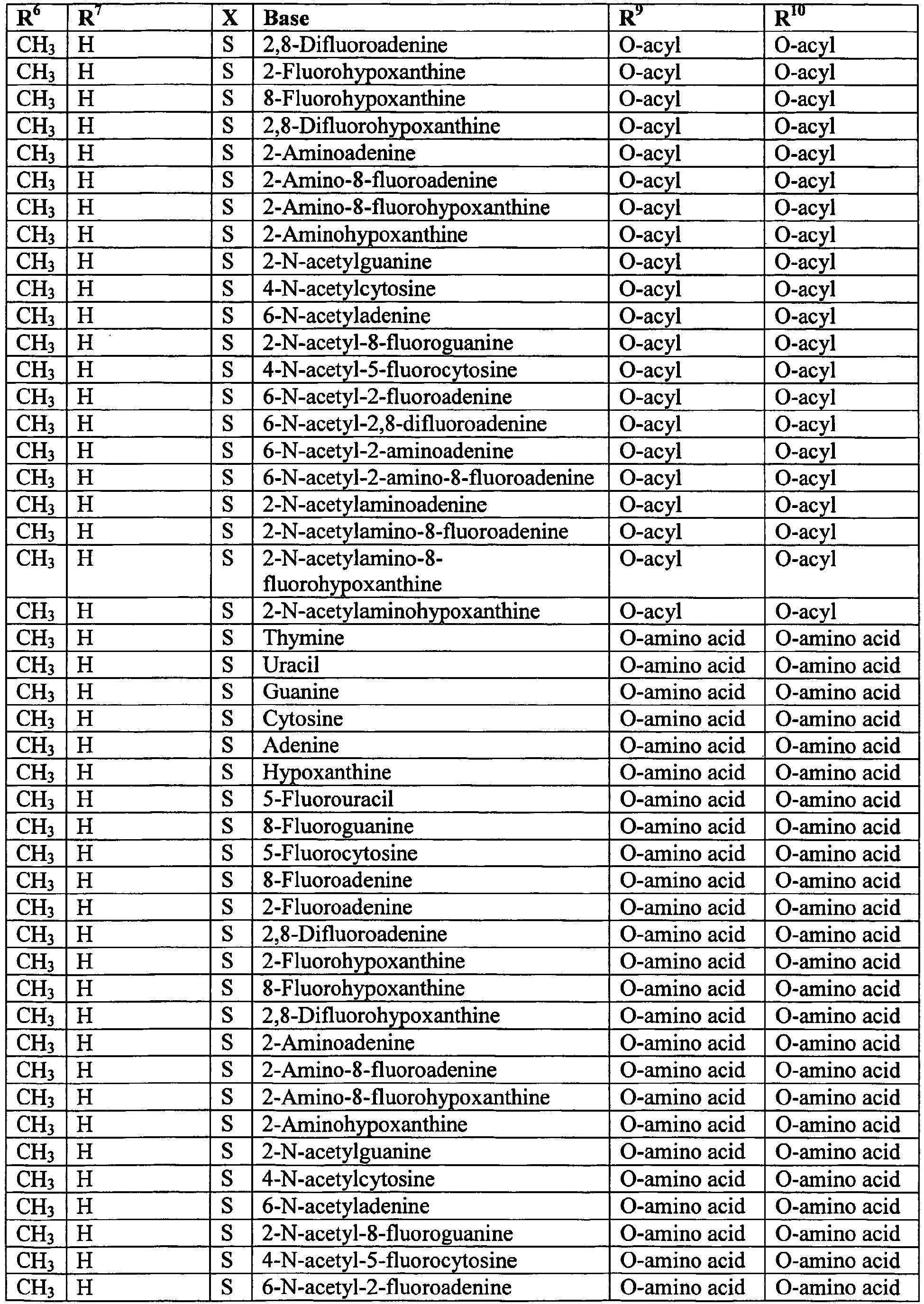

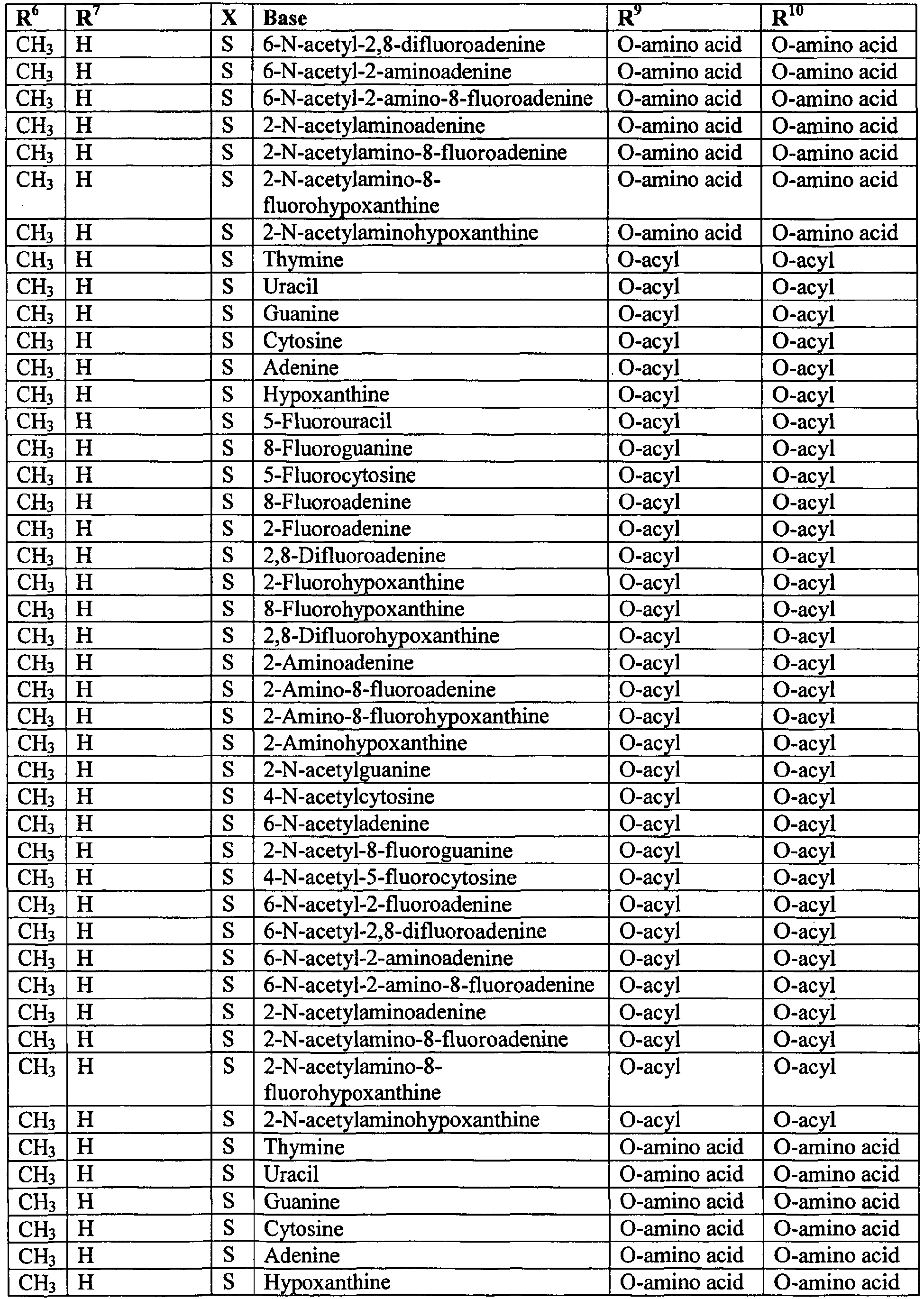

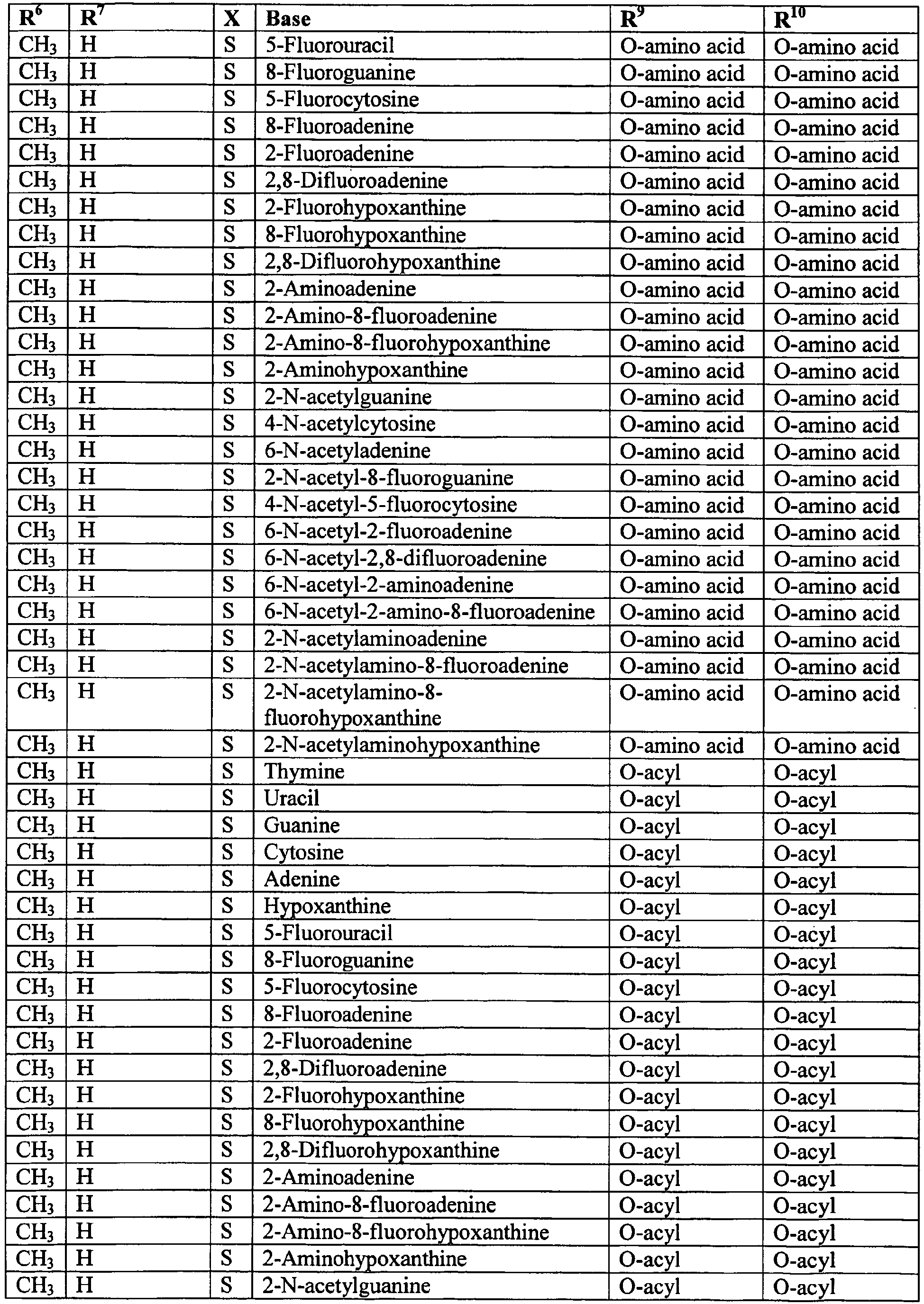

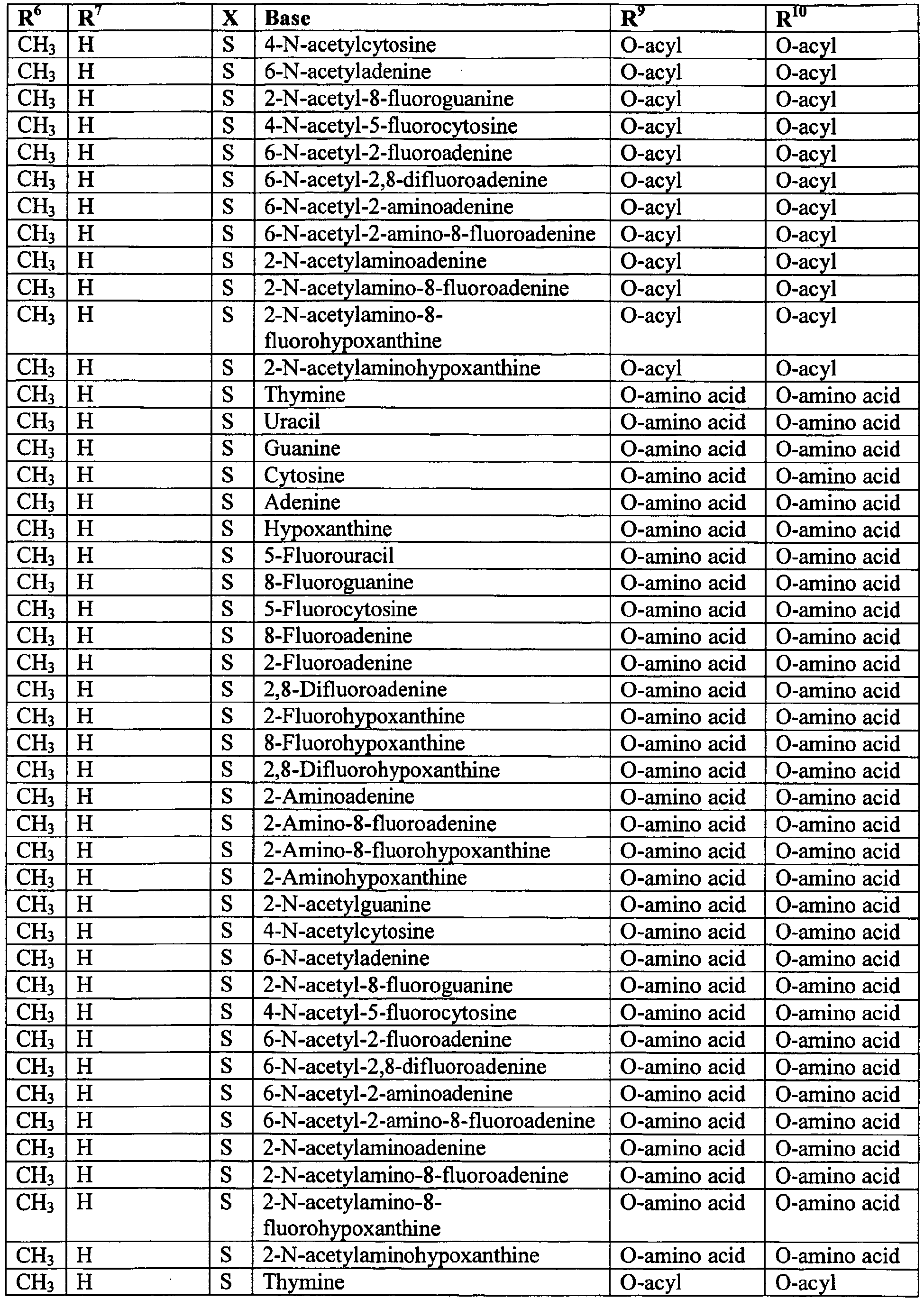

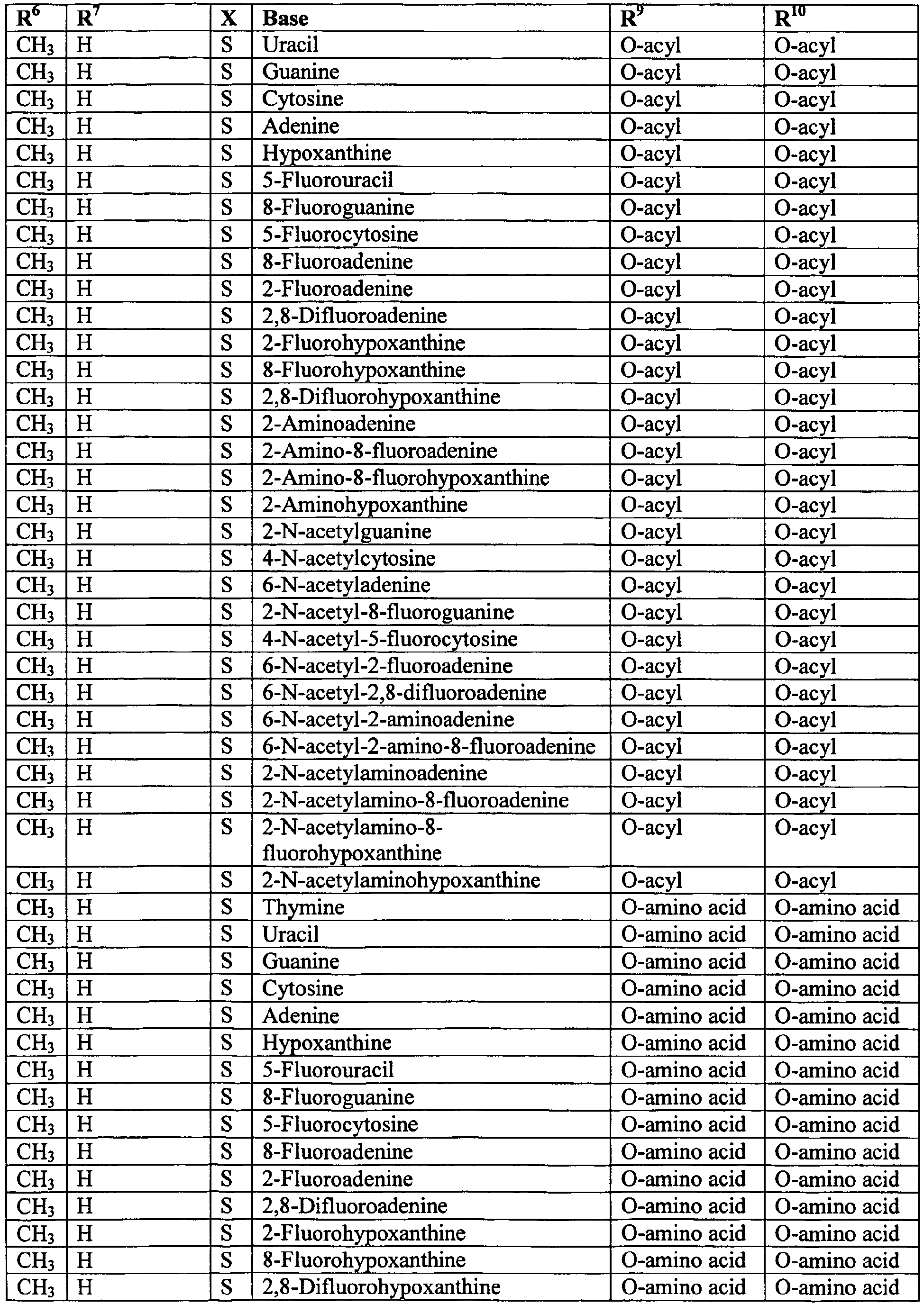

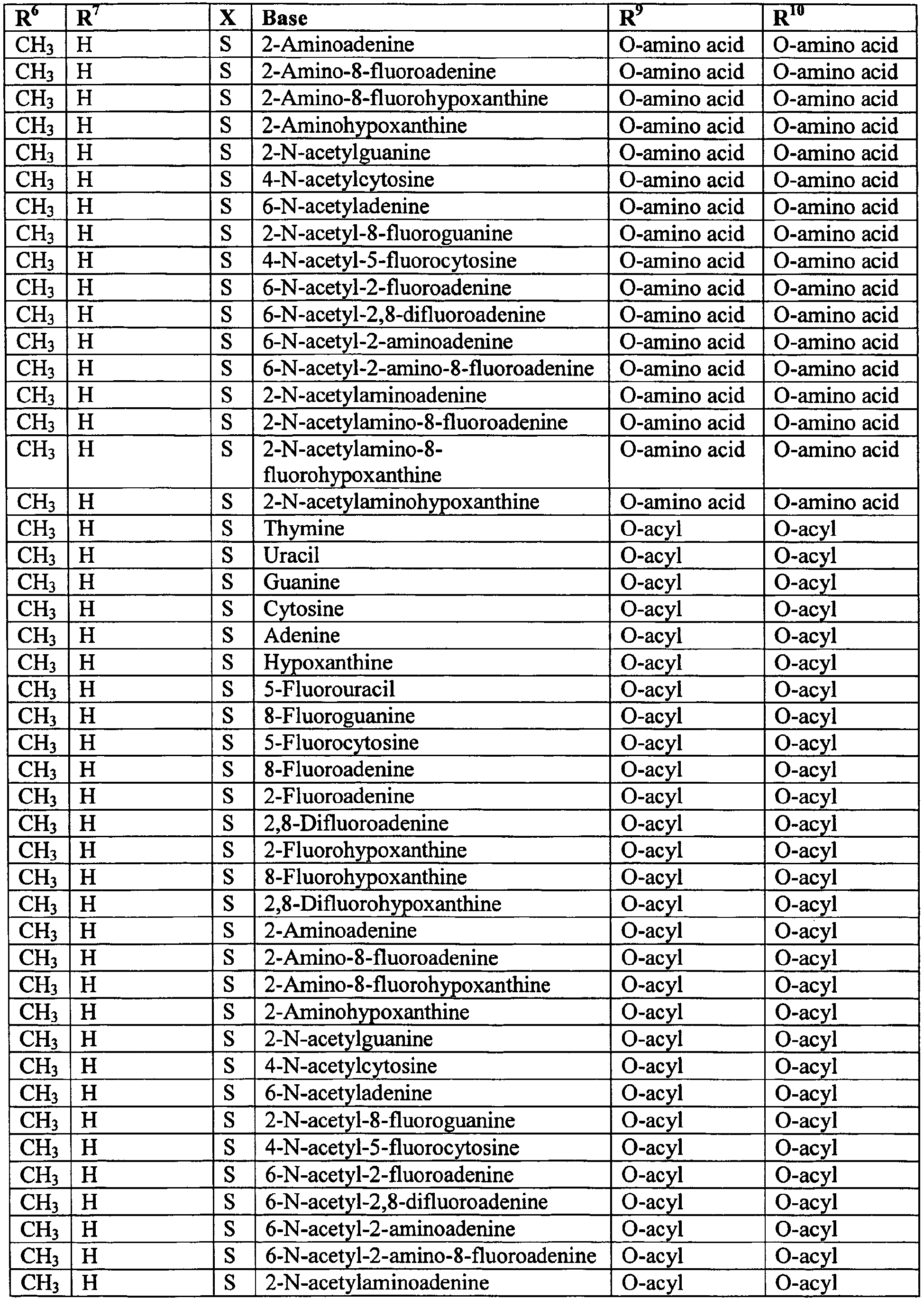

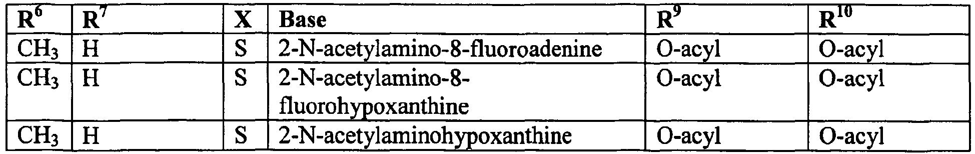

## Claims

1. A compound of formula 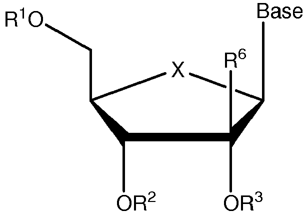 or a pharmaceutically acceptable salt thereof, for use in a method of treating a *Flaviviridae* virus infection in a host
wherein:
Base is a purine or pyrimidine base;
R¹ is H or monophosphate, diphosphate, triphosphate, or a stabilized phosphate;
R² is an amino acid residue;
R³ is H;
R⁶ is unsubstituted alkyl; and
X is O, S, SO₂ or CH₂.

2. The compound for use of claim 1, wherein R¹ is a monophosphate, diphosphate, triphosphate or a stabilized phosphate.

3. The compound for use of claim 1, wherein the compound has the formula: 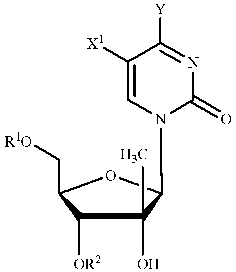 or a pharmaceutically acceptable salt thereof,
wherein:
R¹ is H or monophosphate, diphosphate, triphosphate, or a stabilized phosphate;
R² is an amino acid residue;
Y is hydrogen, bromo, chloro, fluoro, iodo, NH₂ or OH;
X¹ is H or CH₃.

4. The compound for use of any of claims 1-3 wherein the virus is hepatitis C.

5. The compound for use of any of claims 1-4, wherein the pharmaceutically acceptable salt is selected from the group consisting of a tosylate, methanesulfonate, acetate, citrate, malonate, tartarate, succinate, benzoate, ascorbate, α-ketoglutarate, α-glycerophosphate, formate, fumarate, propionate, glycolate, lactate, pyruvate, oxalate, maleate, salicylate, sulfate, nitrate, hydrobromate, hydrochloride, di-hydrochloride and phosphoric acid salt.

6. The compound for use of claim 5, wherein the pharmaceutically acceptable salt is a hydrochloride salt.

7. The compound for use of claim 5, wherein the pharmaceutically acceptable salt is a di-hydrochloride salt.

8. The compound for use of any of claims 1-7, wherein the method comprises administering the said compound in combination or alternation with a second anti-viral agent.

9. The compound for use of claim 8 wherein the second anti-viral agent is selected from the group consisting of an interferon, a ribavirin, an interleukin, a NS3 protease inhibitor, a hepatitis C virus helicase inhibitor, a polymerase inhibitor, a nucleoside, an inhibitor of IRES-dependent translation, and a ribozyme.

10. The compound for use of claim 9, wherein the second anti-viral agent is an interferon.

11. The compound for use of claim 10, wherein the second agent is selected from the group consisting of pegylated interferon alpha 2a, interferon alphacon-1, natural interferon, albuferon, interferon beta-1a, omega interferon, interferon alpha, and interferon gamma-1b.

12. The compound for use of any of claims 1-11, wherein the host is a human.

13. The compound for use of any of claims 1-12, wherein the compound is at least 85% by weight of the β-D-isomer.

14. The compound for use of any of claims 1-12, wherein the compound is at least 90% by weight of the β-D-isomer.

15. The compound for use of any of claims 1-14, wherein the compound is provided in a composition comprising a pharmaceutically acceptable carrier.

16. The compound for use of claim 15, wherein the carrier is suitable for oral delivery.

17. The compound for use of claim 15, wherein the composition is in the form of a dosage unit.

18. The compound for use of claim 17, wherein the dosage unit contains 50 to 1000 mg of the compound.

19. The compound for use of claim 17, wherein said dosage unit is a tablet or capsule.

20. The compound for use of claim 15, wherein the pharmaceutically acceptable carrier is suitable for systemic, topical, parenteral, inhalant or intravenous delivery.

## Patentansprüche

1. Verbindung der Formel 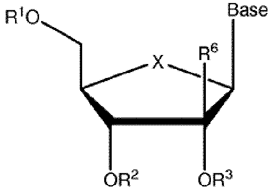 oder ein pharmazeutisch verträgliches Salz davon, zur Verwendung in einem Verfahren zur Behandlung einer *Flaviviridae*-Virusinfektion in einem Wirt,
wobei:
Base ist eine Purin- oder Pyrimidinbase;
R¹ ist H oder Monophosphat, Diphosphat, Triphosphat oder ein stabilisiertes
Phosphat;
R² ist ein Aminosäurerest;
R³ ist H;
R⁶ ist unsubstituiertes Alkyl; und
X ist O, S, SO₂ oder CH₂.

2. Verbindung zur Verwendung nach Anspruch 1, wobei R¹ ein Monophosphat, Diphosphat, Triphosphat oder ein stabilisiertes Phosphat ist.

3. Verbindung zur Verwendung nach Anspruch 1, wobei die Verbindung die Formel aufweist: 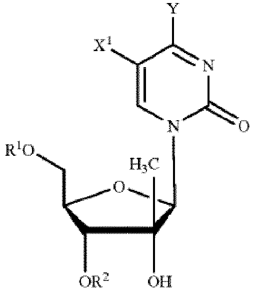 oder ein pharmazeutisch verträgliches Salz davon,
wobei:
R¹ ist H oder Monophosphat, Diphosphat, Triphosphat oder ein stabilisiertes Phosphat;
R² ist ein Aminosäurerest;
Y ist Wasserstoff, Brom, Chlor, Fluor, Iod, NH₂ oder OH;
X¹ ist H oder CH₃.

4. Verbindung zur Verwendung nach einem der Ansprüche 1 bis 3, wobei das Virus Hepatitis C ist.

5. Verbindung zur Verwendung nach einem der Ansprüche 1 bis 4, wobei das pharmazeutisch verträgliche Salz ausgewählt ist von der Gruppe bestehend aus einem Tosylat, Methansulfonat, Acetat, Citrat, Malonat, Tartrat, Succinat, Benzonat, Ascorbat, α-Ketoglutarat, α-Glycerophosphat, Formiat, Fumarat, Propionat, Glykolat, Lactat, Pyruvat, Oxalat, Maleat, Salicylat, Sulfat, Nitrat, Hydrobromat, Hydrochlorid, Di-Hydrochlorid und Phosphorsäuresalz.

6. Verbindung zur Verwendung nach Anspruch 5, wobei das pharmazeutisch verträgliche Salz ein Hydrochloridsalz ist.

7. Verbindung zur Verwendung nach Anspruch 5, wobei das pharmazeutisch verträgliche Salz ein Di-Hydrochloridsalz ist.

8. Verbindung zur Verwendung nach einem der Ansprüche 1 bis 7, wobei das Verfahren die Verabreichung der Verbindung umfasst in Kombination oder Abwechslung mit einem zweiten antiviralen Mittel.

9. Verbindung zur Verwendung nach Anspruch 8, wobei das zweite antivirale Mittel ausgewählt ist von der Gruppe bestehend aus einem Interferon, einem Ribavirin, einem Interleukin, einem NS3-Protease-Inhibitor, einem Hepatitis-C-Virus-Helicase-Inhibitor, einem Polymerase-Inhibitor, einem Nukleosid, einem Inhibitor der IRES-abhängigen Translation und einem Ribozym.

10. Verbindung zur Verwendung nach Anspruch 9, wobei das zweite antivirale Mittel ein Interferon ist.

11. Verbindung zur Verwendung nach Anspruch 10, wobei das zweite Mittel ausgewählt ist von der Gruppe bestehend aus pegyliertem Interferon alpha-2a, Interferon alphacon-1, natürliches Interferon, Albuferon, Interferon beta-1a, Omega-Interferon, Interferon alpha und Interferon gamma-1b.

12. Verbindung zur Verwendung nach einem der Ansprüche 1-11, wobei der Wirt ein Mensch ist.

13. Verbindung zur Verwendung nach einem der Ansprüche 1-12, wobei die Verbindung zumindest 85 Gewichtsprozent von dem β-D-Isomer ist.

14. Verbindung zur Verwendung nach einem der Ansprüche 1 - 12, wobei die Verbindung zumindest 90 Gewichtsprozent von dem β-D-Isomer ist.

15. Verbindung zur Verwendung nach einem der Ansprüche 1-14, wobei die Verbindung in einer Zusammensetzung ist, welche einen pharmazeutisch verträglichen Träger umfasst.

16. Verbindung zur Verwendung nach Anspruch 15, wobei der Träger zur oralen Verabreichung geeignet ist.

17. Verbindung zur Verwendung nach Anspruch 15, wobei die Verbindung in der Form von einer Dosierungseinheit ist.

18. Verbindung zur Verwendung nach Anspruch 17, wobei die Dosierungseinheit 50 bis 1.000 mg von der Verbindung enthält.

19. Verbindung zur Verwendung nach Anspruch 17, wobei die Dosierungseinheit eine Tablette oder Kapsel ist.

20. Verbindung zur Verwendung nach Anspruch 15, wobei der pharmazeutisch verträgliche Träger geeignet ist für systemische, topische, parenterale, inhalative oder intravenöse Verabreichung.

## Revendications

1. Composé de formule : 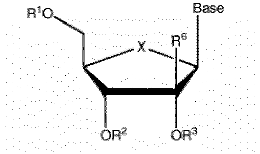 ou un sel pharmaceutiquement acceptable de celui-ci, pour une utilisation dans une méthode de traitement d'un hôte infecté par un virus *Flaviviridae* où:
Base est une base purique ou pyrimidique;
R¹ est un H, ou un monophosphate, diphosphate, triphosphate, ou un phosphate stabilisé;
R² est un résidu d'acide aminé;
R³ est un H;
R⁶ est un alkyle non substitué; et
X est un O, S, SO₂ ou CH₂.

2. Composé pour une utilisation selon la revendication 1, où R¹ est un monophosphate, diphosphate, triphosphate ou un phosphate stabilisé.

3. Composé pour une utilisation selon la revendication 1, où le composé a une formule : 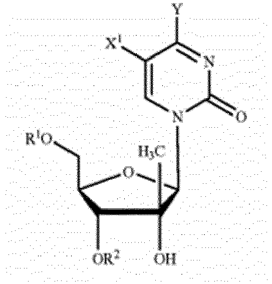 ou un sel pharmaceutiquement acceptable de celui-ci, où:
R¹ est un H, ou un monophosphate, diphosphate, triphosphate, ou un phosphate stabilisé;
R² est un résidu d'acide aminé;
Y est un hydrogène, bromo, chloro, fluoro, iodo, NH₂ ou OH; et
X¹ est un H ou CH₃.

4. Composé pour une utilisation selon l'une quelconque des revendications 1-3 où le virus est l'hépatite C.

5. Composé pour une utilisation selon l'une quelconque des revendications 1-4 où le sel pharmaceutiquement acceptable est choisi dans le groupe constitué par un tosylate, méthanesulfonate, acétate, citrate, malonate, tartarate, succinate, benzoate, ascorbate, α-cétoglutarate, α-glycérophosphate, formate, fumarate, propionate, glycolate, lactate, pyruvate, oxalate, maléate, salicylate, sulfate, nitrate, bromhydrate, chlorhydrate, di-chlorhydrate, et un sel d'acide phosphorique.

6. Composé pour une utilisation selon la revendication 5, où le sel pharmaceutiquement acceptable est un sel de chlorhydrate.

7. Composé pour une utilisation selon la revendication 5, où le sel pharmaceutiquement acceptable est un sel de di-chlorhydrate.

8. Composé pour une utilisation selon l'une quelconque des revendications 1-7, où la méthode comprend une administration dudit composé en combinaison ou en alternance avec un second agent antiviral.

9. Composé pour une utilisation selon la revendication 8, où le second agent antiviral est choisi dans le groupe constitué par un interféron, une ribavirine, une interleukine, un inhibiteur de protéase NS3, un inhibiteur de l'hélicase du virus de l'hépatite C, un inhibiteur de polymérase, un inhibiteur de la traduction IRES-dépendante et un ribozyme.

10. Composé pour une utilisation selon la revendication 9, où le second agent antiviral est un interféron.

11. Composé pour une utilisation selon la revendication 10, où le second agent est choisi dans le groupe constitué par l'interféron alpha 2a pégylé, l'interféron alphacon-1, l'interféron naturel, l'albuferon, l'interféron beta-1a, l'interféron oméga, l'interféron alpha, et l'interféron gamma-1b.

12. Composé pour une utilisation selon l'une quelconque des revendications 1-11, où l'hôte est un humain.

13. Composé pour une utilisation selon l'une quelconque des revendications 1-12, où le composé est au moins égal à 85 % en poids de β-D-isomère.

14. Composé pour une utilisation selon l'une quelconque des revendications 1-12, où le composé est au moins égal à 90 % en poids de β-D-isomère.

15. Composé pour une utilisation selon l'une quelconque des revendications 1-14, où le composé est fourni dans une composition comprenant un support pharmaceutiquement acceptable.

16. Composé pour une utilisation selon la revendication 15, où le support est approprié pour une administration orale.

17. Composé pour une utilisation selon la revendication 15, où la composition est sous la forme d'une unité de dosage.

18. Composé pour une utilisation selon la revendication 17, où l'unité de dosage contient 50 à 1000 mg du composé.

19. Composé pour une utilisation selon la revendication 17, où l'unité de dosage est un comprimé ou une capsule.

20. Composé pour une utilisation selon la revendication 15, où le support pharmaceutiquement acceptable est approprié pour une administration systémique, topique, parentérale, par inhalation ou par intraveineuse.
